(19) Europäisches Patentamt / European Patent Office / Office européen des brevets

(11) **EP 3 692 164 B1**

(12) **EUROPEAN PATENT SPECIFICATION**

(45) Date of publication and mention of the grant of the patent:
**15.10.2025   Bulletin 2025/42**

(21) Application number: **18863856.3**

(22) Date of filing: **05.10.2018**

(51) International Patent Classification (IPC):
**C12Q 1/68** *(2018.01)*      **C12Q 1/08** *(2006.01)*
**C12Q 1/686** *(2018.01)*

(52) Cooperative Patent Classification (CPC):
**C12Q 1/6886; C12Q 2600/112; C12Q 2600/154**

(86) International application number:
**PCT/US2018/054660**

(87) International publication number:
**WO 2019/071161 (11.04.2019 Gazette 2019/15)**

(54) **METHYLATION MARKERS FOR DIAGNOSING CANCER**

METHYLIERUNGSMARKER ZUR DIAGNOSE VON KREBS

MARQUEURS DE MÉTHYLATION POUR LE DIAGNOSTIC DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.10.2017   US 201762569459 P**
                **18.05.2018   US 201862673593 P**

(43) Date of publication of application:
**12.08.2020   Bulletin 2020/33**

(73) Proprietors:
• **Helio Health Inc.**
  **Irvine, California 92618 (US)**
• **The Regents of the University of California**
  **Oakland, CA 94607 (US)**

(72) Inventors:
• **ZHANG, Kang**
  **San Diego, California 92130 (US)**
• **HOU, Rui**
  **Pujiangyuan**
  **Shenyang 110000 (CN)**
• **ZHENG, Lianghong**
  **Pujiangyuan**
  **Shenyang 110000 (CN)**
• **LI, Gen**
  **Pujiangyuan**
  **Shenyang 110000 (CN)**

(74) Representative: **HGF**
  **HGF Limited**
  **1 City Walk**
  **Leeds LS11 9DX (GB)**

(56) References cited:
EP-A2- 2 497 834          WO-A1-2016/115530
WO-A2-2015/021263        WO-A2-2015/091979
US-A1- 2014 255 418      US-A1- 2016 210 403
US-A1- 2019 194 755      US-B2- 10 266 900

• RASMUSSEN SIMON LADEFOGED ET AL: "The prognostic efficacy of cell-free DNA hypermethylation in colorectal cancer", ONCOTARGET, vol. 9, no. 6, 23 January 2018 (2018-01-23), United States, pages 7010 - 7022, XP055806493, ISSN: 1949-2553, DOI: 10.18632/oncotarget.24097
• DE MELLO, VD ET AL.: "Human Liver Epigenetic Alterations in Nonalcoholic Steatohepatitis are Related to Insulin Action", EPIGENETICS, vol. 12, no. 4, 23 February 2017 (2017-02-23), pages 287 - 295, XP055589771

## EP 3 692 164 B1

**Description**

**CROSS-REFERENCE**

This application claims the benefit of U.S. Provisional Application No. 62/569,459, filed October 6, 2017, and U.S. Provisional Application No. 62/673,593, filed May 18, 2018.

**BACKGROUND OF THE DISCLOSURE**

Cancer is a leading cause of deaths worldwide, with annual cases expected to increase from 14 million in 2012 to 22 million during the next two decades (WHO). Diagnostic procedures for liver cancer, in some cases, begin only after a patient is already present with symptoms, leading to costly, invasive, and sometimes time-consuming procedures. In addition, inaccessible areas sometimes prevent an accurate diagnosis. Further, high cancer morbidities and mortalities are associated with late diagnosis.

**SUMMARY OF THE DISCLOSURE**

_ Disclosed herein is a method of detecting a methylation pattern of a set of biomarkers in a subject suspected of having a colorectal cancer, the method comprising: (a) processing an extracted genomic DNA with a deaminating agent to generate a genomic DNA sample comprising deaminated nucleotides, wherein the extracted genomic DNA is obtained from a biological sample from the subject; and (b) detecting the methylation pattern of the set of biomarkers from the processed extracted genomic DNA, wherein the set of biomarkers comprises cg10673833, cg10493436, cg10428836, cg27284288, cg16959747, cg17494199, cg23678254, cg24067911, and cg25459300, by contacting the extracted genomic DNA with a set of probes, wherein the set of probes hybridizes to the one or more biomarkers, and perform a DNA sequencing analysis to determine the methylation patter of the one or more biomarkers.

**The invention is defined in the appended claims. Any of the following figures that do not fall within the scope of the claims are provided for illustrative or comparative purposes only.**

**BRIEF DESCRIPTION OF THE DRAWINGS**

Various aspects of the disclosure are set forth with particularity in the appended claims. The file of this patent contains at least one drawing/photograph executed in color. Copies of this patent with color drawing(s)/photograph(s) will be provided by the Office upon request and payment of the necessary fee. A better understanding of the features and advantages of the present disclosure will be obtained by reference to the following detailed description that sets forth illustrative embodiments, in which the principles of the disclosure are utilized, and the accompanying drawings of which:

Fig. 1 illustrates the methylation status of biomarker 7-1577016.
Fig. 2 illustrates the methylation status of biomarker 11-67177103.
Fig. 3 illustrates the methylation status of biomarker 19-10445516 (cg17126555).
Fig. 4 illustrates the methylation status of biomarker 12-122277360.
Fig. 5 illustrates the methylation status of biomarker 6-72130742 (cg24772267).
Fig. 6 illustrates the methylation status of biomarker 3-15369681.
Fig. 7 illustrates the methylation status of biomarker 3-131081177.

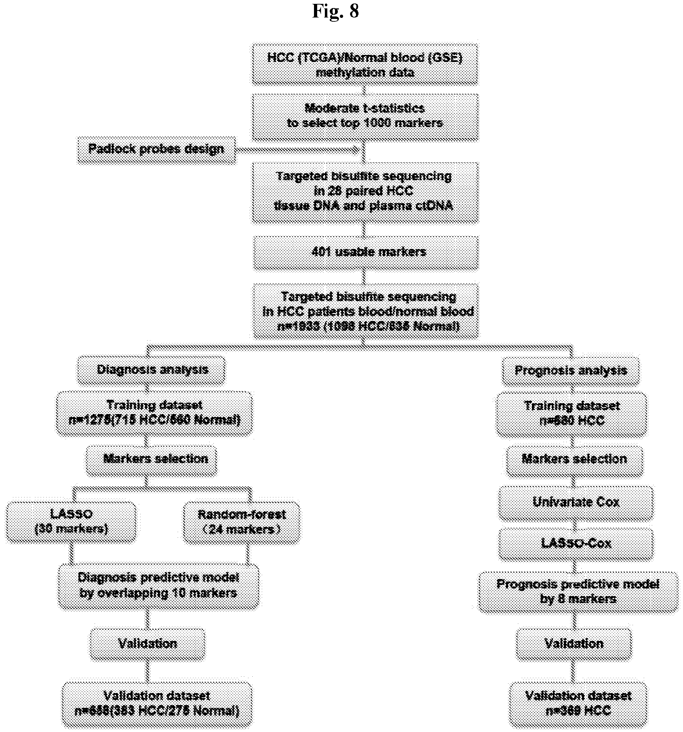

Fig. 8 illustrates workflow chart of data generation and analysis. Whole genome methylation data on HCC and normal lymphocytes were used to identify 401 candidate markers. Diagnostic marker selection: Lasso and Random-forest analyses were applied to a training cohort of 715 HCC and 560 normal patients to identify a final selection of 10 markers. These ten markers were applied to a validation cohort of 383 HCC and 275 normal patients. Prognostic marker selection: univariant-cox and LASSO-Cox were applied to a training cohort of 680 HCC patients with survival data to identify a final selection of eight markers. These eight markers were applied to a validation cohort of 369 HCC with survival data.

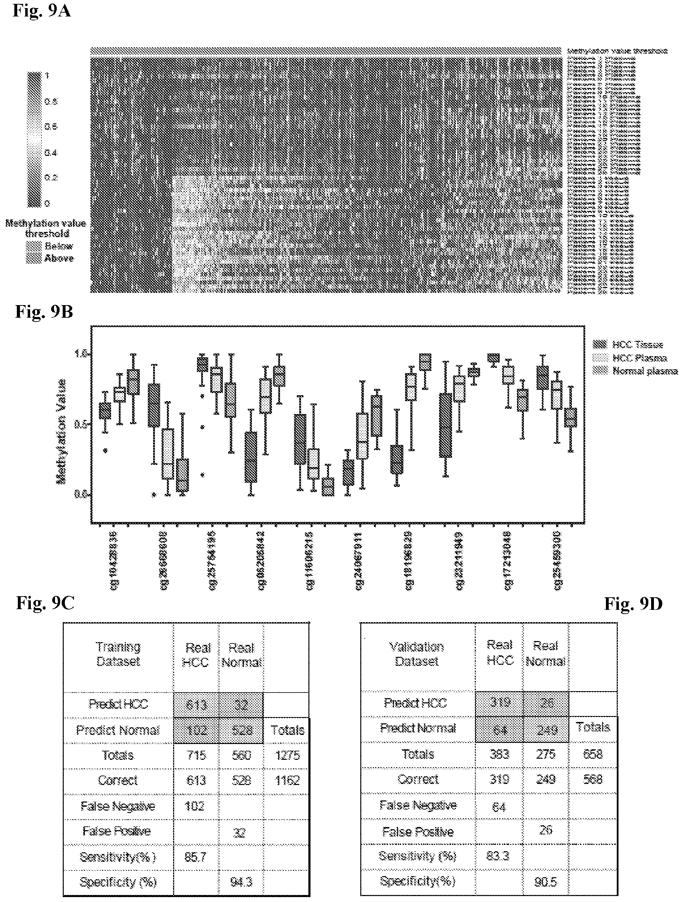

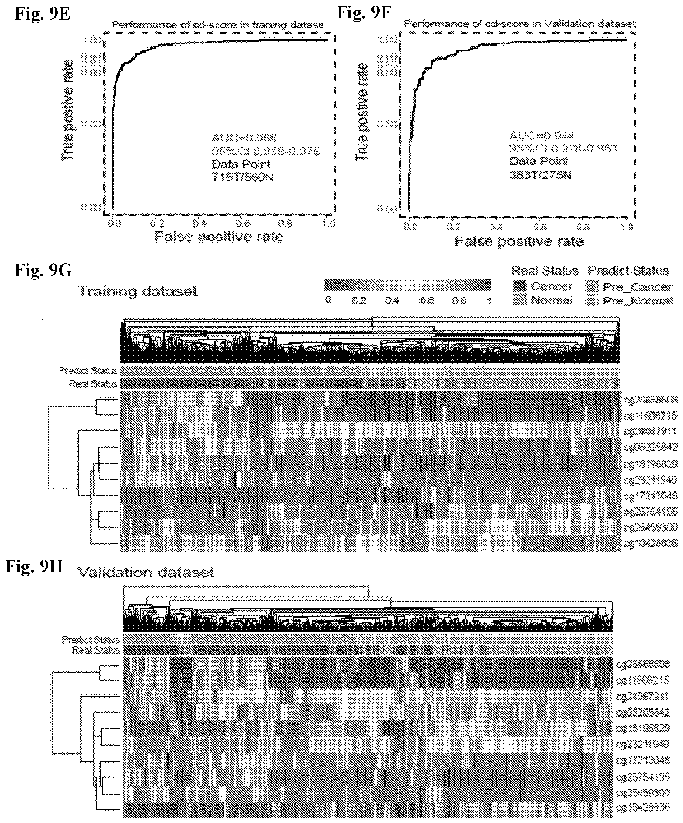

Fig. 9A-Fig. 9H illustrate cfDNA methylation analysis for diagnosis of HCC. Fig. 9A shows the heatmap of methylation of 28 pairs of matched HCC tumor DNA and plasma cfDNA, with a mean methylation value threshold of 0.1 as a cutoff. Fig. 9B shows the methylation values and standard deviations of ten diagnostic markers in normal plasma, HCC tumor DNA, and HCC patient cfDNA. Fig. 9C and Fig. 9D show the confusion tables of binary results of the diagnostic prediction model in the training (Fig. 9C) and validation datasets (Fig. 9D). Fig. 9E and Fig. 9F illustrate ROC of the diagnostic prediction model with methylation markers in the training (Fig. 9E) and validation datasets (Fig. 9F). Fig. 9G and Fig. 9H show the unsupervised hierarchical clustering of ten methylation markers selected for use in the diagnostic prediction model in the training (Fig. 9G) and validation datasets (Fig. 9H).

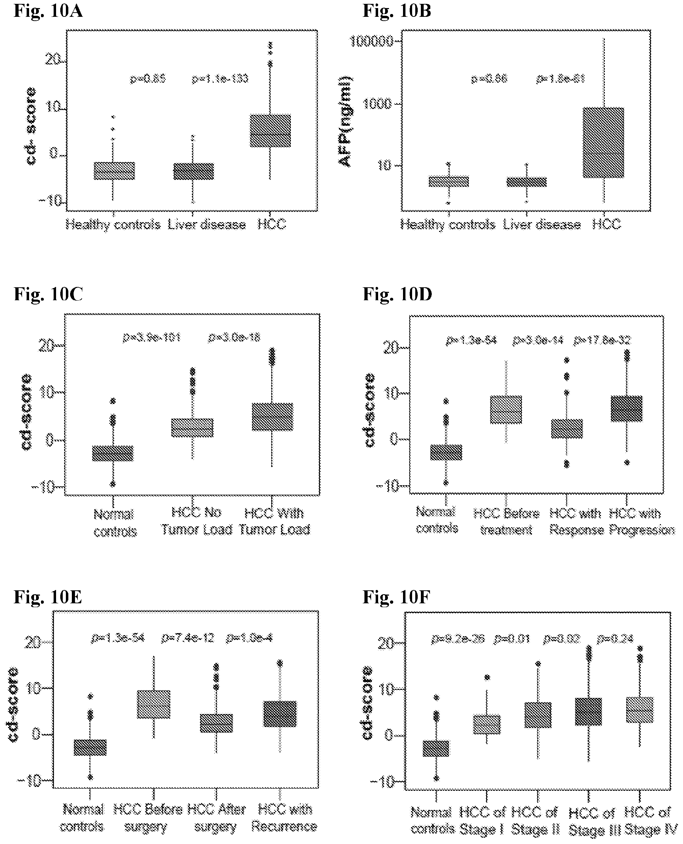

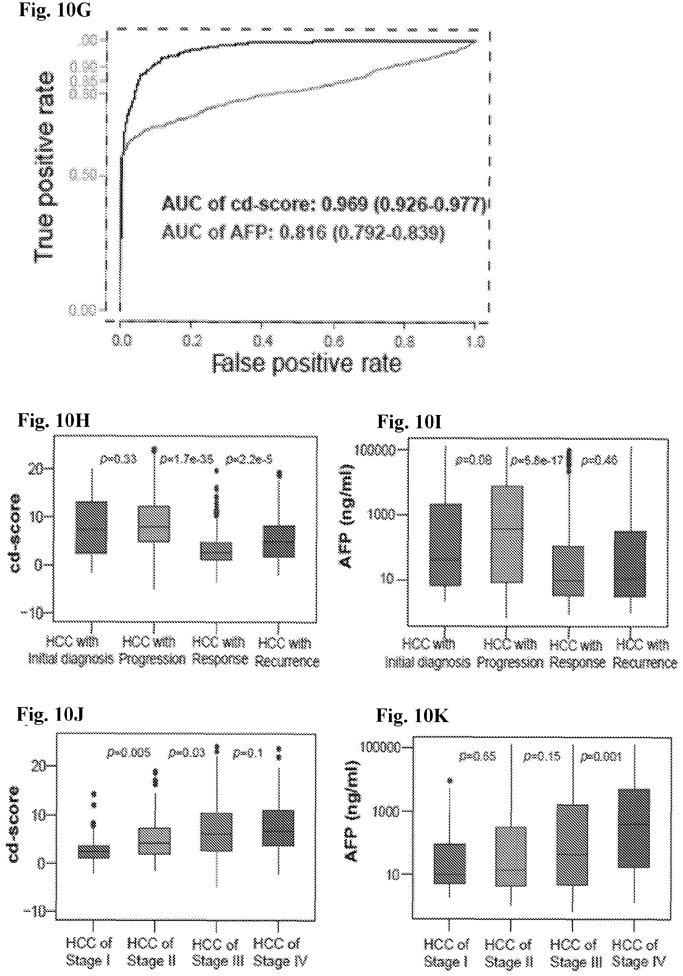

Fig. 10A-Fig. 10K illustrate cfDNA methylation analysis and tumor burden, treatment response, and staging. The combined diagnosis score (cd-score) (Fig. 10A) and AFP (Fig. 10B) in healthy controls, individuals with liver diseases (HBV/HCV infection, cirrhosis, and fatty liver) and HCC patients. Fig. 10C shows the cd-score in normal controls and HCC patients with and without detectable tumor burden. Fig. 10D shows the cd-score in normal controls, HCC patients before treatment, with treatment response, and with progression. Fig. 10E shows the cd-score in normal controls and HCC patients before surgery, after surgery, and with recurrence. Fig. 10F shows the cd-score in normal controls and HCC patients from stage I-IV. Fig. 10G shows the ROC of cd-score and AFP for HCC diagnosis in whole HCC cohort. cd-score (Fig. 10H) and AFP (Fig. 10I) in HCC patients with initial diagnosis (before surgery or other treatment), with treatment response, with progression, and with recurrence. cd-score (Fig. 10J) and AFP (Fig. 10K) in HCC patients from stage I-IV.

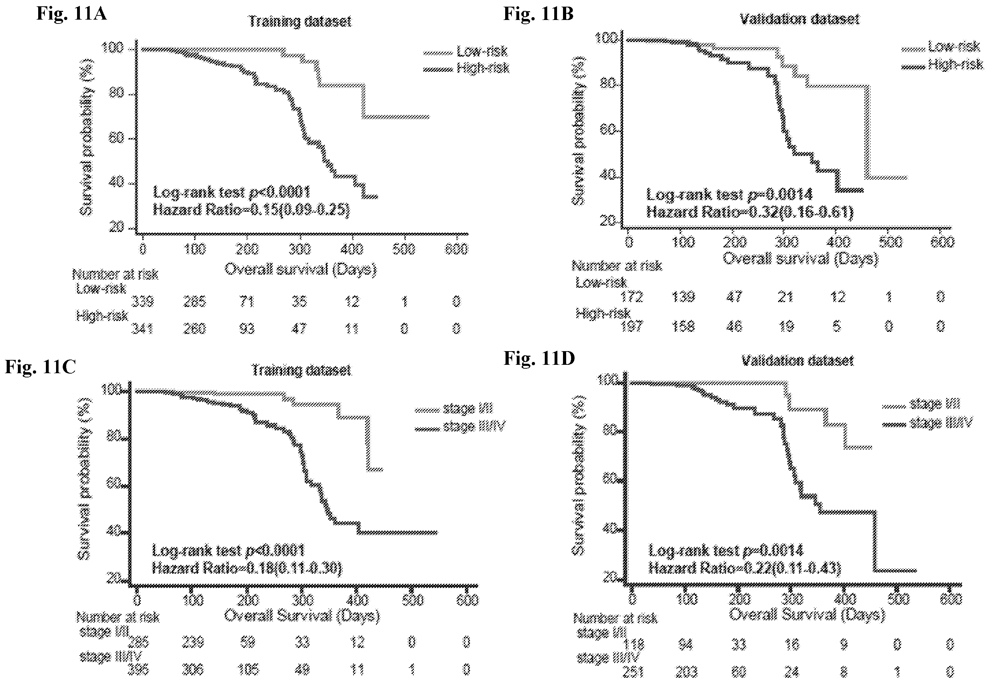

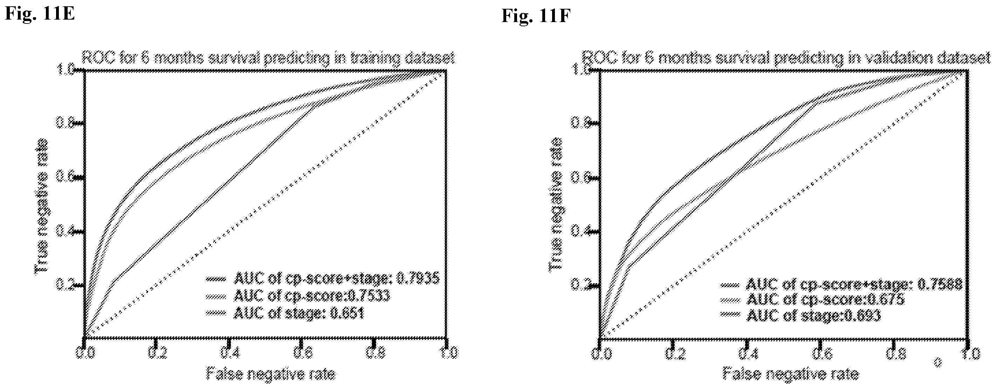

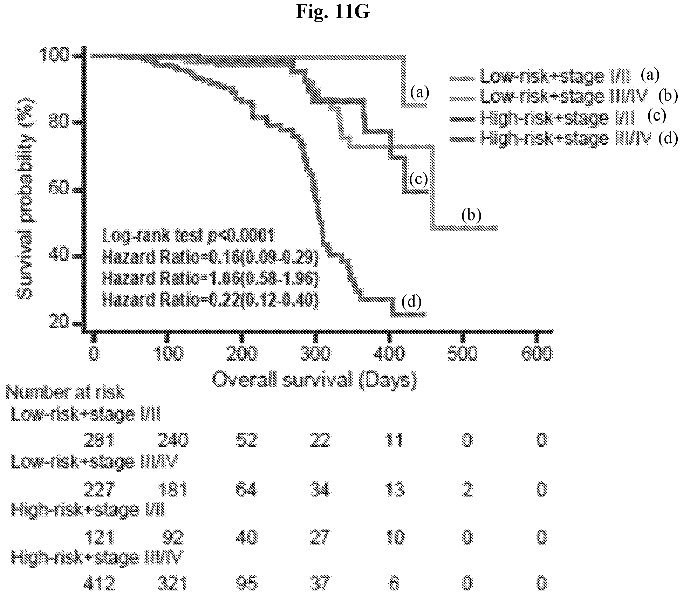

Fig. 11A-Fig. 11G illustrate cfDNA methylation analysis for prognostic prediction HCC survival. Fig. 11A and Fig. 11B show the overall survival curves of HCC patients with low or high risk of death at 6 months, according to the combined prognosis score (cp-score) in the training (Fig. 11A) and validation datasets (Fig. 11B). Survival curves of HCC patients with stage I/II and stage III/IV in the training (Fig. 11C) and validation datasets (Fig. 11D). The ROC for the cp-score, stage, and cp-score combined with stage in the training (Fig. 11E) and validation datasets (Fig. 11F). Fig. 11G shows the survival curves of HCC patients with combinations of cp-score risk and stage in the whole HCC cohort.

Fig. 12 illustrates an unsupervised hierarchical clustering of top 1000 methylation markers differentially methylated between HCC tumor DNA and normal blood. Each column represents an individual patient and each row represents a CpG marker.

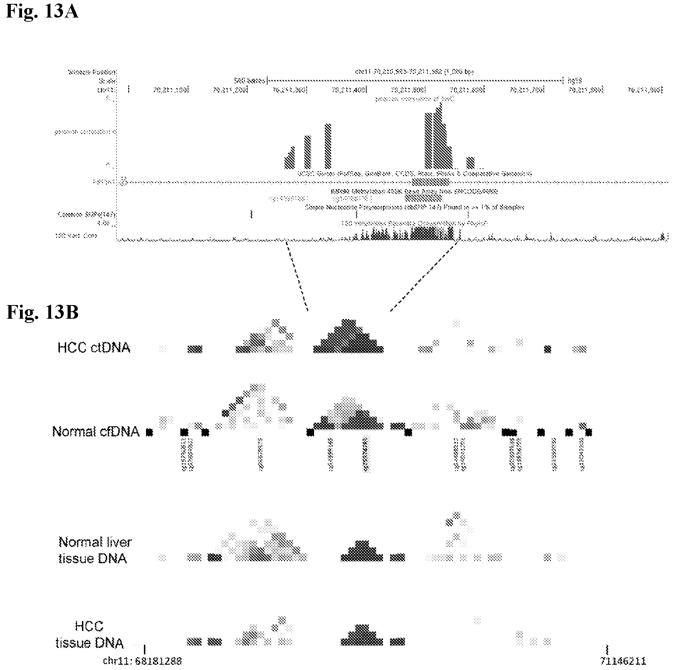

Fig. 13A-Fig. 13B illustrate an exemplary region encompassing two Blocks of Correlated Methylation (BCM) in cfDNA samples of from HCC and normal controls. Fig. 13A shows a genomic neighborhood of the BCM displayed within UCSC genome browser (Pearson correlation track showed correlation data by summing r values for a marker within a BCM. Cg marker names below the Pearson correlation graph (cg14999168, cg14088196, cg25574765) were methylation markers from TCGA. Gene name and common SNPs were also listed. Fig. 13B shows a not-to-scale representation of a set of analyzed cg markers belonging to two BCMs in this region. Boundaries between blocks are indicated by a black rectangle, whereas red squares indicate correlated methylation (r>0.5) between two nearby markers. Correlation between any two markers is represented by a square at the intersection of (virtual) perpendicular lines originating from these two markers. White color indicates no significant correlation. 10 newly identified methylation markers in the left MCB anchored by marker cg14999168 or 11 newly identified methylation markers in the right MCB anchored by cg14088196/cg 25574765 were highly consistent and correlated among HCC ctDNA, normal cfDNA, and HCC tissue DNA. Using markers within the same MCB can significantly enhanced allele calling accuracy. Vertical lines at the bottom of panel b were genomic coordinates of boundaries of two MCBs.

Fig. 14 illustrates an unsupervised hierarchical clustering of exemplary methylation markers for Stage I-Stage IV HCC tumor.

Fig. 15 shows methylation values correlated with treatment outcomes in HCC patients with serial plasma samples. Fig. 15A shows a change in cd-score comparing patients after surgery, with clinical response, and with disease progression (*** $p$<0.001). Fig. 15B shows cd-score trends in individual patients after complete surgical resection with treatment response, and with disease progression. PRE: pre-treatment; POST: after-treatment.

Fig. 16 illustrates a dynamic monitoring of treatment outcomes in individual patients with cd-score and AFP. Dates of treatments are indicated by vertical blue arrows. PD, progressive disease; PR partial response; SD, stable disease; TACE, trans-catheter arterial chemoembolization.

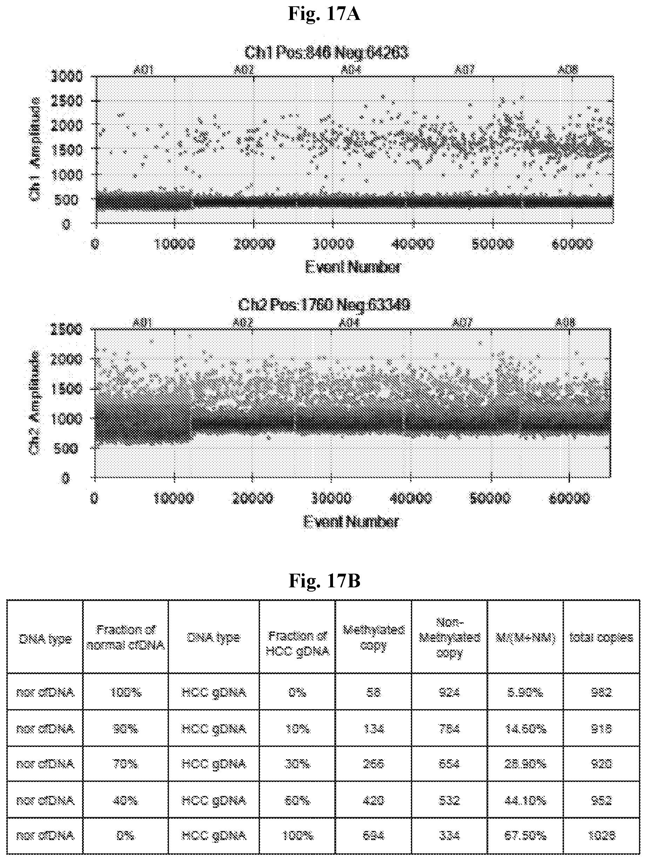

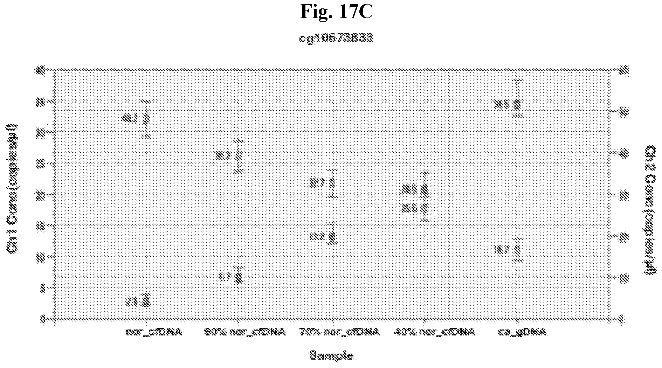

Fig. 17A-Fig. 17C illustrates data analysis of an exemplary marker cg10673833.

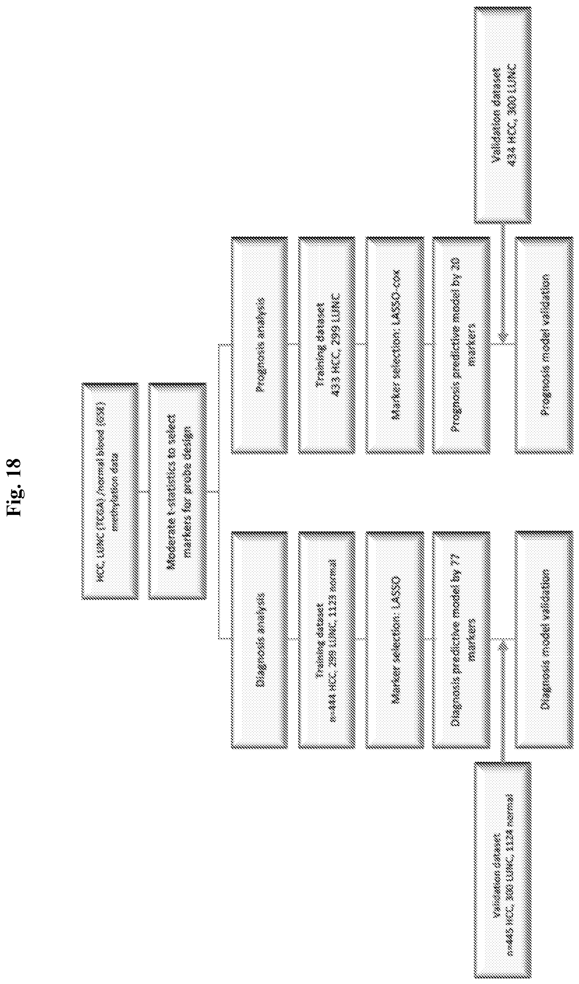

Fig. 18 illustrates a workflow for building the diagnostic and prognostic models. Whole genome methylation data on HCC, LUNC and normal blood were used to identify candidate markers for probe design. Left panel: diagnostic marker selection: LASSO analysis was applied to a training cohort of 444 HCC, 299 LUNC, and 1123 normal patients to identify a final selection of 77 markers. These 77 markers were applied to a validation cohort of 445 HCC, 300 LUNC, and 1124 normal patients. Right panel: prognostic marker selection: LASSO-Cox were applied to a training cohort of 433 HCC and 299 LUNC patients with survival data to identify a final selection of 20 markers. These 20 markers were applied to a validation cohort of 434 HCC and 300 LUNC with survival data.

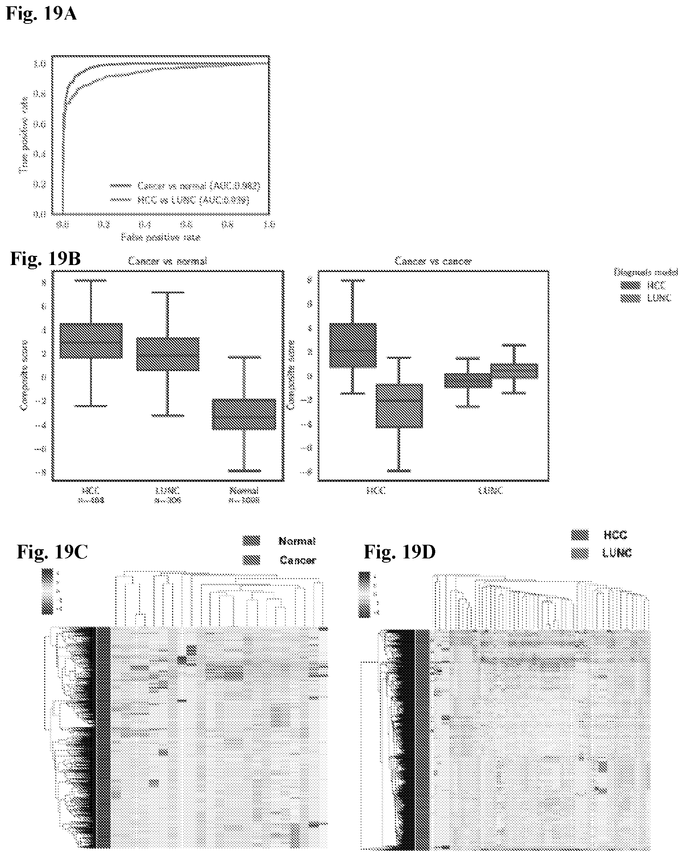

Fig. 19A-Fig. 19D illustrates cfDNA methylation analysis for diagnosis of LUNC and HCC. Fig. 19A shows receiver operating characteristic (ROC) curves and the associated Area Under Curves (AUCs) of the diagnostic prediction model (cd-score) using cfDNA methylation analysis in the validation cohort. Fig. 19B shows box plot of composite scores used to classify normal and cancer patients (left), and LUNC and HCC patients (right). Unsupervised hierarchical clustering of methylation markers differentially methylated between cancer (HCC and LUNC) and normal (Fig. 19C) and between HCC and LUNC (Fig. 19D). Each row represents an individual patient and each column represents a MCB marker.

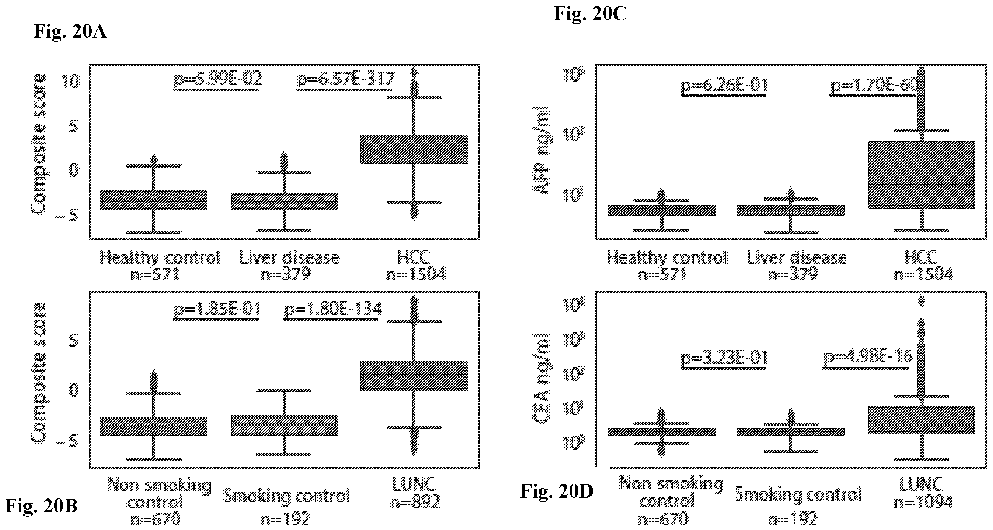

Fig. 20A-Fig. 20D illustrates methylation profiling in healthy control, high-risk patients and cancer patients. Fig. 20A shows methylation profiling differentiates HCC from high risk liver disease patients or normal controls. High risk liver diseases were defined as hepatitis, liver cirrhosis and fatty liver disease. Fig. 20B shows serum AFP differentiates HCC from high risk liver disease patients or normal controls. Fig. 20C shows methylation profiling differentiates LUNC from patients who smoke and normal controls. Fig. 20D shows serum CEA differentiates LUNC from high risk (smoking) patients.

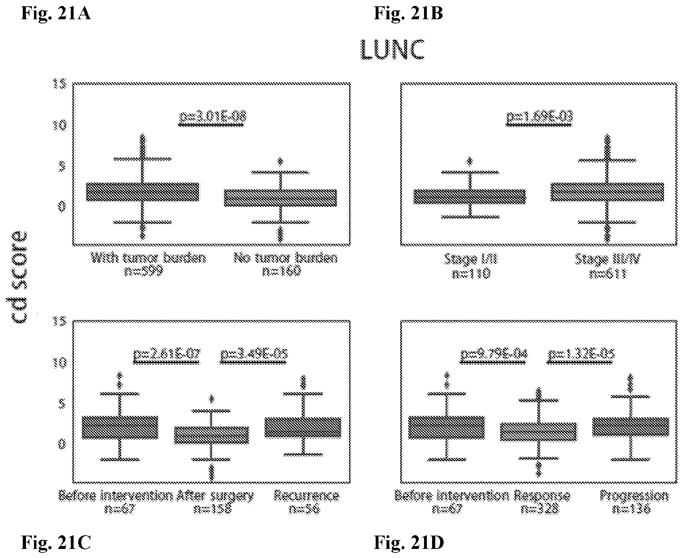

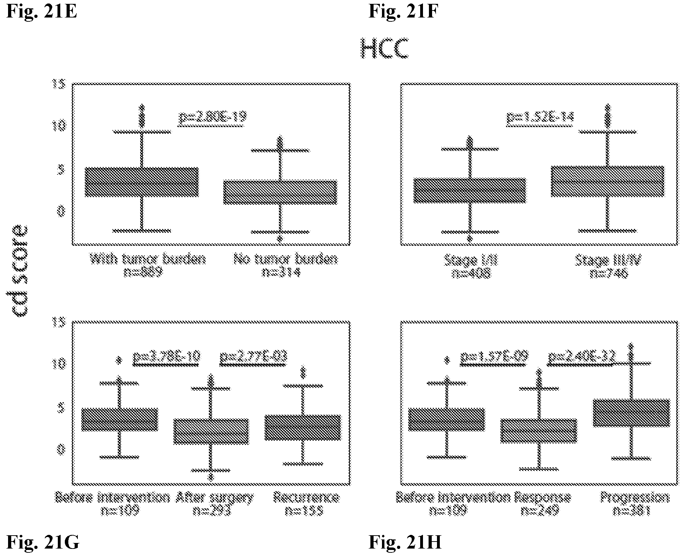

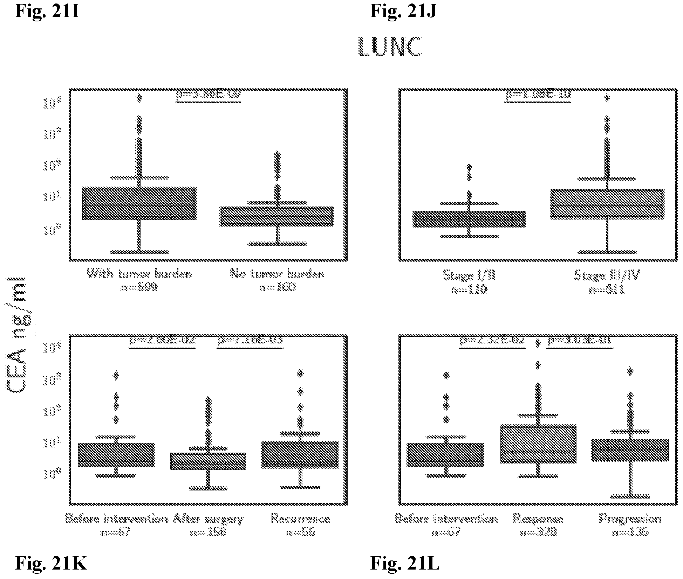

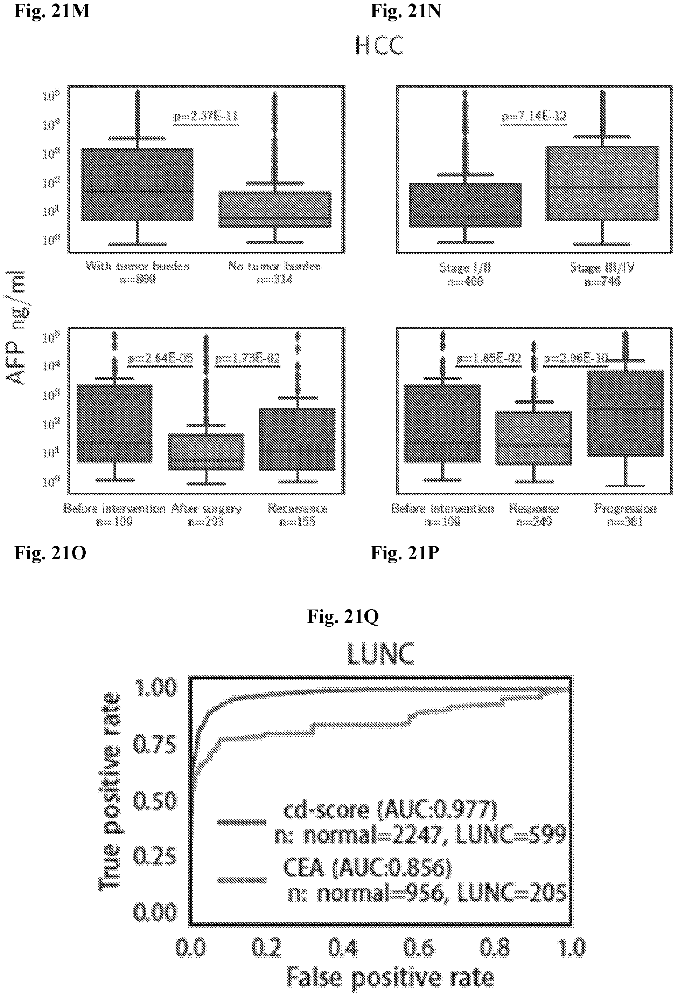

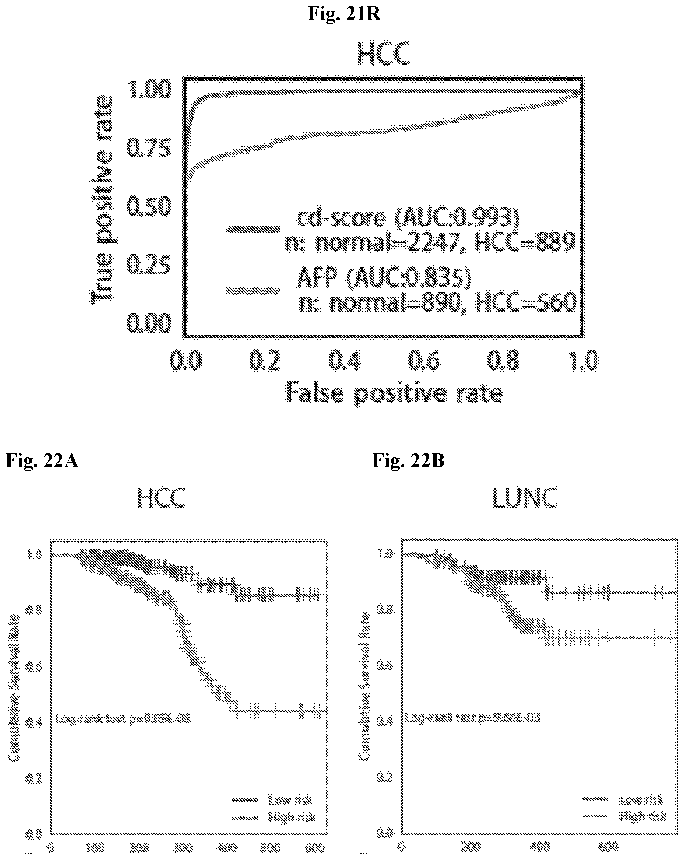

Fig. 21A-Fig. 21R illustrates cfDNA methylation analysis could predict tumor burden, staging, and treatment response using a composite diagnosis score in LUNC and HCC patients. cd-score in patients with and without detectable tumor burden in LUNC (Fig. 21A) when compared to CEA (Fig. 21I) and HCC (Fig. 21E) when compared to AFP (Fig. 21M); cd-score of patients with stage I/II and stage III/IV disease in LUNC (Fig. 21B) when compared to CEA (Fig. 21J) and HCC (Fig. 21F) patients when compared to AFP (Fig. 21N); cd-score in patients before intervention, after surgery, and with recurrence in LUNC (Fig. 21C) when compared to CEA (Fig. 21K) and HCC (Fig. 21G) when compared to AFP (Fig. 21O); cd-score in patients before intervention, with treatment response, and with worsening progression in LUNC (Fig. 21D) when compared to CEA (Fig. 21L) and HCC (Fig. 21H) when compared to AFP (Fig. 21P); Fig. 21Q: The ROC curve and the AUC of cd-score and AFP for LUNC diagnosis in the entire LUNC cohort. Fig. 21R: The ROC curve and the AUC of cd-score and AFP for HCC diagnosis in the entire HCC cohort.

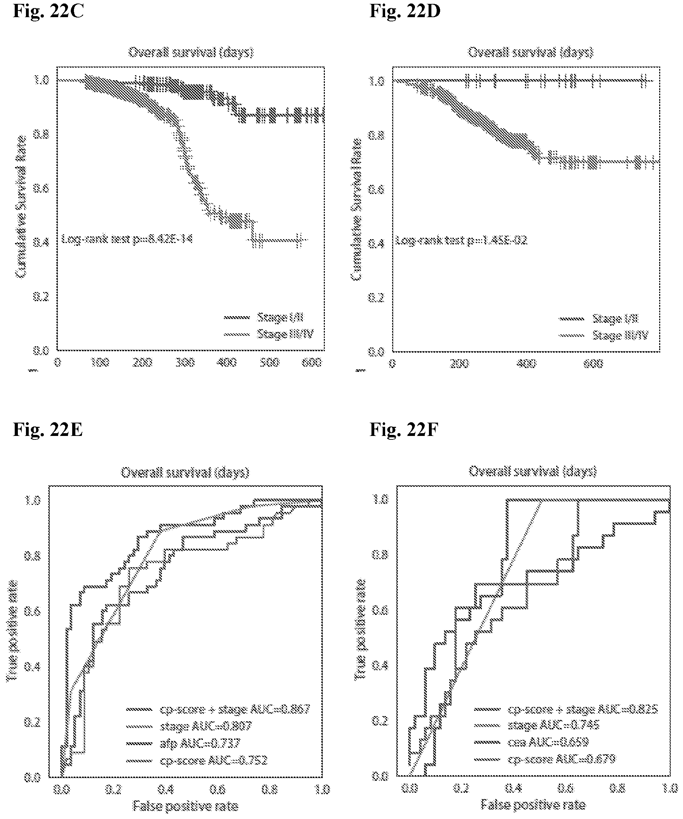

Fig. 22A-Fig. 22F illustrates prognostic prediction in HCC and LUNC survival based on cfDNA methylation profiling. Fig. 22A shows the overall survival curves of HCC patients with low or high risk of death, according to the combined prognosis score (cp-score) in the validation cohort. Fig. 22B shows the overall survival curves of LUNC patients with low or high risk of death, according to the combined prognosis score (cp-score) in the validation dataset. Fig. 22C shows the survival curves of HCC patients with stage I/II and stage III/IV in the validation cohort. Fig. 22D shows the survival curves of patients with stage I/II and stage III/IV LUNC in the validation cohort. The ROC for 12 months survival predicted by cp-score, CEA, AFP, stage, and cp-score combined with stage of HCC (Fig. 22E) and LUNC (Fig. 22F) in the validation cohort.

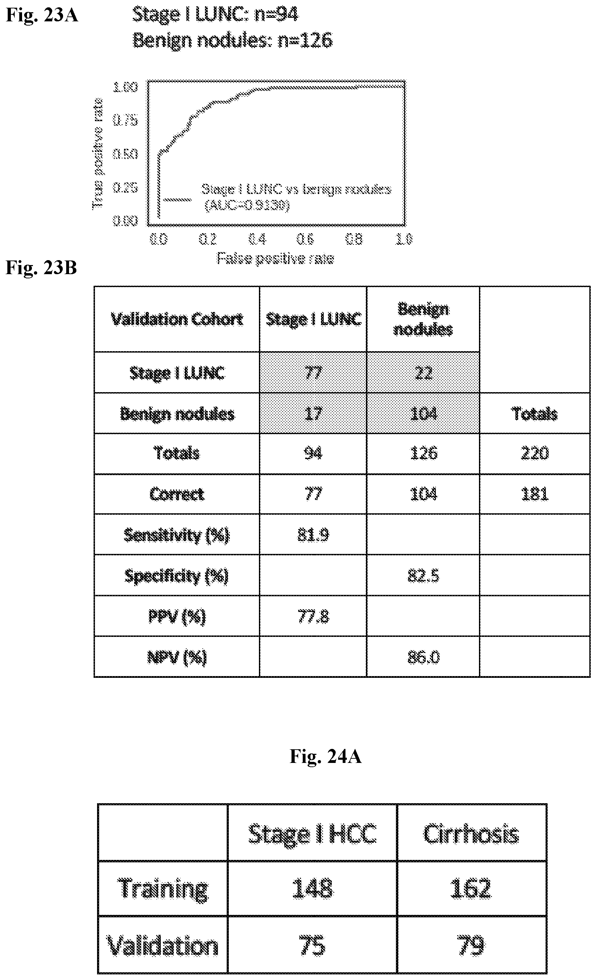

Fig. 23A-Fig. 23B illustrates early detection of LUNC using a cfDNA methylation panel. 208 smoker patients was enrolled with lung nodules between 10 mm and 30 mm in size in a prospective trial and measured a cfDNA LUNC methylation panel. Patients were divided into a training and a testing cohort (Fig. 23A); Receiver operating characteristic (ROC) curves and the associated Area Under Curves (AUCs) of the prediction of Stage I LUNC versus benign lung nodules in the validation cohort with 91.4% accuracy (Fig. 23B); table showing prediction results between Stage I LUNC versus benign lung nodules showing high sensitivity and specificity in the validation cohort.

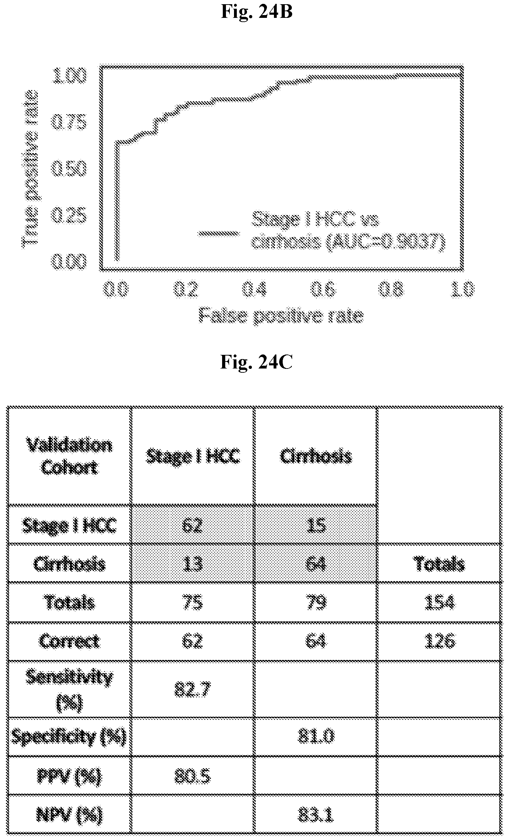

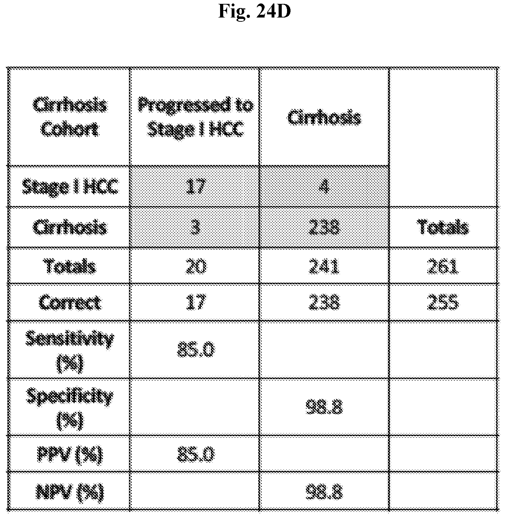

Fig. 24A-Fig. 24D illustrates methylation markers can differentiate between HCC and liver cirrhosis and Detect progression from liver cirrhosis to HCC. A prediction model was first built using 217 HCC and 241 cirrhosis patients and divided patients into a training and a testing cohort (Fig. 24A); Receiver operating characteristic (ROC) curves and the associated Area Under Curves (AUCs) of the prediction of Stage I HCC versus liver cirrhosis in the validation cohort with 89.9% accuracy (Fig. 24B); table showing prediction results between Stage I HCC and liver cirrhosis in a validation cohort (Fig. 24C); table showing prediction results on progression from liver cirrhosis to stage 1HCC with high sensitivity (89.5%) and specificity (98%) (Fig. 24D).

Fig. 25A illustrates unsupervised hierarchical clustering of top 1000 methylation markers differentially methylated in DNA in HCC and LUNC primary tissues versus normal blood.

Fig. 25B shows unsupervised hierarchical clustering of the top 1000 methylation markers differentially methylated between HCC and LUNC tissue DNA. Each column represents an individual patient and each row represents a CpG marker.

Fig. 25C shows global view of supervised hierarchical clustering of all 888 MCBs in the entire cfDNA dataset.

Fig. 26 illustrates Boxplots showing the features of MCBs in cohorts. Top plot: Mean values and deviations of Lasso MCBs in each one versus rest comparison.

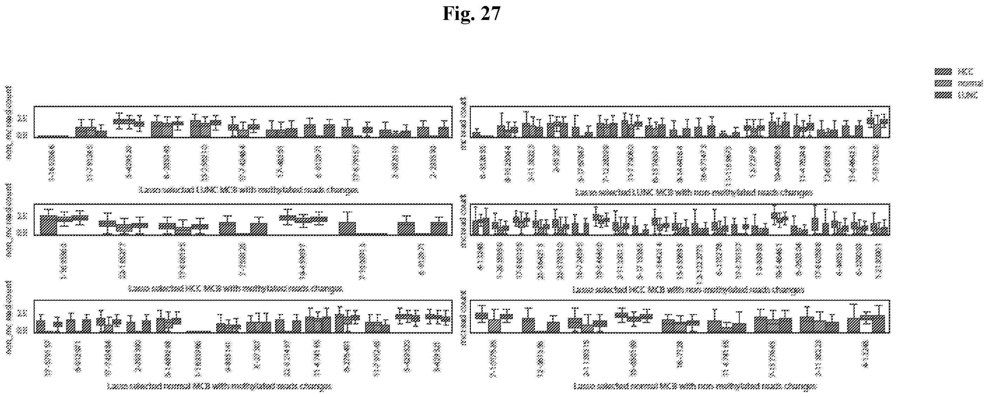

Fig. 27 illustrates methylation values correlated with treatment outcomes in HCC and LUNC patients with serial plasma samples. Summary graphs of change in methylation value comparing patients after surgery, with clinical response (Partial Remission (PR) or Stable Disease (SD), or with disease progression/recurrent (PD).

Fig. 28A shows dynamic monitoring of treatment outcomes using the total methylation copy numbers of an MCB in LUNC patients.

Fig. 28B shows dynamic monitoring of treatment outcomes with the methylation value of an MCB in LUNC patients. PD, progressive disease; PR partial response; SD, stable disease; chemo, chemotherapy.

Fig. 29 illustrates dynamic monitoring of treatment outcomes using the total methylation copy numbers of an MCB and CEA in HCC patients.

Fig. 30 shows dynamic monitoring of treatment outcomes with the methylation rate of an MCB in HCC patients. Dates of treatments are indicated in the figure. PD, progressive disease; PR partial response; SD, stable disease; chemo, chemotherapy, TACE, trans-catheter arterial chemoembolization.

Fig. 31A-Fig. 31B illustrate workflow chart described in Example 5. Fig. 31A illustrates an exemplary workflow for building the diagnostic model, prognostic model, and generating the subtype based ctDNA methylation. Fig. 31B shows the enrollment and outcomes of the prospective screening cohort study.

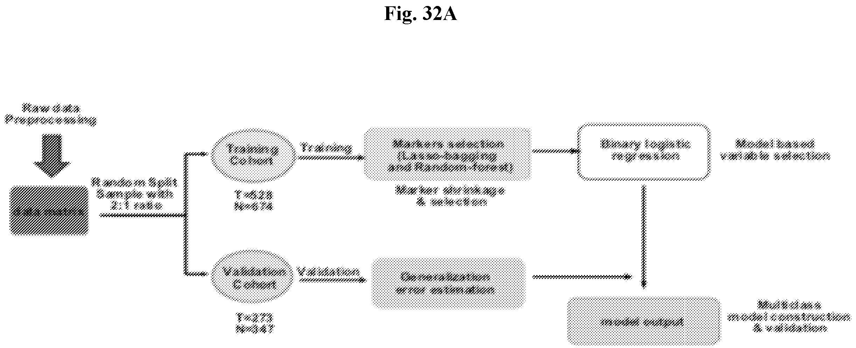

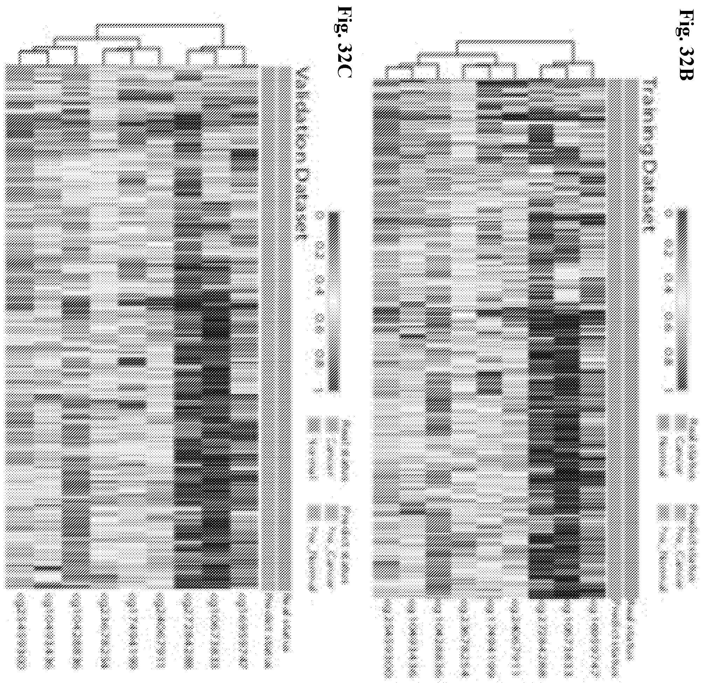

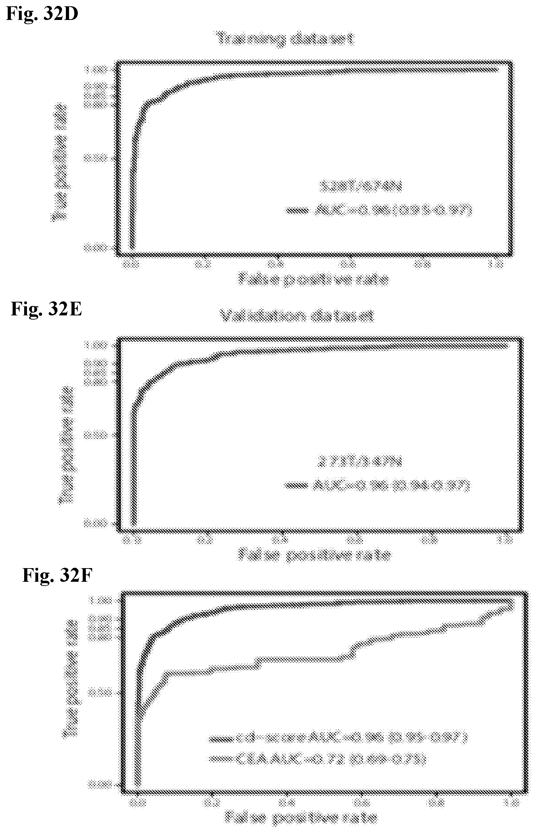

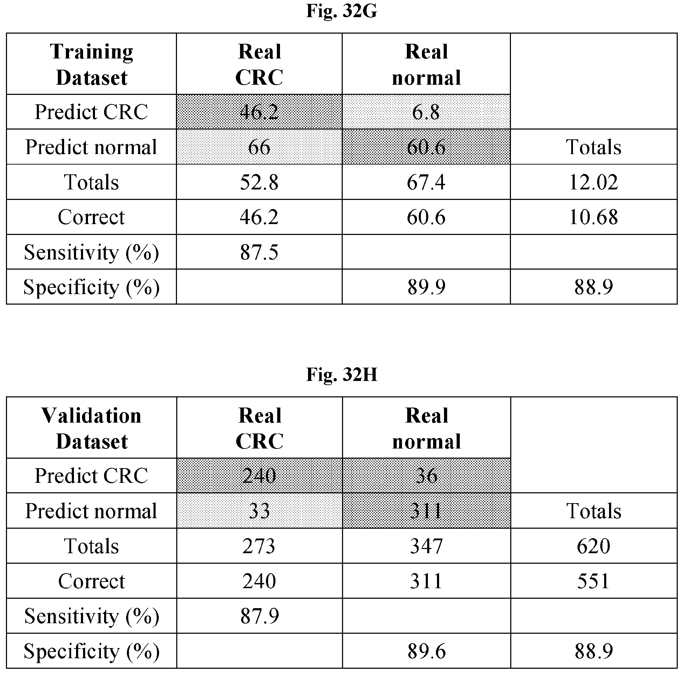

Fig. 32A-Fig. 32H illustrate cfDNA methylation analysis for diagnosis of CRC. Fig. 32A: exemplary workflow for building the diagnostic models. Fig. 32B: Unsupervised hierarchical clustering of methylation markers differentially methylated between cancer (CRC) and normal in the training and the validation (Fig. 32C) testing cohort. Each row represents an individual patient and each column represents a CpG marker. Fig. 32D: Receiver operating characteristic (ROC) curves and the associated Area Under Curves (AUCs) of the diagnostic prediction model (cd-score) using cfDNA methylation analysis in the training and the validation (Fig. 32E) testing cohort. Fig. 32F: ROC curves and corresponding Area Under the Curve (AUCs) of cd-score and CEA for CRC diagnsis. Fig. 32G: Confusion matrices built from diagnostic model prediction in the training and the validation (H) testing cohort.

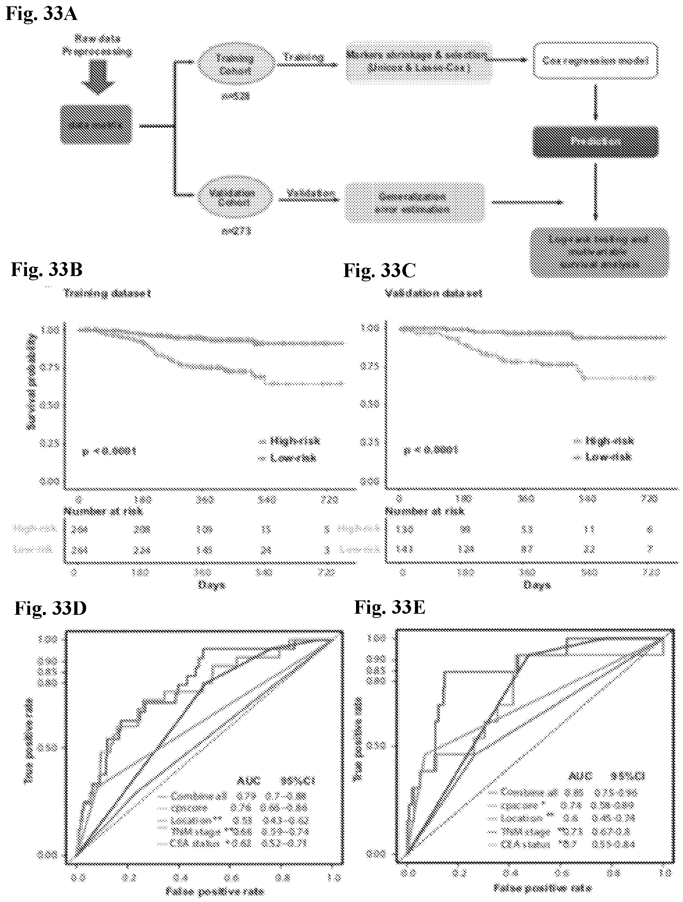

Fig. 33A-Fig. 33E illustrate prognostic prediction in CRC survival based on cfDNA methylation profiling. Fig. 33A: an exemplary workflow for building the prognostic models. Fig. 33B: Overall survival curves of CRC patients with low or high risk of death, according to the combined prognosis score (cp-score) in the training testing cohort. Fig. 33C: Overall survival curves of HCC patients with low or high risk of death, according to the combined prognosis score (cp-score) in the validation testing cohort. The ROC and corresponding AUCs for 12 months survival predicted by cp-score, Primary tumor location, TNM stage, CEA status and combined all in the training (Fig. 33D) and validation (Fig. 33E) testing cohort.

Fig. 34A illustrates a nomogram for predicting one year overall survival of CRC patients using cp-score and other clinical factors.

Fig. 34B illustrates a calibration plot of nomogram in external validation.

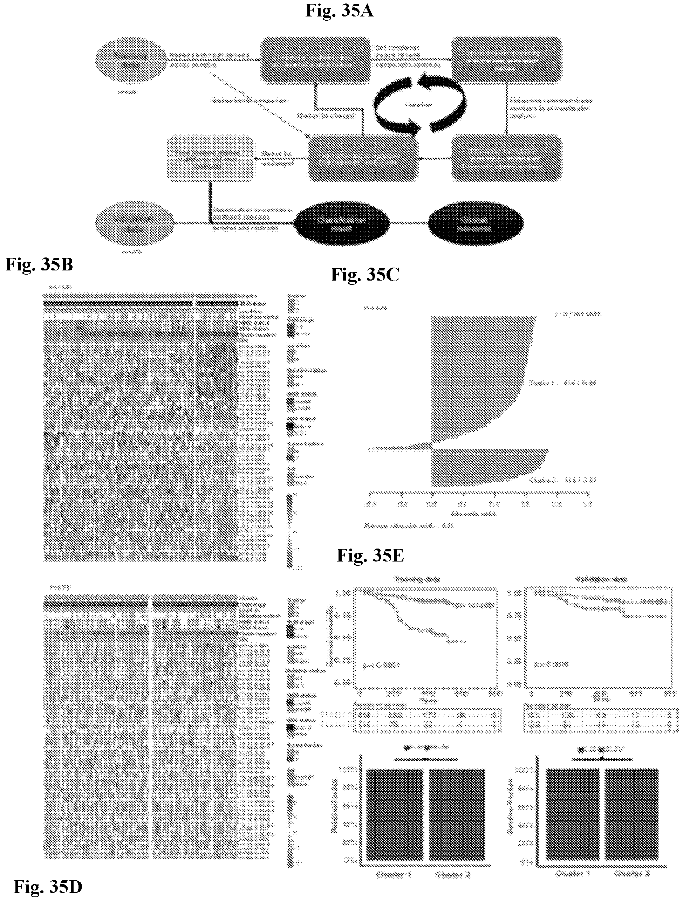

Fig. 35A-Fig. 35E cfDNA methylation subtyping analysis in 801 patients with CRC. Fig. 35A: A schematic diagram shown that the core algorithm utilized in the sample clustering. Fig. 35B: Iteratively unsupervised clustering of cfDNA methylation markers identified two subtypes/clusters in training data. Clinical and molecular features are indicated by the annotation bars above the heatmap. Patients without such information were colored in white. Mutation status was defined by the mutation detected in one of the following genes: *BRAF, KRAS, NRAS* and *PIK3CA*. Fig. 35C: Silhouette analysis of the clusters in the last iteration. Fig. 35D: Predicted subtypes/clusters of validation using the 45 makers. Fig. 35E: upper panel: overall survival for each of the cfDNA methylation in each subtypes. (log rank test p<0.05). lower panel: proportion of III-IV stage CRC patients in two subtypes (Chi-squared test, **$P$<0.01, *$P$<0.05. left, training cohort; right, validation cohort).

Fig. 36A-Fig. 36B presents a list of Methylation Correlated Blocks (MCBs) used for cd-score generation. Fig. 36A: MCBs markers selected by muti-class LASSO. Fig. 36B: Diagnostic marker selection: LASSO-based feature selection identified 13 markers and Random Forest-based feature selection identified 22 markers for discriminating cancer versus normal. There were 9 overlapping markers between these two methods.

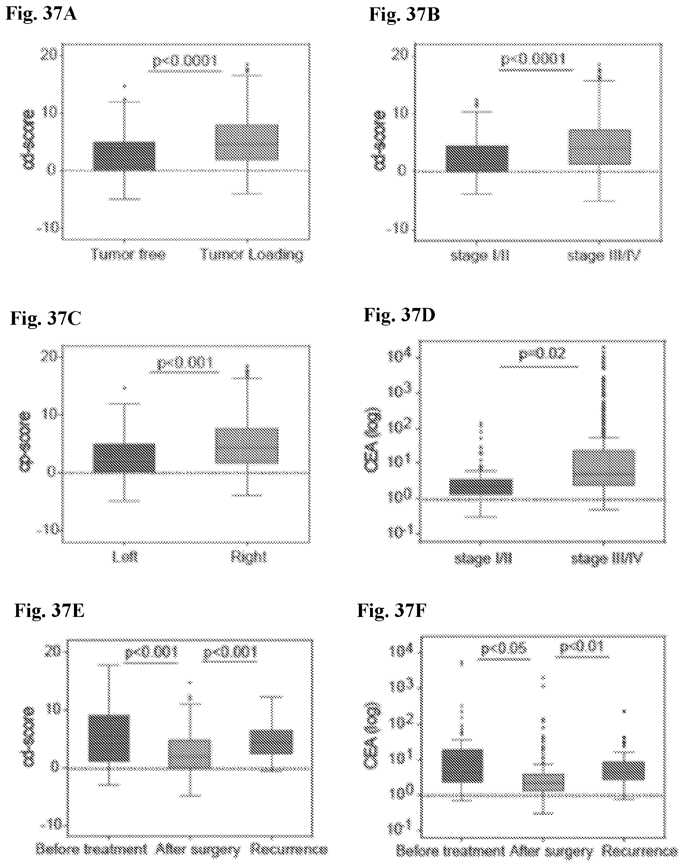

Fig. 37A-Fig. 37F show cfDNA methylation analysis could predict tumor burden, staging, and treatment response using a cd-score in CRC patients. Fig. 37A: cfDNA methylation analysis cd-score in patients with and without detectable tumor burden; Fig. 37B: cd-score of patients with stage I/II and stage III/IV disease; Fig. 37C: cd-score in patients with primary tumor location on left or on right; Fig. 37D: CEA in patients with stage I/II and stage III/IV CRC; Fig. 37E: cd-score in patients before treatment, after surgery ,and with tumor recurrence; Fig. 37F: CEA in CRC patients before treatment, after surgery ,and with tumor recurrence; Recurrence was defined as tumor initially disappeared after treatment/surgery but recurred after a defined period.

Fig. 38A-Fig. 38C illustrate comparison of subtype markers, diagnosis markers and prognosis markers. Fig. 38A: Venn diagram shows the intersects of the three marker lists. Patients in cluster 2 had higher cpscores than those in cluster 1 from the both training cohort (Fig. 38B) and validation cohort (Fig. 38C).

Fig. 39 illustrates patient treatment monitoring with marker methylation level. Dynamic monitoring of treatment outcomes with the methylation value of CpG site cg10673833 (upper panel) and CEA (lower panel) in CRC patients #1-6. Dates of treatments are indicated in the figure. PD, progressive disease; PR partial response; SD, stable disease; chemo, chemotherapy.

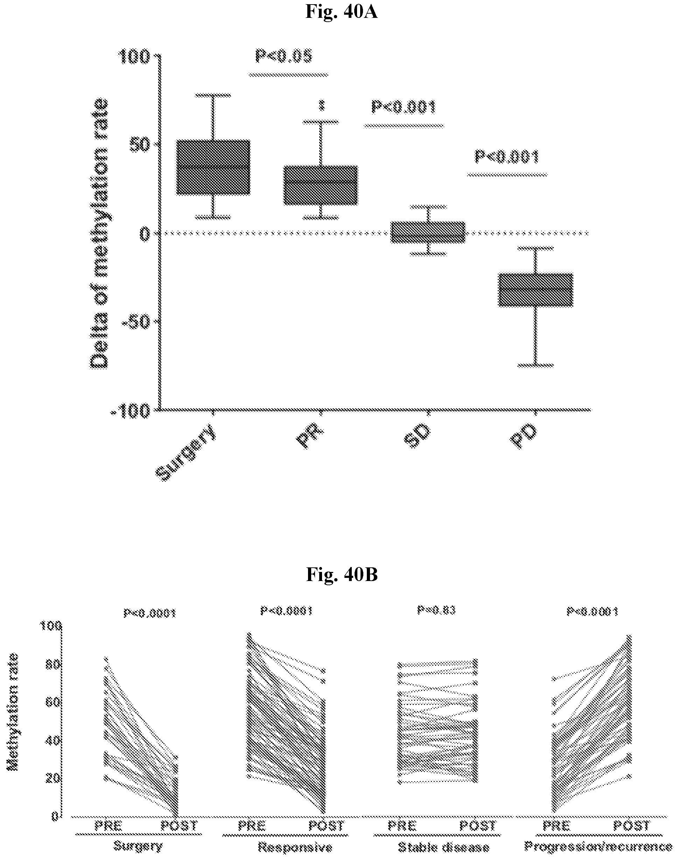

Fig. 40A-Fig. 40B illustrates methylation values correlated with treatment outcomes in CRC patients with serial plasma samples. Fig. 40A: Summary graphs of change in methylation value comparing patients after surgery, with clinical response (Partial Remission (PR) or Stable Disease (SD), or with disease progression/recurrent (PD). Fig. 40B: Methylation value trends in individual patients after complete surgical resection, with treatment response, and with disease progression. Delta methylation rate denotes the methylation value difference before treatment and after treatment. PRE: pre-treatment; POST: after-treatment.

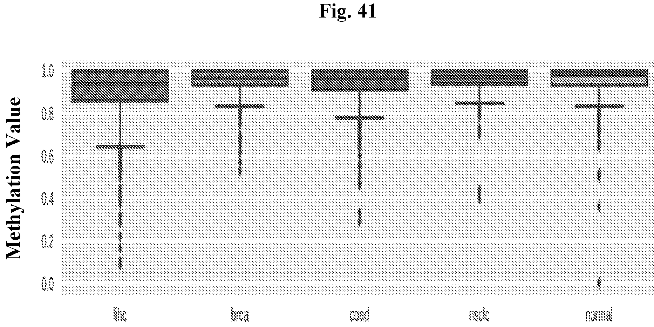

Fig. 41 illustrates the methylation status of CpG marker cg00456086.

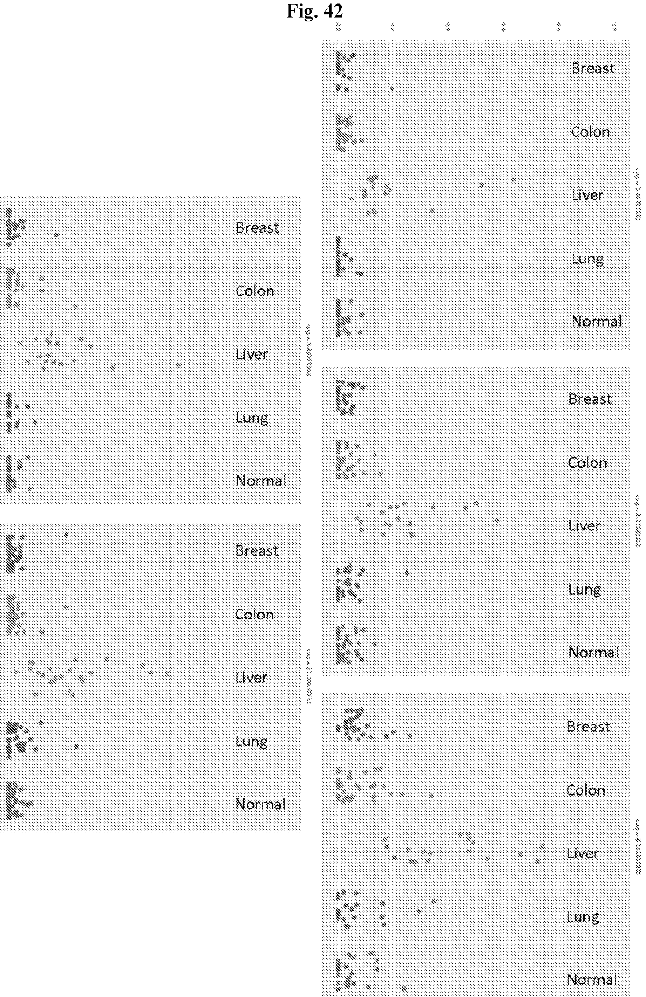

Fig. 42 illustrates the methylation status of biomarker 3-49757316, 8-27183116, 8-141607252, 17-29297711, and 3-49757306.

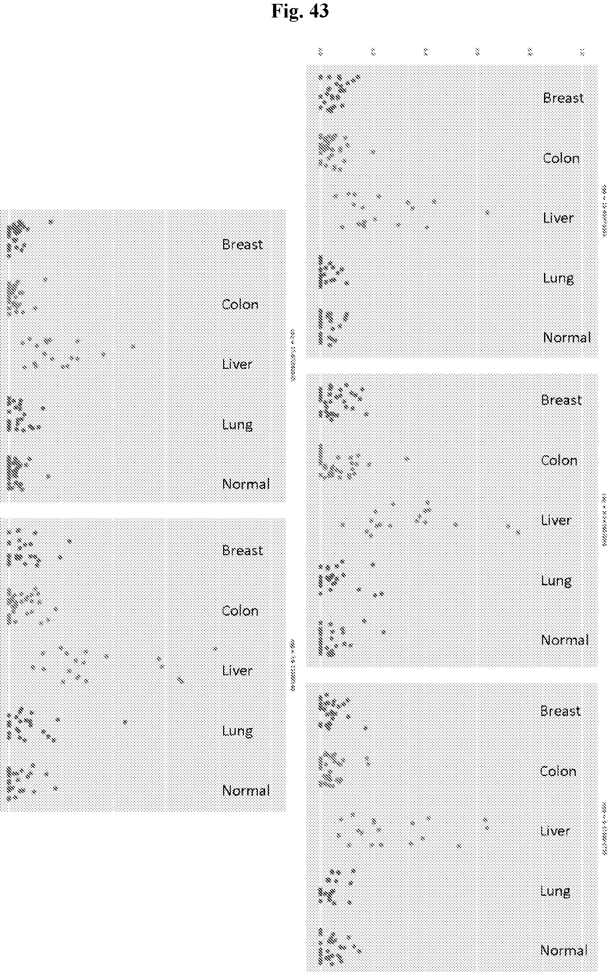

Fig. 43 illustrates the methylation status of biomarker 19-43979341, 8-141607236, 5-176829755, 18-13382140, and 15-65341965.

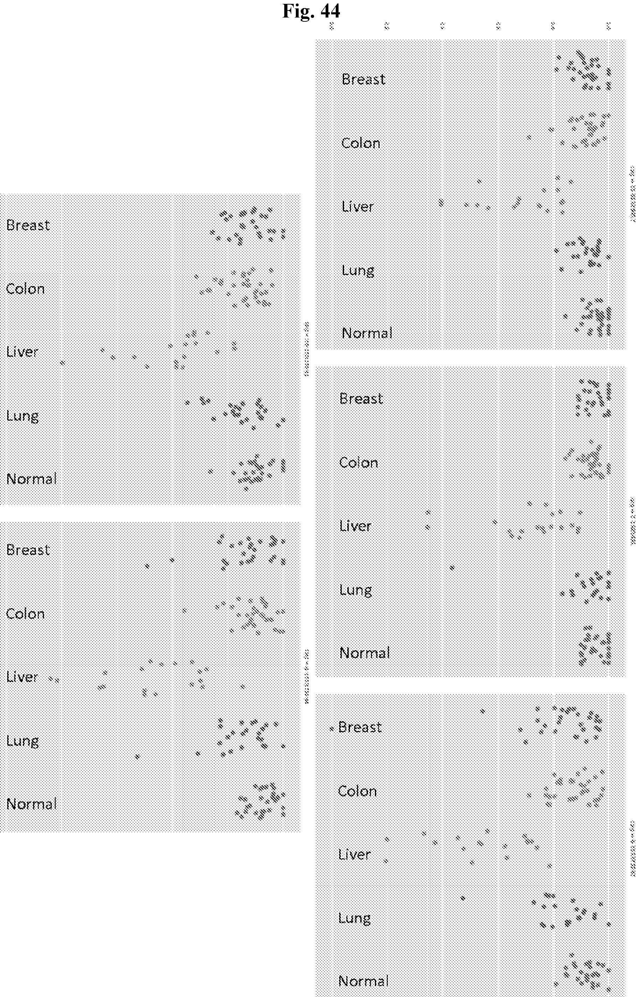

Fig. 44 illustrates the methylation status of biomarker 15-91129457, 2-1625431, 6-151373292, 6-151373294, and 20-25027093.

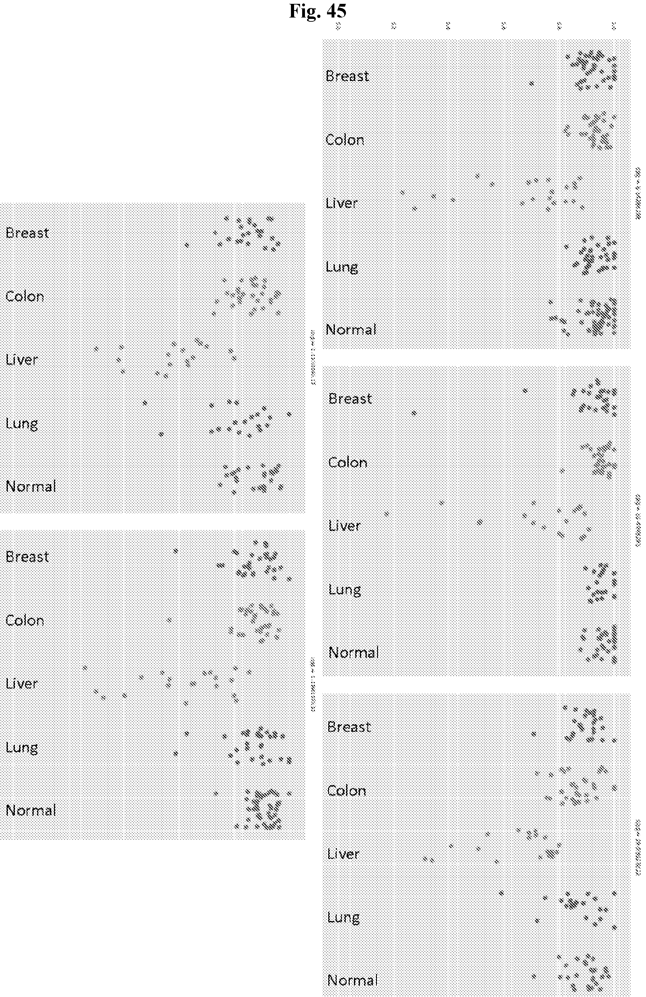

Fig. 45 illustrates the methylation status of biomarker 6-14284198, 10-4049295, 19-59023222, 1-184197132, and 2-131004117.

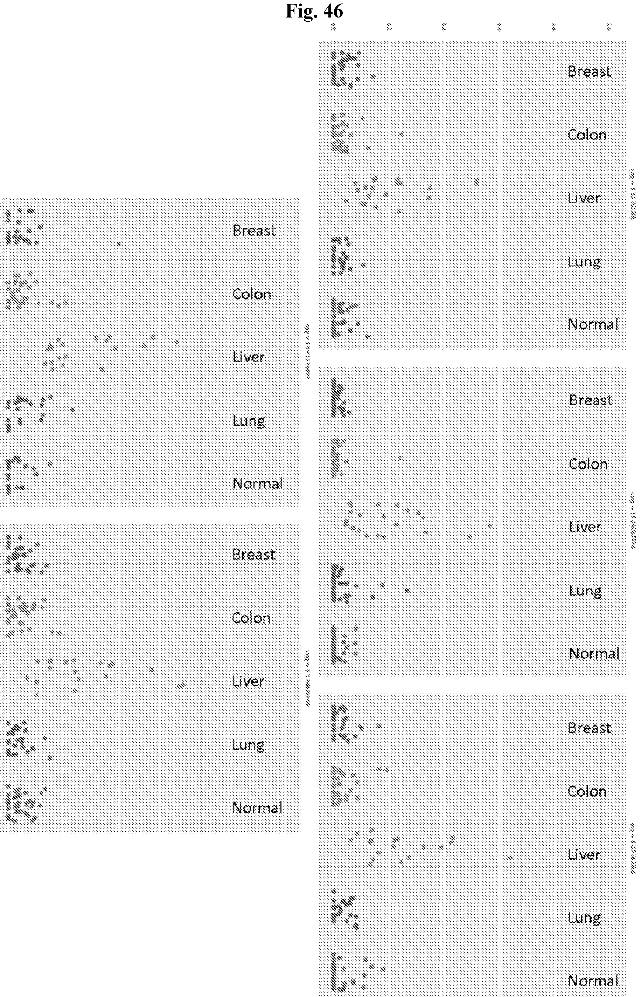

Fig. 46 illustrates the methylation status of biomarker 3-13152305, 17-29297770, 8-27183316, 5-176829740, and 19-41316693.

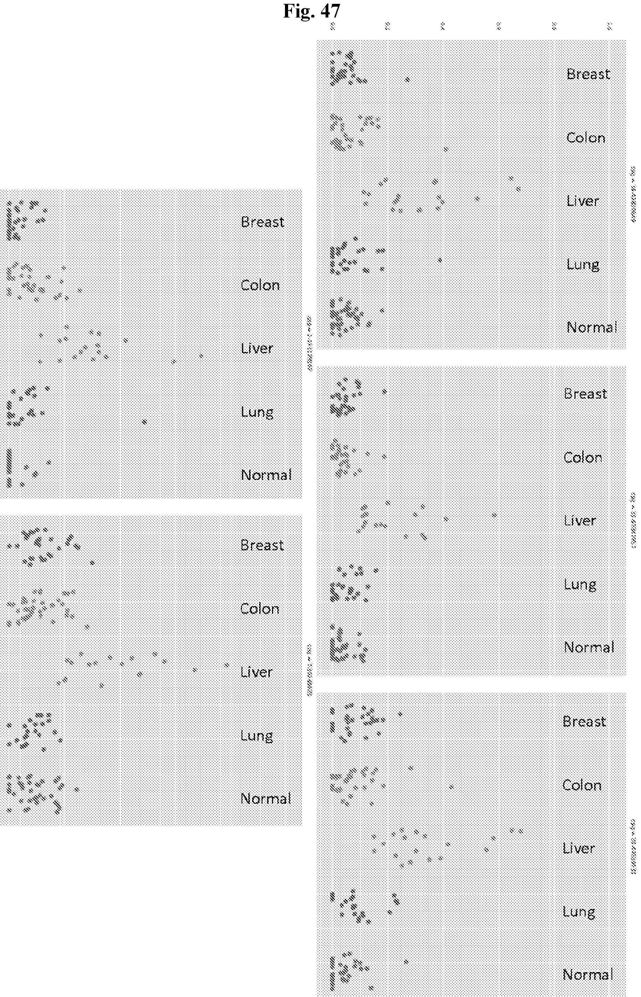

Fig. 47 illustrates the methylation status of biomarker 18-43830649, 15-65341957, 20-44539531, 7-30265625, and 2-131129567.

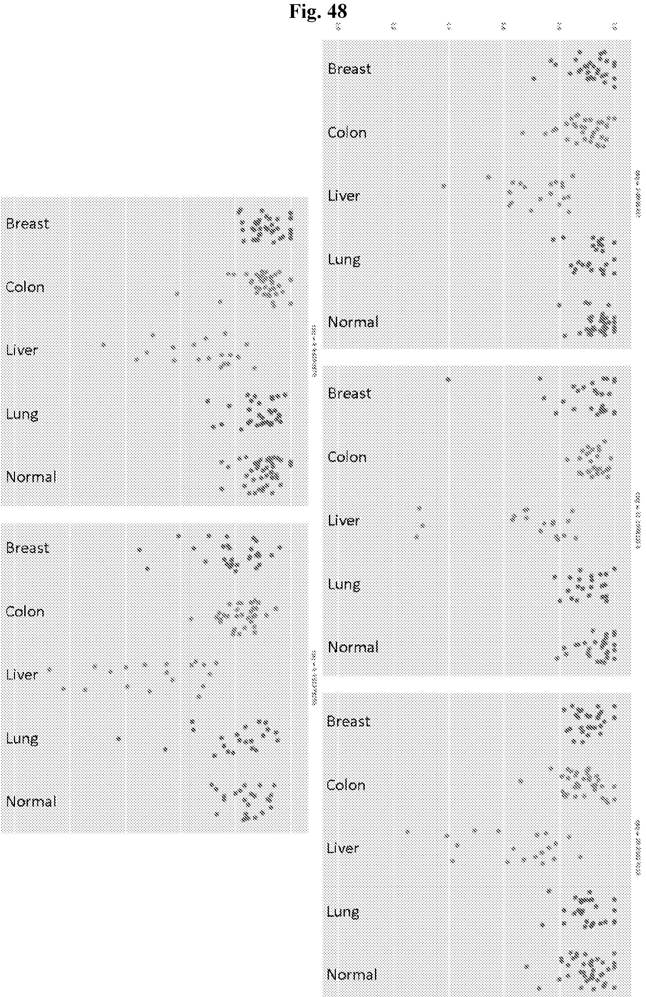

Fig. 48 illustrates the methylation status of biomarker 2-8995417, 12-10782319, 20-25027033, 6-151373256, and 8-86100970.

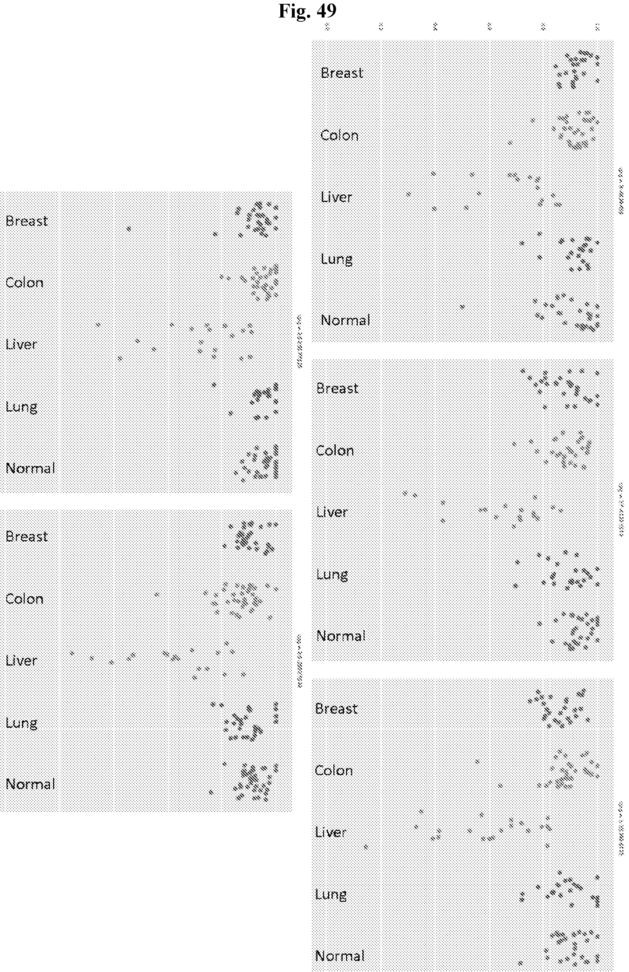

Fig. 49 illustrates the methylation status of biomarker 9-4839459, 17-41221574, 1-153926715, 20-25027044, and 20-20177325.

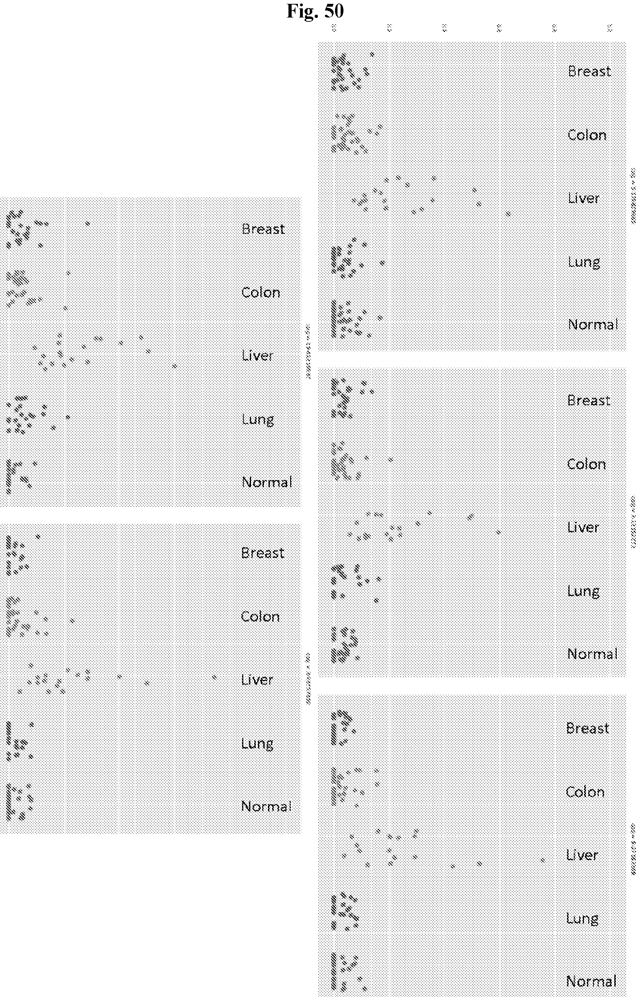

Fig. 50 illustrates the methylation status of biomarker 176829665, 3-13152273, 8-27183348, 3-49757302, and 19-41316697.

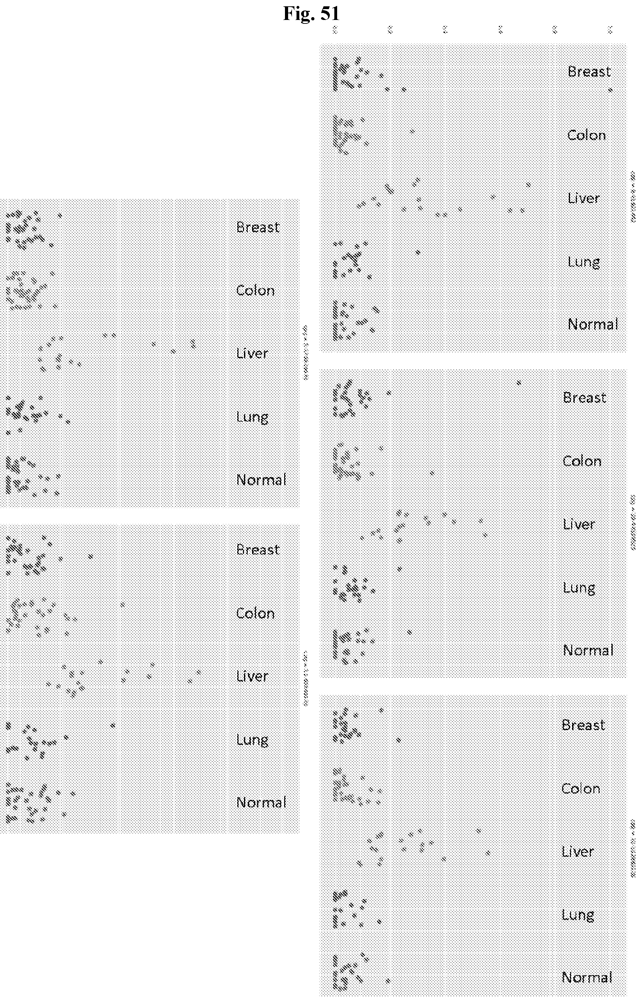

Fig. 51 illustrates the methylation status of biomarker 8-61821442, 20-44539525, 10-102883105, 11-65849129, and 5-176829639.

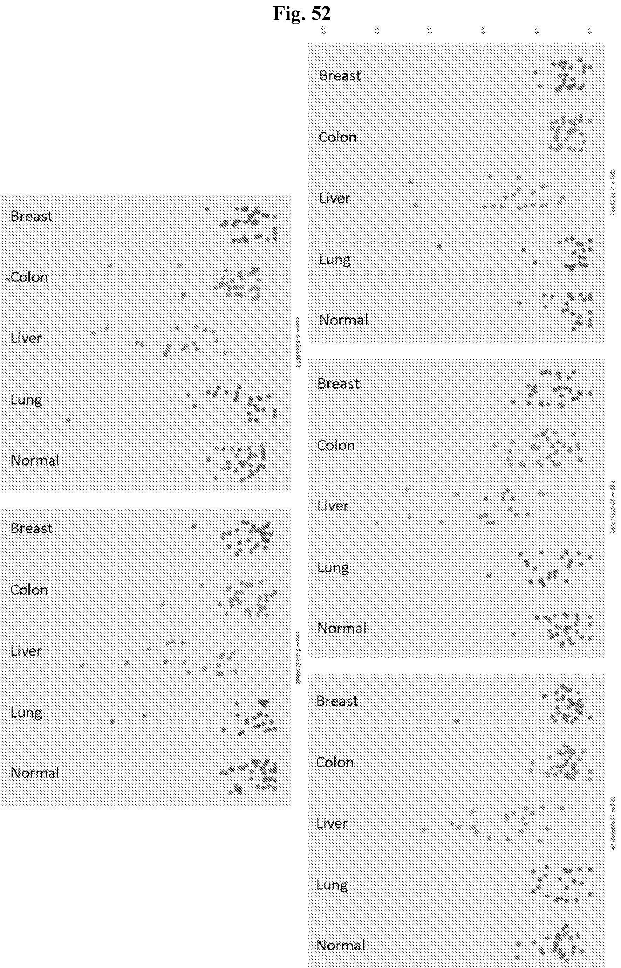

Fig. 52 illustrates the methylation status of biomarker 2-1625443, 20-25027085, 11-69420728, 1-229234865, and 6-13408877.

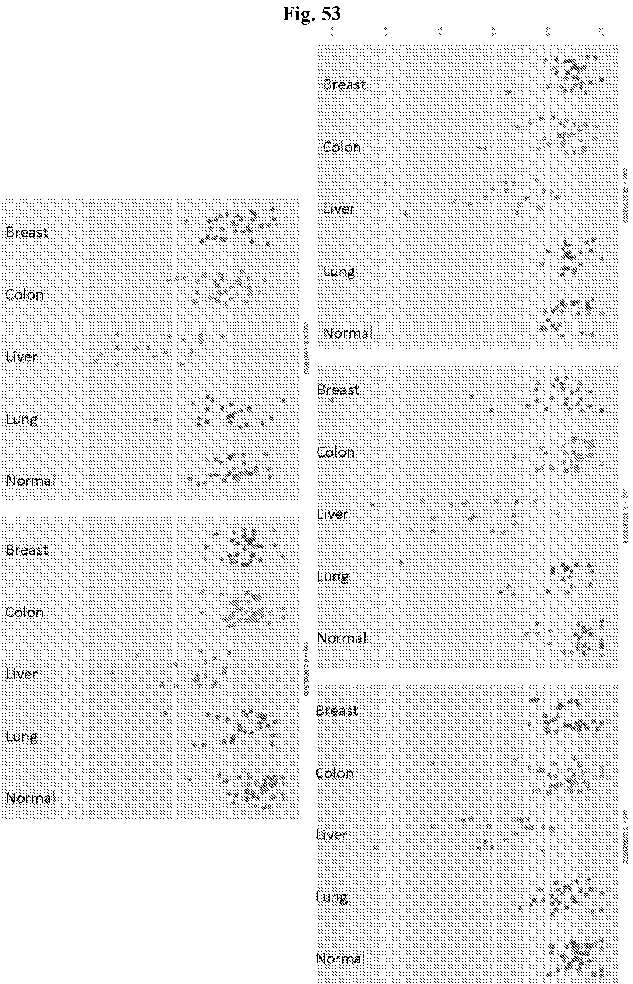

Fig. 53 illustrates the methylation status of biomarker 22-50643735, 6-151373308, 1-232119750, 8-134361508, and 6-13408858.

## DETAILED DESCRIPTION OF THE DISCLOSURE

Cancer is characterized by an abnormal growth of a cell caused by one or more mutations or modifications of a gene leading to dysregulated balance of cell proliferation and cell death. DNA methylation silences expression of tumor suppression genes, and presents itself as one of the first neoplastic changes. Methylation patterns found in neoplastic tissue and plasma demonstrate homogeneity, and in some instances are utilized as a sensitive diagnostic marker. For example, cMethDNA assay has been shown in one study to be about 91% sensitive and about 96% specific when used to diagnose metastatic breast cancer. In another study, circulating tumor DNA (ctDNA) was about 87.2% sensitive and about 99.2% specific when it was used to identify KRAS gene mutation in a large cohort of patients with metastatic colon cancer (Bettegowda et al., Detection of Circulating Tumor DNA in Early- and Late-Stage Human Malignancies. Sci. Transl. Med, 6(224):ra24. 2014). The same study further demonstrated that ctDNA is detectable in >75% of patients with advanced pancreatic, ovarian, colorectal, bladder, gastroesophageal, breast, melanoma, hepatocellular, and head and neck cancers (Bettegowda et al).

Additional studies have demonstrated that CpG methylation pattern correlates with neoplastic progression. For example, in one study of breast cancer methylation patterns, P16 hypermethylation has been found to correlate with early stage breast cancer, while TIMP3 promoter hypermethylation has been correlated with late stage breast cancer. In addition, BMP6, CST6 and TIMP3 promoter hypermethylation have been shown to associate with metastasis into lymph nodes in breast cancer.

In some embodiments, DNA methylation profiling provides higher clinical sensitivity and dynamic range compared to somatic mutation analysis for cancer detection. In other instances, altered DNA methylation signature has been shown to correlate with the prognosis of treatment response for certain cancers. For example, one study illustrated that in a group of patients with advanced rectal cancer, ten differentially methylated regions were used to predict patients' prognosis. Likewise, RASSF1A DNA methylation measurement in serum was used to predict a poor outcome in patients undergoing adjuvant therapy in breast cancer patients in a different study. In addition, SRBC gene hypermethylation was associated with poor outcome in patients with colorectal cancer treated with oxaliplatin in a different study. Another study has demonstrated that ESR1 gene methylation correlates with clinical response in breast cancer patients receiving tamoxifen. Additionally, ARHI gene promoter hypermethylation was shown to be a predictor of long-term survival in breast cancer patients not treated with tamoxifen.

In some embodiments, disclosed herein include methods, probes, and kits for diagnosing the presence of cancer and/or a cancer type. In some instances, described herein is a method of profiling the methylation status of a set of CpG markers (or cg markers). In other instances, described herein is a method for selecting a patient based on the methylation status of a set of CpG markers (or cg markers) for treatment.

## Methods of Use

DNA methylation is the attachment of a methyl group at the C5-position of the nucleotide base cytosine and the N6-position of adenine. Methylation of adenine primarily occurs in prokaryotes, while methylation of cytosine occurs in both prokaryotes and eukaryotes. In some instances, methylation of cytosine occurs in the CpG dinucleotides motif. In other instances, cytosine methylation occurs in, for example CHG and CHH motifs, where H is adenine, cytosine or thymine. In some instances, one or more CpG dinucleotide motif or CpG site forms a CpG island, a short DNA sequence rich in CpG dinucleotide. In some instances, a CpG island is present in the 5' region of about one half of all human genes. CpG islands are typically, but not always, between about 0.2 to about 1 kb in length. Cytosine methylation further comprises 5-methylcytosine (5-mCyt) and 5-hydroxymethylcytosine.

The CpG (cytosine-phosphate-guanine) or CG motif refers to regions of a DNA molecule where a cytosine nucleotide occurs next to a guanine nucleotide in the linear strand. In some instances, a cytosine in a CpG dinucleotide is methylated to form 5-methylcytosine. In some instances, a cytosine in a CpG dinucleotide is methylated to form 5-hydroxymethylcytosine.

In some instances, one or more DNA regions are hypermethylated. In such cases, hypermethylation refers to an increase in methylation event of a region relative to a reference region. In some cases, hypermethylation is observed in one or more cancer types, and is useful, for example, as a diagnostic marker and/or a prognostic marker.

In some instances, one or more DNA regions are hypomethylated. In some cases, hypomethylation refers to a loss of the methyl group in the 5-methylcytosine nucleotide in a first region relative to a reference region. In some cases, hypomethylation is observed in one or cancer types, and is useful, for example, as a diagnostic marker and/or a prognostic marker.

In some embodiments, disclosed herein are CpG methylation markers for diagnosis of a cancer in a subject. In some instances, also disclosed herein is a method of selecting a subject suspected of having cancer for treatment. In some instances, the method comprises (a) contacting treated DNA with at least one probe from a probe panel to generate an amplified product, wherein the at least one probe hybridizes under high stringency condition to a target sequence of a cg marker selected from Table 1, Table 2, Table 7, Table 8, or Table 13, and wherein the treated DNA is processed from a biological sample obtained from the subject; (b) analyzing the amplified product to generate a methylation profile of the cg marker; (c) comparing the methylation profile to a reference model relating methylation profiles of cg markers from Tables 1, 2, 7, 8, and 13 to a set of cancers; (d) based on the comparison of step c), determining: (i) whether the subject has cancer; and (ii) which cancer type the subject has; and (e) administering an effective amount of a therapeutic agent to the subject if the subject is determined to have cancer and the cancer type is determined.

In some instances, the method comprises (a) contacting treated DNA with the probe panel to generate amplified products, wherein each probe of the probe panel hybridizes under high stringency condition to a target sequence of a cg marker selected from Table 1, Table 2, Table 7, or Table 8; (b) analyzing the amplified products to generate a methylation profile of the cg markers targeted by the probe panel; (c) comparing the methylation profile to the reference model relating methylation profiles of cg markers from Tables 1, 2, 7, and 8 to a set of cancers; (d) evaluating an output from the model to determine: (i) whether the subject has cancer; and (ii) which cancer type the subject has; and (e) administering an effective amount of a therapeutic agent to the subject if the subject is determined to have cancer and the cancer type is determined.

In some cases, the biological sample is treated with a deaminating agent to generate the treated DNA.

In some cases, the at least one probe from the probe panel is a padlock probe.

In some instances, the at least one probe hybridizes under high stringency conditions to a target sequence of a cg marker selected from Table 1.

In some instances, the at least one probe hybridizes under high stringency conditions to a target sequence of a cg marker selected from Table 2.

In some instances, the at least one probe hybridizes under high stringency conditions to a target sequence of a cg marker selected from Table 4.

In some instances, the at least one probe hybridizes under high stringency conditions to a target sequence of a cg marker selected from Table 5.

In some instances, the at least one probe hybridizes under high stringency conditions to a target sequence of a cg marker selected from Table 7.

In some instances, the at least one probe hybridizes under high stringency conditions to a target sequence of a cg marker selected from Table 8.

In some instances, the at least one probe hybridizes under high stringency conditions to a target sequence of a cg marker selected from Table 13.

In some instances, the at least one probe hybridizes under high stringency conditions to a target sequence of a cg marker selected from cg19516279, cg06100368, cg25945732, cg19155007, cg17952661, cg04072843, cg01250961, cg08131100, cg03788131, cg17528648, cg07784526, cg18948743, cg23986470, cg00846300, cg01029638, cg08350814, cg05098590, cg18085998, cg06532037, cg15313226, cg16232979, cg26149167, cg01237565, cg16561543, cg13771313, cg13771313, cg08169020, cg08169020, cg21153697, cg07326648, cg14309384, cg20923716, cg09095222, cg22220310, cg21950459, cg13332729, cg10802543, cg20707333, cg13169641, cg25352342, cg09921682, cg02504622, cg17373759, cg06547203, cg06826710, cg00902147, cg17609887, cg15721142, cg08116711, cg00736681, cg18834029, cg06969479, cg24630516, cg16901821, cg20349803, cg23610994, cg19313373, cg16508600, cg24096323, cg24746106, cg12288267, cg10430690, cg24408776, cg05630192, cg12028674, cg24820270, cg12028674, cg26718707, cg10349880, cg09921682, cg25934700, cg14164596, cg24461337, cg23041410, cg07366553, cg26859666, cg06405341, cg08557188, cg00690392, cg03421440, cg07077277, cg00456086, or cg20702527. In some instances, the at least one probe hybridizes under high stringency conditions to a target sequence of a cg marker selected from cg19516279, cg06100368, cg20349803, cg23610994, cg19313373, cg16508600, or cg24096323. In some instances, the at least one probe hybridizes under high stringency conditions to a target sequence of a cg marker selected from cg25945732, cg19155007, cg17952661, cg25934700, cg14164596, cg24461337, cg23041410, cg07366553, cg26859666, or cg00456086. In some instances, the at least one probe hybridizes under high stringency conditions to a target sequence of a cg marker selected from cg04072843, cg01250961, cg24746106, cg12288267, or cg10430690. In some instances, the at least one probe hybridizes under high stringency conditions to a target sequence of a cg marker selected from cg08131100, cg03788131, cg17528648 , cg07784526, cg18948743, cg23986470, cg00846300, cg25352342, cg09921682, cg02504622, cg17373759, cg12028674, cg24820270, cg12028674, cg26718707, cg10349880, or cg09921682. In some instances, the at least one probe hybridizes under high stringency conditions to a target sequence of a cg marker selected from cg01029638, cg08350814, cg05098590, cg18085998, cg06532037, cg15313226, cg16232979, cg26149167, cg06547203, cg06826710, cg00902147, cg17609887, or cg15721142. In some instances, the at least one probe hybridizes under high stringency conditions to a target sequence of a cg marker selected from cg01237565, cg16561543, or cg08116711. In some instances, the at least one probe hybridizes under high stringency conditions to a target sequence of a cg marker selected from cg13771313, cg13771313, or cg08169020. In some instances, the at least one probe hybridizes under high stringency conditions to a target sequence of a cg marker selected from cg08169020, cg21153697, cg07326648, cg14309384, cg20923716, cg22220310, cg21950459, cg13332729, cg10802543, cg20707333, or cg13169641. In some instances, the at least one probe hybridizes under high stringency conditions to a target sequence of a cg marker cg09095222. In some instances, the at least one probe hybridizes under high stringency conditions to a target sequence of a cg marker selected from cg00736681 or cg18834029. In some instances, the at least one probe hybridizes under high stringency conditions to a target sequence of a cg marker selected from cg06969479, cg24630516, or cg16901821. In some instances, the at least one probe hybridizes under high stringency conditions to a target sequence of a cg marker cg24408776. In some instances, the at least one probe hybridizes under high stringency conditions to a target sequence of a cg marker cg05630192. In some instances, the at least one probe hybridizes under high stringency conditions to a target sequence of a cg marker cg06405341. In some instances, the at least one probe hybridizes under high stringency conditions to a target sequence of a cg marker selected from cg08557188, cg00690392, cg03421440, or cg07077277. In some instances, the at least one probe hybridizes under high stringency conditions to a target sequence of a cg marker selected from cg10673833, cg10493436, cg10428836, cg27284288, cg16959747, cg17494199, cg23678254, cg24067911, or cg25459300.

In some instances, the at least one probe hybridizes under high stringency conditions to a target sequence of a gene selected from a gene panel consisting of *BMPR1A, PSD, ARHGAP 25, KLF3, PLAC8, ATXN1*, Chromosome 6:170, Chromosome 6:3, *ATAD2,* and Chromosome 8:20.

some instances, the at least one probe hybridizes under high stringency conditions to a target sequence of a gene selected from a gene panel consisting of *MYO1G, ADAMTS4, BMPR1A, CD6, RBP5,* Chr 13:10, *LGAP5, ATXN1,* and Chr 8:20.

In some instances, the reference model comprises methylation profiles of cg markers from Tables 1 and 2 generated from samples of known cancer types. In some cases, the reference model further comprises methylation profiles of cg markers from Tables 1 and 2 generated from normal samples. In some cases, the reference model comprises methylation profiles of cg markers from Tables 1 and 2 generated from tissue samples.

In some instances, the reference model comprises methylation profiles of cg markers from Tables 7 and 8 generated from samples of known cancer types. In some cases, the reference model further comprises methylation profiles of cg markers from Tables 7 and 8 generated from normal samples. In some cases, the reference model comprises methylation profiles of cg markers from Tables 7 and 8 generated from tissue samples.

In some instances, the reference model comprises methylation profiles of cg markers from Table 13 generated from samples of known cancer types. In some cases, the reference model further comprises methylation profiles of cg markers from Table 13 generated from normal samples. In some cases, the reference model comprises methylation profiles of cg markers from Table 13 generated from tissue samples.

In some cases, the reference model is developed using an algorithm selected from one or more of the following: a principal component analysis, a logistic regression analysis, a nearest neighbor analysis, a support vector machine, and a neural network model.

In some embodiments, the analyzing described above comprises quantitatively detecting the methylation status of the amplified product. In some cases, the detection comprises a real-time quantitative probe-based PCR or a digital probe-based PCR. In some cases, the detection comprises a real-time quantitative probe-based PCR. In other cases, the detection comprises a digital probe-based PCR, optionally, a digital droplet PCR.

In some embodiments, the treatment comprises a chemotherapeutic agent or an agent for a targeted therapy. Exemplary chemotherapeutic agents include, cisplatin, doxorubicin, fluoropyrimidine, gemcitabine, irinotecan, mitoxantrone, oxaliplatin, thalidomide, or a combination thereof. In some cases, the chemotherapeutic agent comprises cisplatin, doxorubicin, fluoropyrimidine, gemcitabine, irinotecan, mitoxantrone, oxaliplatin, thalidomide, or a combination thereof. In some instances, the treatment comprises an agent for a targeted therapy. In additional instances, the treatment comprises surgery.

In some instances, the biological sample is a blood sample, an urine sample, a saliva sample, a sweat sample, or a tear sample. In some cases, the biological sample is a blood sample or an urine sample. In some cases, the biological sample is a tissue biopsy sample. In some cases, the biological sample is a cell-free DNA sample. In some cases, the biological sample comprises circulating tumor cells.

In some embodiments, also disclosed herein is a method of detecting the methylation status of a set of cg markers. In some embodiments, the method comprises (a) processing a biological sample obtained from a subject with a deaminating agent to generate treated DNA comprising deaminated nucleotides; (b) contacting the treated DNA with at least one probe that hybridizes under high stringency condition to a target sequence of a cg marker from Table 1, Table 2, Table 7, Table 8, Table 13, Table 14, or Table 20; and (c) quantitatively detecting the methylation status of the cg marker, wherein said detection comprises a real-time quantitative probe-based PCR or a digital probe-based PCR.

In some embodiments, the method of detecting the methylation status of a set of cg markers comprises (a) processing a biological sample obtained from a subject with a deaminating agent to generate treated DNA comprising deaminated nucleotides; (b) contacting the treated DNA with at least one probe that hybridizes under high stringency condition to a target sequence of a cg marker from Table 1 or Table 2; and (c) quantitatively detecting the methylation status of the cg marker, wherein said detection comprises a real-time quantitative probe-based PCR or a digital probe-based PCR.

In some cases, the detection comprises a real-time quantitative probe-based PCR or a digital probe-based PCR. In some cases, the detection comprises a real-time quantitative probe-based PCR. In other cases, the detection comprises a digital probe-based PCR, optionally, a digital droplet PCR.

In some cases, the at least one probe from the probe panel is a padlock probe.

In some instances, the at least one probe hybridizes under high stringency conditions to a target sequence of a cg marker selected from Table 1.

In some instances, the at least one probe hybridizes under high stringency conditions to a target sequence of a cg marker selected from Table 2.

In some instances, the at least one probe hybridizes under high stringency conditions to a target sequence of a cg marker selected from Table 4.

In some instances, the at least one probe hybridizes under high stringency conditions to a target sequence of a cg marker selected from Table 5.

In some instances, the at least one probe hybridizes under high stringency conditions to a target sequence of a cg marker selected from Table 7.

In some instances, the at least one probe hybridizes under high stringency conditions to a target sequence of a cg marker selected from Table 8.

In some instances, the at least one probe hybridizes under high stringency conditions to a target sequence of a cg marker selected from Table 13.

In some instances, the at least one probe hybridizes under high stringency conditions to a target sequence of a cg marker selected from Table 14.

In some instances, the at least one probe hybridizes under high stringency conditions to a target sequence of a cg marker selected from Table 20.

In some instances, the at least one probe hybridizes under high stringency conditions to a target sequence of a cg marker selected from cg19516279, cg06100368, cg25945732, cg19155007, cg17952661, cg04072843, cg01250961, cg08131100, cg03788131, cg17528648, cg07784526, cg18948743, cg23986470, cg00846300, cg01029638, cg08350814, cg05098590, cg18085998, cg06532037, cg15313226, cg16232979, cg26149167, cg01237565, cg16561543, cg13771313, cg13771313, cg08169020, cg08169020, cg21153697, cg07326648, cg14309384, cg20923716, cg09095222, cg22220310, cg21950459, cg13332729, cg10802543, cg20707333, cg13169641, cg25352342, cg09921682, cg02504622, cg17373759, cg06547203, cg06826710, cg00902147, cg17609887, cg15721142, cg08116711, cg00736681, cg18834029, cg06969479, cg24630516, cg16901821, cg20349803, cg23610994, cg19313373, cg16508600, cg24096323, cg24746106, cg12288267, cg10430690, cg24408776, cg05630192, cg12028674, cg24820270, cg12028674, cg26718707, cg10349880, cg09921682, cg25934700, cg14164596, cg24461337, cg23041410, cg07366553, cg26859666, cg06405341, cg08557188, cg00690392, cg03421440, cg07077277, cg00456086, or cg20702527. In some instances, the at least one probe hybridizes under high stringency conditions to a target sequence of a cg marker selected from cg19516279, cg06100368, cg20349803, cg23610994, cg19313373, cg16508600, or cg24096323. In some instances, the at least one probe hybridizes under high stringency conditions to a target sequence of a cg marker selected from cg25945732, cg19155007, cg17952661, cg25934700, cg14164596, cg24461337, cg23041410, cg07366553, cg26859666, or cg00456086. In some instances, the at least one probe hybridizes under high stringency conditions to a target sequence of a cg marker selected from cg04072843, cg01250961, cg24746106, cg12288267, or cg10430690. In some instances, the at least one probe hybridizes under high stringency conditions to a target sequence of a cg marker selected from cg08131100, cg03788131, cg17528648 , cg07784526, cg18948743, cg23986470, cg00846300, cg25352342, cg09921682, cg02504622, cg17373759, cg12028674, cg24820270, cg12028674, cg26718707, cg10349880, or cg09921682. In some instances, the at least one probe hybridizes under high stringency conditions to a target sequence of a cg marker selected from cg01029638, cg08350814, cg05098590, cg18085998, cg06532037, cg15313226, cg16232979, cg26149167, cg06547203, cg06826710, cg00902147, cg17609887, or cg15721142. In some instances, the at least one probe hybridizes under high stringency conditions to a target sequence of a cg marker selected from cg01237565, cg16561543, or cg08116711. In some instances, the at least one probe hybridizes under high stringency conditions to a target sequence of a cg marker selected from cg13771313, cg13771313, or cg08169020. In some instances, the at least one probe hybridizes under high stringency conditions to a target sequence of a cg marker selected from cg08169020, cg21153697, cg07326648, cg14309384, cg20923716, cg22220310, cg21950459, cg13332729, cg10802543, cg20707333, or cg13169641. In some instances, the at least one probe hybridizes under high stringency conditions to a target sequence of a cg marker cg09095222. In some instances, the at least one probe hybridizes under high stringency conditions to a target sequence of a cg marker selected from cg00736681 or cg18834029. In some instances, the at least one probe hybridizes under high stringency conditions to a target sequence of a cg marker selected from cg06969479, cg24630516, or cg16901821. In some instances, the at least one probe hybridizes under high stringency conditions to a target sequence of a cg marker cg24408776. In some instances, the at least one probe hybridizes under high stringency conditions to a target sequence of a cg marker cg05630192. In some instances, the at least one probe hybridizes under high stringency conditions to a target sequence of a cg marker cg06405341. In some instances, the at least one probe hybridizes under high stringency conditions to a target sequence of a cg marker selected from cg08557188, cg00690392, cg03421440, or cg07077277. In some instances, the at least one probe hybridizes under high stringency conditions to a target sequence of a cg marker selected from cg10673833, cg10493436, cg10428836, cg27284288, cg16959747, cg17494199, cg23678254, cg24067911, or cg25459300. In some instances, the at least one probe hybridizes under high stringency conditions to a target sequence of a cg marker selected from cg05205843, cg11841704, cg06699564, cg08924619, cg11959316, cg08924619, cg06699564, cg01824933, cg08924619, cg05205842, cg08924619, cg04049981, cg09026722, cg03616722, cg08924619, cg05928904, cg08704934, cg09776772, cg17494199, cg01824933, cg16296417, cg09776772, cg09776772, cg05338167, cg10493436, cg011251410, cg16391792, cg06393830, cg09366118, cg22513455, cg17583432, cg23881926, cg09638208, cg12441066, cg27284288, cg04441857, cg17583432, cg10673833, cg19757176, cg08670281, cg17583432, cg04460364, cg16959747, cg15011734, or cg25754195.

In some embodiments, the methylation status or pattern of a set of cg markers is further used to determine whether the subject has a cancer. For example, the methylation status or pattern of at least one cg marker selected from cg19516279, cg06100368, cg20349803, cg23610994, cg19313373, cg16508600, and cg24096323 is used to determine whether the subject has a breast cancer. In some cases, the subject is determined to have a breast cancer if at least one of the cg markers cg19516279 and cg06100368 is hypermethylated. In other cases, the subject is determined to have a breast cancer if at least one of the cg markers cg20349803, cg23610994, cg19313373, cg16508600, and cg24096323 is hypomethylated.

In some instances, the methylation status or pattern of at least one cg marker selected from cg25945732, cg19155007, cg17952661, cg25934700, cg14164596, cg24461337, cg23041410, cg07366553, cg00456086, and cg26859666 is used to determine whether the subject has a liver cancer. In some cases, the subject is determined to have a liver cancer if at least one of the cg markers cg25945732, cg19155007, or cg17952661 is hypermethylated. In other cases, the subject is determined to have a liver cancer if at least one of the cg markers cg25934700, cg14164596, cg24461337, cg23041410, cg07366553, cg26859666, or cg00456086 is hypomethylated.

In some instances, the methylation status or pattern of at least one marker selected from 3-49757316, 8-27183116, 8-141607252, 17-29297711, 3-49757306, 19-43979341, 8-141607236, 5-176829755, 18-13382140, 15-65341965, 3-13152305, 17-29297770, 8-27183316, 5-176829740, 19-41316693, 18-43830649, 15-65341957, 20-44539531, 7-30265625, 2-131129567, 5-176829665, 3-13152273, 8-27183348, 3-49757302, 19-41316697, 8-61821442, 20-44539525, 10-102883105, 11-65849129, 5-176829639, 15-91129457, 2-1625431, 6-151373292, 6-151373294, 20-25027093, 6-14284198, 10-4049295, 19-59023222, 1-184197132, 2-131004117, 2-8995417, 12-10782319, 20-25027033, 6-151373256, 8-86100970, 9-4839459, 17-41221574, 1-153926715, 20-25027044, 20-20177325, 2-1625443, 20-25027085, 11-69420728, 1-229234865, 6-13408877, 22-50643735, 6-151373308, 1-232119750, 8-134361508, or 6-13408858 is used to determine whether the subject has a liver cancer. In some instances, the subject is determined to have a liver cancer if at least one of the markers 3-49757316, 8-27183116, 8-141607252, 17-29297711, 3-49757306, 19-43979341, 8-141607236, 5-176829755, 18-13382140, 15-65341965, 3-13152305, 17-29297770, 8-27183316, 5-176829740, 19-41316693, 18-43830649, 15-65341957, 20-44539531, 7-30265625, 2-131129567, 5-176829665, 3-13152273, 8-27183348, 3-49757302, 19-41316697, 8-61821442, 20-44539525, 10-102883105, 11-65849129, or 5-176829639 is hypermethylated. In some cases, the subject is determined to have a liver cancer if at least one of the markers 15-91129457, 2-1625431, 6-151373292, 6-151373294, 20-25027093, 6-14284198, 10-4049295, 19-59023222, 1-184197132, 2-131004117, 2-8995417, 12-10782319, 20-25027033, 6-151373256, 8-86100970, 9-4839459, 17-41221574, 1-153926715, 20-25027044, 20-20177325, 2-1625443, 20-25027085, 11-69420728, 1-229234865, 6-13408877, 22-50643735, 6-151373308, 1-232119750, 8-134361508, or 6-13408858 is hypomethylated.

In some instances, the methylation status or pattern of at least one cg marker selected from cg04072843, cg01250961, cg24746106, cg12288267, and cg10430690 is used to determine whether the subject has an ovarian cancer. In some cases, the subject is determined to have an ovarian cancer if at least one of the cg markers cg04072843 and cg01250961 is hypermethylated. In other cases, the subject is determined to have an ovarian cancer if at least one of the cg markers cg24746106, cg12288267, and cg10430690 is hypomethylated.

In some instances, the methylation status or pattern of at least one cg marker selected from cg08131100, cg03788131, cg17528648, cg07784526, cg18948743, cg23986470, cg00846300, cg25352342, cg09921682, cg02504622, cg17373759, cg12028674, cg24820270, cg12028674, cg26718707, cg10349880, and cg09921682 is used to determine whether the subject has a colorectal cancer. In some cases, the subject is determined to have a colorectal cancer if at least one of the cg markers cg08131100, cg03788131, cg17528648, cg07784526, cg18948743, cg23986470, or cg00846300 is hypermethylated. In other cases, the subject is determined to have a colorectal cancer if at least one of the cg markers cg25352342, cg09921682, cg02504622, cg17373759, cg12028674, cg24820270, cg12028674, cg26718707, cg10349880, or cg09921682 is hypomethylated.

In some instances, the methylation status or pattern of at least one cg marker selected from cg10673833, cg10493436, cg10428836, cg27284288, cg16959747, cg17494199, cg23678254, cg24067911, or cg25459300 is used to determine whether the subject has a colorectal cancer.

In some instances, the methylation status or pattern of at least one cg marker selected from cg05205843, cg11841704, cg06699564, cg08924619, cg11959316, cg08924619, cg06699564, cg01824933, cg08924619, cg05205842, cg08924619, cg04049981, cg09026722, cg03616722, cg08924619, cg05928904, cg08704934, cg09776772, cg17494199, cg01824933, cg16296417, cg09776772, cg09776772, cg05338167, cg10493436, cg011251410, cg16391792, cg06393830, cg09366118, cg22513455, cg17583432, cg23881926, cg09638208, cg12441066, cg27284288, cg04441857, cg17583432, cg10673833, cg19757176, cg08670281, cg17583432, cg04460364, cg16959747, cg15011734, or cg25754195 is used to determine whether the subject has a colorectal cancer. In some cases, the subject is determined to have a colorectal cancer if at least one of the cg markers cg06393830, cg09366118, cg22513455, cg17583432, cg23881926, cg09638208, cg12441066, cg27284288, cg04441857, cg17583432, cg10673833, cg19757176, cg08670281, cg17583432, cg04460364, cg16959747, cg15011734, or cg25754195 is hypermethylated. In other cases, the subject is determined to have a colorectal cancer if at least one of the cg markers cg05205843, cg11841704, cg06699564, cg08924619, cg11959316, cg08924619, cg06699564, cg01824933, cg08924619, cg05205842, cg08924619, cg04049981, cg09026722, cg03616722, cg08924619, cg05928904, cg08704934, cg09776772, cg17494199, cg01824933, cg16296417, cg09776772, cg09776772, cg05338167, cg10493436, cg011251410, or cg16391792 is hypomethylated.

In some instances, the methylation status or pattern of at least one cg marker selected from cg01029638, cg08350814, cg05098590, cg18085998, cg06532037, cg15313226, cg16232979, cg26149167, cg06547203, cg06826710, cg00902147, cg17609887, and cg15721142 is used to determine whether the subject has a prostate cancer. In some cases, the subject is determined to have a prostate cancer if at least one of the cg markers cg01029638, cg08350814, cg05098590, cg18085998, cg06532037, cg15313226, cg16232979, or cg26149167 is hypermethylated. In other cases, the subject is determined to have a prostate cancer if at least one of the cg markers cg06547203, cg06826710, cg00902147, cg17609887, or cg15721142 is hypomethylated.

In some instances, the methylation status or pattern of at least one cg marker selected from cg01237565, cg16561543, and cg08116711 is used to determine whether the subject has a pancreatic cancer. In some cases, the subject is determined to have a pancreatic cancer if at least one of the cg markers cg01237565 or cg16561543 is hypermethylated. In other cases, the subject is determined to have a pancreatic cancer if cg marker cg08116711 is hypomethylated.

In some instances, the methylation status or pattern of at least one cg marker selected from cg13771313, cg13771313, and cg08169020 is used to determine whether the subject has acute myeloid leukemia.

In some instances, the methylation status or pattern of at least one cg marker selected from cg08169020, cg21153697, cg07326648, cg14309384, cg20923716, cg22220310, cg21950459, cg13332729, cg10802543, cg20707333, and cg13169641 is used to determine whether the subject has cervical cancer. In some instances, the subject is determined to have cervical cancer if at least one of the cg markers cg08169020, cg21153697, cg07326648, cg14309384, or cg20923716 is hypermethylated. In other instances, the subject is determined to have cervical cancer if at least one of the cg markers cg22220310, cg21950459, cg13332729, cg10802543, cg20707333, or cg13169641 is hypomethylated.

In some instances, the methylation status or pattern of one cg marker cg09095222 is used to determine whether the subject has sarcoma. In some cases, the subject is determined to have sarcoma if at least cg marker cg09095222 is hypermethylated.

In some instances, the methylation status or pattern of at least one cg marker selected from cg00736681 and cg18834029 is used to determine whether the subject has stomach cancer. In some cases, the subject is determined to have stomach cancer if at least one of the cg markers cg00736681 or cg18834029 is hypomethylated.

In some instances, the methylation status or pattern of at least one cg marker selected from cg06969479, cg24630516, and cg16901821 is used to determine if the subject has thyroid cancer. In some cases, the subject is determined to have thyroid cancer if at least one of the cg markers cg06969479, cg24630516, or cg16901821 is hypomethylated.

In some instances, the methylation status or pattern of cg marker cg05630192 is used to determine whether the subject has mesothelioma. In some cases, the subject is determined to have mesothelioma if cg marker cg05630192 is hypomethylated.

In some instances, the methylation status or pattern of cg marker cg06405341 is used to determine whether the subject has glioblastoma.

In some instances, the methylation status or pattern of at least one cg marker selected from cg08557188, cg00690392, cg03421440, and cg07077277 is used to determine whether the subject has lung cancer. In some cases, the subject is determined to have lung cancer if at least one of the cg markers cg08557188, cg00690392, cg03421440, or cg07077277 is hypomethylated.

In some embodiments, the methylation status or pattern of one or more genes selected from *MYO1G*, *ADAMTS4, BMPR1A*, *CD6, RBP5*, Chr 13:10, *LGAP5, ATAN1*, Chr 8:20, or a combination thereof is further used to determine whether the subject has a cancer. In some instances, the methylation status of one or more genes selected from *MYO1G*, *ADAMTS4, BMPR1A*, *CD6, RBP5,* Chr 13:10, *LGAP5, ATXN1*, Chr 8:20, or a combination thereof is further used to determine whether the subject has a colorectal cancer.

In some embodiments, also disclosed herein is a method of determining the prognosis of a cancer in a subject in need thereof based on the methylation status of a set of cg markers. In some embodiments, the method comprises (a) processing a biological sample obtained from a subject with a deaminating agent to generate treated DNA comprising deaminated nucleotides; (b) contacting the treated DNA with at least one probe that hybridizes under high stringency condition to a target sequence of cg10673833 or cg25462303; and (c) quantitatively detecting the methylation status of the cg marker, wherein said detection comprises a real-time quantitative probe-based PCR or a digital probe-based PCR. In some instances, the cancer is a colorectal cancer. In some cases, the method of determining the prognosis of a colorectal cancer a subject in need thereof comprises (a) processing a biological sample obtained from a subject with a deaminating agent to generate treated DNA comprising deaminated nucleotides; (b) contacting the treated DNA with at least one probe that hybridizes under high stringency condition to a target sequence of cg10673833 or cg25462303; and (c) quantitatively detecting the methylation status of the cg marker, wherein said detection comprises a real-time quantitative probe-based PCR or a digital probe-based PCR. In some cases, the methylation status of cg10673833, cg25462303, or a combination thereof is used for monitoring a treatment progression of a subject in need thereof. In additional cases, the methylation status of cg10673833, cg25462303, or a combination thereof is used as an early predictor of developing a cancer (e.g., CRC) of a subject in need thereof.

In some embodiments, additional disclosed herein is a method of determining the prognosis of a cancer in a subject in need thereof, comprising (a) processing a biological sample obtained from a subject with a deaminating agent to generate treated DNA comprising deaminated nucleotides; (b) contacting the treated DNA with at least one probe that hybridizes under high stringency condition to a target sequence of cg05205843, cg11841704, cg06699564, cg08924619, cg11959316, cg08924619, cg06699564, cg01824933, cg08924619, cg05205842, cg08924619, cg04049981, cg09026722, cg03616722, cg08924619, cg05928904, cg08704934, cg09776772, cg17494199, cg01824933, cg16296417, cg09776772, cg09776772, cg05338167, cg10493436, cg011251410, cg16391792, cg06393830, cg09366118, cg22513455, cg17583432, cg23881926, cg09638208, cg12441066, cg27284288, cg04441857, cg17583432, cg10673833, cg19757176, cg08670281, cg17583432, cg04460364, cg16959747, cg15011734, or cg25754195; and (c) quantitatively detecting the methylation status of the cg marker, wherein said detection comprises a real-time quantitative probe-based PCR or a digital probe-based PCR. In some instances, the cancer is a colorectal cancer. In some cases, the method of determining the prognosis of a colorectal cancer a subject in need thereof comprises (a) processing a biological sample obtained from a subject with a deaminating agent to generate treated DNA comprising deaminated nucleotides; (b) contacting the treated DNA with at least one probe that hybridizes under high stringency condition to a target sequence of cg05205843, cg11841704, cg06699564, cg08924619, cg11959316, cg08924619, cg06699564, cg01824933, cg08924619, cg05205842, cg08924619, cg04049981, cg09026722, cg03616722, cg08924619, cg05928904, cg08704934, cg09776772, cg17494199, cg01824933, cg16296417, cg09776772, cg09776772, cg05338167, cg10493436, cg011251410, cg16391792, cg06393830, cg09366118, cg22513455, cg17583432, cg23881926, cg09638208, cg12441066, cg27284288, cg04441857, cg17583432, cg10673833, cg19757176, cg08670281, cg17583432, cg04460364, cg16959747, cg15011734, or cg25754195; and (c) quantitatively detecting the methylation status of the cg marker, wherein said detection comprises a real-time quantitative probe-based PCR or a digital probe-based PCR.

In some instances, if one or more cg markers cg06393830, cg09366118, cg22513455, cg17583432, cg23881926, cg09638208, cg12441066, cg27284288, cg04441857, cg17583432, cg10673833, cg19757176, cg08670281, cg17583432, cg04460364, cg16959747, cg15011734, or cg25754195 are hypermethylated, the prognosis of the cancer is correlated with an advanced tumor stage and poor survival.

In some cases, the methylation status or pattern of cg05205843, cg11841704, cg06699564, cg08924619, cg11959316, cg08924619, cg06699564, cg01824933, cg08924619, cg05205842, cg08924619, cg04049981, cg09026722, cg03616722, cg08924619, cg05928904, cg08704934, cg09776772, cg17494199, cg01824933, cg16296417, cg09776772, cg09776772, cg05338167, cg10493436, cg011251410, cg16391792, cg06393830, cg09366118, cg22513455, cg17583432, cg23881926, cg09638208, cg12441066, cg27284288, cg04441857, cg17583432, cg10673833, cg19757176, cg08670281, cg17583432, cg04460364, cg16959747, cg15011734, cg25754195, or a combination thereof is used for monitoring a treatment progression of a subject in need thereof. In additional cases, the methylation status of cg05205843, cg11841704, cg06699564, cg08924619, cg11959316, cg08924619, cg06699564, cg01824933, cg08924619, cg05205842, cg08924619, cg04049981, cg09026722, cg03616722, cg08924619, cg05928904, cg08704934, cg09776772, cg17494199, cg01824933, cg16296417, cg09776772, cg09776772, cg05338167, cg10493436, cg011251410, cg16391792, cg06393830, cg09366118, cg22513455, cg17583432, cg23881926, cg09638208, cg12441066, cg27284288, cg04441857, cg17583432, cg10673833, cg19757176, cg08670281, cg17583432, cg04460364, cg16959747, cg15011734, cg25754195, or a combination thereof is used as an early predictor of developing a cancer (e.g., CRC) of a subject in need thereof.

In some embodiments, the methylation status or pattern of one or more biomarkers selected from 3-49757316, 8-27183116, 8-141607252, 17-29297711, 3-49757306, 19-43979341, 8-141607236, 5-176829755, 18-13382140, 15-65341965, 3-13152305, 17-29297770, 8-27183316, 5-176829740, 19-41316693, 18-43830649, 15-65341957, 20-44539531, 7-30265625, 2-131129567, 5-176829665, 3-13152273, 8-27183348, 3-49757302, 19-41316697, 8-61821442, 20-44539525, 10-102883105, 11-65849129, 5-176829639, 15-91129457, 2-1625431, 6-151373292, 6-151373294, 20-25027093, 6-14284198, 10-4049295, 19-59023222, 1-184197132, 2-131004117, 2-8995417, 12-10782319, 20-25027033, 6-151373256, 8-86100970, 9-4839459, 17-41221574, 1-153926715, 20-25027044, 20-20177325, 2-1625443, 20-25027085, 11-69420728, 1-229234865, 6-13408877, 22-50643735, 6-151373308, 1-232119750, 8-134361508, or 6-13408858 is used for determining the prognosis of a subject having a liver cancer. In additional cases, the methylation status or pattern of one or more biomarkers selected from 3-49757316, 8-27183116, 8-141607252, 17-29297711, 3-49757306, 19-43979341, 8-141607236, 5-176829755, 18-13382140, 15-65341965, 3-13152305, 17-29297770, 8-27183316, 5-176829740, 19-41316693, 18-43830649, 15-65341957, 20-44539531, 7-30265625, 2-131129567, 5-176829665, 3-13152273, 8-27183348, 3-49757302, 19-41316697, 8-61821442, 20-44539525, 10-102883105, 11-65849129, 5-176829639, 15-91129457, 2-1625431, 6-151373292, 6-151373294, 20-25027093, 6-14284198, 10-4049295, 19-59023222, 1-184197132, 2-131004117, 2-8995417, 12-10782319, 20-25027033, 6-151373256, 8-86100970, 9-4839459, 17-41221574, 1-153926715, 20-25027044, 20-20177325, 2-1625443, 20-25027085, 11-69420728, 1-229234865, 6-13408877, 22-50643735, 6-151373308, 1-232119750, 8-134361508, or 6-13408858 is used for monitoring a treatment progression of a subject having a liver cancer.

In some instances, the biological sample is a blood sample, a urine sample, a saliva sample, a sweat sample, or a tear sample. In some cases, the biological sample is a blood sample or a urine sample. In some cases, the biological sample is a tissue biopsy sample. In some cases, the biological sample is a cell-free DNA sample. In some cases, the biological sample comprises circulating tumor cells.

## Detection Methods

In some embodiments, a number of methods are utilized to measure, detect, determine, identify, and characterize the methylation status/level of a gene or a biomarker (e.g., CpG islandcontaining region/fragment) in identifying a subject as having liver cancer, determining the liver cancer subtype, the prognosis of a subject having liver cancer, and the progression or regression of liver cancer in subject in the presence of a therapeutic agent.

In some instances, the methylation profile is generated from a biological sample isolated from an individual. In some embodiments, the biological sample is a biopsy. In some instances, the biological sample is a tissue sample. In some instances, the biological sample is a tissue biopsy sample. In some instances, the biological sample is a blood sample. In other instances, the biological sample is a cell-free biological sample. In other instances, the biological sample is a circulating tumor DNA sample. In one embodiment, the biological sample is a cell free biological sample containing circulating tumor DNA.

In some embodiments, a biomarker (or an epigenetic marker) is obtained from a liquid sample. In some embodiments, the liquid sample comprises blood and other liquid samples of biological origin (including, peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, cowper's fluid or pre-ejaculatory fluid, female ejaculate, sweat, tears, cyst fluid, pleural and peritoneal fluid, pericardial fluid, ascites, lymph, chyme, chyle, bile, interstitial fluid, menses, pus, sebum, vomit, vaginal secretions/flushing, synovial fluid, mucosal secretion, stool water, pancreatic juice, lavage fluids from sinus cavities, bronchopulmonary aspirates, blastocyl cavity fluid, or umbilical cord blood. In some embodiments, the biological fluid is blood, a blood derivative or a blood fraction, e.g., serum or plasma. In a specific embodiment, a sample comprises a blood sample. In another embodiment, a serum sample is used. In another embodiment, a sample comprises urine. In some embodiments, the liquid sample also encompasses a sample that has been manipulated in any way after their procurement, such as by centrifugation, filtration, precipitation, dialysis, chromatography, treatment with reagents, washed, or enriched for certain cell populations.

In some embodiments, a biomarker (or an epigenetic marker) is obtained from a tissue sample. In some instances, a tissue corresponds to any cell(s). Different types of tissue correspond to different types of cells (e.g., liver, lung, blood, connective tissue, and the like), but also healthy cells vs. tumor cells or to tumor cells at various stages of neoplasia, or to displaced malignant tumor cells. In some embodiments, a tissue sample further encompasses a clinical sample, and also includes cells in culture, cell supernatants, organs, and the like. Samples also comprise fresh-frozen and/or formalin- fixed, paraffin-embedded tissue blocks, such as blocks prepared from clinical or pathological biopsies, prepared for pathological analysis or study by immunohistochemistry.

In some embodiments, a biomarker (or an epigenetic marker) is methylated or unmethylated in a normal sample (e.g., normal or control tissue without disease, or normal or control body fluid, stool, blood, serum, amniotic fluid), most importantly in healthy stool, blood, serum, amniotic fluid or other body fluid. In other embodiments, a biomarker (or an epigenetic marker) is hypomethylated or hypermethylated in a sample from a patient having or at risk of a disease (e.g., one or more indications described herein); for example, at a decreased or increased (respectively) methylation frequency of at least about 50%, at least about 60%, at least about 70%, at least about 75%, at least about 80%, at least about 85%, at least about 90%, at least about 95%, or about 100% in comparison to a normal sample. In one embodiment, a sample is also hypomethylated or hypermethylated in comparison to a previously obtained sample analysis of the same patient having or at risk of a disease (e.g., one or more indications described herein), particularly to compare progression of a disease.

In some embodiments, a methylome comprises a set of epigenetic markers or biomarkers, such as a biomarker described above. In some instances, a methylome that corresponds to the methylome of a tumor of an organism (e.g., a human) is classified as a tumor methylome. In some cases, a tumor methylome is determined using tumor tissue or cell-free (or protein-free) tumor DNA in a biological sample. Other examples of methylomes of interest include the methylomes of organs that contribute DNA into a bodily fluid (e.g. methylomes of tissue such as brain, breast, lung, the prostrate and the kidneys, plasma, etc.).

In some embodiments, a plasma methylome is the methylome determined from the plasma or serum of an animal (e.g., a human). In some instances, the plasma methylome is an example of a cell-free or protein-free methylome since plasma and serum include cell-free DNA. The plasma methylome is also an example of a mixed methylome since it is a mixture of tumor and other methylomes of interest. In some instances, the urine methylome is determined from the urine sample of a subject. In some cases, a cellular methylome corresponds to the methylome determined from cells (e.g., blood cells) of the patient. The methylome of the blood cells is called the blood cell methylome (or blood methylome).

In some embodiments, DNA (e.g., genomic DNA such as extracted genomic DNA or treated genomic DNA) is isolated by any means standard in the art, including the use of commercially available kits. Briefly, wherein the DNA of interest is encapsulated in by a cellular membrane the biological sample is disrupted and lysed by enzymatic, chemical or mechanical means. In some cases, the DNA solution is then cleared of proteins and other contaminants e.g. by digestion with proteinase K. The DNA is then recovered from the solution. In such cases, this is carried out by means of a variety of methods including salting out, organic extraction or binding of the DNA to a solid phase support. In some instances, the choice of method is affected by several factors including time, expense and required quantity of DNA.

Wherein the sample DNA is not enclosed in a membrane (e.g. circulating DNA from a cell free sample such as blood or urine) methods standard in the art for the isolation and/or purification of DNA are optionally employed (See, for example, Bettegowda et al. Detection of Circulating Tumor DNA in Early- and Late-Stage Human Malignancies. Sci. Transl. Med, 6(224): ra24. 2014). Such methods include the use of a protein degenerating reagent e.g. chaotropic salt e.g. guanidine hydrochloride or urea; or a detergent e.g. sodium dodecyl sulphate (SDS), cyanogen bromide. Alternative methods include ethanol precipitation or propanol precipitation, vacuum concentration amongst others by means of a centrifuge. In some cases, the person skilled in the art also make use of devices such as filter devices e.g. ultrafiltration, silica surfaces or membranes, magnetic particles, polystyrol particles, polystyrol surfaces, positively charged surfaces, and positively charged membranes, charged membranes, charged surfaces, charged switch membranes, and charged switched surfaces.

In some instances, once the nucleic acids have been extracted, methylation analysis is carried out by any means known in the art. A variety of methylation analysis procedures is known in the art and may be used to practice the methods disclosed herein. These assays allow for determination of the methylation state of one or a plurality of CpG sites within a tissue sample. In addition, these methods may be used for absolute or relative quantification of methylated nucleic acids. Such methylation assays involve, among other techniques, two major steps. The first step is a methylation specific reaction or separation, such as (i) bisulfite treatment, (ii) methylation specific binding, or (iii) methylation specific restriction enzymes. The second major step involves (i) amplification and detection, or (ii) direct detection, by a variety of methods such as (a) PCR (sequence-specific amplification) such as Taqman(R), (b) DNA sequencing of untreated and bisulfite-treated DNA, (c) sequencing by ligation of dye-modified probes (including cyclic ligation and cleavage), (d) pyrosequencing, (e) single-molecule sequencing, (f) mass spectroscopy, or (g) Southern blot analysis.

Additionally, restriction enzyme digestion of PCR products amplified from bisulfite-converted DNA may be used, e.g., the method described by Sadri and Hornsby (1996, Nucl. Acids Res. 24:5058- 5059), or COBRA (Combined Bisulfite Restriction Analysis) (Xiong and Laird, 1997, Nucleic Acids Res. 25:2532- 2534). COBRA analysis is a quantitative methylation assay useful for determining DNA methylation levels at specific gene loci in small amounts of genomic DNA. Briefly, restriction enzyme digestion is used to reveal methylation-dependent sequence differences in PCR products of sodium bisulfite- treated DNA. Methylation-dependent sequence differences are first introduced into the genomic DNA by standard bisulfite treatment according to the procedure described by Frommer et al. (Frommer et al, 1992, Proc. Nat. Acad. Sci. USA, 89, 1827-1831). PCR amplification of the bisulfite converted DNA is then performed using primers specific for the CpG sites of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from micro-dissected paraffin- embedded tissue samples. Typical reagents (e.g., as might be found in a typical COBRA- based kit) for COBRA analysis may include: PCR primers for specific gene (or methylation-altered DNA sequence or CpG island); restriction enzyme and appropriate buffer; gene-hybridization oligo; control hybridization oligo; kinase labeling kit for oligo probe; and radioactive nucleotides. Additionally, bisulfite conversion reagents may include: DNA denaturation buffer; sulfo nation buffer; DNA recovery reagents or kits (e.g., precipitation, ultrafiltration, affinity column); desulfonation buffer; and DNA recovery components.

In an embodiment, the methylation profile of selected CpG sites is determined using methylation-Specific PCR (MSP). MSP allows for assessing the methylation status of virtually any group of CpG sites within a CpG island, independent of the use of methylation- sensitive restriction enzymes (Herman et al, 1996, Proc. Nat. Acad. Sci. USA, 93, 9821- 9826; U.S. Pat. Nos. 5,786,146, 6,017,704, 6,200,756, 6,265,171 (Herman and Baylin); U.S. Pat. Pub. No. 2010/0144836 (Van Engeland et al); which are hereby incorporated by reference in their entirety). Briefly, DNA is modified by a deaminating agent such as sodium bisulfite to convert unmethylated, but not methylated cytosines to uracil, and subsequently amplified with primers specific for methylated versus unmethylated DNA. In some instances, typical reagents (e.g., as might be found in a typical MSP- based kit) for MSP analysis **include**: methylated and unmethylated PCR primers for specific gene (or methylation- altered DNA sequence or CpG island), optimized PCR buffers and deoxynucleotides, and specific probes. One may use quantitative multiplexed methylation specific PCR (QM-PCR), as described by Fackler et al. Fackler et al, 2004, Cancer Res. 64(13) 4442-4452; or Fackler et al, 2006, Clin. Cancer Res. 12(11 Pt 1) 3306-3310.

In an embodiment, the methylation profile of selected CpG sites is determined using MethyLight and/or Heavy Methyl Methods. The MethyLight and Heavy Methyl assays are a high-throughput quantitative methylation assay that utilizes fluorescence- based real-time PCR (Taq Man(R)) technology that requires no further manipulations after the PCR step (Eads, C.A. et al, 2000, Nucleic Acid Res. 28, e 32; Cottrell et al, 2007, J. Urology 177, 1753, U.S. Pat. Nos. 6,331,393 (Laird et al), the contents of which are hereby incorporated by reference in their entirety). Briefly, the MethyLight process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed either in an "unbiased" (with primers that do not overlap known CpG methylation sites) PCR reaction, or in a "biased" (with PCR primers that overlap known CpG dinucleotides) reaction. In some cases, sequence discrimination occurs either at the level of the amplification process or at the level of the fluorescence detection process, or both. In some cases, the MethyLight assay is used as a quantitative test for methylation patterns in the genomic DNA sample, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence- based version of the "MSP" technique), or with oligonucleotides covering potential methylation sites. Typical reagents (e.g., as might be found in a typical MethyLight- based kit) for MethyLight analysis may include : PCR primers for specific gene (or methylation-altered DNA sequence or CpG island); TaqMan(R) probes; optimized PCR buffers and deoxynucleotides; and Taq polymerase.

Quantitative MethyLight uses bisulfite to convert genomic DNA and the methylated sites are amplified using PCR with methylation independent primers. Detection probes specific for the methylated and unmethylated sites with two different fluorophores provides simultaneous quantitative measurement of the methylation. The Heavy Methyl technique begins with bisulfate conversion of DNA. Next specific blockers prevent the amplification of unmethylated DNA. Methylated genomic DNA does not bind the blockers and their sequences will be amplified. The amplified sequences are detected with a methylation specific probe. (Cottrell et al, 2004, Nuc. Acids Res. 32:e10, the contents of which is hereby incorporated by reference in its entirety).

The Ms-SNuPE technique is a quantitative method for assessing methylation differences at specific CpG sites based on bisulfite treatment of DNA, followed by singlenucleotide primer extension (Gonzalgo and Jones, 1997, Nucleic Acids Res. 25, 2529-2531). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. In some cases, small amounts of DNA are analyzed (e.g., micro-dissected pathology sections), and the method avoids utilization of restriction enzymes for determining the methylation status at CpG sites. Typical reagents (e.g., as is found in a typical Ms-SNuPE-based kit) for Ms- SNuPE analysis include : PCR primers for specific gene (or methylation-altered DNA sequence or CpG island); optimized PCR buffers and deoxynucleotides; gel extraction kit; positive control primers; Ms-SNuPE primers for specific gene; reaction buffer (for the Ms-SNuPE reaction); and radioactive nucleotides. Additionally, bisulfite conversion reagents may include: DNA denaturation buffer; sulfonation buffer; DNA recovery regents or kit (e.g., precipitation, ultrafiltration, affinity column); desulfonation buffer; and DNA recovery components.

In another embodiment, the methylation status of selected CpG sites is determined using differential Binding-based Methylation Detection Methods. For identification of differentially methylated regions, one approach is to capture methylated DNA. This approach uses a protein, in which the methyl binding domain of MBD2 is fused to the Fc fragment of an antibody (MBD-FC) (Gebhard et al, 2006, Cancer Res. 66:6118-6128; and PCT Pub. No. WO 2006/056480 A2 (Relhi), the contents of which are hereby incorporated by reference in their entirety). This fusion protein has several advantages over conventional methylation specific antibodies. The MBD FC has a higher affinity to methylated DNA and it binds double stranded DNA. Most importantly the two proteins differ in the way they bind DNA. Methylation specific antibodies bind DNA stochastically, which means that only a binary answer can be obtained. The methyl binding domain of MBD-FC, on the other hand, binds DNA molecules regardless of their methylation status. The strength of this protein - DNA interaction is defined by the level of DNA methylation. After binding genomic DNA, eluate solutions of increasing salt concentrations can be used to fractionate non- methylated and methylated DNA allowing for a more controlled separation (Gebhard et al, 2006, Nucleic Acids Res. 34: e82). Consequently this method, called Methyl-CpG immunoprecipitation (MCIP), not only enriches, but also fractionates genomic DNA according to methylation level, which is particularly helpful when the unmethylated DNA fraction should be investigated as well.

In an alternative embodiment, a 5 -methyl cytidine antibody to bind and precipitate methylated DNA. Antibodies are available from Abcam (Cambridge, MA), Diagenode (Sparta, NJ) or Eurogentec (c/o AnaSpec, Fremont, CA). Once the methylated fragments have been separated they may be sequenced using microarray based techniques such as methylated CpG-island recovery assay (MIRA) or methylated DNA immunoprecipitation (MeDIP) (Pelizzola et al, 2008, Genome Res. 18, 1652-1659; O'Geen et al, 2006, BioTechniques 41(5), 577-580, Weber et al, 2005, Nat. Genet. 37, 853-862; Horak and Snyder, 2002, Methods Enzymol, 350, 469-83; Lieb, 2003, Methods Mol Biol, 224, 99-109). Another technique is methyl-CpG binding domain column/segregation of partly melted molecules (MBD/SPM, Shiraishi et al, 1999, Proc. Natl. Acad. Sci. USA 96(6):2913-2918).

In some embodiments, methods for detecting methylation include randomly shearing or randomly fragmenting the genomic DNA, cutting the DNA with a methylation-dependent or methylation-sensitive restriction enzyme and subsequently selectively identifying and/or analyzing the cut or uncut DNA. Selective identification can include, for example, separating cut and uncut DNA (e.g., by size) and quantifying a sequence of interest that was cut or, alternatively, that was not cut. See, e.g., U.S. Patent No. 7,186,512. Alternatively, the method can encompass amplifying intact DNA after restriction enzyme digestion, thereby only amplifying DNA that was not cleaved by the restriction enzyme in the area amplified. See, e.g., U.S. Patents No. 7,910,296; No. 7,901,880; and No. 7,459,274. In some embodiments, amplification can be performed using primers that are gene specific.

For example, there are methyl-sensitive enzymes that preferentially or substantially cleave or digest at their DNA recognition sequence if it is non-methylated. Thus, an unmethylated DNA sample is cut into smaller fragments than a methylated DNA sample. Similarly, a hypermethylated DNA sample is not cleaved. In contrast, there are methyl- sensitive enzymes that cleave at their DNA recognition sequence only if it is methylated. Methyl-sensitive enzymes that digest unmethylated DNA suitable for use in methods of the technology include, Hpall, Hhal, Maell, BstUI and Acil. In some instances, an enzyme that is used is Hpall that cuts only the unmethylated sequence CCGG. In other instances, another enzyme that is used is Hhal that cuts only the unmethylated sequence GCGC. Both enzymes are available from New England BioLabs(R), Inc. Combinations of two or more methyl-sensitive enzymes that digest only unmethylated DNA are also used. Suitable enzymes that digest only methylated DNA include, Dpnl, which only cuts at fully methylated 5'-GATC sequences, and McrBC, an endonuclease, which cuts DNA containing modified cytosines (5-methylcytosine or 5-hydroxymethylcytosine or N4- methylcytosine) and cuts at recognition site 5'... PumC(N40-3000) PumC... 3' (New England BioLabs, Inc., Beverly, MA). Cleavage methods and procedures for selected restriction enzymes for cutting DNA at specific sites are well known to the skilled artisan. For example, many suppliers of restriction enzymes provide information on conditions and types of DNA sequences cut by specific restriction enzymes, including New England BioLabs, Promega Biochems, Boehringer-Mannheim, and the like. Sambrook et al. (See Sambrook et al. Molecular Biology: A Laboratory Approach, Cold Spring Harbor, N.Y. 1989) provide a general description of methods for using restriction enzymes and other enzymes.

In some instances, a methylation-dependent restriction enzyme is a restriction enzyme that cleaves or digests DNA at or in proximity to a methylated recognition sequence, but does not cleave DNA at or near the same sequence when the recognition sequence is not methylated. Methylation-dependent restriction enzymes include those that cut at a methylated recognition sequence (e.g., Dpnl) and enzymes that cut at a sequence near but not at the recognition sequence (e.g., McrBC). For example, McrBC's recognition sequence is 5' RmC (N40-3000) RmC 3' where "R" is a purine and "mC" is a methylated cytosine and "N40-3000" indicates the distance between the two RmC half sites for which a restriction event has been observed. McrBC generally cuts close to one half-site or the other, but cleavage positions are typically distributed over several base pairs, approximately 30 base pairs from the methylated base. McrBC sometimes cuts 3' of both half sites, sometimes 5' of both half sites, and sometimes between the two sites. Exemplary methylation-dependent restriction enzymes include, e.g., McrBC, McrA, MrrA, Bisl, Glal and Dpnl. One of skill in the art will appreciate that any methylation-dependent restriction enzyme, including homologs and orthologs of the restriction enzymes described herein, is also suitable for use with one or more methods described herein.

In some cases, a methylation-sensitive restriction enzyme is a restriction enzyme that cleaves DNA at or in proximity to an unmethylated recognition sequence but does not cleave at or in proximity to the same sequence when the recognition sequence is methylated. Exemplary methylation-sensitive restriction enzymes are described in, e.g., McClelland et al, 22(17) NUCLEIC ACIDS RES. 3640-59 (1994). Suitable methylation-sensitive restriction enzymes that do not cleave DNA at or near their recognition sequence when a cytosine within the recognition sequence is methylated at position C5 include, e.g., Aat II, Aci I, Acd I, Age I, Alu I, Asc I, Ase I, AsiS I, Bbe I, BsaA I, BsaH I, BsiE I, BsiW I, BsrF I, BssH II, BssK I, BstB I, BstN I, BstU I, Cla I, Eae I, Eag I, Fau I, Fse I, Hha I, HinPI I, HinC II, Hpa II, Hpy99 I, HpyCH4 IV, Kas I, Mbo I, Mlu I, MapAl I, Msp I, Nae I, Nar I, Not I, Pml I, Pst I, Pvu I, Rsr II, Sac II, Sap I, Sau3A I, Sfl I, Sfo I, SgrA I, Sma I, SnaB I, Tsc I, Xma I, and Zra I. Suitable methylation-sensitive restriction enzymes that do not cleave DNA at or near their recognition sequence when an adenosine within the recognition sequence is methylated at position N6 include, e.g., Mbo I. One of skill in the art will appreciate that any methylation-sensitive restriction enzyme, including homologs and orthologs of the restriction enzymes described herein, is also suitable for use with one or more of the methods described herein. One of skill in the art will further appreciate that a methylation-sensitive restriction enzyme that fails to cut in the presence of methylation of a cytosine at or near its recognition sequence may be insensitive to the presence of methylation of an adenosine at or near its recognition sequence. Likewise, a methylation-sensitive restriction enzyme that fails to cut in the presence of methylation of an adenosine at or near its recognition sequence may be insensitive to the presence of methylation of a cytosine at or near its recognition sequence. For example, Sau3AI is sensitive (i.e., fails to cut) to the presence of a methylated cytosine at or near its recognition sequence, but is insensitive (i.e., cuts) to the presence of a methylated adenosine at or near its recognition sequence. One of skill in the art will also appreciate that some methylation-sensitive restriction enzymes are blocked by methylation of bases on one or both strands of DNA encompassing of their recognition sequence, while other methylation-sensitive restriction enzymes are blocked only by methylation on both strands, but can cut if a recognition site is hemi-methylated.

In alternative embodiments, adaptors are optionally added to the ends of the randomly fragmented DNA, the DNA is then digested with a methylation-dependent or methylation-sensitive restriction enzyme, and intact DNA is subsequently amplified using primers that hybridize to the adaptor sequences. In this case, a second step is performed to determine the presence, absence or quantity of a particular gene in an amplified pool of DNA. In some embodiments, the DNA is amplified using real-time, quantitative PCR.

In other embodiments, the methods comprise quantifying the average methylation density in a target sequence within a population of genomic DNA. In some embodiments, the method comprises contacting genomic DNA with a methylation-dependent restriction enzyme or methylation-sensitive restriction enzyme under conditions that allow for at least some copies of potential restriction enzyme cleavage sites in the locus to remain uncleaved; quantifying intact copies of the locus; and comparing the quantity of amplified product to a control value representing the quantity of methylation of control DNA, thereby quantifying the average methylation density in the locus compared to the methylation density of the control DNA.

In some instances, the quantity of methylation of a locus of DNA is determined by providing a sample of genomic DNA comprising the locus, cleaving the DNA with a restriction enzyme that is either methylation-sensitive or methylation-dependent, and then quantifying the amount of intact DNA or quantifying the amount of cut DNA at the DNA locus of interest. The amount of intact or cut DNA will depend on the initial amount of genomic DNA containing the locus, the amount of methylation in the locus, and the number (i.e., the fraction) of nucleotides in the locus that are methylated in the genomic DNA. The amount of methylation in a DNA locus can be determined by comparing the quantity of intact DNA or cut DNA to a control value representing the quantity of intact DNA or cut DNA in a similarly-treated DNA sample. The control value can represent a known or predicted number of methylated nucleotides. Alternatively, the control value can represent the quantity of intact or cut DNA from the same locus in another (e.g., normal, non-diseased) cell or a second locus.

By using at least one methylation-sensitive or methylation-dependent restriction enzyme under conditions that allow for at least some copies of potential restriction enzyme cleavage sites in the locus to remain uncleaved and subsequently quantifying the remaining intact copies and comparing the quantity to a control, average methylation density of a locus can be determined. If the methylation-sensitive restriction enzyme is contacted to copies of a DNA locus under conditions that allow for at least some copies of potential restriction enzyme cleavage sites in the locus to remain uncleaved, then the remaining intact DNA will be directly proportional to the methylation density, and thus may be compared to a control to determine the relative methylation density of the locus in the sample. Similarly, if a methylation-dependent restriction enzyme is contacted to copies of a DNA locus under conditions that allow for at least some copies of potential restriction enzyme cleavage sites in the locus to remain uncleaved, then the remaining intact DNA will be inversely proportional to the methylation density, and thus may be compared to a control to determine the relative methylation density of the locus in the sample. Such assays are disclosed in, e.g., U.S. Patent No. 7,910,296.

The methylated CpG island amplification (MCA) technique is a method that can be used to screen for altered methylation patterns in genomic DNA, and to isolate specific sequences associated with these changes (Toyota et al, 1999, Cancer Res. 59, 2307-2312, U.S. Pat. No. 7,700,324 (Issa et al), the contents of which are hereby incorporated by reference in their entirety). Briefly, restriction enzymes with different sensitivities to cytosine methylation in their recognition sites are used to digest genomic DNAs from primary tumors, cell lines, and normal tissues prior to arbitrarily primed PCR amplification. Fragments that show differential methylation are cloned and sequenced after resolving the PCR products on high-resolution polyacrylamide gels. The cloned fragments are then used as probes for Southern analysis to confirm differential methylation of these regions. Typical reagents (e.g., as might be found in a typical MCA-based kit) for MCA analysis may include : PCR primers for arbitrary priming Genomic DNA; PCR buffers and nucleotides, restriction enzymes and appropriate buffers; gene-hybridization oligos or probes; control hybridization oligos or probes.

Additional methylation detection methods include those methods described in, e.g., U.S. Patents No. 7,553,627; No. 6,331,393; U.S. Patent Serial No. 12/476,981; U.S. Patent Publication No. 2005/0069879; Rein, et al, 26(10) NUCLEIC ACIDS RES. 2255-64 (1998); and Olek et al, 17(3) NAT. GENET. 275-6 (1997).

In another embodiment, the methylation status of selected CpG sites is determined using Methylation-Sensitive High Resolution Melting (HRM). Recently, Wojdacz et al. reported methylation-sensitive high resolution melting as a technique to assess methylation. (Wojdacz and Dobrovic, 2007, Nuc. Acids Res. 35(6) e41; Wojdacz et al. 2008, Nat. Prot. 3(12) 1903-1908; Balic et al, 2009 J. Mol. Diagn. 11 102- 108; and US Pat. Pub. No. 2009/0155791 (Wojdacz et al), the contents of which are hereby incorporated by reference in their entirety). A variety of commercially available real time PCR machines have HRM systems including the Roche LightCycler480, Corbett Research RotorGene6000, and the Applied Biosystems 7500. HRM may also be combined with other amplification techniques such as pyrosequencing as described by Candiloro et al. (Candiloro et al, 2011, Epigenetics 6(4) 500-507).

In another embodiment, the methylation status of selected CpG locus is determined using a primer extension assay, including an optimized PCR amplification reaction that produces amplified targets for analysis using mass spectrometry. The assay can also be done in multiplex. Mass spectrometry is a particularly effective method for the detection of polynucleotides associated with the differentially methylated regulatory elements. The presence of the polynucleotide sequence is verified by comparing the mass of the detected signal with the expected mass of the polynucleotide of interest. The relative signal strength, e.g., mass peak on a spectra, for a particular polynucleotide sequence indicates the relative population of a specific allele, thus enabling calculation of the allele ratio directly from the data. This method is described in detail in PCT Pub. No. WO 2005/012578A1 (Beaulieu et al), which is hereby incorporated by reference in its entirety. For methylation analysis, the assay can be adopted to detect bisulfite introduced methylation dependent C to T sequence changes. These methods are particularly useful for performing multiplexed amplification reactions and multiplexed primer extension reactions (e.g., multiplexed homogeneous primer mass extension (hME) assays) in a single well to further increase the throughput and reduce the cost per reaction for primer extension reactions.

Other methods for DNA methylation analysis include restriction landmark genomic scanning (RLGS, Costello et al, 2002, Meth. Mol Biol, 200, 53-70), methylation- sensitiverepresentational difference analysis (MS-RDA, Ushijima and Yamashita, 2009, Methods Mol Biol 507, 1 17-130). Comprehensive high-throughput arrays for relative methylation (CHARM) techniques are described in WO 2009/021141 (Feinberg and Irizarry). The Roche(R) NimbleGen(R) microarrays including the Chromatin Immunoprecipitation-on- chip (ChIP-chip) or methylated DNA immunoprecipitation-on-chip (MeDIP-chip). These tools have been used for a variety of cancer applications including melanoma, liver cancer and lung cancer (Koga et al, 2009, Genome Res., 19, 1462-1470; Acevedo et al, 2008, Cancer Res., 68, 2641-2651; Rauch et al, 2008, Proc. Nat. Acad. Sci. USA, 105, 252-257). Others have reported bisulfate conversion, padlock probe hybridization, circularization, amplification and next generation or multiplexed sequencing for high throughput detection of methylation (Deng et al, 2009, Nat. Biotechnol 27, 353-360; Ball et al, 2009, Nat. Biotechnol 27, 361-368; U.S. Pat. No. 7,611,869 (Fan)). As an alternative to bisulfate oxidation, Bayeyt et al. have reported selective oxidants that oxidize 5-methylcytosine, without reacting with thymidine, which are followed by PCR or pyro sequencing (WO 2009/049916 (Bayeyt et al). These references for these techniques are hereby incorporated by reference in their entirety.

In some instances, quantitative amplification methods (e.g., quantitative PCR or quantitative linear amplification) are used to quantify the amount of intact DNA within a locus flanked by amplification primers following restriction digestion. Methods of quantitative amplification are disclosed in, e.g., U.S. Patents No. 6, 180,349; No. 6,033,854; and No. 5,972,602, as well as in, e.g., DeGraves, et al, 34(1) BIOTECHNIQUES 106-15 (2003); Deiman B, et al., 20(2) MOL. BIOTECHNOL. 163-79 (2002); and Gibson et al, 6 GENOME RESEARCH 995-1001 (1996).

Following reaction or separation of nucleic acid in a methylation specific manner, the nucleic acid in some cases are subjected to sequence-based analysis. For example, once it is determined that one particular genomic sequence from a sample is hypermethylated or hypomethylated compared to its counterpart, the amount of this genomic sequence can be determined. Subsequently, this amount can be compared to a standard control value and used to determine the present of liver cancer in the sample. In many instances, it is desirable to amplify a nucleic acid sequence using any of several nucleic acid amplification procedures which are well known in the art. Specifically, nucleic acid amplification is the chemical or enzymatic synthesis of nucleic acid copies which contain a sequence that is complementary to a nucleic acid sequence being amplified (template). The methods and kits may use any nucleic acid amplification or detection methods known to one skilled in the art, such as those described in U.S. Pat. Nos. 5,525,462 (Takarada et al); 6,114,117 (Hepp et al); 6,127,120 (Graham et al); 6,344,317 (Urnovitz); 6,448,001 (Oku); 6,528,632 (Catanzariti et al); and PCT Pub. No. WO 2005/111209 (Nakajima et al); all of which are incorporated herein by reference in their entirety.

In some embodiments, the nucleic acids are amplified by PCR amplification using methodologies known to one skilled in the art. One skilled in the art will recognize, however, that amplification can be accomplished by any known method, such as ligase chain reaction (LCR), Q -replicas amplification, rolling circle amplification, transcription amplification, selfsustained sequence replication, nucleic acid sequence-based amplification (NASBA), each of which provides sufficient amplification. Branched-DNA technology is also optionally used to qualitatively demonstrate the presence of a sequence of the technology, which represents a particular methylation pattern, or to quantitatively determine the amount of this particular genomic sequence in a sample. Nolte reviews branched-DNA signal amplification for direct quantitation of nucleic acid sequences in clinical samples (Nolte, 1998, Adv. Clin. Chem. 33:201-235).

The PCR process is well known in the art and include, for example, reverse transcription PCR, ligation mediated PCR, digital PCR (dPCR), or droplet digital PCR (ddPCR). For a review of PCR methods and protocols, see, e.g., Innis et al, eds., PCR Protocols, A Guide to Methods and Application, Academic Press, Inc., San Diego, Calif. 1990; U.S. Pat. No. 4,683,202 (Mullis). PCR reagents and protocols are also available from commercial vendors, such as Roche Molecular Systems. In some instances, PCR is carried out as an automated process with a thermostable enzyme. In this process, the temperature of the reaction mixture is cycled through a denaturing region, a primer annealing region, and an extension reaction region automatically. Machines specifically adapted for this purpose are commercially available.

In some embodiments, amplified sequences are also measured using invasive cleavage reactions such as the Invader(R) technology (Zou et al, 2010, Association of Clinical Chemistry (AACC) poster presentation on July 28, 2010, "Sensitive Quantification of Methylated Markers with a Novel Methylation Specific Technology; and U.S. Pat. No. 7,011,944 (Prudent et al)).

Suitable next generation sequencing technologies are widely available. Examples include the 454 Life Sciences platform (Roche, Branford, CT) (Margulies et al. 2005 Nature, 437, 376-380); Illumina's Genome Analyzer, GoldenGate Methylation Assay, or Infinium Methylation Assays, i.e., Infinium HumanMethylation 27K BeadArray or VeraCode GoldenGate methylation array (Illumina, San Diego, CA; Bibkova et al, 2006, Genome Res. 16, 383-393; U.S. Pat. Nos. 6,306,597 and 7,598,035 (Macevicz); 7,232,656 (Balasubramanian et al.)); QX200™ Droplet Digital™ PCR System from Bio-Rad; or DNA Sequencing by Ligation, SOLiD System (Applied Biosystems/Life Technologies; U.S. Pat. Nos. 6,797,470, 7,083,917, 7,166,434, 7,320,865, 7,332,285, 7,364,858, and 7,429,453 (Barany et al); the Helicos True Single Molecule DNA sequencing technology (Harris et al, 2008 Science, 320, 106-109; U.S. Pat. Nos. 7,037,687 and 7,645,596 (Williams et al); 7, 169,560 (Lapidus et al); 7,769,400 (Harris)), the single molecule, real-time (SMRT™) technology of Pacific Biosciences, and sequencing (Soni and Meller, 2007, Clin. Chem. 53, 1996-2001); semiconductor sequencing (Ion Torrent; Personal Genome Machine); DNA nanoball sequencing; sequencing using technology from Dover Systems (Polonator), and technologies that do not require amplification or otherwise transform native DNA prior to sequencing (e.g., Pacific Biosciences and Helicos), such as nanopore-based strategies (e.g., Oxford Nanopore, Genia Technologies, and Nabsys). These systems allow the sequencing of many nucleic acid molecules isolated from a specimen at high orders of multiplexing in a parallel fashion. Each of these platforms allows sequencing of clonally expanded or non-amplified single molecules of nucleic acid fragments. Certain platforms involve, for example, (i) sequencing by ligation of dye-modified probes (including cyclic ligation and cleavage), (ii) pyrosequencing, and (iii) single-molecule sequencing.

Pyrosequencing is a nucleic acid sequencing method based on sequencing by synthesis, which relies on detection of a pyrophosphate released on nucleotide incorporation. Generally, sequencing by synthesis involves synthesizing, one nucleotide at a time, a DNA strand complimentary to the strand whose sequence is being sought. Study nucleic acids may be immobilized to a solid support, hybridized with a sequencing primer, incubated with DNA polymerase, ATP sulfurylase, luciferase, apyrase, adenosine 5' phosphsulfate and luciferin. Nucleotide solutions are sequentially added and removed. Correct incorporation of a nucleotide releases a pyrophosphate, which interacts with ATP sulfurylase and produces ATP in the presence of adenosine 5' phosphsulfate, fueling the luciferin reaction, which produces a chemiluminescent signal allowing sequence determination. Machines for pyrosequencing and methylation specific reagents are available from Qiagen, Inc. (Valencia, CA). See also Tost and Gut, 2007, Nat. Prot. 2 2265-2275. An example of a system that can be used by a person of ordinary skill based on pyrosequencing generally involves the following steps: ligating an adaptor nucleic acid to a study nucleic acid and hybridizing the study nucleic acid to a bead; amplifying a nucleotide sequence in the study nucleic acid in an emulsion; sorting beads using a picoliter multiwell solid support; and sequencing amplified nucleotide sequences by pyrosequencing methodology (e.g., Nakano et al, 2003, J. Biotech. 102, 117-124). Such a system can be used to exponentially amplify amplification products generated by a process described herein, e.g., by ligating a heterologous nucleic acid to the first amplification product generated by a process described herein.

## CpG Methylation Data Analysis Methods

In certain embodiments, the methylation values measured for biomarkers of a biomarker panel are mathematically combined and the combined value is correlated to the underlying diagnostic question. In some instances, methylated biomarker values are combined by any appropriate state of the art mathematical method. Well-known mathematical methods for correlating a biomarker combination to a disease status employ methods like discriminant analysis (DA) (e.g., linear-, quadratic-, regularized-DA), Discriminant Functional Analysis (DFA), Kernel Methods (e.g., SVM), Multidimensional Scaling (MDS), Nonparametric Methods (e.g., k-Nearest-Neighbor Classifiers), PLS (Partial Least Squares), Tree-Based Methods (e.g., Logic Regression, CART, Random Forest Methods, Boosting/Bagging Methods), Generalized Linear Models (e.g., Logistic Regression), Principal Components based Methods (e.g., SIMCA), Generalized Additive Models, Fuzzy Logic based Methods, Neural Networks and Genetic Algorithms based Methods. The skilled artisan will have no problem in selecting an appropriate method to evaluate an epigenetic marker or biomarker combination described herein. In one embodiment, the method used in a correlating methylation status of an epigenetic marker or biomarker combination, e.g. to diagnose liver cancer or a liver cancer subtype, is selected from DA (e.g., Linear-, Quadratic-, Regularized Discriminant Analysis), DFA, Kernel Methods (e.g., SVM), MDS, Nonparametric Methods (e.g., k-Nearest-Neighbor Classifiers), PLS (Partial Least Squares), Tree-Based Methods (e.g., Logic Regression, CART, Random Forest Methods, Boosting Methods), or Generalized Linear Models (e.g., Logistic Regression), and Principal Components Analysis. Details relating to these statistical methods are found in the following references: Ruczinski et al., 12 J. OF COMPUTATIONAL AND GRAPHICAL STATISTICS 475-511 (2003); Friedman, J. H., 84 J. OF THE AMERICAN STATISTICAL ASSOCIATION 165-75 (1989); Hastie, Trevor, Tibshirani, Robert, Friedman, Jerome, The Elements of Statistical Learning, Springer Series in Statistics (2001); Breiman, L., Friedman, J. H., Olshen, R. A., Stone, C. J. Classification and regression trees, California: Wadsworth (1984); Breiman, L., 45 MACHINE LEARNING 5-32 (2001); Pepe, M. S., The Statistical Evaluation of Medical Tests for Classification and Prediction, Oxford Statistical Science Series, 28 (2003); and Duda, R. O., Hart, P. E., Stork, D. O., Pattern Classification, Wiley Interscience, 2nd Edition (2001).

In one embodiment, the correlated results for each methylation panel are rated by their correlation to the disease or tumor type positive state, such as for example, by p-value test or t-value test or F-test. Rated (best first, i.e. low p- or t-value) biomarkers are then subsequently selected and added to the methylation panel until a certain diagnostic value is reached. Such methods include identification of methylation panels, or more broadly, genes that were differentially methylated among several classes using, for example, a random-variance t-test (Wright G. W. and Simon R, Bioinformatics 19:2448-2455,2003). Other methods include the step of specifying a significance level to be used for determining the epigenetic markers that will be included in the biomarker panel. Epigenetic markers that are differentially methylated between the classes at a univariate parametric significance level less than the specified threshold are included in the panel. It doesn't matter whether the specified significance level is small enough to exclude enough false discoveries. In some problems better prediction is achieved by being more liberal about the biomarker panels used as features. In some cases, the panels are biologically interpretable and clinically applicable, however, if fewer markers are included. Similar to cross-validation, biomarker selection is repeated for each training set created in the cross-validation process. That is for the purpose of providing an unbiased estimate of prediction error. The methylation panel for use with new patient sample data is the one resulting from application of the methylation selection and classifier of the "known" methylation information, or control methylation panel.

Models for utilizing methylation profile to predict the class of future samples can also be used. These models may be based on the Compound Covariate Predictor (Radmacher et al. Journal of Computational Biology 9:505-511, 2002), Diagonal Linear Discriminant Analysis (Dudoit et al. Journal of the American Statistical Association 97:77-87, 2002), Nearest Neighbor Classification (also Dudoit et al.), and Support Vector Machines with linear kernel (Ramaswamy et al.

PNAS USA 98:15149-54, 2001). The models incorporated markers that were differentially methylated at a given significance level (e.g. 0.01, 0.05 or 0.1) as assessed by the random variance t-test (Wright G. W. and Simon R. Bioinformatics 19:2448-2455, 2003). The prediction error of each model using cross validation, preferably leave-one-out cross-validation (Simon et al. Journal of the National Cancer Institute 95:14-18, 2003can be estimated. For each leave-one-out cross-validation training set, the entire model building process is repeated, including the epigenetic marker selection process. In some instances, it is also evaluated in whether the cross-validated error rate estimate for a model is significantly less than one would expect from random prediction. In some cases, the class labels are randomly permuted and the entire leave-one-out cross-validation process is then repeated. The significance level is the proportion of the random permutations that gives a cross-validated error rate no greater than the cross-validated error rate obtained with the real methylation data.

Another classification method is the greedy-pairs method described by Bo and Jonassen (Genome Biology 3(4):research0017.1-0017.11, 2002). The greedy-pairs approach starts with ranking all markers based on their individual t-scores on the training set. This method attempts to select pairs of markers that work well together to discriminate the classes.

Furthermore, a binary tree classifier for utilizing methylation profile is optionally used to predict the class of future samples. The first node of the tree incorporated a binary classifier that distinguished two subsets of the total set of classes. The individual binary classifiers are based on the "Support Vector Machines" incorporating markers that were differentially expressed among markers at the significance level (e.g. 0.01, 0.05 or 0.1) as assessed by the random variance t-test (Wright G. W. and Simon R. Bioinformatics 19:2448-2455, 2003). Classifiers for all possible binary partitions are evaluated and the partition selected is that for which the cross-validated prediction error is minimum. The process is then repeated successively for the two subsets of classes determined by the previous binary split. The prediction error of the binary tree classifier can be estimated by cross-validating the entire tree building process. This overall cross-validation includes re-selection of the optimal partitions at each node and re-selection of the markers used for each cross-validated training set as described by Simon et al. (Simon et al. Journal of the National Cancer Institute 95:14-18, 2003). Several-fold cross validation in which a fraction of the samples is withheld, a binary tree developed on the remaining samples, and then class membership is predicted for the samples withheld. This is repeated several times, each time withholding a different percentage of the samples. The samples are randomly partitioned into fractional test sets (Simon R and Lam A. BRB-ArrayTools User Guide, version 3.2. Biometric Research Branch, National Cancer Institute).

Thus, in one embodiment, the correlated results for each marker b) are rated by their correct correlation to the disease, preferably by p-value test. It is also possible to include a step in that the markers are selected d) in order of their rating.

In additional embodiments, factors such as the value, level, feature, characteristic, property, etc. of a transcription rate, mRNA level, translation rate, protein level, biological activity, cellular characteristic or property, genotype, phenotype, etc. can be utilized in addition prior to, during, or after administering a therapy to a patient to enable further analysis of the patient's cancer status.

In some embodiments, a diagnostic test to correctly predict status is measured as the sensitivity of the assay, the specificity of the assay or the area under a receiver operated characteristic ("ROC") curve. In some instances, sensitivity is the percentage of true positives that are predicted by a test to be positive, while specificity is the percentage of true negatives that are predicted by a test to be negative. In some cases, an ROC curve provides the sensitivity of a test as a function of 1- specificity. The greater the area under the ROC curve, for example, the more accurate or powerful the predictive value of the test. Other useful measures of the utility of a test include positive predictive value and negative predictive value. Positive predictive value is the percentage of people who test positive that are actually positive. Negative predictive value is the percentage of people who test negative that are actually negative.

In some embodiments, one or more of the biomarkers disclosed herein show a statistical difference in different samples of at least $p<0.05$, $p<10^{-2}$, $p<10^{-3}$, $p<10^{-4}$ or $p<10^{-5}$. Diagnostic tests that use these biomarkers may show an ROC of at least 0.6, at least about 0.7, at least about 0.8, or at least about 0.9. In some instances, the biomarkers are differentially methylated in different subjects with or without liver cancer. In additional instances, the biomarkers for different subtypes of liver cancer are differentially methylated. In certain embodiments, the biomarkers are measured in a patient sample using the methods described herein and compared, for example, to predefined biomarker levels and are used to determine whether the patient has liver cancer, which liver cancer subtype does the patient have, and/or what is the prognosis of the patient having liver cancer. In other embodiments, the correlation of a combination of biomarkers in a patient sample is compared, for example, to a predefined set of biomarkers. In some embodiments, the measurement(s) is then compared with a relevant diagnostic amount(s), cut-off(s), or multivariate model scores that distinguish between the presence or absence of liver cancer, between liver cancer subtypes, and between a "good" or a "poor" prognosis. As is well understood in the art, by adjusting the particular diagnostic cut-off(s) used in an assay, one can increase sensitivity or specificity of the diagnostic assay depending on the preference of the diagnostician. In some embodiments, the particular diagnostic cut-off is determined, for example, by measuring the amount of biomarker hypermethylation or hypomethylation in a statistically significant number of samples from patients with or without liver cancer and from patients with different liver cancer subtypes, and drawing the cut-off to suit the desired levels of specificity and sensitivity.

**Kits/Article of Manufacture**

In some embodiments, provided herein include kits for detecting and/or characterizing the methylation profile of a biomarker described herein. In some instances, the kit comprises a plurality of primers or probes to detect or measure the methylation status/levels of one or more samples. Such kits comprise, in some instances, at least one polynucleotide that hybridizes to at least one of the methylation marker sequences described herein and at least one reagent for detection of gene methylation. Reagents for detection of methylation include, e.g., sodium bisulfate, polynucleotides designed to hybridize to sequence that is the product of a marker sequence if the marker sequence is not methylated (e.g., containing at least one C-U conversion), and/or a methylation- sensitive or methylation-dependent restriction enzyme. In some cases, the kits provide solid supports in the form of an assay apparatus that is adapted to use in the assay. In some instances, the kits further comprise detectable labels, optionally linked to a polynucleotide, e.g., a probe, in the kit.

In some embodiments, the kits comprise one or more (e.g., 1, 2, 3, 4, or more) different polynucleotides (e.g., primers and/or probes) capable of specifically amplifying at least a portion of a DNA region of a biomarker described herein. Optionally, one or more detectably-labeled polypeptides capable of hybridizing to the amplified portion are also included in the kit. In some embodiments, the kits comprise sufficient primers to amplify 2, 3, 4, 5, 6, 7, 8, 9, 10, or more different DNA regions or portions thereof, and optionally include detectably-labeled polynucleotides capable of hybridizing to each amplified DNA region or portion thereof. The kits further can comprise a methylation-dependent or methylation sensitive restriction enzyme and/or sodium bisulfite.

In some embodiments, the kits comprise sodium bisulfite, primers and adapters (e.g., oligonucleotides that can be ligated or otherwise linked to genomic fragments) for whole genome amplification, and polynucleotides (e.g., detectably-labeled polynucleotides) to quantify the presence of the converted methylated and or the converted unmethylated sequence of at least one cytosine from a DNA region of an epigenetic marker described herein.

In some embodiments, the kits comprise methylation sensing restriction enzymes (e.g., a methylation-dependent restriction enzyme and/or a methylation-sensitive restriction enzyme), primers and adapters for whole genome amplification, and polynucleotides to quantify the number of copies of at least a portion of a DNA region of an epigenetic marker described herein.

In some embodiments, the kits comprise a methylation binding moiety and one or more polynucleotides to quantify the number of copies of at least a portion of a DNA region of a marker described herein. A methylation binding moiety refers to a molecule (e.g., a polypeptide) that specifically binds to methyl-cytosine.

Examples include restriction enzymes or fragments thereof that lack DNA cutting activity but retain the ability to bind methylated DNA, antibodies that specifically bind to methylated DNA, etc.).

In some embodiments, the kit includes a packaging material. As used herein, the term "packaging material" can refer to a physical structure housing the components of the kit. In some instances, the packaging material maintains sterility of the kit components, and is made of material commonly used for such purposes (e.g., paper, corrugated fiber, glass, plastic, foil, ampules, etc.). Other materials useful in the performance of the assays are included in the kits, including test tubes, transfer pipettes, and the like. In some cases, the kits also include written instructions for the use of one or more of these reagents in any of the assays described herein.

In some embodiments, kits also include a buffering agent, a preservative, or a protein/nucleic acid stabilizing agent. In some cases, kits also include other components of a reaction mixture as described herein. For example, kits include one or more aliquots of thermostable DNA polymerase as described herein, and/or one or more aliquots of dNTPs. In some cases, kits also include control samples of known amounts of template DNA molecules harboring the individual alleles of a locus. In some embodiments, the kit includes a negative control sample, e.g., a sample that does not contain DNA molecules harboring the individual alleles of a locus. In some embodiments, the kit includes a positive control sample, e.g., a sample containing known amounts of one or more of the individual alleles of a locus.

**Certain Terminology**

Unless defined otherwise, all technical and scientific terms used herein have the same meaning as is commonly understood by one of skill in the art to which the claimed subject matter belongs. It is to be understood that the foregoing general description and the following detailed description are exemplary and explanatory only and are not restrictive of any subject matter claimed. In this application, the use of the singular includes the plural unless specifically stated otherwise. It must be noted that, as used in the specification and the appended claims, the singular forms "a," "an" and "the" include plural referents unless the context clearly dictates otherwise. In this application, the use of "or" means "and/or" unless stated otherwise. Furthermore, use of the term "including" as well as other forms, such as "include", "includes," and "included," is not limiting.

As used herein, ranges and amounts can be expressed as "about" a particular value or range. About also includes the exact amount. Hence "about 5 μL" means "about 5 μL" and also "5 μL." Generally, the term "about" includes an amount that would be expected to be within experimental error.

The section headings used herein are for organizational purposes only and are not to be construed as limiting the subject matter described.

As used herein, the terms "individual(s)", "subject(s)" and "patient(s)" mean any mammal. In some embodiments, the mammal is a human. In some embodiments, the mammal is a non-human. None of the terms require or are limited to situations characterized by the supervision (e.g. constant or intermittent) of a health care worker (e.g. a doctor, a registered nurse, a nurse practitioner, a physician's assistant, an orderly or a hospice worker).

A "site" corresponds to a single site, which in some cases is a single base position or a group of correlated base positions, e.g., a CpG site. A "locus" corresponds to a region that includes multiple sites. In some instances, a locus includes one site.

## EXAMPLES

These examples are provided for illustrative purposes only and not to limit the scope of the claims provided herein.

## EXAMPLE 1

Table 1 illustrates top 100 cg markers per cancer type, subdivided based on tissue comparison categories. Table 1 is included at the end of the Examples section.

Table 2 illustrates exemplary 20 cg makers per cancer type.

| BLCA | Marker | CpG | BRCA | Marker | CpG |
|---|---|---|---|---|---|
| | 17-75447478 | cg14517217 | | 6-33739558 | cg18005901 |
| | 6-106958474 | cg09184502 | | 9-129445599 | cg14100888 |
| | 17-75447504 | cg18104645 | | 6-33739406 | cg07979401 |
| | 17-17686141 | cg19872299 | | 1-6508954 | cg25097074 |
| | 17-17685407 | cg25927164 | | 1-6508592 | cg15274864 |
| | 17-75447785 | cg11991516 | | 2-241034966 | cg18663033 |
| | 22-21319661 | cg07960806 | | 9-129373499 | cg19337180 |
| | 17-17685582 | cg15906409 | | 2-100175805 | cg17165836 |
| | 17-48619308 | cg00698906 | | 11-65405492 | cg27068330 |
| | 6-28911474 | cg05127899 | | 2-100175704 | cg18261069 |
| Hypermethylated | 2-20866242 | cg19113641 | Hypermethylated | 9-129388668 | cg04693928 |
| | 17-48619672 | cg27437806 | | 21-46352817 | cg17648210 |
| | 6-106958645 | cg22231056 | | 1-155043322 | |
| | 17-8055756 | cg23363911 | | 11-128392102 | cg18565473 |
| | 2-20866231 | cg21039778 | | 9-36037454 | |
| | 17-46671298 | cg02293936 | | 6-160769754 | cg07237939 |
| | 6-106958303 | cg04067276 | | 11-65405760 | cg01685242 |
| | 22-21319668 | cg11782550 | | 2-100175768 | cg24797187 |
| | 20-34189411 | cg13544006 | | 19-11354132 | |
| | 6-106958640 | cg25944720 | | 9-36037340 | |

(continued)

| BLCA | Marker | CpG | BRCA | Marker | CpG |
|---|---|---|---|---|---|
| Hypomethylated | 20-50419274 | cg12288473 | Hypomethylated | 8-128403369 | cg11118198 |
| | 16-1576146 | cg27316811 | | 6-32135186 | cg11108115 |
| | 7-1932321 | cg23618217 | | 19-45575023 | cg15732530 |
| | 17-767334 | cg25236028 | | 2-121036440 | cg20349803 |
| | 4-141188341 | cg06775361 | | 2-121036760 | cg25950520 |
| | 8-123966105 | cg01886570 | | 12-132286017 | cg13251533 |
| | 13-114187990 | cg13241681 | | 1-204531767 | cg00827973 |
| | 2-25427108 | cg20422417 | | 2-85622094 | cg27090078 |
| | 17-767301 | cg13476078 | | 9-108424905 | cg06119575 |
| | 6-18368796 | cg23221431 | | 6-33288804 | cg15070677 |
| | 6-106582669 | cg25581448 | | 2-121036705 | cg27505472 |
| | 6-106582638 | cg17356964 | | 1-245925961 | cg23610994 |
| | 12-90313611 | cg20490773 | | 8-126082720 | |
| | 1-8384659 | cg10508347 | | 14-81865007 | cg10334928 |
| | 5-180219707 | cg18114890 | | 4-141564017 | cg03135535 |
| | 19-8670086 | cg11745755 | | 1-168250628 | cg17095936 |
| | 3-12332780 | cg16827534 | | 1-162694184 | cg19313373 |
| | 2-219256018 | cg23212579 | | 9-97856079 | cg13763287 |
| | 17-25619946 | cg07564598 | | 7-127984393 | cg08309135 |
| | 2-219256101 | cg16926316 | | 15-39472102 | cg13182816 |

(continued)

| CHOL | Marker | CpG | COAD | Marker | CpG |
|---|---|---|---|---|---|
| | 6-32547019 | cg07984380 | | 2-29338432 | cg08808128 |
| | 1-198904195 | cg09517106 | | 2-66803033 | cg02764245 |
| | 5-174150712 | cg26092471 | | 15-28351906 | cg04803843 |
| | 12-108733275 | cg12142354 | | 4-55991782 | cg07893544 |
| | 1-113498106 | cg12551029 | | 1-76080727 | |
| | 12-102872937 | cg11612617 | | 5-38556435 | cg12587766 |
| | 6-27782628 | cg05444312 | | 20-61051039 | cg14980983 |
| | 3-120627406 | cg05293820 | | 7-149411652 | cg08131100 |
| | 6-27840105 | cg05511483 | | 20-34894648 | |
| Hypermethylated | 2-158114424 | | Hypermethylated | 15-28352098 | cg03061682 |
| | 2-136743460 | cg18110444 | | 20-61051032 | cg20265733 |
| | 6-32522683 | cg25140213 | | 12-45444895 | |
| | 6-27782484 | cg16347724 | | 8-97506675 | cg04261408 |
| | 1-113497932 | cg16251079 | | 1-115880865 | cg09008705 |
| | 3-157824209 | cg14961949 | | 6-127440000 | cg19365062 |
| | 4-147443095 | cg20910303 | | 9-707166 | cg14399863 |
| | 1-113498174 | cg17020695 | | 9-707387 | cg13877502 |
| | 5-111115809 | cg11657018 | | 4-55991860 | cg00989765 |
| | 1-113497999 | cg07423363 | | 4-157997554 | cg11163620 |
| | 12-4381788 | cg15993083 | | 1-57889035 | |

(continued)

| CHOL | Marker | CpG | COAD | Marker | CpG |
|---|---|---|---|---|---|
| | 13-111553101 | cg23052386 | | 19-39306244 | cg12028674 |
| | 1-23418405 | cg15897613 | | 3-150238257 | cg02504622 |
| | 6-36237037 | cg27319730 | | 10-518370 | cg26718707 |
| | 7-1946447 | cg25786640 | | 20-57616191 | cg20726575 |
| | 6-105854768 | cg18879177 | | 4-187629336 | cg24820270 |
| | 1-117489843 | cg23093116 | | 4-187629387 | cg25352342 |
| | 13-101241430 | | | 4-187629328 | cg21399040 |
| | 3-24213704 | cg25087352 | | 17-48845009 | cg25518968 |
| | 1-202559755 | cg11143857 | | 7-150069026 | cg17677524 |
| | 20-36033352 | cg14637027 | | 4-6945745 | cg16574807 |
| Hypomethylated | 10-5542941 | cg26552733 | Hypomethylated | 5-179393917 | cg26722544 |
| | 6-34231501 | cg15495717 | | 2-106959257 | cg24419520 |
| | 11-134118834 | cg22756211 | | 17-80535428 | cg03454705 |
| | 6-16147669 | cg15404791 | | 1-1095607 | cg02971920 |
| | 10-135097477 | cg27633010 | | 2-8850020 | cg00240378 |
| | 10-76801843 | cg23077498 | | 7-73408058 | cg16233515 |
| | 15-102261251 | cg14588686 | | 11-2287734 | cg14907310 |
| | 14-65210900 | cg11849213 | | 7-73408101 | cg18780627 |
| | 10-119101310 | cg17182036 | | 2-106959384 | cg 13656404 |
| | 11-126072352 | cg26351663 | | 2-112808038 | cg25890678 |

(continued)

| ESCA | Marker | CpG | GBM | Marker | CpG |
|---|---|---|---|---|---|
| Hypermethylated | 19-57078765 | | Hypermethylated | 6-43237330 | cg23514619 |
| | 19-56915732 | | | 19-19336240 | cg25814383 |
| | 19-56915650 | | | 17-55938871 | cg22451358 |
| | 19-56915658 | | | 12-57504948 | cg01063813 |
| | 19-56915655 | | | 19-1468943 | cg10565187 |
| | 19-56915653 | | | 6-43237511 | cg20434586 |
| | 7-37960873 | | | 4-81123613 | cg22054918 |
| | 19-57078780 | | | 19-35531817 | cg24139898 |
| | 19-12175935 | | | 14-70038925 | cg02380334 |
| | 19-57078783 | | | 6-74233510 | cg09681335 |
| | 19-53696642 | | | 17-55938748 | cg00023001 |
| | 19-12175631 | | | 19-35531859 | cg21484586 |
| | 15-48470516 | | | 19-7937107 | cg00438215 |
| | 19-40315143 | cg00498691 | | 1-27894955 | cg12610832 |
| | 7-37960317 | | | 11-65314161 | |
| | 7-37960731 | | | 19-2614086 | cg19250790 |
| | 2-80531483 | | | 1-26690537 | cg12813724 |
| | 12-94543449 | | | 16-744328 | cg05542681 |
| | 14-60097247 | | | 16-4233246 | cg05006755 |
| | 19-53696649 | cg01620580 | | 2-58273552 | cg18998670 |

(continued)

| ESCA | Marker | CpG | GBM | Marker | CpG |
|---|---|---|---|---|---|
| Hypomethylated | 1-1563500 | cg09159050 | Hypomethylated | 12-131452297 | cg23617848 |
| | 5-131725325 | cg23475112 | | 1-10240017 | cg27508545 |
| | X-2836027 | | | 7-4747216 | cg01275887 |
| | 7-157204364 | cg18362281 | | 2-30041338 | cg00073794 |
| | 7-112431576 | cg04876978 | | 18-42325086 | cg07015525 |
| | 1-19280186 | cg11697440 | | 4-146804010 | cg01578875 |
| | 19-57105832 | cg19641839 | | 7-101849120 | cg06010390 |
| | 7-63772811 | | | 1-10240025 | cg23661000 |
| | 9-139913436 | | | 4-3372206 | |
| | 1-156232301 | cg10199857 | | 1-6390793 | cg00081799 |
| | 19-57082536 | | | 6-24140899 | cg08908855 |
| | 5-138032270 | | | 7-4747261 | cg07076109 |
| | 21-44195410 | | | 16-50897271 | cg00900231 |
| | 16-84446919 | cg00838040 | | 5-140865433 | cg11830096 |
| | 11-47213196 | cg05584439 | | 10-405225 | cg20595750 |
| | 8-19026658 | | | 17-76220929 | cg07366188 |
| | 6-163730283 | cg10584587 | | 17-63535223 | cg06405341 |
| | 1-228362509 | cg18277682 | | 22-42611379 | cg26799416 |
| | 9-139907691 | cg14173587 | | 8-9537322 | cg10419849 |
| | 1-43971496 | cg25211006 | | 18-42324965 | cg03722909 |

(continued)

| KICH | Marker | CpG | KIRC | Marker | CpG |
|---|---|---|---|---|---|
| | 1-184357556 | cg10061342 | | 13-113623844 | cg09678836 |
| | 16-4103492 | cg10177032 | | 5-66299786 | cg02632185 |
| | 15-70767570 | cg16973527 | | 5-66300503 | cg27116842 |
| | 4-74570525 | cg23099587 | | 13-113623639 | cg00127894 |
| | 5-43772975 | | | 13-113623659 | cg15179805 |
| | X-78201182 | | | 5-66300406 | cg17449851 |
| | 7-28726216 | cg05083033 | | 5-66300433 | cg11831286 |
| | X-107020894 | | | 12-54427636 | cg16983211 |
| | 1-9224198 | cg22373770 | | 12-52214119 | cg19495013 |
| | 15-96957617 | | | 13-113623300 | cg16737670 |
| Hypermethylated | 16-474271 | cg09555736 | Hypermethylated | 12-54422350 | cg27441225 |
| | 10-34320548 | cg10772185 | | 8-107669856 | cg17176732 |
| | 6-168134760 | cg01797450 | | 5-66299875 | cg18118497 |
| | 16-30347333 | | | 8-107669783 | cg17136799 |
| | 10-17273187 | cg26063719 | | 13-113623233 | cg02223067 |
| | 12-10022688 | cg12591315 | | 8-107669787 | cg26622232 |
| | 6-166970727 | cg11387340 | | 17-18908235 | cg14807365 |
| | 16-15766942 | | | 2-73151879 | cg00486564 |
| | 16-474430 | cg02102075 | | 12-54427700 | cg15700739 |
| | 11-7728814 | cg13563074 | | 12-54422488 | cg22378817 |

(continued)

| KICH | Marker | CpG | KIRC | Marker | CpG |
|---|---|---|---|---|---|
| Hypomethylated | 4-185362534 | cg02354388 | Hypomethylated | 13-96086098 | cg20278383 |
| | 16-87873837 | cg06665333 | | 2-239169591 | |
| | 22-35694370 | cg27436324 | | 8-30209391 | cg14895559 |
| | 8-6616240 | cg24485696 | | 16-81565917 | cg03212183 |
| | 20-57471672 | | | 13-111880998 | cg21980364 |
| | 11-66494177 | cg22015277 | | 4-87993109 | cg27000934 |
| | 5-173307471 | cg13384849 | | 16-15127193 | cg06978461 |
| | 20-57471660 | | | 1-217724849 | cg10325088 |
| | 4-38006787 | cg23289854 | | 20-6025272 | cg21209310 |
| | 17-81045501 | cg14521546 | | 7-101556684 | cg15600935 |
| | 16-479994 | cg10357909 | | 17-78700928 | cg06975080 |
| | 10-126314680 | cg04426802 | | 11-64323816 | cg21039563 |
| | 7-98667581 | cg09221667 | | 17-18855313 | cg00306284 |
| | 7-76871793 | | | 3-149051159 | cg11011913 |
| | 3-196693809 | cg21429725 | | 14-89628141 | cg02212698 |
| | 19-45316509 | cg21978694 | | 15-39958882 | |
| | 7-23723014 | cg20200811 | | 17-40064155 | cg03950716 |
| | 19-45316493 | cg12249345 | | 1-101094117 | cg00902464 |
| | 14-95585023 | cg23488578 | | 7-101566857 | cg02307880 |
| | 13-33768013 | | | 5-111017556 | cg01885839 |

(continued)

| LIHC | Marker | CpG | LUAD | Marker | CpG |
|---|---|---|---|---|---|
| Hypermethylated | 2-264178 | cg20749741 | Hypermethylated | 6-152623304 | cg05917732 |
| | 1-145395753 | cg17952661 | | 10-8092165 | cg15803869 |
| | 19-54369556 | | | 6-152623479 | cg12023625 |
| | 3-183543564 | cg19155007 | | 1-23668691 | cg00907427 |
| | 2-264199 | cg00108164 | | 10-119304102 | |
| | 19-50096610 | cg15032314 | | 1-23668792 | cg08718097 |
| | 6-43044771 | cg21663580 | | 21-40984780 | cg08448665 |
| | 1-145395846 | cg02395363 | | 10-119304104 | cg12894449 |
| | 6-56819429 | | | 7-2271722 | cg03694580 |
| | 3-183543587 | cg09738156 | | 21-40984777 | cg17900854 |
| | 19-54369576 | cg23305567 | | 11-31832246 | cg26848086 |
| | 2-233792394 | cg08165971 | | 17-70119120 | cg21126344 |
| | 2-264204 | cg25945732 | | 10-8096370 | cg15187550 |
| | 19-6740952 | cg03705926 | | 21-40984886 | cg03701992 |
| | 5-176831297 | cg14126493 | | 1-166134346 | |
| | 11-75917844 | cg17583449 | | 11-31832353 | cg05840031 |
| | 5-176831638 | cg05876864 | | 10-8092264 | cg16710894 |
| | 1-160370208 | cg13428480 | | 6-50680997 | cg17489695 |
| | 2-264164 | cg03468349 | | 10-8091753 | cg22783180 |
| | 1-21616619 | cg17216478 | | 6-152623387 | cg02682457 |

(continued)

| LIHC | Marker | CpG | LUAD | Marker | CpG |
|---|---|---|---|---|---|
| Hypomethylated | 22-50644755 | cg25934700 | Hypomethylated | 7-2473529 | cg17907628 |
| | 5-16794882 | cg12852139 | | 7-2473859 | cg18259487 |
| | 15-99427016 | cg03310087 | | 5-141722174 | cg02084814 |
| | 10-1334207 | cg04992974 | | 10-1120831 | |
| | 16-87970367 | cg05640992 | | 6-25726912 | cg07077277 |
| | 11-46741373 | cg26453360 | | 16-3139060 | cg20364187 |
| | 7-1197113 | cg03923535 | | 2-232113244 | cg27273946 |
| | 12-69199037 | cg10635494 | | 14-74957673 | |
| | 8-142263802 | cg00971369 | | 6-25727291 | |
| | 11-6462110 | cg14164596 | | 14-91691190 | cg14304073 |
| | 5-17275783 | cg01860297 | | 13-113445870 | |
| | 16-87970376 | cg03550864 | | 6-25727107 | |
| | 7-20269648 | cg19086110 | | 13-98869844 | cg16689193 |
| | 1-167882440 | cg24461337 | | 14-91691129 | cg16996571 |
| | 2-44065725 | cg20926720 | | 4-37962455 | |
| | 7-4012372 | cg04609841 | | 6-25726953 | |
| | 11-102638470 | cg03367136 | | 10-52156632 | |
| | 17-26694939 | cg18536123 | | 6-25726956 | |
| | 10-93390347 | cg24288527 | | 1-207975699 | |
| | 22-21128411 | cg09634469 | | 16-3641320 | |

(continued)

| LUSC | Marker | CpG | OV | Marker | CpG |
|---|---|---|---|---|---|
| **Hypermethylated** | 19-10406145 | cg23936587 | | 19-58281450 | cg14038484 |
| | 2-132348705 | cg11467141 | | 19-58281016 | cg18575209 |
| | 15-73889528 | cg06332339 | | 19-58280832 | cg03584535 |
| | 2-239970152 | cg08781549 | | 19-58281117 | cg22956410 |
| | 2-239970075 | cg26126749 | | 19-58280927 | cg13636880 |
| | 1-183204836 | cg14420230 | | 19-58280891 | cg07685728 |
| | 12-21547892 | cg14191244 | | 19-58280801 | cg27046034 |
| | 12-63195796 | cg27185063 | | 19-58281019 | cg20751795 |
| | 1-183204789 | cg22684969 | | 19-58280994 | cg24298255 |
| | 5-14160853 | | | 11-133582402 | cg11368617 |
| | 15-73889561 | cg08435157 | **Hypermethylated** | 12-130529951 | cg16236851 |
| **Hypomethylated** | 3-185704206 | cg09562655 | | 10-95327305 | cg05613447 |
| | 12-11286360 | cg24741148 | | 10-95327292 | cg10905401 |
| | 12-132643897 | cg16860971 | | 10-74870311 | cg01701415 |
| | 12-93101181 | cg17344080 | | 17-36104218 | |
| | 7-148481030 | cg25477176 | | 8-54569668 | cg05483021 |
| | 22-30572326 | cg24211826 | | 10-74870240 | cg05898953 |
| | 19-5789699 | cg22961623 | | 1-205399945 | cg06849719 |
| | 7-101603335 | cg05855116 | | 13-110899996 | cg15416440 |
| | 1-64992433 | | | 1-205400197 | cg06968878 |
| | 15-73524906 | cg08177743 | | | |

(continued)

| PAAD | Marker | CpG | PRAD | Marker | CpG |
|---|---|---|---|---|---|
| Hypermethylated | 14-105715025 | cg01237565 | Hypermethylated | 19-51416098 | cg17355294 |
| | 16-1202468 | cg16427096 | | 11-58940866 | cg05415131 |
| | 14-105714843 | cg16561543 | | 19-16187631 | cg16232979 |
| | 14-105714589 | cg23034818 | | 6-146136749 | cg09094393 |
| | 20-55204593 | cg12521353 | | 7-32981826 | cg08946731 |
| | 10-11059577 | cg23858040 | | 10-74020976 | cg12799885 |
| | 13-32605406 | cg22219254 | | 2-191045309 | cg08350814 |
| | 13-32605611 | cg16941656 | | 7-120628874 | cg01029638 |
| | 2-177053345 | cg19001226 | | 5-43193681 | cg10641714 |
| | 4-141293985 | | | 19-16187889 | cg26149167 |
| | 16-86231948 | cg07989221 | | 9-27529339 | cg21244846 |
| | 5-172756558 | cg16443866 | | 15-35014270 | cg24922143 |
| | 5-172756550 | cg08711858 | | 17-46830260 | cg13132370 |
| | 1-182584341 | cg00153856 | | 12-89744701 | cg05769889 |
| | 14-105714833 | cg15474367 | | 19-51416517 | cg20115266 |
| | 11-61276678 | | | 6-146136563 | cg23095615 |
| | 4-141294009 | | | 10-111767379 | cg05098590 |
| | 22-17849639 | cg26796679 | | 1-116711077 | cg17933722 |
| | 9-126773879 | cg10755973 | | 2-191045668 | cg15313226 |
| | 2-107503672 | | | 1-33938224 | cg18085998 |

(continued)

| SKCM | Marker | CpG | UCEC | Marker | CpG |
|---|---|---|---|---|---|
| Hypermethylated | 7-5468436 | | Hypermethylated | 17-42092403 | cg06511389 |
| | 2-166650606 | | | 17-42092315 | cg27116819 |
| | 11-19798742 | | | 17-42092187 | cg18801599 |
| | 17-77815779 | | | 19-50836561 | cg01017355 |
| | 6-10419927 | | | 17-42092370 | cg08372947 |
| | 11-441986 | | | 17-42092247 | cg24638647 |
| | 12-112204829 | | | 17-42092450 | cg13847066 |
| | 14-61188431 | | | 18-5238310 | cg20757519 |
| | 12-48398374 | cg17054969 | | 17-42091909 | cg04879755 |
| | 6-1311342 | | | 18-5238589 | cg10885961 |
| | 10-25241427 | | | 18-5238219 | cg15258847 |
| | 6-10420079 | | | 17-42092488 | cg25868286 |
| | 1-111747228 | | | 17-42092472 | cg01635193 |
| | 6-26332204 | | | 3-169482895 | cg15494117 |
| | 21-38630728 | | | 6-26045663 | cg25438963 |
| | 10-25241462 | | | 17-42092431 | cg12259256 |
| | 7-5469535 | | | 19-50836910 | cg03251287 |
| | 11-19798914 | | | 3-169482358 | cg27020690 |
| | 1-6265894 | | | 3-10206731 | cg11029301 |
| | 4-186732926 | cg04392082 | | 3-164914575 | |
| | | | | | |

(continued)

| normal blood | Marker | CpG | |
|---|---|---|---|
| | 3-188012918 | cg02524983 | |
| | 1-43814358 | cg18856478 | |
| | 22-51136325 | cg18982286 | |
| | 12-58129855 | cg16402452 | |
| | 19-15568360 | cg08846870 | |
| | 15-22798986 | | |
| | 19-39086923 | cg05258935 | |
| | 4-159969703 | cg10393744 | |
| | 16-67687754 | cg09316954 | |
| Hypermethylated | 11-57090226 | cg11180921 | |
| | 6-31510729 | | |
| | 10-17391003 | cg05576619 | |
| | 1-3135712 | | |
| | 2-30737741 | | |
| | 1-221613658 | cg24686918 | |
| | 1-164581169 | cg20812370 | |
| | 10-63809073 | cg14789659 | |
| | 17-43318735 | | |
| | 6-12718370 | | |
| | 5-10551547 | cg24100671 | |

Table 3 illustrates cancer name and its respective abbreviation.

| Abbreviation | Name | Abbreviation | Name |
|---|---|---|---|
| LAML | Acute Myeloid Leukemia | LUSC | Lung squamous cell carcinoma |
| ACC | Adrenocortical carcinoma | DLBC | Lymphoid Neoplasm Diffuse Large B-cell Lymphoma |
| BLCA | Bladder Urothelial Carcinoma | MESO | Mesothelioma |
| LGG | Brain Lower Grade Glioma | MISC | Miscellaneous |
| BRCA | Breast invasive carcinoma | OV | Ovarian serous cystadenocarcinoma |
| CESC | Cervical squamous cell carcinoma and endocervical adenocarcinoma | PAAD | Pancreatic adenocarcinoma |
| CHOL | Cholangiocarcinoma | PCPG | Pheochromocytoma and Paraganglioma |
| LCML | Chronic Myelogenous Leukemia | PRAD | Prostate adenocarcinoma |
| COAD | Colon adenocarcinoma | READ | Rectum adenocarcinoma |
| CNTL | Controls | SARC | Sarcoma |
| ESCA | Esophageal carcinoma | SKCM | Skin Cutaneous Melanoma |
| FPPP | FFPE Pilot Phase II | STAD | Stomach adenocarcinoma |
| GBM | Glioblastoma multiforme | TGCT | Testicular Germ Cell Tumors |
| HNSC | Head and Neck squamous cell carcinoma | THYM | Thymoma |

(continued)

| Abbreviation | Name | Abbreviation | Name |
|---|---|---|---|
| KICH | Kidney Chromophobe | THCA | Thyroid carcinoma |
| KIRC | Kidney renal clear cell carcinoma | UCS | Uterine Carcinosarcoma |
| KIRP | Kidney renal papillary cell carcinoma | UCEC | Uterine Corpus Endometrial Carcinoma |
| LIHC | Liver hepatocellular carcinoma | UVM | Uveal Melanoma |
| LUAD | Lung adenocarcinoma | | |

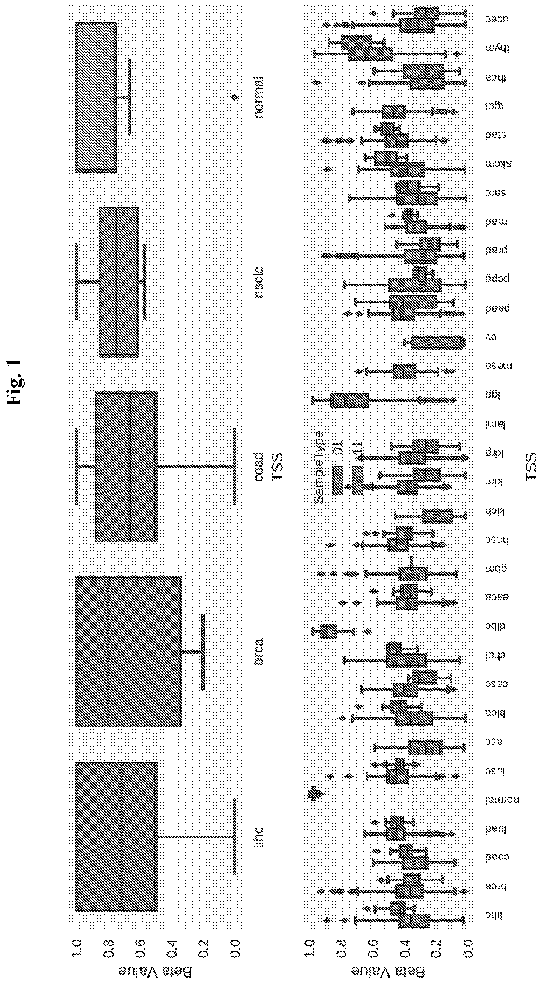

Fig. 1 illustrates the methylation status of marker 7-1577016. In some instances, marker 7-1577016 is hypomethylated. In some cases, marker 7-1577016 is used as a pan cancer marker.

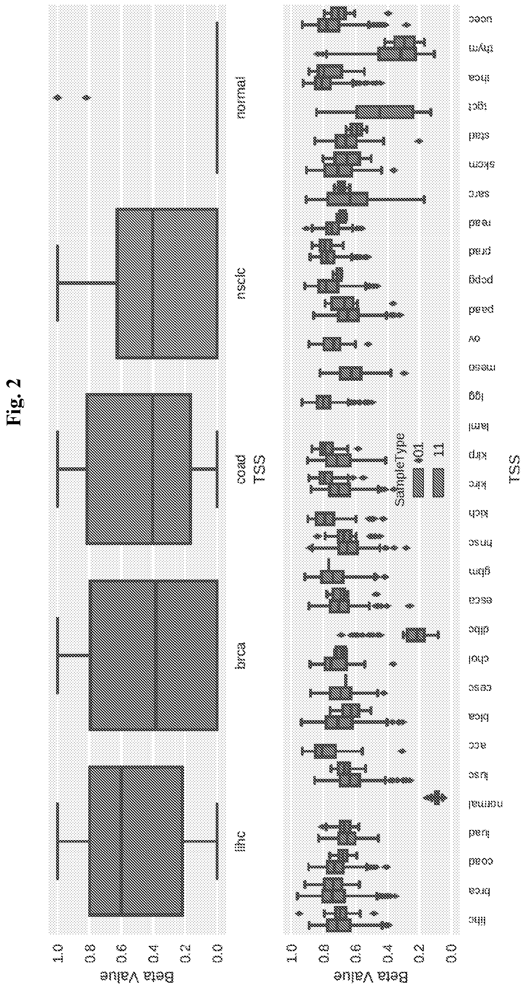

Fig. 2 illustrates the methylation status of marker 11-67177103. In some cases, marker 11-67177103 is used as a pan cancer marker.

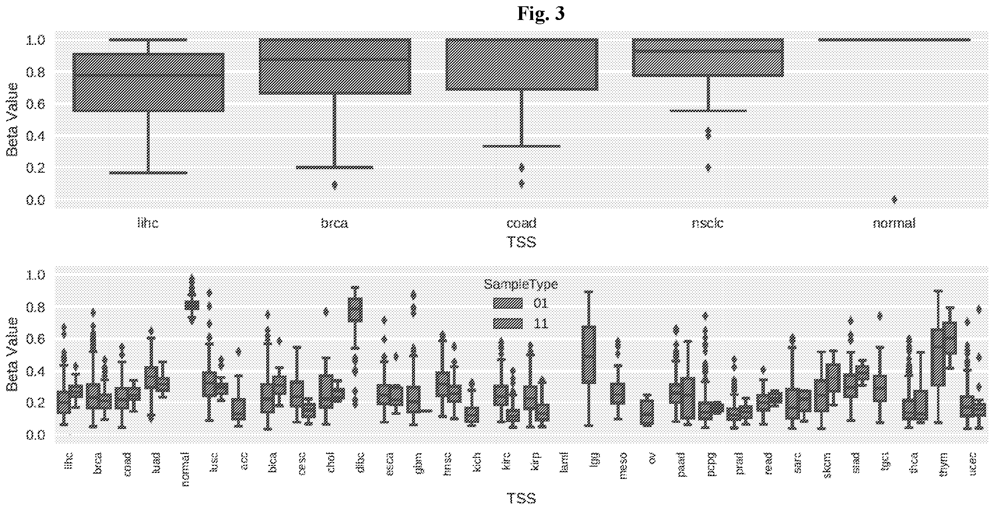

Fig. 3 illustrates the methylation status of marker 19-10445516 (cg17126555). In some cases, marker 19-10445516 (cg17126555) is used as a pan cancer marker.

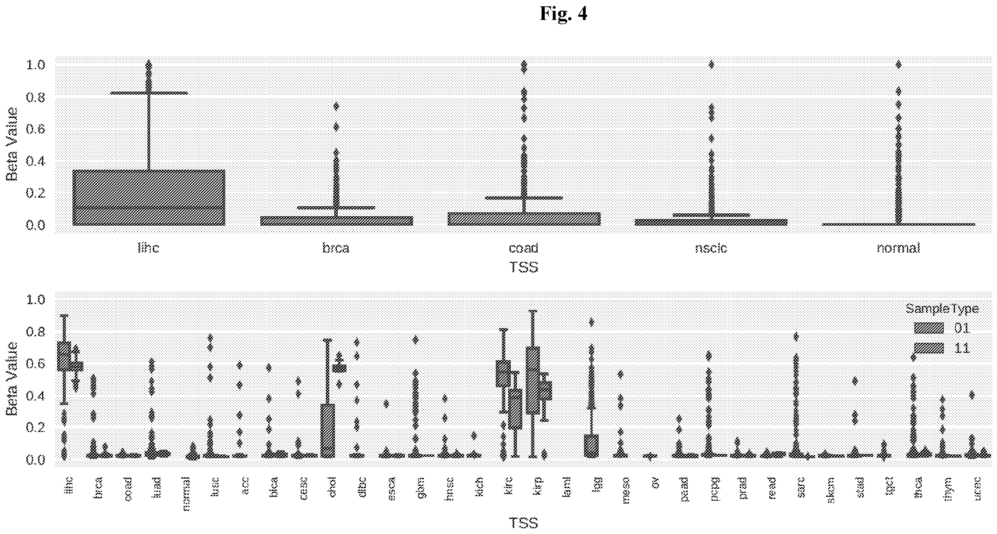

Fig. 4 illustrates the methylation status of marker 12-122277360. In some cases, marker 12-122277360 is used as a liver cancer diagnostic marker.

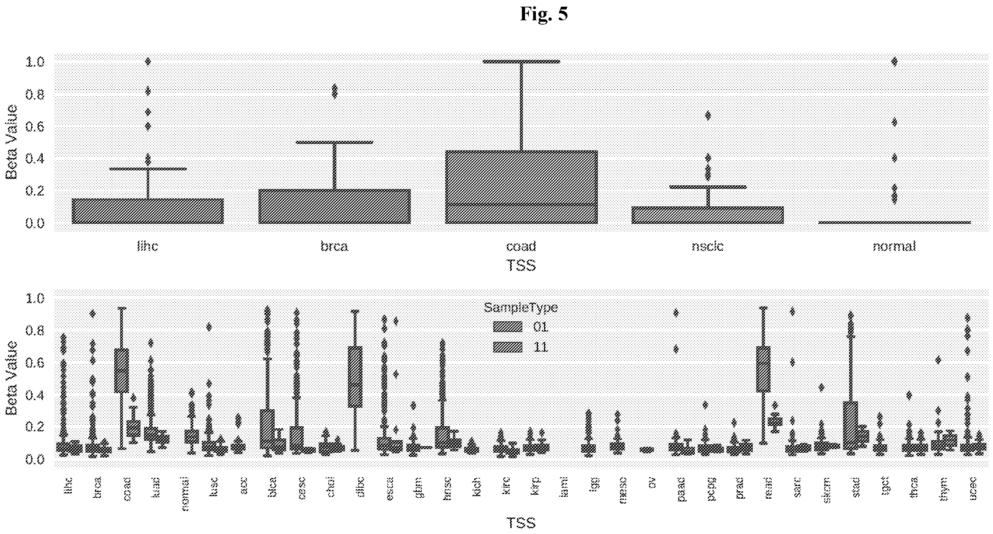

Fig. 5 illustrates the methylation status of marker 6-72130742 (cg24772267). In some cases, marker 6-72130742 (cg24772267) is used as a colon cancer diagnostic marker.

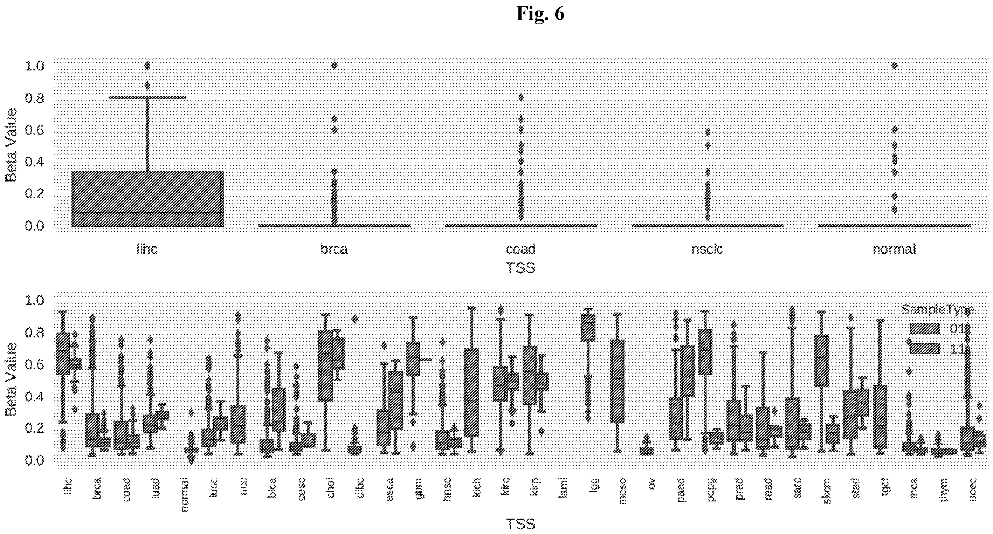

Fig. 6 illustrates the methylation status of marker 3-15369681. In some cases, marker 3-15369681 is used as a liver cancer diagnostic marker.

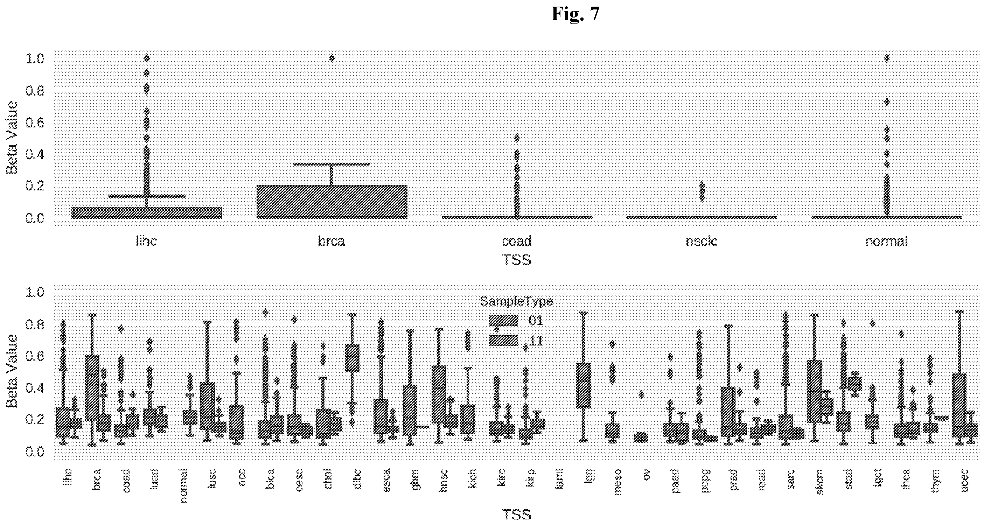

Fig. 7 illustrates the methylation status of marker 3-131081177. In some cases, marker 3-131081177 is used as a breast cancer diagnostic marker.

**EXAMPLE 2** - **Generating a reference cg marker panel**

**Data sources**

DNA methylation data from initial training set and first testing set were obtained from The Cancer Genome Atlas (TCGA).

**Generating a reference cg marker** set

Cancer type specific signature was identified by comparing the pair-wise methylation difference between a particular cancer type versus its corresponding normal tissue, the difference between two different cancer types, as well as difference between two different normal tissues, with a total of 12 tissue groups including 6 tumor groups and 6 normal tissue groups. Patient samples were randomly divided from the TCGA representing 9 cancer types from 6 different tissues with matched adjacent-normal tissue into training and validation cohorts. To do this, a total of 12 * 11 / 2 = 66 unique pair-wise comparisons were performed. Using an Illumina 450,000 CpG methylation microarray, 450k markers were compared from one group to another group using the [column t test] colttests() function in the R genefilter package. Markers with the lowest p values by t-statistic and the largest difference in a mean methylation fraction between each comparison were ranked and the top ten markers in each group were selected for further validation analysis.

**Calculate weights for top ten markers in each comparison**

The Principle Component analysis was applied to the top ten markers in each comparison group using the function in the stats environment: prcomp() and the weights in the first principle component of each group were extracted and matched with the ten corresponding markers in each group. There were 45 groupings of weights with markers. These markers were used to classify the samples with several algorithms including Neural Networks, Logistic Regression, Nearest Neighbor (NN) and Support Vector Machines (SVM), all of which generated consistent results. Analyses using SVM were found to be most robust and were therefore used in all subsequent analyses.

For each tumor type, samples were divided into two groups based on the resulting methylation signatures and their survival was plotted using Kaplan-Meier curves. Subgroups based on tumor stage and the presence of residual tumor following treatment was also analyzed. These methylation profiles were able to predict highly statistically significant differences in survival in all tumor types and most subgroups examined.

**Generate Variables**

45 variables for each of the samples in the data were generated. Using the weight/marker combination, each variable V was calculated using the following equation:

$$V = \sum_{10}^{1} (W * M)$$

where W is the weight and M is the methylation Beta-value between 0 and 1 of the corresponding marker. A matrix was generated where the dimensions are (1) the number of samples by (2) 190 variables.

**Classifying Samples**

The above mentioned matrix was used to classify the samples. There are several classification algorithms that were used here including Logistic Regression, Nearest Neighbor (NN) and Support Vector Machines (SVM). Analysis using SVM were used in all subsequent analyses.

The Kernel-Based Machine Learning Lab (kernlab) library for R was used to generate the Support Vector Machines. The best results were with the "RBF" kernel. The Crammer, Singer algorithm had slightly better results than the Weston, Watson algorithm. In the analysis, four potential types of classification errors were seen.

1. Incorrect Tissue; e.g. colon tissue is identified as lung tissue.
2. False negative; e.g. lung cancer is identified as normal lung
3. False positive; e.g. normal colon is identified as colon cancer
4. Correct tissue, incorrect cancer type; e.g. kidney renal clear cell carcinoma is identified as kidney renal papillary cell carcinoma.

Three methods were used to validate the results:

1. The samples were divided into five equal parts and 4 of the parts were used for training and the fifth part was used to test the results.
2. Leave one out scenario was used where all of the samples were used for training except one. The one left out was used for testing. This was repeated for each sample until they had all been tested.
3. Two stage replication study: The samples were divided into two sets at the beginning of the process. With the training set, 10 markers in each comparison with the highest t-test scores were identified. These markers were then used to generate principal components and then used these variables to create a SVM. The obtained markers were applied to the test set, and principal components and SVM results were generated.

**Tumor DNA extraction**

Genomic DNA extraction from pieces of freshly frozen healthy or cancer tissues was performed with QIAamp DNA Mini Kit (Qiagen) according to manufacturer's recommendations. Roughly 0.5 mg of tissue was used to obtain on average 5 $\mu$g of genomic DNA. DNA was stored at -20$^O$C and analyzed within one week of preparation.

**DNA extraction from FFPE samples**

Genomic DNA from frozen FFPE samples was extracted using QIAamp DNA FFPE Tissue Kit with several modifications. DNA was stored at -20$^O$C for further analysis.

**Bisulfite conversion of genomic DNA**

1$\mu$g of genomic DNA was converted to bis-DNA using EZ DNA Methylation-Lightning™ Kit (Zymo Research) according to the manufacturer's protocol. Resulting bis-DNA had a size distribution of ~200-3000 bp, with a peak around ~500-1000 bp. The efficiency of bisulfite conversion was >99.8% as verified by deep-sequencing of bis-DNA and analyzing the ratio of C to T conversion of CH (non-CG) dinucleotides.

**Padlock Probe Designs**

CpG markers whose methylation levels significantly differed in any of the comparison between a cancer tissue and normal tissue were used to design padlock probes for sequencing.

Padlock probes were designed using the ppDesigner software. The average length of the captured region was 70 bp, with the CpG marker located in the central portion of the captured region. To prevent bias introduced by unknown methylation status of CpG markers, capturing arms were positioned exclusively within sequences devoid of CG dinucleotides. Linker sequence between arms contained binding sequences for amplification primers separated by a variable stretch of Cs to produce probes of equal length. The average length of probes was 91 bp. Probes incorporated a 6-bp unique molecular identifier (UMI) sequence to allow for the identification of individual molecular capture events and accurate scoring of DNA methylation levels.

Probes were synthesized as separate oligonucleotides using standard commercial synthesis methods. For capture experiments, probes were mixed, in-vitro phosphorylated with T4 PNK (NEB) according to manufacturer's recommendations and purified using P-30 Micro Bio-Spin columns (Bio-Rad).

**Bis-DNA capture**

20 ng of bisulfite-converted DNA was mixed with a defined molar ratio of padlock probes in 20 $\mu$l reactions containing 1X Ampligase buffer (Epicentre). The optimal molar ratio of probes to DNA was determined experimentally to be 20,000: 1. Reactions were covered with 50$\mu$l of mineral oil to prevent evaporation. To anneal probes to DNA, 30 second denaturation at 95°C was followed by a slow cooling to 55°C at a rate of 0.02°C per second. Hybridization was left to complete for 15hrs at 55°C. To fill gaps between annealed arms, 5$\mu$l of the following mixture was added to each reaction: 2U of PfuTurboCx polymerase (pre-activated for 3 min at 95°C (Agilent)), 0.5U of Ampligase (Epicentre) and 250 pmol of each dNTP in 1X Ampligase buffer. After 5 hour incubation at 55°C, reactions were denatured for 2 minutes at 94°C and snap-cooled on ice. 5 $\mu$l of exonuclease mix (20U of Exo I and 100U of ExoIII, both from Epicentre) was added and single-stranded DNA degradation was carried out at 37°C for 2 hours, followed by enzyme inactivation for 2 minutes at 94°C.

Circular products of site specific capture were amplified by PCR with concomitant barcoding of separate samples. Amplification was carried out using primers specific to linker DNA within padlock probes, one of which contained specific 6bp barcodes. Both primers contained Illumina next-generation sequencing adaptor sequences. PCR was done as follows: 1X Phusion Flash Master Mix, 3 $\mu$l of captured DNA and 200nM final [c] of primers, using the following cycle: 10s @ 98°C, 8X of (1s @ 98°C, 5s @ 58°C, 10s @ 72°C), 25X of (1s @ 98°C, 15s @ 72°C), 60s @ 72°C. PCR reactions were mixed and the resulting library was size selected to include effective captures (~230bp) and exclude "empty" captures (~150bp) using Agencourt AMPure XP beads (Beckman Coulter). Purity of the libraries was verified by PCR using Illumina flowcell adaptor primers (P5 and P7) and the concentrations were determined using Qubit dsDNA HS assay (Thermo Fisher). Libraries were sequenced using MiSeq and HiSeq2500 systems (Illumina).

**Optimization of capture coverage uniformity**

Deep sequencing of the original pilot capture experiments showed significant differences between number of reads captured by most efficient probes and non-efficient probes (60-65% of captured regions with coverage >0.2 of average). To ameliorate this, relative efficiencies were calculated from sequencing data and probes were mixed at adjusted molar ratios. This increased capture uniformity to 85% of regions at > 0.2 of average coverage.

**Sequencing data analysis**

Mapping of sequencing reads was done using the software tool bisReadMapper (Diep, D, Nat Methods. 2012 Feb 5;9(3):270-272) with some modifications. First, UMI were extracted from each sequencing read and appended to read headers within FASTQ files using a custom script generously provided by D.D. Reads were on-the-fly converted as if all C were non-methylated and mapped to in-silico converted DNA strands of the human genome, also as if all C were non-methylated, using Bowtie2 (Langmead B, Salzberg S. Fast gapped-read alignment with Bowtie 2. Nature Methods. 2012, 9:357-359). Original reads were merged and filtered for single UMI, i.e. reads carrying the same UMI were discarded leaving a single one. Methylation frequencies were extracted for all CpG markers for which padlock probes were designed. Markers with less than 20 reads in any sample were excluded from analysis.

**EXAMPLE 3 - Generating a cg marker panel for diagnosis and prognosis of hepatocellular carcinoma from cfDNA**

**Patient data**

Tissue DNA methylation data was obtained from The Cancer Genome Atlas (TCGA). Complete clinical, molecular, and histopathological datasets are available at the TCGA website. Individual institutions that contributed samples coordinated the consent process and obtained informed written consent from each patient in accordance to their respective institutional review boards.

A second independent Chinese cohort consisted of HCC patients at the Sun Yat-sen University Cancer Center in Guangzhou, Xijing Hospital in Xi'an and the West China Hospital in Chengdu, China. Those who presented with HCC from stage I-IV were selected and enrolled in this study. Patient characteristics and tumor features are summarized in Supplementary Table 1. The TNM staging classification for HCC is according to the 7th edition of the AJCC cancer staging manual . The TNM Staging System is one of the most commonly used tumor staging systems. This system was developed and is maintained by the American Joint Committee on Cancer (AJCC) and adopted by the Union for International Cancer Control (UICC). The TNM classification system was developed as a tool for oncologists to stage different types of cancer based on certain standard criteria. The TNM Staging System is based on the extent of the tumor (T), the extent of spread to the lymph nodes (N), and the presence of metastasis (M). This project was approved by the IRBs of Sun Yat-sen University Cancer Center, Xijing Hospital, and West China Hospital. Informed consent was obtained from all patients. Tumor and normal tissues were obtained as clinically indicated for patient care and were retained for this study. Human blood samples were collected by venipuncture and plasma samples were obtained by taking supernatant after centrifugation and stored at -80°C before cfDNA extraction. The key raw data were verified and uploaded onto the Research Data Deposit public platform with an approval number RDDB2017000132.

**Tumor DNA extraction**

Genomic DNA extraction from freshly frozen healthy or cancer tissues was performed with QIAamp DNA Mini Kit (Qiagen) according to manufacturer's recommendations. Roughly 0.5 mg of tissue was used to obtain on average 5 $\mu$g of genomic DNA. DNA was stored at -20$^O$C and analyzed within one week of preparation.

**DNA extraction from FFPE samples**

Genomic DNA from frozen FFPE samples was extracted using QIAamp DNA FFPE Tissue Kit with several modifications. DNA were stored at -20°C for further analysis.

**Cell-free DNA extraction from plasma samples**

cfDNA extraction from 1.5 ml of plasma samples was performed with QIAamp cfDNA Kit (Qiagen) according to manufacturer's recommendations.

**Bisulfite conversion of genomic DNA**

About 10 ng of cf DNA was converted to bis-DNA using EZ DNA Methylation-Lightning™ Kit (Zymo Research) according to the manufacturer's protocol. Resulting bis-DNA had a size distribution of ~200-3000 bp, with a peak around ~500-1000 bp. The efficiency of bisulfite conversion was >99.8% as verified by deep-sequencing of bis-DNA and analyzing the ratio of C to T conversion of CH (non-CG) dinucleotides.

**Determination of DNA methylation levels by deep sequencing of bis-DNA captured with molecular-inversion (padlock) probes**

CpG markers whose methylation levels significantly differed in any of the comparisons between any cancer tissue and any normal tissue were used to design padlock probes for capture and sequencing of cfDNA. Padlock-capture of bis-DNA was based on the technique on published methods with modifications.

**Probe design and synthesis**

Padlock probes were designed using the ppDesigner software. The average length of the captured region was 100 bp, with the CpG marker located in the central portion of the captured region. Linker sequence between arms contained binding sequences for amplification primers separated by a variable stretch of Cs to produced probes of equal length. A 6-bp unique molecular identifier (UMI) sequence was incorporated in probe design to allow for the identification of unique individual molecular capture events and accurate scoring of DNA methylation levels.

Probes were synthesized as separate oligonucleotides using standard commercial synthesis methods (ITD). For capture experiments, probes were mixed, in-vitro phosphorylated with T4 PNK (NEB) according to manufacturer's recommendations and purified using P-30 Micro Bio-Spin columns (Bio-Rad).

**Bis-DNA capture**

About 10 ng of bisulfite-converted DNA was mixed with padlock probes in 20 µl reactions containing 1X Ampligase buffer (Epicentre). To anneal probes to DNA, 30 second denaturation at 95°C was followed by a slow cooling to 55°C at a rate of 0.02°C per second. Hybridization was left to complete for 15 hrs at 55°C. To fill gaps between annealed arms, 5µl of the following mixture was added to each reaction: 2U of PfuTurboCx polymerase (Agilent), 0.5U of Ampligase (Epicentre) and 250 pmol of each dNTP in 1X Ampligase buffer. After 5 hour incubation at 55°C, reactions were denatured for 2 minutes at 94°C. 5 µl of exonuclease mix (20U of Exo I and 100U of ExoIII, both from Epicentre) was added and single-stranded DNA degradation was carried out at 37°C for 2 hours, followed by enzyme inactivation for 2 minutes at 94°C.

Circular products of site-specific capture were amplified by PCR with concomitant barcoding of separate samples. Amplification was carried out using primers specific to linker DNA within padlock probes, one of which contained specific 6bp barcodes. Both primers contained Illumina next-generation sequencing adaptor sequences. PCR was done as follows: 1X Phusion Flash Master Mix, 3 µl of captured DNA and 200nM primers, using the following cycle: 10s @ 98°C, 8X of (1s @ 98°C, 5s @ 58°C, 10s @ 72°C), 25X of (1s @ 98°C, 15s @ 72°C), 60s @ 72°C. PCR reactions were mixed and the resulting library was size selected to include effective captures (~230bp) and exclude "empty" captures (~150bp) using Agencourt AMPure XP beads (Beckman Coulter). Purity of the libraries was verified by PCR using Illumina flowcell adaptor primers (P5 and P7) and the concentrations were determined using Qubit dsDNA HS assay (Thermo Fisher). Libraries were sequenced using MiSeq and HiSeq2500 systems (Illumina).

**Optimization of capture coverage uniformity**

Deep sequencing of the original pilot capture experiments showed significant differences between number of reads captured by most efficient probes and non-efficient probes (60-65% of captured regions with coverage >0.2x of average). To ameliorate this, relative efficiencies were calculated from sequencing data and probes were mixed at adjusted molar ratios. This increased capture uniformity to 85% of regions at > 0.2x of average coverage.

**Sequencing data analysis**

Mapping of sequencing reads was done using the software tool bisReadMapper with some modifications. First, UMI were extracted from each sequencing read and appended to read headers within FASTQ files using a custom script. Reads were on-the-fly converted as if all C were non-methylated and mapped to in-silico converted DNA strands of the human genome, also as if all C were non-methylated, using Bowtie2[28]. Original reads were merged and filtered for single UMI, i.e. reads carrying the same UMI were discarded leaving a single, unique read. Methylation frequencies were calculated for all CpG dinucleotides contained within the regions captured by padlock probes by dividing the numbers of unique reads carrying a C at the interrogated position by the total number of reads covering the interrogated position.

**Identification of Blocks of Correlated Methylation (BCM)**

Pearson correlation coefficients between methylation frequencies of each pair of CpG markers separated by no more than 200bp were calculated separately across 50 cfDNA samples from each of the two diagnostic categories, ie normal health blood and HCC. A value of Pearson's r<0.5 was used to identify transition spots (boundaries) between any two adjacent markers indicating uncorrelated methylation. Markers not separated by a boundary were combined into Blocks of Correlated Methylation (BCM). This procedure identified a total of ~1550 BCM in each diagnostic category within our padlock data, combining between 2 and 22 CpG positions in each block. Methylation frequencies for entire BCMs were calculated by summing up the numbers of Cs at all interrogated CpG positions within a BCM and dividing by the total number of C+Ts at those positions.

**DNA isolation and digital Quantitative PCR**

Tumor and corresponding plasma samples were obtained from patients undergoing surgical tumor resection; samples were frozen and preserved in at -80°C until use. Isolation of DNA and RNA from samples was performed using AllPrep DNA/RNA Mini kit and a cfDNA extraction kit, respectively (Qiagen, Valencia, CA).

**Data sources**

DNA methylation data of 485,000 sites generated using the Infinium 450K Methylation Array were obtained from the TCGA and dataset generated from our previous study (GSE40279) in which DNA methylation profiles for HCC and blood were analyzed. IDAT format files of the methylation data were generated containing the ratio values of each scanned bead. Using the minfi package from Bioconductor, these data files were converted into a score, referred to as a Beta value. Methylation values of the Chinese cohort were obtained by targeted bisulfate sequencing using a molecular inversion probe and analyzed as described below.

**Statistical Analysis --DNA methylation marker pre-selection for diagnostic and prognostic analysis**

A differential methylation analysis on TCGA data using a "moderated t-statistics shrinking" approach was first performed and the P-value for each marker was then corrected by multiple testing by the Benjamini-Hochberg procedure to control FDR at a significance level of 0.05. The list was ranked by adjusted P-value and selected the top 1000 markers for designing padlock probes. cfDNA samples with low quality or fewer than 20,000 reads per sample were also eliminated. Methylation values for each marker were defined as the proportion of read counts with methylation divided by total read counts. Methylation markers with a range of methylation values less than 0.1 in matched tumor tissue and tumor blood samples were eliminated.

**Building a cg marker panel from cfDNA**

The cfDNA dataset was randomly split into training and validation cohorts with a 2:1 ratio. Two variable selection methods suitable for high-dimensionality on the prescreened training dataset were applied: Least Absolute Shrinkage and Selection Operator (LASSO) and Random Forest based variable selection method using OOB error. As results can depend strongly on the arbitrary choice of a random sample split for sparse high-dimensional data, an analysis of the "multi-split" method were adopted, which improves variable selection consistency while controlling finite sample error. For LASSO selection operator, 75 percent of the dataset was subsampled without replacement 500 times and selected the markers with repeat occurrence frequency more than 450. The tuning parameters was determined according to the expected generalization error estimated from 10-fold cross-validation and information-based criteria AIC/BIC, and the largest value of lambda was adopted such that the error was within one standard error of the minimum, known as "1-se" lambda. For the random forest analysis, using the OOB error as a minimization criterion, variable elimination from the random forest was carried out by setting variable a dropping fraction of each iteration at 0.3. Ten overlapping methylation markers were chosen by the two methods for model building a binary prediction. A logistic regression model was fitted using these 10 markers as the covariates and obtained a combined diagnosis score (designated as cd-score) by multiplying the unbiased coefficient estimates and the marker methylation value matrix in both the training and validation datasets. The predictability of the model was evaluated by area under ROC (AUC, also known as C-index), which calculated the proportions of concordant pairs among all pairs of observations with 1.0 indicating a perfect prediction accuracy. Confusion tables were generated using an optimized cd-score cutoff with a maximum Youden's index.

The pre-treatment or initial methylation level was evaluated at baseline, and the postmethylation level was evaluated approximately 2 months after treatment, where the treatment referred to either chemotherapy or surgical resection of tumor. The primary endpoint (including response to treatment: progressive disease (PD), partial response (PR) and stable disease (SD)) were defined according to the RECIST guideline. For patients treated with surgical removal and no recurrence at time of evaluation, it was assumed that they had complete response (CR). The difference of cd-score distribution between clinical categories was examined by Wilcoxon Rank Sum test as cd-score was tested to be non-normally distributed using a Shapiro-Wilk Test.

**Patient and sample characteristics**

Clinical characteristics and molecular profiling including methylation data for comparison between HCC and blood lymphocytes were assembled from sources including 377 HCC tumor samples from The Cancer Genome Atlas (TCGA) and 754 blood leukocyte samples of healthy control individuals from a dataset on aging (GSE40279). To study ctDNA in HCC, plasma samples were obtained from Chinese patients with HCC and randomly selected healthy controls undergoing routine health care maintenance, resulting in a training cohort of 715 HCC patients and 560 normal healthy controls and a validation cohort of 383 HCC patients and 275 healthy controls. All participants provided written informed consent.

## Identification of methylation markers differentiating HCC and blood

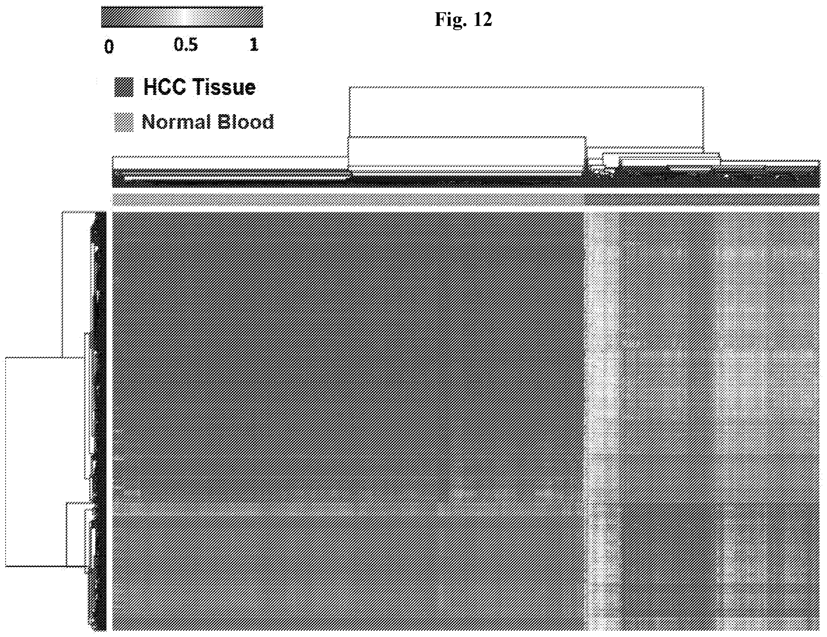

It was hypothesized that CpG markers with a maximal difference in methylation between HCC and blood leukocytes in normal individuals would be most likely to demonstrate detectable methylation differences in the cfDNA of HCC patients when compared to that of normal controls. The "moderated t-statistics" method was used with Empirical Bayes for shrinking the variance, and the Benjamini-Hochberg procedure to control the FDR at a significance level of 0.05 to identify the top 1000 markers with the most significantly different rates of methylation (i.e. those with the lowest p-values) between HCC and blood. Unsupervised hierarchical clustering of these top 1000 markers was able to distinguish between HCC and blood leukocytes in normal individuals (Fig. 12). Molecular inversion (padlock) probes was designed corresponding to these 1000 markers and tested them in 28 pairs of HCC tissue DNA and matched plasma ctDNA from the same patient. The methylation profiles in HCC tumor DNA and matched plasma ctDNA were consistent (Fig. 9A, Fig. 9B). 401 markers with a good experimental amplification profile and dynamic methylation range were selected for further analysis.

## Methylation block structure for improved allele calling accuracy

The concept of genetic linkage disequilibrium (LD block) was employed to study the degree of co-methylation among different DNA strands, with the underlying assumption that DNA sites in close proximity are more likely to be co-methylated than distant sites. A paired-end Illumina sequencing reads was used to identify each individual methylation block (mBlock). A Pearson correlation method to quantify co-methylation or mBlock was used. All common mBlocks of a region were compiled by calculating different mBlock fractions. The genome was then partitioned into blocks of tightly co-methylated CpG sites termed methylation correlated blocks (MCBs), using an $r^2$ cutoff of 0.5. MCBs was surveyed in cfDNA of 500 normal samples and found that MCBs are highly consistent. It was next determined methylation levels within an MCB in the cfDNA from 500 HCC samples. It was found that a highly consistent methylation pattern in MCBs when comparing normal versus HCC cfDNA samples, which significantly enhanced allele-calling accuracy (Fig. 13). This technique was employed in all subsequent sequencing analysis.

## cfDNA diagnostic prediction for HCC

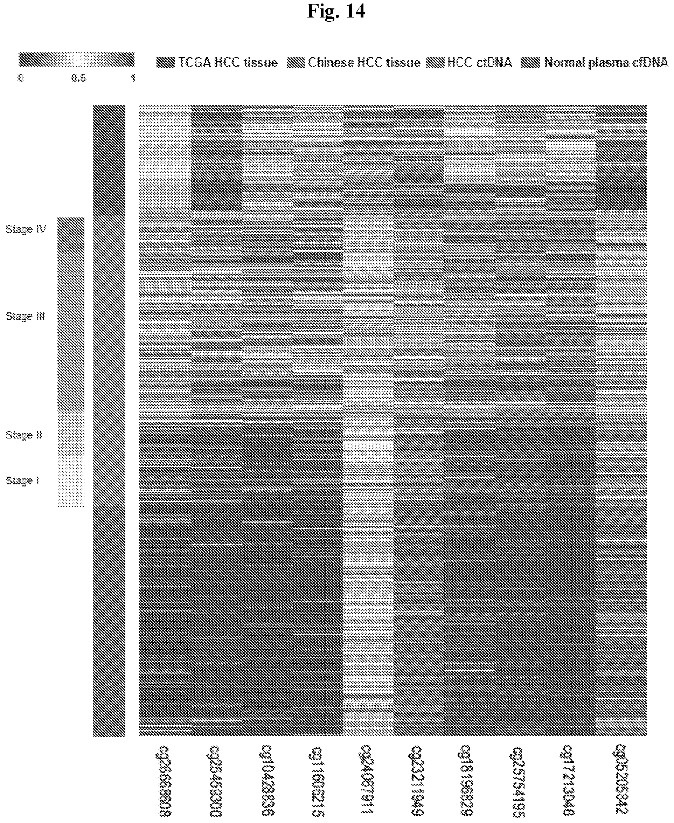

The methylation values of the 401 selected markers that showed good methylation ranges in cfDNA samples were analyzed by Random Forest and Least Absolute Shrinkage and Selection Operator (LASSO) methods to further reduce the number of markers by modeling them in 715 HCC ctDNA and 560 normal cfDNA samples (Fig. 8). 24 markers were obtained using the Random Forest analysis. 30 markers were obtained using a LASSO analysis in which selected markers were required to appear over 450 times out of a total of 500 repetitions. There were 10 overlapping markers between these two methods (Table 4). Using a logistic regression method, a diagnostic prediction model was constructed with these 10 markers. Applying the model yielded a sensitivity of 94.3% and specificity of 85.7% for HCC in the training dataset of 715 HCC and 560 normal samples (Fig. 9C) and a sensitivity of 90.5% and specificity of 83.2% in the validation dataset of 383 HCC and 275 normal samples (Fig. 9D). It was also demonstrated this model could differentiate HCC from normal controls both in the training dataset (AUC =0.966) and the validation dataset (AUC=0.944) (Fig. 9E, Fig. 9F). Unsupervised hierarchical clustering of these 10 markers was able to distinguish HCC from normal controls with high specificity and sensitivity (Fig. 9G, Fig. 10H, Fig. 14).

**Table 4**

| Markers | Ref Gene | Coefficients | SE | z value | p value |
|---------|----------|--------------|------|---------|---------|
|  |  | 15.595 | 2.395 | 6.513 | <0.001 |
| cg10428836 | BMPRIA | 11.543 | 0.885 | -13.040 | <0.001 |
| cg26668608 | PSD | 4.557 | 0.889 | 5.129 | <0.001 |
| cg25754195 | ARHGAP25 | 2.519 | 0.722 | 3.487 | <0.001 |
| cg05205842 | KLF3 | -3.612 | 0.954 | -3.785 | <0.001 |
| cg11606215 | PLAC8 | 6.865 | 1.095 | 6.271 | <0.001 |
| cg24067911 | ATXN1 | -5.439 | 0.868 | -6.265 | <0.001 |
| cg18196829 | Chr 6:170 | -9.078 | 1.355 | -6.698 | <0.001 |
| cg23211949 | Chr 6:3 | -5.209 | 1.081 | -4.819 | <0.001 |
| cg17213048 | ATAD2 | 6.660 | 1.422 | 4.683 | <0.001 |

(continued)

| Markers | Ref Gene | Coefficients | SE | z value | p value |
|---|---|---|---|---|---|
| cg25459300 | Chr 8:20 | 1.994 | 1.029 | 1.938 | 0.053 |
| **SE:** standard errors of coefficient; **z value:** Wald z-statistic value | | | | | |

It was next assessed that a combined diagnostic score (cd-score) of the model for differentiating between liver diseases (HBV/HCV infection, and fatty liver) and HCC, since these liver diseases are known major risk factors for HCC. It was found that the cd-score could differentiate HCC patients from those with liver diseases or healthy controls (Fig. 10A). These results were consistent and comparable with those predicted by AFP levels (Fig. 10B).

**Methylation markers predicted tumor load, treatment response, and staging**

It was next studied that the utility of the cd-score in assessing treatment response, the presence of residual tumor following treatment, and staging of HCC. Clinical and demographic characteristics, such as age, gender, race, and AJCC stage were included in the analysis. The cd-scores of patients with detectable residual tumor following treatment (n=828) were significantly higher than those with no detectable tumor (n=270), and both were significantly greater than normal controls (n=835) ($p<0.0001$, Fig. 10C). Similarly, cd-scores were significantly higher in patients before treatment (n=109) or with progression (n=381) compared to those with treatment response (n=248) ($p< 0.0001$, Fig. 10D). In addition, cd-scores were significantly lower in patients with complete tumor resection after surgery (n=170) compared with those before surgery (n=109), yet were higher in patients with recurrence (n=155) ($p< 0.0001$, Fig. 10E). Furthermore, there is good correlation between the cd-scores and tumor stage. Patients with early stage disease (I, II) had substantially lower cd-scores compared to those with advanced stage disease (III, IV) ($p<0.05$, Fig. 10F). Collectively, these results suggest that the cd-score (i.e., the amount of ctDNA in plasma) correlates well with tumor burden and may have utility in predicting tumor response and surveillance for recurrence.

**Utility of ctDNA diagnostic prediction and AFP**

In some instances, the blood biomarker for risk assessment and surveillance of HCC is serum AFP levels. However, its low sensitivity makes it inadequate to detect all patients that will develop HCC and severely limits its clinical utility. In fact, many cirrhotic patients develop HCC without any increase in AFP levels. Strikingly, 40% patients of the HCC study cohort have a normal serum AFP (<25 ng/ml).

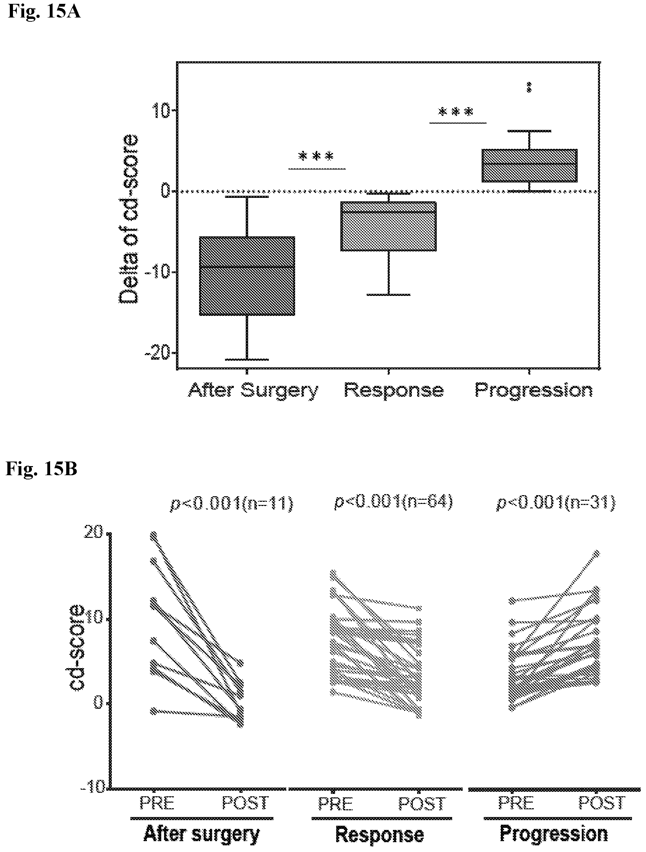

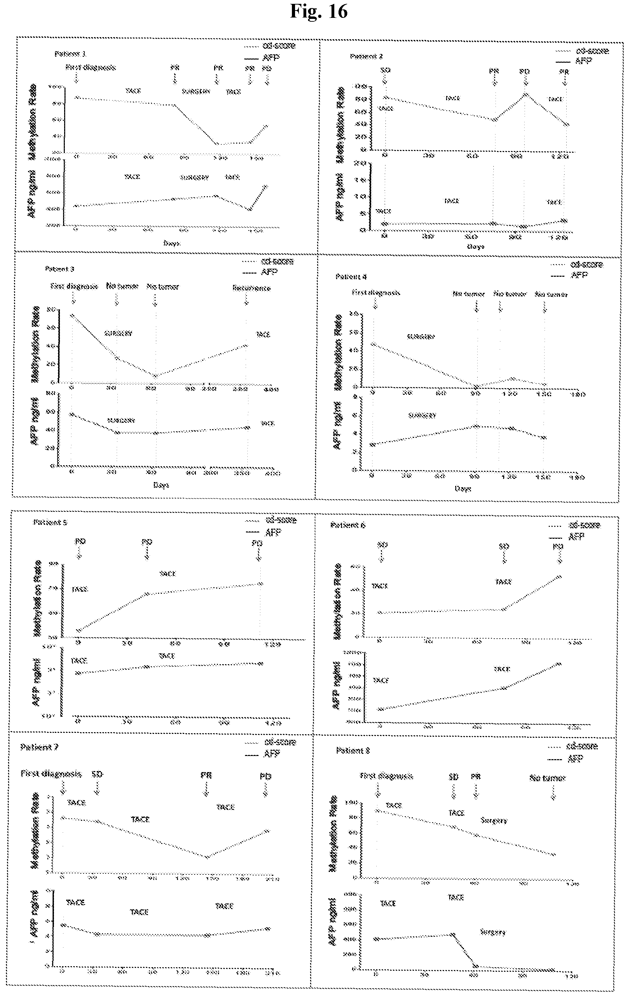

In biopsy-proven HCC patients, the cd-score demonstrated superior sensitivity and specificity than AFP for HCC diagnosis (AUC 0.969 vs 0.816, Fig. 10G). In patients with treatment response, tumor recurrence, or progression, cd-score showed more significant changes compared to testing at initial diagnosis than AFP (Fig. 10H, Fig. 10I). In patients with serial samples, those with a positive treatment response had a concomitant significant decrease in cd-score compared to that prior to treatment, and there was an even further decrease in patients after surgery. By contrast, our patients with progressive or recurrent disease all had an increase in cd-score (Fig. 15). By comparison, AFP was less sensitive for assessing treatment efficacy in individual patients (Fig. 16). In addition, while cd-score correlated well with tumor stage (Fig. 10J), particularly among patients with stage I, II and III, there was no significant difference in AFP values in patients with different stages, except between patients with stage III and IV (Fig. 10K), indicating an advantage of cd-score over AFP in differentiation of early stage HCC.

**ctDNA prognostic prediction for HCC**

It was then investigated the potential of using methylation markers in ctDNA for prediction of prognosis in HCC in combination with clinical and demographic characteristics including age, gender, race, and AJCC stage. The 1049 HCC patients were randomly split with complete survival information into training and validation datasets with an allocation of 2:1. Unicox and LASSO-cox methods were implemented to reduce the dimensionality and constructed a cox-model to predict prognosis with an 8-marker panel (Table 5). Kaplan-Meier curves was generated in training and validation datasets using a combined prognosis score (cp-score) with these markers. The high-risk group (cp-score > -0.24) had 341 observations with 53 events in training dataset and 197 observations with 26 events in validation dataset; and the low-risk group (cp-score ≤ -0.24) has 339 observations with 7 events in training dataset and 172 observations with 9 events in validation dataset. Median survival was significantly different in both the training set ($p<0.0001$) and the validation set ($p= 0.0014$) by log-rank test (Fig. 11A, Fig. 11B).

**Table 5**

| Markers | Ref Gene | Coefficients | HR | CI | SE | z value | p value |
|---|---|---|---|---|---|---|---|
| cg23461741 | SH3PXD2A | -1.264 | 0.282 | 0.024-3.340 | 1.2604 | 1.003 | 0.316 |
| cg06482904 | Cllorf9 | -0.247 | 0.781 | 0.067-9.100 | 1.2530 | 0.197 | 0.844 |
| cg25574765 | PPFIA1 | 1.026 | 2.790 | 0.488-15.900 | 0.8894 | 1.153 | 0.249 |
| cg07459019 | Chr 17:78 | -8.156 | 0.000 | 0.000-0.012 | 1.9112 | 4.267 | <0.001 |
| cg20490031 | SERPINB5 | 6.082 | 438.000 | 13.200-14600.000 | 1.7885 | 3.400 | 0.001 |
| cg01643250 | NOTCH3 | -5.368 | 0.005 | 0.000-0.140 | 1.7357 | 3.093 | 0.002 |
| cg11397370 | GRHL2 | 1.497 | 4.470 | 1.030-19.400 | 0.7506 | 1.994 | 0.046 |
| cg11825899 | TMEM8B | 2.094 | 8.120 | 0.957-68.900 | 1.0909 | 1.920 | 0.055 |

**HR:** Hazard Ratio; **CI:** 95.0% confidence interval; **SE:** standard errors of coefficients; **z value:** Wald z-statistic value

Multivariate variable analysis showed that the cp-score was significantly correlated with risk of death both in the training and validation dataset and that the cp-score was an independent risk factor of survival (hazard ratio [HR]: 2.512; 95% confidence interval [CI]: 1.966-3.210; $p$<0.001 in training set; HR: 1.553, CI: 1.240-1.944; $p$<0.001 in validation set. Interestingly, AFP was no longer significant as a risk factor when cp-score and other clinical characteristics were taken into account.

As expected, TNM stage predicted the prognosis of patients in the training and validation dataset (Fig. 11C, Fig. 11D). However, the combination of cp-score and TNM staging improved the ability to predict prognosis in both the training (AUC 0.7935, Fig. 11E) and validation datasets (AUC 0.7586, Fig. 11F). Kaplan-Meier curves also showed that patients separated by both cp-score and staging have different prognosis ($p$<0.0001, Fig. 11G). These results demonstrate that ctDNA methylation analysis may contribute to risk stratification and prediction of prognosis in patients with HCC.

In this study, differentially methylated CpG sites were first determined between HCC tumor samples and blood leukocytes in normal individuals for an HCC-specific panel. A diagnostic prediction model then constructed was using a 10-methylation marker panel (cd-score) for use in cfDNA; the cd-score effectively discriminated patients with HCC from individuals with HBV/HCV infection, cirrhosis, and fatty liver as well as healthy controls. Given that patients with these liver diseases are the target screening population under current guidelines, it is important that a serum test reliably distinguish these disease states from HCC. In the study, the sensitivity of the cd-score for HCC is comparable to liver ultrasound, the current standard for HCC screening, markedly superior to AFP, and may represent a more cost-effective and less resourceintensive approach. Furthermore, the cd-score of the model showed high correlation with HCC tumor burden, treatment response, and stage, and is superior to the performance of AFP in the instant cohort. In some cases, the cd-score is useful for assessment of treatment response and surveillance for recurrence.

**EXAMPLE 4** - **Cell-free DNA methylation markers for diagnosis and prognosis of lung cancer and hepatocellular carcinoma**

**Primary tissue patient data**

Both primary solid tissues from cancer patients and blood tissues from healthy donor were measured by Illumina 450k infimum bead chip. Primary tumor DNA methylation data of 485,000 sites was obtained from The Cancer Genome Atlas (TCGA). Complete clinical, molecular, and histopathological datasets are available at the TCGA website. Individual institutions that contributed samples coordinated the consent process and obtained informed written consent from each patient in accordance to their respective institutional review boards. Blood tissue DNA methylation data from healthy donor were obtained and generated based on study from Hannum et al., 2013, Mol Cell 49, 359-367 (GSE40279) in which DNA methylation profiles for HCC and blood were analyzed.

**Serum sample patient data**

A second independent Chinese cohort consisted of LUNC and HCC patients at the Sun Yat-sen University Cancer Center in Guangzhou, Xijing Hospital in Xi'an, and the West China Hospital in Chengdu, China. Patients who presented with LUNC and HCC from stage I-IV were selected and enrolled in this study. Patient characteristics and tumor features are summarized in Table 9. The TNM staging classification for LUNC and HCC is according to the 7th edition of the AJCC cancer staging manual. This project was approved by the IRBs of Sun Yat-sen University Cancer Center, Xijing Hospital, and West China Hospital. Informed consent was obtained from all patients. Two prospective trials on early detection of LUNC and HCC patients using methylation markers for predicting cancer occurrence in high-risk populations were conducted. In the first study, patients were recruited from a group of smokers that were undergoing CT scan-based lung cancer screening from Dec 2015 to Dec 2016. Patients presenting with lung nodules (<10mm, n=232, Table 12) were selected to undergo methylation profiling at the time of screening and were subsequently followed through secondary testing to determine whether nodules were due to cancer or inflammatory or infectious conditions by tissue biopsy and pathology diagnosis verification. In the second trial, high risk patients with liver cirrhosis were enrolled (n=242).

Tumor and normal tissues were obtained as clinically indicated for patient care and were retained for this study. Human blood samples were collected by venipuncture, and plasma samples were obtained by taking the supernatant after centrifugation and stored at -80°C before cfDNA extraction.

The pre-treatment serum samples were obtained at the initial diagnosis, and the post-treatment serum samples were evaluated approximately 2 months after treatment, where the treatment referred to either chemotherapy or surgical resection of tumor. The primary endpoint (including response to treatment: progressive disease (PD), partial response (PR) and stable disease (SD)) was defined according to the RECIST guideline. For patients treated with surgical removal and no recurrence at time of evaluation, it was assumed that they had complete response (CR).

### Extraction of cfDNA from plasma

It was determined that the minimal volume of plasma required to get consistent amounts of cfDNA for targeted sequencing. As a rough guide, it was aimed at ~20X coverage at 90% of markers covered by the padlock probe panel (see below). It was observed that 20,000 or more total unique reads per sample fulfilled this criterion. It was found that 1.5 ml or more plasma could reliably yield enough cfDNA to produce >20,000 unique reads. The relationship between amount of cfDNA in 1.5 ml plasma and detected copy numbers was further investigated using digital droplet PCR. It was found that 1.5ml of plasma yielded > 10ng, what produced at least 140 copies of detected amplicons in each digital droplet PCR assay. It was therefore settled on using 15 ng/1.5 ml as a cutoff in all of our experiments to obtain consistent and reliable measurements of DNA methylation.

cfDNA from 1.5ml of plasma was extracted using EliteHealth cfDNA extraction Kit (EliteHealth, Guangzhou Youze, China) according to manufacturer's recommendations.

### Bisulfite conversion of genomic or cfDNA

10 ng of DNA was converted to bis-DNA using EZ DNA Methylation-Lightning™ Kit (Zymo Research) according to the manufacturer's protocol. Resulting bis-DNA had a size distribution of ~200-3000 bp, with a peak around ~500-1000 bp. The efficiency of bisulfite conversion was >99.8% as verified by deep-sequencing of bis-DNA and analyzing the ratio of C to T conversion of CH (non-CG) dinucleotides.

### Marker selection for padlock probe panel design

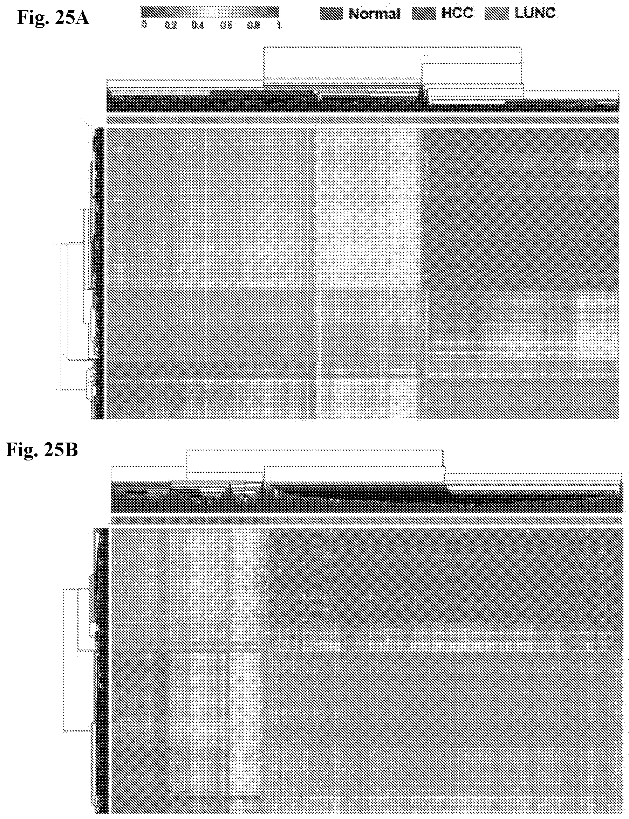

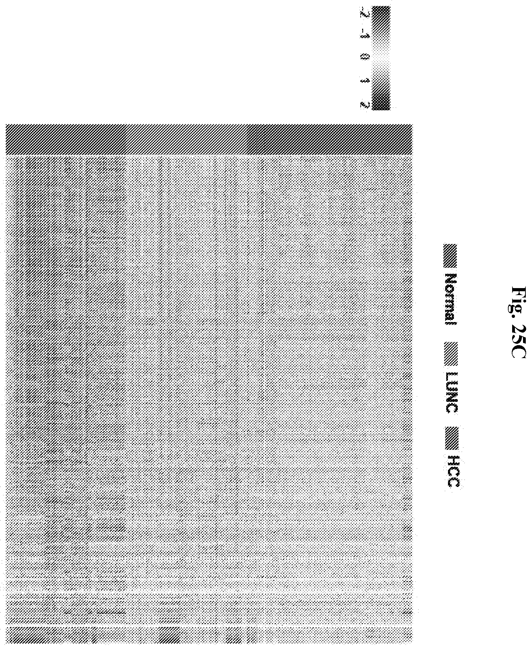

To identify markers to differentiate HCC, LUNC and normal blood methylation signatures the "moderated t-statistics shrinking" approach on 450k methylation data with Benjamini-Hochberg procedure was employed to control FDR at a significance level of 0.05 using pairwise comparisons of 377 HCC samples and 827 LUNC samples (TCGA) and 754 normal blood samples (GSE40279, our previous study (HANNUM REF)). The lists was ranked by adjusted p-value and selected the top 1000 markers for designing padlock probes for differentiating cancer (both LUNC and HCC) versus normal samples and a separate group of 1000 markers for differentiating LUNC versus HCC (Fig. 25).

All 2000 markers were used to design padlock probes for capture and sequencing of cfDNA. Padlock-capture of bis-DNA was based on the technique on methods of Deng, et al., 2009, Nature Biotechnology 27, 353-360; Diep, et al., 2012, Nature Methods 9, 270-272; and Porreca, et al., 2007, Nature Methods 4, 931-936; and with further modifications. Because of a relatively modest total size of captured regions/cg markers, this approach offers much lower cost of sequencing than any current methods including whole methylome-wide seqnencing, therefore enabling us to evaluate a large number of samples. Furthermore, the direct targeted sequencing approach offers digital readout, and requires much less starting cfDNA material (10-15 ng) than more traditional recent methods based on hybridization on a chip (eg. Infinium, Illumina) or target-enrichment by hybridization (eg. SureSelect, Agilent). This approach is also less sensitive to unequal amplification as it utilizes molecular identifiers (UMIs).

### Padlock probe design, synthesis and validation

All probes were designed using the ppDesigner software. The average length of the captured region was 70 bp, with the CpG marker located in the central 80% of the captured region. A 6bp 6-bp unique molecular identifier (UMI) flanked capture arms to aid in eliminating amplification bias in determination of DNA methylation frequencies. Linker sequence between arms contained binding sequences for amplification primers separated by a variable stretch of Cs to produce probes of equal length. Probes were synthesized as separate oligonucleotides (IDT). For capture experiments, probes were mixed in equimolar quantities and purified on Qiagen columns.

Deep sequencing of the original pilot capture experiments showed significant differences between number of reads captured by most efficient probes and non-efficient probes (60-65% of captured regions with coverage >0.2x of average). To ameliorate this, relative efficiencies were calculated from sequencing data and probes were mixed at adjusted molar ratios. This increased capture uniformity to 85% of regions at > 0.5x of average coverage.

**Targeted methylation sequencing using bis-DNA padlock probe capture**

10 ng of bisulfite-converted DNA was mixed with padlock probes in 20 $\mu$l reactions containing 1X Ampligase buffer (Epicentre). To anneal probes to DNA, 30 second denaturation at 95°C was followed by a slow cooling to 55°C at a rate of 0.02°C per second and incubation for 15 hrs at 55°C. To fill gaps between annealed arms, 5$\mu$l of the following mixture was added to each reaction: 2U of PfuTurboCx polymerase (Agilent), 0.5U of Ampligase (Epicentre) and 250 pmol of each dNTP in 1X Ampligase buffer. After 5-hour incubation at 55°C, reactions were denatured for 2 minutes at 94°C. 5 $\mu$l of exonuclease mix (20U of Exo I and 100U ExoIII of, Epicentre) was added and single-stranded DNA degradation was carried out at 37°C for 2 hours, followed by enzyme inactivation for 2 minutes at 94°C.

Circular capture products were amplified by PCR using primers specific to linker DNA within padlock probes. Both primers contained 10bp barcodes for unique dual-index multiplexing, and Illumina next-generation sequencing adaptor sequences. PCR was performed as follows: 1X Phusion Flash Master Mix, 3 $\mu$l of captured DNA and 200nM primers, using the following cycle: 10s @ 98°C, 8X of (1s @ 98°C, 5s @ 58°C, 10s @ 72°C), 25X of (1s @ 98°C, 15s @ 72°C), 60s @ 72°C. PCR reactions were mixed and the resulting library was size selected on 2.5% agarose gels to include effective captures (~230bp) and exclude "empty" captures (~150bp). Purity of the libraries was verified by TapeStation (Agilent) and PCR using Illumina flowcell adaptor primers (p5 and p7) and the concentrations were determined using Qubit dsDNA HS assay (Thermo Fisher). Libraries were sequenced on MiSeq and HiSeq2500 systems (Illumina) using PE100 reads. Median total reads for each sample was 500,000 and on-target mappability 25% (~125,000 on-target non-unique reads).

**Optimization of capture coverage uniformity**

Deep sequencing of the original pilot capture experiments showed significant differences between number of reads captured by most efficient probes and non-efficient probes (60-65% of captured regions with coverage >0.2x of average). To ameliorate this, relative efficiencies were calculated from sequencing data and probes were mixed at adjusted molar ratios. This increased capture uniformity to 85% of regions at > 0.5x of average coverage.

**Sequencing data analysis**

Mapping of sequencing reads was done using the software tool bisReadMapper with some modifications. First, UMI were extracted from each sequencing read and appended to read headers within FASTQ files using a custom script. Reads were on-the-fly converted as if all C were non-methylated and mapped to in-silico converted DNA strands of the human genome, also as if all C were non-methylated, using Bowtie2. Original reads were merged and filtered for single UMI, i.e. reads carrying the same UMI were discarded leaving a single, unique read. Methylation frequencies were calculated for all CpG dinucleotides contained within the regions captured by padlock probes by dividing the numbers of unique reads carrying a C at the interrogated position by the total number of reads covering the interrogated position.

**DNA isolation and digital Quantitative PCR**

Tumor and corresponding plasma samples were obtained from patients undergoing surgical tumor resection; samples were frozen and preserved in at -80°C until use. Isolation of DNA and RNA from samples was performed using DNA/RNA MiniPrep kit and a cfDNA extraction kit, respectively (EliteHealth, Guangzhou Youze, China). To estimate tumor cfDNA fractions, mixing experiments were performed with various fractions of normal cfDNA and HCC tumor genomic DNA (gDNA) and assayed methylation values and copy numbers by dPCR (see next section for details). Digital droplet PCR (ddPCR) was performed according to the manufacturer's specifications (Bio-Rad, Hercules, CA). The following ddPCR assay was used in this study: cg10590292 - forward primer 5'-TGTTAGTTTTTATGGAAGTTT, reverse primer 5'-AAACIAACAAAATACTCAAA; fluorescent probe for methylated allele detection 5'/6-FAM/TGGGAGAGCGGGA GAT/BHQ1/-3'; probe for unmethylated allele detection, 5'/HEX/TTTGGGAGAGTGGGAGATTT/BHQ1/-3'. ddPCR was performed according to the manufacturer's specifications (Bio-Rad, Hercules, CA). using the following cycling conditions: 1X of 10 mins @ 98°C, 40X of (30s @ 98°C, 60s @ 53°C), 1X of 10 mins @ 98°C.

### Calculation of tumor cfDNA fraction

It was assumed that a particular methylation value observed for an HCC cfDNA sample results from the combined contribution of normal and tumor cfDNA. The fraction of cfDNA originating from the tumor was estimated using the following formula: fraction contributed from tumor DNA in sample i = [methylation value in HCC cfDNA in sample i - mean methylation value of normal cfDNA] / [mean methylation value of tumor DNA - mean methylation value of normal cfDNA]. Using this approach, it was estimated that on average the tumor fraction is around 23% in HCC cfDNA samples. Samples were then grouped according to factors that evaluate tumor load, such as an advanced stage and pre-treatment status, since these factors are expected to affect the tumor fraction in ctDNA. Indeed, it was observed that conditions associated with a higher tumor staging and severity also tended to have a larger tumor fraction. To further vet this approach, a mixing experiment with different fractions of normal cfDNA (0-100%) and tumor genomic DNA (0-100%) was performed and assayed methylation values using digital PCR. It was shown that incremental addition of tumor genomic DNA can increase methylation fraction percentage up to the values observed in the HCC patient samples. Specifically, addition of 10%, 20%, 40%, 60% or 100% fraction of tumor genomic DNA can be predicted by the above formula, when using methylation values obtained from the experiment.

### Statistical Analysis

### DNA methylation marker selection for diagnostic and prognostic analysis

Out of 2000 initially designed padlock probes, only 1673 were informative, i.e able to give positive and specific PCR amplification signals, and thus were used as capture probes in the subsequent experiments in cfDNA samples. Sequencing depth was used as a sample inclusion criterion. Samples where less than 100 MCB (see below) showed 10x read coverage were excluded from further analysis. Since each MCB incorporated on average ~3 CG markers, the 10x coverage ensured at least 30 methylation measurements per MCB. Using these criteria, 73% of all samples with a median of 34K mapped reads per sample were included.

After having obtained DNA methylation data for 1673 CG markers, the concept of MCBs to merge proximal CpG markers into a MCB was used, resulting in a total of 888 MCBs. For each MCB, the MCB-specific methylation value was quantified with two numbers: log10 (total methylated read count +1) and log10 (total unmethylated read count +1), using the log transform to reduce outlier effects.

About 1673 informative padlock probes were obtained that were able to give positive and specific PCR amplification signals and they were used as capture probes in the subsequent experiments in cfDNA samples. cfDNA samples with less than 100 MCB of >30x coverage were also eliminated. Methylated reads for each marker were defined as total unique methylated reads and methylation values for each marker were defined as the proportion of read counts with methylation divided by total read counts.

### cfDNA-based diagnostic classifier construction using MCBs (cd-score)

cfDNA sample data obtained from patients diagnosed with liver cancer (HCC), lung cancer (LUNC) and normal controls were divided into training and validation cohorts. The full dataset was randomly split with a 1:1 ratio to form the training and validation cohorts.

Marker selections: Within the training cohort, the "randomized lasso" scheme was adopted to reduce the sampling dependency and stabilize variable selection in order to select biomarkers with high confidence. The training set was first randomly divided with 1:1 ratio. The variable selection procedure on two thirds of the samples was conducted and withheld a third of the samples for evaluating performance of the feature selection process. The feature selection process consisted of two steps repeated 50 times. MCBs were included for training the final model if they were selected in 40 out of 50 feature selection iteration. A multi-class prediction system based on Friedman et al., 2010, J Stat Softw 33, 1-22 was constructed to predict the group membership of samples in the test data using the panel of MCBs selected. A confusion matrix and ROC curves were also provided to evaluate sensitivity and specificity, in addition to prediction accuracy based on the held out partition of the training set.

Classification process: a two-step classification process was employed: cancer vs normal, LUNC vs HCC by building two binary multinomial logistic regression models. The multinomial logistic regression has the advantage where it can yield an intuitive probability score and allow for easier interpretation. For example, if the cancer-vs-normal model yield a probability score of 70% for a given methylation profile, it suggests that the patient has a 70% chance of having cancer. In order to minimize the number of false cancer predictions, the cancer prediction confidence threshold was set to 80%. For patients with at least 80% chance of cancer, the cancer-vs-cancer regression model was applied for classifying between LUNC and HCC, the classification model would decide only if the classified sample has a confidence of over 55%.

## Building a predictive model for prognosis and survival

The potential to use a combined prognosis score (cp-score) system based on both methylation reads and non-methylated reads was investigated for each MCB in cfDNA for prediction of prognosis in LUNC and HCC in combination with clinical and demographic characteristics including age, gender, and AJCC stage. For each type of cancer, a cp-score model was build and validate it by randomly selecting half of the observations from the full dataset as the training cohort, and treated the rest as the validation cohort. Variable selection on the training cohort was conducted and built the composite score on the validation cohort. Within the training cohort, the "randomized lasso" scheme was adopted to reduce the sampling dependency to stabilize the variable selection in order to select biomarkers with a high confidence. The entire cohort was randomly divided with a 1:1 ratio. The variable selection procedure was conducted on two-thirds of the training cohort. LASSO was implemented with an optimal tuning parameter determined by either the expected generalization error from the 10-fold cross validation or the information based criteria AIC / BIC, whichever yielded the highest (the proportion of explained randomness) with the selected biomarkers. The 10 most recurring features from HCC and in LUNC (Table 8) was then aggregated. To evaluate the predictability of each panel externally, a composite score was obtained for each patient in the validation cohort by multiplying the unbiased coefficient estimates from the Cox regression and the methylation reads. A Kaplan-Meier curve and log-rank test were generated using the dichotomized composite score, which formed a high-risk and low-risk group membership assignment according to its median. This segmentation was compatible with that formed by AJCC stage. Time-dependent ROC was used to summarize the discrimination potential of the composite score, AJCC stage and the combination of two, with ROC curves varying as a function of time and accommodating censored data. Finally, a multivariate Cox regression model was also fitted to assess the significance of potential risk factors.

All the analysis was conducted in R (version 3.2.3) and python (version 2.7.13) with the following packages used: 'glnmet', 'limma','survival', 'sklearn', 'lifeline' ,'survival ROC', 'survcomp',

All hypothesis testing was done by two-sided with p-value < 0.05 considered to be statistically significant unless specifically stated otherwise.

## Patient and sample characteristics

Clinical characteristics and molecular DNA methylation profiles were collected for 827 LUNC and 377 HCC tumor samples from The Cancer Genome Atlas (TCGA) and 754 normal samples from a dataset used in our previous methylation study on aging (GSE40279) (Hannum et al., 2013). Two cohorts of patients were studied. The first cohort was from solid tumor samples from TCGA and the second cohort was from plasma samples from China. To study cfDNA in LUNC and HCC, plasma samples were obtained from 2,396 Chinese patients with HCC or LUNC, and from randomly selected, population-matched healthy controls undergoing routine health care maintenance, resulting in a cohort of 892 LUNC and 1504 HCC patients and 2247 normal healthy controls. Informed written consent was obtained from each study participant. Clinical characteristics of all patients and controls are listed in Table 9.

## Identification of methylation markers differentiating LUNC and HCC and blood

Previous reports indicate that plasma contains DNA released from tissues within the body. It was hypothesized that because cfDNA originating from tumor cells can be detected in a background of cfDNA predominantly released from leukocytes, CpG markers with a maximal difference in methylation values between LUNC or HCC versus normal leukocytes would be most likely to demonstrate detectable methylation differences in the cfDNA of HCC or LUNC patients when compared to that of normal controls. To identify putative markers, methylation data derived from cancer tissue DNA from the TCGA and normal blood including 827 LUNC, 377 HCC, and 754 blood samples from healthy controls were compared. In order to identify DNA sites with significantly different rates of methylation between LUNC or HCC and normal blood, a t-statistic with Empirical Bayes was used for shrinking the variance and selected the top 1000 significant markers, using the Benjamini-Hochberg procedure to control the FDR at a significance level of 0.05. Unsupervised hierarchical clustering of these top 1000 markers was able to distinguish between LUNC, HCC, and normal blood, and between LUNC and HCC (Fig. 25). About 2,000 molecular inversion (padlock) probes corresponding to these 2000 markers for capture-sequencing cfDNA from plasma (1000 for cancer versus normal and 1000 for LUNC versus HCC) were then designed.

## cfDNA diagnostic prediction model for LUNC and HCC

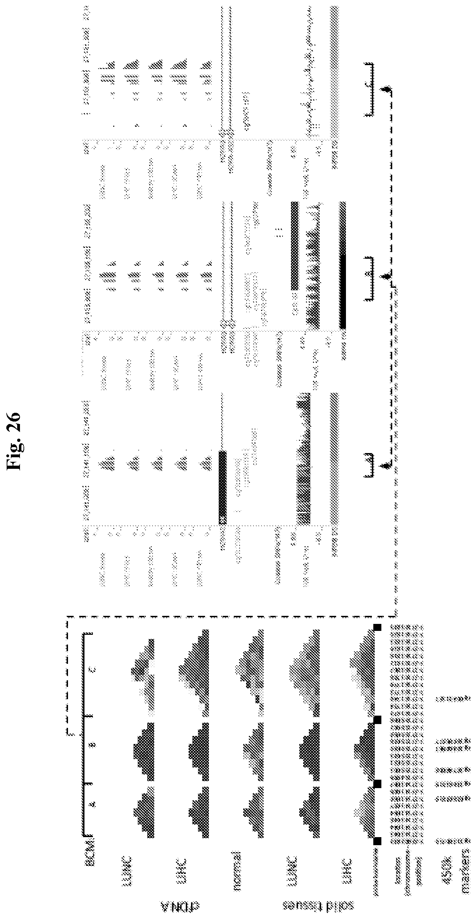

The methylation data of the 888 selected Methylation Correlated Blocks (MCB) that showed good methylation ranges in cfDNA samples were further analyzed to identify MCBs that showed significantly different methylation between cancer samples (LUNC and HCC) versus normal control samples. Unsupervised hierarchical clustering of these selected MCBs using methylated reads across samples is shown in Fig. 25C, and distributions of MCB methylated read values for normal, LUNC and HCC samples is shown in Fig. 26. The entire methylation dataset of 888 MCBs was therefore analyzed by Least Absolute Shrinkage and Selection Operator (LASSO) method and further reduced the number of MCBs. LASSO-based feature selection identified 28 MCBs for discriminating LUNC versus HCC and normal, 27 MCBs for discriminating of HCC versus LUNC and normal, 22 MCBs for discriminating of normal vs HCC and LUNC, resulting in 77 unique markers (5 MCBs overlap between models). This approach combined the information captured by the MCBs into a composite cfDNA-based score (composite diagnostic score: cd-score). The utility of this score was evaluated for predicting the presence of LUNC or HCC using a hold-out strategy where samples were randomly assigned to a training set and a validation set with a 1:1 ratio. The scoring system was trained using 229 LUNC, 444 HCC and 1123 normal control cfDNA samples and then validated on 300 LUNC, 445 HCC and 1124 normal samples. Applying the fitted model to the validation set samples yielded a sensitivity of 92.4% for HCC and 85.8% for LUNC, and a specificity of 99.0% for normal controls in a multi-classification scheme (Table 6A). It was found that this model could successfully differentiate LUNC and HCC samples from normal controls in the validation cohort (AUC cancer vs normal=0.979; AUC LUNC vs HCC=0.924; Fig. 19A, Table 6B, Table 6C). Unsupervised hierarchical clustering of the 77 MCBs was able to distinguish HCC and LUNC from normal controls with high specificity and sensitivity (Fig. 19C and Fig. 19D).

Liver diseases, such acirrhosis, and fatty liver, are major risk factors for HCC. Thus, the cd-score of the model was assessed for differentiating between liver diseases and. It was found that the cd-score was able to differentiate HCC patients from those with liver diseases or healthy controls (Fig. 20A). These results were consistent and comparable with those predicted by AFP levels in HCC (Fig. 20B). The cd-score could also differentiate between LUNC patients and non-LUNC patients with a smoking history (> 1 pack/day for ten years) who were at an increased risk of LUNC (Fig. 20C). These results were consistent and comparable with those predicted by AFP levels in HCC (Fig. 20D).

**Methylation profiles predicted tumor burden, treatment response and staging**

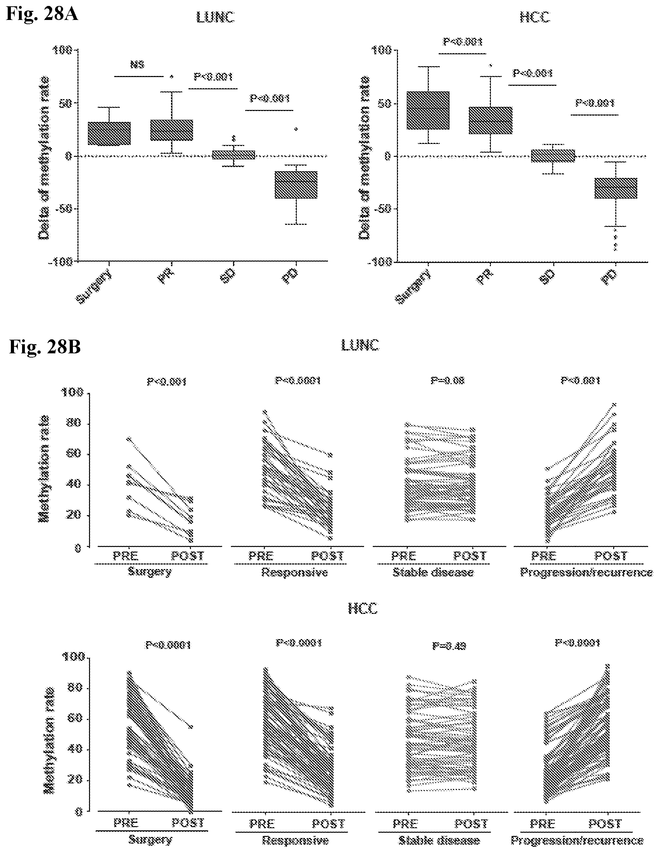

Next, the utility of the cd-score was studied in assessing treatment response, the presence of residual tumor following treatment, and staging of LUNC and HCC. In LUNC, the cd-scores of patients with detectable residual tumor following treatment (n=559) were significantly higher than those with no detectable tumor (n=160) ($p$<0.001, Fig. 21A). Similarly, there was good correlation between the cd-scores and tumor stage. Patients with early stage disease (I, II) had substantially lower cd-scores compared to those with advanced stage disease (III, IV) ($p$=<0.005, Fig. 21B). In addition, the cd-scores were significantly lower in patients with complete tumor resection after surgery (n=158) compared with those before surgery (n=67), yet became higher in patients with recurrence (n=56) ($p$< 0.01, Fig. 21C). Furthermore, the cd-scores were significantly higher in patients before treatment (n=67) or with progression (n=136) compared to those with a positive treatment response (n=328) (p< 0.001, Fig. 21D). In HCC, The cd-scores of patients with detectable residual tumor following treatment (n=889) were significantly higher than those with no detectable tumor (n=314) ($p$<0.0001, Fig. 21E). Similarly, there was a highly positive correlation between cd-scores and tumor stage. Patients with early stage disease (I, II) had substantially lower cd-scores compared to those with advanced stage disease (III, IV) ($p$<0.001, Fig. 21F). In addition, the cd-scores were significantly lower in patients with complete tumor resection after surgery (n=293) compared with those before intervention (n=109), yet became higher in patients with recurrence (n=155) ($p$<0.01, Fig. 21G). Furthermore, the cd-scores were significantly higher in patients before treatment (n=109) or with progression (n=381) compared to those with treatment response (n=249) (p< 0.001, Fig. 21H). Serial longitudinal dynamic changes were obtained of methylation values of CpG site cg10673833 in several individuals with LUNC or HCC patient in order to monitor treatment response and found there was a high correlation between methylation values and treatment outcomes (Fig. 28, Fig. 29 and Fig. 30). Collectively, the results showed the significant correlation between he cd-score (i.e., the amount of cfDNA in plasma) and tumor burden, demonstrating its utility for the prediction of tumor response and for surveillance to detect recurrence.

**Diagnostic utility of cfDNA as compared with AFP and CEA**

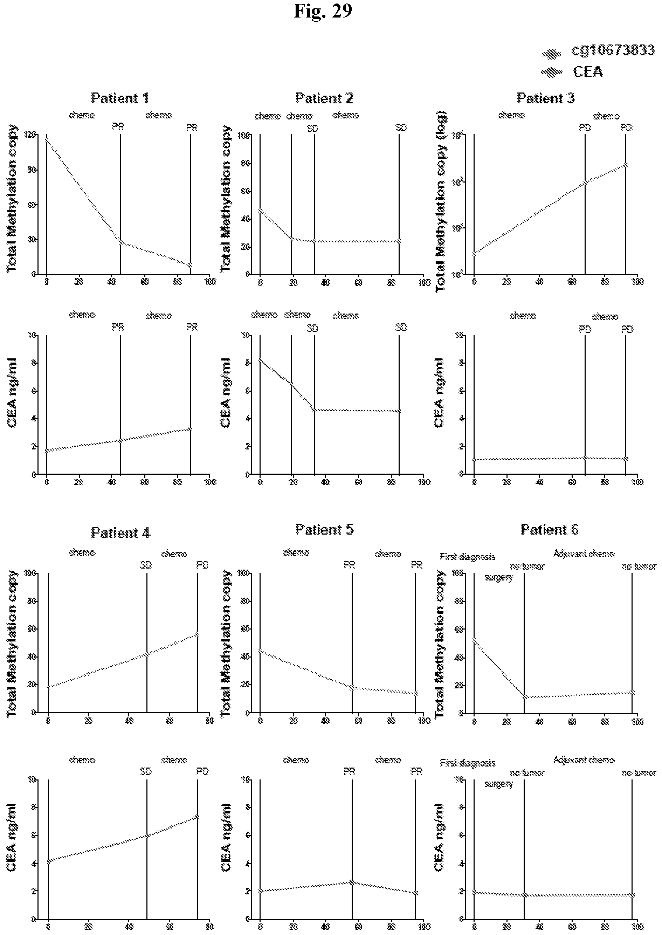

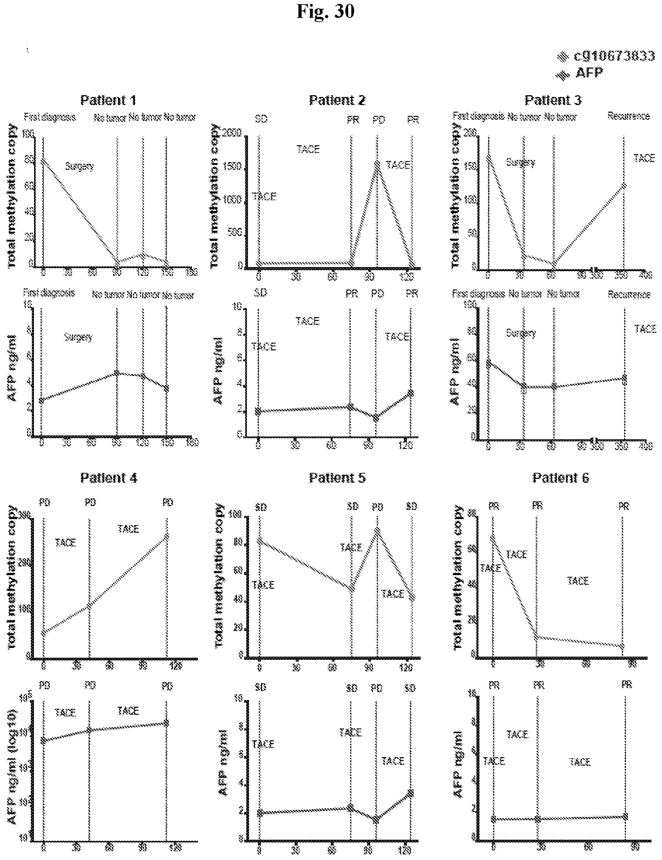

Despite enormous efforts, an effective noninvasive blood-based biomarker for surveillance and diagnosis of LUNC and HCC is still lacking. CEA (cancer embryonic antigen) and AFP have filled this role for lung cancer and HCC for decades, but its sensitivity and specificity are inadequate. Moreover, some patients with squamous cell carcinoma or small cell lung cancer will not have increased blood CEA levels. AFP has low sensitivity of 60%, making it inadequate for detection of all patients that will develop HCC and thus severely limiting its clinical utility. In fact, it is common for cirrhotic patients with HCC to show no increase in AFP levels. Strikingly, 30% patients of the HCC study cohort have a normal AFP value (<25 ng/ml). In biopsy-proven LUNC patients of the entire cohort, the cd-score demonstrated superior sensitivity and specificity to CEA for LUNC diagnosis (AUC 0.977 (cd-score) vs 0.856 (CEA), Fig. 21Q). Both cd-score and CEA values were highly correlated with tumor stage (Fig. 21J, Fig. 21B). On other hand, the cd-score demonstrated superior sensitivity and specificity to AFP for HCC diagnosis (AUC 0.993 vs 0.835, Figure 4R) in biopsy-proven HCC patients. Both cd-score and AFP values were highly correlated with tumor stage (Fig. 21F and Fig. 21N). In patients with treatment response, tumor recurrence, or progression, the cd-score showed more changes from initial diagnosis than that of AFP (Fig. 21G and Fig. 21H, Fig. 21O and Fig. 21P). In LUNC patients with treatment response, tumor recurrence, or progression, the cd-score showed more significant changes from initial diagnosis than that of CEA (Fig. 21C and Fig. 21D, Fig. 21K and Fig. 21L). In LUNC and HCC patients with serial samples, there was a concomitant and significant decrease in cd-score in patients with a positive treatment response than in patients prior to treatment. There was an even further reduction in cd-score in patients after surgery. In contrast, there was an increase in methylation rate in patients with progressive or recurrent disease (Fig. 28). By comparison, CEA and AFP were less sensitive for assessing treatment efficacy in individual patients (Fig. 29 and Fig. 30).

**cfDNA prognostic model for HCC and LUNC**

The potential of using a combined prognosis score (cp-score) based on cfDNA methylation analysis for prediction of prognosis in LUNC and HCC in combination with clinical and demographic characteristics including age, gender, AJCC stage, and AFP value was investigated. Totally 599 LUNC patients and 867 HCC patients enrolled in prognosis analysis (patients without tumor burden are excluded from the analysis). The median follow up time was 9.5 months (rang 0.6-26 months) in LUNC cohort and 6.7 months (rang 1.2-21.0months) in HCC cohort. In the HCC cohort, the training dataset contained 433 observations with 41 events and the validation dataset contained 434 observations with 58 events. By using statistical learning methods, a predictive model was constructed using 10 CpG MCBs (Table 8) that can separate the HCC cohort into high and low risk groups, with median survival significantly greater in the low-risk group than in the high-risk group (log-rank test = 24.323, df=1, $p<0.001$) (Fig. 22A). In the LUNC cohort, the training dataset contained 299 observations with 61 events and the validation dataset contained 434 observations with 58 events. A panel of 10 CpG markers (Table 8) was able to divide the LUNC cohort into high and low risk groups, with median survival significantly greater in the low-risk group than in the high-risk group (log-rank test= 6.697, df=1, $p<0.001$) (Fig. 22B).

Multivariate Cox regression model showed that the cp-score was significantly correlated with incidence of mortality in both HCC and LUNC. The cp-score was an independent risk factor of survival both in HCC and borderline in LUNC validation cohorts (harzard ratio=2.4881, p=0.000721 in HCC; hazard ratio=1.74, p=0.068 in LUNC; $p$=0.0017 in LUNC; Table 10). Interestingly, when cp-score and other clinical characteristics were taken into account in HCC, AFP was no longer significant as a risk factor (Table 11). As expected, TNM stage (as defined by AJCC guidelines) predicted the prognosis of patients both in HCC (Fig. 22C) and LUNC (Fig. 22D). The combination of cp-score and TNM staging improved our ability to predict prognosis in both HCC (AUC 0.867, Fig. 22E) and LUNC cohort (AUC 0.825, Fig. 22F).

**Methylation markers in early diagnosis of LUNC and HCC**

Since LUNC and HCC are very aggressive cancers with poor prognosis and survival, and surgical removal of cancer at stage 1 carries a much more favorable prognosis, early detection becomes a key strategy in reducing morbidity and mortality. The method of using methylation markers for predicting cancer occurrence in high-risk populations was investigated in two prospective studies. In the first study, consecutive patients were recruited from a group of patients with solid lung nodules >10mm identified on chest CT scans. These patients were enrolled in a study for early lung detection in smokers and underwent CT scan-based lung cancer screening. Patients presenting with a solid lung nodule (between 10mm and 30mm in size, n=208, Table 11) were selected to undergo methylation profiling at the time of screening. These patients were subsequently followed through secondary testing to determine whether nodules were due to LUNC or a benign condition due to inflammation or infection by tissue biopsy and pathology verification. The methylation profile was sufficient to differentiate patients with biopsy-proven stage 1 LUNC lesions compared to patients with benign nodules due to inflammatory or infectious conditions (Fig. 23, Table 12). Among the patients with at least 59% confidence for diagnosis, Positive predictive value (PPV) of stage I cancer was 95.9% and negative predictive value (NPV) was 97.4 %. Similarly, high risk HCC patients with liver cirrhosis (n=236, Table 11) were prospectively enrolled. The methylation profile was able to predict progression to stage 1 HCC with 89.5% sensitivity and 98.2% specificity (Fig. 24, Table 12) among the patients with at least 58% confidence for diagnosis. PPV was 80.9% and NPV was 99.1%.

In this study, differentially methylated CpG sites were first determined in LUNC and HCC tumor samples versus normal blood. Then, these markers were interrogated in the cfDNA of a large cohort of LUNC and HCC patients as well normal controls. A diagnostic model (cd-score) was developed using methylation of cfDNA to predict the presence of cancer, while at the same time differentiating between LUNC and HCC.

The cd-score discriminated patients with HCC from individuals with HBV/HCV infection, cirrhosis, and fatty liver disease as well as healthy controls. In some instances, it is important that a serum test reliably distinguish these disease states from HCC. According to the results, the sensitivity of the cd-score for HCC is comparable to liver ultrasound, the current standard for HCC screening. In addition, in some instances it is superior to AFP, the only clinically used biomarker for HCC, making cd-score a more cost-effective and less resourceintensive approach. Furthermore, by showing its high correlation with HCC tumor burden, treatment response, and stage, the cd-score of the model demonstrated superior performance than AFP in the instant cohort (AFP values were within a normal range for 40% of our HCC patients during the entire course of their disease). In some cases, the cd-score may be particularly useful for assessment of treatment response and surveillance for recurrence in HCC. Since nearly all of the HCC patients had hepatitis (most likely hepatitis B) in the study, HCC arising from other etiologies may have different cfDNA methylation patterns. Similar to HCC, screening for lung cancer has a high cost, involving CT imaging of the chest, which has an associated radiation exposure and a high false-positive rate. In some cases, the cd-score reliably distinguished smokers and patients with lung cancer and may also have utility in improving screening and surveillance.

Prognostic prediction models were also constructed for HCC and LUNC from the cp-score. The cp-score effectively distinguished HCC and LUNC patients with different prognosis and was validated as an independent prognostic risk factor in a multi-variable analysis in our cohorts. Of note, for predicting prognosis in HCC, cfDNA analysis was again superior to AFP. In some cases, this type of analysis is helpful for identification of patients for whom more or less aggressive treatment and surveillance is needed.

Table 6A. Contingency table of multi-classification diagnosis in validation cohort

| Prediction | HCC | LUNC | Normal |
|---|---|---|---|
| HCC | 329 | 19 | 6 |
| LUNC | 23 | 145 | 6 |
| Normal | 4 | 5 | 921 |
| Undecided | 89 | 131 | 191 |
| Totals | 354 | 174 | 930 |
| Correct | 329 | 145 | 921 |
| Sensitivity (%) | 92.4 | 85.8 | |
| Specificity (%) | | | 99.0 |

Table 6B. Contingency table of binary classification diagnosis between HCC and normal

| Prediction | HCC | Normal |
|---|---|---|
| cancer | 371 | 16 |
| normal | 4 | 921 |
| undecided | 70 | 187 |
| Totals | 375 | 937 |
| Correct | 371 | 921 |
| Sensitivity (%) | 98.9 | |
| Specificity (%) | | 98.3 |

Table 6C. Contingency table of binary classification diagnosis between LUNC and normal

| Prediction | LUNC | Normal |
|---|---|---|
| cancer | 188 | 16 |
| normal | 5 | 921 |
| undecided | 107 | 187 |
| Totals | 193 | 937 |
| Correct | 188 | 921 |
| Sensitivity (%) | 97.4 | |
| Specificity (%) | | 98.3 |

Table 7. List of MCBs selected by multi-class LASSO and used for cd-score

| generation. A, normal; B, LUNC; C, HCC. | | | | | | |
|---|---|---|---|---|---|---|
| **A, Normal** | | | | | | |
| **Diagnosis** | **MCBs** | **Read counts** | **Target ID** | **RefGene** | **Logistic regression coefficients** | |
| | | | | | **LUNC vs HCC** | **cancer vs normal** |
| **Normal** | 1-1693966 | mc | cg00100121 | C1orf114 | 0.058289236 | -0.460201686 |
| | | non_mc | cg00100121 | C1orf114 | -0.264294546 | -0.362527983 |
| | 11-474165 | mc | cg13912307 | SLC39A13 | -0.019821436 | -0.342748675 |
| | | non_mc | cg13912307 | SLC39A13 | -0.119495297 | -0.097830945 |
| | 11-791245 | mc | cg08794954 | ODZ4 | -0.502459754 | -0.119001342 |
| | | non_mc | cg08794954 | ODZ4 | -0.050537884 | -0.237868292 |
| | 12-561356 | mc | cg00344358 | GDF11 | 0.013443025 | -0.152292508 |
| | | non_mc | cg00344358 | GDF11 | 0.210631385 | -0.24781796 |
| | 16-7128 | mc | cg05773599 | WDR90 | -0.220759943 | -0.438656833 |
| | | non_mc | cg05773599 | WDR90 | 0.123092944 | -0.14690629 |
| | 16-856199 | mc | cg10174683 | - | 0.05314766 | 0.335012824 |
| | | non_mc | cg10174683 | - | -0.182838798 | -0.279797616 |
| | 17-579157 | mc | cg12054453 | TMEM49 | 0.062499156 | -0.478242327 |
| | | non_mc | cg12054453 | TMEM49 | 0.549358501 | 0.925660526 |
| | 17-742484 | mc | cg24166450 | - | 0.240682421 | 0.067853997 |
| | | non_mc | cg24166450 | - | 0.443449771 | 0.566346654 |
| | 2-1139315 | mc | cg09366118 | PSD4 | 0.098868936 | 0.269196214 |
| | | non_mc | cg09366118 | PSD4 | 0.039975437 | -0.005683265 |
| | 2-293390 | mc | cg21972382 | CLIP4 | -0.099097051 | -0.61331161 |
| | | non_mc | cg21972382 | CLIP4 | 0.074745373 | 0.2650865 |
| | 22-321497 | mc | cg03550506 | DEPDC5 | -0.068645581 | -0.755902786 |
| | | non_mc | cg03550506 | DEPDC5 | -0.046222783 | -0.124629981 |
| | 3-1138223 | mc | cg06722069 | - | -0.427422216 | -0.26910555 |
| | | non_mc | cg06722069 | - | 0.089047399 | -0.071525276 |
| | 4-13248 | mc | cg07748255 | MAEA | 0.217866978 | -0.457591409 |
| | | non_mc | cg07748255 | MAEA | -0.011474644 | -0.340582424 |
| | 5-1489298 | mc | cg22928002 | CSNK1A1 | -0.19633718 | -0.006367323 |
| | | non_mc | cg22928002 | CSNK1A1 | -0.131881054 | 0.459975236 |
| | 5-429520 | mc | cg17757602 | - | 0.041309712 | 0.029262464 |
| | | non_mc | cg17757602 | - | -0.133713183 | -0.355503949 |
| | 5-429521 | mc | cg17757602 | - | 0.127773182 | 0.169659022 |
| | | non_mc | cg17757602 | - | -0.3026458 | -0.526377272 |
| | 6-276491 | mc | cg03161803 | - | -0.134554024 | 0.040255772 |
| | | non_mc | cg03161803 | - | -0.076945207 | -0.517353281 |

(continued)

| generation. A, normal; B, LUNC; C, HCC. | | | | | | |
|---|---|---|---|---|---|---|
| **A, Normal** | | | | | | |
| **Diagnosis** | **MCBs** | **Read counts** | **Target ID** | **RefGene** | **Logistic regression coefficients** | |
| | | | | | **LUNC vs HCC** | **cancer vs normal** |
| | 6-912971 | mc | cg01087382 | MAP3K7 | 0.132083258 | -0.263202543 |
| | | non_mc | cg01087382 | MAP3K7 | 0.023651284 | 0.314783378 |
| | 7-1017626 | mc | cg06721601 | CUX1 | 0.305704307 | 0.347231675 |
| | | non_mc | cg06721601 | CUX1 | -0.061678409 | 0.340362292 |
| | 7-1577443 | mc | cg27104173 | PTPRN2 | 0.029132702 | -0.207025939 |
| | | non_mc | cg27104173 | PTPRN2 | 0.004420766 | -0.098341178 |
| | 9-885141 | mc | cg13740515 | - | 0.028390415 | -0.269846773 |
| | | non_mc | cg13740515 | - | -0.208939054 | -0.193859618 |
| | X-27307 | mc | cg13176022 | XG | 0.511857283 | 0.042177783 |
| | | non_mc | cg13176022 | XG | 0.048888339 | -0.232292001 |

**B: LUNC**

| Diagnosis | MCBs | Read counts | Target ID | RefGene | Logistic regression coefficients | |
|---|---|---|---|---|---|---|
| | | | | | LUNC vs HCC | cancer vs normal |
| LUNC | 1-1693966 | mc | cg00100121 | C1orf114 | 0.058289236 | -0.460201686 |
| | | non_mc | cg00100121 | C1orf114 | -0.264294546 | -0.362527983 |
| | 11-1169673 | mc | cg16858415 | SIK3 | 0.374145093 | 0.446746019 |
| | | non_mc | cg16858415 | SIK3 | 0.297361321 | 0.42281836 |
| | 11-476248 | mc | cg05585544 | - | 0.570677409 | 0.329004001 |
| | | non_mc | cg05585544 | - | 0.127193074 | -0.357434967 |
| | 11-646423 | mc | cg18518074 | EHD1 | 0.305179647 | 0.273679057 |
| | | non_mc | cg18518074 | EHD1 | -0.170377122 | -0.416439448 |
| | 11-779080 | mc | cg03423942 | USP35 | 0.049737225 | 0.05832888 |
| | | non_mc | cg03423942 | USP35 | -0.152714594 | 0.278866832 |
| | 11-791245 | mc | cg08794954 | ODZ4 | -0.502459754 | -0.119001342 |
| | | non_mc | cg08794954 | ODZ4 | -0.050537884 | -0.237868292 |
| | 12-687588 | mc | cg20323175 | - | 0.148739937 | 0.171957693 |
| | | non_mc | cg20323175 | - | -0.249738102 | 0.397951832 |
| | 12-72767 | mc | cg16959747 | RBP5 | 0.09713964 | -0.079430598 |
| | | non_mc | cg16959747 | RBP5 | 0.055654584 | 0.2763291 |
| | 13-256210 | mc | cg25366582 | - | 0.168798263 | -0.174946818 |
| | | non_mc | cg25366582 | - | 0.031754087 | 0.303827389 |
| | 16-571473 | mc | cg06880930 | CPNE2 | 0.235333764 | 0.168616245 |
| | | non_mc | cg06880930 | CPNE2 | -0.072680275 | -0.290080025 |
| | 17-48361 | mc | cg25526759 | GP1BA | -0.01244657 | 0.090902282 |
| | | non_mc | cg25526759 | GP1BA | 0.14744695 | 0.344718909 |
| | 17-579157 | mc | cg12054453 | TMEM49 | 0.062499156 | -0.478242327 |
| | | non_mc | cg12054453 | TMEM49 | 0.549358501 | 0.925660526 |
| | 17-742484 | mc | cg24166450 | - | 0.240682421 | 0.067853997 |
| | | non_mc | cg24166450 | - | 0.443449771 | 0.566346654 |
| | 19-460568 | mc | cg27391679 | OPA3 | 0.044319948 | 0.082373402 |
| | | non_mc | cg27391679 | OPA3 | -0.243746298 | -0.096052603 |
| | 2-293390 | mc | cg21972382 | CLIP4 | -0.099097051 | -0.61331161 |
| | | non_mc | cg21972382 | CLIP4 | 0.074745373 | 0.2650865 |
| | 2-382010 | mc | cg20626840 | FAM82A1 | 0.237250003 | 0.741677389 |

(continued)

| Diagnosis | MCBs | Read counts | Target ID | RefGene | Logistic regression coefficients | |
|---|---|---|---|---|---|---|
| | | | | | LUNC vs HCC | cancer vs normal |
| | | non_mc | cg20626840 | FAM82A1 | 0.272198397 | 0.04877136 |
| | 2-95267 | mc | cg15545942 | ASAP2 | -0.557516889 | -0.185093352 |
| | | non_mc | cg15545942 | ASAP2 | -0.11738118 | -0.171014921 |
| | 3-1138223 | mc | cg06722069 | - | -0.427422216 | -0.26910555 |
| | | non_mc | cg06722069 | - | 0.089047399 | -0.071525276 |
| | 5-1767847 | mc | cg04466840 | RGS14 | -0.206022964 | -0.032074773 |
| | | non_mc | cg04466840 | RGS14 | -0.029935092 | 0.110871368 |
| | 5-429520 | mc | cg17757602 | - | 0.041309712 | 0.029262464 |
| | | non_mc | cg17757602 | - | -0.133713183 | -0.355503949 |
| | 6-1574304 | mc | cg17475813 | ARIDIB | 0.129475454 | 0.045380298 |
| | | non_mc | cg17475813 | ARID1B | -0.398696708 | -0.14398793 |
| | 6-283040 | mc | cg08343881 | ZNF323 | 0.002401379 | 0.00651448 |
| | | non_mc | cg08343881 | ZNF323 | 0.255186684 | 0.345026825 |
| | 6-352655 | mc | cg02919168 | DEF6 | -0.372390965 | -0.054727228 |
| | | non_mc | cg02919168 | DEF6 | 0.070785826 | -0.098822441 |
| | 6-912971 | mc | cg01087382 | MAP3K7 | 0.132083258 | -0.263202543 |
| | | non_mc | cg01087382 | MAP3K7 | 0.023651284 | 0.314783378 |
| | 7-1017626 | mc | cg06721601 | CUX1 | 0.305704307 | 0.347231675 |
| | | non_mc | cg06721601 | CUX1 | -0.061678409 | 0.340362292 |
| | 7-1228399 | mc | cg22024657 | SLC13A1 | 0.013826947 | 0.222664259 |
| | | non_mc | cg22024657 | SLC13A1 | -0.056854833 | 0.037841165 |
| | 8-1025044 | mc | cg18004756 | GRHL2 | -0.293288901 | 0.034342458 |
| | | non_mc | cg18004756 | GRHL2 | -0.043777572 | -0.203017346 |
| | 8-1444164 | mc | cg12188860 | TOP1MT | 0.094522888 | -0.083700307 |
| | | non_mc | cg12188860 | TOP1MT | -0.083425117 | -0.026557506 |

**C: HCC**

| Diagnosis | MCBs | Read Counts | Target ID | RefGene | Logistic regression coefficients | |
|---|---|---|---|---|---|---|
| | | | | | LUNC vs | Cancer vs |
| HCC | 1-1695560 | mc | cg16054275 | F5 | -0.214291458 | -0.606905543 |
| | | non_mc | cg16054275 | F5 | 0.524710881 | -0.648261504 |
| | 1-2035950 | mc | cg06637618 | ATP2B4 | -0.198233007 | -0.368854771 |
| | | non_mc | cg06637618 | ATP2B4 | 0.023774113 | 0.157886336 |
| | 1-2130901 | mc | cg04607844 | - | -0.293894301 | -0.012727778 |
| | | non_mc | cg04607844 | - | 0.119395918 | 0.246150856 |
| | 10-80958 | mc | cg18187680 | FLJ45983 | -0.028534778 | 0.369256194 |
| | | non_mc | cg18187680 | FLJ45983 | -0.130812947 | -0.247877838 |
| | 12-1222773 | mc | cg26386472 | HPD | -0.472436655 | 0.005424793 |

(continued)

| Diagnosis | MCBs | Read Counts | Target ID | RefGene | Logistic regression coefficients | |
|---|---|---|---|---|---|---|
| | | | | | LUNC vs | Cancer vs |
| | | non_mc | cg26386472 | HPD | 0.131065837 | -0.084249928 |
| | 15-555695 | mc | cg02712036 | RAB27A | -0.178526212 | 0.136059681 |
| | | non_mc | cg02712036 | RAB27A | -0.136916211 | 0.13904572 |
| | 16-724595 | mc | cg07864976 | - | 0.159138061 | 0.220936294 |
| | | non_mc | cg07864976 | - | -0.174358026 | 0.041182013 |
| | 17-579157 | mc | cg12054453 | TMEM49 | 0.062499156 | -0.478242327 |
| | | non_mc | cg12054453 | TMEM49 | 0.549358501 | 0.925660526 |
| | 17-800195 | mc | cg20651080 | DUSIL | 0.16564247 | 0.179098878 |
| | | non_mc | cg20651080 | DUSIL | -0.072821844 | 0.405200855 |
| | 17-803588 | mc | cg11252953 | C17orf101 | 0.009872935 | 0.197906181 |
| | | non_mc | cg11252953 | C17orf101 | -0.088619833 | -0.047902639 |
| | 19-459097 | mc | cg06663668 | CD3EAP | 0.026258194 | 0.077247979 |
| | | non_mc | cg06663668 | CD3EAP | -0.209537326 | 0.55413999 |
| | 19-546460 | mc | cg11441617 | CNOT3 | -0.272150647 | 0.31583956 |
| | | non_mc | cg11441617 | CNOT3 | -0.485831577 | 0.011388271 |
| | 19-546461 | mc | cg11441617 | CNOT3 | 0.345456601 | 0.601027916 |
| | | non_mc | cg11441617 | CNOT3 | 0.252775143 | 0.151478291 |
| | 2-1139315 | mc | cg09366118 | PSD4 | 0.098868936 | 0.269196214 |
| | | non_mc | cg09366118 | PSD4 | 0.039975437 | -0.005683265 |
| | 21-364214 | mc | cg01519261 | RUNX1 | 0.350276252 | 0.086780025 |
| | | non_mc | cg01519261 | RUNX1 | 0.205448988 | -0.230292808 |
| | 21-364215 | mc | cg01519261 | RUNX1 | -0.068910744 | 0.175787941 |
| | | non_mc | cg01519261 | RUNX1 | -0.470630509 | -0.686309747 |
| | 22-185277 | mc | cg02415779 | - | 0.056697461 | 0.313717112 |
| | | non_mc | cg02415779 | - | 0.122239689 | -0.243806777 |
| | 22-378130 | mc | cg00107982 | ELFN2 | -0.03021978 | 0.143360514 |
| | | non_mc | cg00107982 | ELFN2 | -0.063348947 | 0.490504707 |
| | 4-13248 | mc | cg07748255 | MAEA | 0.217866978 | -0.457591409 |
| | | non_mc | cg07748255 | MAEA | -0.011474644 | -0.340582424 |
| | 4-840359 | mc | cg19255783 | PLAC8 | 0.024851345 | 0.211399591 |
| | | non_mc | cg19255783 | PLAC8 | -0.154998544 | -0.217029094 |
| | 5-1715385 | mc | cg25650256 | STK10 | -0.296078256 | 0.069437129 |
| | | non_mc | cg25650256 | STK10 | -0.134013643 | 0.178025221 |
| | 6-262504 | mc | cg05414338 | HIST1H3F | -0.291906307 | 0.385669376 |
| | | non_mc | cg05414338 | HIST1H3F | -0.188285503 | 0.279553121 |
| | 6-315278 | mc | cg06393830 | NFKBIL 1 | 0.120351939 | 0.122094253 |
| | | non_mc | cg06393830 | NFKBIL1 | 0.206309569 | -0.082505936 |
| | 6-329093 | mc | cg00862588 | HLA-DMB | -0.322857861 | 0.005678509 |

(continued)

| Diagnosis | MCBs | Read Counts | Target ID | RefGene | Logistic regression coefficients | |
|---|---|---|---|---|---|---|
| | | | | | LUNC vs | Cancer vs |
| | | non_mc | cg00862588 | HLA-DMB | 0.382920486 | -0.054366936 |
| | 6-912971 | mc | cg01087382 | MAP3K7 | 0.132083258 | -0.263202543 |
| | | non_mc | cg01087382 | MAP3K7 | 0.023651284 | 0.314783378 |
| | 7-1000913 | mc | cg03113878 | C7orf51 | -0.173651065 | -0.291203774 |
| | | non_mc | cg03113878 | C7orf51 | -0.009576582 | 0.090427416 |
| | 7-759325 | mc | cg21217886 | HSPB1 | -0.135144273 | -0.558395663 |
| | | non_mc | cg21217886 | HSPB1 | -0.163997602 | -0.133445148 |

Table 8. Characteristics of 10 MCBs in LUNC prognosis prediction and 10 MCBs in HCC prognosis prediction

| | Features | Target ID | RefGene |
|---|---|---|---|
| LUNC | mc_17-742484 | cg24166450 | - |
| | non_mc_2-741532 | cg02478828 | DGUOK |
| | mc_2-2355288 | cg08436738 | - |
| | mc_1-295863 | cg04933208 | PTPRU |
| | non_mc_22-358223 | cg20146967 | MCM5 |
| | non_mc_16-900927 | cg07860918 | GAS8 |
| | mc_20-374337 | cg16119522 | PPP1R16B |
| | non_mc_3-1960650 | cg05556202 | TM4SF19 |
| | mc_12-687588 | cg20323175 | - |
| | non_mc_10-299484 | cg13324103 | SVIL |
| HCC | mc_6-262503 | cg05414338 | HIST1H3F |
| | mc_6-733300 | cg17126142 | KCNQ5 |
| | non_mc_7-450187 | cg06787669 | MYOIG |
| | mc_19-185898 | cg06747543 | ELL |
| | mc_12-1222773 | cg26386472 | HPD |
| | non_mc_1-20665 | cg00866690 | PRKCZ |
| | mc_12-939663 | cg11225410 | SOCS2 |
| | mc_7-450187 | cg06787669 | MYOIG |
| | mc_6-283040 | cg08343881 | ZNF323 |
| | mc_6-733299 | cg17126142 | KCNQ5 |

Table 9. Clinical characteristics of study cohort

| Characteristic | TCGA HCC tissue | TCGA LUNC tissue | GSE Normal | HCC blood | LUNC blood | Normal blood |
|---|---|---|---|---|---|---|
| Total (n) | 377 | 827 | 754 | 1504 | 892 | 2247 |
| **Gender** | | | | | | |
| Female-no. (%) | 122(32.4) | 340(41.1) | 401(53.2) | 146(9.7) | 263(29.5) | 507(22.6) |
| Male-no. (%) | 255(67.6) | 487(58.9) | 353(46.8) | 991(65.9) | 487(54.6) | 480(21.4) |
| NA | 0 | 0 | 0 | 367(24.4) | 142(15.9) | 1260(56.1) |

(continued)

| Age (years) | | | | | | |
|---|---|---|---|---|---|---|
| Mean | 61 | 68 | 63 | 54 | 58 | 48 |
| Range | 16-90 | 33-90 | 19-101 | 11- 85 | 19-85 | 19-90 |
| **Pathology** | | | | | | |
| Hepatocellular | 367(97.3) | 0 | NA | 1504(100) | 0 | NA |
| Adenocarcinoma(%) | 0 | 458(55.4) | NA | 0 | 402 (45.1) | NA |
| Squamous cell | 0 | 369(44.6) | NA | 0 | 138(15.5) | NA |
| Small Cell Lung | 0 | 0 | NA | 0 | 79(8.9) | NA |
| Others(%) | 10(2.7) | 0 | NA | 0 | 273(30.6) | NA |
| **Stage** | | | | | | |
| I (%) | 175(46.4) | 424(51.3) | NA | 206(13.7) | 58(6.5) | NA |
| II (%) | 87(23.1) | 115(13.9) | NA | 202(13.4) | 52(5.8) | NA |
| III (%) | 86(22.8) | 261(31.6) | NA | 612(40.7) | 148(16.6) | NA |
| IV (%) | 6(1.6) | 25(3.0) | NA | 134(8.9) | 463(51.9) | NA |
| NA (%) | 23(6.1) | 2(0.2) | NA | 350(23.3) | 171(19.2) | NA |
| **Tumor burden** | | | | | | |
| Tumor free (%) | 236(62.6) | 503(60.8) | NA | 314(20.9) | 160(17.9) | NA |
| With tumor (%) | 114(30.2) | 159(19.2) | NA | 889(59.1) | 599(67.2) | NA |
| NA (%) | 27(7.2) | 165(20.0) | NA | 301(20.0) | 133(14.9) | NA |
| **EGFR status** | | | | | | |
| Wide type (%) | NA | 400(48.4) | NA | NA | 102(11.4) | NA |
| Mutation (%) | NA | 100(12.1) | NA | NA | 69(7.7) | NA |
| NA (%) | NA | 327(39.5) | NA | NA | 721(80.8) | NA |
| **Hepatitis** | | | | | | |
| Positive (%) | 120(31.8) | NA | NA | 623(95.3) | NA | 379(16.9) |
| Negative (%) | 119(31.6) | NA | NA | 10(1.5) | NA | 571(25.4) |
| NA (%) | 138(36.6) | NA | NA | 21(3.2) | NA | 1297(57.7) |
| **Smoking (%)** | | | | | | |
| Current smoker (%) | NA | 725(87.7) | NA | NA | NA | 192(8.5) |
| Non-smoker (%) | NA | 80(9.7) | NA | NA | NA | 670(29.8) |
| NA (%) | NA | 22(2.6) | NA | NA | NA | 1385(61.6) |

Table 10. Multivariate survival analysis for HCC patients and LUNC patients with composite-score of methylation markers (cp-score) and relevant variables in validation cohorts

| HCC | | | | | | | |
|---|---|---|---|---|---|---|---|
| | coef | exp(coef) | se(coef) | z | p | lower 0.95 | upper 0.95 |
| cp-score | 0.9115 | 2.4881 | 0.2696 | 3.3813 | 0.0007 | 0.3830 | 1.4400 |
| stage | 0.3336 | 1.3960 | 0.3034 | 1.0995 | 0.2715 | -0.2612 | 0.9284 |
| AFP | -0.0861 | 0.9175 | 0.2115 | -0.4069 | 0.6841 | -0.5008 | 0.3286 |
| age | -330.8028 | 0.0000 | 75.7050 | -4.3696 | 0.0000 | -479.2145 | -182.3911 |
| Gender | -0.0885 | 0.9153 | 0.2361 | -0.3748 | 0.7078 | -0.5512 | 0.3743 |
| LUNC | | | | | | | |
| | coef | exp(coef) | se(coef) | z | p | lower 0.95 | upper 0.95 |
| cp-score | 0.5577 | 1.7467 | 0.3056 | 1.8249 | 0.0680 | -0.0414 | 1.1569 |
| stage | 0.4647 | 1.5916 | 0.8422 | 0.5518 | 0.5811 | -1.1863 | 2.1157 |

(continued)

| LUNC | | | | | | | |
|---|---|---|---|---|---|---|---|
| | coef | exp(coef) | se(coef) | z | p | lower 0.95 | upper 0.95 |
| CEA | 0.2695 | 1.3093 | 0.6035 | 0.4466 | 0.6552 | -0.9135 | 1.4525 |
| age | -321.0835 | 0.0000 | 94.6796 | -3.3913 | 0.0007 | -506.6928 | -135.4742 |
| Gender | 0.0897 | 1.0938 | 0.3858 | 0.2325 | 0.8162 | -0.6666 | 0.8460 |

Table 11. Clinical characteristics and sensitivity/specificity for detection of stage I LUNC and benign lung nodules

| Sensitivity and Specificity for the Detection of Lung Cancer | | |
|---|---|---|
| Characteristic | Stage I lung cancer N=116 | Benign nodules N=116 |
| Age-yr | | |
| Median | 61 | 52 |
| Range | 29-83 | 26-86 |
| Gender | | |
| Female | 80 | 53 |
| male | 36 | 63 |
| Pathology | | |
| Adenocarcinoma | 100 | NA |
| Squamous cell carcinoma | 6 | NA |
| Small Cell Lung Cancer | 0 | NA |
| Others | 0 | NA |
| Nodule size-mm | | |
| Mean | 14.6±6.1 | 7.2±2.1 |
| Median | 15.0 | 6.4 |
| Range | 3-58 | 2.7-18.2 |
| Stage | | |
| AIS | 7 | NA |
| MIA | 8 | NA |
| IA | 78 | NA |
| IB | 13 | NA |
| Sensitivity - % (95% CI) | 77.8 | |
| Specificity - % (95% CI) | 85.3 | |
| Positive predictive value - % (95% CI) | 81.0 | |
| Negative predictive value - % (95% CI) | 84.1 | |

Table 12. Clinical characteristics and sensitivity/specificity for detection of progression to stage I HCC from liver cirrhosis

| Sensitivity and Specificity for the Detection of Liver Cancer | | |
|---|---|---|
| Characteristic | Stage I liver cancer N=204 | Cirrhosis N=242 |
| Age-yr | | |
| Median | 62 | 52 |

(continued)

| Sensitivity and Specificity for the Detection of Liver Cancer | | | |
|---|---|---|---|
| Characteristic | | Stage I liver cancer N=204 | Cirrhosis N=242 |
| Age-yr | | | |
| | Range | 21-81 | 25-82 |
| Gender | | | |
| | Female | 32 | 47 |
| | Male | 172 | 195 |
| Hepatitis B | | | |
| | Positive | 198 | 193 |
| | Negative | 4 | 13 |
| | NA | 2 | 36 |
| AFP | | | |
| | <25ng/ml | 113 | 196 |
| | >25 | 43 | 29 |
| | NA | 48 | 17 |
| Sensitivity - % (95% CI) | 94.9 | | |
| Specificity - % (95% CI) | 92.6 | | |
| Positive predictive value - % (95% CI) | 94.9 | | |
| Negative predictive value - % (95% CI) | 92.8 | | |

**EXAMPLE** 5 - **Circulating Tumor DNA Methylation Profiles for Diagnosis and Prognosis of Colorectal Cancer**

Colorectal cancer (CRC) is one of the most common cancers in the world. In some instances, detection of CRC at early and/or intermediate stage provides a better prognosis than detection at an advanced stage. Serum Carcinoembryonic antigen (CEA) quantification is a noninvasive tool for the detection of cancer. However, in some instances, the CEA test has a low sensitivity for the detection of CRC.

Circulating tumor DNA (ctDNA) is tumor-derived fragmented DNA in the circulatory system, comes, e.g., from dead tumor cells through necrosis and apoptosis. In this study, DNA methylation status of genes obtained from ctDNA samples are determined and are further utilized for detection of CRC.

**Patient data**

Tissue DNA methylation data was obtained from The Cancer Genome Atlas (TCGA). Complete clinical, molecular, and histopathological datasets are available at the TCGA website. Whole blood DNA methylation profiles from healthy donors were generated in an aging study (GSE40279) in which DNA methylation profiles for CRC and blood were analyzed. Individual institutions that contributed samples coordinated the consent process and obtained informed written consent from each patient in accordance to their respective institutional review boards.

The cfDNA cohort consisted of 801 CRC patients and 1021 normal control from the Sun Yat-sen University Cancer Center in Guangzhou, Xijing Hospital in Xi'an, and the West China Hospital in Chengdu, China. Patients who presented with CRC from stage I-IV were selected and enrolled in this study. Patient characteristics and tumor features are summarized in Table 18. The TNM staging classification for CRC is according to the 7th edition of the AJCC cancer staging manual. This project was approved by the institutional review board of Sun Yat-sen University Cancer Center, Xijing Hospital, and West China Hospital. Informed consent was obtained from all patients. Human blood samples were collected by venipuncture, and plasma samples were obtained by taking the supernatant after centrifugation and stored at -80°C before cfDNA extraction.

**Prospective CRC screening cohort study**

A CRC study was conducted using plasma samples on screening and early detection of a high-risk screening population in order to assess feasibility of using methylation markers for predicting CRC occurrence in high-risk populations. The cohort included individuals whom based on questionnaires were definition as high risk with CRC, and individuals whom are asymptomatic and aged 45 or above scheduled for screening colonoscopy from Sep 2015- Dec 2017. A total of 1450 subjects at high risk of CRC were scheduled for colonoscopy and cfDNA methylation test with the following situation: (i) age >45 years, (ii) ever smoking, alcohol consumption, diabetes mellitus; (iii) family history present (two or more first degree relatives with CRC or one or more with CRC at age 50 years or less; or known Lynch syndrome or familial adenomatous polyposis); (iv) had positive results on fecal blood testing or change in bowel habit. excluded subjects were those who had a personal history of colorectal neoplasia, digestive cancer, or inflammatory bowel disease; had undergone colonoscopy within the previous 10 years or a barium enema, computed tomographic colonography, or sigmoidoscopy within the previous 5 years; had undergone colorectal resection for any reason other than sigmoid diverticula; had overt rectal bleeding within the previous 30 days. The present study prospectively recruited screening subjects who gave informed consent and received colonoscopy in this program. This project was approved by the IRBs of Sun Yat-sen University Cancer Center, Xijing Hospital, and West China Hospital.

**Statistical Analysis**

**Solid tumor and whole blood methylation profiles**

DNA methylation data of 485,000 sites generated using the Infinium 450K Methylation Array were obtained from the TCGA and a dataset generated from an aging study (GSE40279) in which DNA methylation profiles for CRC and blood were analyzed. Both primary solid tissues from cancer patients and whole blood from healthy donor were measured by Illumina 450k infimum bead chip. IDAT format files of the methylation data were generated containing the ratio values of each scanned bead. Using the minfi package from Bioconductor, these data files were converted into a score, referred to as a Beta value. Methylation values of the Chinese cohort were obtained by targeted bisulfite sequencing using a molecular inversion probe and analyzed as described below.

**DNA methylation marker pre-selection for diagnostic and prognostic analysis**

A differential methylation analysis on TCGA data using a "moderated t-statistics shrinking" approach was performed and the p-value for each marker was then corrected by multiple testing by the Benjamini-Hochberg procedure to control FDR at a significance level of 0.05. The list was ranked by adjusted p-value and the top 1000 markers were selected for designing padlock probes for differentiating CRC versus normal samples. 1,000 molecular inversion (padlock) probes were then designed corresponding to these 1000 markers for capture-sequencing of cfDNA in CRC plasma. All padlock probe design and capture of bis-DNA were based on published techniques with some modification. Only 544 padlock probes were able to give positive and specific PCR amplification signals and they were therefore used as capture probes in the subsequent experiments in cfDNA samples. cfDNA samples with low quality or fewer than 30,000 reads per sample were also eliminated. About 1822 cfDNA samples were included in the subsequent study (801 CRC blood samples and 1021 normal blood samples). Methylated reads for each marker were defined as total unique methylated reads and methylation values for each marker were defined as the proportion of read counts with methylation divided by total read counts. For particular methylation markers with less than 20 unique reads, an imputed mean methylation value of CRC or normal healthy controls were used.

**Building a diagnostic model**

cfDNA sample data obtained from patients diagnosed with CRC (n=801) and healthy contol (n=1021) was randomly split into training and validation cohorts with a 2:1 ratio. Next, two variable selection methods were applied which were suitable for high-dimensionality on the prescreened training dataset: Least Absolute Shrinkage and Selection Operator (LASSO) and Random Forest based variable selection method using OOB error. As results can depend strongly on the arbitrary choice of a random sample split for sparse high-dimensional data, an analysis of the "multi-split" method was adopted, which improved variable selection consistency while controlling finite sample error. For LASSO selection operator, 75 percent of the dataset were subsampled replacement 500 times and the markers were selected with repeat occurrence frequency of more than 450. The tuning parameters was determined according to the expected generalization error estimated from 10-fold cross-validation and information-based criteria AIC/BIC, and the largest value of lambda was adopted such that the error was within one standard error of the minimum, known as "1-se" lambda. For the random forest analysis, using the OOB error as a minimization criterion, variable elimination was carried out from the random forest by setting variable a dropping fraction of each iteration at 0.3. The overlapping methylation markers were then selected by the two methods for model building a binary prediction. A logistic regression model was fitted using these 9 markers as the covariates and obtained a combined diagnosis score (designated as cd-score) by multiplying the unbiased coefficient estimates and the marker methylation value matrix in both the training and validation datasets. The predictability of the model was evaluated by area under ROC (AUC, also known as C-index), which calculated the proportions of concordant pairs among all pairs of observations with 1.0 indicating a perfect prediction accuracy. Confusion tables were generated using an optimized cd-score cutoff with a maximum Youden's index.

The pre-treatment or initial methylation level was obtained at the initial diagnosis, and the post-treatment level was evaluated approximately 2 months after treatment, where the treatment referred to either chemotherapy or surgical resection of tumor. The primary endpoint (including response to treatment: progressive disease (PD), partial response (PR) and stable disease (SD)) was defined according to the RECIST guideline. For patients treated with surgical removal and no recurrence at time of evaluation, it was assumed that they had complete response (CR). The difference of cd-score distribution between clinical categories was examined by two-sided t-test as the cd-score was shown to be non-normally distributed using a Shapiro-Wilk Test.

## Building a predictive model for prognosis and survival

The potential to use a combined prognosis score (cp-score) system for prediction of prognosis in CRC combination with clinical and demographic characteristics including age, gender, and AJCC stage was investigated. Tumor samples were selected from diagnosis's training cohort as training data set, and the tumor samples from validation cohort as the validation data set. Variable selection was conducted on the training data set and built the composite score on the validation cohort. A univariate pre-screening procedure was first performed to remove excessive noise to facilitate the computational analysis, which is generally recommended prior to applying any variable selection method. A marker with p-value < 0.05 from the Wald statistic was retained in the dataset. Second, a similar subsampling strategy in a diagnosis marker selecting process based on LASSO-cox method was used to shrink the marker numbers to a reasonable range (less than events). Slightly different from the binary classifier, subsampling was carried out in the training dataset with replacement in case that the event proportion was too low for a model construction. The frequency cutoff was set as 50 to retain approximately 1/10th of total events. The above analysis generated 2 final markers to construct a prognostic signature (Table 14). The cp-score was then calculated from the linear predictor of multi variate Cox regression model with the 2 markers. A Kaplan-Meier curve and log-rank test were generated using the dichotomized cp-score, which formed a high-risk and low-risk group membership assignment according to its median. This segmentation was compatible with that formed by AJCC stage. Time-dependent ROC was used to summarize the discrimination potential of the cp-score, AJCC stage, CEA level, primary tumor location and the combination of all factors, with ROC curves varying as a function of time and accommodating censored data. Finally, a multivariate Cox regression model was fitted to assess the significance of potential risk factors.

All hypothesis testing in the prognostic analysis section were done by two-sided with p-value < 0.05 considered to be statistically significant. All the analysis was conducted in R version 3.4.3 with the following packages used: 'glmnet', 'pROC', 'limma', 'survival', 'survival ROC', 'survcomp'.

## Unsupervised discovery of ctDNA methylation-based subtypes

Training dataset (n=528) was adopted to discover CRC groups/subtypes. Aiming to narrow down markers and get meaningful clustering by methylation information provided itself, an algorithm was used with iterative refinement of key features that was modified for better represents each cluster. This algorithm was modified as follows. Briefly, the markers were first filtered out with low variability using a threshold of median absolute deviation <0.5. The matrix was then adjusted by mean centering on markers and samples in training dataset. Secondly, an iterator procedure was used to classify and get a marker list for predicting subtypes: (i) Consensus Clustering was used to initially cluster the dataset and Cluster numbers was determined by relative change in area under CDF curve ($\Delta$A, cutoff=0.05); (ii) Calculated centroid of each cluster and get correlation coefficient vector of each sample by multiply methylation matrix and centroid matrix. (iii) Re-cluster samples based on correlation coefficient vectors. The number of new clusters was determined by the maximum average silhouette width; (iv) Get differentially methylated markers among the new clusters by using a moderated F-test (from 'limma' package); Then feed the subset of matrix in terms of differential methylated markers into next iteration. The iteration stopped when differential methylated marker list did not change from the previous run and this marker set was finally used as subtype signature for predicting CRC subtypes.

Validation was performed on the predefined validation dataset (n=273). To get the subtype of validation samples, centroid methylation value of each cluster was first calculated with the methylation level of selected subtyping signature in the training dataset. Here, the centroid methylation value was defined as a representative methylation value of a cluster of samples by get mean value of signature across samples. Secondly, samples from validation cohort were assigned to clusters according to maximum Pearson correlation coefficient to each centroid value.

## Cell-free DNA extraction from plasma samples

Plasma samples were frozen and preserved in at -80°C until use. 20,000 or more total unique reads per sample were observed to fulfilled this criterion, and in order to reliably produce >20,000 unique reads, a volume of 1.5ml or more plasma was required. This relationship between the amount of cfDNA in plasma and detected copy number was further accessed using digital droplet PCR. In addition, it was found that 1.5ml of plasma yielded > 10ng of cfDNA, which produced at least 140 copies of detected amplicons in each digital droplet PCR assay. Based on these findings, 15 ng/1.5 ml was used as a cutoff to obtain reliable measurements of DNA methylation for all experiments in this study. For all cfDNA extractions, the EliteHealth cfDNA extraction Kit was used (EliteHealth, Guangzhou, China) according to the manufacturer's recommendations.

## Bisulfite conversion of genomic or cfDNA

10 ng of DNA was converted to bis-DNA using EZ DNA Methylation-Lightning™ Kit (Zymo Research) according to the manufacturer's protocol. Resulting bis-DNA had a size distribution of ~200-3000 bp, with a peak around ~500-1000 bp. The efficiency of bisulfite conversion was >99.8% as verified by deep-sequencing of bis-DNA and analyzing the ratio of C to T conversion of CH (non-CG) dinucleotides.

## Determination of DNA methylation levels by deep sequencing of bis-DNA captured with molecular-inversion (padlock) probes

In order to identify DNA sites with significantly different rates of methylation between CRC and normal blood, the variance was shrank using a t-statistic with Empirical Bayes36 and selected the top 1000 significant markers with the Benjamini-Hochberg procedure 37 controlling the FDR at a significance level of 0.05. CRC and normal blood were successfully distinguished by unsupervised hierarchical clustering of these top 1000 markers. 1,000 molecular inversion (padlock) probes corresponding to these 1000 markers were then designed for capture-sequencing of cfDNA. Capture and sequencing were performed in bisulfite-converted cfDNA samples.

## Probe design, synthesis and validation

All probes were designed using the ppDesigner software. The average length of the captured region was 100 bp, with the CpG marker located in the central portion of the captured region. Linker sequence between arms contained binding sequences for amplification primers separated by a variable stretch of Cs to produce probes of equal length. A 6-bp unique molecular identifier (UMI) sequence was incorporated in probe design to allow for the identification of unique individual molecular capture events and accurate scoring of DNA methylation levels. For capture experiments, probes were synthesized as separate oligonucleotides using standard commercial synthesis methods (IDT) and mixed in equimolar quantities, followed by purification using Qiagen columns.

There were significant differences between the number of reads captured by the most efficient probes and the non-efficient probes (60-65% of captured regions with coverage >0.2x of average) as shown by deep sequencing of the original pilot capture experiments. To ameliorate this, relative efficiencies were calculated from sequencing data and probes were mixed at adjusted molar ratios, which increased capture uniformity to 85% of regions at > 0.5x of average coverage.

## Bis-DNA capture

To anneal probes to DNA, 10 ng of bisulfite-converted DNA was mixed with padlock probes in 20 μl reactions containing 1X Ampligase buffer (Epicentre), followed by 30 second denaturation at 95°C was followed by a slow cooling to 55°C at a rate of 0.02°C per second. Hybridization was left to complete for 15 hrs at 55°C. To fill gaps between annealed arms, 5μl of the following mixture was added to each reaction: 2U of PfuTurboCx polymerase (Agilent), 0.5U of Ampligase (Epicentre) and 250 pmol of each dNTP in 1X Ampligase buffer. After 5-hour incubation at 55°C, reactions were denatured for 2 minutes at 94°C. 5 μl of exonuclease mix (20U of Exo I and 100U of ExoIII, both from Epicentre) was added and single-stranded DNA degradation was carried out at 37°C for 2 hours, followed by enzyme inactivation for 2 minutes at 94°C.

Circular products of site-specific capture were amplified by PCR with concomitant barcoding of separate samples. Amplification was carried out using primers specific to linker DNA within padlock probes, one of which contained specific 6bp barcodes. Both primers contained Illumina next-generation sequencing adaptor sequences. PCR was performed as follows: 1X Phusion Flash Master Mix, 3 μl of captured DNA and 200nM primers, using the following cycle:

10s @ 98°C, 8X of (1s @ 98°C, 5s @ 58°C, 10s @ 72°C), 25X of (1s @ 98°C, 15s @ 72°C), 60s @ 72°C. PCR reactions were mixed and the resulting library was size selected to include effective captures (~230bp) and exclude "empty" captures (~150bp) using Agencourt AMPure XP beads (Beckman Coulter). Purity of the libraries was verified by PCR using Illumina flowcell adaptor primers (P5 and P7) and the concentrations were determined using Qubit dsDNA HS assay (Thermo Fisher). Libraries were sequenced using MiSeq and HiSeq2500 systems (Illumina).

**Optimization of capture coverage uniformity**

Deep sequencing of the original pilot capture experiments showed significant differences between number of reads captured by most efficient probes and non-efficient probes (60-65% of captured regions with coverage >0.2x of average). To ameliorate this, relative efficiencies were calculated from sequencing data and probes were mixed at adjusted molar ratios. This increased capture uniformity to 85% of regions at > 0.2x of average coverage.

**Sequencing data analysis**

Mapping of sequencing reads was done using the software tool bisReadMapper with some modifications. First, UMI were extracted from each sequencing read and appended to read headers within FASTQ files using a custom script. Reads were on-the-fly converted as if all C were non-methylated and mapped to in-silico converted DNA strands of the human genome, also as if all C were non-methylated, using Bowtie2. Original reads were merged and filtered for single UMI, i.e. reads carrying the same UMI were discarded leaving a single, unique read. Methylation frequencies were calculated for all CpG dinucleotides contained within the regions captured by padlock probes by dividing the numbers of unique reads carrying a C at the interrogated position by the total number of reads covering the interrogated position.

**Identification of Methylation Correlated Blocks (MCB)**

In order to maximize the ability to measure small differences in DNA methylation advantage was taken of the notion that closely positioned CpG tend to have similar methylation levels, what is believed to be a result of the processivity and lack of sequence-specificity of DNA methyltransferases and demethylases, as well as the concept of haplotype blocks in genetic linkage analysis. Sequencing data was used to a generate MCB map using CRC and normal cfDNA samples. Pearson correlation coefficients between methylation frequencies of each pair of CpG markers separated by no more than 200bp were calculated separately across 50 cfDNA samples from each of the two diagnostic categories, e.g. normal blood and CRC. A value of Pearson's r < 0.5 was used to identify boundaries between adjacent markers with uncorrelated methylation. Markers not separated by a boundary were combined into Methylation Correlated Blocks (MCBs). This procedure identified a total of ~1550 MCBs in each diagnostic category within the padlock data, combining between 2 and 22 CpG positions in each block. Methylation frequencies for entire MCBs were calculated by summing up the numbers of Cs at all interrogated CpG positions within a MCB and dividing by the total number of C+Ts at those positions.

**Droplet digital PCR**

The methylation status of cg10673833 was determined for each of these samples using a droplet digital PCR paradigm featuring a Bio-Rad (Carlsbad, CA) QX-200 Droplet Reader and an Automated Droplet Generator (AutoDG). In brief, 10 ng of DNA from each subject was bisulfite converted using EZ DNA Methylation-Lightning™ Kit (Zymo Research). An aliquot of each sample was pre-amplified, diluted 1:3,000, and then PCR amplified using fluorescent, dual labeled primer probe sets specific for cg10673833 from Behavioral Diagnostics and Universal Digital PCR reagents and protocols from Bio-Rad. The number of droplets was determined using a QX-200 droplet counter and analyzed using QuantiSoft software. The results were expressed as a percent methylation. Reactions were excluded if fewer than 10,000 droplets were counted.

**Patient and sample characteristics**

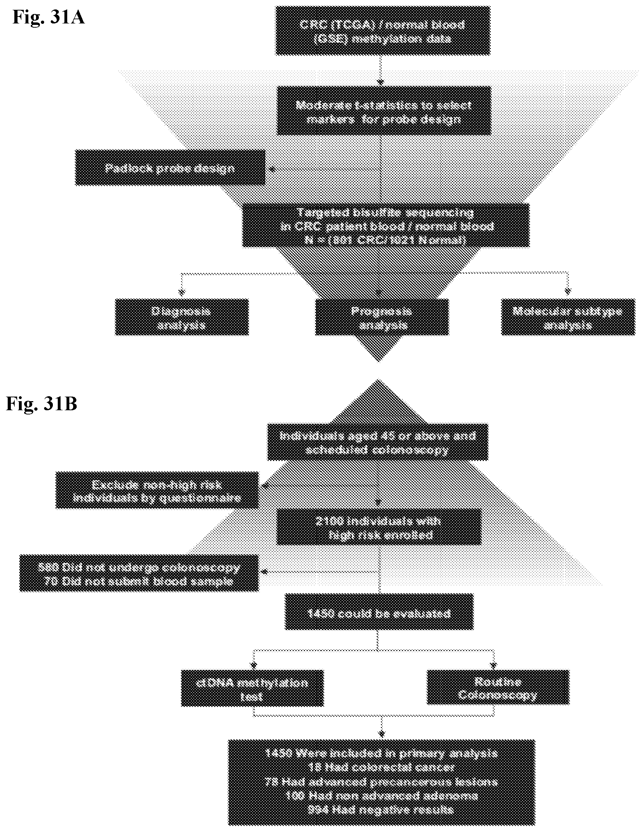

The clinical characteristics and the methylation profiling of 459 CRC tumor samples were collected from The Cancer Genome Atlas (TCGA) and 754 normal samples from a dataset used in a previous methylation study on aging (GSE40279) to identify the CRC specific methylation markers. To study cfDNA in CRC, plasma samples were obtained from 801 Chinese patients with CRC, and from randomly selected, population-matched 1021 healthy controls undergoing routine health care maintenance (Fig. 31A). Informed written consent was obtained from each participant. Clinical characteristics of all patients and controls are listed in Table 18.

A total of 1450 participants with high-risks of CRC were enrolled in the prospective screening cohort study and undergoing colonoscopy and cfDNA methylation test (Fig. 31B). Among them 18 participants were found to have colorectal cancer on colonoscopy (prevalence, 1.2%), 78 participants had advanced precancerous lesions (prevalence, 5.3%). Clinical characteristics of all participants from this cohort are listed in Table 19.

**Identification of methylation markers differentiating CRC and blood**

To identify putative markers that differentiate between CRC and normal blood samples, methylation data derived from CRC tissue DNA from the TCGA and normal blood were compared. The methylation data was obtained from 459 CRC and 754 blood samples from healthy controls. A "moderated t-statistic" analysis with Empirical Bayes for shrinking the variance was used and the top 1000 significant markers were selected by controlling the false discovery rate (FDR) at significance level 0.05 using the Benjamini-Hochberg procedure. The molecular-inversion (padlock) probes corresponding to these 1000 markers were designed for capture-sequencing cfDNA from plasma and 544 markers were selected with good experimental amplification profile and dynamic methylation range for further analysis. The genetic linkage disequilibrium (LD block) concept was used to study the degree of co-methylation among different DNA strands, with the underlying assumption that DNA sites in close proximity are more likely to be co-methylated than distant sites.

**cfDNA diagnostic prediction model for CRC**

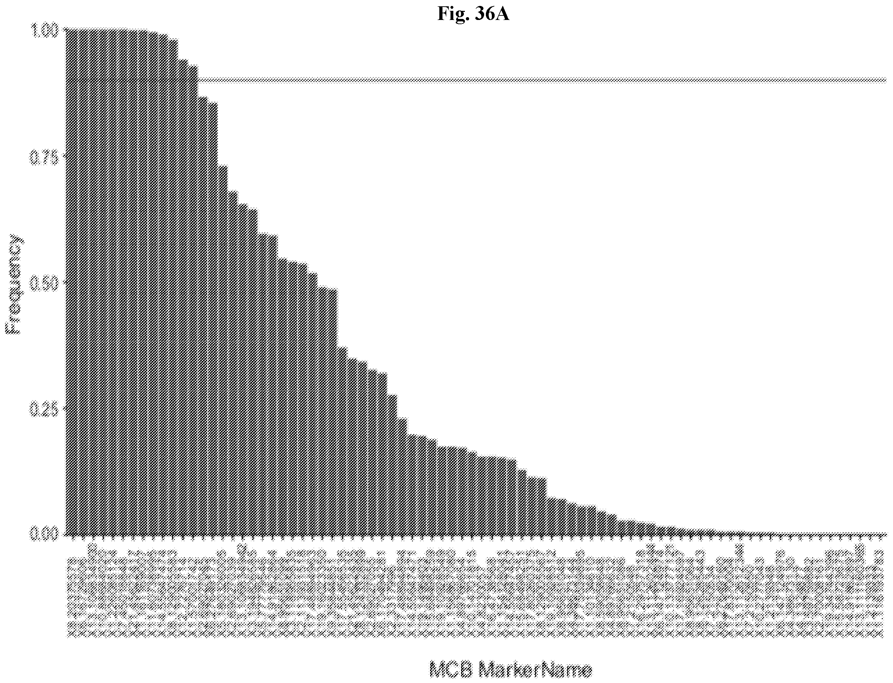

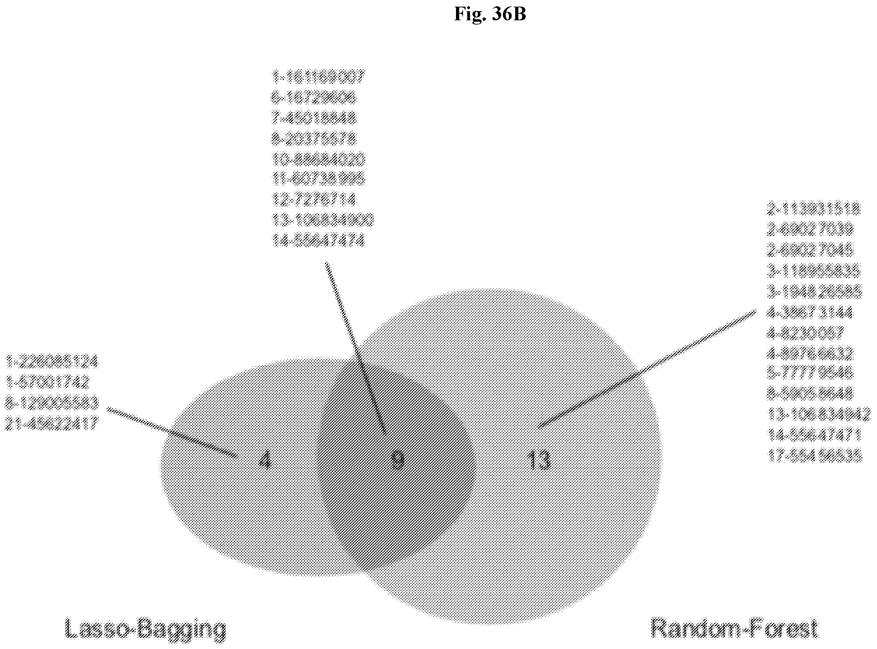

The entire methylation dataset of 544 markers was analyzed by Least Absolute Shrinkage and Selection Operator (LASSO) and Random Forest to reduce the number of markers (Fig. 36). 801 CRC samples and 1021 normal control samples were randomly assigned to a training set and a validation set with a 2:1 ratio (Fig. 32A). LASSO-based feature selection identified 13 markers and Random Forest-based feature selection identified 22 markers for discriminating CRC versus normal. There were 9 overlapping markers between these two methods (Table 13). A diagnostic prediction model was constructed with these 9 markers and a combined diagnostic score system (cd-score) was formulated according to the coefficients from the multinomial logistic regression. Applying this model, a high consistency was observed between predicted results and pathological diagnosis results in both the training dataset and validation dataset (Fig. 32B and 32C). The area of AUC was 0.96 (95%CI 0.95-0.97) in training dataset (Fig. 32D) and 0.96 (95%CI 0.94-0.97) in validation dataset (Fig. 32E) respectively. By using a best cutoff value determined via Youden index method, the model yielded a sensitivity of 87.5% and a specificity of 89.9% for discriminating CRC from normal controls in the training dataset and a sensitivity of 87.9% and a specificity of 89.6% in the validation dataset (Fig. 32G and 32H). While these results clearly demonstrated the potential of ctDNA methylation markers for predicting the presence of CRC.
The utility of the cd-score in assessing the staging of CRC was then examined, the presence of residual tumor after treatment, the response of treatment (such as surgery or chemotherapy). The cd-scores of patients with detectable residual tumor following treatment were significantly higher than those without detectable tumor ($p=<0.001$, Fig. 37A). Similarly, there was good correlation between the cd-scores and tumor stage. Patients with early stage disease (I, II) had substantially lower cd-scores compared to those with advanced stage disease (III, IV) ($p=<0.001$, Fig. 37B). Furthermore, the cd-scores were significantly higher in patients before treatment compared to those received surgery ($p< 0.001$, Fig. 37E). When the tumor recurrence, the cd-score increase again (Fig. 37E).
CEA has been used in diagnosis and surveillance of CRC for decades. But its sensitivity and specificity are not satisfied, which led to the necessity of invasive approaches, like colonoscopy, in most suspected CRC patients. In biopsy-proven CRC patients of the cohort, the cd-score demonstrated superior sensitivity and specificity to CEA for CRC diagnosis (AUC 0.96 vs 0.72, Fig. 37F). Both cd-score and CEA values were highly correlated with tumor stage (Fig. 37B and Fig. 37D). In patients with treatment response or tumor recurrence, the cd-score showed more significant changes from initial diagnosis than that of CEA (Fig. 37E and Fig. 37F).

**cfDNA prognostic prediction model for CRC**

Next the potential to use a combined prognosis score (cp-score) based on ctDNA methylation analysis was investigated for prediction of prognosis of CRC in combination with clinical and demographic characteristics including age, gender, primary tumor site and AJCC stage. Follow-up information of 801 CRC patients for prognostic score-based analysis was completed. The median follow-up times were 11.9 months (rang 0.5-25.7 months). The same training dataset as diagnosis section containing 528 observations with 73 events and validation dataset containing 273 observations with 32 events was applied. A variable selection was conducted on the training set and built the composite score on the validation set. UniCox and LASSO-Cox methods were implemented to reduce the dimensionality and constructed a Cox-model to predict prognosis with a two-markers panel (Fig. 33A and Table 14). A Kaplan-Meier curve and log-rank test were generated using the dichotomized composite score, which resulted in a high-risk and low-risk group membership assignment according to its median. Median survival time in low-risk group was significantly better than that in high-risk group ($p<0.001$) (Fig. 33B and Fig. 33C).
Time-depended ROC was used to summarize the discrimination potential of the composite score, AJCC stage, CEA level, primary tumor location, and the combination of all the existing biomarkers. Multivariate Cox regression indicated that the cp-score significantly correlated with risk of death and was an independent risk factor of survival both in training set and validation set (Table 15). TNM stage (as defined by AJCC guidelines), CEA level, primary tumor location also predicted the 12-month survival of patients with CRC (Table 15). Furthermore, the combination of cp-score and clinical characteristics improved the ability to predict prognosis (AUC 0.79, 95%CI 0.70-0.88 in training cohort and 0.85, 95%CI 0.75-0.96) in validation cohort) (Fig. 33D and Fig. 33E).

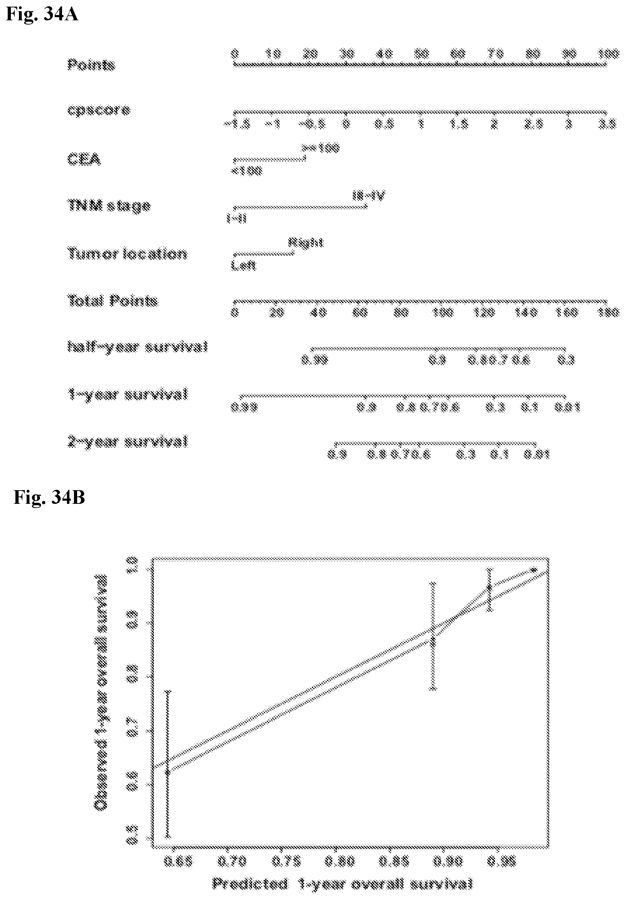

Nomograms create a simple graphical representation of a statistical predictive model that generates a numerical probability of a clinical event. It can reduce statistical predictive models into a single numerical estimate of the probability of death. Multivariate Cox regression analysis identified four variables as independent predictive factors (cp-score, CEA level, TNM stage and primary tumor location, Table 15) in both training and validation cohort. As such, a nomogram was developed with point scales of these 4 variables to predict overall survival for CRC patients (Fig. 34A). The sum of each variable point was plotted on the total point axis, and the estimated median 1- and 2-year overall survival rates were obtained by drawing a vertical line from the plotted total point axis straight down to the outcome axis. In validation cohort, the c-index of this model was 0.839, indicating a good discrimination. Fig. 34B showed the calibration graph for the nomogram, in which the probability of 1-year overall survival as predicted by the nomogram is plotted against the corresponding observed survival rates obtained by the Kaplan-Meier method.

**cfDNA methylation based subtyping of CRC**

To generate a cfDNA methylation based subtypes of CRC, an unsupervised clustering method was utilized. The method applied an iteration strategy that iteratively got rid of markers had less contributions to the initial clusters (Fig. 35A). Using the same training dataset as in building prognosis model, two clusters of CRC samples were obtained with a combined total of 45 markers that were differential methylated between clusters (Fig. 35B). From in silhouette analysis of the last round of iterations, a high separation distance was observed (Fig. 35C). The validation set was also classified by computing their correlation coefficients to centroid profile of clusters from training set.

The validation set was divided into two groups that shows a distinctly different methylation profile of the 45 markers (Fig. 35D). To explore the clinical relevance of the two subtypes, associations between the subtype and all available clinical factors including TNM stage, tumor site, MMR status, MSS status, tumor burden, sex, mutation status of a limited gene panels and survival outcomes were systematically tested. As a result, the second cluster in both training and validation data set have a significant poor survival rate than that of the first cluster (Fig. 35E, upper panel, both $p<0.01$, Log-rank Test). It was also found that the proportion of high stage CRC in cluster 2 were significantly higher than cluster 1 (Fig. 35E, lower panel, both $p<0.05$, Chi-squared Test). Considering the potential multicollinearity of the subtype and TNM stage in discriminating cohort with poor survival, multivariate cox regression analysis was performed and both factors were mutually independent for predicting overall survivals (Table 16).

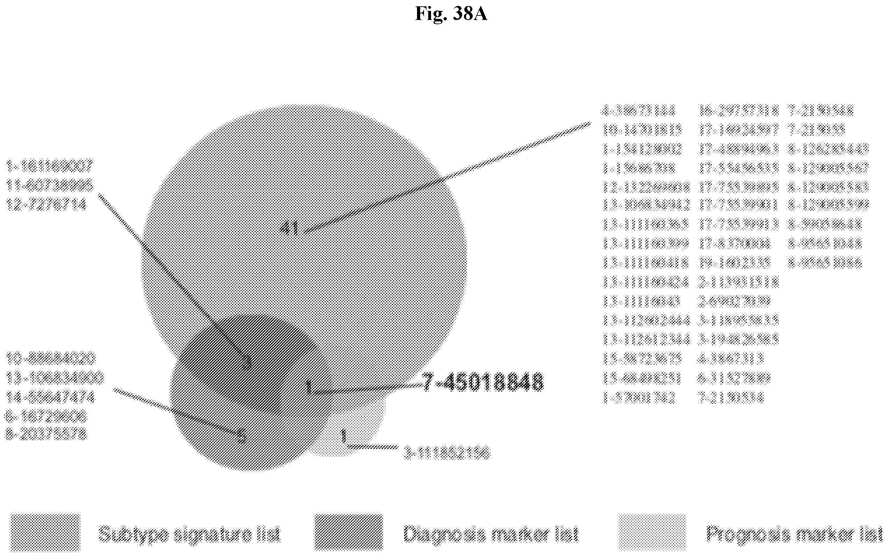

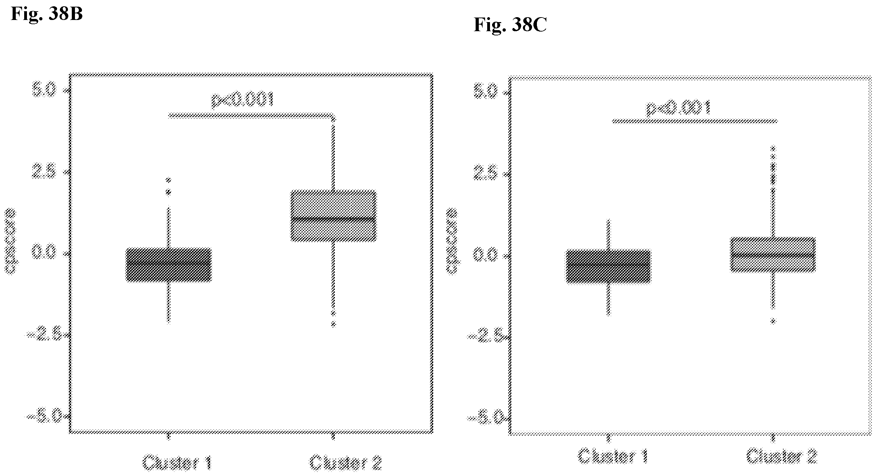

The 45 markers for subtyping was classified into two groups according to the hypo- or hyper- methylation in clusters (27 hypo and 18 hyper in cluster 2, Table 20). Among these markers, three was also identified in the list of diagnosis markers and one was identified in both the diagnosis and prognosis marker lists (Fig. 38). Further analysis showed that cp-scores in cluster2 were significantly higher than cluster1 in two datasets (Fig. 40B, $p<0.001$, Wilcox Test). Given that 7-45018848 was identified in the three separate marker lists and was shown to be hyper-methylated in cluster 2, its presence may contribute to the vary survival outcomes between the two subtypes.

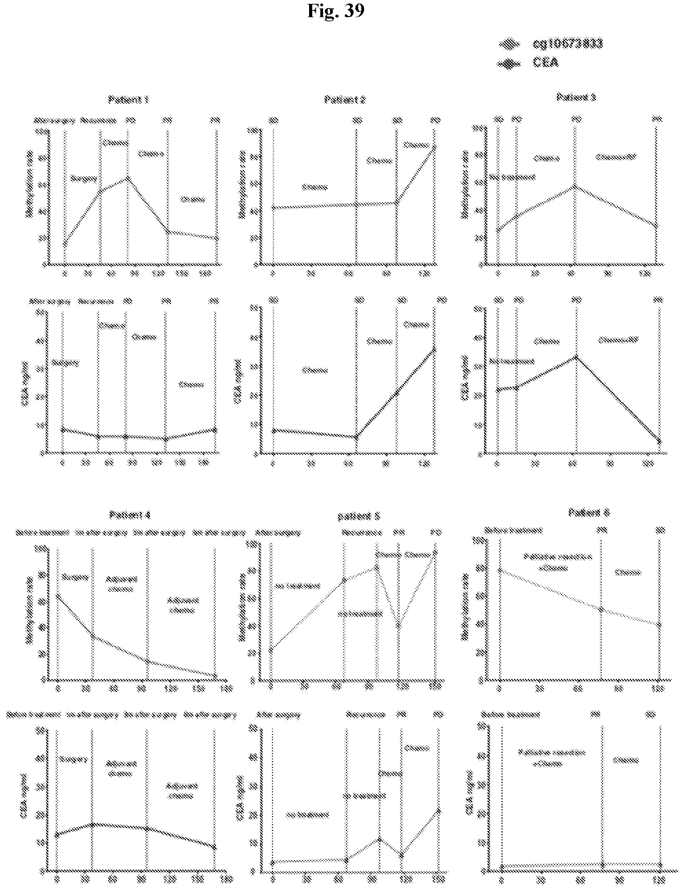

As CpG site cg10673833 (7-45018848) was the only overlapping marker in diagnosis, prognosis, and molecular subtype analysis, the utility of cg10673833 as a potential marker in monitoring treatment response was investigate. Serial longitudinal dynamic changes of methylation values of cg10673833 were obtained in a group of CRC patients. The results showed that there was a high correlation between methylation values of cg10673833 and treatment outcomes, which was greater than that observed for CEA (Fig. 39). In patients with serial samples, those with a positive treatment response had a concomitant and significant decrease in methylation values of cg10673833 compared to that prior to treatment, and there was an even further reduction in methylation values of cg10673833 in patients after surgery. In contrast, patients with progressive or recurrent disease had an increase methylation rate (Fig. 40).

**Methylation markers for screening and early diagnosis of CRC in high-risk populations using 7-45018848**

The potential of cg10673833 as a methylation marker in detection of CRC and precancerous lesions in high-risk population based on plasma samples was investigated. From January 2015 through June 2017, 1450 participants who were recognized with high risks of CRC, were scheduled to undergo screening colonoscopy and methylation test of cg10673833 (Fig. 31B).

Table 17 showed the screening results of colonoscopy and cg10673833 methylation testing. cg10673833 methylation testing identified 9/10 participants with CRC and 7/8 participants with CRC in situ, for sensitivity of 88.9% (95% CI, 0.74-1.00) and specificity of 86.5% (95% CI, 0.85-0.88). Positive predictive value (PPV) and negative predictive value (NPV) were 0.077 (95% CI, 0.041 to 0.113) and 0.998 (95% CI, 0.996-1.00) (Table 21), respectively. For advanced precancerous lesions, the sensitivity was 33.3% (95% CI, 0.229-0.438), significant higher than the positivity rate for subjects without any pathology (12.3%, 95% CI, 0.106-0.141).

Table 13. Characteristics of night methylation markers and their coefficients in diagnosis.

| MCBs | Target ID | Ref Gene | Coefficients |
|---|---|---|---|
| | Intersect | | -2.76 |
| 7-45018848 | cg10673833 | MYO1G | 15.98 |
| 1-161169007 | cg10493436 | ADAMTS4 | 1.88 |
| 10-88684020 | cg10428836 | BMPR1A | -6.05 |
| 11-60738995 | cg27284288 | CD6 | 3.29 |
| 12-7276714 | cg16959747 | RBP5 | 1.45 |
| 13-106834900 | cg17494199 | Chr 13:10 | -2.24 |
| 14-55647474 | cg23678254 | LGAP5 | 2.71 |
| 6-16729606 | cg24067911 | ATXN1 | -3.41 |
| 8-20375578 | cg25459300 | Chr 8:20 | 4.06 |

Table 14. Characteristics of two methylation markers and their coefficients in prognosis.

| MCBs | Target ID | Ref Gene | Coefficients | HR | CI (lower) |
|---|---|---|---|---|---|
| 7-45018848 | cg10673833 | MYOIG | 2.06 | 7.81 | 2.11 |
| 3-111852156 | cg25462303 | GCET2 | 3.05 | 21.13 | 5.98 |

Table 15. Multivariable cox regression analysis with covariates including cp-score, gender, age, tumor location, TNM stage, CEA for overall survival.

| Factor | Training dataset | | | | | |
|---|---|---|---|---|---|---|
| | coef | Exp (coef) | Se (coef) | z | p | coef |
| cp-score: H vs L | 0.75 | 2.11 | 0.13 | 5.88 | **<0.001** | 0.72 |
| Gender: M vs F | -0.31 | 0.73 | 0.26 | -1.19 | 0.232 | -0.15 |
| Age: >=65 vs <65 | 0.02 | 1.02 | 0.01 | 1.91 | 0.056 | -0.01 |
| Stage: III/IV vs I/II | 0.92 | 2.51 | 0.27 | 3.45 | **0.001** | 1.95 |
| Tumor site: R vs L | 0.62 | 1.85 | 0.26 | 2.36 | **0.018** | 0.86 |
| CEA : >= vs < 100 | 0.7 | 2.02 | 0.31 | 2.26 | **0.024** | 1.33 |

Table 16. Association between cfDNA methylation based CRC subtypes and CRC prognosis in both the training and validation set (the same cohort as the prognosis model analysis).

| | Univariate | | Multivariate | |
|---|---|---|---|---|
| | HR (95% CI) | *P* | HR (95% CI) | *P* |
| **Training Set** | | | | |
| Subtype: 2 vs 1 | 6.36 (3.98-10.17) | >0.001 | 5.24 (3.26-8.4) | >0.001 |
| TNM stage: III-IV vs I-II | 7.46 (2.35-23.7) | >0.001 | 4.95 (1.54-15.9) | 0.007 |
| **Validation Set** | | | | |
| Subtype: 2 vs 1 | 2.61 (1.25-5.14) | 0.01 | 2.28 (1.1-4.73) | 0.027 |
| TNM stage: III-IV vs I-II | 10.65 (1.45-78.02) | 0.02 | 9.54 (1.3-70.12) | 0.027 |

Table 17. Sensitivity and Specificity of the ctDNA methylation test for the findings on colonoscopy.

| Colonoscopy Finding | Persons with Finding (n=1450) | | | ctDNA methylation test (n=1450) | |
|---|---|---|---|---|---|
| | | Positive Results | Negative Results | Sensitivity | Specificity |
| Colorectal cancer | No. | No. | No. | (95% CI) | (95% CI) |
| Stage I-III colorectal cancer | 10 | 9 | 1 | 90.0 (71.4-108) | |
| High grade dysplasia | 8 | 7 | 1 | 87.5 (64.6-110) | |
| All Colorectal Cancer | | | | **88.9 (74.4-103)** | |
| Advanced precancerous lesion* | 78 | 26 | 52 | **33.3 (22.9-43.8)** | 66.7 (0.56-0.77) |
| Non-advanced adenoma | 100 | 24 | 76 | 24.0 (15.6-32.2) | 76.0 (0.68-0.84) |
| Polyps | 250 | 20 | 230 | 8.0 (4.6-11.4) | 92.0 (88.6-95.4) |
| Inflammatory bowel disease | 10 | 0 | 10 | | 100 |
| Negative on colonoscopy | 994 | 123 | 871 | | **87.6 (85.6-89.7)** |
| All non-advanced adenoma, non-neoplastic finding, and negative results on colonoscopy | 1354 | 167 | 1187 | | **87.7 (85.9-89.4)** |

Table 18. Clinical characteristics of study cohort.

| Characteristic | TCGA CRC tissue | GSE Normal blood | CRC serum | Normal control |
|---|---|---|---|---|
| Total (n) | 459 | 754 | 801 | 1021 |
| Gender | | | | |
| Female-no.(%) | 216 (47.1) | 401 (53.2) | 305 (38.1) | 486 (47.6) |
| Male-no.(%) | 243 (52.9) | 353 (46.8) | 496 (61.9) | 470 (46.0) |
| NA | 0 | 0 | 0 | 65 (6.4) |
| Age (years) | | | | |
| Mean | 68 | 63 | 58 | 47 |
| Range | 33-90 | 19-101 | 24-85 | 19-90 |
| Stage | | | | |
| I | 76 (16.6) | NA | 38 (4.7) | NA |
| II | 179 (39.0) | NA | 139 (17.4) | NA |
| III | 131 (28.5) | NA | 209 (26.1) | NA |
| IV | 65 (14.2) | NA | 406 (50.7) | NA |
| NA | 8 (1.7) | NA | 9 (1.1) | NA |
| Tumor Burden | | | | |
| Tumor free | 199 (43.4) | NA | 290 (36.2) | NA |
| With tumor | 215 (46.8) | NA | 511 (63.8) | NA |

(continued)

| Tumor Burden | | | | |
|---|---|---|---|---|
| NA | 45 (9.8) | NA | 0 | NA |
| RAS Status | | | | |
| Wide type | 24 (5.3) | NA | 122 (15.2) | NA |
| Mutation | 23 (5.0) | NA | 78 (9.8) | NA |
| NA | 412 (89.7) | NA | 601 (75.0) | NA |
| MMR Status | | | | |
| Proficient | 81 (17.7) | NA | 476 (59.4) | NA |
| Deficient | 12 (2.6) | NA | 35 (4.4) | NA |
| NA | 366 (79.7) | NA | 290 (36.2) | NA |
| Tumor Site | | | | |
| Right colon | 257 (55.9) | NA | 197 (24.6) | NA |
| Left colon | 182 (39.7) | NA | 593 (74.0) | NA |
| NA | 20 (4.4) | NA | 11 (1.4) | NA |

Table 19. Clinical characteristic of screening study cohort.

| | CRC | AA | Others |
|---|---|---|---|
| Characteristic | N=18 | N=78 | N=1354 |
| Age ( mean [SD] ) | | | |
| 45-49 yr-no. (%) | 0 | 4 (5.1) | 338 (25.0) |
| 50-59 yr-no. (%) | 8 (44.4) | 18 (23.1) | 446 (32.9) |
| 60-69 yr-no. (%) | 10 (55.6) | 42 (53.9) | 472 (34.9) |
| >70 yr-no. (%) | 0 | 14 (17.9) | 98 (7.2) |
| Sex | | | |
| Female (%) | 8 (44.4) | 30 (38.5) | 698 (51.6) |
| Male (%) | 10 (55.6) | 48 (61.5) | 656 (48.4) |

Table 20. Characteristics of 45 methylation markers in ctDNA methylation based subtyping of CRC.

| MCBs | Target ID | Methylation status | Ref Gene |
|---|---|---|---|
| 4-38673144 | cg05205843 | Hypo in cluster 1 | *KLF3* |
| 17-75539913 | cg11841704 | Hypo in cluster 1 | NA |
| 8-95651048 | cg06699564 | Hypo in cluster 1 | NA |
| 13-111160399 | cg08924619 | Hypo in cluster 1 | *COL4A2* |
| 1-57001742 | cg11959316 | Hypo in cluster 1 | *PPAP2B* |
| 13-111160365 | cg08924619 | Hypo in cluster 1 | *COL4A2* |
| 8-95651086 | cg06699564 | Hypo in cluster 1 | NA |
| 17-75539901 | cg01824933 | Hypo in cluster 1 | NA |
| 13-111160418 | cg08924619 | Hypo in cluster 1 | *COL4A2* |
| 4-3867313 | cg05205842 | Hypo in cluster 1 | *KLF3* |
| 13-111160424 | cg08924619 | Hypo in cluster 1 | *COL4A2* |
| 17-48894963 | cg04049981 | Hypo in cluster 1 | NA |
| 1-15686708 | cg09026722 | Hypo in cluster 1 | *PEAR1* |
| 13-112602444 | cg03616722 | Hypo in cluster 1 | NA |

(continued)

| MCBs | Target ID | Methylation status | Ref Gene |
|---|---|---|---|
| 13-11116043 | cg08924619 | Hypo in cluster 1 | COL4A2 |
| 17-16924597 | cg05928904 | Hypo in cluster 1 | NA |
| 3-194826585 | cg08704934 | Hypo in cluster 1 | C3orf21 |
| 7-2150548 | cg09776772 | Hypo in cluster 1 | MAD1L1 |
| 13-106834942 | cg17494199 | Hypo in cluster 1 | NA |
| 17-75539895 | cg01824933 | Hypo in cluster 1 | NA |
| 8-126285443 | cg16296417 | Hypo in cluster 1 | NSMCE2 |
| 7-2150534 | cg09776772 | Hypo in cluster 1 | MAD1L1 |
| 7-215055 | cg09776772 | Hypo in cluster 1 | MAD1L1 |
| 15-68498251 | cg05338167 | Hypo in cluster 1 | CALML4 |
| 1-161169007 | cg10493436 | Hypo in cluster 1 | ADAMTS4 |
| 13-112612344 | cg011251410 | Hypo in cluster 1 | NA |
| 15-58723675 | cg16391792 | Hypo in cluster 1 | LIPC |
| 6-31527889 | cg06393830 | Hyper in cluster 2 | NA |
| 2-113931518 | cg09366118 | Hyper in cluster 2 | PSD4 |
| 3-118955835 | cg22513455 | Hyper in cluster 2 | B4GALT4 |
| 8-129005567 | cg17583432 | Hyper in cluster 2 | PVT1 |
| 8-59058648 | cg23881926 | Hyper in cluster 2 | FAM110B |
| 16-29757318 | cg09638208 | Hyper in cluster 2 | C16orf54 |
| 17-55456535 | cg12441066 | Hyper in cluster 2 | MSI2 |
| 11-60738995 | cg27284288 | Hyper in cluster 2 | CD6 |
| 10-14701815 | cg04441857 | Hyper in cluster 2 | FAM107B |
| 8-129005599 | cg17583432 | Hyper in cluster 2 | PVT1 |
| 7-45018848 | cg10673833 | Hyper in cluster 2 | MYO1G |
| 1-154128002 | cg19757176 | Hyper in cluster 2 | TPM3 |
| 19-1602335 | cg08670281 | Hyper in cluster 2 | CYP4F11 |
| 8-129005583 | cg17583432 | Hyper in cluster 2 | PVT1 |
| 17-8370004 | cg04460364 | Hyper in cluster 2 | NDEL1 |
| 12-7276714 | cg16959747 | Hyper in cluster 2 | RBP5 |
| 12-132269608 | cg15011734 | Hyper in cluster 2 | SFRS8 |
| 2-69027039 | cg25754195 | Hyper in cluster 2 | ARHGAP25 |

Table 21. Positive and negative predictive values of cfDNA methylation test.

| Outcome | N of participations | Positive Predictive Value (95% CI) | Negative Predictive Value (95% CI) |
|---|---|---|---|
| Colorectal cancer | 18/1450 | 0.077 (0.041-0.113) | 0.998 (0.996-1.00) |
| Advanced precancerous lesion | 78/1450 | 0.134 (0.087-0.183) | 0.042 (0.031-0.053) |

While preferred embodiments of the present disclosure have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the disclosure described herein may be employed in practicing the disclosure. It is intended that the following claims define the scope of the disclosure and that methods and structures within the scope of these claims and their equivalents be covered thereby.

## Table 1

| Marker | CpG | Shared_among | Marker | CpG | Shared_among |
|---|---|---|---|---|---|
| BLCA (cancer vs cancer) | | | LIHC (cancer vs cancer) | | |
| Status - Hyper | | | Status - Hyper | | |
| 1-26662567 | cg23053573 | (cancer vs cancer) (cancer+normal vs cancer+normal) | 1-145395607 | cg12420472 | (cancer vs cancer) (cancer+normal vs cancer+normal) |
| 1-27050491 | cg22042546 | (cancer vs cancer) (cancer+normal vs cancer+normal) | 1-145395753 | cg17952661 | (cancer vs cancer) (normal vs normal) (cancer+normal vs cancer+normal) |
| 1-29586570 | cg03894975 | (cancer vs cancer) | 1-145395846 | cg02395363 | (cancer vs cancer) (cancer+normal vs cancer+normal) |
| 10-105420501 | cg19007269 | (cancer vs cancer) | 1-145455384 | cg21961202 | (cancer vs cancer) (cancer+normal vs cancer+normal) |
| 10-22518317 | cg15900657 | (cancer vs cancer) (cancer+normal vs cancer+normal) | 1-160370208 | cg13428480 | (cancer vs cancer) |
| 10-45914525 | cg21981270 | (cancer vs cancer) | 1-206730839 | cg23520347 | (cancer vs cancer) (cancer+normal vs cancer+normal) |
| 10-45914840 | cg00675569 | (cancer vs cancer) | 1-21616619 | cg17216478 | (cancer vs cancer) (cancer+normal vs cancer+normal) |
| 10-45914949 | cg09806262 | (cancer vs cancer) | 1-21616641 | cg06819200 | (cancer vs cancer) (cancer+normal vs cancer+normal) |
| 11-59333490 | cg14665813 | (cancer vs cancer) | 1-24645885 | cg21289280 | (cancer vs cancer) (cancer+normal vs cancer+normal) |
| 12-53591766 | cg04972065 | (cancer vs cancer) | 1-247495769 | cg11684022 | (cancer vs cancer) |
| 15-76633086 | cg05143116 | (cancer vs cancer) | 1-47489413 | cg00255821 | (cancer vs cancer) |
| 15-76633981 | cg20540209 | (cancer vs cancer) | 10-113943648 | cg03157698 | (cancer vs cancer) |
| 15-76634516 | cg14433497 | (cancer vs cancer) | 10-17272117 | cg20018469 | (cancer vs cancer) |
| 15-96884100 | cg07729059 | (cancer vs cancer) | 10-97804280 | cg06207432 | (cancer vs cancer) (cancer+normal vs cancer+normal) |
| 15-96888959 | cg00107682 | (cancer vs cancer) | 11-134201954 | cg07759394 | (cancer vs cancer) |
| 17-17685407 | cg25927164 | (cancer vs cancer) (cancer vs cancer) (cancer+normal vs cancer+normal) | 11-13690122 | cg02831587 | (cancer vs cancer) |
| 17-17685582 | cg15906409 | (cancer vs cancer) (cancer vs cancer) (cancer+normal vs cancer+normal) | 11-13690157 | cg26240185 | (cancer vs cancer) |
| 17-17686141 | cg19872299 | (cancer vs cancer) (cancer vs cancer) (normal vs normal) (cancer+normal vs cancer+normal) | 11-13690160 | cg03326059 | (cancer vs cancer) |
| 17-17686332 | cg01743962 | (cancer vs cancer) (cancer+normal vs | 11-13690188 | cg10572969 | (cancer vs cancer) |

| | | | | | |
|---|---|---|---|---|---|
| | | cancer+normal) | | | |
| 17-17687239 | cg04581327 | (cancer vs cancer) (cancer+normal vs cancer+normal) | 11-13690194 | cg24412501 | (cancer vs cancer) |
| 17-40932746 | cg08108311 | (cancer vs cancer) | 11-2160540 | cg18087943 | (cancer vs cancer) (cancer+normal vs cancer+normal) |
| 17-40932983 | cg23299262 | (cancer vs cancer) (cancer+normal vs cancer+normal) | 11-2160554 | cg14608156 | (cancer vs cancer) (cancer+normal vs cancer+normal) |
| 17-4544925 | cg09872233 | (cancer vs cancer) | 11-2160560 | cg12773325 | (cancer vs cancer) (cancer+normal vs cancer+normal) |
| 17-46671010 | cg16495265 | (cancer vs cancer) | 11-2160564 | cg12614029 | (cancer vs cancer) (cancer+normal vs cancer+normal) |
| 17-46671077 | cg03809346 | (cancer vs cancer) | 11-555724 | cg00716245 | (cancer vs cancer) (cancer+normal vs cancer+normal) |
| 17-46671083 | cg05085809 | (cancer vs cancer) | 11-66034896 | cg25618573 | (cancer vs cancer) |
| 17-46671131 | cg06266993 | (cancer vs cancer) | 11-66034922 | cg15201417 | (cancer vs cancer) |
| 17-46671234 | cg11155697 | (cancer vs cancer) | 11-75917404 | cg02579136 | (cancer vs cancer) (cancer+normal vs cancer+normal) |
| 17-46671244 | cg02712075 | (cancer vs cancer) (cancer+normal vs cancer+normal) | 11-75917724 | cg23400002 | (cancer vs cancer) (cancer+normal vs cancer+normal) |
| 17-46671293 | cg10153335 | (cancer vs cancer) (normal vs normal) | 11-75917844 | cg17583449 | (cancer vs cancer) (cancer+normal vs cancer+normal) |
| 17-46671298 | cg02293936 | (cancer vs cancer) (normal vs normal) | 12-100557738 | cg17316564 | (cancer vs cancer) (cancer+normal vs cancer+normal) |
| 17-46671332 | cg24626312 | (cancer vs cancer) (cancer+normal vs cancer+normal) | 12-14413090 | cg20184271 | (cancer vs cancer) (cancer+normal vs cancer+normal) |
| 17-46671497 | cg14126688 | (cancer vs cancer) | 12-14413185 | cg06872313 | (cancer vs cancer) (cancer+normal vs cancer+normal) |
| 17-48619308 | cg00698906 | (cancer vs cancer) (cancer+normal vs cancer+normal) | 12-21810489 | cg00753478 | (cancer vs cancer) |
| 17-48619672 | cg27437806 | (cancer vs cancer) (cancer vs cancer) (cancer+normal vs cancer+normal) | 12-98897216 | cg23471612 | (cancer vs cancer) (cancer+normal vs cancer+normal) |
| 17-48619753 | cg12791192 | (cancer vs cancer) (cancer+normal vs cancer+normal) | 13-107186870 | cg18771357 | (cancer vs cancer) |
| 17-75369051 | cg19554255 | (cancer vs cancer) | 13-107187412 | cg11173146 | (cancer vs cancer) |
| 17-75369055 | cg16779463 | (cancer vs cancer) (normal vs normal) | 14-105940377 | cg23411981 | (cancer vs cancer) (cancer+normal vs cancer+normal) |
| 17-75369091 | cg17300544 | (cancer vs cancer) (normal vs normal) | 14-24020053 | cg11723850 | (cancer vs cancer) |
| 17-75369219 | cg03804136 | (cancer vs cancer) (normal vs normal) | 14-51027861 | cg21107197 | (cancer vs cancer) (cancer+normal vs cancer+normal) |

| | | | | | |
|---|---|---|---|---|---|
| 17-75447478 | cg14517217 | (cancer vs cancer)<br>(cancer vs cancer)<br>(normal vs normal)<br>(cancer+normal vs cancer+normal) | 14-61188239 | cg06396724 | (cancer vs cancer) |
| 17-75447504 | cg18104645 | (cancer vs cancer)<br>(cancer vs cancer)<br>(normal vs normal)<br>(cancer+normal vs cancer+normal) | 15-45422062 | cg05804220 | (cancer vs cancer) |
| 17-75447655 | cg20955894 | (cancer vs cancer)<br>(cancer+normal vs cancer+normal) | 15-68871412 | cg15565032 | (cancer vs cancer) |
| 17-75447785 | cg11991516 | (cancer vs cancer)<br>(cancer+normal vs cancer+normal) | 15-72612221 | cg20809087 | (cancer vs cancer) |
| 17-75448089 | cg14697743 | (cancer vs cancer)<br>(cancer+normal vs cancer+normal) | 15-90544015 | cg02454890 | (cancer vs cancer)<br>(cancer+normal vs cancer+normal) |
| 17-8055733 | cg07592976 | (cancer vs cancer)<br>(cancer+normal vs cancer+normal) | 15-99193466 | cg22375192 | (cancer vs cancer) |
| 17-8055756 | cg23363911 | (cancer vs cancer)<br>(cancer vs cancer)<br>(cancer+normal vs cancer+normal) | 16-30389104 | cg07506299 | (cancer vs cancer) |
| 17-8055831 | cg14158601 | (cancer vs cancer)<br>(cancer+normal vs cancer+normal) | 17-80291775 | cg02553663 | (cancer vs cancer) |
| 17-8055834 | cg21093368 | (cancer vs cancer)<br>(cancer+normal vs cancer+normal) | 18-12407797 | cg07836661 | (cancer vs cancer) |
| 17-8055888 | cg16545079 | (cancer vs cancer)<br>(cancer+normal vs cancer+normal) | 19-10172825 | cg07749412 | (cancer vs cancer)<br>(cancer+normal vs cancer+normal) |
| 19-18811575 | cg07614018 | (cancer vs cancer) | 19-18210017 | cg06905726 | (cancer vs cancer)<br>(cancer+normal vs cancer+normal) |
| 19-45656942 | cg21574675 | (cancer vs cancer)<br>(cancer vs cancer)<br>(cancer+normal vs cancer+normal) | 19-24269890 | cg09777776 | (cancer vs cancer)<br>(cancer+normal vs cancer+normal) |
| 19-45657270 | cg24122218 | (cancer vs cancer)<br>(cancer+normal vs cancer+normal) | 19-24270468 | cg01000657 | (cancer vs cancer)<br>(cancer+normal vs cancer+normal) |
| 19-59074005 | cg05014211 | (cancer vs cancer)<br>(cancer+normal vs cancer+normal) | 19-36389833 | cg27297851 | (cancer vs cancer)<br>(cancer+normal vs cancer+normal) |
| 19-59074308 | cg08447733 | (cancer vs cancer)<br>(cancer+normal vs cancer+normal) | 19-42901325 | cg17409893 | (cancer vs cancer) |
| 19-59074482 | cg17735983 | (cancer vs cancer)<br>(cancer+normal vs cancer+normal) | 19-47776728 | cg25253217 | (cancer vs cancer) |
| 19-9903777 | cg06458554 | (cancer vs cancer)<br>(cancer+normal vs cancer+normal) | 19-50096610 | cg15032314 | (cancer vs cancer)<br>(cancer+normal vs cancer+normal) |
| 2-20866107 | cg25160978 | (cancer vs cancer) | 19-54369436 | cg06665109 | (cancer vs cancer)<br>(cancer+normal vs cancer+normal) |
| 2- | cg09150450 | (cancer vs cancer) | 19-6740812 | cg2207697 | (cancer vs cancer) |

| | | | | | |
|---|---|---|---|---|---|
| 20866178 | | (cancer+normal vs cancer+normal) | | 2 | (cancer+normal vs cancer+normal) |
| 2-20866231 | cg21039778 | (cancer vs cancer) (cancer+normal vs cancer+normal) | 19-6740888 | cg13341720 | (cancer vs cancer) (cancer+normal vs cancer+normal) |
| 2-20866242 | cg19113641 | (cancer vs cancer) (cancer+normal vs cancer+normal) | 19-6740952 | cg03705926 | (cancer vs cancer) (cancer+normal vs cancer+normal) |
| 2-20866261 | cg12266953 | (cancer vs cancer) (cancer+normal vs cancer+normal) | 2-202899877 | cg23246885 | (cancer vs cancer) |
| 2-45162196 | cg06168026 | (cancer vs cancer) | 2-233792394 | cg08165971 | (cancer vs cancer) (cancer+normal vs cancer+normal) |
| 2-45162467 | cg21236315 | (cancer vs cancer) | 2-233793120 | cg06828015 | (cancer vs cancer) (cancer+normal vs cancer+normal) |
| 20-34189411 | cg13544006 | (cancer vs cancer) (normal vs normal) | 2-264120 | cg10181419 | (cancer vs cancer) (cancer+normal vs cancer+normal) |
| 22-21319245 | cg16590005 | (cancer vs cancer) (cancer vs cancer) (cancer+normal vs cancer+normal) | 2-264164 | cg03468349 | (cancer vs cancer) |
| 22-21319303 | cg10348922 | (cancer vs cancer) (cancer+normal vs cancer+normal) | 2-264166 | cg21825027 | (cancer vs cancer) |
| 22-21319661 | cg07960806 | (cancer vs cancer) (cancer vs cancer) (cancer+normal vs cancer+normal) | 2-264178 | cg20749741 | (cancer vs cancer) (cancer+normal vs cancer+normal) |
| 22-21319668 | cg11782550 | (cancer vs cancer) (cancer+normal vs cancer+normal) | 2-264199 | cg00108164 | (cancer vs cancer) (cancer+normal vs cancer+normal) |
| 3-128217091 | cg24954207 | (cancer vs cancer) | 2-264204 | cg25945732 | (cancer vs cancer) (cancer+normal vs cancer+normal) |
| 3-187457732 | cg10072850 | (cancer vs cancer) (cancer+normal vs cancer+normal) | 2-74425523 | cg16367511 | (cancer vs cancer) (cancer+normal vs cancer+normal) |
| 5-125930496 | cg05689600 | (cancer vs cancer) (cancer+normal vs cancer+normal) | 2-74425580 | cg13755546 | (cancer vs cancer) (cancer+normal vs cancer+normal) |
| 5-125930667 | cg01814098 | (cancer vs cancer) (cancer+normal vs cancer+normal) | 2-74425749 | cg09868451 | (cancer vs cancer) |
| 5-125931001 | cg19551232 | (cancer vs cancer) (cancer+normal vs cancer+normal) | 2-9144629 | cg07960067 | (cancer vs cancer) (cancer+normal vs cancer+normal) |
| 5-125931120 | cg26327732 | (cancer vs cancer) (cancer+normal vs cancer+normal) | 20-825240 | cg20364050 | (cancer vs cancer) |
| 5-125931166 | cg16738971 | (cancer vs cancer) (cancer+normal vs cancer+normal) | 20-825268 | cg20353496 | (cancer vs cancer) |
| 5-151150340 | cg13373406 | (cancer vs cancer) (cancer+normal vs cancer+normal) | 20-825408 | cg19424265 | (cancer vs cancer) (cancer+normal vs cancer+normal) |
| 5-151150431 | cg20775840 | (cancer vs cancer) | 22-36236303 | cg25488206 | (cancer vs cancer) (cancer+normal vs cancer+normal) |

| | | | | | |
|---|---|---|---|---|---|
| 5-151150581 | cg00977110 | (cancer vs cancer) (cancer+normal vs cancer+normal) | 3-119041529 | cg0707454 3 | (cancer vs cancer) |
| 5-157098338 | cg18375421 | (cancer vs cancer) | 3-183542914 | cg1272737 4 | (cancer vs cancer) (normal vs normal) (cancer+normal vs cancer+normal) |
| 5-171384415 | cg25959472 | (cancer vs cancer) (cancer+normal vs cancer+normal) | 3-183543564 | cg1915500 7 | (cancer vs cancer) (cancer+normal vs cancer+normal) |
| 5-42995203 | cg18161950 | (cancer vs cancer) | 3-183543587 | cg0973815 6 | (cancer vs cancer) (normal vs normal) (cancer+normal vs cancer+normal) |
| 5-76011304 | cg14718568 | (cancer vs cancer) | 3-183543704 | cg1034703 2 | (cancer vs cancer) |
| 5-76011323 | cg14773260 | (cancer vs cancer) | 3-197281468 | cg0303868 5 | (cancer vs cancer) (cancer+normal vs cancer+normal) |
| 6-106958303 | cg04067276 | (cancer vs cancer) (cancer+normal vs cancer+normal) | 3-197281934 | cg0379276 8 | (cancer vs cancer) (cancer+normal vs cancer+normal) |
| 6-106958388 | cg23413349 | (cancer vs cancer) (normal vs normal) (cancer+normal vs cancer+normal) | 3-49757016 | cg2472211 2 | (cancer vs cancer) (cancer+normal vs cancer+normal) |
| 6-106958474 | cg09184502 | (cancer vs cancer) (cancer+normal vs cancer+normal) | 3-49757239 | cg2575510 7 | (cancer vs cancer) (cancer+normal vs cancer+normal) |
| 6-106958640 | cg25944720 | (cancer vs cancer) (cancer+normal vs cancer+normal) | 3-49757306 | cg0443259 7 | (cancer vs cancer) (cancer+normal vs cancer+normal) |
| 6-106958645 | cg22231056 | (cancer vs cancer) (cancer+normal vs cancer+normal) | 5-176831297 | cg1412649 3 | (cancer vs cancer) (cancer+normal vs cancer+normal) |
| 6-28911468 | cg01742627 | (cancer vs cancer) (cancer+normal vs cancer+normal) | 5-176831638 | cg0587686 4 | (cancer vs cancer) (cancer+normal vs cancer+normal) |
| 6-28911474 | cg05127899 | (cancer vs cancer) (cancer+normal vs cancer+normal) | 6-158957433 | cg0059003 6 | (cancer vs cancer) (cancer+normal vs cancer+normal) |
| 6-28911759 | cg23326313 | (cancer vs cancer) (cancer+normal vs cancer+normal) | 6-24360304 | cg1106834 3 | (cancer vs cancer) |
| 6-28911926 | cg15088539 | (cancer vs cancer) (cancer+normal vs cancer+normal) | 6-24360391 | cg0703641 2 | (cancer vs cancer) |
| 6-28921469 | cg17372996 | (cancer vs cancer) (cancer+normal vs cancer+normal) | 6-33244755 | cg1722512 9 | (cancer vs cancer) (normal vs normal) |
| 6-28921485 | cg08827322 | (cancer vs cancer) (cancer+normal vs cancer+normal) | 6-43044239 | cg2599868 0 | (cancer vs cancer) (cancer+normal vs cancer+normal) |
| 6-28921499 | cg09633604 | (cancer vs cancer) (cancer+normal vs cancer+normal) | 6-43044771 | cg2166358 0 | (cancer vs cancer) (cancer+normal vs cancer+normal) |
| 7-193262 | cg08782337 | (cancer vs cancer) | 7-76624761 | cg1923667 5 | (cancer vs cancer) |
| 7-65878911 | cg14428815 | (cancer vs cancer) (cancer+normal vs cancer+normal) | 8-142318170 | cg0165751 1 | (cancer vs cancer) |

| | | | | | |
|---|---|---|---|---|---|
| 8-1949246 | cg09689137 | (cancer vs cancer) | 8-22457446 | cg0222786 7 | (cancer vs cancer) (cancer+normal vs cancer+normal) |
| 8-1949342 | cg25021970 | (cancer vs cancer) | 9-131219302 | cg1415312 6 | (cancer vs cancer) (cancer+normal vs cancer+normal) |
| **Status - Hypo** | | | **Status - Hypo** | | |
| 1-1358109 | cg12286462 | (cancer vs cancer) | 1-11107048 | cg1288811 3 | (cancer vs cancer) (cancer+normal vs cancer+normal) |
| 1-231174153 | cg16908552 | (cancer vs cancer) (cancer+normal vs cancer+normal) | 1-167882440 | cg2446133 7 | (cancer vs cancer) (cancer+normal vs cancer+normal) |
| 1-23520083 | cg11811391 | (cancer vs cancer) (cancer+normal vs cancer+normal) | 1-167883298 | cg2685966 6 | (cancer vs cancer) (cancer+normal vs cancer+normal) |
| 1-236557682 | cg09809672 | (cancer vs cancer) | 1-171059935 | cg2577816 6 | (cancer vs cancer) (cancer+normal vs cancer+normal) |
| 1-246930969 | cg13187885 | (normal vs normal) (cancer vs cancer) (cancer+normal vs cancer+normal) | 1-171060071 | cg1806314 9 | (normal vs normal) (cancer vs cancer) (cancer+normal vs cancer+normal) |
| 1-6134338 | cg24446610 | (cancer vs cancer) | 1-173886454 | cg1733065 2 | (cancer vs cancer) (cancer+normal vs cancer+normal) |
| 1-7180862 | cg09148344 | (cancer vs cancer) | 1-179059407 | cg1759157 4 | (cancer vs cancer) (cancer+normal vs cancer+normal) |
| 1-8384659 | cg10508347 | (cancer vs cancer) (cancer+normal vs cancer+normal) | 1-207277018 | cg2233227 6 | (cancer vs cancer) (cancer+normal vs cancer+normal) |
| 1-95289024 | cg18396714 | (cancer vs cancer) (cancer+normal vs cancer+normal) | 1-207277031 | cg1115396 9 | (cancer vs cancer) (cancer+normal vs cancer+normal) |
| 10-14014548 | cg04819048 | (cancer vs cancer) | 1-209406266 | cg0263168 4 | (cancer vs cancer) |
| 10-43849970 | cg23033014 | (cancer vs cancer) (cancer+normal vs cancer+normal) | 1-209847618 | cg2364637 5 | (cancer vs cancer) (cancer+normal vs cancer+normal) |
| 10-5504438 | cg01275661 | (cancer vs cancer) (cancer+normal vs cancer+normal) | 1-241795170 | cg0491883 1 | (cancer vs cancer) (cancer+normal vs cancer+normal) |
| 10-88719950 | cg20459238 | (cancer vs cancer) (cancer vs cancer) (cancer+normal vs cancer+normal) | 1-245812674 | cg0156529 1 | (cancer vs cancer) |
| 11-102339610 | cg08547848 | (cancer vs cancer) (cancer+normal vs cancer+normal) | 1-95584445 | cg2193267 2 | (cancer vs cancer) (cancer+normal vs cancer+normal) |
| 11-111311280 | cg17256234 | (cancer vs cancer) (cancer+normal vs cancer+normal) | 10-128275008 | cg1411532 5 | (cancer vs cancer) |
| 11-4010752 | cg14849140 | (cancer vs cancer) (cancer+normal vs cancer+normal) | 10-1334207 | cg0499297 4 | (cancer vs cancer) |
| 11-71229642 | cg03614381 | (normal vs normal) (cancer vs cancer) (cancer+normal vs cancer+normal) | 10-1576684 | cg0676438 7 | (cancer vs cancer) |
| 12- | cg21575634 | (cancer vs cancer) | 10-1714535 | cg2498004 | (cancer vs cancer) |

| | | | | | |
|---|---|---|---|---|---|
| 131310417 | | | | 0 | |
| 12-23999045 | cg11243173 | (cancer vs cancer) | 10-4500136 | cg16873334 | (cancer vs cancer) |
| 12-47471907 | cg18436898 | (cancer vs cancer) (cancer vs cancer) (cancer+normal vs cancer+normal) | 10-8546509 | cg11875044 | (normal vs normal) (cancer vs cancer) (cancer+normal vs cancer+normal) |
| 12-90313589 | cg00015699 | (cancer vs cancer) (cancer+normal vs cancer+normal) | 10-93390347 | cg24288527 | (cancer vs cancer) (cancer+normal vs cancer+normal) |
| 12-90313611 | cg20490773 | (cancer vs cancer) (cancer+normal vs cancer+normal) | 11-102638470 | cg03367136 | (cancer vs cancer) |
| 13-113301717 | cg08405286 | (cancer vs cancer) | 11-13700098 | cg14350120 | (cancer vs cancer) |
| 13-114187973 | cg22612590 | (cancer vs cancer) (cancer+normal vs cancer+normal) | 11-46740481 | cg00371195 | (cancer vs cancer) (cancer+normal vs cancer+normal) |
| 13-114187990 | cg13241681 | (cancer vs cancer) (cancer+normal vs cancer+normal) | 11-46741373 | cg26453360 | (cancer vs cancer) (cancer+normal vs cancer+normal) |
| 13-114188051 | cg20539175 | (cancer vs cancer) (cancer+normal vs cancer+normal) | 11-46741405 | cg09004691 | (cancer vs cancer) (cancer+normal vs cancer+normal) |
| 15-65042722 | cg22423363 | (normal vs normal) (cancer vs cancer) (cancer vs cancer) (cancer+normal vs cancer+normal) | 11-6462110 | cg14164596 | (cancer vs cancer) (cancer+normal vs cancer+normal) |
| 16-1576146 | cg27316811 | (normal vs normal) (cancer vs cancer) (cancer+normal vs cancer+normal) | 12-69199037 | cg10635494 | (normal vs normal) (cancer vs cancer) (cancer+normal vs cancer+normal) |
| 16-29204994 | cg27587310 | (cancer vs cancer) (cancer+normal vs cancer+normal) | 15-99427016 | cg03310087 | (cancer vs cancer) |
| 16-51197459 | cg02705762 | (cancer vs cancer) | 16-20709238 | cg08879255 | (cancer vs cancer) (cancer+normal vs cancer+normal) |
| 16-85289758 | cg02576153 | (cancer vs cancer) | 16-87970367 | cg05640992 | (cancer vs cancer) (cancer+normal vs cancer+normal) |
| 17-25619946 | cg07564598 | (cancer vs cancer) (cancer+normal vs cancer+normal) | 16-87970376 | cg03550864 | (cancer vs cancer) (cancer+normal vs cancer+normal) |
| 17-56569119 | cg25504605 | (cancer vs cancer) (cancer+normal vs cancer+normal) | 17-26694939 | cg18536123 | (cancer vs cancer) (cancer+normal vs cancer+normal) |
| 17-75237550 | cg02746913 | (normal vs normal) (cancer vs cancer) (cancer+normal vs cancer+normal) | 17-27370988 | cg16608731 | (cancer vs cancer) (cancer+normal vs cancer+normal) |
| 17-767301 | cg13476078 | (normal vs normal) (cancer vs cancer) (cancer vs cancer) (cancer+normal vs cancer+normal) | 17-39344868 | cg24802078 | (cancer vs cancer) |
| 17-767334 | cg25236028 | (normal vs normal) (cancer vs cancer) (cancer+normal vs cancer+normal) | 17-51900887 | cg19720780 | (cancer vs cancer) |

| | | | | | |
|---|---|---|---|---|---|
| 17-767380 | cg13120258 | (cancer vs cancer) (cancer+normal vs cancer+normal) | 17-7019284 | cg18964954 | (cancer vs cancer) (cancer+normal vs cancer+normal) |
| 17-78035629 | cg07724971 | (cancer vs cancer) (cancer+normal vs cancer+normal) | 17-71547405 | cg16916549 | (cancer vs cancer) |
| 18-3411821 | cg12931591 | (cancer vs cancer) | 18-77201972 | cg02714192 | (cancer vs cancer) |
| 19-39052032 | cg19338222 | (cancer vs cancer) (cancer vs cancer) (cancer+normal vs cancer+normal) | 19-45449006 | cg27436184 | (cancer vs cancer) (cancer+normal vs cancer+normal) |
| 19-39084308 | cg19786121 | (cancer vs cancer) (cancer+normal vs cancer+normal) | 2-113884447 | cg23041410 | (cancer vs cancer) (cancer+normal vs cancer+normal) |
| 19-41190396 | cg18668041 | (cancer vs cancer) (cancer+normal vs cancer+normal) | 2-21267334 | cg26112457 | (cancer vs cancer) (cancer+normal vs cancer+normal) |
| 19-8669959 | cg21798669 | (cancer vs cancer) | 2-234627450 | cg04437648 | (cancer vs cancer) (cancer+normal vs cancer+normal) |
| 19-8670086 | cg11745755 | (cancer vs cancer) (cancer+normal vs cancer+normal) | 2-44065725 | cg20926720 | (cancer vs cancer) (cancer+normal vs cancer+normal) |
| 2-10686963 | cg15153684 | (cancer vs cancer) | 2-44066294 | cg19123339 | (cancer vs cancer) (cancer+normal vs cancer+normal) |
| 2-219256018 | cg23212579 | (cancer vs cancer) (cancer vs cancer) (cancer+normal vs cancer+normal) | 2-44067042 | cg00760272 | (cancer vs cancer) (cancer+normal vs cancer+normal) |
| 2-219256101 | cg16926316 | (cancer vs cancer) (cancer+normal vs cancer+normal) | 2-451875 | cg07919936 | (cancer vs cancer) |
| 2-242868376 | cg10397934 | (cancer vs cancer) (cancer+normal vs cancer+normal) | 2-46707678 | cg08279055 | (cancer vs cancer) |
| 2-24584023 | cg13279009 | (cancer vs cancer) | 2-503193 | cg11573608 | (cancer vs cancer) |
| 2-25427108 | cg20422417 | (cancer vs cancer) (cancer+normal vs cancer+normal) | 2-60812640 | cg03230133 | (cancer vs cancer) |
| 2-68672750 | cg22493364 | (cancer vs cancer) | 20-56227286 | cg26042618 | (cancer vs cancer) |
| 2-75078286 | cg11672772 | (cancer vs cancer) (cancer vs cancer) (cancer+normal vs cancer+normal) | 21-37852822 | cg15310162 | (cancer vs cancer) (cancer+normal vs cancer+normal) |
| 2-84517763 | cg22037978 | (cancer vs cancer) | 21-47575134 | cg04413147 | (cancer vs cancer) (cancer+normal vs cancer+normal) |
| 2-95939339 | cg24560809 | (cancer vs cancer) (cancer+normal vs cancer+normal) | 22-21128411 | cg09634469 | (cancer vs cancer) (cancer+normal vs cancer+normal) |
| 20-31446363 | cg16208206 | (cancer vs cancer) (cancer+normal vs cancer+normal) | 22-24891666 | cg04033580 | (cancer vs cancer) (cancer+normal vs cancer+normal) |
| 20-32316843 | cg04772817 | (cancer vs cancer) (cancer+normal vs cancer+normal) | 22-41630982 | cg18659483 | (cancer vs cancer) (cancer+normal vs cancer+normal) |
| 20- | cg24332577 | (cancer vs cancer) | 22- | cg0736655 | (cancer vs cancer) |

| | | | | | |
|---|---|---|---|---|---|
| 50419248 | | (cancer+normal vs cancer+normal) | 50644468 | 3 | (cancer+normal vs cancer+normal) |
| 20-50419274 | cg12288473 | (cancer vs cancer) (cancer+normal vs cancer+normal) | 22-50644682 | cg21874862 | (cancer vs cancer) (cancer+normal vs cancer+normal) |
| 20-50419348 | cg10941835 | (cancer vs cancer) | 22-50644755 | cg25934700 | (cancer vs cancer) (cancer+normal vs cancer+normal) |
| 20-56076737 | cg25169524 | (cancer vs cancer) (cancer+normal vs cancer+normal) | 3-142681704 | cg26301689 | (cancer vs cancer) (cancer+normal vs cancer+normal) |
| 20-60510235 | cg11853697 | (cancer vs cancer) (cancer+normal vs cancer+normal) | 4-1198278 | cg21380181 | (cancer vs cancer) (cancer+normal vs cancer+normal) |
| 21-46323665 | cg27019201 | (cancer vs cancer) (cancer vs cancer) | 4-30745743 | cg16895026 | (cancer vs cancer) |
| 21-46896174 | cg13180920 | (cancer vs cancer) (cancer+normal vs cancer+normal) | 4-80329483 | cg15063695 | (cancer vs cancer) |
| 21-46912506 | cg25390243 | (cancer vs cancer) | 4-80329486 | cg26226487 | (cancer vs cancer) |
| 3-12332780 | cg16827534 | (cancer vs cancer) (cancer+normal vs cancer+normal) | 5-139939823 | cg19616807 | (cancer vs cancer) (cancer+normal vs cancer+normal) |
| 3-184052502 | cg03513893 | (cancer vs cancer) (cancer+normal vs cancer+normal) | 5-141346431 | cg04182865 | (cancer vs cancer) (cancer+normal vs cancer+normal) |
| 3-186559147 | cg14584085 | (cancer vs cancer) | 5-142187317 | cg01204911 | (cancer vs cancer) (cancer+normal vs cancer+normal) |
| 3-194573301 | cg10985281 | (cancer vs cancer) (cancer vs cancer) (cancer+normal vs cancer+normal) | 5-16794882 | cg12852139 | (cancer vs cancer) |
| 3-194573323 | cg07206256 | (cancer vs cancer) (cancer+normal vs cancer+normal) | 5-17147858 | cg20941894 | (cancer vs cancer) |
| 3-45801044 | cg01451880 | (cancer vs cancer) | 5-17275783 | cg01860297 | (cancer vs cancer) |
| 4-140969039 | cg15844438 | (cancer vs cancer) | 5-176836695 | cg06625767 | (cancer vs cancer) (cancer+normal vs cancer+normal) |
| 4-141188341 | cg06775361 | (cancer vs cancer) (cancer+normal vs cancer+normal) | 5-178209759 | cg06651605 | (cancer vs cancer) |
| 4-3480656 | cg13455623 | (cancer vs cancer) (cancer+normal vs cancer+normal) | 5-4644304 | cg21944740 | (cancer vs cancer) |
| 5-180219707 | cg18114890 | (cancer vs cancer) (cancer+normal vs cancer+normal) | 6-168970787 | cg13517866 | (normal vs normal) (cancer vs cancer) |
| 5-9557609 | cg23920603 | (cancer vs cancer) (cancer+normal vs cancer+normal) | 6-27185676 | cg09817162 | (cancer vs cancer) |
| 6-106582638 | cg17356964 | (cancer vs cancer) (cancer+normal vs cancer+normal) | 6-29588988 | cg18126978 | (normal vs normal) (cancer vs cancer) |
| 6-106582669 | cg25581448 | (cancer vs cancer) (cancer vs cancer) (cancer+normal vs cancer+normal) | 6-29589545 | cg25437807 | (cancer vs cancer) |

| | | | | | |
|---|---|---|---|---|---|
| 6-167189272 | cg03329755 | (cancer vs cancer) (cancer vs cancer) (cancer+normal vs cancer+normal) | 6-29589631 | cg01373089 | (cancer vs cancer) |
| 6-168594903 | cg10896609 | (cancer vs cancer) (cancer vs cancer) | 6-29590143 | cg17071948 | (cancer vs cancer) |
| 6-168613308 | cg05418218 | (cancer vs cancer) | 6-30168380 | cg03781123 | (cancer vs cancer) (cancer+normal vs cancer+normal) |
| 6-18368796 | cg23221431 | (cancer vs cancer) (cancer+normal vs cancer+normal) | 6-72298704 | cg14389840 | (cancer vs cancer) |
| 6-18368844 | cg14645856 | (cancer vs cancer) (cancer+normal vs cancer+normal) | 7-1197113 | cg03923535 | (cancer vs cancer) (cancer+normal vs cancer+normal) |
| 6-24429162 | cg23562023 | (cancer vs cancer) (cancer+normal vs cancer+normal) | 7-20269648 | cg19086110 | (cancer vs cancer) |
| 6-32112546 | cg18572200 | (cancer vs cancer) | 7-2959105 | cg13106306 | (cancer vs cancer) |
| 6-33996013 | cg05745748 | (cancer vs cancer) | 7-2959121 | cg06830441 | (cancer vs cancer) |
| 6-45891455 | cg18434367 | (cancer vs cancer) | 7-3849359 | cg05529686 | (cancer vs cancer) |
| 6-47202394 | cg21552001 | (cancer vs cancer) (cancer vs cancer) (cancer+normal vs cancer+normal) | 7-4012372 | cg04609841 | (cancer vs cancer) |
| 7-143220 | cg19755314 | (cancer vs cancer) | 7-4014070 | cg17176209 | (cancer vs cancer) |
| 7-151553819 | cg24251111 | (cancer vs cancer) | 7-4030038 | cg21053935 | (cancer vs cancer) |
| 7-158596552 | cg23730037 | (cancer vs cancer) (cancer+normal vs cancer+normal) | 7-4030413 | cg04358834 | (cancer vs cancer) |
| 7-170692 | cg19210140 | (cancer vs cancer) (cancer+normal vs cancer+normal) | 7-4118609 | cg01310605 | (cancer vs cancer) |
| 7-1932321 | cg23618217 | (cancer vs cancer) (cancer+normal vs cancer+normal) | 7-42446248 | cg19596050 | (cancer vs cancer) |
| 7-1961869 | cg15997393 | (cancer vs cancer) (cancer+normal vs cancer+normal) | 7-80544479 | cg15556990 | (cancer vs cancer) (cancer+normal vs cancer+normal) |
| 7-210075 | cg12458039 | (cancer vs cancer) (cancer vs cancer) (cancer+normal vs cancer+normal) | 7-80682238 | cg04264129 | (cancer vs cancer) |
| 7-28848902 | cg19588408 | (cancer vs cancer) | 7-82286378 | cg03644984 | (cancer vs cancer) |
| 7-28848933 | cg19559984 | (cancer vs cancer) | 7-895646 | cg25151376 | (cancer vs cancer) (cancer+normal vs cancer+normal) |
| 7-36959356 | cg12570246 | (cancer vs cancer) | 7-99637061 | cg04193037 | (cancer vs cancer) (cancer+normal vs cancer+normal) |
| 8-123966105 | cg01886570 | (normal vs normal) (cancer vs cancer) (cancer vs cancer) (cancer+normal vs cancer+normal) | 8-142263802 | cg00971369 | (cancer vs cancer) (cancer+normal vs cancer+normal) |

| | | | | | | |
|---|---|---|---|---|---|---|
| 8-59259110 | cg21287347 | (cancer vs cancer) | 8-61086268 | cg25611796 | (cancer vs cancer) | |
| 9-4294020 | cg13573115 | (cancer vs cancer) | 9-135393396 | cg14633268 | (cancer vs cancer) | |
| **(cancer+normal vs cancer+normal)** | | | **(cancer+normal vs cancer+normal)** | | | |
| **Status - Hyper** | | | **Status - Hyper** | | | |
| 1-152943306 | cg19602452 | (cancer+normal vs cancer+normal) | 1-145395607 | cg12420472 | (cancer vs cancer) (cancer+normal vs cancer+normal) | |
| 1-26662567 | cg23053573 | (cancer vs cancer) (cancer+normal vs cancer+normal) | 1-145395753 | cg17952661 | (cancer vs cancer) (normal vs normal) (cancer+normal vs cancer+normal) | |
| 1-27050491 | cg22042546 | (cancer vs cancer) (cancer+normal vs cancer+normal) | 1-145395846 | cg02395363 | (cancer vs cancer) (cancer+normal vs cancer+normal) | |
| 1-29586414 | cg04933208 | (normal vs normal) (cancer+normal vs cancer+normal) | 1-145455384 | cg21961202 | (cancer vs cancer) (cancer+normal vs cancer+normal) | |
| 1-29586418 | cg26884027 | (cancer+normal vs cancer+normal) | 1-206730839 | cg23520347 | (cancer vs cancer) (cancer+normal vs cancer+normal) | |
| 1-46782408 | cg02852174 | (cancer+normal vs cancer+normal) | 1-21616619 | cg17216478 | (cancer vs cancer) (cancer+normal vs cancer+normal) | |
| 10-135206831 | cg05676341 | (cancer+normal vs cancer+normal) (cancer+normal vs cancer+normal) | 1-21616641 | cg06819200 | (cancer vs cancer) (cancer+normal vs cancer+normal) | |
| 10-22518317 | cg15900657 | (cancer vs cancer) (cancer+normal vs cancer+normal) | 1-24645853 | cg20242797 | (cancer+normal vs cancer+normal) | |
| 11-119235062 | cg20822818 | (cancer+normal vs cancer+normal) | 1-24645885 | cg21289280 | (cancer vs cancer) (cancer+normal vs cancer+normal) | |
| 11-27015592 | cg02565993 | (cancer+normal vs cancer+normal) | 1-24645917 | cg04552500 | (cancer+normal vs cancer+normal) | |
| 11-35440525 | cg09017174 | (cancer vs cancer) (cancer+normal vs cancer+normal) | 1-65211677 | cg01002030 | (cancer+normal vs cancer+normal) | |
| 12-114834713 | cg06875424 | (cancer+normal vs cancer+normal) | 10-106028543 | cg16071118 | (cancer+normal vs cancer+normal) | |
| 12-114836014 | cg04562909 | (cancer+normal vs cancer+normal) | 10-76584710 | cg18462764 | (cancer+normal vs cancer+normal) | |
| 12-114840942 | cg23820885 | (normal vs normal) (cancer+normal vs cancer+normal) | 10-97804280 | cg06207432 | (cancer vs cancer) (cancer+normal vs cancer+normal) | |
| 12-114841202 | cg04685570 | (cancer+normal vs cancer+normal) | 11-2160540 | cg18087943 | (cancer vs cancer) (cancer+normal vs cancer+normal) | |
| 12-114841792 | cg22045225 | (cancer+normal vs cancer+normal) | 11-2160554 | cg14608156 | (cancer vs cancer) (cancer+normal vs cancer+normal) | |
| 12-114852027 | cg12437821 | (normal vs normal) (cancer+normal vs cancer+normal) | 11-2160560 | cg12773325 | (cancer vs cancer) (cancer+normal vs cancer+normal) | |
| 13-110437561 | cg10488031 | (cancer+normal vs cancer+normal) | 11-2160564 | cg12614029 | (cancer vs cancer) (cancer+normal vs cancer+normal) | |
| 15- | cg06650067 | (cancer+normal vs | 11-2160904 | cg1900233 | (cancer+normal vs | |

| | | | | | |
|---|---|---|---|---|---|
| 66084924 | | cancer+normal) | | 7 | cancer+normal) |
| 16-87988631 | cg02331262 | (cancer+normal vs cancer+normal) | 11-2160932 | cg24366657 | (cancer+normal vs cancer+normal) |
| 16-89654085 | cg10499753 | (cancer+normal vs cancer+normal) | 11-2165136 | cg04112019 | (cancer+normal vs cancer+normal) |
| 17-17685407 | cg25927164 | (cancer vs cancer) (cancer vs cancer) (cancer+normal vs cancer+normal) | 11-555724 | cg00716245 | (cancer vs cancer) (cancer+normal vs cancer+normal) |
| 17-17685582 | cg15906409 | (cancer vs cancer) (cancer vs cancer) (cancer+normal vs cancer+normal) | 11-68780866 | cg03522668 | (cancer+normal vs cancer+normal) |
| 17-17686141 | cg19872299 | (cancer vs cancer) (cancer vs cancer) (normal vs normal) (cancer+normal vs cancer+normal) | 11-75917404 | cg02579136 | (cancer vs cancer) (cancer+normal vs cancer+normal) |
| 17-17686332 | cg01743962 | (cancer vs cancer) (cancer+normal vs cancer+normal) | 11-75917724 | cg23400002 | (cancer vs cancer) (cancer+normal vs cancer+normal) |
| 17-17687239 | cg04581327 | (cancer vs cancer) (cancer+normal vs cancer+normal) | 11-75917844 | cg17583449 | (cancer vs cancer) (cancer+normal vs cancer+normal) |
| 17-17726759 | cg10415767 | (cancer+normal vs cancer+normal) | 11-75945841 | cg10624860 | (cancer+normal vs cancer+normal) |
| 17-40932953 | cg23103688 | (cancer+normal vs cancer+normal) | 12-100557738 | cg17316564 | (cancer vs cancer) (cancer+normal vs cancer+normal) |
| 17-40932983 | cg23299262 | (cancer vs cancer) (cancer+normal vs cancer+normal) | 12-133137879 | cg01805480 | (cancer+normal vs cancer+normal) |
| 17-46671244 | cg02712075 | (cancer vs cancer) (cancer+normal vs cancer+normal) | 12-14413090 | cg20184271 | (cancer vs cancer) (cancer+normal vs cancer+normal) |
| 17-46671332 | cg24626312 | (cancer vs cancer) (cancer+normal vs cancer+normal) | 12-14413185 | cg06872313 | (cancer vs cancer) (cancer+normal vs cancer+normal) |
| 17-48619308 | cg00698906 | (cancer vs cancer) (cancer+normal vs cancer+normal) | 12-98897187 | cg02584193 | (cancer+normal vs cancer+normal) |
| 17-48619672 | cg27437806 | (cancer vs cancer) (cancer vs cancer) (cancer+normal vs cancer+normal) | 12-98897216 | cg23471612 | (cancer vs cancer) (cancer+normal vs cancer+normal) |
| 17-48619753 | cg12791192 | (cancer vs cancer) (cancer+normal vs cancer+normal) | 13-50570250 | cg12368542 | (cancer+normal vs cancer+normal) |
| 17-55474945 | cg18783374 | (cancer+normal vs cancer+normal) | 14-105940377 | cg23411981 | (cancer vs cancer) (cancer+normal vs cancer+normal) |
| 17-58468300 | cg00691729 | (cancer+normal vs cancer+normal) | 14-51027861 | cg21107197 | (cancer vs cancer) (cancer+normal vs cancer+normal) |
| 17-68166021 | cg18673401 | (cancer+normal vs cancer+normal) | 15-90208810 | cg05009389 | (cancer+normal vs cancer+normal) |
| 17-75447478 | cg14517217 | (cancer vs cancer) (cancer vs cancer) (normal vs normal) (cancer+normal vs cancer+normal) | 15-90208915 | cg26703661 | (cancer+normal vs cancer+normal) |

| | | | | | |
|---|---|---|---|---|---|
| 17-75447504 | cg18104645 | (cancer vs cancer) (cancer vs cancer) (normal vs normal) (cancer+normal vs cancer+normal) | 15-90544015 | cg02454890 | (cancer vs cancer) (cancer+normal vs cancer+normal) |
| 17-75447655 | cg20955894 | (cancer vs cancer) (cancer+normal vs cancer+normal) | 16-30671644 | cg04232972 | (cancer+normal vs cancer+normal) |
| 17-75447785 | cg11991516 | (cancer vs cancer) (cancer+normal vs cancer+normal) | 16-30935822 | cg04477789 | (cancer+normal vs cancer+normal) |
| 17-75448089 | cg14697743 | (cancer vs cancer) (cancer+normal vs cancer+normal) | 16-69760564 | cg13969273 | (cancer+normal vs cancer+normal) |
| 17-8055733 | cg07592976 | (cancer vs cancer) (cancer+normal vs cancer+normal) | 17-38226278 | cg20717932 | (cancer+normal vs cancer+normal) |
| 17-8055756 | cg23363911 | (cancer vs cancer) (cancer vs cancer) (cancer+normal vs cancer+normal) | 19-10172712 | cg02317055 | (cancer+normal vs cancer+normal) |
| 17-8055831 | cg14158601 | (cancer vs cancer) (cancer+normal vs cancer+normal) | 19-10172825 | cg07749412 | (cancer vs cancer) (cancer+normal vs cancer+normal) |
| 17-8055834 | cg21093368 | (cancer vs cancer) (cancer+normal vs cancer+normal) | 19-18210017 | cg06905726 | (cancer vs cancer) (cancer+normal vs cancer+normal) |
| 17-8055888 | cg16545079 | (cancer vs cancer) (cancer+normal vs cancer+normal) | 19-24269890 | cg09777776 | (cancer vs cancer) (cancer+normal vs cancer+normal) |
| 19-45656942 | cg21574675 | (cancer vs cancer) (cancer vs cancer) (cancer+normal vs cancer+normal) | 19-24270468 | cg01000657 | (cancer vs cancer) (cancer+normal vs cancer+normal) |
| 19-45657270 | cg24122218 | (cancer vs cancer) (cancer+normal vs cancer+normal) | 19-34012935 | cg18701660 | (cancer+normal vs cancer+normal) |
| 19-59074005 | cg05014211 | (cancer vs cancer) (cancer+normal vs cancer+normal) | 19-35491951 | cg07759570 | (cancer+normal vs cancer+normal) |
| 19-59074308 | cg08447733 | (cancer vs cancer) (cancer+normal vs cancer+normal) | 19-36389833 | cg27297851 | (cancer vs cancer) (cancer+normal vs cancer+normal) |
| 19-59074482 | cg17735983 | (cancer vs cancer) (cancer+normal vs cancer+normal) | 19-42806228 | cg22220467 | (cancer+normal vs cancer+normal) |
| 19-9903777 | cg06458554 | (cancer vs cancer) (cancer+normal vs cancer+normal) | 19-47614071 | cg09276315 | (cancer+normal vs cancer+normal) |
| 2-20866178 | cg09150450 | (cancer vs cancer) (cancer+normal vs cancer+normal) | 19-50096610 | cg15032314 | (cancer vs cancer) (cancer+normal vs cancer+normal) |
| 2-20866231 | cg21039778 | (cancer vs cancer) (cancer+normal vs cancer+normal) | 19-50096791 | cg15022387 | (cancer+normal vs cancer+normal) |
| 2-20866242 | cg19113641 | (cancer vs cancer) (cancer+normal vs cancer+normal) | 19-54369436 | cg06665109 | (cancer vs cancer) (cancer+normal vs cancer+normal) |
| 2-20866261 | cg12266953 | (cancer vs cancer) (cancer+normal vs cancer+normal) | 19-6740812 | cg22076972 | (cancer vs cancer) (cancer+normal vs cancer+normal) |

| | | | | | |
|---|---|---|---|---|---|
| 2-9549336 | cg09734418 | (cancer+normal vs cancer+normal) | 19-6740888 | cg13341720 | (cancer vs cancer) (cancer+normal vs cancer+normal) |
| 21-40752116 | cg11388866 | (cancer+normal vs cancer+normal) | 19-6740952 | cg03705926 | (cancer vs cancer) (cancer+normal vs cancer+normal) |
| 22-21319245 | cg16590005 | (cancer vs cancer) (cancer vs cancer) (cancer+normal vs cancer+normal) | 2-197852904 | cg13283691 | (cancer+normal vs cancer+normal) |
| 22-21319303 | cg10348922 | (cancer vs cancer) (cancer+normal vs cancer+normal) | 2-216877947 | cg01778114 | (cancer+normal vs cancer+normal) |
| 22-21319661 | cg07960806 | (cancer vs cancer) (cancer vs cancer) (cancer+normal vs cancer+normal) | 2-233792394 | cg08165971 | (cancer vs cancer) (cancer+normal vs cancer+normal) |
| 22-21319668 | cg11782550 | (cancer vs cancer) (cancer+normal vs cancer+normal) | 2-233793120 | cg06828015 | (cancer vs cancer) (cancer+normal vs cancer+normal) |
| 22-27152698 | cg15907944 | (cancer+normal vs cancer+normal) | 2-264120 | cg10181419 | (cancer vs cancer) (cancer+normal vs cancer+normal) |
| 3-181436643 | cg04599356 | (cancer+normal vs cancer+normal) | 2-264178 | cg20749741 | (cancer vs cancer) (cancer+normal vs cancer+normal) |
| 3-187456579 | cg13483916 | (cancer+normal vs cancer+normal) | 2-264199 | cg00108164 | (cancer vs cancer) (cancer+normal vs cancer+normal) |
| 3-187457732 | cg10072850 | (cancer vs cancer) (cancer+normal vs cancer+normal) | 2-264204 | cg25945732 | (cancer vs cancer) (cancer+normal vs cancer+normal) |
| 4-111531926 | cg06374962 | (normal vs normal) (cancer+normal vs cancer+normal) | 2-74425523 | cg16367511 | (cancer vs cancer) (cancer+normal vs cancer+normal) |
| 4-111531975 | cg18064927 | (normal vs normal) (cancer+normal vs cancer+normal) | 2-74425580 | cg13755546 | (cancer vs cancer) (cancer+normal vs cancer+normal) |
| 4-111532035 | cg22006640 | (normal vs normal) (cancer+normal vs cancer+normal) | 2-9144618 | cg15311822 | (cancer+normal vs cancer+normal) |
| 4-111532410 | cg11416290 | (normal vs normal) (cancer+normal vs cancer+normal) | 2-9144629 | cg07960067 | (cancer vs cancer) (cancer+normal vs cancer+normal) |
| 5-118324104 | cg08755283 | (cancer+normal vs cancer+normal) | 20-33759753 | cg05852537 | (cancer+normal vs cancer+normal) |
| 5-125930496 | cg05689600 | (cancer vs cancer) (cancer+normal vs cancer+normal) | 20-825408 | cg19424265 | (cancer vs cancer) (cancer+normal vs cancer+normal) |
| 5-125930667 | cg01814098 | (cancer vs cancer) (cancer+normal vs cancer+normal) | 20-825415 | cg14855292 | (cancer+normal vs cancer+normal) |
| 5-125931001 | cg19551232 | (cancer vs cancer) (cancer+normal vs cancer+normal) | 22-19973978 | cg01138652 | (cancer+normal vs cancer+normal) |
| 5-125931120 | cg26327732 | (cancer vs cancer) (cancer+normal vs cancer+normal) | 22-36236303 | cg25488206 | (cancer vs cancer) (cancer+normal vs cancer+normal) |
| 5-125931166 | cg16738971 | (cancer vs cancer) (cancer+normal vs cancer+normal) | 3-119041204 | cg05894970 | (cancer+normal vs cancer+normal) |

| | | | | | |
|---|---|---|---|---|---|
| 5-151150340 | cg13373406 | (cancer vs cancer) (cancer+normal vs cancer+normal) | 3-183542914 | cg12727374 | (cancer vs cancer) (normal vs normal) (cancer+normal vs cancer+normal) |
| 5-151150581 | cg00977110 | (cancer vs cancer) (cancer+normal vs cancer+normal) | 3-183543564 | cg19155007 | (cancer vs cancer) (cancer+normal vs cancer+normal) |
| 5-171384415 | cg25959472 | (cancer vs cancer) (cancer+normal vs cancer+normal) | 3-183543587 | cg09738156 | (cancer vs cancer) (normal vs normal) (cancer+normal vs cancer+normal) |
| 6-106958303 | cg04067276 | (cancer vs cancer) (cancer+normal vs cancer+normal) | 3-197281468 | cg03038685 | (cancer vs cancer) (cancer+normal vs cancer+normal) |
| 6-106958388 | cg23413349 | (cancer vs cancer) (normal vs normal) (cancer+normal vs cancer+normal) | 3-197281934 | cg03792768 | (cancer vs cancer) (cancer+normal vs cancer+normal) |
| 6-106958474 | cg09184502 | (cancer vs cancer) (cancer+normal vs cancer+normal) | 3-49757016 | cg24722112 | (cancer vs cancer) (cancer+normal vs cancer+normal) |
| 6-106958640 | cg25944720 | (cancer vs cancer) (cancer+normal vs cancer+normal) | 3-49757239 | cg25755107 | (cancer vs cancer) (cancer+normal vs cancer+normal) |
| 6-106958645 | cg22231056 | (cancer vs cancer) (cancer+normal vs cancer+normal) | 3-49757306 | cg04432597 | (cancer vs cancer) (cancer+normal vs cancer+normal) |
| 6-157198648 | cg23603995 | (cancer+normal vs cancer+normal) | 3-52567130 | cg02231066 | (cancer+normal vs cancer+normal) |
| 6-28911468 | cg01742627 | (cancer vs cancer) (cancer+normal vs cancer+normal) | 5-176831297 | cg14126493 | (cancer vs cancer) (cancer+normal vs cancer+normal) |
| 6-28911474 | cg05127899 | (cancer vs cancer) (cancer+normal vs cancer+normal) | 5-176831638 | cg05876864 | (cancer vs cancer) (cancer+normal vs cancer+normal) |
| 6-28911759 | cg23326313 | (cancer vs cancer) (cancer+normal vs cancer+normal) | 6-158957433 | cg00590036 | (cancer vs cancer) (cancer+normal vs cancer+normal) |
| 6-28911926 | cg15088539 | (cancer vs cancer) (cancer+normal vs cancer+normal) | 6-33245128 | cg27098900 | (cancer+normal vs cancer+normal) |
| 6-28921469 | cg17372996 | (cancer vs cancer) (cancer+normal vs cancer+normal) | 6-33245328 | cg11772919 | (cancer+normal vs cancer+normal) |
| 6-28921485 | cg08827322 | (cancer vs cancer) (cancer+normal vs cancer+normal) | 6-43044239 | cg25998680 | (cancer vs cancer) (cancer+normal vs cancer+normal) |
| 6-28921499 | cg09633604 | (cancer vs cancer) (cancer+normal vs cancer+normal) | 6-43044537 | cg09332655 | (cancer+normal vs cancer+normal) |
| 7-150924351 | cg10436877 | (cancer+normal vs cancer+normal) | 6-43044771 | cg21663580 | (cancer vs cancer) (cancer+normal vs cancer+normal) |
| 7-156433350 | cg07671603 | (cancer+normal vs cancer+normal) | 6-43044876 | cg25033767 | (cancer+normal vs cancer+normal) |
| 7-35297138 | cg22051964 | (normal vs normal) (cancer+normal vs cancer+normal) | 8-128750871 | cg11688275 | (cancer+normal vs cancer+normal) |
| 7-35298720 | cg13537061 | (normal vs normal) (cancer+normal vs cancer+normal) | 8-22457446 | cg02227867 | (cancer vs cancer) (cancer+normal vs cancer+normal) |

| | | | | | |
|---|---|---|---|---|---|
| 7-65878734 | cg14552801 | (cancer+normal vs cancer+normal) | 9-131219302 | cg1415312 6 | (cancer vs cancer) (cancer+normal vs cancer+normal) |
| 7-65878911 | cg14428815 | (cancer vs cancer) (cancer+normal vs cancer+normal) | 9-132935646 | cg1341462 9 | (cancer+normal vs cancer+normal) |
| 7-65878976 | cg26579491 | (cancer+normal vs cancer+normal) | 9-136023905 | cg2122237 0 | (cancer+normal vs cancer+normal) |
| **Status - Hypo** | | | **Status - Hypo** | | |
| 1-224698080 | cg16553083 | (normal vs normal) (cancer+normal vs cancer+normal) | 1-11107048 | cg1288811 3 | (cancer vs cancer) (cancer+normal vs cancer+normal) |
| 1-231174153 | cg16908552 | (cancer vs cancer) (cancer+normal vs cancer+normal) | 1-11107452 | cg0450821 6 | (cancer+normal vs cancer+normal) |
| 1-23520083 | cg11811391 | (cancer vs cancer) (cancer+normal vs cancer+normal) | 1-150948030 | cg0563011 1 | (normal vs normal) (cancer+normal vs cancer+normal) |
| 1-246930955 | cg24633745 | (cancer+normal vs cancer+normal) | 1-167882440 | cg2446133 7 | (cancer vs cancer) (cancer+normal vs cancer+normal) |
| 1-246930969 | cg13187885 | (normal vs normal) (cancer vs cancer) (cancer+normal vs cancer+normal) | 1-167883298 | cg2685966 6 | (cancer vs cancer) (cancer+normal vs cancer+normal) |
| 1-28422839 | cg27573570 | (cancer+normal vs cancer+normal) | 1-171059935 | cg2577816 6 | (cancer vs cancer) (cancer+normal vs cancer+normal) |
| 1-56883736 | cg20687616 | (cancer+normal vs cancer+normal) | 1-171060071 | cg1806314 9 | (normal vs normal) (cancer vs cancer) (cancer+normal vs cancer+normal) |
| 1-8384659 | cg10508347 | (cancer vs cancer) (cancer+normal vs cancer+normal) | 1-173886454 | cg1733065 2 | (cancer vs cancer) (cancer+normal vs cancer+normal) |
| 1-95289024 | cg18396714 | (cancer vs cancer) (cancer+normal vs cancer+normal) | 1-179059407 | cg1759157 4 | (cancer vs cancer) (cancer+normal vs cancer+normal) |
| 10-43849970 | cg23033014 | (cancer vs cancer) (cancer+normal vs cancer+normal) | 1-207277018 | cg2233227 6 | (cancer vs cancer) (cancer+normal vs cancer+normal) |
| 10-5504438 | cg01275661 | (cancer vs cancer) (cancer+normal vs cancer+normal) | 1-207277031 | cg1115396 9 | (cancer vs cancer) (cancer+normal vs cancer+normal) |
| 10-88719950 | cg20459238 | (cancer vs cancer) (cancer vs cancer) (cancer+normal vs cancer+normal) | 1-209847618 | cg2364637 5 | (cancer vs cancer) (cancer+normal vs cancer+normal) |
| 10-88720025 | cg07092097 | (normal vs normal) (cancer vs cancer) (cancer+normal vs cancer+normal) | 1-209877941 | cg2688052 5 | (cancer+normal vs cancer+normal) |
| 11-102339610 | cg08547848 | (cancer vs cancer) (cancer+normal vs cancer+normal) | 1-241795170 | cg0491883 1 | (cancer vs cancer) (cancer+normal vs cancer+normal) |
| 11-111311280 | cg17256234 | (cancer vs cancer) (cancer+normal vs cancer+normal) | 1-95584445 | cg2193267 2 | (cancer vs cancer) (cancer+normal vs cancer+normal) |
| 11-125999805 | cg15215830 | (cancer+normal vs cancer+normal) | 10-105616523 | cg0172741 9 | (cancer+normal vs cancer+normal) |
| 11- | cg06679296 | (cancer+normal vs | 10-8546509 | cg1187504 | (normal vs normal) |

| | | | | | |
|---|---|---|---|---|---|
| 1949032 | | cancer+normal) | | 4 | (cancer vs cancer) (cancer+normal vs cancer+normal) |
| 11-1949039 | cg06503573 | (cancer+normal vs cancer+normal) | 10-93390311 | cg18354248 | (cancer+normal vs cancer+normal) |
| 11-4010752 | cg14849140 | (cancer vs cancer) (cancer+normal vs cancer+normal) | 10-93390347 | cg24288527 | (cancer vs cancer) (cancer+normal vs cancer+normal) |
| 11-71229642 | cg03614381 | (normal vs normal) (cancer vs cancer) (cancer+normal vs cancer+normal) | 10-93390387 | cg14037652 | (cancer+normal vs cancer+normal) |
| 11-71230734 | cg04665565 | (normal vs normal) (cancer+normal vs cancer+normal) | 11-126147781 | cg18482593 | (cancer+normal vs cancer+normal) |
| 11-73278053 | cg04939326 | (cancer+normal vs cancer+normal) | 11-46740481 | cg00371195 | (cancer vs cancer) (cancer+normal vs cancer+normal) |
| 12-133435770 | cg21432513 | (cancer+normal vs cancer+normal) | 11-46741373 | cg26453360 | (cancer vs cancer) (cancer+normal vs cancer+normal) |
| 12-47471907 | cg18436898 | (cancer vs cancer) (cancer vs cancer) (cancer+normal vs cancer+normal) | 11-46741405 | cg09004691 | (cancer vs cancer) (cancer+normal vs cancer+normal) |
| 12-89811742 | cg19198386 | (normal vs normal) (cancer+normal vs cancer+normal) | 11-6462110 | cg14164596 | (cancer vs cancer) (cancer+normal vs cancer+normal) |
| 12-90313589 | cg00015699 | (cancer vs cancer) (cancer+normal vs cancer+normal) | 11-6462828 | cg17272126 | (cancer+normal vs cancer+normal) |
| 12-90313611 | cg20490773 | (cancer vs cancer) (cancer+normal vs cancer+normal) | 11-68456607 | cg19252741 | (cancer+normal vs cancer+normal) |
| 13-114187548 | cg06069407 | (cancer+normal vs cancer+normal) | 12-133249241 | cg16956999 | (cancer+normal vs cancer+normal) |
| 13-114187973 | cg22612590 | (cancer vs cancer) (cancer+normal vs cancer+normal) | 12-56515094 | cg05159909 | (cancer+normal vs cancer+normal) |
| 13-114187990 | cg13241681 | (cancer vs cancer) (cancer+normal vs cancer+normal) | 12-57828847 | cg03399971 | (cancer+normal vs cancer+normal) |
| 13-114188051 | cg20539175 | (cancer vs cancer) (cancer+normal vs cancer+normal) | 12-69199037 | cg10635494 | (normal vs normal) (cancer vs cancer) (cancer+normal vs cancer+normal) |
| 15-65042722 | cg22423363 | (normal vs normal) (cancer vs cancer) (cancer vs cancer) (cancer+normal vs cancer+normal) | 15-70372105 | cg17972789 | (cancer+normal vs cancer+normal) |
| 15-69126265 | cg06049171 | (cancer+normal vs cancer+normal) | 16-20709238 | cg08879255 | (cancer vs cancer) (cancer+normal vs cancer+normal) |
| 16-1576146 | cg27316811 | (normal vs normal) (cancer vs cancer) (cancer+normal vs cancer+normal) | 16-2088960 | cg05194102 | (cancer+normal vs cancer+normal) |
| 16-1576773 | cg07019252 | (cancer+normal vs cancer+normal) | 16-2522266 | cg06604968 | (cancer+normal vs cancer+normal) |
| 16- | cg27587310 | (cancer vs cancer) | 16- | cg0630229 | (cancer+normal vs |

| | | | | | |
|---|---|---|---|---|---|
| 29204994 | | (cancer+normal vs cancer+normal) | 58764886 | 5 | cancer+normal) |
| 17-25619946 | cg07564598 | (cancer vs cancer) (cancer+normal vs cancer+normal) | 16-83981557 | cg01800744 | (cancer+normal vs cancer+normal) |
| 17-43208740 | cg04970158 | (cancer+normal vs cancer+normal) | 16-83981678 | cg06143864 | (cancer+normal vs cancer+normal) |
| 17-56569119 | cg25504605 | (cancer vs cancer) (cancer+normal vs cancer+normal) | 16-87969962 | cg11667117 | (cancer+normal vs cancer+normal) |
| 17-75237550 | cg02746913 | (normal vs normal) (cancer vs cancer) (cancer+normal vs cancer+normal) | 16-87970367 | cg05640992 | (cancer vs cancer) (cancer+normal vs cancer+normal) |
| 17-767301 | cg13476078 | (normal vs normal) (cancer vs cancer) (cancer vs cancer) (cancer+normal vs cancer+normal) | 16-87970376 | cg03550864 | (cancer vs cancer) (cancer+normal vs cancer+normal) |
| 17-767334 | cg25236028 | (normal vs normal) (cancer vs cancer) (cancer+normal vs cancer+normal) | 17-17485934 | cg02094018 | (cancer+normal vs cancer+normal) |
| 17-767380 | cg13120258 | (cancer vs cancer) (cancer+normal vs cancer+normal) | 17-19249164 | cg26619894 | (cancer+normal vs cancer+normal) |
| 17-78035629 | cg07724971 | (cancer vs cancer) (cancer+normal vs cancer+normal) | 17-26694939 | cg18536123 | (cancer vs cancer) (cancer+normal vs cancer+normal) |
| 18-5956002 | cg13431373 | (cancer+normal vs cancer+normal) | 17-27370988 | cg16608731 | (cancer vs cancer) (cancer+normal vs cancer+normal) |
| 19-39052032 | cg19338222 | (cancer vs cancer) (cancer vs cancer) (cancer+normal vs cancer+normal) | 17-7019284 | cg18964954 | (cancer vs cancer) (cancer+normal vs cancer+normal) |
| 19-39084308 | cg19786121 | (cancer vs cancer) (cancer+normal vs cancer+normal) | 17-77706717 | cg06901238 | (cancer+normal vs cancer+normal) |
| 19-41190396 | cg18668041 | (cancer vs cancer) (cancer+normal vs cancer+normal) | 19-11351027 | cg27499925 | (cancer+normal vs cancer+normal) |
| 19-8670086 | cg11745755 | (cancer vs cancer) (cancer+normal vs cancer+normal) | 19-2790585 | cg25806370 | (cancer+normal vs cancer+normal) |
| 2-135532566 | cg16200531 | (cancer vs cancer) (cancer+normal vs cancer+normal) | 19-4537901 | cg13733923 | (cancer+normal vs cancer+normal) |
| 2-219256018 | cg23212579 | (cancer vs cancer) (cancer vs cancer) (cancer+normal vs cancer+normal) | 19-45449006 | cg27436184 | (cancer vs cancer) (cancer+normal vs cancer+normal) |
| 2-219256101 | cg16926316 | (cancer vs cancer) (cancer+normal vs cancer+normal) | 2-113884447 | cg23041410 | (cancer vs cancer) (cancer+normal vs cancer+normal) |
| 2-242868376 | cg10397934 | (cancer vs cancer) (cancer+normal vs cancer+normal) | 2-21267334 | cg26112457 | (cancer vs cancer) (cancer+normal vs cancer+normal) |
| 2-25427108 | cg20422417 | (cancer vs cancer) (cancer+normal vs cancer+normal) | 2-234580842 | cg23927405 | (cancer+normal vs cancer+normal) |

| | | | | | |
|---|---|---|---|---|---|
| 2-25809988 | cg08369014 | (cancer+normal vs cancer+normal) | 2-234627450 | cg04437648 | (cancer vs cancer) (cancer+normal vs cancer+normal) |
| 2-42175351 | cg21708738 | (cancer+normal vs cancer+normal) | 2-44065003 | cg01186613 | (cancer+normal vs cancer+normal) |
| 2-45497191 | cg08570458 | (normal vs normal) (cancer+normal vs cancer+normal) | 2-44065725 | cg20926720 | (cancer vs cancer) (cancer+normal vs cancer+normal) |
| 2-75078286 | cg11672772 | (cancer vs cancer) (cancer vs cancer) (cancer+normal vs cancer+normal) | 2-44066294 | cg19123339 | (cancer vs cancer) (cancer+normal vs cancer+normal) |
| 2-95939339 | cg24560809 | (cancer vs cancer) (cancer+normal vs cancer+normal) | 2-44067042 | cg00760272 | (cancer vs cancer) (cancer+normal vs cancer+normal) |
| 20-31446363 | cg16208206 | (cancer vs cancer) (cancer+normal vs cancer+normal) | 21-37852822 | cg15310162 | (cancer vs cancer) (cancer+normal vs cancer+normal) |
| 20-32316843 | cg04772817 | (cancer vs cancer) (cancer+normal vs cancer+normal) | 21-37853496 | cg23808704 | (cancer+normal vs cancer+normal) |
| 20-50419248 | cg24332577 | (cancer vs cancer) (cancer+normal vs cancer+normal) | 21-37853516 | cg16865442 | (cancer+normal vs cancer+normal) |
| 20-50419274 | cg12288473 | (cancer vs cancer) (cancer+normal vs cancer+normal) | 21-44590650 | cg22989407 | (cancer+normal vs cancer+normal) |
| 20-56076737 | cg25169524 | (cancer vs cancer) (cancer+normal vs cancer+normal) | 21-47575134 | cg04413147 | (cancer vs cancer) (cancer+normal vs cancer+normal) |
| 20-60510235 | cg11853697 | (cancer vs cancer) (cancer+normal vs cancer+normal) | 22-21128411 | cg09634469 | (cancer vs cancer) (cancer+normal vs cancer+normal) |
| 21-46768124 | cg22117968 | (cancer+normal vs cancer+normal) | 22-24891666 | cg04033580 | (cancer vs cancer) (cancer+normal vs cancer+normal) |
| 21-46896174 | cg13180920 | (cancer vs cancer) (cancer+normal vs cancer+normal) | 22-41630982 | cg18659483 | (cancer vs cancer) (cancer+normal vs cancer+normal) |
| 3-12332780 | cg16827534 | (cancer vs cancer) (cancer+normal vs cancer+normal) | 22-50644361 | cg10201084 | (cancer+normal vs cancer+normal) |
| 3-184052502 | cg03513893 | (cancer vs cancer) (cancer+normal vs cancer+normal) | 22-50644468 | cg07366553 | (cancer vs cancer) (cancer+normal vs cancer+normal) |
| 3-194573301 | cg10985281 | (cancer vs cancer) (cancer vs cancer) (cancer+normal vs cancer+normal) | 22-50644682 | cg21874862 | (cancer vs cancer) (cancer+normal vs cancer+normal) |
| 3-194573323 | cg07206256 | (cancer vs cancer) (cancer+normal vs cancer+normal) | 22-50644755 | cg25934700 | (cancer vs cancer) (cancer+normal vs cancer+normal) |
| 4-11370314 | cg03609493 | (normal vs normal) (cancer+normal vs cancer+normal) | 3-142681704 | cg26301689 | (cancer vs cancer) (cancer+normal vs cancer+normal) |
| 4-125631168 | cg09646593 | (cancer+normal vs cancer+normal) | 3-194071770 | cg11122795 | (cancer+normal vs cancer+normal) |
| 4-141188341 | cg06775361 | (cancer vs cancer) (cancer+normal vs cancer+normal) | 3-52812973 | cg08148488 | (cancer+normal vs cancer+normal) |
| 4-3480656 | cg13455623 | (cancer vs cancer) | 3-52828594 | cg2586555 | (cancer+normal vs |

| | | | | | |
|---|---|---|---|---|---|
| | | (cancer+normal vs cancer+normal) | | 3 | cancer+normal) |
| 4-7203326 | cg18011273 | (cancer+normal vs cancer+normal) | 4-1198278 | cg21380181 | (cancer vs cancer) (cancer+normal vs cancer+normal) |
| 4-802844 | cg02689072 | (cancer+normal vs cancer+normal) | 4-185724646 | cg08823975 | (cancer+normal vs cancer+normal) |
| 5-180219707 | cg18114890 | (cancer vs cancer) (cancer+normal vs cancer+normal) | 4-186734530 | cg19358738 | (cancer+normal vs cancer+normal) |
| 5-9557609 | cg23920603 | (cancer vs cancer) (cancer+normal vs cancer+normal) | 4-186734622 | cg04195454 | (cancer+normal vs cancer+normal) |
| 6-106582638 | cg17356964 | (cancer vs cancer) (cancer+normal vs cancer+normal) | 4-187088169 | cg25074170 | (cancer+normal vs cancer+normal) |
| 6-106582669 | cg25581448 | (cancer vs cancer) (cancer vs cancer) (cancer+normal vs cancer+normal) | 4-187088191 | cg14477780 | (cancer+normal vs cancer+normal) |
| 6-142959262 | cg01692809 | (cancer+normal vs cancer+normal) | 5-127872329 | cg26802026 | (cancer+normal vs cancer+normal) |
| 6-149778058 | cg03813151 | (cancer+normal vs cancer+normal) | 5-139939823 | cg19616807 | (cancer vs cancer) (cancer+normal vs cancer+normal) |
| 6-167189272 | cg03329755 | (cancer vs cancer) (cancer vs cancer) (cancer+normal vs cancer+normal) | 5-141346431 | cg04182865 | (cancer vs cancer) (cancer+normal vs cancer+normal) |
| 6-18368796 | cg23221431 | (cancer vs cancer) (cancer+normal vs cancer+normal) | 5-142187317 | cg01204911 | (cancer vs cancer) (cancer+normal vs cancer+normal) |
| 6-18368844 | cg14645856 | (cancer vs cancer) (cancer+normal vs cancer+normal) | 5-176836695 | cg06625767 | (cancer vs cancer) (cancer+normal vs cancer+normal) |
| 6-24429162 | cg23562023 | (cancer vs cancer) (cancer+normal vs cancer+normal) | 6-160500620 | cg21178851 | (cancer+normal vs cancer+normal) |
| 6-31779598 | cg26097381 | (normal vs normal) (cancer+normal vs cancer+normal) | 6-160500689 | cg02396797 | (cancer+normal vs cancer+normal) |
| 6-47202394 | cg21552001 | (cancer vs cancer) (cancer vs cancer) (cancer+normal vs cancer+normal) | 6-30001557 | cg17693013 | (cancer+normal vs cancer+normal) |
| 7-13155344 | cg02486364 | (cancer+normal vs cancer+normal) | 6-30168380 | cg03781123 | (cancer vs cancer) (cancer+normal vs cancer+normal) |
| 7-152621979 | cg08202754 | (cancer+normal vs cancer+normal) | 6-44196412 | cg11990902 | (cancer+normal vs cancer+normal) |
| 7-158596552 | cg23730037 | (cancer vs cancer) (cancer+normal vs cancer+normal) | 6-7142578 | cg16243359 | (cancer+normal vs cancer+normal) |
| 7-170692 | cg19210140 | (cancer vs cancer) (cancer+normal vs cancer+normal) | 6-7142638 | cg09866303 | (cancer+normal vs cancer+normal) |
| 7-17379753 | cg07156115 | (cancer+normal vs cancer+normal) | 6-7220085 | cg01351783 | (cancer+normal vs cancer+normal) |
| 7-190423 | cg17427491 | (cancer+normal vs cancer+normal) | 7-101901663 | cg19346623 | (cancer+normal vs cancer+normal) |
| 7-1932321 | cg23618217 | (cancer vs cancer) | 7-1197113 | cg0392353 | (cancer vs cancer) |

| | | |
|---|---|---|
| | | (cancer+normal vs cancer+normal) |
| 7-1961869 | cg15997393 | (cancer vs cancer) (cancer+normal vs cancer+normal) |
| 7-210075 | cg12458039 | (cancer vs cancer) (cancer vs cancer) (cancer+normal vs cancer+normal) |
| 8-123966105 | cg01886570 | (normal vs normal) (cancer vs cancer) (cancer vs cancer) (cancer+normal vs cancer+normal) |
| 8-37643811 | cg05516773 | (cancer+normal vs cancer+normal) |
| **(normal vs normal)** | | |
| **Status - Hyper** | | |
| 1-29586414 | cg04933208 | (normal vs normal) (cancer+normal vs cancer+normal) |
| 1-29586580 | cg21929771 | (cancer vs cancer) (normal vs normal) |
| 1-39873077 | cg15158106 | (normal vs normal) |
| 1-9602701 | cg26607031 | (normal vs normal) |
| 10-126826902 | cg05749728 | (normal vs normal) |
| 10-45914688 | cg10069493 | (normal vs normal) |
| 11-128321662 | cg10616216 | (normal vs normal) |
| 11-128321749 | cg10334741 | (normal vs normal) |
| 11-128559075 | cg14801518 | (normal vs normal) |
| 12-114833043 | cg08900101 | (normal vs normal) |
| 12-114837572 | cg25399352 | (normal vs normal) |
| 12-114840942 | cg23820885 | (normal vs normal) (cancer+normal vs cancer+normal) |
| 12-114841536 | cg11841394 | (normal vs normal) |
| 12-114841708 | cg00642359 | (normal vs normal) |
| 12-114852027 | cg12437821 | (normal vs normal) (cancer+normal vs cancer+normal) |
| 12-114852308 | cg20249252 | (normal vs normal) |
| 12-114852359 | cg06912966 | (normal vs normal) |
| 12-54772804 | cg17970299 | (normal vs normal) |
| 12- | cg00781208 | (normal vs normal) |

| | | |
|---|---|---|
| | 5 | (cancer+normal vs cancer+normal) |
| 7-80544479 | cg15556990 | (cancer vs cancer) (cancer+normal vs cancer+normal) |
| 7-895646 | cg25151376 | (cancer vs cancer) (cancer+normal vs cancer+normal) |
| 7-99637061 | cg04193037 | (cancer vs cancer) (cancer+normal vs cancer+normal) |
| 8-142263802 | cg00971369 | (cancer vs cancer) (cancer+normal vs cancer+normal) |
| **(normal vs normal)** | | |
| **Status - Hyper** | | |
| 1-101003634 | cg09408571 | (normal vs normal) |
| 1-14026360 | cg11344626 | (normal vs normal) |
| 1-145395753 | cg17952661 | (cancer vs cancer) (normal vs normal) (cancer+normal vs cancer+normal) |
| 1-151812421 | cg08703872 | (normal vs normal) |
| 1-151812435 | cg24425838 | (normal vs normal) |
| 1-170899285 | cg24368876 | (normal vs normal) |
| 1-205011999 | cg12269394 | (normal vs normal) |
| 1-205195627 | cg13852486 | (normal vs normal) |
| 1-207496045 | cg12467404 | (normal vs normal) |
| 1-231475345 | cg17355809 | (normal vs normal) |
| 1-27817404 | cg24162579 | (normal vs normal) |
| 1-36915864 | cg08878450 | (normal vs normal) |
| 10-112256729 | cg15667844 | (normal vs normal) |
| 10-126434204 | cg15044573 | (normal vs normal) |
| 10-557605 | cg22737241 | (normal vs normal) |
| 10-75771490 | cg16722001 | (normal vs normal) |
| 10-93350190 | cg04012986 | (normal vs normal) |
| 11-118402280 | cg15694715 | (normal vs normal) |
| 11- | cg2691693 | (normal vs normal) |

| | | | | | |
|---|---|---|---|---|---|
| 64433590 | | | 121970613 | 6 | |
| 13-110433784 | cg24526103 | (normal vs normal) | 11-124824789 | cg15298875 | (normal vs normal) |
| 13-110434016 | cg01569664 | (normal vs normal) | 11-128446070 | cg25578781 | (normal vs normal) |
| 16-67457663 | cg26624628 | (normal vs normal) | 11-27061756 | cg19320730 | (normal vs normal) |
| 16-86606899 | cg09941601 | (normal vs normal) | 11-61322255 | cg17147820 | (normal vs normal) |
| 17-17686141 | cg19872299 | (cancer vs cancer) (cancer vs cancer) (normal vs normal) (cancer+normal vs cancer+normal) | 11-68350410 | cg18840931 | (normal vs normal) |
| 17-46713962 | cg13769834 | (normal vs normal) | 11-859670 | cg24134897 | (normal vs normal) |
| 17-46802740 | cg12698628 | (normal vs normal) | 12-12867724 | cg20525461 | (normal vs normal) |
| 17-46802888 | cg09000583 | (normal vs normal) | 12-2048973 | cg19244722 | (normal vs normal) |
| 17-46803008 | cg08594218 | (normal vs normal) | 12-2944493 | cg10231675 | (normal vs normal) |
| 17-46804239 | cg21865150 | (normal vs normal) | 12-7038866 | cg10162209 | (normal vs normal) |
| 17-75369055 | cg16779463 | (cancer vs cancer) (normal vs normal) | 12-8191646 | cg17815933 | (normal vs normal) |
| 17-75369091 | cg17300544 | (cancer vs cancer) (normal vs normal) | 12-95226993 | cg08922308 | (normal vs normal) |
| 17-75369219 | cg03804136 | (cancer vs cancer) (normal vs normal) | 14-104163025 | cg18984103 | (normal vs normal) |
| 17-75369224 | cg15044248 | (normal vs normal) | 14-90168304 | cg01885071 | (normal vs normal) |
| 17-75369228 | cg02884239 | (normal vs normal) | 14-90168351 | cg18901895 | (normal vs normal) |
| 17-75369484 | cg20275528 | (normal vs normal) | 16-419975 | cg26913058 | (normal vs normal) |
| 17-75447478 | cg14517217 | (cancer vs cancer) (cancer vs cancer) (normal vs normal) (cancer+normal vs cancer+normal) | 16-4324280 | cg00021532 | (normal vs normal) |
| 17-75447504 | cg18104645 | (cancer vs cancer) (cancer vs cancer) (normal vs normal) (cancer+normal vs cancer+normal) | 16-56228744 | cg27073113 | (normal vs normal) |
| 19-52134393 | cg03965138 | (normal vs normal) | 16-75183231 | cg27162196 | (normal vs normal) |
| 2-11679845 | cg15707428 | (normal vs normal) | 16-84538885 | cg08277369 | (normal vs normal) |
| 2-11679872 | cg13808071 | (normal vs normal) | 16-84538889 | cg09259081 | (normal vs normal) |
| 2-11679879 | cg14653284 | (normal vs normal) | 17-38220694 | cg11512009 | (normal vs normal) |
| 2-11680086 | cg17208060 | (normal vs normal) | 17-42199063 | cg11049075 | (normal vs normal) |
| 2-176959229 | cg24914355 | (normal vs normal) | 17-55682851 | cg17178761 | (normal vs normal) |
| 2-176960255 | cg17495130 | (normal vs normal) | 17-79477206 | cg03909849 | (normal vs normal) |
| 2- | cg20605061 | (normal vs normal) | 17- | cg0435230 | (normal vs normal) |

| | | | | | |
|---|---|---|---|---|---|
| 176973835 | | | 80654922 | 5 | |
| 2-176974934 | cg11272491 | (normal vs normal) | 19-2327692 | cg15570293 | (normal vs normal) |
| 2-176976081 | cg00095728 | (normal vs normal) | 19-49376722 | cg04396998 | (normal vs normal) |
| 2-176978893 | cg18064824 | (normal vs normal) | 19-54369576 | cg23305567 | (normal vs normal) |
| 2-176978909 | cg13911052 | (normal vs normal) | 19-8674501 | cg12085570 | (normal vs normal) |
| 2-176979345 | cg00679731 | (normal vs normal) | 2-119067579 | cg11124130 | (normal vs normal) |
| 20-34189411 | cg13544006 | (cancer vs cancer) (normal vs normal) | 2-208370032 | cg15965190 | (normal vs normal) |
| 3-128200723 | cg15038286 | (normal vs normal) | 2-226913713 | cg05293897 | (normal vs normal) |
| 3-145969625 | cg07038342 | (normal vs normal) | 2-44058776 | cg14583675 | (normal vs normal) |
| 3-155570582 | cg03876340 | (normal vs normal) | 2-44460827 | cg19769080 | (normal vs normal) |
| 4-111531926 | cg06374962 | (normal vs normal) (cancer+normal vs cancer+normal) | 20-1371017 | cg20254718 | (normal vs normal) |
| 4-111531975 | cg18064927 | (normal vs normal) (cancer+normal vs cancer+normal) | 20-33759605 | cg11864490 | (normal vs normal) |
| 4-111532035 | cg22006640 | (normal vs normal) (cancer+normal vs cancer+normal) | 20-33759608 | cg16116973 | (normal vs normal) |
| 4-111532410 | cg11416290 | (normal vs normal) (cancer+normal vs cancer+normal) | 21-32932016 | cg25046584 | (normal vs normal) |
| 4-111533195 | cg23404248 | (normal vs normal) | 21-35320667 | cg27037013 | (normal vs normal) |
| 4-111533267 | cg12652641 | (normal vs normal) | 22-36236656 | cg26196531 | (normal vs normal) |
| 4-111533823 | cg02719154 | (normal vs normal) | 22-36236760 | cg18613324 | (normal vs normal) |
| 4-111533883 | cg11930400 | (normal vs normal) | 3-143567031 | cg23538718 | (normal vs normal) |
| 4-111533951 | cg04355159 | (normal vs normal) | 3-14494726 | cg13496041 | (normal vs normal) |
| 4-111535334 | cg13292042 | (normal vs normal) | 3-193922630 | cg23003225 | (normal vs normal) |
| 4-111535528 | cg23806894 | (normal vs normal) | 3-38067350 | cg13487918 | (normal vs normal) |
| 4-111535597 | cg09780241 | (normal vs normal) | 3-49378124 | cg26353296 | (normal vs normal) |
| 4-111536767 | cg19849728 | (normal vs normal) | 3-53196104 | cg20886263 | (normal vs normal) |
| 4-111536970 | cg23646776 | (normal vs normal) | 3-87277465 | cg19007988 | (normal vs normal) |
| 4-111551642 | cg01951086 | (normal vs normal) | 4-116035082 | cg26869131 | (normal vs normal) |
| 4-111551829 | cg02725370 | (normal vs normal) | 4-160149604 | cg00730887 | (normal vs normal) |
| 4-111552308 | cg03943773 | (normal vs normal) | 4-173961344 | cg06814616 | (cancer vs cancer) (normal vs normal) |
| 5-153853154 | cg23024136 | (normal vs normal) | 4-3071494 | cg10106725 | (normal vs normal) |
| 5-153854401 | cg17009842 | (normal vs normal) | 4-80977132 | cg12214366 | (normal vs normal) |

| | | | | | |
|---|---|---|---|---|---|
| 5-153855031 | cg23178714 | (normal vs normal) | 4-84205065 | cg16536390 | (normal vs normal) |
| 6-106958388 | cg23413349 | (cancer vs cancer) (normal vs normal) (cancer+normal vs cancer+normal) | 4-87814065 | cg10685359 | (normal vs normal) |
| 6-1384591 | cg14258623 | (normal vs normal) | 5-125759767 | cg25218470 | (normal vs normal) |
| 6-1384705 | cg16424078 | (normal vs normal) | 5-132111426 | cg18090331 | (normal vs normal) |
| 6-1384780 | cg09651522 | (normal vs normal) | 5-35003575 | cg10196026 | (normal vs normal) |
| 6-1395278 | cg16935061 | (normal vs normal) | 5-400201 | cg03604011 | (normal vs normal) |
| 6-31506990 | cg05945401 | (normal vs normal) | 5-90369235 | cg24708882 | (normal vs normal) |
| 7-130626376 | cg04132407 | (normal vs normal) | 5-90458832 | cg05033239 | (normal vs normal) |
| 7-130626474 | cg09662496 | (normal vs normal) | 6-116832794 | cg14547726 | (normal vs normal) |
| 7-27162090 | cg26297005 | (normal vs normal) | 6-126660984 | cg25454804 | (normal vs normal) |
| 7-35294949 | cg11117878 | (normal vs normal) | 6-26421231 | cg15676542 | (normal vs normal) |
| 7-35295120 | cg18011916 | (normal vs normal) | 6-2970624 | cg11112615 | (normal vs normal) |
| 7-35296040 | cg22056346 | (normal vs normal) | 6-30641220 | cg20117675 | (normal vs normal) |
| 7-35296189 | cg11838668 | (normal vs normal) | 6-30923370 | cg05913906 | (normal vs normal) |
| 7-35296213 | cg15311814 | (normal vs normal) | 6-30923545 | cg21776583 | (normal vs normal) |
| 7-35296968 | cg01688536 | (normal vs normal) | 6-32121261 | cg18049167 | (normal vs normal) |
| 7-35297138 | cg22051964 | (normal vs normal) (cancer+normal vs cancer+normal) | 6-40995388 | cg09712135 | (normal vs normal) |
| 7-35298212 | cg22584618 | (normal vs normal) | 6-6700205 | cg18941322 | (normal vs normal) |
| 7-35298391 | cg08732352 | (normal vs normal) | 6-80853625 | cg24302752 | (normal vs normal) |
| 7-35298720 | cg13537061 | (normal vs normal) (cancer+normal vs cancer+normal) | 7-100231672 | cg15603896 | (normal vs normal) |
| 7-35301188 | cg05809668 | (normal vs normal) | 7-151328116 | cg09449988 | (normal vs normal) |
| 7-35301861 | cg20061010 | (normal vs normal) | 7-15555129 | cg27120543 | (normal vs normal) |
| 7-65879115 | cg15649852 | (normal vs normal) | 7-1765313 | cg09160231 | (normal vs normal) |
| 8-27560492 | cg13949137 | (normal vs normal) | 7-8010894 | cg07107420 | (normal vs normal) |
| 8-72469507 | cg11878331 | (normal vs normal) | 8-101960390 | cg26192520 | (normal vs normal) |
| 9-116372034 | cg13663116 | (normal vs normal) | 8-142289648 | cg10138338 | (normal vs normal) |
| 9-18474243 | cg00116234 | (normal vs normal) | 9-133700022 | cg21195763 | (normal vs normal) |
| **Status - Hypo** | | | **Status - Hypo** | | |
| 1-1118157 | cg16366262 | (normal vs normal) | 1-121259813 | cg16724800 | (normal vs normal) |

| | | | | | |
|---|---|---|---|---|---|
| 1-1396296 | cg20951194 | (normal vs normal) | 1-145366536 | cg02973441 | (normal vs normal) |
| 1-17734771 | cg09797837 | (normal vs normal) | 1-145375603 | cg13199178 | (normal vs normal) |
| 1-177850564 | cg27542729 | (normal vs normal) | 1-150948030 | cg05630111 | (normal vs normal) (cancer+normal vs cancer+normal) |
| 1-224698080 | cg16553083 | (normal vs normal) (cancer+normal vs cancer+normal) | 1-157670710 | cg08786003 | (normal vs normal) |
| 1-246930969 | cg13187885 | (normal vs normal) (cancer vs cancer) (cancer+normal vs cancer+normal) | 1-159684431 | cg08474603 | (normal vs normal) |
| 1-67636724 | cg27168493 | (normal vs normal) | 1-16163775 | cg01429859 | (normal vs normal) |
| 1-92942075 | cg09515098 | (normal vs normal) | 1-1689452 | cg04439557 | (normal vs normal) |
| 10-115860297 | cg16734433 | (normal vs normal) | 1-171060071 | cg18063149 | (normal vs normal) (cancer vs cancer) (cancer+normal vs cancer+normal) |
| 10-1509224 | cg04636331 | (normal vs normal) | 1-217241497 | cg10090568 | (normal vs normal) |
| 10-3138418 | cg20327845 | (normal vs normal) | 1-29585898 | cg00914222 | (normal vs normal) |
| 10-65186953 | cg17983571 | (normal vs normal) | 1-4171594 | cg23960736 | (normal vs normal) |
| 10-682841 | cg25020678 | (normal vs normal) | 1-45969600 | cg10089626 | (normal vs normal) |
| 10-88720025 | cg07092097 | (normal vs normal) (cancer vs cancer) (cancer+normal vs cancer+normal) | 1-45986120 | cg06070002 | (normal vs normal) |
| 11-124940631 | cg00379713 | (normal vs normal) | 10-104574906 | cg03210827 | (normal vs normal) |
| 11-2555576 | cg05898618 | (normal vs normal) | 10-110084660 | cg05673079 | (normal vs normal) |
| 11-50220389 | cg13179056 | (normal vs normal) | 10-121775065 | cg15272641 | (normal vs normal) |
| 11-71229642 | cg03614381 | (normal vs normal) (cancer vs cancer) (cancer+normal vs cancer+normal) | 10-135340785 | cg14250048 | (normal vs normal) |
| 11-71230734 | cg04665565 | (normal vs normal) (cancer+normal vs cancer+normal) | 10-1453818 | cg26394196 | (normal vs normal) |
| 11-98437942 | cg03274739 | (normal vs normal) | 10-61320994 | cg13525697 | (normal vs normal) |
| 12-100550390 | cg26112390 | (normal vs normal) | 10-74879093 | cg11930274 | (normal vs normal) |
| 12-22486029 | cg26277026 | (normal vs normal) | 10-8546509 | cg11875044 | (normal vs normal) (cancer vs cancer) (cancer+normal vs cancer+normal) |
| 12-30947871 | cg23378074 | (normal vs normal) | 10-99933209 | cg06821075 | (normal vs normal) |
| 12-76531007 | cg27272293 | (normal vs normal) | 11-112162100 | cg10049631 | (normal vs normal) |
| 12-89811742 | cg19198386 | (normal vs normal) (cancer+normal vs | 11-1704632 | cg22386442 | (normal vs normal) |

| | | | | | |
|---|---|---|---|---|---|
| | | cancer+normal) | | | |
| 13-20805385 | cg18363035 | (normal vs normal) | 11-65203222 | cg17022830 | (normal vs normal) |
| 13-20805656 | cg03473518 | (normal vs normal) | 11-79113178 | cg05473648 | (normal vs normal) |
| 13-20805679 | cg22377389 | (normal vs normal) | 12-102874832 | cg20874044 | (normal vs normal) |
| 13-23952576 | cg07165066 | (normal vs normal) | 12-45266836 | cg17878273 | (normal vs normal) |
| 13-45970273 | cg18865990 | (normal vs normal) | 12-69199037 | cg10635494 | (normal vs normal) (cancer vs cancer) (cancer+normal vs cancer+normal) |
| 13-49108353 | cg17300047 | (normal vs normal) | 13-106117425 | cg01297020 | (normal vs normal) |
| 14-55203969 | cg07921759 | (normal vs normal) | 13-113793268 | cg18535410 | (normal vs normal) |
| 14-62331287 | cg04791162 | (normal vs normal) (cancer+normal vs cancer+normal) | 14-38725750 | cg18403361 | (normal vs normal) |
| 15-100537686 | cg18471471 | (normal vs normal) | 15-41787940 | cg02330683 | (normal vs normal) |
| 15-25486871 | cg04200704 | (normal vs normal) | 15-70371992 | cg05185738 | (normal vs normal) |
| 15-37782940 | cg10548669 | (normal vs normal) | 16-12355834 | cg19133466 | (normal vs normal) |
| 15-55559759 | cg03050022 | (normal vs normal) | 16-30348681 | cg27281036 | (normal vs normal) |
| 15-65042722 | cg22423363 | (normal vs normal) (cancer vs cancer) (cancer vs cancer) (cancer+normal vs cancer+normal) | 16-33259538 | cg13416109 | (normal vs normal) |
| 16-1576146 | cg27316811 | (normal vs normal) (cancer vs cancer) (cancer+normal vs cancer+normal) | 16-73644907 | cg04999058 | (normal vs normal) |
| 16-29152077 | cg08524717 | (normal vs normal) | 17-21194739 | cg15332951 | (normal vs normal) |
| 16-75680535 | cg08585897 | (normal vs normal) | 17-3377265 | cg00747241 | (normal vs normal) |
| 17-16521057 | cg03499000 | (normal vs normal) | 17-64225346 | cg17095279 | (normal vs normal) |
| 17-22017062 | cg25657218 | (normal vs normal) | 17-73501109 | cg26565893 | (normal vs normal) |
| 17-26443366 | cg11792281 | (normal vs normal) | 18-55092509 | cg16874580 | (normal vs normal) |
| 17-292975 | cg23015327 | (normal vs normal) | 18-76073399 | cg03725652 | (normal vs normal) |
| 17-40322138 | cg01218720 | (normal vs normal) | 19-17516282 | cg12090003 | (normal vs normal) |
| 17-74565009 | cg01883155 | (normal vs normal) (cancer vs cancer) (cancer+normal vs cancer+normal) | 19-17516329 | cg16363586 | (normal vs normal) |
| 17-75237550 | cg02746913 | (normal vs normal) (cancer vs cancer) (cancer+normal vs cancer+normal) | 19-17517008 | cg09993699 | (normal vs normal) |
| 17-767301 | cg13476078 | (normal vs normal) (cancer vs cancer) | 19-17517221 | cg20092122 | (normal vs normal) |

| | | | | | |
|---|---|---|---|---|---|
| | | (cancer vs cancer) (cancer+normal vs cancer+normal) | | | |
| 17-767334 | cg25236028 | (normal vs normal) (cancer vs cancer) (cancer+normal vs cancer+normal) | 19-5076681 | cg19367047 | (normal vs normal) |
| 17-78796363 | cg14073057 | (normal vs normal) | 2-101752032 | cg02347113 | (normal vs normal) |
| 17-79913327 | cg25105415 | (normal vs normal) | 2-121261361 | cg25152584 | (normal vs normal) |
| 17-80708367 | cg22325292 | (normal vs normal) | 2-188419830 | cg16478145 | (normal vs normal) |
| 19-32833011 | cg17387694 | (normal vs normal) | 2-234668930 | cg03607648 | (normal vs normal) |
| 2-10360265 | cg14501281 | (normal vs normal) | 2-234669166 | cg11811840 | (normal vs normal) |
| 2-139659766 | cg13024117 | (normal vs normal) | 2-60643473 | cg12223243 | (normal vs normal) |
| 2-161057625 | cg23008083 | (normal vs normal) | 2-79386720 | cg24240626 | (normal vs normal) |
| 2-175462522 | cg22047295 | (normal vs normal) | 2-80221468 | cg03027227 | (normal vs normal) |
| 2-175532338 | cg19275653 | (normal vs normal) | 20-25583652 | cg21878598 | (normal vs normal) |
| 2-200527297 | cg25300318 | (normal vs normal) | 20-56225978 | cg07908223 | (normal vs normal) |
| 2-232044866 | cg12930176 | (normal vs normal) | 20-60119508 | cg05492975 | (normal vs normal) |
| 2-45497191 | cg08570458 | (normal vs normal) (cancer+normal vs cancer+normal) | 20-62364801 | cg04379041 | (normal vs normal) |
| 2-88958370 | cg24267275 | (normal vs normal) | 21-41027960 | cg26709720 | (normal vs normal) |
| 21-43823604 | cg20516845 | (normal vs normal) | 22-50584184 | cg07331098 | (normal vs normal) |
| 22-24348549 | cg11141652 | (normal vs normal) | 3-142681516 | cg07269003 | (normal vs normal) |
| 22-31202478 | cg11549874 | (normal vs normal) | 3-145790097 | cg15726045 | (normal vs normal) |
| 3-127543493 | cg01562552 | (normal vs normal) | 3-184035508 | cg12369869 | (normal vs normal) |
| 3-152939561 | cg21502476 | (normal vs normal) | 3-188495102 | cg17392043 | (normal vs normal) |
| 3-75263641 | cg12798564 | (normal vs normal) | 3-191887304 | cg03905500 | (normal vs normal) |
| 4-111866929 | cg10820262 | (normal vs normal) | 3-196908930 | cg12594803 | (normal vs normal) |
| 4-11370314 | cg03609493 | (normal vs normal) (cancer+normal vs cancer+normal) | 4-103553119 | cg07973026 | (normal vs normal) |
| 4-26334859 | cg21843114 | (normal vs normal) | 4-151502939 | cg20334738 | (normal vs normal) |
| 4-52861831 | cg09614876 | (normal vs normal) | 4-187291940 | cg00675260 | (normal vs normal) |
| 4-6809109 | cg16506970 | (normal vs normal) | 4-188953677 | cg00469814 | (normal vs normal) |
| 5-122432628 | cg26938014 | (normal vs normal) | 4-72650992 | cg07376029 | (normal vs normal) |
| 5-126387527 | cg27323430 | (normal vs normal) | 5-1099592 | cg17733824 | (normal vs normal) |

| | | | | | |
|---|---|---|---|---|---|
| 5-1462820 | cg04005843 | (normal vs normal) | 5-1109031 | cg18997983 | (normal vs normal) |
| 5-156280628 | cg07245677 | (normal vs normal) | 5-127872200 | cg12630983 | (normal vs normal) |
| 5-2338998 | cg26986989 | (normal vs normal) | 5-1339812 | cg23377852 | (normal vs normal) |
| 5-32522852 | cg24585035 | (normal vs normal) | 5-140614765 | cg02539153 | (normal vs normal) |
| 5-56679194 | cg23408558 | (normal vs normal) | 5-17595441 | cg27619489 | (normal vs normal) |
| 6-110967726 | cg02424007 | (normal vs normal) | 5-42991657 | cg10615765 | (normal vs normal) |
| 6-130758149 | cg02769951 | (normal vs normal) | 6-100442489 | cg08430325 | (normal vs normal) |
| 6-1515524 | cg09344041 | (normal vs normal) | 6-157982213 | cg10880973 | (normal vs normal) |
| 6-29604148 | cg08875433 | (normal vs normal) | 6-157982358 | cg17622855 | (normal vs normal) |
| 6-31779598 | cg26097381 | (normal vs normal) (cancer+normal vs cancer+normal) | 6-160500971 | cg09511662 | (normal vs normal) |
| 7-150054588 | cg26613811 | (normal vs normal) | 6-29588988 | cg18126978 | (normal vs normal) (cancer vs cancer) |
| 7-150211061 | cg08037327 | (normal vs normal) | 6-29599248 | cg21644740 | (normal vs normal) |
| 7-155581411 | cg18116285 | (normal vs normal) | 6-29893273 | cg04246123 | (normal vs normal) |
| 7-157533065 | cg14584961 | (normal vs normal) | 6-32006079 | cg03157588 | (normal vs normal) |
| 7-32802765 | cg07573366 | (normal vs normal) (cancer vs cancer) (cancer+normal vs cancer+normal) | 6-41377288 | cg24676346 | (normal vs normal) |
| 7-72742151 | cg03951180 | (normal vs normal) | 7-157446092 | cg26593067 | (normal vs normal) |
| 7-93474158 | cg01381374 | (normal vs normal) | 7-1902093 | cg04555379 | (normal vs normal) |
| 8-123966105 | cg01886570 | (normal vs normal) (cancer vs cancer) (cancer vs cancer) (cancer+normal vs cancer+normal) | 7-214779 | cg05031931 | (normal vs normal) |
| 8-143429730 | cg06795722 | (normal vs normal) | 7-41747322 | cg26478599 | (normal vs normal) |
| 8-25678826 | cg03602212 | (normal vs normal) | 8-71159989 | cg16282160 | (normal vs normal) |
| 8-42012513 | cg26243740 | (normal vs normal) | 8-7631214 | cg09297252 | (normal vs normal) |
| 8-793504 | cg02840008 | (normal vs normal) | 8-93076177 | cg00045118 | (normal vs normal) |
| 8-82434497 | cg25680105 | (normal vs normal) | 9-117093052 | cg00969271 | (normal vs normal) |
| 8-82434543 | cg06895436 | (normal vs normal) | 9-95378254 | cg14255700 | (normal vs normal) |
| **BRCA (cancer vs cancer)** | | | **LUAD (cancer vs cancer)** | | |
| **Status - Hyper** | | | **Status - Hyper** | | |
| 1-151694108 | cg22790257 | (cancer vs cancer) (cancer+normal vs cancer+normal) | 1-151032962 | cg02918910 | (cancer vs cancer) |
| 1- | cg06100368 | (cancer vs cancer) | 1- | cg1032560 | (cancer vs cancer) |

| | | | | | |
|---|---|---|---|---|---|
| 151694123 | | (cancer+normal vs cancer+normal) | 220101785 | 0 | |
| 1-151694281 | cg05160181 | (cancer vs cancer) | 1-23668691 | cg00907427 | (cancer vs cancer) (cancer vs cancer) (normal vs normal) (cancer+normal vs cancer+normal) |
| 1-154842746 | cg11902458 | (cancer vs cancer) | 1-23668792 | cg08718097 | (cancer vs cancer) (cancer vs cancer) (normal vs normal) (cancer+normal vs cancer+normal) |
| 1-155043413 | cg23414228 | (cancer vs cancer) | 1-28764523 | cg08123074 | (cancer vs cancer) (cancer vs cancer) |
| 1-156611194 | cg12664038 | (cancer vs cancer) | 1-40609565 | cg04865976 | (cancer vs cancer) (cancer vs cancer) (cancer+normal vs cancer+normal) |
| 1-171220575 | cg11199869 | (cancer vs cancer) (cancer+normal vs cancer+normal) | 10-102440856 | cg10150592 | (cancer vs cancer) |
| 1-231555522 | cg07040244 | (cancer vs cancer) | 10-102489344 | cg22748290 | (cancer vs cancer) (cancer vs cancer) |
| 1-2985218 | cg08528984 | (cancer vs cancer) | 10-119304104 | cg12894449 | (cancer vs cancer) |
| 1-2985312 | cg19356825 | (cancer vs cancer) | 10-8091753 | cg22783180 | (cancer vs cancer) (cancer vs cancer) (cancer+normal vs cancer+normal) |
| 1-6508592 | cg15274864 | (cancer vs cancer) (cancer+normal vs cancer+normal) | 10-8092036 | cg18072629 | (cancer vs cancer) (cancer vs cancer) (cancer+normal vs cancer+normal) |
| 1-6508954 | cg25097074 | (cancer vs cancer) (cancer+normal vs cancer+normal) | 10-8092165 | cg15803869 | (cancer vs cancer) (cancer vs cancer) (cancer+normal vs cancer+normal) |
| 1-75549256 | cg12303627 | (cancer vs cancer) | 10-8092264 | cg16710894 | (cancer vs cancer) (cancer vs cancer) (cancer+normal vs cancer+normal) |
| 10-135171503 | cg19306063 | (cancer vs cancer) | 10-8095288 | cg05721515 | (cancer vs cancer) (cancer vs cancer) (cancer+normal vs cancer+normal) |
| 11-128392102 | cg18565473 | (cancer vs cancer) (cancer+normal vs cancer+normal) | 10-8096305 | cg09728012 | (cancer vs cancer) (cancer vs cancer) |
| 11-61062704 | cg04017555 | (cancer vs cancer) (cancer+normal vs cancer+normal) | 10-8096311 | cg24647276 | (cancer vs cancer) (cancer vs cancer) |
| 11-61062837 | cg27175288 | (cancer vs cancer) (normal vs normal) (cancer+normal vs cancer+normal) | 10-8096368 | cg01224891 | (cancer vs cancer) |
| 11-61062843 | cg05190577 | (cancer vs cancer) (normal vs normal) (cancer+normal vs cancer+normal) | 10-8096370 | cg15187550 | (cancer vs cancer) (cancer vs cancer) (cancer+normal vs cancer+normal) |
| 11-61062910 | cg06282270 | (cancer vs cancer) (normal vs normal) | 11-279950 | cg02471658 | (cancer vs cancer) (cancer vs cancer) |

| | | | | | |
|---|---|---|---|---|---|
| | | (cancer+normal vs cancer+normal) | | | (cancer+normal vs cancer+normal) |
| 11-65405362 | cg25361844 | (cancer vs cancer) | 11-31831974 | cg18270629 | (cancer vs cancer) (cancer vs cancer) (cancer+normal vs cancer+normal) |
| 11-65405466 | cg17480646 | (cancer vs cancer) (cancer+normal vs cancer+normal) | 11-31832246 | cg26848086 | (cancer vs cancer) (cancer vs cancer) (cancer+normal vs cancer+normal) |
| 11-65405484 | cg01640437 | (cancer vs cancer) (cancer+normal vs cancer+normal) | 11-31832353 | cg05840031 | (cancer vs cancer) (cancer vs cancer) (cancer+normal vs cancer+normal) |
| 11-65405492 | cg27068330 | (cancer vs cancer) (cancer+normal vs cancer+normal) | 11-36615998 | cg10364182 | (cancer vs cancer) (cancer vs cancer) |
| 11-65405760 | cg01685242 | (cancer vs cancer) | 11-57529255 | cg21272996 | (cancer vs cancer) (cancer vs cancer) |
| 12-115125549 | cg20489228 | (cancer vs cancer) (cancer+normal vs cancer+normal) | 11-57529419 | cg10831212 | (cancer vs cancer) (cancer vs cancer) |
| 12-116946354 | cg03396103 | (cancer vs cancer) | 11-57529465 | cg19210276 | (cancer vs cancer) (cancer vs cancer) |
| 12-1770782 | cg05227549 | (cancer vs cancer) | 11-68081226 | cg14245804 | (cancer vs cancer) |
| 12-54329010 | cg07731191 | (cancer vs cancer) (cancer+normal vs cancer+normal) | 12-107711648 | cg24124443 | (cancer vs cancer) |
| 12-54611596 | cg04070651 | (cancer vs cancer) | 12-115120847 | cg27630311 | (cancer vs cancer) (cancer vs cancer) |
| 12-58004731 | cg26503178 | (cancer vs cancer) | 12-115121016 | cg19713038 | (cancer vs cancer) (cancer vs cancer) |
| 13-80912906 | cg16766325 | (cancer vs cancer) | 12-2161437 | cg23524436 | (cancer vs cancer) |
| 13-95201296 | cg15247434 | (cancer vs cancer) (cancer+normal vs cancer+normal) | 15-67814880 | cg22706883 | (cancer vs cancer) (cancer vs cancer) |
| 15-72072813 | cg05739476 | (cancer vs cancer) (cancer+normal vs cancer+normal) | 16-15489614 | cg04981088 | (cancer vs cancer) |
| 15-99789637 | cg25385940 | (cancer vs cancer) | 16-55090870 | cg07367395 | (cancer vs cancer) |
| 16-21831860 | cg07070305 | (cancer vs cancer) | 16-55090946 | cg11181171 | (cancer vs cancer) (cancer vs cancer) (cancer+normal vs cancer+normal) |
| 16-46824143 | cg09705457 | (cancer vs cancer) (cancer+normal vs cancer+normal) | 16-55405200 | cg01317772 | (cancer vs cancer) |
| 17-34077244 | cg14527050 | (cancer vs cancer) (cancer+normal vs cancer+normal) | 16-86478190 | cg04276084 | (cancer vs cancer) (cancer vs cancer) |
| 17-36103036 | cg09679923 | (cancer vs cancer) | 16-86609963 | cg01313904 | (cancer vs cancer) (cancer vs cancer) (cancer+normal vs cancer+normal) |
| 17-7108305 | cg15802396 | (cancer vs cancer) | 17-38821429 | cg11641791 | (cancer vs cancer) |
| 17-75798605 | cg22764700 | (cancer vs cancer) (cancer+normal vs | 17-38821487 | cg15056128 | (cancer vs cancer) |

| | | | | | |
|---|---|---|---|---|---|
| | | cancer+normal) | 17-48284395 | cg06190244 | (cancer vs cancer) |
| 19-15695299 | cg27067425 | (cancer vs cancer) | 17-70119120 | cg21126344 | (cancer vs cancer) |
| 19-15695817 | cg03730474 | (cancer vs cancer) | 18-29304111 | cg07064495 | (cancer vs cancer) (cancer vs cancer) |
| 19-41414353 | cg19493303 | (cancer vs cancer) | 18-72124685 | cg18820209 | (cancer vs cancer) |
| 2-100175704 | cg18261069 | (cancer vs cancer) (cancer+normal vs cancer+normal) | 19-13107252 | cg06197482 | (cancer vs cancer) (cancer vs cancer) |
| 2-100175768 | cg24797187 | (cancer vs cancer) (cancer+normal vs cancer+normal) | 19-13134254 | cg20665774 | (cancer vs cancer) |
| 2-100175805 | cg17165836 | (cancer vs cancer) (cancer+normal vs cancer+normal) | 19-1676011 | cg08091147 | (cancer vs cancer) (cancer vs cancer) |
| 2-10587627 | cg01381586 | (cancer vs cancer) (cancer+normal vs cancer+normal) | 19-17007648 | cg11282936 | (cancer vs cancer) (cancer+normal vs cancer+normal) |
| 2-211090171 | cg09068528 | (cancer vs cancer) | 19-17007698 | cg24548833 | (cancer vs cancer) (cancer+normal vs cancer+normal) |
| 2-219763330 | cg05454076 | (cancer vs cancer) | 19-1753636 | cg15527348 | (cancer vs cancer) |
| 2-241034966 | cg18663033 | (cancer vs cancer) (cancer+normal vs cancer+normal) | 19-31211050 | cg14761773 | (cancer vs cancer) |
| 20-30193892 | cg09923107 | (cancer vs cancer) (normal vs normal) (cancer+normal vs cancer+normal) | 19-58661833 | cg24884572 | (cancer vs cancer) (cancer vs cancer) |
| 20-30194024 | cg00494337 | (cancer vs cancer) (cancer+normal vs cancer+normal) | 2-224467359 | cg21616047 | (cancer vs cancer) (cancer vs cancer) (cancer+normal vs cancer+normal) |
| 20-37274692 | cg16666964 | (cancer vs cancer) (cancer+normal vs cancer+normal) | 20-20349168 | cg13326227 | (cancer vs cancer) |
| 20-37275069 | cg23884187 | (cancer vs cancer) (cancer+normal vs cancer+normal) | 20-4130531 | cg06616077 | (cancer vs cancer) |
| 21-46352817 | cg17648210 | (cancer vs cancer) (cancer+normal vs cancer+normal) | 20-45523294 | cg12041848 | (cancer vs cancer) |
| 22-19930177 | cg07194846 | (cancer vs cancer) | 20-46413743 | cg21343919 | (cancer vs cancer) (cancer vs cancer) (cancer+normal vs cancer+normal) |
| 22-50919493 | cg02893180 | (cancer vs cancer) | 20-46414934 | cg22084611 | (cancer vs cancer) (cancer vs cancer) |
| 3-18485529 | cg07185974 | (cancer vs cancer) | 21-38071807 | cg14769207 | (cancer vs cancer) (cancer vs cancer) |
| 3-19189751 | cg24668089 | (cancer vs cancer) | 21-40984553 | cg24358603 | (cancer vs cancer) (cancer vs cancer) |
| 3-72149807 | cg02607683 | (cancer vs cancer) (cancer+normal vs cancer+normal) | 21-40984610 | cg20269359 | (cancer vs cancer) (cancer vs cancer) |
| 3-72149921 | cg19996971 | (cancer vs cancer) (cancer+normal vs cancer+normal) | | | |

| | | | | | |
|---|---|---|---|---|---|
| 3-85010677 | cg03416562 | (cancer vs cancer) (normal vs normal) (cancer+normal vs cancer+normal) | 21-40984777 | cg17900854 | (cancer vs cancer) (cancer+normal vs cancer+normal) |
| 4-102712082 | cg27064287 | (cancer vs cancer) | 21-40984780 | cg08448665 | (cancer vs cancer) (cancer vs cancer) (cancer+normal vs cancer+normal) |
| 4-14603924 | cg09690103 | (cancer vs cancer) (cancer+normal vs cancer+normal) | 21-40984886 | cg03701992 | (cancer vs cancer) |
| 4-1811823 | cg22548054 | (cancer vs cancer) | 22-46469091 | cg09137533 | (cancer vs cancer) |
| 5-151153922 | cg19516279 | (cancer vs cancer) (cancer+normal vs cancer+normal) | 3-132843565 | cg00052578 | (cancer vs cancer) (cancer vs cancer) (cancer+normal vs cancer+normal) |
| 5-174120078 | cg27615363 | (cancer vs cancer) (cancer+normal vs cancer+normal) | 4-57522094 | cg12791492 | (cancer vs cancer) (cancer vs cancer) |
| 5-176240038 | cg00041599 | (cancer vs cancer) | 5-135528543 | cg00054702 | (cancer vs cancer) (cancer vs cancer) |
| 5-43041146 | cg17269733 | (cancer vs cancer) | 5-174178256 | cg24996482 | (cancer vs cancer) (cancer vs cancer) |
| 5-43602380 | cg08358683 | (cancer vs cancer) | 5-174178647 | cg24271863 | (cancer vs cancer) |
| 6-1313730 | cg14732248 | (cancer vs cancer) (cancer+normal vs cancer+normal) | 5-175298564 | cg26121591 | (cancer vs cancer) |
| 6-160769359 | cg17364114 | (cancer vs cancer) | 5-175298655 | cg16025094 | (cancer vs cancer) (cancer vs cancer) |
| 6-160769754 | cg07237939 | (cancer vs cancer) (cancer+normal vs cancer+normal) | 5-175298869 | cg13564889 | (cancer vs cancer) (cancer vs cancer) (cancer+normal vs cancer+normal) |
| 6-3053739 | cg02335642 | (cancer vs cancer) (normal vs normal) (cancer+normal vs cancer+normal) | 5-59189934 | cg08554295 | (cancer vs cancer) |
| 6-33739406 | cg07979401 | (cancer vs cancer) (cancer+normal vs cancer+normal) | 6-106442588 | cg00624404 | (cancer vs cancer) |
| 6-33739558 | cg18005901 | (cancer vs cancer) (cancer+normal vs cancer+normal) | 6-110736865 | cg06413398 | (cancer vs cancer) (cancer vs cancer) (cancer+normal vs cancer+normal) |
| 6-33739607 | cg16010596 | (cancer vs cancer) (cancer+normal vs cancer+normal) | 6-110736941 | cg00804078 | (cancer vs cancer) (cancer vs cancer) (cancer+normal vs cancer+normal) |
| 6-33739653 | cg13859433 | (cancer vs cancer) | 6-144608500 | cg15785898 | (cancer vs cancer) (cancer vs cancer) (cancer+normal vs cancer+normal) |
| 6-71122524 | cg23194609 | (cancer vs cancer) | 6-152623304 | cg05917732 | (cancer vs cancer) (cancer vs cancer) (normal vs normal) (cancer+normal vs cancer+normal) |
| 7-100091575 | cg06962782 | (cancer vs cancer) (cancer+normal vs | 6-152623387 | cg02682457 | (cancer vs cancer) (cancer vs cancer) |

| | | | | | |
|---|---|---|---|---|---|
| | | cancer+normal) | | | (normal vs normal) (cancer+normal vs cancer+normal) |
| 7-20826188 | cg24881205 | (cancer vs cancer) | 6-152623479 | cg12023625 | (cancer vs cancer) (cancer vs cancer) (normal vs normal) (cancer+normal vs cancer+normal) |
| 7-23510082 | cg27135125 | (cancer vs cancer) (cancer+normal vs cancer+normal) | 6-165341320 | cg23309836 | (cancer vs cancer) (cancer vs cancer) (cancer+normal vs cancer+normal) |
| 7-23510089 | cg20265043 | (cancer vs cancer) (cancer+normal vs cancer+normal) | 6-26197481 | cg23407396 | (cancer vs cancer) |
| 7-23510112 | cg07297397 | (cancer vs cancer) (cancer+normal vs cancer+normal) | 6-26216541 | cg27133864 | (cancer vs cancer) (cancer vs cancer) |
| 7-23510123 | cg22826239 | (cancer vs cancer) (cancer+normal vs cancer+normal) | 6-26224070 | cg03854865 | (cancer vs cancer) (cancer vs cancer) |
| 7-23510693 | cg16466899 | (cancer vs cancer) (cancer+normal vs cancer+normal) | 6-26224392 | cg16070018 | (cancer vs cancer) (cancer vs cancer) |
| 7-23513335 | cg27322103 | (cancer vs cancer) | 6-26224668 | cg24440941 | (cancer vs cancer) (cancer vs cancer) (cancer+normal vs cancer+normal) |
| 7-55088104 | cg11849717 | (cancer vs cancer) | 6-33267996 | cg15929078 | (cancer vs cancer) |
| 8-24857567 | cg03533519 | (cancer vs cancer) | 6-50680997 | cg17489695 | (cancer vs cancer) (cancer vs cancer) |
| 8-99076734 | cg12845520 | (cancer vs cancer) | 7-130419057 | cg25109431 | (cancer vs cancer) |
| 9-129372349 | cg01027151 | (cancer vs cancer) (normal vs normal) (cancer+normal vs cancer+normal) | 7-130419064 | cg05651960 | (cancer vs cancer) (cancer+normal vs cancer+normal) |
| 9-129372478 | cg14082645 | (cancer vs cancer) (cancer+normal vs cancer+normal) | 7-130419066 | cg18751682 | (cancer vs cancer) |
| 9-129373499 | cg19337180 | (cancer vs cancer) (normal vs normal) (cancer+normal vs cancer+normal) | 7-2271722 | cg03694580 | (cancer vs cancer) (cancer vs cancer) (cancer+normal vs cancer+normal) |
| 9-129386671 | cg14642696 | (cancer vs cancer) (cancer+normal vs cancer+normal) | 7-27213971 | cg16967880 | (cancer vs cancer) |
| 9-129387303 | cg12777520 | (cancer vs cancer) (normal vs normal) (cancer+normal vs cancer+normal) | 7-81399587 | cg06745740 | (cancer vs cancer) |
| 9-129387721 | cg15689835 | (cancer vs cancer) (cancer+normal vs cancer+normal) | 8-21987861 | cg26045434 | (cancer vs cancer) (cancer vs cancer) |
| 9-129388281 | cg13979277 | (cancer vs cancer) (cancer+normal vs cancer+normal) | 8-21988147 | cg26702929 | (cancer vs cancer) (cancer vs cancer) |
| 9-129388668 | cg04693928 | (cancer vs cancer) (cancer+normal vs cancer+normal) | 8-49293005 | cg22459755 | (cancer vs cancer) |

| | | | | | |
|---|---|---|---|---|---|
| 9-129445219 | cg14409032 | (cancer vs cancer) | 9-81871926 | cg14560872 | (cancer vs cancer)<br>(cancer vs cancer)<br>(cancer+normal vs cancer+normal) |
| 9-129445599 | cg14100888 | (cancer vs cancer)<br>(cancer+normal vs cancer+normal) | 9-89561175 | cg13848566 | (cancer vs cancer)<br>(cancer vs cancer) |
| **Status - Hypo** | | | **Status - Hypo** | | |
| 1-108232421 | cg09896211 | (cancer vs cancer)<br>(cancer+normal vs cancer+normal) | 1-110923328 | cg24058365 | (cancer vs cancer)<br>(cancer vs cancer)<br>(cancer+normal vs cancer+normal) |
| 1-114218186 | cg20616821 | (cancer vs cancer) | 1-117151711 | cg12643502 | (cancer vs cancer)<br>(cancer vs cancer) |
| 1-118502178 | cg04500239 | (cancer vs cancer)<br>(cancer+normal vs cancer+normal) | 1-154179856 | cg14520957 | (cancer vs cancer)<br>(cancer vs cancer)<br>(cancer+normal vs cancer+normal) |
| 1-144513999 | cg16385939 | (cancer vs cancer) | 1-154179861 | cg04934776 | (cancer vs cancer) |
| 1-150479509 | cg26799398 | (cancer vs cancer) | 1-154179996 | cg02896768 | (cancer vs cancer)<br>(cancer vs cancer)<br>(cancer+normal vs cancer+normal) |
| 1-161170110 | cg14448116 | (cancer vs cancer)<br>(cancer+normal vs cancer+normal) | 1-155881944 | cg24337215 | (normal vs normal)<br>(cancer vs cancer) |
| 1-162694184 | cg19313373 | (cancer vs cancer) | 1-166806295 | cg04719166 | (cancer vs cancer)<br>(cancer vs cancer)<br>(cancer+normal vs cancer+normal) |
| 1-168250628 | cg17095936 | (cancer vs cancer)<br>(cancer+normal vs cancer+normal) | 1-212671907 | cg00426089 | (cancer vs cancer)<br>(cancer vs cancer)<br>(cancer+normal vs cancer+normal) |
| 1-168940137 | cg22723143 | (cancer vs cancer) | 1-225697019 | cg00690392 | (cancer vs cancer)<br>(cancer vs cancer) |
| 1-204531383 | cg16508600 | (cancer vs cancer) | 1-236162295 | cg16524818 | (cancer vs cancer)<br>(cancer vs cancer)<br>(cancer+normal vs cancer+normal) |
| 1-204531767 | cg00827973 | (cancer vs cancer)<br>(cancer+normal vs cancer+normal) | 1-61750543 | cg00560072 | (cancer vs cancer)<br>(cancer vs cancer)<br>(cancer+normal vs cancer+normal) |
| 1-206603444 | cg00796611 | (cancer vs cancer)<br>(cancer+normal vs cancer+normal) | 1-86048540 | cg20293609 | (cancer vs cancer)<br>(cancer+normal vs cancer+normal) |
| 1-210558509 | cg08240383 | (cancer vs cancer) | 1-9211836 | cg00909706 | (cancer vs cancer)<br>(cancer+normal vs cancer+normal) |
| 1-236924392 | cg20282303 | (cancer vs cancer) | 1-92414295 | cg01515515 | (normal vs normal)<br>(cancer vs cancer)<br>(cancer+normal vs cancer+normal) |
| 1-245925961 | cg23610994 | (cancer vs cancer)<br>(cancer+normal vs cancer+normal) | 1-92414344 | cg06442199 | (cancer vs cancer)<br>(cancer vs cancer)<br>(cancer+normal vs cancer+normal) |
| 1-246446300 | cg25199005 | (cancer vs cancer) | 1-92414520 | cg03421440 | (cancer vs cancer)<br>(cancer vs cancer) |

| | | | | | |
|---|---|---|---|---|---|
| | | | | | (cancer+normal vs cancer+normal) |
| 10-112630738 | cg27408345 | (cancer vs cancer) (cancer+normal vs cancer+normal) | 10-131592944 | cg06633615 | (cancer vs cancer) (cancer vs cancer) (cancer+normal vs cancer+normal) |
| 10-698258 | cg27233847 | (cancer vs cancer) (cancer+normal vs cancer+normal) | 10-294353 | cg18570800 | (cancer vs cancer) (cancer vs cancer) |
| 10-70010790 | cg06467951 | (cancer vs cancer) | 10-90644018 | cg07510282 | (cancer vs cancer) (cancer vs cancer) |
| 10-79679462 | cg27328839 | (cancer vs cancer) | 11-10332052 | cg14359798 | (cancer vs cancer) |
| 10-81143681 | cg15058572 | (cancer vs cancer) | 11-130089890 | cg03933026 | (cancer vs cancer) (cancer vs cancer) (cancer+normal vs cancer+normal) |
| 11-118313315 | cg19547141 | (cancer vs cancer) | 11-19841423 | cg21664351 | (cancer vs cancer) |
| 11-120255253 | cg10407488 | (cancer vs cancer) (cancer+normal vs cancer+normal) | 11-86224410 | cg27309871 | (cancer vs cancer) (cancer vs cancer) (cancer+normal vs cancer+normal) |
| 11-128327665 | cg14057644 | (cancer vs cancer) (cancer+normal vs cancer+normal) | 12-106703262 | cg25575349 | (cancer vs cancer) |
| 11-6499575 | cg17403702 | (cancer vs cancer) (cancer+normal vs cancer+normal) | 12-132259591 | cg15817163 | (cancer vs cancer) (cancer vs cancer) |
| 11-68181218 | cg26915558 | (cancer vs cancer) | 12-95711510 | cg04876905 | (cancer vs cancer) (cancer vs cancer) |
| 12-132285459 | cg10319629 | (cancer vs cancer) | 13-113420371 | cg08557188 | (normal vs normal) (cancer vs cancer) (cancer vs cancer) (cancer+normal vs cancer+normal) |
| 12-132286017 | cg13251533 | (cancer vs cancer) (cancer+normal vs cancer+normal) | 13-97787063 | cg03550548 | (cancer vs cancer) (cancer vs cancer) (cancer+normal vs cancer+normal) |
| 12-51480175 | cg02764722 | (cancer vs cancer) (cancer+normal vs cancer+normal) | 13-98869706 | cg05194316 | (cancer vs cancer) (cancer+normal vs cancer+normal) |
| 12-63133578 | cg00679520 | (cancer vs cancer) (cancer+normal vs cancer+normal) | 13-98869844 | cg16689193 | (cancer vs cancer) (cancer vs cancer) (cancer+normal vs cancer+normal) |
| 12-94761621 | cg04835652 | (cancer vs cancer) | 14-91691129 | cg16996571 | (cancer vs cancer) (cancer+normal vs cancer+normal) |
| 12-96661032 | cg13429194 | (cancer vs cancer) | 14-91691190 | cg14304073 | (cancer vs cancer) (cancer vs cancer) (cancer+normal vs cancer+normal) |
| 13-28240073 | cg20548564 | (cancer vs cancer) (cancer+normal vs cancer+normal) | 15-71092216 | cg25954269 | (cancer vs cancer) (cancer vs cancer) |
| 13-30107044 | cg10752421 | (cancer vs cancer) (cancer+normal vs cancer+normal) | 15-80217380 | cg22389121 | (normal vs normal) (cancer vs cancer) (cancer vs cancer) (cancer+normal vs |

| | | | | | |
|---|---|---|---|---|---|
| | | | | | (cancer+normal) |
| 13-30107081 | cg13224075 | (cancer vs cancer) (cancer+normal vs cancer+normal) | 16-2544909 | cg00794055 | (cancer vs cancer) (cancer vs cancer) (cancer+normal vs cancer+normal) |
| 14-70129013 | cg24096323 | (cancer vs cancer) | 16-3139060 | cg20364187 | (cancer vs cancer) (cancer vs cancer) (cancer+normal vs cancer+normal) |
| 14-78388728 | cg18505710 | (cancer vs cancer) | 16-678127 | cg06013113 | (cancer vs cancer) (cancer+normal vs cancer+normal) |
| 14-81865007 | cg10334928 | (cancer vs cancer) (cancer+normal vs cancer+normal) | 17-44820573 | cg05485769 | (cancer vs cancer) |
| 15-29429054 | cg19838164 | (cancer vs cancer) | 17-73360252 | cg13282068 | (cancer vs cancer) (cancer vs cancer) |
| 15-39472102 | cg13182816 | (cancer vs cancer) | 17-79952861 | cg02647835 | (cancer vs cancer) |
| 15-85215021 | cg19606131 | (normal vs normal) (cancer vs cancer) (cancer+normal vs cancer+normal) | 17-79953059 | cg14419393 | (cancer vs cancer) (cancer vs cancer) |
| 16-19584490 | cg06105093 | (cancer vs cancer) | 17-80970825 | cg11824564 | (cancer vs cancer) (cancer vs cancer) (cancer+normal vs cancer+normal) |
| 16-3193410 | cg02660541 | (cancer vs cancer) (cancer+normal vs cancer+normal) | 19-4099405 | cg18156417 | (cancer vs cancer) (cancer vs cancer) (cancer+normal vs cancer+normal) |
| 16-69358580 | cg03998598 | (cancer vs cancer) (cancer+normal vs cancer+normal) | 2-15830021 | cg09520554 | (cancer vs cancer) (cancer vs cancer) |
| 17-39067081 | cg11809014 | (cancer vs cancer) (cancer+normal vs cancer+normal) | 2-18736132 | cg05937392 | (normal vs normal) (cancer vs cancer) (cancer vs cancer) (cancer+normal vs cancer+normal) |
| 17-43978756 | cg12633764 | (cancer vs cancer) | 2-18792548 | cg07918712 | (cancer vs cancer) |
| 17-48545805 | cg15506894 | (cancer vs cancer) (cancer+normal vs cancer+normal) | 2-232113244 | cg27273946 | (normal vs normal) (cancer vs cancer) (cancer vs cancer) (cancer+normal vs cancer+normal) |
| 17-73805997 | cg05647756 | (cancer vs cancer) (cancer+normal vs cancer+normal) | 2-232135730 | cg18113295 | (cancer vs cancer) (cancer+normal vs cancer+normal) |
| 17-76171794 | cg08115732 | (cancer vs cancer) (cancer+normal vs cancer+normal) | 2-234373030 | cg08354835 | (cancer vs cancer) (cancer vs cancer) (cancer+normal vs cancer+normal) |
| 17-76425249 | cg00204249 | (cancer vs cancer) (cancer+normal vs cancer+normal) | 2-234394478 | cg16719865 | (cancer vs cancer) (cancer vs cancer) (cancer+normal vs cancer+normal) |
| 19-45575023 | cg15732530 | (cancer vs cancer) (cancer+normal vs cancer+normal) | 2-234394541 | cg17212304 | (cancer vs cancer) (cancer vs cancer) (cancer+normal vs cancer+normal) |

| | | | | | |
|---|---|---|---|---|---|
| 19-45575134 | cg24030434 | (cancer vs cancer) (cancer+normal vs cancer+normal) | 2-234394645 | cg25923162 | (cancer vs cancer) (cancer vs cancer) (cancer+normal vs cancer+normal) |
| 19-45575142 | cg23633937 | (cancer vs cancer) (cancer+normal vs cancer+normal) | 2-236672749 | cg01206017 | (cancer vs cancer) (cancer vs cancer) (cancer+normal vs cancer+normal) |
| 2-121036351 | cg15425061 | (cancer vs cancer) (cancer+normal vs cancer+normal) | 2-240086087 | cg20702527 | (cancer vs cancer) |
| 2-121036440 | cg20349803 | (cancer vs cancer) (cancer+normal vs cancer+normal) | 2-240086178 | cg08444004 | (cancer vs cancer) |
| 2-121036705 | cg27505472 | (cancer vs cancer) | 2-45557351 | cg12804104 | (cancer vs cancer) (cancer vs cancer) (cancer+normal vs cancer+normal) |
| 2-121036760 | cg25950520 | (cancer vs cancer) | 22-29441760 | cg18661550 | (cancer vs cancer) (cancer vs cancer) (cancer+normal vs cancer+normal) |
| 2-173686677 | cg23253991 | (cancer vs cancer) (cancer+normal vs cancer+normal) | 3-118865026 | cg09434534 | (cancer vs cancer) |
| 2-236416064 | cg23337031 | (cancer vs cancer) (cancer+normal vs cancer+normal) | 3-128079984 | cg05801066 | (cancer vs cancer) (cancer vs cancer) (cancer+normal vs cancer+normal) |
| 2-238406432 | cg00784161 | (cancer vs cancer) (cancer+normal vs cancer+normal) | 3-141161580 | cg06294561 | (cancer vs cancer) (cancer vs cancer) (cancer+normal vs cancer+normal) |
| 2-242702553 | cg06022561 | (cancer vs cancer) | 3-188429875 | cg04441577 | (cancer vs cancer) (cancer+normal vs cancer+normal) |
| 2-3452919 | cg11589885 | (cancer vs cancer) (cancer+normal vs cancer+normal) | 3-32408427 | cg24924051 | (cancer vs cancer) (cancer vs cancer) |
| 2-68691913 | cg18769357 | (cancer vs cancer) | 3-45004671 | cg22601496 | (cancer vs cancer) (cancer+normal vs cancer+normal) |
| 2-85622094 | cg27090078 | (cancer vs cancer) (cancer+normal vs cancer+normal) | 3-72537392 | cg15491385 | (cancer vs cancer) (cancer vs cancer) (cancer+normal vs cancer+normal) |
| 2-97508486 | cg14692453 | (cancer vs cancer) | 4-8386198 | cg18999185 | (cancer vs cancer) (cancer vs cancer) (cancer+normal vs cancer+normal) |
| 3-69812434 | cg15023858 | (cancer vs cancer) | 5-131408149 | cg16465129 | (cancer vs cancer) (cancer vs cancer) (cancer+normal vs cancer+normal) |
| 4-141564017 | cg03135535 | (cancer vs cancer) (cancer+normal vs cancer+normal) | 5-141722174 | cg02084814 | (cancer vs cancer) (cancer vs cancer) (cancer+normal vs cancer+normal) |
| 4-141564271 | cg06147143 | (cancer vs cancer) (cancer+normal vs cancer+normal) | 5-143026910 | cg23194776 | (cancer vs cancer) (cancer vs cancer) (cancer+normal vs |

| | | | | | cancer+normal) |
|---|---|---|---|---|---|
| 4-16261642 | cg19604703 | (cancer vs cancer) | 5-1546960 | cg02165151 | (cancer vs cancer) (cancer vs cancer) (cancer+normal vs cancer+normal) |
| 4-1827968 | cg03387103 | (cancer vs cancer) | 5-1596053 | cg02475547 | (cancer vs cancer) (cancer vs cancer) |
| 4-48301717 | cg26306052 | (normal vs normal) (cancer vs cancer) (cancer+normal vs cancer+normal) | 6-143575123 | cg08612271 | (cancer vs cancer) (cancer vs cancer) (cancer+normal vs cancer+normal) |
| 5-126182526 | cg21910416 | (cancer vs cancer) (cancer+normal vs cancer+normal) | 6-143834261 | cg10134204 | (normal vs normal) (cancer vs cancer) (cancer+normal vs cancer+normal) |
| 5-131806593 | cg04659396 | (cancer vs cancer) (cancer+normal vs cancer+normal) | 6-170047483 | cg11412527 | (cancer vs cancer) (cancer vs cancer) (cancer+normal vs cancer+normal) |
| 5-60720320 | cg19003326 | (cancer vs cancer) | 6-25726912 | cg07077277 | (cancer vs cancer) (cancer vs cancer) (cancer+normal vs cancer+normal) |
| 5-75009014 | cg08972357 | (cancer vs cancer) | 6-28875356 | cg14059339 | (cancer vs cancer) (cancer vs cancer) (cancer+normal vs cancer+normal) |
| 6-137493017 | cg00415333 | (cancer vs cancer) (cancer+normal vs cancer+normal) | 6-28875370 | cg08279189 | (cancer vs cancer) (cancer vs cancer) (cancer+normal vs cancer+normal) |
| 6-32135083 | cg13197308 | (cancer vs cancer) (cancer+normal vs cancer+normal) | 6-30900678 | cg10006979 | (cancer vs cancer) (cancer vs cancer) (cancer+normal vs cancer+normal) |
| 6-32135129 | cg03488588 | (cancer vs cancer) | 6-33156236 | cg03790047 | (cancer vs cancer) (cancer+normal vs cancer+normal) |
| 6-32135186 | cg11108115 | (cancer vs cancer) (cancer+normal vs cancer+normal) | 6-39072458 | cg21645089 | (cancer vs cancer) (cancer vs cancer) (cancer+normal vs cancer+normal) |
| 6-32135195 | cg08472222 | (cancer vs cancer) (cancer+normal vs cancer+normal) | 6-42678314 | cg21228424 | (cancer vs cancer) (cancer+normal vs cancer+normal) |
| 6-32135326 | cg26687830 | (cancer vs cancer) | 6-46890102 | cg10770662 | (cancer vs cancer) (cancer+normal vs cancer+normal) |
| 6-32135370 | cg10262770 | (cancer vs cancer) (cancer+normal vs cancer+normal) | 6-46890220 | cg03558010 | (cancer vs cancer) (cancer vs cancer) (cancer+normal vs cancer+normal) |
| 6-32135379 | cg07754347 | (cancer vs cancer) (cancer+normal vs cancer+normal) | 7-105254235 | cg26932690 | (cancer vs cancer) (cancer vs cancer) |
| 6-33288804 | cg15070677 | (normal vs normal) (cancer vs cancer) (cancer+normal vs cancer+normal) | 7-128767280 | cg08281469 | (cancer vs cancer) (cancer vs cancer) (cancer+normal vs cancer+normal) |
| 6-33288891 | cg07098566 | (normal vs normal) (cancer vs cancer) | 7-129778936 | cg04334751 | (cancer vs cancer) (cancer vs cancer) |

| | | | | | |
|---|---|---|---|---|---|
| | | (cancer+normal vs cancer+normal) | | | (cancer+normal vs cancer+normal) |
| 7-127984393 | cg08309135 | (cancer vs cancer) (cancer+normal vs cancer+normal) | 7-2473462 | cg05499880 | (cancer vs cancer) |
| 7-154788856 | cg22030483 | (cancer vs cancer) (cancer+normal vs cancer+normal) | 7-2473529 | cg17907628 | (cancer vs cancer) (cancer vs cancer) (cancer+normal vs cancer+normal) |
| 7-47443041 | cg03958512 | (cancer vs cancer) | 7-2473859 | cg18259487 | (cancer vs cancer) (cancer vs cancer) (cancer+normal vs cancer+normal) |
| 7-6689218 | cg21383341 | (cancer vs cancer) (cancer+normal vs cancer+normal) | 7-34026657 | cg11646259 | (cancer vs cancer) (cancer vs cancer) |
| 8-116676938 | cg17131553 | (cancer vs cancer) | 7-4751611 | cg08797598 | (cancer vs cancer) (cancer vs cancer) |
| 8-128403369 | cg11118198 | (normal vs normal) (cancer vs cancer) (cancer+normal vs cancer+normal) | 7-4752983 | cg27640064 | (cancer vs cancer) (cancer vs cancer) |
| 8-133689006 | cg22678977 | (cancer vs cancer) | 7-4753002 | cg04261496 | (cancer vs cancer) (cancer vs cancer) |
| 8-144948315 | cg04904458 | (cancer vs cancer) | 7-5262452 | cg08310400 | (cancer vs cancer) (cancer vs cancer) (cancer+normal vs cancer+normal) |
| 8-41798556 | cg21603275 | (cancer vs cancer) (cancer+normal vs cancer+normal) | 7-75357247 | cg18724912 | (cancer vs cancer) (cancer vs cancer) |
| 8-81213351 | cg19591612 | (normal vs normal) (cancer vs cancer) | 7-77126994 | cg02166725 | (normal vs normal) (cancer vs cancer) (cancer vs cancer) |
| 8-95168291 | cg05323683 | (cancer vs cancer) (cancer+normal vs cancer+normal) | 7-98996656 | cg19420101 | (cancer vs cancer) (cancer vs cancer) (cancer+normal vs cancer+normal) |
| 8-96040122 | cg02693150 | (cancer vs cancer) | 8-17655156 | cg18109537 | (cancer vs cancer) (cancer vs cancer) (cancer+normal vs cancer+normal) |
| 9-108424905 | cg06119575 | (cancer vs cancer) (cancer+normal vs cancer+normal) | 8-54931229 | cg12277366 | (cancer vs cancer) |
| 9-34316383 | cg01062470 | (cancer vs cancer) | 9-14690531 | cg21185283 | (cancer vs cancer) (cancer vs cancer) (cancer+normal vs cancer+normal) |
| 9-97856079 | cg13763287 | (cancer vs cancer) (cancer+normal vs cancer+normal) | 9-99450036 | cg21174344 | (cancer vs cancer) (cancer vs cancer) |
| **(cancer+normal vs cancer+normal)** | | | **(cancer+normal vs cancer+normal)** | | |
| **Status - Hyper** | | | **Status - Hyper** | | |
| 1-151694108 | cg22790257 | (cancer vs cancer) (cancer+normal vs cancer+normal) | 1-12806027 | cg00945108 | (cancer+normal vs cancer+normal) |
| 1-151694123 | cg06100368 | (cancer vs cancer) (cancer+normal vs cancer+normal) | 1-155035388 | cg00585901 | (cancer+normal vs cancer+normal) |

| | | | | | |
|---|---|---|---|---|---|
| 1-156400160 | cg10945917 | (cancer+normal vs cancer+normal) | 1-201708788 | cg17180631 | (cancer+normal vs cancer+normal) |
| 1-156400432 | cg14177914 | (cancer+normal vs cancer+normal) | 1-201708836 | cg16023122 | (normal vs normal) (cancer+normal vs cancer+normal) |
| 1-171220575 | cg11199869 | (cancer vs cancer) (cancer+normal vs cancer+normal) | 1-201708888 | cg13877974 | (cancer+normal vs cancer+normal) |
| 1-212839040 | cg03739354 | (normal vs normal) (cancer+normal vs cancer+normal) | 1-201708932 | cg01828733 | (cancer+normal vs cancer+normal) |
| 1-6508592 | cg15274864 | (cancer vs cancer) (cancer+normal vs cancer+normal) | 1-201709135 | cg14377596 | (normal vs normal) (cancer+normal vs cancer+normal) |
| 1-6508954 | cg25097074 | (cancer vs cancer) (cancer+normal vs cancer+normal) | 1-20988590 | cg08911391 | (cancer+normal vs cancer+normal) |
| 10-125619351 | cg23985702 | (cancer+normal vs cancer+normal) | 1-23668691 | cg00907427 | (cancer vs cancer) (cancer vs cancer) (normal vs normal) (cancer+normal vs cancer+normal) |
| 11-128392102 | cg18565473 | (cancer vs cancer) (cancer+normal vs cancer+normal) | 1-23668792 | cg08718097 | (cancer vs cancer) (cancer vs cancer) (normal vs normal) (cancer+normal vs cancer+normal) |
| 11-61062704 | cg04017555 | (cancer vs cancer) (cancer+normal vs cancer+normal) | 1-40609565 | cg04865976 | (cancer vs cancer) (cancer vs cancer) (cancer+normal vs cancer+normal) |
| 11-61062837 | cg27175288 | (cancer vs cancer) (normal vs normal) (cancer+normal vs cancer+normal) | 10-13481944 | cg24686497 | (cancer+normal vs cancer+normal) |
| 11-61062843 | cg05190577 | (cancer vs cancer) (normal vs normal) (cancer+normal vs cancer+normal) | 10-29698470 | cg07562483 | (cancer+normal vs cancer+normal) |
| 11-61062910 | cg06282270 | (cancer vs cancer) (normal vs normal) (cancer+normal vs cancer+normal) | 10-29699043 | cg16036409 | (cancer+normal vs cancer+normal) |
| 11-65405466 | cg17480646 | (cancer vs cancer) (cancer+normal vs cancer+normal) | 10-8091753 | cg22783180 | (cancer vs cancer) (cancer vs cancer) (cancer+normal vs cancer+normal) |
| 11-65405484 | cg01640437 | (cancer vs cancer) (cancer+normal vs cancer+normal) | 10-8092036 | cg18072629 | (cancer vs cancer) (cancer vs cancer) (cancer+normal vs cancer+normal) |
| 11-65405492 | cg27068330 | (cancer vs cancer) (cancer+normal vs cancer+normal) | 10-8092165 | cg15803869 | (cancer vs cancer) (cancer vs cancer) (cancer+normal vs cancer+normal) |
| 12-112204761 | cg21470387 | (cancer+normal vs cancer+normal) | 10-8092264 | cg16710894 | (cancer vs cancer) (cancer vs cancer) (cancer+normal vs cancer+normal) |
| 12-114832429 | cg19746940 | (cancer+normal vs cancer+normal) | 10-8095288 | cg05721515 | (cancer vs cancer) (cancer vs cancer) |

| | | | | | |
|---|---|---|---|---|---|
| | | | | | (cancer+normal vs cancer+normal) |
| 12-114836794 | cg17554126 | (cancer+normal vs cancer+normal) | 10-8096370 | cg15187550 | (cancer vs cancer) (cancer vs cancer) (cancer+normal vs cancer+normal) |
| 12-115105220 | cg14987787 | (cancer+normal vs cancer+normal) | 11-123525237 | cg00648476 | (cancer vs cancer) (cancer+normal vs cancer+normal) |
| 12-115125549 | cg20489228 | (cancer vs cancer) (cancer+normal vs cancer+normal) | 11-130030103 | cg10089145 | (cancer vs cancer) (cancer+normal vs cancer+normal) |
| 12-115126061 | cg18220920 | (cancer+normal vs cancer+normal) | 11-279950 | cg02471658 | (cancer vs cancer) (cancer vs cancer) (cancer+normal vs cancer+normal) |
| 12-49627010 | cg15661753 | (cancer+normal vs cancer+normal) | 11-31831974 | cg18270629 | (cancer vs cancer) (cancer vs cancer) (cancer+normal vs cancer+normal) |
| 12-54329010 | cg07731191 | (cancer vs cancer) (cancer+normal vs cancer+normal) | 11-31832246 | cg26848086 | (cancer vs cancer) (cancer vs cancer) (cancer+normal vs cancer+normal) |
| 12-54409321 | cg01473837 | (cancer+normal vs cancer+normal) | 11-31832353 | cg05840031 | (cancer vs cancer) (cancer vs cancer) (cancer+normal vs cancer+normal) |
| 12-54409525 | cg06714180 | (cancer+normal vs cancer+normal) | 11-49579457 | cg17528189 | (cancer+normal vs cancer+normal) |
| 13-95201296 | cg15247434 | (cancer vs cancer) (cancer+normal vs cancer+normal) | 11-8832111 | cg19113668 | (normal vs normal) (cancer+normal vs cancer+normal) |
| 13-95201417 | cg13580857 | (cancer+normal vs cancer+normal) | 11-8832203 | cg26942121 | (cancer+normal vs cancer+normal) |
| 15-72072813 | cg05739476 | (cancer vs cancer) (cancer+normal vs cancer+normal) | 11-8832272 | cg23939875 | (cancer+normal vs cancer+normal) |
| 16-46824143 | cg09705457 | (cancer vs cancer) (cancer+normal vs cancer+normal) | 11-8832283 | cg17046776 | (cancer+normal vs cancer+normal) |
| 16-55361816 | cg03992149 | (cancer+normal vs cancer+normal) | 12-15942969 | cg19321018 | (cancer+normal vs cancer+normal) |
| 16-55363601 | cg09662325 | (cancer+normal vs cancer+normal) | 12-34756291 | cg25343246 | (cancer+normal vs cancer+normal) |
| 17-34077244 | cg14527050 | (cancer vs cancer) (cancer+normal vs cancer+normal) | 12-58138320 | cg15357934 | (cancer+normal vs cancer+normal) |
| 17-34077293 | cg13376404 | (cancer+normal vs cancer+normal) | 12-58138329 | cg24502192 | (cancer+normal vs cancer+normal) |
| 17-75798605 | cg22764700 | (cancer vs cancer) (cancer+normal vs cancer+normal) | 13-100633995 | cg15773704 | (cancer+normal vs cancer+normal) |
| 17-79455576 | cg04763450 | (cancer+normal vs cancer+normal) | 15-101262162 | cg05575505 | (cancer+normal vs cancer+normal) |
| 18-12253749 | cg18649459 | (cancer+normal vs cancer+normal) | 15-41218265 | cg07431199 | (cancer+normal vs cancer+normal) |
| 18-56937906 | cg03160145 | (cancer+normal vs cancer+normal) | 15-41218333 | cg16836355 | (cancer+normal vs cancer+normal) |
| 2-100175704 | cg18261069 | (cancer vs cancer) (cancer+normal vs | 15-41218352 | cg22835157 | (cancer+normal vs cancer+normal) |

| | | | | | |
|---|---|---|---|---|---|
| | | cancer+normal) | | | |
| 2-100175768 | cg24797187 | (cancer vs cancer) (cancer+normal vs cancer+normal) | 16-30759178 | cg03315662 | (cancer+normal vs cancer+normal) |
| 2-100175805 | cg17165836 | (cancer vs cancer) (cancer+normal vs cancer+normal) | 16-55090946 | cg11181171 | (cancer vs cancer) (cancer vs cancer) (cancer+normal vs cancer+normal) |
| 2-10587627 | cg01381586 | (cancer vs cancer) (cancer+normal vs cancer+normal) | 16-58535093 | cg10383447 | (cancer+normal vs cancer+normal) |
| 2-12856422 | cg19706900 | (cancer+normal vs cancer+normal) | 16-83842404 | cg08068296 | (cancer vs cancer) (cancer+normal vs cancer+normal) |
| 2-133428502 | cg20265661 | (cancer+normal vs cancer+normal) | 16-86609963 | cg01313904 | (cancer vs cancer) (cancer vs cancer) (cancer+normal vs cancer+normal) |
| 2-133428510 | cg01850449 | (cancer+normal vs cancer+normal) | 16-89723364 | cg05441039 | (cancer vs cancer) (cancer+normal vs cancer+normal) |
| 2-133428523 | cg09622982 | (cancer+normal vs cancer+normal) | 17-26685074 | cg08076044 | (cancer vs cancer) (cancer+normal vs cancer+normal) |
| 2-172968881 | cg19303187 | (cancer+normal vs cancer+normal) | 17-47297130 | cg11164649 | (cancer+normal vs cancer+normal) |
| 2-220378996 | cg10515731 | (cancer+normal vs cancer+normal) | 17-60005610 | cg25400864 | (cancer+normal vs cancer+normal) |
| 2-220379000 | cg18844301 | (cancer+normal vs cancer+normal) | 19-13734932 | cg12501546 | (cancer+normal vs cancer+normal) |
| 2-220379016 | cg09726982 | (cancer+normal vs cancer+normal) | 19-16771394 | cg16345226 | (cancer+normal vs cancer+normal) |
| 2-220379044 | cg17610929 | (normal vs normal) (cancer+normal vs cancer+normal) | 19-17007648 | cg11282936 | (cancer vs cancer) (cancer+normal vs cancer+normal) |
| 2-220379058 | cg24743778 | (normal vs normal) (cancer+normal vs cancer+normal) | 19-17007698 | cg24548833 | (cancer vs cancer) (cancer+normal vs cancer+normal) |
| 2-220379199 | cg12046414 | (normal vs normal) (cancer+normal vs cancer+normal) | 19-2945492 | cg13076718 | (cancer+normal vs cancer+normal) |
| 2-241034966 | cg18663033 | (cancer vs cancer) (cancer+normal vs cancer+normal) | 19-45395297 | cg02613937 | (cancer+normal vs cancer+normal) |
| 20-30193892 | cg09923107 | (cancer vs cancer) (normal vs normal) (cancer+normal vs cancer+normal) | 19-46097075 | cg16620032 | (cancer+normal vs cancer+normal) |
| 20-30194024 | cg00494337 | (cancer vs cancer) (cancer+normal vs cancer+normal) | 19-56348889 | cg22747516 | (cancer+normal vs cancer+normal) |
| 20-37274692 | cg16666964 | (cancer vs cancer) (cancer+normal vs cancer+normal) | 2-169312418 | cg12334247 | (cancer+normal vs cancer+normal) |
| 20-37275069 | cg23884187 | (cancer vs cancer) (cancer+normal vs cancer+normal) | 2-200324879 | cg26792080 | (cancer vs cancer) (cancer+normal vs cancer+normal) |
| 21-15135580 | cg16706690 | (cancer+normal vs cancer+normal) | 2-224467359 | cg21616047 | (cancer vs cancer) (cancer vs cancer) (cancer+normal vs cancer+normal) |

| | | | | | |
|---|---|---|---|---|---|
| 21-46352817 | cg17648210 | (cancer vs cancer) (cancer+normal vs cancer+normal) | 2-33359529 | cg03906115 | (cancer+normal vs cancer+normal) |
| 3-72149807 | cg02607683 | (cancer vs cancer) (cancer+normal vs cancer+normal) | 2-33359550 | cg08357990 | (cancer+normal vs cancer+normal) |
| 3-72149921 | cg19996971 | (cancer vs cancer) (cancer+normal vs cancer+normal) | 2-43823708 | cg14640509 | (cancer+normal vs cancer+normal) |
| 3-85010677 | cg03416562 | (cancer vs cancer) (normal vs normal) (cancer+normal vs cancer+normal) | 20-30605916 | cg23929284 | (cancer+normal vs cancer+normal) |
| 4-100368380 | cg16863617 | (cancer+normal vs cancer+normal) | 20-46413743 | cg21343919 | (cancer vs cancer) (cancer vs cancer) (cancer+normal vs cancer+normal) |
| 4-14603924 | cg09690103 | (cancer vs cancer) (cancer+normal vs cancer+normal) | 21-10888524 | cg19433175 | (cancer+normal vs cancer+normal) |
| 4-189158066 | cg05865660 | (cancer+normal vs cancer+normal) | 21-40984777 | cg17900854 | (cancer vs cancer) (cancer+normal vs cancer+normal) |
| 4-33062752 | cg21054842 | (cancer+normal vs cancer+normal) | 21-40984780 | cg08448665 | (cancer vs cancer) (cancer vs cancer) (cancer+normal vs cancer+normal) |
| 5-151153922 | cg19516279 | (cancer vs cancer) (cancer+normal vs cancer+normal) | 3-121264201 | cg21456320 | (cancer+normal vs cancer+normal) |
| 5-160971794 | cg21242208 | (cancer+normal vs cancer+normal) | 3-12200427 | cg10173186 | (cancer+normal vs cancer+normal) |
| 5-174120078 | cg27615363 | (cancer vs cancer) (cancer+normal vs cancer+normal) | 3-126398566 | cg04472001 | (cancer+normal vs cancer+normal) |
| 6-1313730 | cg14732248 | (cancer vs cancer) (cancer+normal vs cancer+normal) | 3-129062937 | cg13242558 | (cancer+normal vs cancer+normal) |
| 6-137736547 | cg11953881 | (cancer+normal vs cancer+normal) | 3-129063090 | cg14781521 | (cancer+normal vs cancer+normal) |
| 6-160769754 | cg07237939 | (cancer vs cancer) (cancer+normal vs cancer+normal) | 3-132843565 | cg00052578 | (cancer vs cancer) (cancer vs cancer) (cancer+normal vs cancer+normal) |
| 6-18990714 | cg25042346 | (normal vs normal) (cancer+normal vs cancer+normal) | 3-193922037 | cg27427514 | (cancer+normal vs cancer+normal) |
| 6-29933971 | cg23363602 | (cancer+normal vs cancer+normal) | 4-154143694 | cg12453905 | (normal vs normal) (cancer+normal vs cancer+normal) |
| 6-3053739 | cg02335642 | (cancer vs cancer) (normal vs normal) (cancer+normal vs cancer+normal) | 5-131705035 | cg14196790 | (cancer+normal vs cancer+normal) |
| 6-33739406 | cg07979401 | (cancer vs cancer) (cancer+normal vs cancer+normal) | 5-172386833 | cg27107585 | (cancer+normal vs cancer+normal) |
| 6-33739558 | cg18005901 | (cancer vs cancer) (cancer+normal vs cancer+normal) | 5-175298869 | cg13564889 | (cancer vs cancer) (cancer vs cancer) (cancer+normal vs cancer+normal) |

| | | | | | |
|---|---|---|---|---|---|
| 6-33739607 | cg16010596 | (cancer vs cancer) (cancer+normal vs cancer+normal) | 5-65018961 | cg17329494 | (cancer vs cancer) (cancer+normal vs cancer+normal) |
| 6-43894639 | cg18120259 | (cancer+normal vs cancer+normal) | 6-100064885 | cg01156070 | (cancer vs cancer) (cancer+normal vs cancer+normal) |
| 7-100091575 | cg06962782 | (cancer vs cancer) (cancer+normal vs cancer+normal) | 6-110736865 | cg06413398 | (cancer vs cancer) (cancer vs cancer) (cancer+normal vs cancer+normal) |
| 7-151137048 | cg02512352 | (cancer+normal vs cancer+normal) | 6-110736941 | cg00804078 | (cancer vs cancer) (cancer vs cancer) (cancer+normal vs cancer+normal) |
| 7-151137192 | cg05551889 | (cancer+normal vs cancer+normal) | 6-144608500 | cg15785898 | (cancer vs cancer) (cancer vs cancer) (cancer+normal vs cancer+normal) |
| 7-23510082 | cg27135125 | (cancer vs cancer) (cancer+normal vs cancer+normal) | 6-152623304 | cg05917732 | (cancer vs cancer) (cancer vs cancer) (normal vs normal) (cancer+normal vs cancer+normal) |
| 7-23510089 | cg20265043 | (cancer vs cancer) (cancer+normal vs cancer+normal) | 6-152623387 | cg02682457 | (cancer vs cancer) (cancer vs cancer) (normal vs normal) (cancer+normal vs cancer+normal) |
| 7-23510112 | cg07297397 | (cancer vs cancer) (cancer+normal vs cancer+normal) | 6-152623479 | cg12023625 | (cancer vs cancer) (cancer vs cancer) (normal vs normal) (cancer+normal vs cancer+normal) |
| 7-23510123 | cg22826239 | (cancer vs cancer) (cancer+normal vs cancer+normal) | 6-165341320 | cg23309836 | (cancer vs cancer) (cancer vs cancer) (cancer+normal vs cancer+normal) |
| 7-23510693 | cg16466899 | (cancer vs cancer) (cancer+normal vs cancer+normal) | 6-26224668 | cg24440941 | (cancer vs cancer) (cancer vs cancer) (cancer+normal vs cancer+normal) |
| 8-57594909 | cg21752469 | (normal vs normal) (cancer+normal vs cancer+normal) | 6-31762630 | cg12670305 | (cancer+normal vs cancer+normal) |
| 8-82276987 | cg21037180 | (cancer+normal vs cancer+normal) | 6-4022895 | cg13533069 | (cancer+normal vs cancer+normal) |
| 9-129372349 | cg01027151 | (cancer vs cancer) (normal vs normal) (cancer+normal vs cancer+normal) | 6-53660854 | cg01881322 | (cancer+normal vs cancer+normal) |
| 9-129372478 | cg14082645 | (cancer vs cancer) (cancer+normal vs cancer+normal) | 7-100895999 | cg08601038 | (cancer+normal vs cancer+normal) |
| 9-129373499 | cg19337180 | (cancer vs cancer) (normal vs normal) (cancer+normal vs cancer+normal) | 7-130419064 | cg05651960 | (cancer vs cancer) (cancer+normal vs cancer+normal) |
| 9-129386671 | cg14642696 | (cancer vs cancer) (cancer+normal vs cancer+normal) | 7-2271722 | cg03694580 | (cancer vs cancer) (cancer vs cancer) (cancer+normal vs cancer+normal) |

| | | | | | |
|---|---|---|---|---|---|
| 9-129387303 | cg12777520 | (cancer vs cancer) (normal vs normal) (cancer+normal vs cancer+normal) | 7-75831678 | cg1376724 5 | (cancer vs cancer) (cancer+normal vs cancer+normal) |
| 9-129387721 | cg15689835 | (cancer vs cancer) (cancer+normal vs cancer+normal) | 8-135843385 | cg2290481 5 | (cancer vs cancer) (cancer+normal vs cancer+normal) |
| 9-129388281 | cg13979277 | (cancer vs cancer) (cancer+normal vs cancer+normal) | 9-100396777 | cg2122037 4 | (cancer+normal vs cancer+normal) |
| 9-129388668 | cg04693928 | (cancer vs cancer) (cancer+normal vs cancer+normal) | 9-124976219 | cg2118332 9 | (cancer+normal vs cancer+normal) |
| 9-129445599 | cg14100888 | (cancer vs cancer) (cancer+normal vs cancer+normal) | 9-81871926 | cg1456087 2 | (cancer vs cancer) (cancer vs cancer) (cancer+normal vs cancer+normal) |
| **Status - Hypo** | | | **Status - Hypo** | | |
| 1-108232421 | cg09896211 | (cancer vs cancer) (cancer+normal vs cancer+normal) | 1-110923328 | cg2405836 5 | (cancer vs cancer) (cancer vs cancer) (cancer+normal vs cancer+normal) |
| 1-118502178 | cg04500239 | (cancer vs cancer) (cancer+normal vs cancer+normal) | 1-154179856 | cg1452095 7 | (cancer vs cancer) (cancer vs cancer) (cancer+normal vs cancer+normal) |
| 1-161170110 | cg14448116 | (cancer vs cancer) (cancer+normal vs cancer+normal) | 1-154179996 | cg0289676 8 | (cancer vs cancer) (cancer vs cancer) (cancer+normal vs cancer+normal) |
| 1-1611999 | cg09825670 | (cancer+normal vs cancer+normal) | 1-166806295 | cg0471916 6 | (cancer vs cancer) (cancer vs cancer) (cancer+normal vs cancer+normal) |
| 1-168250628 | cg17095936 | (cancer vs cancer) (cancer+normal vs cancer+normal) | 1-212671907 | cg0042608 9 | (cancer vs cancer) (cancer vs cancer) (cancer+normal vs cancer+normal) |
| 1-17958884 | cg11807529 | (cancer+normal vs cancer+normal) | 1-221908735 | cg1344220 1 | (cancer+normal vs cancer+normal) |
| 1-204531767 | cg00827973 | (cancer vs cancer) (cancer+normal vs cancer+normal) | 1-236162295 | cg1652481 8 | (cancer vs cancer) (cancer vs cancer) (cancer+normal vs cancer+normal) |
| 1-206603444 | cg00796611 | (cancer vs cancer) (cancer+normal vs cancer+normal) | 1-61750543 | cg0056007 2 | (cancer vs cancer) (cancer vs cancer) (cancer+normal vs cancer+normal) |
| 1-245925961 | cg23610994 | (cancer vs cancer) (cancer+normal vs cancer+normal) | 1-86048540 | cg2029360 9 | (cancer vs cancer) (cancer+normal vs cancer+normal) |
| 10-112630738 | cg27408345 | (cancer vs cancer) (cancer+normal vs cancer+normal) | 1-9211836 | cg0090970 6 | (cancer vs cancer) (cancer+normal vs cancer+normal) |
| 10-112630835 | cg01709312 | (cancer+normal vs cancer+normal) | 1-92414295 | cg0151551 5 | (normal vs normal) (cancer vs cancer) (cancer+normal vs cancer+normal) |
| 10-6270423 | cg12235073 | (cancer+normal vs cancer+normal) | 1-92414344 | cg0644219 9 | (cancer vs cancer) (cancer vs cancer) (cancer+normal vs |

| | | | | | |
|---|---|---|---|---|---|
| | | | | | cancer+normal) |
| 10-664762 | cg18474072 | (cancer+normal vs cancer+normal) | 1-92414520 | cg03421440 | (cancer vs cancer) (cancer vs cancer) (cancer+normal vs cancer+normal) |
| 10-698258 | cg27233847 | (cancer vs cancer) (cancer+normal vs cancer+normal) | 10-131592944 | cg06633615 | (cancer vs cancer) (cancer vs cancer) (cancer+normal vs cancer+normal) |
| 11-118774009 | cg08983011 | (cancer+normal vs cancer+normal) | 11-130089890 | cg03933026 | (cancer vs cancer) (cancer vs cancer) (cancer+normal vs cancer+normal) |
| 11-120255253 | cg10407488 | (cancer vs cancer) (cancer+normal vs cancer+normal) | 11-86224410 | cg27309871 | (cancer vs cancer) (cancer vs cancer) (cancer+normal vs cancer+normal) |
| 11-128327665 | cg14057644 | (cancer vs cancer) (cancer+normal vs cancer+normal) | 12-102216984 | cg17214456 | (cancer vs cancer) (cancer+normal vs cancer+normal) |
| 11-6499575 | cg17403702 | (cancer vs cancer) (cancer+normal vs cancer+normal) | 12-56914766 | cg19628333 | (cancer+normal vs cancer+normal) |
| 11-75050147 | cg12041266 | (cancer+normal vs cancer+normal) | 13-113420371 | cg08557188 | (normal vs normal) (cancer vs cancer) (cancer vs cancer) (cancer+normal vs cancer+normal) |
| 12-132286017 | cg13251533 | (cancer vs cancer) (cancer+normal vs cancer+normal) | 13-113422497 | cg09714789 | (cancer+normal vs cancer+normal) |
| 12-45751145 | cg22251684 | (cancer+normal vs cancer+normal) | 13-97787063 | cg03550548 | (cancer vs cancer) (cancer vs cancer) (cancer+normal vs cancer+normal) |
| 12-51480175 | cg02764722 | (cancer vs cancer) (cancer+normal vs cancer+normal) | 13-98869706 | cg05194316 | (cancer vs cancer) (cancer+normal vs cancer+normal) |
| 12-63133578 | cg00679520 | (cancer vs cancer) (cancer+normal vs cancer+normal) | 13-98869844 | cg16689193 | (cancer vs cancer) (cancer vs cancer) (cancer+normal vs cancer+normal) |
| 13-113365464 | cg21786786 | (cancer+normal vs cancer+normal) | 14-24741821 | cg18254364 | (cancer+normal vs cancer+normal) |
| 13-24797136 | cg10381113 | (cancer+normal vs cancer+normal) | 14-73894532 | cg21889054 | (cancer+normal vs cancer+normal) |
| 13-28240073 | cg20548564 | (cancer vs cancer) (cancer+normal vs cancer+normal) | 14-91691129 | cg16996571 | (cancer vs cancer) (cancer+normal vs cancer+normal) |
| 13-30107044 | cg10752421 | (cancer vs cancer) (cancer+normal vs cancer+normal) | 14-91691190 | cg14304073 | (cancer vs cancer) (cancer vs cancer) (cancer+normal vs cancer+normal) |
| 13-30107081 | cg13224075 | (cancer vs cancer) (cancer+normal vs cancer+normal) | 15-80217380 | cg22389121 | (normal vs normal) (cancer vs cancer) (cancer vs cancer) (cancer+normal vs cancer+normal) |
| 14-105181796 | cg18519050 | (cancer+normal vs cancer+normal) | 16-2544909 | cg00794055 | (cancer vs cancer) (cancer vs cancer) (cancer+normal vs |

| | | | | | cancer+normal) |
|---|---|---|---|---|---|
| 14-69136587 | cg14144229 | (cancer+normal vs cancer+normal) | 16-29857167 | cg03681150 | (cancer vs cancer) (cancer+normal vs cancer+normal) |
| 14-81865007 | cg10334928 | (cancer vs cancer) (cancer+normal vs cancer+normal) | 16-3139060 | cg20364187 | (cancer vs cancer) (cancer vs cancer) (cancer+normal vs cancer+normal) |
| 15-85215021 | cg19606131 | (normal vs normal) (cancer vs cancer) (cancer+normal vs cancer+normal) | 16-678127 | cg06013113 | (cancer vs cancer) (cancer+normal vs cancer+normal) |
| 16-3193410 | cg02660541 | (cancer vs cancer) (cancer+normal vs cancer+normal) | 17-80970799 | cg03989711 | (cancer vs cancer) (cancer+normal vs cancer+normal) |
| 16-85578516 | cg09479650 | (cancer+normal vs cancer+normal) | 17-80970825 | cg11824564 | (cancer vs cancer) (cancer vs cancer) (cancer+normal vs cancer+normal) |
| 16-87442996 | cg10119288 | (cancer+normal vs cancer+normal) | 17-80970892 | cg10820736 | (cancer+normal vs cancer+normal) |
| 17-30348814 | cg00748958 | (cancer+normal vs cancer+normal) | 17-8928028 | cg01862363 | (cancer vs cancer) (cancer+normal vs cancer+normal) |
| 17-39067081 | cg11809014 | (cancer vs cancer) (cancer+normal vs cancer+normal) | 19-4099405 | cg18156417 | (cancer vs cancer) (cancer vs cancer) (cancer+normal vs cancer+normal) |
| 17-48545805 | cg15506894 | (cancer vs cancer) (cancer+normal vs cancer+normal) | 2-161230046 | cg00754604 | (cancer+normal vs cancer+normal) |
| 17-73805997 | cg05647756 | (cancer vs cancer) (cancer+normal vs cancer+normal) | 2-18736132 | cg05937392 | (normal vs normal) (cancer vs cancer) (cancer vs cancer) (cancer+normal vs cancer+normal) |
| 17-76171794 | cg08115732 | (cancer vs cancer) (cancer+normal vs cancer+normal) | 2-202004766 | cg26627956 | (cancer+normal vs cancer+normal) |
| 17-76425249 | cg00204249 | (cancer vs cancer) (cancer+normal vs cancer+normal) | 2-232113244 | cg27273946 | (normal vs normal) (cancer vs cancer) (cancer vs cancer) (cancer+normal vs cancer+normal) |
| 17-8384595 | cg02059952 | (normal vs normal) (cancer+normal vs cancer+normal) | 2-232135730 | cg18113295 | (cancer vs cancer) (cancer+normal vs cancer+normal) |
| 17-8384607 | cg06557376 | (normal vs normal) (cancer+normal vs cancer+normal) | 2-234373030 | cg08354835 | (cancer vs cancer) (cancer vs cancer) (cancer+normal vs cancer+normal) |
| 19-45574210 | cg08692295 | (cancer+normal vs cancer+normal) | 2-234394478 | cg16719865 | (cancer vs cancer) (cancer vs cancer) (cancer+normal vs cancer+normal) |
| 19-45575023 | cg15732530 | (cancer vs cancer) (cancer+normal vs cancer+normal) | 2-234394541 | cg17212304 | (cancer vs cancer) (cancer vs cancer) (cancer+normal vs cancer+normal) |
| 19-45575134 | cg24030434 | (cancer vs cancer) (cancer+normal vs | 2-234394645 | cg25923162 | (cancer vs cancer) (cancer vs cancer) |

| | | cancer+normal) | | | (cancer+normal vs cancer+normal) |
|---|---|---|---|---|---|
| 19-45575142 | cg23633937 | (cancer vs cancer) (cancer+normal vs cancer+normal) | 2-236672749 | cg0120601 7 | (cancer vs cancer) (cancer vs cancer) (cancer+normal vs cancer+normal) |
| 19-45575442 | cg27077312 | (cancer+normal vs cancer+normal) | 2-242219370 | cg1912508 1 | (cancer+normal vs cancer+normal) |
| 2-121036351 | cg15425061 | (cancer vs cancer) (cancer+normal vs cancer+normal) | 2-37883934 | cg1328658 2 | (cancer vs cancer) (cancer+normal vs cancer+normal) |
| 2-121036440 | cg20349803 | (cancer vs cancer) (cancer+normal vs cancer+normal) | 2-45557351 | cg1280410 4 | (cancer vs cancer) (cancer vs cancer) (cancer+normal vs cancer+normal) |
| 2-121745872 | cg23087358 | (normal vs normal) (cancer+normal vs cancer+normal) | 2-46593223 | cg1399778 8 | (cancer+normal vs cancer+normal) |
| 2-173686677 | cg23253991 | (cancer vs cancer) (cancer+normal vs cancer+normal) | 22-29441760 | cg1866155 0 | (cancer vs cancer) (cancer vs cancer) (cancer+normal vs cancer+normal) |
| 2-236416064 | cg23337031 | (cancer vs cancer) (cancer+normal vs cancer+normal) | 3-128079984 | cg0580106 6 | (cancer vs cancer) (cancer vs cancer) (cancer+normal vs cancer+normal) |
| 2-238406432 | cg00784161 | (cancer vs cancer) (cancer+normal vs cancer+normal) | 3-132077190 | cg2576941 2 | (cancer vs cancer) (cancer+normal vs cancer+normal) |
| 2-241640826 | cg17801295 | (cancer+normal vs cancer+normal) | 3-141161580 | cg0629456 1 | (cancer vs cancer) (cancer vs cancer) (cancer+normal vs cancer+normal) |
| 2-3452919 | cg11589885 | (cancer vs cancer) (cancer+normal vs cancer+normal) | 3-149126842 | cg1143172 3 | (cancer vs cancer) (cancer+normal vs cancer+normal) |
| 2-46909601 | cg23892924 | (cancer+normal vs cancer+normal) | 3-188429875 | cg0444157 7 | (cancer vs cancer) (cancer+normal vs cancer+normal) |
| 2-85622094 | cg27090078 | (cancer vs cancer) (cancer+normal vs cancer+normal) | 3-45004671 | cg2260149 6 | (cancer vs cancer) (cancer+normal vs cancer+normal) |
| 22-46457998 | cg24489344 | (cancer+normal vs cancer+normal) | 3-72537392 | cg1549138 5 | (cancer vs cancer) (cancer vs cancer) (cancer+normal vs cancer+normal) |
| 3-146026497 | cg21552014 | (cancer+normal vs cancer+normal) | 4-121991345 | cg0095769 8 | (cancer+normal vs cancer+normal) |
| 4-141564017 | cg03135535 | (cancer vs cancer) (cancer+normal vs cancer+normal) | 4-121991576 | cg1083265 5 | (cancer+normal vs cancer+normal) |
| 4-141564271 | cg06147143 | (cancer vs cancer) (cancer+normal vs cancer+normal) | 4-184941494 | cg1706115 6 | (cancer+normal vs cancer+normal) |
| 4-48301717 | cg26306052 | (normal vs normal) (cancer vs cancer) (cancer+normal vs cancer+normal) | 4-8386188 | cg0820607 9 | (cancer vs cancer) (cancer+normal vs cancer+normal) |
| 5-126182526 | cg21910416 | (cancer vs cancer) (cancer+normal vs cancer+normal) | 4-8386198 | cg1899918 5 | (cancer vs cancer) (cancer vs cancer) (cancer+normal vs |

| | | | | | |
|---|---|---|---|---|---|
| | | | | | cancer+normal) |
| 5-131806593 | cg04659396 | (cancer vs cancer) (cancer+normal vs cancer+normal) | 5-131408149 | cg16465129 | (cancer vs cancer) (cancer vs cancer) (cancer+normal vs cancer+normal) |
| 5-137803102 | cg09102257 | (normal vs normal) (cancer+normal vs cancer+normal) | 5-134451535 | cg23201938 | (cancer+normal vs cancer+normal) |
| 5-57753951 | cg02077216 | (cancer+normal vs cancer+normal) | 5-141722174 | cg02084814 | (cancer vs cancer) (cancer vs cancer) (cancer+normal vs cancer+normal) |
| 5-79070077 | cg07209550 | (normal vs normal) (cancer+normal vs cancer+normal) | 5-143026910 | cg23194776 | (cancer vs cancer) (cancer vs cancer) (cancer+normal vs cancer+normal) |
| 6-11279680 | cg15972264 | (cancer+normal vs cancer+normal) | 5-1518133 | cg22185977 | (cancer+normal vs cancer+normal) |
| 6-137493017 | cg00415333 | (cancer vs cancer) (cancer+normal vs cancer+normal) | 5-1519265 | cg26934362 | (cancer+normal vs cancer+normal) |
| 6-32135061 | cg17527814 | (cancer+normal vs cancer+normal) | 5-1546960 | cg02165151 | (cancer vs cancer) (cancer vs cancer) (cancer+normal vs cancer+normal) |
| 6-32135083 | cg13197308 | (cancer vs cancer) (cancer+normal vs cancer+normal) | 6-108615613 | cg17969853 | (cancer vs cancer) (cancer+normal vs cancer+normal) |
| 6-32135186 | cg11108115 | (cancer vs cancer) (cancer+normal vs cancer+normal) | 6-143575123 | cg08612271 | (cancer vs cancer) (cancer vs cancer) (cancer+normal vs cancer+normal) |
| 6-32135195 | cg08472222 | (cancer vs cancer) (cancer+normal vs cancer+normal) | 6-143834261 | cg10134204 | (normal vs normal) (cancer vs cancer) (cancer+normal vs cancer+normal) |
| 6-32135370 | cg10262770 | (cancer vs cancer) (cancer+normal vs cancer+normal) | 6-170047483 | cg11412527 | (cancer vs cancer) (cancer vs cancer) (cancer+normal vs cancer+normal) |
| 6-32135379 | cg07754347 | (cancer vs cancer) (cancer+normal vs cancer+normal) | 6-25726912 | cg07077277 | (cancer vs cancer) (cancer vs cancer) (cancer+normal vs cancer+normal) |
| 6-32141591 | cg09597192 | (cancer+normal vs cancer+normal) | 6-28875356 | cg14059339 | (cancer vs cancer) (cancer vs cancer) (cancer+normal vs cancer+normal) |
| 6-33288734 | cg05147195 | (cancer+normal vs cancer+normal) | 6-28875370 | cg08279189 | (cancer vs cancer) (cancer vs cancer) (cancer+normal vs cancer+normal) |
| 6-33288773 | cg14499959 | (normal vs normal) (cancer+normal vs cancer+normal) | 6-30900678 | cg10006979 | (cancer vs cancer) (cancer vs cancer) (cancer+normal vs cancer+normal) |
| 6-33288785 | cg21350498 | (cancer+normal vs cancer+normal) | 6-33156236 | cg03790047 | (cancer vs cancer) (cancer+normal vs cancer+normal) |
| 6-33288804 | cg15070677 | (normal vs normal) (cancer vs cancer) | 6-39072458 | cg21645089 | (cancer vs cancer) (cancer vs cancer) |

| | | (cancer+normal vs cancer+normal) | | | (cancer+normal vs cancer+normal) |
|---|---|---|---|---|---|
| 6-33288851 | cg18704865 | (cancer+normal vs cancer+normal) | 6-39159259 | cg00859574 | (cancer vs cancer) (cancer+normal vs cancer+normal) |
| 6-33288891 | cg07098566 | (normal vs normal) (cancer vs cancer) (cancer+normal vs cancer+normal) | 6-42678314 | cg21228424 | (cancer vs cancer) (cancer+normal vs cancer+normal) |
| 6-33288941 | cg03243551 | (cancer+normal vs cancer+normal) | 6-46890102 | cg10770662 | (cancer vs cancer) (cancer+normal vs cancer+normal) |
| 6-33288964 | cg14199833 | (cancer+normal vs cancer+normal) | 6-46890220 | cg03558010 | (cancer vs cancer) (cancer vs cancer) (cancer+normal vs cancer+normal) |
| 6-33289243 | cg20849032 | (normal vs normal) (cancer+normal vs cancer+normal) | 6-46890512 | cg13975362 | (cancer+normal vs cancer+normal) |
| 7-127984393 | cg08309135 | (cancer vs cancer) (cancer+normal vs cancer+normal) | 7-128767280 | cg08281469 | (cancer vs cancer) (cancer vs cancer) (cancer+normal vs cancer+normal) |
| 7-154788856 | cg22030483 | (cancer vs cancer) (cancer+normal vs cancer+normal) | 7-129778936 | cg04334751 | (cancer vs cancer) (cancer vs cancer) (cancer+normal vs cancer+normal) |
| 7-2030229 | cg05434287 | (cancer+normal vs cancer+normal) | 7-2473529 | cg17907628 | (cancer vs cancer) (cancer vs cancer) (cancer+normal vs cancer+normal) |
| 7-4169604 | cg08250787 | (normal vs normal) (cancer+normal vs cancer+normal) | 7-2473859 | cg18259487 | (cancer vs cancer) (cancer vs cancer) (cancer+normal vs cancer+normal) |
| 7-4169630 | cg13818127 | (normal vs normal) (cancer+normal vs cancer+normal) | 7-4786943 | cg25594100 | (cancer+normal vs cancer+normal) |
| 7-609693 | cg04666346 | (cancer+normal vs cancer+normal) | 7-5262452 | cg08310400 | (cancer vs cancer) (cancer vs cancer) (cancer+normal vs cancer+normal) |
| 7-6689218 | cg21383341 | (cancer vs cancer) (cancer+normal vs cancer+normal) | 7-5272208 | cg15277378 | (normal vs normal) (cancer+normal vs cancer+normal) |
| 8-128403369 | cg11118198 | (normal vs normal) (cancer vs cancer) (cancer+normal vs cancer+normal) | 7-5272275 | cg20099458 | (cancer vs cancer) (cancer+normal vs cancer+normal) |
| 8-144568432 | cg26185884 | (cancer+normal vs cancer+normal) | 7-98996656 | cg19420101 | (cancer vs cancer) (cancer vs cancer) (cancer+normal vs cancer+normal) |
| 8-41798556 | cg21603275 | (cancer vs cancer) (cancer+normal vs cancer+normal) | 8-17655156 | cg18109537 | (cancer vs cancer) (cancer vs cancer) (cancer+normal vs cancer+normal) |
| 8-56882373 | cg13475877 | (cancer+normal vs cancer+normal) | 8-28625366 | cg26333660 | (cancer vs cancer) (cancer+normal vs cancer+normal) |
| 8- | cg05323683 | (cancer vs cancer) | 8-28950303 | cg0723765 | (cancer+normal vs |

| | | | | | |
|---|---|---|---|---|---|
| 95168291 | | (cancer+normal vs cancer+normal) | | 3 | cancer+normal) |
| 9-108424905 | cg06119575 | (cancer vs cancer) (cancer+normal vs cancer+normal) | 9-126182794 | cg13413792 | (cancer+normal vs cancer+normal) |
| 9-97856079 | cg13763287 | (cancer vs cancer) (cancer+normal vs cancer+normal) | 9-14690531 | cg21185283 | (cancer vs cancer) (cancer vs cancer) (cancer+normal vs cancer+normal) |
| **(normal vs normal)** | | | **(normal vs normal)** | | |
| **Status - Hyper** | | | **Status - Hyper** | | |
| 1-119535500 | cg07817024 | (normal vs normal) | 1-161008542 | cg11731300 | (normal vs normal) |
| 1-119542295 | cg24434800 | (normal vs normal) | 1-173990399 | cg12420283 | (normal vs normal) |
| 1-212839040 | cg03739354 | (normal vs normal) (cancer+normal vs cancer+normal) | 1-184355420 | cg05135156 | (normal vs normal) |
| 1-219834537 | cg03599978 | (cancer vs cancer) (normal vs normal) (cancer+normal vs cancer+normal) | 1-201708836 | cg16023122 | (normal vs normal) (cancer+normal vs cancer+normal) |
| 1-226867960 | cg22444124 | (normal vs normal) | 1-201709135 | cg14377596 | (normal vs normal) (cancer+normal vs cancer+normal) |
| 1-6064094 | cg03895593 | (normal vs normal) | 1-201915989 | cg16966962 | (normal vs normal) |
| 10-126043549 | cg18736101 | (normal vs normal) | 1-201978949 | cg03254916 | (normal vs normal) |
| 10-28036151 | cg14947429 | (normal vs normal) | 1-23668691 | cg00907427 | (cancer vs cancer) (cancer vs cancer) (normal vs normal) (cancer+normal vs cancer+normal) |
| 11-61062837 | cg27175288 | (cancer vs cancer) (normal vs normal) (cancer+normal vs cancer+normal) | 1-23668792 | cg08718097 | (cancer vs cancer) (cancer vs cancer) (normal vs normal) (cancer+normal vs cancer+normal) |
| 11-61062843 | cg05190577 | (cancer vs cancer) (normal vs normal) (cancer+normal vs cancer+normal) | 1-241802556 | cg02820309 | (normal vs normal) |
| 11-61062902 | cg07402729 | (normal vs normal) | 1-247171572 | cg22502715 | (normal vs normal) |
| 11-61062910 | cg06282270 | (cancer vs cancer) (normal vs normal) (cancer+normal vs cancer+normal) | 1-26800075 | cg19621076 | (normal vs normal) |
| 11-61062964 | cg01698298 | (normal vs normal) | 1-45082704 | cg24591770 | (normal vs normal) |
| 11-71952356 | cg23619769 | (normal vs normal) | 1-8042702 | cg12809925 | (normal vs normal) |
| 11-8615356 | cg26668837 | (normal vs normal) | 1-85667951 | cg22949578 | (normal vs normal) |
| 12-16757954 | cg01181415 | (normal vs normal) | 10-52835002 | cg05867154 | (normal vs normal) |
| 12-16757985 | cg10143811 | (normal vs normal) | 10-93392399 | cg26667395 | (normal vs normal) |
| 12-54405379 | cg18540674 | (normal vs normal) | 11-19368277 | cg22855325 | (normal vs normal) |

| | | | | | |
|---|---|---|---|---|---|
| 12-570155 | cg26388816 | (normal vs normal) | 11-61735789 | cg25270670 | (normal vs normal) |
| 13-37004990 | cg24154053 | (normal vs normal) | 11-64577338 | cg22897141 | (normal vs normal) |
| 15-37180325 | cg11092048 | (normal vs normal) | 11-8832111 | cg19113668 | (normal vs normal) (cancer+normal vs cancer+normal) |
| 15-73611451 | cg08450752 | (normal vs normal) | 12-108078821 | cg00632811 | (normal vs normal) |
| 16-60233305 | cg08511886 | (normal vs normal) | 12-4384879 | cg18584387 | (normal vs normal) |
| 16-88496788 | cg03314029 | (normal vs normal) | 12-53399544 | cg24502904 | (normal vs normal) |
| 16-88497007 | cg07215504 | (normal vs normal) | 12-66286141 | cg21822187 | (normal vs normal) |
| 17-42988784 | cg02407342 | (normal vs normal) | 12-68044208 | cg13442969 | (normal vs normal) |
| 17-46666926 | cg02642822 | (normal vs normal) | 12-75874499 | cg09171882 | (normal vs normal) |
| 17-46666958 | cg09704116 | (normal vs normal) | 12-86267881 | cg18877737 | (normal vs normal) |
| 17-46667587 | cg10588962 | (normal vs normal) | 14-100659756 | cg23197867 | (normal vs normal) |
| 17-46667683 | cg21242356 | (normal vs normal) | 14-50088544 | cg11700584 | (normal vs normal) |
| 17-73073700 | cg25660390 | (normal vs normal) | 14-57859336 | cg01639635 | (normal vs normal) |
| 17-73073940 | cg16757821 | (normal vs normal) | 14-93581049 | cg09257735 | (normal vs normal) |
| 19-13209548 | cg00689651 | (normal vs normal) | 16-30709162 | cg05979332 | (normal vs normal) |
| 19-47507461 | cg17431280 | (normal vs normal) | 16-87351824 | cg10067538 | (normal vs normal) |
| 19-5339092 | cg24122124 | (normal vs normal) | 17-16947406 | cg20898358 | (normal vs normal) |
| 2-105464104 | cg06292076 | (normal vs normal) | 17-4802735 | cg05529816 | (normal vs normal) |
| 2-119589844 | cg10330950 | (normal vs normal) | 17-54655325 | cg08527324 | (normal vs normal) |
| 2-119592325 | cg10543450 | (normal vs normal) | 18-60985645 | cg17602451 | (normal vs normal) |
| 2-119594696 | cg02958634 | (normal vs normal) | 19-10444506 | cg07976603 | (normal vs normal) |
| 2-119607603 | cg18093372 | (normal vs normal) | 19-1356315 | cg23057220 | (normal vs normal) |
| 2-119608121 | cg25317460 | (cancer vs cancer) (normal vs normal) | 19-40732518 | cg12099279 | (normal vs normal) |
| 2-119608651 | cg21301942 | (normal vs normal) | 19-45281006 | cg23635599 | (normal vs normal) |
| 2-119609592 | cg11236727 | (normal vs normal) | 19-5624574 | cg21201657 | (normal vs normal) |
| 2-119609642 | cg13367169 | (normal vs normal) | 19-57831549 | cg14603375 | (normal vs normal) |
| 2-145210222 | cg12336777 | (normal vs normal) | 2-17699904 | cg15124757 | (normal vs normal) |
| 2-220375605 | cg20509869 | (normal vs normal) | 2-177043427 | cg10305451 | (normal vs normal) |
| 2-220379044 | cg17610929 | (normal vs normal) (cancer+normal vs cancer+normal) | 2-190044601 | cg07875385 | (normal vs normal) |
| 2- | cg24743778 | (normal vs normal) | 2- | cg2277447 | (normal vs normal) |

| | | | | | |
|---|---|---|---|---|---|
| 220379058 | | (cancer+normal vs cancer+normal) | 190044636 | 2 | |
| 2-220379199 | cg12046414 | (normal vs normal) (cancer+normal vs cancer+normal) | 2-191224886 | cg24663311 | (normal vs normal) |
| 20-62092502 | cg14498592 | (normal vs normal) | 2-196943333 | cg17288643 | (normal vs normal) |
| 22-43116077 | cg00241712 | (normal vs normal) | 2-28112917 | cg03177025 | (normal vs normal) |
| 3-137488697 | cg06346667 | (normal vs normal) | 2-43450965 | cg00968638 | (normal vs normal) |
| 3-157816094 | cg09095222 | (cancer vs cancer) (normal vs normal) (cancer+normal vs cancer+normal) | 2-43451842 | cg16876647 | (cancer vs cancer) (normal vs normal) (cancer+normal vs cancer+normal) |
| 3-184209105 | cg18482112 | (normal vs normal) | 2-43451868 | cg08193650 | (normal vs normal) |
| 3-184209224 | cg00786761 | (normal vs normal) | 2-96926568 | cg11714752 | (normal vs normal) |
| 3-36248859 | cg24931191 | (normal vs normal) | 20-43160249 | cg19525717 | (normal vs normal) |
| 3-85010677 | cg03416562 | (cancer vs cancer) (normal vs normal) (cancer+normal vs cancer+normal) | 20-52211744 | cg15871544 | (normal vs normal) |
| 3-99595026 | cg20276377 | (normal vs normal) | 21-38362725 | cg14785464 | (normal vs normal) |
| 3-99595145 | cg22092811 | (normal vs normal) | 21-38362727 | cg06167719 | (normal vs normal) |
| 4-102712010 | cg00332153 | (normal vs normal) | 22-24237672 | cg10819733 | (normal vs normal) |
| 4-102712216 | cg27140058 | (normal vs normal) | 3-112770545 | cg00872865 | (normal vs normal) |
| 4-102712397 | cg14855874 | (normal vs normal) | 3-149530294 | cg13512830 | (normal vs normal) |
| 4-111545628 | cg17929238 | (normal vs normal) | 3-184033620 | cg06398251 | (normal vs normal) |
| 5-124126863 | cg01876548 | (normal vs normal) | 3-25469125 | cg00371702 | (normal vs normal) |
| 5-139525657 | cg08763705 | (normal vs normal) | 3-50402563 | cg02855924 | (normal vs normal) |
| 5-172673098 | cg08506654 | (normal vs normal) | 3-50402751 | cg24159214 | (normal vs normal) |
| 5-1875548 | cg17629929 | (normal vs normal) | 4-139773369 | cg11537946 | (normal vs normal) |
| 5-1875611 | cg14024788 | (normal vs normal) | 4-154143694 | cg12453905 | (normal vs normal) (cancer+normal vs cancer+normal) |
| 5-1875886 | cg18856388 | (normal vs normal) | 4-24913973 | cg24371114 | (normal vs normal) |
| 5-33309433 | cg03889507 | (normal vs normal) | 4-57521657 | cg10800833 | (normal vs normal) |
| 5-3589702 | cg01549315 | (normal vs normal) | 4-57522632 | cg24852548 | (normal vs normal) |
| 5-3592435 | cg19814981 | (normal vs normal) | 5-15538923 | cg08849558 | (normal vs normal) |
| 5-3592466 | cg09382127 | (normal vs normal) | 5-35618383 | cg24892139 | (normal vs normal) |
| 5-67483678 | cg17780731 | (normal vs normal) | 5-55776364 | cg26646659 | (normal vs normal) |
| 6- | cg12646820 | (normal vs normal) | 5-60921891 | cg1363424 | (normal vs normal) |

| | | | | | |
|---|---|---|---|---|---|
| 13924406 | | | | 2 | |
| 6-18990650 | cg05065239 | (normal vs normal) | 5-87439429 | cg13672078 | (normal vs normal) |
| 6-18990714 | cg25042346 | (normal vs normal) (cancer+normal vs cancer+normal) | 5-87440121 | cg10830145 | (normal vs normal) |
| 6-3053739 | cg02335642 | (cancer vs cancer) (normal vs normal) (cancer+normal vs cancer+normal) | 6-139013146 | cg18136963 | (normal vs normal) |
| 6-50819054 | cg04276508 | (normal vs normal) | 6-152623304 | cg05917732 | (cancer vs cancer) (cancer vs cancer) (normal vs normal) (cancer+normal vs cancer+normal) |
| 6-50819236 | cg07436152 | (normal vs normal) | 6-152623387 | cg02682457 | (cancer vs cancer) (cancer vs cancer) (normal vs normal) (cancer+normal vs cancer+normal) |
| 6-56111812 | cg07143470 | (normal vs normal) | 6-152623479 | cg12023625 | (cancer vs cancer) (cancer vs cancer) (normal vs normal) (cancer+normal vs cancer+normal) |
| 6-5994588 | cg20421446 | (normal vs normal) | 6-27101662 | cg01533108 | (normal vs normal) |
| 7-100091255 | cg24525457 | (normal vs normal) | 6-27255829 | cg07739113 | (normal vs normal) |
| 7-100091364 | cg03113878 | (normal vs normal) | 6-33560953 | cg19735538 | (normal vs normal) |
| 7-27208285 | cg20884887 | (normal vs normal) | 6-38683221 | cg03166753 | (normal vs normal) |
| 7-27215606 | cg27157482 | (normal vs normal) | 6-42859023 | cg06493154 | (normal vs normal) |
| 7-27215610 | cg02483701 | (normal vs normal) | 6-43021181 | cg07495357 | (normal vs normal) |
| 7-27218867 | cg10724867 | (normal vs normal) | 6-5999186 | cg14386951 | (normal vs normal) |
| 7-27220049 | cg05517976 | (normal vs normal) | 6-72892597 | cg24401219 | (normal vs normal) |
| 7-27226329 | cg00705992 | (normal vs normal) | 6-72892901 | cg16655338 | (normal vs normal) |
| 8-142428240 | cg00467244 | (normal vs normal) | 7-100465051 | cg12507655 | (normal vs normal) |
| 8-22562469 | cg10423052 | (normal vs normal) | 7-134576290 | cg02087931 | (normal vs normal) |
| 8-24815840 | cg15416643 | (normal vs normal) | 7-27187556 | cg17994139 | (normal vs normal) |
| 8-26721736 | cg09557462 | (normal vs normal) | 7-27187691 | cg12110087 | (normal vs normal) |
| 8-57594909 | cg21752469 | (normal vs normal) (cancer+normal vs cancer+normal) | 7-99769432 | cg01942863 | (normal vs normal) |
| 8-66472955 | cg21935519 | (normal vs normal) | 8-131961316 | cg23686556 | (normal vs normal) |
| 9-129372349 | cg01027151 | (cancer vs cancer) (normal vs normal) (cancer+normal vs cancer+normal) | 8-144161874 | cg05251359 | (normal vs normal) |
| 9- | cg15751452 | (normal vs normal) | 8- | cg0211085 | (cancer vs cancer) |

| | | |
|---|---|---|
| 129372974 | | |
| 9-129373499 | cg19337180 | (cancer vs cancer) (normal vs normal) (cancer+normal vs cancer+normal) |
| 9-129387303 | cg12777520 | (cancer vs cancer) (normal vs normal) (cancer+normal vs cancer+normal) |
| **Status - Hypo** | | |
| 1-1144658 | cg02296931 | (normal vs normal) |
| 1-230457906 | cg04095826 | (normal vs normal) |
| 1-230457961 | cg05266497 | (normal vs normal) |
| 1-2827833 | cg15356461 | (normal vs normal) |
| 10-134692167 | cg02638348 | (normal vs normal) |
| 10-134692406 | cg03653518 | (normal vs normal) |
| 10-134774781 | cg01307776 | (normal vs normal) |
| 10-134944446 | cg15595525 | (normal vs normal) |
| 10-135018298 | cg14636714 | (normal vs normal) |
| 10-1531034 | cg07660114 | (normal vs normal) |
| 10-1767830 | cg01341216 | (normal vs normal) |
| 10-30980898 | cg06655291 | (normal vs normal) |
| 11-101787410 | cg26682904 | (normal vs normal) |
| 11-110862216 | cg25615926 | (normal vs normal) |
| 12-10339077 | cg26467753 | (normal vs normal) |
| 12-124788627 | cg25368917 | (normal vs normal) |
| 12-124788791 | cg03024698 | (normal vs normal) |
| 12-124788814 | cg05712979 | (normal vs normal) |
| 12-124789036 | cg18672389 | (normal vs normal) |
| 12-133380471 | cg10158717 | (normal vs normal) |
| 13-113260139 | cg03555276 | (normal vs normal) |
| 13-74780742 | cg25506686 | (normal vs normal) |
| 14-100658902 | cg23112821 | (normal vs normal) |
| 14-105048531 | cg13276428 | (normal vs normal) |

| | | |
|---|---|---|
| 145028222 | 8 | (normal vs normal) |
| 8-145028230 | cg05595301 | (normal vs normal) |
| 9-130741881 | cg07954423 | (normal vs normal) |
| **Status - Hypo** | | |
| 1-112299491 | cg22728128 | (normal vs normal) |
| 1-203763196 | cg00862417 | (normal vs normal) |
| 1-6501240 | cg24050109 | (normal vs normal) |
| 10-34998341 | cg11161396 | (normal vs normal) |
| 11-116612907 | cg13385644 | (normal vs normal) |
| 11-120911604 | cg08228600 | (normal vs normal) |
| 11-33730665 | cg25080571 | (normal vs normal) |
| 11-78075960 | cg05100279 | (normal vs normal) |
| 11-96117844 | cg25189608 | (normal vs normal) |
| 12-101413143 | cg00605628 | (normal vs normal) |
| 12-117588951 | cg18860868 | (normal vs normal) |
| 12-12421246 | cg24776498 | (normal vs normal) |
| 12-130711711 | cg22808438 | (normal vs normal) |
| 12-24850642 | cg06887454 | (normal vs normal) |
| 12-53610772 | cg21746001 | (normal vs normal) |
| 12-94619636 | cg06595153 | (normal vs normal) |
| 13-113356724 | cg20279561 | (normal vs normal) |
| 13-113357112 | cg00990020 | (normal vs normal) |
| 13-113400754 | cg08247289 | (normal vs normal) |
| 13-113420371 | cg08557188 | (normal vs normal) (cancer vs cancer) (cancer vs cancer) (cancer+normal vs cancer+normal) |
| 13-113423268 | cg18748064 | (normal vs normal) |
| 13-113425298 | cg21163969 | (normal vs normal) |
| 13-19759420 | cg03399574 | (normal vs normal) |
| 14-100305758 | cg19737540 | (normal vs normal) |

| | | | | | |
|---|---|---|---|---|---|
| 14-30649039 | cg05055001 | (normal vs normal) | 14-35447884 | cg16400425 | (normal vs normal) |
| 15-85215021 | cg19606131 | (normal vs normal) (cancer vs cancer) (cancer+normal vs cancer+normal) | 14-70757518 | cg06796682 | (normal vs normal) |
| 16-1154951 | cg04727521 | (normal vs normal) | 14-75522151 | cg01546919 | (normal vs normal) |
| 16-1478504 | cg27239473 | (normal vs normal) | 14-89254566 | cg26215807 | (normal vs normal) |
| 16-49525139 | cg06186992 | (normal vs normal) | 15-77481069 | cg13168698 | (normal vs normal) |
| 16-49525225 | cg02101892 | (normal vs normal) | 15-80217380 | cg22389121 | (normal vs normal) (cancer vs cancer) (cancer vs cancer) (cancer+normal vs cancer+normal) |
| 16-75148743 | cg07565228 | (normal vs normal) | 15-93995636 | cg09449012 | (normal vs normal) |
| 16-86756576 | cg06678912 | (normal vs normal) | 16-4717772 | cg03420907 | (normal vs normal) |
| 16-88166905 | cg27271738 | (normal vs normal) | 16-56995739 | cg26624021 | (normal vs normal) |
| 17-40440022 | cg16777510 | (normal vs normal) | 16-86121574 | cg05167857 | (normal vs normal) |
| 17-58499911 | cg02172058 | (normal vs normal) | 17-13502304 | cg02400773 | (normal vs normal) |
| 17-80278947 | cg09340279 | (normal vs normal) | 17-39136589 | cg17703934 | (normal vs normal) |
| 17-8384595 | cg02059952 | (normal vs normal) (cancer+normal vs cancer+normal) | 17-62778230 | cg25333602 | (normal vs normal) |
| 17-8384607 | cg06557376 | (normal vs normal) (cancer+normal vs cancer+normal) | 17-76102450 | cg18748888 | (normal vs normal) |
| 17-8814822 | cg07335619 | (normal vs normal) | 17-79047681 | cg16831331 | (normal vs normal) |
| 18-10589357 | cg07277756 | (normal vs normal) | 19-18359562 | cg09914513 | (normal vs normal) |
| 18-46987786 | cg23865597 | (normal vs normal) | 19-39696134 | cg15427040 | (normal vs normal) |
| 18-77236335 | cg01514831 | (normal vs normal) | 2-18736132 | cg05937392 | (normal vs normal) (cancer vs cancer) (cancer vs cancer) (cancer+normal vs cancer+normal) |
| 2-121745872 | cg23087358 | (normal vs normal) (cancer+normal vs cancer+normal) | 2-232113244 | cg27273946 | (normal vs normal) (cancer vs cancer) (cancer vs cancer) (cancer+normal vs cancer+normal) |
| 2-162164127 | cg13806767 | (normal vs normal) | 2-25190741 | cg20731999 | (normal vs normal) |
| 2-169927922 | cg00768195 | (normal vs normal) | 2-61352525 | cg13409174 | (normal vs normal) (cancer vs cancer) |
| 2-240040219 | cg07215298 | (normal vs normal) | 2-95538610 | cg15371418 | (normal vs normal) |
| 2-240531507 | cg17249452 | (normal vs normal) | 22-31336785 | cg23825480 | (normal vs normal) |
| 2-241640444 | cg22594737 | (normal vs normal) | 22-37491595 | cg17956747 | (normal vs normal) |

| | | | | | |
|---|---|---|---|---|---|
| 2-242791120 | cg11503661 | (normal vs normal) | 22-43193524 | cg27120833 | (normal vs normal) |
| 2-98351171 | cg21585640 | (normal vs normal) | 3-100516031 | cg02493205 | (normal vs normal) |
| 20-170368 | cg02301189 | (normal vs normal) | 3-28386748 | cg15564207 | (normal vs normal) |
| 20-32859896 | cg04343045 | (normal vs normal) | 3-46985840 | cg16306228 | (normal vs normal) |
| 20-5896145 | cg18082354 | (normal vs normal) | 3-52802660 | cg02636488 | (normal vs normal) |
| 21-37756397 | cg00660452 | (normal vs normal) | 3-53161967 | cg21154426 | (normal vs normal) |
| 21-47458587 | cg22391205 | (normal vs normal) | 3-56503840 | cg07100595 | (normal vs normal) |
| 3-138170552 | cg04554240 | (normal vs normal) | 3-61826848 | cg27606618 | (normal vs normal) |
| 3-138170939 | cg05301252 | (normal vs normal) | 4-125369980 | cg10792660 | (normal vs normal) |
| 3-195946921 | cg04855678 | (normal vs normal) | 4-156364125 | cg27179353 | (normal vs normal) |
| 3-54067965 | cg08489309 | (normal vs normal) | 4-184441284 | cg22935533 | (normal vs normal) |
| 3-64524911 | cg07777540 | (normal vs normal) | 4-25071580 | cg13279643 | (normal vs normal) |
| 3-96494778 | cg13491005 | (normal vs normal) | 4-42504518 | cg18695665 | (normal vs normal) |
| 4-1049922 | cg03744197 | (normal vs normal) | 5-110077272 | cg26660316 | (normal vs normal) |
| 4-153549242 | cg09527687 | (normal vs normal) | 5-159448398 | cg16186001 | (normal vs normal) |
| 4-1994462 | cg04996238 | (normal vs normal) | 5-159820339 | cg11151402 | (normal vs normal) |
| 4-48301717 | cg26306052 | (normal vs normal) (cancer vs cancer) (cancer+normal vs cancer+normal) | 5-54922354 | cg05462905 | (normal vs normal) |
| 5-137803102 | cg09102257 | (normal vs normal) (cancer+normal vs cancer+normal) | 5-73872294 | cg04719634 | (normal vs normal) |
| 5-13960359 | cg06825415 | (normal vs normal) | 5-9348193 | cg02397179 | (normal vs normal) |
| 5-160335 | cg26612409 | (normal vs normal) | 6-141520754 | cg17016145 | (normal vs normal) |
| 5-160518 | cg09444426 | (normal vs normal) | 6-143834261 | cg10134204 | (normal vs normal) (cancer vs cancer) (cancer+normal vs cancer+normal) |
| 5-167836834 | cg05941376 | (normal vs normal) | 6-146203625 | cg04409048 | (normal vs normal) |
| 5-177980755 | cg07841353 | (normal vs normal) | 6-15298461 | cg04097131 | (normal vs normal) |
| 5-38446192 | cg05606376 | (normal vs normal) | 6-157210471 | cg10059410 | (normal vs normal) |
| 5-79070077 | cg07209550 | (normal vs normal) (cancer+normal vs cancer+normal) | 6-27116113 | cg02704907 | (normal vs normal) |
| 6-15243923 | cg02084214 | (normal vs normal) | 6-28496405 | cg08944443 | (normal vs normal) |
| 6-156983304 | cg08839808 | (normal vs normal) | 6-28542563 | cg05241721 | (normal vs normal) |
| 6- | cg00269725 | (normal vs normal) | 6-39637466 | cg1547898 | (normal vs normal) |

| | | | | | |
|---|---|---|---|---|---|
| 156983315 | | | | 1 | |
| 6-32180238 | cg00780364 | (normal vs normal) | 6-41934765 | cg02524834 | (normal vs normal) |
| 6-32180305 | cg01873637 | (normal vs normal) | 6-85328105 | cg07213771 | (normal vs normal) |
| 6-33288561 | cg16315106 | (normal vs normal) | 6-97752691 | cg01823120 | (normal vs normal) |
| 6-33288773 | cg14499959 | (normal vs normal) (cancer+normal vs cancer+normal) | 7-115977777 | cg20301677 | (normal vs normal) |
| 6-33288804 | cg15070677 | (normal vs normal) (cancer vs cancer) (cancer+normal vs cancer+normal) | 7-116164108 | cg25515317 | (normal vs normal) |
| 6-33288891 | cg07098566 | (normal vs normal) (cancer vs cancer) (cancer+normal vs cancer+normal) | 7-116659609 | cg03280299 | (normal vs normal) |
| 6-33289202 | cg19384106 | (normal vs normal) | 7-127589252 | cg06768393 | (normal vs normal) |
| 6-33289243 | cg20849032 | (normal vs normal) (cancer+normal vs cancer+normal) | 7-142723312 | cg11480029 | (normal vs normal) |
| 7-148951560 | cg15176338 | (normal vs normal) | 7-156434403 | cg20964021 | (normal vs normal) |
| 7-1632265 | cg01669161 | (normal vs normal) | 7-158551108 | cg16762085 | (normal vs normal) |
| 7-1976964 | cg01178603 | (normal vs normal) | 7-5272208 | cg15277378 | (normal vs normal) (cancer+normal vs cancer+normal) |
| 7-4169604 | cg08250787 | (normal vs normal) (cancer+normal vs cancer+normal) | 7-56131519 | cg10233953 | (normal vs normal) |
| 7-4169630 | cg13818127 | (normal vs normal) (cancer+normal vs cancer+normal) | 7-65941812 | cg13147721 | (normal vs normal) |
| 7-43575891 | cg06931356 | (normal vs normal) | 7-77126994 | cg02166725 | (normal vs normal) (cancer vs cancer) (cancer vs cancer) |
| 8-126403700 | cg15149205 | (normal vs normal) | 7-77584545 | cg19396253 | (normal vs normal) (cancer vs cancer) |
| 8-128403369 | cg11118198 | (normal vs normal) (cancer vs cancer) (cancer+normal vs cancer+normal) | 8-117777782 | cg14084888 | (normal vs normal) |
| 8-328499 | cg20223392 | (normal vs normal) | 8-126216633 | cg03579446 | (normal vs normal) |
| 8-50653901 | cg04418647 | (normal vs normal) | 8-134224890 | cg15463563 | (normal vs normal) |
| 8-50653939 | cg10951117 | (normal vs normal) | 8-135813202 | cg01417339 | (normal vs normal) |
| 8-73848937 | cg23244463 | (normal vs normal) | 8-30240920 | cg21622977 | (normal vs normal) |
| 8-73849039 | cg08294044 | (normal vs normal) | 8-62412760 | cg16648094 | (normal vs normal) (cancer vs cancer) |
| 8-81213351 | cg19591612 | (normal vs normal) (cancer vs cancer) | 8-82395948 | cg07234508 | (normal vs normal) |
| 9-129349764 | cg13604154 | (normal vs normal) | 8-93487304 | cg22013407 | (normal vs normal) |
| 9-134724316 | cg13706315 | (normal vs normal) | 8-93581364 | cg01188466 | (normal vs normal) |

| BrCaG (cancer vs cancer) | | | LUSC (cancer vs cancer) | | |
|---|---|---|---|---|---|
| Status - Hyper | | | Status - Hyper | | |
| 1-12678641 | cg17935811 | (cancer vs cancer) (normal vs normal) (cancer+normal vs cancer+normal) | 1-18969717 | cg07221967 | (cancer vs cancer) |
| 1-12678761 | cg01346152 | (cancer vs cancer) (normal vs normal) (cancer+normal vs cancer+normal) | 1-230850050 | cg24474852 | (cancer vs cancer) |
| 1-212769641 | cg10985914 | (cancer vs cancer) (normal vs normal) (cancer+normal vs cancer+normal) | 1-24885664 | cg03655701 | (cancer vs cancer) |
| 1-24828586 | cg06718763 | (cancer vs cancer) | 1-33160900 | cg17339202 | (cancer vs cancer) |
| 1-26690537 | cg12813724 | (cancer vs cancer) | 1-44884096 | cg13880950 | (cancer vs cancer) |
| 1-27894955 | cg12610832 | (cancer vs cancer) (cancer vs cancer) (cancer+normal vs cancer+normal) | 1-44884109 | cg12548634 | (cancer vs cancer) |
| 1-59280952 | cg15687600 | (cancer vs cancer) (normal vs normal) (cancer+normal vs cancer+normal) | 1-44884150 | cg11715828 | (cancer vs cancer) |
| 1-59281067 | cg03611060 | (cancer vs cancer) (normal vs normal) (cancer+normal vs cancer+normal) | 1-44884204 | cg12049688 | (cancer vs cancer) (cancer vs cancer) (cancer+normal vs cancer+normal) |
| 11-126153561 | cg15932284 | (cancer vs cancer) | 1-50885132 | cg04931216 | (cancer vs cancer) |
| 11-518726 | cg06367925 | (cancer vs cancer) | 1-50892511 | cg10375890 | (cancer vs cancer) |
| 12-123259789 | cg01428378 | (cancer vs cancer) | 1-50892794 | cg04023996 | (cancer vs cancer) |
| 12-125670513 | cg06880239 | (cancer vs cancer) (cancer vs cancer) | 1-91550 | cg03130891 | (cancer vs cancer) |
| 12-57504948 | cg01063813 | (cancer vs cancer) (cancer vs cancer) (normal vs normal) (cancer+normal vs cancer+normal) | 10-102982492 | cg18334881 | (cancer vs cancer) (cancer vs cancer) |
| 12-6493003 | cg23079808 | (cancer vs cancer) (cancer vs cancer) (cancer+normal vs cancer+normal) | 10-102985514 | cg11730722 | (cancer vs cancer) |
| 12-6493264 | cg07648238 | (cancer vs cancer) | 10-102986973 | cg03404143 | (cancer vs cancer) |
| 12-6493270 | cg08740698 | (cancer vs cancer) | 10-118924620 | cg19905856 | (cancer vs cancer) (cancer+normal vs cancer+normal) |
| 12-6493278 | cg10452531 | (cancer vs cancer) (cancer vs cancer) | 10-124911034 | cg24804172 | (cancer vs cancer) |
| 12-6493495 | cg14947547 | (cancer vs cancer) (cancer+normal vs cancer+normal) | 11-67777823 | cg02331303 | (cancer vs cancer) (cancer vs cancer) (cancer+normal vs cancer+normal) |
| 13-114927854 | cg11997708 | (cancer vs cancer) (cancer vs cancer) | 11-67777952 | cg07730301 | (cancer vs cancer) (cancer+normal vs |

| | | | | | |
|---|---|---|---|---|---|
| | | (normal vs normal) (cancer+normal vs cancer+normal) | | | cancer+normal) |
| 13-41593385 | cg01440489 | (cancer vs cancer) | 11-86382900 | cg09193479 | (cancer vs cancer) |
| 13-41593416 | cg02632314 | (cancer vs cancer) (cancer+normal vs cancer+normal) | 11-86383182 | cg07560096 | (cancer vs cancer) |
| 14-24835006 | cg12930714 | (cancer vs cancer) (cancer vs cancer) | 11-86383236 | cg13391116 | (cancer vs cancer) |
| 14-32670315 | cg01174771 | (cancer vs cancer) (cancer vs cancer) | 11-86383241 | cg14881618 | (cancer vs cancer) |
| 14-32673368 | cg23627301 | (cancer vs cancer) | 12-114200279 | cg08800396 | (cancer vs cancer) |
| 14-70038925 | cg02380334 | (cancer vs cancer) (cancer vs cancer) (normal vs normal) (cancer+normal vs cancer+normal) | 12-28128567 | cg00467600 | (cancer vs cancer) (cancer vs cancer) (cancer+normal vs cancer+normal) |
| 14-70038949 | cg14539393 | (cancer vs cancer) (normal vs normal) (cancer+normal vs cancer+normal) | 12-28128669 | cg17271428 | (cancer vs cancer) |
| 14-73958280 | cg20065569 | (cancer vs cancer) | 12-54387917 | cg19435724 | (cancer vs cancer) |
| 15-101629310 | cg03826564 | (cancer vs cancer) | 13-25321494 | cg25794529 | (cancer vs cancer) |
| 15-89954991 | cg10308906 | (cancer vs cancer) | 14-57275386 | cg17205316 | (cancer vs cancer) (cancer+normal vs cancer+normal) |
| 15-93198495 | cg18558647 | (cancer vs cancer) (cancer vs cancer) | 14-57276490 | cg00822495 | (cancer vs cancer) |
| 15-93722499 | cg21610221 | (cancer vs cancer) (cancer+normal vs cancer+normal) | 15-37403242 | cg16656979 | (cancer vs cancer) |
| 16-20774960 | cg10060338 | (cancer vs cancer) (cancer+normal vs cancer+normal) | 15-41198534 | cg23125506 | (cancer vs cancer) |
| 16-31008574 | cg04586407 | (cancer vs cancer) | 16-34258406 | cg06999008 | (cancer vs cancer) |
| 16-31008960 | cg04374711 | (cancer vs cancer) | 17-2907895 | cg09915396 | (cancer vs cancer) |
| 16-4233246 | cg05006755 | (cancer vs cancer) (cancer vs cancer) (cancer+normal vs cancer+normal) | 17-2908023 | cg00739778 | (cancer vs cancer) |
| 16-50730737 | cg08554257 | (cancer vs cancer) (cancer+normal vs cancer+normal) | 17-46655387 | cg21546671 | (cancer vs cancer) (cancer+normal vs cancer+normal) |
| 16-744328 | cg05542681 | (cancer vs cancer) (cancer+normal vs cancer+normal) | 17-46655724 | cg13789775 | (cancer vs cancer) |
| 16-744861 | cg03349020 | (cancer vs cancer) | 17-46692534 | cg25928579 | (cancer vs cancer) |
| 16-744939 | cg04005677 | (cancer vs cancer) (cancer+normal vs cancer+normal) | 17-59539397 | cg24030622 | (cancer vs cancer) (cancer+normal vs cancer+normal) |
| 16-745663 | cg02958327 | (cancer vs cancer) (normal vs normal) (cancer+normal vs cancer+normal) | 19-10405955 | cg15194935 | (cancer vs cancer) |

| | | | | | |
|---|---|---|---|---|---|
| 16-745687 | cg07482202 | (cancer vs cancer)<br>(normal vs normal)<br>(cancer+normal vs cancer+normal) | 19-10406145 | cg2393658<br>7 | (cancer vs cancer)<br>(cancer+normal vs cancer+normal) |
| 17-1553202 | cg04313875 | (cancer vs cancer)<br>(cancer vs cancer)<br>(normal vs normal)<br>(cancer+normal vs cancer+normal) | 19-2169160 | cg2707306<br>6 | (cancer vs cancer)<br>(cancer vs cancer) |
| 17-16256990 | cg13180678 | (cancer vs cancer)<br>(cancer vs cancer) | 2-118981748 | cg0526820<br>3 | (cancer vs cancer)<br>(cancer+normal vs cancer+normal) |
| 17-42392697 | cg11088422 | (cancer vs cancer)<br>(normal vs normal) | 2-118981783 | cg0653049<br>0 | (cancer vs cancer)<br>(cancer+normal vs cancer+normal) |
| 17-4802981 | cg20814095 | (cancer vs cancer) | 2-119606631 | cg0694645<br>2 | (cancer vs cancer) |
| 17-55938748 | cg00023001 | (cancer vs cancer)<br>(cancer vs cancer)<br>(normal vs normal)<br>(cancer+normal vs cancer+normal) | 2-175192013 | cg0258363<br>3 | (cancer vs cancer) |
| 17-55938871 | cg22451358 | (cancer vs cancer)<br>(cancer vs cancer)<br>(normal vs normal)<br>(cancer+normal vs cancer+normal) | 2-175192189 | cg1662510<br>9 | (cancer vs cancer)<br>(cancer+normal vs cancer+normal) |
| 17-72439044 | cg11963912 | (cancer vs cancer)<br>(cancer+normal vs cancer+normal) | 2-175192312 | cg2559252<br>6 | (cancer vs cancer)<br>(cancer+normal vs cancer+normal) |
| 19-1455513 | cg06615719 | (cancer vs cancer)<br>(normal vs normal)<br>(cancer+normal vs cancer+normal) | 2-175199315 | cg1715356<br>8 | (cancer vs cancer)<br>(cancer+normal vs cancer+normal) |
| 19-1468943 | cg10565187 | (cancer vs cancer)<br>(cancer vs cancer)<br>(normal vs normal)<br>(cancer+normal vs cancer+normal) | 2-175199349 | cg1860978<br>3 | (cancer vs cancer) |
| 19-1470292 | cg06508886 | (cancer vs cancer) | 2-175199464 | cg1381978<br>7 | (cancer vs cancer) |
| 19-19336150 | cg04314308 | (cancer vs cancer) | 2-223165320 | cg0271057<br>1 | (cancer vs cancer) |
| 19-19336240 | cg25814383 | (cancer vs cancer)<br>(cancer vs cancer)<br>(normal vs normal)<br>(cancer+normal vs cancer+normal) | 2-223165753 | cg1406435<br>6 | (cancer vs cancer)<br>(cancer+normal vs cancer+normal) |
| 19-2289682 | cg23054321 | (cancer vs cancer) | 2-223168727 | cg2276443<br>6 | (cancer vs cancer) |
| 19-2614086 | cg19250790 | (cancer vs cancer)<br>(cancer vs cancer)<br>(cancer+normal vs cancer+normal) | 2-223170370 | cg0796399<br>0 | (cancer vs cancer)<br>(cancer+normal vs cancer+normal) |
| 19-35531817 | cg24139898 | (cancer vs cancer)<br>(cancer vs cancer)<br>(cancer+normal vs cancer+normal) | 2-45228633 | cg0956835<br>5 | (cancer vs cancer) |
| 19-35531859 | cg21484586 | (cancer vs cancer)<br>(cancer vs cancer) | 2-45228640 | cg0201957<br>4 | (cancer vs cancer) |
| 19- | cg10369688 | (cancer vs cancer) | 2-45228716 | cg2160860 | (cancer vs cancer) |

| | | | | | |
|---|---|---|---|---|---|
| 39898283 | | (normal vs normal) (cancer+normal vs cancer+normal) | | 0 | |
| 19-40971204 | cg06663644 | (cancer vs cancer) | 20-60943241 | cg09831553 | (cancer vs cancer) |
| 19-41055208 | cg08118159 | (cancer vs cancer) | 21-38073307 | cg27325152 | (cancer vs cancer) (cancer+normal vs cancer+normal) |
| 19-46144774 | cg23533683 | (cancer vs cancer) (cancer+normal vs cancer+normal) | 21-38073463 | cg08297082 | (cancer vs cancer) (cancer+normal vs cancer+normal) |
| 19-48824622 | cg07925085 | (cancer vs cancer) (cancer vs cancer) | 21-38073517 | cg27312652 | (cancer vs cancer) (cancer+normal vs cancer+normal) |
| 19-7937107 | cg00438215 | (cancer vs cancer) | 22-19754251 | cg08382235 | (cancer vs cancer) |
| 2-113238376 | cg09759994 | (cancer vs cancer) | 22-19754448 | cg21507095 | (cancer vs cancer) |
| 2-224702734 | cg10413944 | (cancer vs cancer) | 22-19754815 | cg04999026 | (cancer vs cancer) |
| 2-58273552 | cg18998670 | (cancer vs cancer) (cancer vs cancer) | 3-156324038 | cg26405475 | (cancer vs cancer) |
| 20-31262314 | cg24467337 | (cancer vs cancer) (cancer+normal vs cancer+normal) | 3-169384648 | cg10703884 | (cancer vs cancer) |
| 3-111577973 | cg19148281 | (cancer vs cancer) | 3-170101493 | cg02848106 | (cancer vs cancer) (cancer+normal vs cancer+normal) |
| 3-111577981 | cg06828505 | (cancer vs cancer) | 3-176893572 | cg26365152 | (cancer vs cancer) |
| 3-13008800 | cg22481960 | (cancer vs cancer) (cancer vs cancer) | 3-178560988 | cg18701707 | (cancer vs cancer) |
| 3-28283857 | cg15038811 | (cancer vs cancer) (cancer+normal vs cancer+normal) | 3-180328345 | cg20038368 | (cancer vs cancer) (cancer+normal vs cancer+normal) |
| 4-48272077 | cg07849581 | (cancer vs cancer) | 3-181421951 | cg20675389 | (cancer vs cancer) |
| 4-48272082 | cg06798642 | (cancer vs cancer) | 3-181422202 | cg08488945 | (cancer vs cancer) |
| 4-4854690 | cg17807131 | (cancer vs cancer) | 3-186490344 | cg12435415 | (cancer vs cancer) (cancer+normal vs cancer+normal) |
| 4-81123613 | cg22054918 | (cancer vs cancer) | 3-187786456 | cg26790818 | (cancer vs cancer) |
| 5-95298041 | cg11571761 | (cancer vs cancer) | 3-48507087 | cg01870865 | (cancer vs cancer) |
| 5-95298066 | cg20415170 | (cancer vs cancer) | 4-16084676 | cg19162333 | (cancer vs cancer) (cancer+normal vs cancer+normal) |
| 6-157041096 | cg04671145 | (cancer vs cancer) (cancer vs cancer) | 4-16084682 | cg03101849 | (cancer vs cancer) (cancer+normal vs cancer+normal) |
| 6-25962987 | cg08501292 | (cancer vs cancer) (cancer vs cancer) (normal vs normal) (cancer+normal vs cancer+normal) | 4-16085401 | cg07817686 | (cancer vs cancer) (cancer+normal vs cancer+normal) |
| 6-25963109 | cg06153893 | (cancer vs cancer) (normal vs normal) (cancer+normal vs cancer+normal) | 5-153855353 | cg26202847 | (cancer vs cancer) |

| | | | | | |
|---|---|---|---|---|---|
| 6-25963442 | cg21844956 | (cancer vs cancer) (normal vs normal) (cancer+normal vs cancer+normal) | 5-172751061 | cg15420720 | (cancer vs cancer) |
| 6-43237330 | cg23514619 | (cancer vs cancer) (cancer vs cancer) (normal vs normal) (cancer+normal vs cancer+normal) | 5-72715731 | cg17288397 | (cancer vs cancer) |
| 6-43237511 | cg20434586 | (cancer vs cancer) (cancer vs cancer) (normal vs normal) (cancer+normal vs cancer+normal) | 6-1378238 | cg06779672 | (cancer vs cancer) (cancer+normal vs cancer+normal) |
| 6-74233399 | cg21427300 | (cancer vs cancer) | 6-1378677 | cg08975922 | (cancer vs cancer) |
| 6-74233510 | cg09681335 | (cancer vs cancer) (cancer vs cancer) | 6-1380898 | cg11841038 | (cancer vs cancer) (cancer+normal vs cancer+normal) |
| 6-74233640 | cg21953307 | (cancer vs cancer) | 6-1383533 | cg04018288 | (cancer vs cancer) |
| 7-149119681 | cg26269703 | (cancer vs cancer) | 6-1383631 | cg22324934 | (cancer vs cancer) (cancer+normal vs cancer+normal) |
| 7-150020269 | cg11026333 | (cancer vs cancer) | 6-1383982 | cg05872706 | (cancer vs cancer) |
| 7-150020925 | cg11347411 | (cancer vs cancer) (cancer vs cancer) | 6-50812995 | cg14470998 | (cancer vs cancer) |
| 7-17339481 | cg13534901 | (cancer vs cancer) | 6-99292438 | cg08434574 | (cancer vs cancer) |
| 7-22894557 | cg06980547 | (cancer vs cancer) (cancer vs cancer) | 7-113723093 | cg03167413 | (cancer vs cancer) |
| 8-104310713 | cg24115571 | (cancer vs cancer) (cancer vs cancer) | 7-113723569 | cg21053509 | (cancer vs cancer) |
| 8-143556749 | cg23685597 | (cancer vs cancer) | 7-27187502 | cg03529432 | (cancer vs cancer) |
| 8-23082789 | cg01725531 | (cancer vs cancer) (cancer vs cancer) | 7-27204005 | cg16913789 | (cancer vs cancer) (cancer+normal vs cancer+normal) |
| 8-30580804 | cg08305799 | (cancer vs cancer) (cancer vs cancer) (cancer+normal vs cancer+normal) | 7-27204052 | cg25188395 | (cancer vs cancer) |
| 8-80803196 | cg20577468 | (cancer vs cancer) (normal vs normal) | 7-27228627 | cg06669276 | (cancer vs cancer) |
| 8-80803365 | cg06107816 | (cancer vs cancer) (cancer+normal vs cancer+normal) | 7-47611235 | cg17996401 | (cancer vs cancer) |
| 9-123631308 | cg14543508 | (cancer vs cancer) | 8-144503421 | cg26387966 | (cancer vs cancer) |
| 9-123631620 | cg03808351 | (cancer vs cancer) | 8-144503586 | cg19262377 | (cancer vs cancer) |
| 9-34379890 | cg13912930 | (cancer vs cancer) | 8-99952591 | cg15830431 | (cancer vs cancer) |
| **Status - Hypo** | | | **Status - Hypo** | | |
| 1-10240017 | cg27508545 | (normal vs normal) (cancer vs cancer) (cancer vs cancer) (cancer+normal vs cancer+normal) | 1-117350694 | cg11258381 | (cancer vs cancer) |
| 1- | cg23661000 | (normal vs normal) | 1-12304452 | cg2372886 | (cancer vs cancer) |

| | | | | | |
|---|---|---|---|---|---|
| 10240025 | | (cancer vs cancer) (cancer vs cancer) (cancer+normal vs cancer+normal) | | 7 | |
| 1-213500126 | cg26110834 | (cancer vs cancer) | 1-175568216 | cg09473826 | (cancer vs cancer) |
| 1-3128606 | cg22031783 | (cancer vs cancer) (cancer vs cancer) | 1-183204789 | cg22684969 | (cancer vs cancer) (cancer vs cancer) (cancer+normal vs cancer+normal) |
| 1-36559506 | cg09506001 | (cancer vs cancer) (cancer vs cancer) (cancer+normal vs cancer+normal) | 1-183204836 | cg14420230 | (cancer vs cancer) (cancer vs cancer) (cancer+normal vs cancer+normal) |
| 1-6390793 | cg00081799 | (cancer vs cancer) (cancer vs cancer) | 1-1913799 | cg14639753 | (cancer vs cancer) |
| 1-9448660 | cg12658495 | (cancer vs cancer) (cancer vs cancer) (cancer+normal vs cancer+normal) | 1-211918768 | cg17145862 | (cancer vs cancer) |
| 10-115738883 | cg01467532 | (cancer vs cancer) (cancer vs cancer) (cancer+normal vs cancer+normal) | 1-244613363 | cg01012879 | (cancer vs cancer) (cancer+normal vs cancer+normal) |
| 10-405225 | cg20595750 | (normal vs normal) (cancer vs cancer) (cancer vs cancer) (cancer+normal vs cancer+normal) | 1-844499 | cg04410003 | (cancer vs cancer) |
| 10-661009 | cg19926144 | (cancer vs cancer) (cancer vs cancer) (cancer+normal vs cancer+normal) | 10-119749726 | cg11298446 | (cancer vs cancer) |
| 11-113577434 | cg23871994 | (cancer vs cancer) (cancer vs cancer) (cancer+normal vs cancer+normal) | 10-123493091 | cg25724837 | (cancer vs cancer) (cancer+normal vs cancer+normal) |
| 11-1486341 | cg07737063 | (cancer vs cancer) (cancer vs cancer) (cancer+normal vs cancer+normal) | 10-135246701 | cg19197888 | (cancer vs cancer) (cancer+normal vs cancer+normal) |
| 12-123738291 | cg03061518 | (cancer vs cancer) (cancer vs cancer) | 10-35080979 | cg03398910 | (cancer vs cancer) |
| 12-124905230 | cg17429587 | (cancer vs cancer) (cancer vs cancer) | 11-1006303 | cg06494775 | (cancer vs cancer) |
| 12-131452297 | cg23617848 | (cancer vs cancer) (cancer vs cancer) (cancer+normal vs cancer+normal) | 11-1669278 | cg02952849 | (cancer vs cancer) |
| 12-133351421 | cg11136205 | (cancer vs cancer) | 11-65102891 | cg09609051 | (cancer vs cancer) |
| 12-4596426 | cg24211304 | (cancer vs cancer) (cancer vs cancer) (cancer+normal vs cancer+normal) | 11-72724062 | cg26739233 | (cancer vs cancer) (cancer+normal vs cancer+normal) |
| 12-656703 | cg03539204 | (cancer vs cancer) (cancer vs cancer) (cancer+normal vs cancer+normal) | 11-85989647 | cg10981648 | (cancer vs cancer) |
| 13-111893537 | cg26951340 | (normal vs normal) (cancer vs cancer) | 12-104855500 | cg12883014 | (cancer vs cancer) (cancer+normal vs |

| | | | | | |
|---|---|---|---|---|---|
| | | (cancer vs cancer) (cancer+normal vs cancer+normal) | | | cancer+normal) |
| 13-113436564 | cg06206670 | (cancer vs cancer) (cancer vs cancer) (cancer+normal vs cancer+normal) | 12-11286360 | cg24741148 | (cancer vs cancer) |
| 13-113436568 | cg14737571 | (cancer vs cancer) (cancer vs cancer) (cancer+normal vs cancer+normal) | 12-132643897 | cg16860971 | (cancer vs cancer) |
| 13-80585265 | cg03010009 | (cancer vs cancer) (cancer vs cancer) (cancer+normal vs cancer+normal) | 12-21547892 | cg14191244 | (cancer vs cancer) |
| 14-32798387 | cg03403507 | (cancer vs cancer) (cancer vs cancer) (cancer+normal vs cancer+normal) | 12-51347058 | cg18059802 | (cancer vs cancer) |
| 14-32798762 | cg24812523 | (cancer vs cancer) (cancer vs cancer) (cancer+normal vs cancer+normal) | 12-51347099 | cg04427360 | (cancer vs cancer) |
| 14-51293793 | cg18459806 | (cancer vs cancer) (cancer vs cancer) | 12-63015414 | cg05837036 | (cancer vs cancer) |
| 14-69052728 | cg04147497 | (cancer vs cancer) | 12-63195796 | cg27185063 | (cancer vs cancer) |
| 14-95599667 | cg24158307 | (cancer vs cancer) (cancer vs cancer) (cancer+normal vs cancer+normal) | 12-93101181 | cg17344080 | (cancer vs cancer) |
| 15-71685353 | cg13191951 | (cancer vs cancer) (cancer vs cancer) | 12-94121046 | cg25955126 | (cancer vs cancer) |
| 16-2177269 | cg03494430 | (cancer vs cancer) (cancer+normal vs cancer+normal) | 13-114115128 | cg08034413 | (cancer vs cancer) (cancer+normal vs cancer+normal) |
| 16-50897271 | cg00900231 | (normal vs normal) (cancer vs cancer) (cancer vs cancer) (cancer+normal vs cancer+normal) | 14-100920326 | cg13589463 | (cancer vs cancer) (cancer+normal vs cancer+normal) |
| 16-69854895 | cg03346415 | (cancer vs cancer) (cancer+normal vs cancer+normal) | 14-74182387 | cg19461252 | (cancer vs cancer) (cancer+normal vs cancer+normal) |
| 16-72460083 | cg02619656 | (normal vs normal) (cancer vs cancer) (cancer vs cancer) (cancer+normal vs cancer+normal) | 15-101099492 | cg10007075 | (cancer vs cancer) |
| 16-72460114 | cg05347108 | (normal vs normal) (cancer vs cancer) (cancer vs cancer) (cancer+normal vs cancer+normal) | 15-73524906 | cg08177743 | (cancer vs cancer) |
| 16-75300861 | cg00319595 | (cancer vs cancer) (cancer vs cancer) (cancer+normal vs cancer+normal) | 15-73889528 | cg06332339 | (cancer vs cancer) (cancer vs cancer) (cancer+normal vs cancer+normal) |
| 16-88041289 | cg03551062 | (cancer vs cancer) (cancer vs cancer) (cancer+normal vs | 15-73889561 | cg08435157 | (cancer vs cancer) |

| | | cancer+normal) | | | |
|---|---|---|---|---|---|
| 16-89603606 | cg02207944 | (cancer vs cancer) (cancer+normal vs cancer+normal) | 16-1405095 | cg06093201 | (cancer vs cancer) |
| 17-2319906 | cg00145352 | (normal vs normal) (cancer vs cancer) (cancer vs cancer) (cancer+normal vs cancer+normal) | 17-57839538 | cg00002749 | (cancer vs cancer) |
| 17-63535223 | cg06405341 | (cancer vs cancer) (cancer vs cancer) | 18-60481018 | cg07187971 | (cancer vs cancer) |
| 17-71345538 | cg00711291 | (cancer vs cancer) (cancer vs cancer) (cancer+normal vs cancer+normal) | 19-1351766 | cg25718451 | (cancer vs cancer) |
| 17-76220929 | cg07366188 | (cancer vs cancer) (cancer vs cancer) (cancer+normal vs cancer+normal) | 19-36024253 | cg21453378 | (cancer vs cancer) (cancer+normal vs cancer+normal) |
| 17-76220955 | cg10070788 | (cancer vs cancer) (cancer vs cancer) (cancer+normal vs cancer+normal) | 19-5789699 | cg22961623 | (cancer vs cancer) (cancer vs cancer) (cancer+normal vs cancer+normal) |
| 17-9151012 | cg12130672 | (cancer vs cancer) (cancer vs cancer) (cancer+normal vs cancer+normal) | 2-109559185 | cg15775779 | (cancer vs cancer) |
| 18-32398181 | cg22291942 | (normal vs normal) (cancer vs cancer) (cancer vs cancer) (cancer+normal vs cancer+normal) | 2-132348705 | cg11467141 | (cancer vs cancer) (cancer vs cancer) (cancer+normal vs cancer+normal) |
| 18-42324965 | cg03722909 | (normal vs normal) (cancer vs cancer) (cancer vs cancer) (cancer+normal vs cancer+normal) | 2-132348901 | cg03167415 | (cancer vs cancer) |
| 18-42325086 | cg07015525 | (normal vs normal) (cancer vs cancer) (cancer vs cancer) (cancer+normal vs cancer+normal) | 2-191540713 | cg20807948 | (cancer vs cancer) |
| 18-686346 | cg01037496 | (normal vs normal) (cancer vs cancer) (cancer+normal vs cancer+normal) | 2-19427767 | cg17174307 | (cancer vs cancer) |
| 19-1255516 | cg24300446 | (cancer vs cancer) (cancer vs cancer) (cancer+normal vs cancer+normal) | 2-239970075 | cg26126749 | (cancer vs cancer) (cancer vs cancer) (cancer+normal vs cancer+normal) |
| 19-18455508 | cg02383626 | (cancer vs cancer) | 2-239970152 | cg08781549 | (cancer vs cancer) (cancer vs cancer) (cancer+normal vs cancer+normal) |
| 19-47770746 | cg15080870 | (cancer vs cancer) (cancer vs cancer) (cancer+normal vs cancer+normal) | 2-56067277 | cg24166814 | (cancer vs cancer) |
| 2-236716219 | cg22764497 | (cancer vs cancer) (cancer vs cancer) (cancer+normal vs | 2-63274476 | cg25518132 | (cancer vs cancer) |

| | | | | | | |
|---|---|---|---|---|---|---|
| | | cancer+normal) | | | | |
| 2-236716285 | cg06694561 | (cancer vs cancer)<br>(cancer vs cancer)<br>(cancer+normal vs cancer+normal) | 2-99735582 | cg18965322 | | (cancer vs cancer) |
| 2-236716372 | cg06747919 | (cancer vs cancer)<br>(cancer vs cancer)<br>(cancer+normal vs cancer+normal) | 22-30572326 | cg24211826 | | (cancer vs cancer) |
| 2-23879137 | cg00746446 | (cancer vs cancer)<br>(cancer vs cancer)<br>(cancer+normal vs cancer+normal) | 22-38525530 | cg03409699 | | (cancer vs cancer) |
| 2-239306169 | cg09675853 | (cancer vs cancer)<br>(cancer vs cancer) | 3-121380233 | cg11256445 | | (cancer vs cancer) |
| 2-240117222 | cg06257052 | (normal vs normal)<br>(cancer vs cancer)<br>(cancer vs cancer)<br>(cancer+normal vs cancer+normal) | 3-121380236 | cg01364833 | | (cancer vs cancer) |
| 2-240117640 | cg20853370 | (cancer vs cancer)<br>(cancer vs cancer)<br>(cancer+normal vs cancer+normal) | 3-124885459 | cg17997976 | | (cancer vs cancer)<br>(cancer vs cancer)<br>(cancer+normal vs cancer+normal) |
| 2-30041338 | cg00073794 | (cancer vs cancer)<br>(cancer vs cancer)<br>(cancer+normal vs cancer+normal) | 3-139012477 | cg21644077 | | (cancer vs cancer) |
| 2-3292757 | cg03025986 | (normal vs normal)<br>(cancer vs cancer)<br>(cancer vs cancer)<br>(cancer+normal vs cancer+normal) | 3-139062594 | cg13431226 | | (cancer vs cancer)<br>(cancer+normal vs cancer+normal) |
| 2-3480658 | cg05777919 | (cancer vs cancer)<br>(cancer vs cancer)<br>(cancer+normal vs cancer+normal) | 3-149510376 | cg19974283 | | (cancer vs cancer) |
| 2-55174473 | cg04768463 | (cancer vs cancer)<br>(cancer vs cancer)<br>(cancer+normal vs cancer+normal) | 3-170626822 | cg16450309 | | (cancer vs cancer) |
| 2-97213642 | cg00820405 | (cancer vs cancer)<br>(cancer vs cancer) | 3-178738564 | cg15084405 | | (cancer vs cancer) |
| 20-5985495 | cg04316905 | (cancer vs cancer)<br>(cancer vs cancer) | 3-179263203 | cg02500075 | | (cancer vs cancer) |
| 22-22288473 | cg12894883 | (normal vs normal)<br>(cancer vs cancer)<br>(cancer vs cancer)<br>(cancer+normal vs cancer+normal) | 3-182123703 | cg25008182 | | (cancer vs cancer) |
| 22-24041104 | cg00489795 | (cancer vs cancer)<br>(cancer vs cancer)<br>(cancer+normal vs cancer+normal) | 3-185704206 | cg09562655 | | (cancer vs cancer) |
| 22-42611379 | cg26799416 | (cancer vs cancer)<br>(cancer vs cancer)<br>(cancer+normal vs cancer+normal) | 3-187960622 | cg02579706 | | (cancer vs cancer)<br>(cancer vs cancer)<br>(cancer+normal vs cancer+normal) |
| 3-127327369 | cg12678686 | (cancer vs cancer)<br>(cancer vs cancer)<br>(cancer+normal vs | 3-188479059 | cg04423294 | | (cancer vs cancer) |

| | | | | | |
|---|---|---|---|---|---|
| | | cancer+normal) | | | |
| 3-129408521 | cg19272592 | (cancer vs cancer)<br>(cancer vs cancer)<br>(cancer+normal vs cancer+normal) | 4-110484104 | cg05248542 | (cancer vs cancer) |
| 3-147657586 | cg03320743 | (cancer vs cancer)<br>(cancer vs cancer)<br>(cancer+normal vs cancer+normal) | 4-141538969 | cg13056653 | (cancer vs cancer)<br>(cancer+normal vs cancer+normal) |
| 3-195616361 | cg20224111 | (cancer vs cancer)<br>(cancer vs cancer)<br>(cancer+normal vs cancer+normal) | 4-2065749 | cg21962115 | (cancer vs cancer) |
| 3-30722557 | cg15171154 | (cancer vs cancer) | 5-41275792 | cg17507751 | (cancer vs cancer) |
| 4-146804010 | cg01578875 | (normal vs normal)<br>(cancer vs cancer)<br>(cancer vs cancer)<br>(cancer+normal vs cancer+normal) | 6-135094957 | cg13627234 | (cancer vs cancer) |
| 4-147187180 | cg05701706 | (cancer vs cancer)<br>(cancer vs cancer) | 6-137144557 | cg04766529 | (cancer vs cancer) |
| 4-24982540 | cg10569820 | (normal vs normal)<br>(cancer vs cancer)<br>(cancer vs cancer)<br>(cancer+normal vs cancer+normal) | 6-142738443 | cg23192824 | (cancer vs cancer) |
| 4-39064477 | cg26784682 | (cancer vs cancer)<br>(cancer vs cancer) | 6-157335170 | cg02247625 | (cancer vs cancer)<br>(cancer+normal vs cancer+normal) |
| 5-102834858 | cg06612355 | (cancer vs cancer)<br>(cancer vs cancer)<br>(cancer+normal vs cancer+normal) | 6-158093883 | cg03435677 | (cancer vs cancer) |
| 5-140865433 | cg11830096 | (cancer vs cancer)<br>(cancer vs cancer) | 6-21781118 | cg01860763 | (cancer vs cancer) |
| 5-77289836 | cg09645993 | (normal vs normal)<br>(cancer vs cancer)<br>(cancer vs cancer)<br>(cancer+normal vs cancer+normal) | 6-25324431 | cg03372385 | (cancer vs cancer) |
| 6-102224868 | cg24574819 | (normal vs normal)<br>(cancer vs cancer)<br>(cancer vs cancer)<br>(cancer+normal vs cancer+normal) | 6-31765083 | cg24235613 | (cancer vs cancer) |
| 6-125341187 | cg01080927 | (cancer vs cancer)<br>(cancer vs cancer) | 6-37606394 | cg21825443 | (cancer vs cancer) |
| 6-157493773 | cg17596249 | (cancer vs cancer)<br>(cancer vs cancer)<br>(cancer+normal vs cancer+normal) | 6-5412087 | cg08193821 | (cancer vs cancer) |
| 6-24140822 | cg14549524 | (cancer vs cancer)<br>(cancer vs cancer)<br>(cancer+normal vs cancer+normal) | 7-101603335 | cg05855116 | (cancer vs cancer) |
| 6-24140899 | cg08908855 | (cancer vs cancer)<br>(cancer vs cancer)<br>(cancer+normal vs cancer+normal) | 7-116779435 | cg26619790 | (cancer vs cancer)<br>(cancer vs cancer)<br>(cancer+normal vs cancer+normal) |
| 7- | cg06010390 | (normal vs normal) | 7- | cg0130748 | (cancer vs cancer) |

| | | | | | |
|---|---|---|---|---|---|
| 101849120 | | (cancer vs cancer) (cancer vs cancer) (cancer+normal vs cancer+normal) | 129254604 | 3 | |
| 7-101849473 | cg01234984 | (cancer vs cancer) (cancer vs cancer) (cancer+normal vs cancer+normal) | 7-140452281 | cg08428486 | (cancer vs cancer) |
| 7-105283982 | cg11895696 | (cancer vs cancer) (cancer vs cancer) (cancer+normal vs cancer+normal) | 7-148481030 | cg25477176 | (cancer vs cancer) |
| 7-140034703 | cg17102674 | (cancer vs cancer) (cancer+normal vs cancer+normal) | 7-157336976 | cg16708264 | (cancer vs cancer) |
| 7-2148642 | cg16329842 | (normal vs normal) (cancer vs cancer) (cancer+normal vs cancer+normal) | 7-1615334 | cg13724248 | (cancer vs cancer) (cancer vs cancer) |
| 7-4746837 | cg23587176 | (cancer vs cancer) | 7-1615406 | cg19251962 | (cancer vs cancer) |
| 7-4747166 | cg10982851 | (cancer vs cancer) | 7-55209213 | cg18452131 | (cancer vs cancer) |
| 7-4747216 | cg01275887 | (cancer vs cancer) (cancer vs cancer) (cancer+normal vs cancer+normal) | 7-6208128 | cg01256278 | (cancer vs cancer) |
| 7-4747261 | cg07076109 | (cancer vs cancer) (cancer vs cancer) | 7-87905335 | cg03603987 | (cancer vs cancer) |
| 7-5535692 | cg09286367 | (cancer vs cancer) (cancer vs cancer) | 7-925664 | cg01642827 | (cancer vs cancer) |
| 7-92398176 | cg03895496 | (cancer vs cancer) | 7-925778 | cg26988331 | (cancer vs cancer) |
| 8-87570714 | cg02712949 | (cancer vs cancer) (cancer vs cancer) (cancer+normal vs cancer+normal) | 7-926431 | cg16222581 | (cancer vs cancer) |
| 8-9260354 | cg10359460 | (normal vs normal) (cancer vs cancer) (cancer vs cancer) (cancer+normal vs cancer+normal) | 8-1308280 | cg15428949 | (cancer vs cancer) |
| 8-9537322 | cg10419849 | (cancer vs cancer) (cancer vs cancer) (cancer+normal vs cancer+normal) | 8-143424799 | cg02230133 | (cancer vs cancer) |
| 9-130854713 | cg14554539 | (cancer vs cancer) (cancer vs cancer) | 8-48172992 | cg04220541 | (cancer vs cancer) |
| 9-131145355 | cg14630692 | (cancer vs cancer) | 8-97278556 | cg23432707 | (cancer vs cancer) (cancer+normal vs cancer+normal) |
| 9-139410910 | cg14494733 | (cancer vs cancer) (cancer vs cancer) | 9-135769402 | cg14322570 | (cancer vs cancer) |
| 9-99264127 | cg14155501 | (normal vs normal) (cancer vs cancer) (cancer vs cancer) (cancer+normal vs cancer+normal) | 9-138661146 | cg13872541 | (cancer vs cancer) |
| **CESC (cancer vs cancer)** | | | **(cancer+normal vs cancer+normal)** | | |
| **Status - Hyper** | | | **Status - Hyper** | | |

| | | | | | |
|---|---|---|---|---|---|
| 1-163039184 | cg03892631 | (cancer vs cancer) | 1-164536135 | cg21436120 | (cancer+normal vs cancer+normal) |
| 1-166134282 | cg21868774 | (cancer vs cancer) | 1-181062601 | cg20708199 | (cancer+normal vs cancer+normal) |
| 1-166134699 | cg02229993 | (cancer vs cancer) | 1-44884204 | cg12049688 | (cancer vs cancer) (cancer vs cancer) (cancer+normal vs cancer+normal) |
| 1-190448126 | cg24365464 | (cancer vs cancer) | 10-102983065 | cg05889234 | (cancer+normal vs cancer+normal) |
| 1-239549626 | cg04412646 | (cancer vs cancer) (cancer+normal vs cancer+normal) | 10-118924620 | cg19905856 | (cancer vs cancer) (cancer+normal vs cancer+normal) |
| 1-239550378 | cg00371368 | (cancer vs cancer) (cancer+normal vs cancer+normal) | 10-7448857 | cg24020806 | (cancer+normal vs cancer+normal) |
| 1-240775075 | cg07808348 | (cancer vs cancer) (cancer+normal vs cancer+normal) | 11-67777952 | cg07730301 | (cancer vs cancer) (cancer+normal vs cancer+normal) |
| 1-240775160 | cg00155397 | (cancer vs cancer) (cancer+normal vs cancer+normal) | 12-16190004 | cg25496104 | (cancer+normal vs cancer+normal) |
| 1-241520598 | cg24843474 | (cancer vs cancer) | 12-28128567 | cg00467600 | (cancer vs cancer) (cancer vs cancer) (cancer+normal vs cancer+normal) |
| 1-66798063 | cg00805867 | (cancer vs cancer) | 14-57275386 | cg17205316 | (cancer vs cancer) (cancer+normal vs cancer+normal) |
| 1-72748124 | cg04645342 | (cancer vs cancer) | 15-37369914 | cg12790758 | (cancer+normal vs cancer+normal) |
| 1-76540168 | cg15090083 | (cancer vs cancer) (cancer+normal vs cancer+normal) | 16-58535384 | cg08092105 | (cancer+normal vs cancer+normal) |
| 10-112837240 | cg26926521 | (cancer vs cancer) | 16-58535416 | cg11640773 | (cancer+normal vs cancer+normal) |
| 12-101604056 | cg26490054 | (cancer vs cancer) | 16-58535418 | cg27102864 | (cancer+normal vs cancer+normal) |
| 12-103358881 | cg03239178 | (cancer vs cancer) | 16-58535556 | cg01084435 | (cancer+normal vs cancer+normal) |
| 12-127865826 | cg07366196 | (cancer vs cancer) (cancer+normal vs cancer+normal) | 16-86321899 | cg08927145 | (cancer vs cancer) (cancer+normal vs cancer+normal) |
| 13-100648306 | cg15470836 | (cancer vs cancer) (cancer+normal vs cancer+normal) | 17-21101734 | cg20758791 | (cancer+normal vs cancer+normal) |
| 13-28494161 | cg04633225 | (cancer vs cancer) | 17-41832873 | cg13077545 | (cancer+normal vs cancer+normal) |
| 13-28542727 | cg07213036 | (cancer vs cancer) (cancer+normal vs cancer+normal) | 17-41832882 | cg04930469 | (cancer+normal vs cancer+normal) |
| 13-29292888 | cg24321030 | (cancer vs cancer) (cancer+normal vs cancer+normal) | 17-41832975 | cg12473285 | (cancer+normal vs cancer+normal) |
| 13-29293133 | cg12775884 | (cancer vs cancer) (cancer+normal vs cancer+normal) | 17-46655387 | cg21546671 | (cancer vs cancer) (cancer+normal vs cancer+normal) |
| 13-29293196 | cg17128349 | (cancer vs cancer) (cancer+normal vs cancer+normal) | 17-46655736 | cg04609859 | (cancer+normal vs cancer+normal) |
| 13- | cg20752818 | (cancer vs cancer) | 17- | cg2298209 | (cancer+normal vs |

| | | | | | |
|---|---|---|---|---|---|
| 29293213 | | (cancer+normal vs cancer+normal) | 55339169 | 8 | cancer+normal) |
| 13-29293245 | cg21789898 | (cancer vs cancer) (cancer+normal vs cancer+normal) | 17-59539397 | cg24030622 | (cancer vs cancer) (cancer+normal vs cancer+normal) |
| 13-29293279 | cg18682423 | (cancer vs cancer) (cancer+normal vs cancer+normal) | 19-10403403 | cg08845333 | (cancer+normal vs cancer+normal) |
| 13-61989701 | cg05034702 | (cancer vs cancer) (cancer+normal vs cancer+normal) | 19-10406145 | cg23936587 | (cancer vs cancer) (cancer+normal vs cancer+normal) |
| 13-61989859 | cg02246190 | (cancer vs cancer) (cancer+normal vs cancer+normal) | 2-118981748 | cg05268203 | (cancer vs cancer) (cancer+normal vs cancer+normal) |
| 13-92050675 | cg13744663 | (cancer vs cancer) (cancer+normal vs cancer+normal) | 2-118981783 | cg06530490 | (cancer vs cancer) (cancer+normal vs cancer+normal) |
| 13-92050726 | cg12678562 | (cancer vs cancer) | 2-175192189 | cg16625109 | (cancer vs cancer) (cancer+normal vs cancer+normal) |
| 13-92050991 | cg19162106 | (cancer vs cancer) (cancer+normal vs cancer+normal) | 2-175192312 | cg25592526 | (cancer vs cancer) (cancer+normal vs cancer+normal) |
| 14-21101007 | cg01762070 | (cancer vs cancer) | 2-175199315 | cg17153568 | (cancer vs cancer) (cancer+normal vs cancer+normal) |
| 14-76819246 | cg11025763 | (cancer vs cancer) (cancer+normal vs cancer+normal) | 2-223165753 | cg14064356 | (cancer vs cancer) (cancer+normal vs cancer+normal) |
| 16-1031944 | cg09635053 | (cancer vs cancer) | 2-223170370 | cg07963990 | (cancer vs cancer) (cancer+normal vs cancer+normal) |
| 16-7137080 | cg04056343 | (cancer vs cancer) | 2-223176398 | cg17981058 | (cancer+normal vs cancer+normal) |
| 16-8223276 | cg00253851 | (cancer vs cancer) (cancer+normal vs cancer+normal) | 2-44172074 | cg08781755 | (cancer+normal vs cancer+normal) |
| 17-58227714 | cg26297547 | (cancer vs cancer) | 2-58386677 | cg00869533 | (cancer+normal vs cancer+normal) |
| 18-32556655 | cg05735765 | (cancer vs cancer) (cancer+normal vs cancer+normal) | 2-65496336 | cg16480634 | (cancer+normal vs cancer+normal) |
| 18-32557266 | cg14309384 | (cancer vs cancer) (cancer+normal vs cancer+normal) | 20-43954866 | cg27440965 | (cancer+normal vs cancer+normal) |
| 18-32557882 | cg24692068 | (cancer vs cancer) (cancer+normal vs cancer+normal) | 21-38073307 | cg27325152 | (cancer vs cancer) (cancer+normal vs cancer+normal) |
| 18-32558066 | cg13550107 | (cancer vs cancer) (cancer+normal vs cancer+normal) | 21-38073463 | cg08297082 | (cancer vs cancer) (cancer+normal vs cancer+normal) |
| 19-12076051 | cg06647417 | (cancer vs cancer) (cancer+normal vs cancer+normal) | 21-38073517 | cg27312652 | (cancer vs cancer) (cancer+normal vs cancer+normal) |
| 19-20011721 | cg05661139 | (cancer vs cancer) | 3-105831607 | cg12021988 | (cancer+normal vs cancer+normal) |
| 19-20011770 | cg23501467 | (cancer vs cancer) (cancer+normal vs cancer+normal) | 3-113529180 | cg16758764 | (cancer+normal vs cancer+normal) |
| 19-21579902 | cg15420387 | (cancer vs cancer) (cancer+normal vs | 3-170101493 | cg02848106 | (cancer vs cancer) (cancer+normal vs |

| | | cancer+normal) |
|---|---|---|
| 19-23578260 | cg17853707 | (cancer vs cancer) (cancer+normal vs cancer+normal) |
| 19-23869985 | cg19361542 | (cancer vs cancer) (cancer+normal vs cancer+normal) |
| 19-2702986 | cg21340148 | (cancer vs cancer) (cancer+normal vs cancer+normal) |
| 19-49224165 | cg13974464 | (cancer vs cancer) (cancer+normal vs cancer+normal) |
| 19-54410305 | cg02660823 | (cancer vs cancer) (cancer+normal vs cancer+normal) |
| 19-57999026 | cg12501868 | (cancer vs cancer) (cancer+normal vs cancer+normal) |
| 19-57999036 | cg08134106 | (cancer vs cancer) (cancer+normal vs cancer+normal) |
| 19-57999041 | cg15745619 | (cancer vs cancer) (cancer+normal vs cancer+normal) |
| 19-57999177 | cg09260640 | (cancer vs cancer) (cancer+normal vs cancer+normal) |
| 2-127643971 | cg08535260 | (cancer vs cancer) (cancer+normal vs cancer+normal) |
| 2-136875315 | cg13854983 | (cancer vs cancer) (cancer+normal vs cancer+normal) |
| 2-155554688 | cg23491599 | (cancer vs cancer) (cancer+normal vs cancer+normal) |
| 2-155554707 | cg11706467 | (cancer vs cancer) (cancer+normal vs cancer+normal) |
| 2-168149401 | cg18395675 | (cancer vs cancer) (cancer+normal vs cancer+normal) |
| 2-168149519 | cg21364278 | (cancer vs cancer) |
| 3-137483063 | cg16921083 | (cancer vs cancer) (cancer+normal vs cancer+normal) |
| 3-137483099 | cg13217064 | (cancer vs cancer) |
| 3-137484517 | cg05880330 | (cancer vs cancer) (cancer+normal vs cancer+normal) |
| 3-137487081 | cg17823751 | (cancer vs cancer) |
| 3-137490228 | cg16434657 | (cancer vs cancer) |
| 3-62859461 | cg15997484 | (cancer vs cancer) (cancer+normal vs cancer+normal) |

| | | cancer+normal) |
|---|---|---|
| 3-170146757 | cg22463444 | (cancer+normal vs cancer+normal) |
| 3-170714485 | cg11417598 | (cancer+normal vs cancer+normal) |
| 3-171791143 | cg08495617 | (cancer+normal vs cancer+normal) |
| 3-171890144 | cg03177593 | (cancer+normal vs cancer+normal) |
| 3-180328345 | cg20038368 | (cancer vs cancer) (cancer+normal vs cancer+normal) |
| 3-180704359 | cg09830059 | (cancer+normal vs cancer+normal) |
| 3-181669865 | cg19114999 | (cancer+normal vs cancer+normal) |
| 3-186490344 | cg12435415 | (cancer vs cancer) (cancer+normal vs cancer+normal) |
| 3-189432110 | cg07132449 | (cancer+normal vs cancer+normal) |
| 3-192960454 | cg09729155 | (cancer+normal vs cancer+normal) |
| 4-16084676 | cg19162333 | (cancer vs cancer) (cancer+normal vs cancer+normal) |
| 4-16084682 | cg03101849 | (cancer vs cancer) (cancer+normal vs cancer+normal) |
| 4-16085401 | cg07817686 | (cancer vs cancer) (cancer+normal vs cancer+normal) |
| 5-109961 | cg06697017 | (cancer+normal vs cancer+normal) |
| 5-17230962 | cg26883517 | (cancer+normal vs cancer+normal) |
| 6-1378238 | cg06779672 | (cancer vs cancer) (cancer+normal vs cancer+normal) |
| 6-1380898 | cg11841038 | (cancer vs cancer) (cancer+normal vs cancer+normal) |
| 6-1383631 | cg22324934 | (cancer vs cancer) (cancer+normal vs cancer+normal) |
| 6-150276311 | cg22745624 | (cancer+normal vs cancer+normal) |
| 6-49110730 | cg01955492 | (cancer+normal vs cancer+normal) |
| 7-129543162 | cg16720564 | (cancer+normal vs cancer+normal) |

| | | |
|---|---|---|
| 4-46391694 | cg12205729 | (cancer vs cancer) (cancer+normal vs cancer+normal) |
| 5-145724482 | cg24799909 | (cancer vs cancer) |
| 5-146614298 | cg09088988 | (cancer vs cancer) |
| 5-160973736 | cg00524310 | (cancer vs cancer) (cancer+normal vs cancer+normal) |
| 5-39425545 | cg00261416 | (cancer vs cancer) |
| 5-57878745 | cg08261837 | (cancer vs cancer) (cancer+normal vs cancer+normal) |
| 5-57878882 | cg21617058 | (cancer vs cancer) (cancer+normal vs cancer+normal) |
| 5-57878919 | cg08445409 | (cancer vs cancer) (cancer+normal vs cancer+normal) |
| 5-57878953 | cg23123909 | (cancer vs cancer) (cancer+normal vs cancer+normal) |
| 5-87437106 | cg07513791 | (cancer vs cancer) (cancer+normal vs cancer+normal) |
| 5-87437337 | cg21153697 | (cancer vs cancer) (cancer+normal vs cancer+normal) |
| 5-87437400 | cg26246127 | (cancer vs cancer) (cancer+normal vs cancer+normal) |
| 5-87976156 | cg19625088 | (cancer vs cancer) (cancer+normal vs cancer+normal) |
| 5-87976294 | cg24025896 | (cancer vs cancer) (cancer+normal vs cancer+normal) |
| 5-87986642 | cg06523916 | (cancer vs cancer) (cancer+normal vs cancer+normal) |
| 6-166400665 | cg16289417 | (cancer vs cancer) |
| 6-166401464 | cg14026561 | (cancer vs cancer) |
| 6-28762552 | cg11400401 | (cancer vs cancer) (cancer+normal vs cancer+normal) |
| 7-11871535 | cg26616283 | (cancer vs cancer) (cancer+normal vs cancer+normal) |
| 7-11871690 | cg26748945 | (cancer vs cancer) |
| 7-11871837 | cg12348203 | (cancer vs cancer) (cancer+normal vs cancer+normal) |
| 7- | cg15400117 | (cancer vs cancer) |
| 7-27204005 | cg16913789 | (cancer vs cancer) (cancer+normal vs cancer+normal) |
| 7-5666097 | cg20821466 | (cancer+normal vs cancer+normal) |
| 9-124982087 | cg25542041 | (cancer+normal vs cancer+normal) |
| **Status - Hypo** | | |
| 1-17012728 | cg02769477 | (cancer+normal vs cancer+normal) |
| 1-183204789 | cg22684969 | (cancer vs cancer) (cancer vs cancer) (cancer+normal vs cancer+normal) |
| 1-183204836 | cg14420230 | (cancer vs cancer) (cancer vs cancer) (cancer+normal vs cancer+normal) |
| 1-201523041 | cg04158698 | (cancer+normal vs cancer+normal) |
| 1-228890884 | cg23926439 | (cancer+normal vs cancer+normal) |
| 1-244613363 | cg01012879 | (cancer vs cancer) (cancer+normal vs cancer+normal) |
| 1-27440721 | cg25130381 | (cancer+normal vs cancer+normal) |
| 1-47694517 | cg11399254 | (cancer+normal vs cancer+normal) |
| 1-61378118 | cg22340807 | (cancer+normal vs cancer+normal) |
| 10-133251574 | cg14435498 | (cancer+normal vs cancer+normal) |
| 10-135246701 | cg19197888 | (cancer vs cancer) (cancer+normal vs cancer+normal) |
| 11-124767455 | cg10148841 | (cancer+normal vs cancer+normal) |
| 11-72724062 | cg26739233 | (cancer vs cancer) (cancer+normal vs cancer+normal) |
| 11-95093809 | cg24941342 | (cancer+normal vs cancer+normal) |
| 12-104855500 | cg12883014 | (cancer vs cancer) (cancer+normal vs cancer+normal) |
| 12-111470657 | cg25752163 | (cancer+normal vs cancer+normal) |
| 12-111470672 | cg08710739 | (cancer+normal vs cancer+normal) |
| 12- | cg1178716 | (cancer+normal vs |

| | | | | | |
|---|---|---|---|---|---|
| 11871843 | | | 113515332 | 0 | cancer+normal) |
| 7-123673549 | cg01426853 | (cancer vs cancer) | 12-113515835 | cg27664496 | (cancer+normal vs cancer+normal) |
| 7-1265325 | cg21768566 | (cancer vs cancer) (cancer+normal vs cancer+normal) | 12-3364419 | cg14533605 | (cancer+normal vs cancer+normal) |
| 7-152591581 | cg16806794 | (cancer vs cancer) (cancer+normal vs cancer+normal) | 12-49726761 | cg11949060 | (cancer+normal vs cancer+normal) |
| 7-154862021 | cg21633143 | (cancer vs cancer) | 12-56587526 | cg06850687 | (cancer+normal vs cancer+normal) |
| 7-94537870 | cg09724492 | (cancer vs cancer) | 12-92524968 | cg05976481 | (cancer+normal vs cancer+normal) |
| 8-12990437 | cg06164608 | (cancer vs cancer) (cancer+normal vs cancer+normal) | 13-114115128 | cg08034413 | (cancer vs cancer) (cancer+normal vs cancer+normal) |
| 8-12990622 | cg19024344 | (cancer vs cancer) (cancer+normal vs cancer+normal) | 14-100920326 | cg13589463 | (cancer vs cancer) (cancer+normal vs cancer+normal) |
| 8-12990744 | cg26464821 | (cancer vs cancer) (cancer+normal vs cancer+normal) | 14-53508673 | cg21842967 | (cancer+normal vs cancer+normal) |
| 8-12990898 | cg14904305 | (cancer vs cancer) (cancer+normal vs cancer+normal) | 14-74182387 | cg19461252 | (cancer vs cancer) (cancer+normal vs cancer+normal) |
| 8-12991143 | cg24819686 | (cancer vs cancer) (cancer+normal vs cancer+normal) | 14-77689345 | cg16478977 | (cancer+normal vs cancer+normal) |
| 8-12991196 | cg19040483 | (cancer vs cancer) (cancer+normal vs cancer+normal) | 15-29442040 | cg13832135 | (cancer+normal vs cancer+normal) |
| 8-13134144 | cg03304380 | (cancer vs cancer) (cancer+normal vs cancer+normal) | 15-40727743 | cg27365324 | (cancer+normal vs cancer+normal) |
| 8-13134166 | cg20536983 | (cancer vs cancer) (normal vs normal) (cancer+normal vs cancer+normal) | 15-65032585 | cg25840536 | (cancer+normal vs cancer+normal) |
| **Status - Hypo** | | | 15-69222592 | cg17286244 | (cancer+normal vs cancer+normal) |
| 1-1168432 | cg13924635 | (cancer vs cancer) (cancer+normal vs cancer+normal) | 15-73889528 | cg06332339 | (cancer vs cancer) (cancer vs cancer) (cancer+normal vs cancer+normal) |
| 1-1168541 | cg22220310 | (cancer vs cancer) (cancer+normal vs cancer+normal) | 16-10523485 | cg00461841 | (cancer+normal vs cancer+normal) |
| 1-1168550 | cg14477263 | (cancer vs cancer) (cancer+normal vs cancer+normal) | 16-1339782 | cg09192369 | (cancer+normal vs cancer+normal) |
| 1-149871945 | cg09045105 | (cancer vs cancer) (cancer+normal vs cancer+normal) | 16-1587684 | cg14470121 | (cancer+normal vs cancer+normal) |
| 1-149872165 | cg13169641 | (cancer vs cancer) | 16-1610898 | cg00305585 | (cancer+normal vs cancer+normal) |
| 1-168254154 | cg06941869 | (cancer vs cancer) | 16-19721858 | cg06685949 | (cancer+normal vs cancer+normal) |
| 1-19280186 | cg11697440 | (cancer vs cancer) | 16-27482516 | cg08907118 | (cancer+normal vs cancer+normal) |
| 1-19687257 | cg12763668 | (cancer vs cancer) (cancer+normal vs | 16-29643261 | cg26678835 | (cancer+normal vs cancer+normal) |

| | | cancer+normal) | | | |
|---|---|---|---|---|---|
| 1-202848987 | cg02709178 | (cancer vs cancer) | 16-56995813 | cg09889350 | (cancer+normal vs cancer+normal) |
| 1-32826969 | cg08843283 | (cancer vs cancer) (cancer+normal vs cancer+normal) | 17-35423816 | cg11794215 | (cancer+normal vs cancer+normal) |
| 1-32827150 | cg16379513 | (cancer vs cancer) | 17-40442580 | cg12259792 | (cancer+normal vs cancer+normal) |
| 1-33772032 | cg00326300 | (cancer vs cancer) | 17-76421538 | cg00913879 | (cancer+normal vs cancer+normal) |
| 10-77468337 | cg02571436 | (cancer vs cancer) | 17-7758102 | cg03719978 | (cancer+normal vs cancer+normal) |
| 10-82221597 | cg16260349 | (cancer vs cancer) | 17-80804126 | cg07450698 | (normal vs normal) (cancer+normal vs cancer+normal) |
| 11-460914 | cg26889437 | (cancer vs cancer) | 17-998711 | cg02135245 | (cancer+normal vs cancer+normal) |
| 11-65306731 | cg13826564 | (cancer vs cancer) | 19-18980443 | cg01215442 | (cancer+normal vs cancer+normal) |
| 11-65307006 | cg04007841 | (cancer vs cancer) | 19-36024253 | cg21453378 | (cancer vs cancer) (cancer+normal vs cancer+normal) |
| 11-68133159 | cg20892743 | (cancer vs cancer) | 19-50189880 | cg00346180 | (cancer+normal vs cancer+normal) |
| 11-68133177 | cg06989074 | (cancer vs cancer) | 19-5789699 | cg22961623 | (cancer vs cancer) (cancer vs cancer) (cancer+normal vs cancer+normal) |
| 11-68133235 | cg24728180 | (cancer vs cancer) | 2-127819455 | cg24750513 | (cancer+normal vs cancer+normal) |
| 11-75140681 | cg16547529 | (cancer vs cancer) | 2-132348705 | cg11467141 | (cancer vs cancer) (cancer vs cancer) (cancer+normal vs cancer+normal) |
| 12-110940444 | cg17382939 | (cancer vs cancer) | 2-20442131 | cg08044427 | (cancer+normal vs cancer+normal) |
| 14-103746266 | cg19544707 | (cancer vs cancer) | 2-233352951 | cg02932167 | (cancer+normal vs cancer+normal) |
| 14-24780890 | cg12853742 | (cancer vs cancer) | 2-239970075 | cg26126749 | (cancer vs cancer) (cancer vs cancer) (cancer+normal vs cancer+normal) |
| 16-11276460 | cg26442107 | (cancer vs cancer) | 2-239970152 | cg08781549 | (cancer vs cancer) (cancer vs cancer) (cancer+normal vs cancer+normal) |
| 16-2043015 | cg04519296 | (cancer vs cancer) | 2-240153820 | cg12547218 | (cancer+normal vs cancer+normal) |
| 16-2229298 | cg06831064 | (cancer vs cancer) | 2-240302637 | cg26876664 | (cancer+normal vs cancer+normal) |
| 16-2391402 | cg07915849 | (cancer vs cancer) | 2-30334305 | cg15998518 | (cancer+normal vs cancer+normal) |
| 16-30442009 | cg03941745 | (cancer vs cancer) | 2-46385031 | cg14398243 | (cancer+normal vs cancer+normal) |
| 16-31154636 | cg00915858 | (cancer vs cancer) | 2-54697181 | cg16667251 | (normal vs normal) (cancer+normal vs cancer+normal) |
| 16-31500439 | cg00029825 | (cancer vs cancer) | 2-61918195 | cg01899355 | (cancer+normal vs cancer+normal) |
| 16-4589127 | cg03105756 | (cancer vs cancer) | 20-20258001 | cg11577716 | (cancer+normal vs cancer+normal) |

146

| | | | | | |
|---|---|---|---|---|---|
| 16-67314073 | cg06426253 | (cancer vs cancer) | 20-30176106 | cg0834075 5 | (normal vs normal) (cancer+normal vs cancer+normal) |
| 17-59521084 | cg03964554 | (cancer vs cancer) | 20-50010224 | cg1161781 6 | (cancer+normal vs cancer+normal) |
| 17-59521299 | cg02934718 | (cancer vs cancer) | 22-17956453 | cg0101021 1 | (normal vs normal) (cancer+normal vs cancer+normal) |
| 17-59521422 | cg06454901 | (cancer vs cancer) | 22-17956455 | cg2407447 7 | (cancer+normal vs cancer+normal) |
| 17-60757589 | cg14690510 | (cancer vs cancer) | 22-17956462 | cg0340890 4 | (cancer+normal vs cancer+normal) |
| 17-60757632 | cg01943414 | (cancer vs cancer) | 3-124885459 | cg1799797 6 | (cancer vs cancer) (cancer vs cancer) (cancer+normal vs cancer+normal) |
| 17-61926910 | cg00280220 | (cancer vs cancer) | 3-127299468 | cg2165086 1 | (cancer+normal vs cancer+normal) |
| 17-61927036 | cg13332729 | (cancer vs cancer) (cancer+normal vs cancer+normal) | 3-139062594 | cg1343122 6 | (cancer vs cancer) (cancer+normal vs cancer+normal) |
| 17-7486551 | cg17887478 | (cancer vs cancer) | 3-153840379 | cg1282966 6 | (cancer+normal vs cancer+normal) |
| 17-7486615 | cg05086567 | (cancer vs cancer) | 3-171159987 | cg1235419 2 | (cancer+normal vs cancer+normal) |
| 17-80551747 | cg23765707 | (cancer vs cancer) | 3-187960622 | cg0257970 6 | (cancer vs cancer) (cancer vs cancer) (cancer+normal vs cancer+normal) |
| 18-10590639 | cg03778258 | (cancer vs cancer) | 3-193629645 | cg2720356 0 | (cancer+normal vs cancer+normal) |
| 19-39055500 | cg18154954 | (cancer vs cancer) | 3-50357644 | cg0046765 2 | (cancer+normal vs cancer+normal) |
| 19-39260304 | cg26703956 | (cancer vs cancer) | 4-141538969 | cg1305665 3 | (cancer vs cancer) (cancer+normal vs cancer+normal) |
| 19-49232187 | cg12258179 | (cancer vs cancer) | 4-155702411 | cg0828242 8 | (cancer+normal vs cancer+normal) |
| 19-49232824 | cg14116320 | (cancer vs cancer) | 5-122433740 | cg0705628 5 | (cancer+normal vs cancer+normal) |
| 19-50392917 | cg23074703 | (cancer vs cancer) (cancer+normal vs cancer+normal) | 5-148810177 | cg1167136 3 | (cancer+normal vs cancer+normal) |
| 2-39186743 | cg27117491 | (cancer vs cancer) | 5-1514140 | cg1404999 0 | (cancer+normal vs cancer+normal) |
| 2-39186885 | cg02826525 | (cancer vs cancer) | 6-110418140 | cg1622686 6 | (cancer+normal vs cancer+normal) |
| 2-39188006 | cg03690080 | (cancer vs cancer) (cancer+normal vs cancer+normal) | 6-119221279 | cg0418342 5 | (cancer+normal vs cancer+normal) |
| 2-74743839 | cg14877896 | (cancer vs cancer) | 6-157335170 | cg0224762 5 | (cancer vs cancer) (cancer+normal vs cancer+normal) |
| 2-75186308 | cg04748828 | (cancer vs cancer) | 6-32151517 | cg1813980 0 | (cancer+normal vs cancer+normal) |
| 20-35723332 | cg00045515 | (cancer vs cancer) | 6-33040535 | cg2582421 7 | (cancer+normal vs cancer+normal) |
| 20-55904677 | cg19746719 | (cancer vs cancer) | 7-116779435 | cg2661979 0 | (cancer vs cancer) (cancer vs cancer) (cancer+normal vs cancer+normal) |

| | | | | | |
|---|---|---|---|---|---|
| 20-55904686 | cg23998200 | (cancer vs cancer) | 7-128577593 | cg12816198 | (cancer+normal vs cancer+normal) |
| 20-58507171 | cg07347645 | (cancer vs cancer) | 7-148720088 | cg25326952 | (cancer+normal vs cancer+normal) |
| 20-58507227 | cg23241473 | (cancer vs cancer) | 7-151145235 | cg06464775 | (cancer+normal vs cancer+normal) |
| 20-58507232 | cg22214414 | (cancer vs cancer) | 7-152622135 | cg17403699 | (cancer+normal vs cancer+normal) |
| 20-58508076 | cg21950459 | (cancer vs cancer) (cancer+normal vs cancer+normal) | 7-2116648 | cg22662844 | (cancer+normal vs cancer+normal) |
| 20-58508123 | cg12181372 | (cancer vs cancer) (cancer+normal vs cancer+normal) | 8-12987449 | cg26244164 | (cancer+normal vs cancer+normal) |
| 20-58514079 | cg20707333 | (cancer vs cancer) | 8-21905462 | cg15246619 | (cancer+normal vs cancer+normal) |
| 20-58514201 | cg00400263 | (cancer vs cancer) | 8-25095288 | cg27185978 | (cancer+normal vs cancer+normal) |
| 21-44831040 | cg03566291 | (cancer vs cancer) | 8-60032926 | cg02892925 | (cancer+normal vs cancer+normal) |
| 21-44831062 | cg04824511 | (cancer vs cancer) | 8-97278556 | cg23432707 | (cancer vs cancer) (cancer+normal vs cancer+normal) |
| 22-29445711 | cg21918728 | (cancer vs cancer) | 9-112731026 | cg14178013 | (cancer+normal vs cancer+normal) |
| 22-29445903 | cg09560452 | (cancer vs cancer) | 9-139557920 | cg08529852 | (normal vs normal) (cancer+normal vs cancer+normal) |
| 22-36902578 | cg05475277 | (cancer vs cancer) | **(normal vs normal)** | | |
| 22-42395235 | cg22959474 | (cancer vs cancer) | **Status - Hyper** | | |
| 22-43254043 | cg23778094 | (cancer vs cancer) | 1-161089118 | cg04991214 | (normal vs normal) |
| 22-43254081 | cg00539564 | (cancer vs cancer) | 1-172413492 | cg02288969 | (normal vs normal) |
| 22-47023005 | cg12649343 | (cancer vs cancer) | 1-172413542 | cg03748243 | (normal vs normal) |
| 3-127317798 | cg08889930 | (cancer vs cancer) | 1-221061837 | cg06951677 | (normal vs normal) |
| 3-148804720 | cg21111416 | (cancer vs cancer) | 1-228503882 | cg03419458 | (normal vs normal) |
| 3-148804727 | cg04731810 | (cancer vs cancer) | 1-33330006 | cg03976754 | (normal vs normal) |
| 3-148804743 | cg04120413 | (cancer vs cancer) | 1-8480963 | cg13400249 | (normal vs normal) |
| 3-148804746 | cg02611507 | (cancer vs cancer) (cancer+normal vs cancer+normal) | 10-31108101 | cg06822067 | (normal vs normal) |
| 3-148804790 | cg07275648 | (cancer vs cancer) | 10-53004308 | cg05131940 | (normal vs normal) |
| 3-148804818 | cg09352720 | (cancer vs cancer) | 10-79684776 | cg01275566 | (normal vs normal) |
| 3-155304922 | cg00757822 | (cancer vs cancer) | 10-8125688 | cg24284640 | (normal vs normal) |
| 3-185191446 | cg18572353 | (cancer vs cancer) | 10-92079600 | cg12127811 | (normal vs normal) |
| 3-49755764 | cg25090325 | (cancer vs cancer) | 10-94455543 | cg10299976 | (normal vs normal) |
| 5-134364968 | cg04101060 | (cancer vs cancer) | 10-97175272 | cg10741308 | (normal vs normal) |

| | | | | | |
|---|---|---|---|---|---|
| 5-157079312 | cg16313910 | (cancer vs cancer) | 11-116948652 | cg06646174 | (normal vs normal) |
| 5-42991495 | cg06348134 | (cancer vs cancer) | 11-120090023 | cg00406023 | (normal vs normal) |
| 5-96042628 | cg12866104 | (cancer vs cancer) | 11-2612781 | cg09315662 | (normal vs normal) |
| 6-30458586 | cg00340855 | (cancer vs cancer) | 11-2612825 | cg21695475 | (normal vs normal) |
| 6-30458601 | cg11594821 | (cancer vs cancer) | 11-2774756 | cg11585942 | (normal vs normal) |
| 6-32819911 | cg01673307 | (cancer vs cancer) | 11-63532530 | cg09244748 | (normal vs normal) |
| 6-32819921 | cg24111025 | (cancer vs cancer) | 11-63852954 | cg15769472 | (normal vs normal) |
| 6-37616482 | cg14926196 | (cancer vs cancer) | 11-96518853 | cg16589644 | (normal vs normal) |
| 7-100730520 | cg00013374 | (cancer vs cancer) | 12-104868173 | cg09038971 | (normal vs normal) |
| 7-156432293 | cg02849937 | (cancer vs cancer) | 12-119715090 | cg13977620 | (normal vs normal) |
| 7-156432526 | cg00465958 | (cancer vs cancer) | 12-19284928 | cg19755459 | (normal vs normal) |
| 8-143782585 | cg10802543 | (cancer vs cancer) (cancer+normal vs cancer+normal) | 12-22777430 | cg21477262 | (normal vs normal) |
| 8-27549068 | cg26794938 | (cancer vs cancer) | 12-32655192 | cg08738571 | (normal vs normal) |
| 8-28325115 | cg10699291 | (cancer vs cancer) | 12-51381135 | cg20872692 | (normal vs normal) |
| 9-100849700 | cg19461260 | (cancer vs cancer) | 12-68873953 | cg20557603 | (normal vs normal) |
| 9-127215440 | cg13515842 | (cancer vs cancer) | 13-113653004 | cg21331791 | (normal vs normal) |
| **(cancer+normal vs cancer+normal)** | | | 13-25754851 | cg21442730 | (normal vs normal) |
| **Status - Hyper** | | | 13-29057716 | cg18662080 | (normal vs normal) |
| 1-214160848 | cg23992410 | (cancer+normal vs cancer+normal) | 13-45883505 | cg23515330 | (normal vs normal) |
| 1-239549626 | cg04412646 | (cancer vs cancer) (cancer+normal vs cancer+normal) | 13-49683801 | cg20433989 | (normal vs normal) |
| 1-239550378 | cg00371368 | (cancer vs cancer) (cancer+normal vs cancer+normal) | 13-78428203 | cg13487183 | (normal vs normal) |
| 1-240775075 | cg07808348 | (cancer vs cancer) (cancer+normal vs cancer+normal) | 14-105802918 | cg01052428 | (normal vs normal) |
| 1-240775160 | cg00155397 | (cancer vs cancer) (cancer+normal vs cancer+normal) | 14-65203330 | cg11802553 | (normal vs normal) |
| 1-76540168 | cg15090083 | (cancer vs cancer) (cancer+normal vs cancer+normal) | 15-101593693 | cg02262866 | (normal vs normal) |
| 1-76620271 | cg24580071 | (cancer+normal vs cancer+normal) | 15-43662311 | cg06158227 | (normal vs normal) |
| 11-14996456 | cg15240568 | (cancer+normal vs cancer+normal) | 15-80872041 | cg15647584 | (normal vs normal) |
| 12-127865826 | cg07366196 | (cancer vs cancer) (cancer+normal vs cancer+normal) | 15-89899729 | cg05042492 | (normal vs normal) |

| | | | | | |
|---|---|---|---|---|---|
| 13-100648306 | cg15470836 | (cancer vs cancer) (cancer+normal vs cancer+normal) | 15-94346887 | cg24334512 | (normal vs normal) |
| 13-28494497 | cg11153912 | (cancer+normal vs cancer+normal) | 16-10989530 | cg05371498 | (normal vs normal) |
| 13-28542727 | cg07213036 | (cancer vs cancer) (cancer+normal vs cancer+normal) | 16-67232921 | cg26178184 | (normal vs normal) |
| 13-29292888 | cg24321030 | (cancer vs cancer) (cancer+normal vs cancer+normal) | 16-67478355 | cg26774927 | (normal vs normal) |
| 13-29293133 | cg12775884 | (cancer vs cancer) (cancer+normal vs cancer+normal) | 16-70794856 | cg07563611 | (normal vs normal) |
| 13-29293196 | cg17128349 | (cancer vs cancer) (cancer+normal vs cancer+normal) | 17-182656 | cg18771195 | (normal vs normal) |
| 13-29293213 | cg20752818 | (cancer vs cancer) (cancer+normal vs cancer+normal) | 17-21454238 | cg22648282 | (normal vs normal) |
| 13-29293245 | cg21789898 | (cancer vs cancer) (cancer+normal vs cancer+normal) | 17-2608793 | cg23499846 | (normal vs normal) |
| 13-29293279 | cg18682423 | (cancer vs cancer) (cancer+normal vs cancer+normal) | 18-60984485 | cg27569829 | (normal vs normal) |
| 13-61989701 | cg05034702 | (cancer vs cancer) (cancer+normal vs cancer+normal) | 19-10407538 | cg21448471 | (normal vs normal) |
| 13-61989859 | cg02246190 | (cancer vs cancer) (cancer+normal vs cancer+normal) | 19-14313682 | cg02024579 | (normal vs normal) |
| 13-92050675 | cg13744663 | (cancer vs cancer) (cancer+normal vs cancer+normal) | 19-14376960 | cg22914338 | (normal vs normal) |
| 13-92050991 | cg19162106 | (cancer vs cancer) (cancer+normal vs cancer+normal) | 19-46807272 | cg26749414 | (normal vs normal) |
| 14-76819246 | cg11025763 | (cancer vs cancer) (cancer+normal vs cancer+normal) | 19-6534774 | cg14995475 | (normal vs normal) |
| 16-8223276 | cg00253851 | (cancer vs cancer) (cancer+normal vs cancer+normal) | 2-216947149 | cg13500113 | (normal vs normal) |
| 16-8223313 | cg08617528 | (cancer+normal vs cancer+normal) | 2-227850069 | cg09157320 | (normal vs normal) |
| 17-43663579 | cg22968622 | (normal vs normal) (cancer+normal vs cancer+normal) | 2-233995465 | cg25926572 | (normal vs normal) |
| 17-80279430 | cg11740099 | (cancer+normal vs cancer+normal) | 2-27604131 | cg02059082 | (normal vs normal) |
| 18-32556655 | cg05735765 | (cancer vs cancer) (cancer+normal vs cancer+normal) | 2-69519143 | cg02116942 | (normal vs normal) |
| 18-32557027 | cg20923716 | (cancer+normal vs cancer+normal) | 22-39852666 | cg21461856 | (normal vs normal) |
| 18-32557266 | cg14309384 | (cancer vs cancer) (cancer+normal vs cancer+normal) | 3-104556025 | cg17342916 | (normal vs normal) |
| 18-32557882 | cg24692068 | (cancer vs cancer) (cancer+normal vs | 3-11036537 | cg21771682 | (normal vs normal) |

| | | | | | |
|---|---|---|---|---|---|
| 18-32558052 | cg07326648 | (cancer+normal vs cancer+normal) | 3-129383357 | cg07443717 | (normal vs normal) |
| 18-32558066 | cg13550107 | (cancer vs cancer) (cancer+normal vs cancer+normal) | 3-137573683 | cg25358250 | (normal vs normal) |
| 19-12076051 | cg06647417 | (cancer vs cancer) (cancer+normal vs cancer+normal) | 3-184097168 | cg14047271 | (normal vs normal) |
| 19-20011770 | cg23501467 | (cancer vs cancer) (cancer+normal vs cancer+normal) | 3-186891900 | cg05491858 | (normal vs normal) |
| 19-21579902 | cg15420387 | (cancer vs cancer) (cancer+normal vs cancer+normal) | 4-101238231 | cg13149566 | (normal vs normal) |
| 19-21579911 | cg26151087 | (cancer+normal vs cancer+normal) | 4-74542972 | cg17679619 | (normal vs normal) |
| 19-23578063 | cg20103107 | (cancer+normal vs cancer+normal) | 5-102612899 | cg17605604 | (normal vs normal) |
| 19-23578260 | cg17853707 | (cancer vs cancer) (cancer+normal vs cancer+normal) | 5-1394484 | cg26924408 | (normal vs normal) |
| 19-23869985 | cg19361542 | (cancer vs cancer) (cancer+normal vs cancer+normal) | 5-1394633 | cg21163347 | (normal vs normal) |
| 19-2702986 | cg21340148 | (cancer vs cancer) (cancer+normal vs cancer+normal) | 5-143021669 | cg22877024 | (normal vs normal) |
| 19-28284813 | cg03170611 | (cancer+normal vs cancer+normal) | 5-169532758 | cg08295639 | (normal vs normal) |
| 19-49224165 | cg13974464 | (cancer vs cancer) (cancer+normal vs cancer+normal) | 5-24645487 | cg01058368 | (normal vs normal) |
| 19-52531703 | cg17428324 | (cancer+normal vs cancer+normal) | 5-389355 | cg17472786 | (normal vs normal) |
| 19-54410305 | cg02660823 | (cancer vs cancer) (cancer+normal vs cancer+normal) | 5-393366 | cg04551776 | (normal vs normal) |
| 19-57999026 | cg12501868 | (cancer vs cancer) (cancer+normal vs cancer+normal) | 5-54287462 | cg22473333 | (normal vs normal) |
| 19-57999036 | cg08134106 | (cancer vs cancer) (cancer+normal vs cancer+normal) | 5-64330880 | cg01061714 | (normal vs normal) |
| 19-57999041 | cg15745619 | (cancer vs cancer) (cancer+normal vs cancer+normal) | 6-13014871 | cg09510202 | (normal vs normal) |
| 19-57999044 | cg07873812 | (cancer+normal vs cancer+normal) | 6-138814716 | cg24601480 | (normal vs normal) |
| 19-57999177 | cg09260640 | (cancer vs cancer) (cancer+normal vs cancer+normal) | 6-144508118 | cg04281069 | (normal vs normal) |
| 19-57999447 | cg23015949 | (cancer+normal vs cancer+normal) | 6-167135152 | cg15215978 | (normal vs normal) |
| 19-58144608 | cg24863791 | (cancer+normal vs cancer+normal) | 6-32487314 | cg15708909 | (normal vs normal) |
| 2-127643971 | cg08535260 | (cancer vs cancer) (cancer+normal vs cancer+normal) | 7-100862300 | cg25527547 | (normal vs normal) |
| 2-136875315 | cg13854983 | (cancer vs cancer) (cancer+normal vs | 7-108137995 | cg24576298 | (normal vs normal) |

151

| | | cancer+normal) | | | |
|---|---|---|---|---|---|
| 2-155554688 | cg23491599 | (cancer vs cancer) (cancer+normal vs cancer+normal) | 7-130625473 | cg02729383 | (normal vs normal) |
| 2-155554707 | cg11706467 | (cancer vs cancer) (cancer+normal vs cancer+normal) | 7-13072314 | cg06226516 | (normal vs normal) |
| 2-168149401 | cg18395675 | (cancer vs cancer) (cancer+normal vs cancer+normal) | 7-143747474 | cg07055879 | (normal vs normal) |
| 3-137483063 | cg16921083 | (cancer vs cancer) (cancer+normal vs cancer+normal) | 7-149734481 | cg18356634 | (normal vs normal) (cancer+normal vs cancer+normal) |
| 3-137484517 | cg05880330 | (cancer vs cancer) (cancer+normal vs cancer+normal) | 7-1967621 | cg25193073 | (normal vs normal) |
| 3-138657291 | cg01711746 | (normal vs normal) (cancer+normal vs cancer+normal) | 7-39662467 | cg02433785 | (normal vs normal) |
| 3-138657429 | cg07287724 | (normal vs normal) (cancer+normal vs cancer+normal) | 8-28560467 | cg17048233 | (normal vs normal) |
| 3-138657541 | cg17769084 | (normal vs normal) (cancer+normal vs cancer+normal) | 8-30300223 | cg23830290 | (normal vs normal) |
| 3-138657666 | cg11023900 | (normal vs normal) (cancer+normal vs cancer+normal) | 8-49731668 | cg08218944 | (normal vs normal) |
| 3-138657774 | cg21530266 | (normal vs normal) (cancer+normal vs cancer+normal) | 8-55368762 | cg02898665 | (normal vs normal) |
| 3-62859461 | cg15997484 | (cancer vs cancer) (cancer+normal vs cancer+normal) | 8-61085389 | cg22628709 | (normal vs normal) |
| 4-46391694 | cg12205729 | (cancer vs cancer) (cancer+normal vs cancer+normal) | 8-93150831 | cg17308545 | (normal vs normal) |
| 4-64767210 | cg09615276 | (cancer+normal vs cancer+normal) | 8-93978802 | cg15244129 | (normal vs normal) |
| 5-113699604 | cg25194822 | (cancer+normal vs cancer+normal) | 9-137701344 | cg14656180 | (normal vs normal) |
| 5-124081751 | cg26413942 | (cancer+normal vs cancer+normal) | **Status - Hypo** | | |
| 5-160973736 | cg00524310 | (cancer vs cancer) (cancer+normal vs cancer+normal) | 1-112016586 | cg23068939 | (normal vs normal) |
| 5-168729558 | cg21545840 | (cancer+normal vs cancer+normal) | 1-112016615 | cg23894854 | (normal vs normal) |
| 5-39425547 | cg25770096 | (cancer+normal vs cancer+normal) | 1-11413470 | cg04136456 | (normal vs normal) |
| 5-57878745 | cg08261837 | (cancer vs cancer) (cancer+normal vs cancer+normal) | 1-149517402 | cg19372327 | (normal vs normal) |
| 5-57878750 | cg19416050 | (cancer+normal vs cancer+normal) | 1-156863643 | cg06188545 | (normal vs normal) |
| 5-57878853 | cg22023952 | (cancer+normal vs cancer+normal) | 1-44513358 | cg00002837 | (normal vs normal) |
| 5-57878882 | cg21617058 | (cancer vs cancer) (cancer+normal vs cancer+normal) | 1-46632465 | cg15711727 | (normal vs normal) |
| 5- | cg08445409 | (cancer vs cancer) | 1-64942153 | cg2622401 | (normal vs normal) |

| | | | | | |
|---|---|---|---|---|---|
| 57878919 | | (cancer+normal vs cancer+normal) | | 8 | |
| 5-57878953 | cg23123909 | (cancer vs cancer) (cancer+normal vs cancer+normal) | 1-92414221 | cg19245011 | (normal vs normal) |
| 5-87437106 | cg07513791 | (cancer vs cancer) (cancer+normal vs cancer+normal) | 1-92414295 | cg01515515 | (normal vs normal) (cancer vs cancer) (cancer+normal vs cancer+normal) |
| 5-87437337 | cg21153697 | (cancer vs cancer) (cancer+normal vs cancer+normal) | 10-119443899 | cg06592982 | (normal vs normal) |
| 5-87437400 | cg26246127 | (cancer vs cancer) (cancer+normal vs cancer+normal) | 10-134150489 | cg21370255 | (normal vs normal) |
| 5-87976156 | cg19625088 | (cancer vs cancer) (cancer+normal vs cancer+normal) | 10-28657329 | cg14552947 | (normal vs normal) |
| 5-87976294 | cg24025896 | (cancer vs cancer) (cancer+normal vs cancer+normal) | 10-35740216 | cg00201133 | (normal vs normal) |
| 5-87976545 | cg02627887 | (cancer+normal vs cancer+normal) | 10-35926227 | cg19230501 | (normal vs normal) |
| 5-87986642 | cg06523916 | (cancer vs cancer) (cancer+normal vs cancer+normal) | 10-89292231 | cg17940587 | (normal vs normal) |
| 6-166400871 | cg05861708 | (cancer+normal vs cancer+normal) | 10-95222867 | cg14060519 | (normal vs normal) |
| 6-28762552 | cg11400401 | (cancer vs cancer) (cancer+normal vs cancer+normal) | 11-33890131 | cg12695685 | (normal vs normal) |
| 6-32909003 | cg20392842 | (cancer+normal vs cancer+normal) | 11-47416535 | cg13912307 | (normal vs normal) |
| 7-11871535 | cg26616283 | (cancer vs cancer) (cancer+normal vs cancer+normal) | 11-47885166 | cg01423916 | (normal vs normal) |
| 7-11871837 | cg12348203 | (cancer vs cancer) (cancer+normal vs cancer+normal) | 11-65647270 | cg19792802 | (normal vs normal) |
| 7-1265325 | cg21768566 | (cancer vs cancer) (cancer+normal vs cancer+normal) | 12-66583431 | cg07914866 | (normal vs normal) |
| 7-152591581 | cg16806794 | (cancer vs cancer) (cancer+normal vs cancer+normal) | 13-40026426 | cg06309788 | (normal vs normal) |
| 8-12990437 | cg06164608 | (cancer vs cancer) (cancer+normal vs cancer+normal) | 14-105973925 | cg25974380 | (normal vs normal) |
| 8-12990622 | cg19024344 | (cancer vs cancer) (cancer+normal vs cancer+normal) | 14-21024898 | cg16701266 | (normal vs normal) |
| 8-12990744 | cg26464821 | (cancer vs cancer) (cancer+normal vs cancer+normal) | 14-92325159 | cg04794141 | (normal vs normal) |
| 8-12990898 | cg14904305 | (cancer vs cancer) (cancer+normal vs cancer+normal) | 14-93532563 | cg14181956 | (normal vs normal) |
| 8-12991143 | cg24819686 | (cancer vs cancer) (cancer+normal vs cancer+normal) | 15-27111435 | cg10146203 | (normal vs normal) |
| 8- | cg19040483 | (cancer vs cancer) | 15- | cg0967245 | (normal vs normal) |

| | | | | | |
|---|---|---|---|---|---|
| 12991196 | | (cancer+normal vs cancer+normal) | 55665236 | 2 | |
| 8-13134144 | cg03304380 | (cancer vs cancer) (cancer+normal vs cancer+normal) | 15-57592222 | cg13319446 | (normal vs normal) |
| 8-13134166 | cg20536983 | (cancer vs cancer) (normal vs normal) (cancer+normal vs cancer+normal) | 15-65198005 | cg00152322 | (normal vs normal) |
| **Status - Hypo** | | | 15-65342522 | cg09325101 | (normal vs normal) |
| 1-116311395 | cg21648376 | (normal vs normal) (cancer+normal vs cancer+normal) | 16-20756332 | cg04695882 | (normal vs normal) |
| 1-116311561 | cg17903306 | (normal vs normal) (cancer+normal vs cancer+normal) | 16-54404709 | cg00253379 | (normal vs normal) |
| 1-1168432 | cg13924635 | (cancer vs cancer) (cancer+normal vs cancer+normal) | 16-55359698 | cg01554410 | (normal vs normal) |
| 1-1168541 | cg22220310 | (cancer vs cancer) (cancer+normal vs cancer+normal) | 16-84228377 | cg07484910 | (normal vs normal) |
| 1-1168550 | cg14477263 | (cancer vs cancer) (cancer+normal vs cancer+normal) | 16-86029554 | cg08787837 | (normal vs normal) |
| 1-149871945 | cg09045105 | (cancer vs cancer) (cancer+normal vs cancer+normal) | 17-2839187 | cg11869193 | (normal vs normal) |
| 1-154238265 | cg14859874 | (normal vs normal) (normal vs normal) (cancer+normal vs cancer+normal) | 17-32964596 | cg17276853 | (normal vs normal) |
| 1-164812337 | cg24039780 | (cancer+normal vs cancer+normal) | 17-34198571 | cg14796318 | (normal vs normal) |
| 1-180917873 | cg15109744 | (normal vs normal) (cancer+normal vs cancer+normal) | 17-34345136 | cg14916288 | (normal vs normal) |
| 1-19687257 | cg12763668 | (cancer vs cancer) (cancer+normal vs cancer+normal) | 17-34345172 | cg10439431 | (normal vs normal) |
| 1-19764821 | cg23111655 | (cancer+normal vs cancer+normal) | 17-35717813 | cg07375836 | (normal vs normal) |
| 1-2236443 | cg20168901 | (cancer+normal vs cancer+normal) | 17-43037309 | cg02395846 | (normal vs normal) (normal vs normal) |
| 1-2236650 | cg07425568 | (cancer+normal vs cancer+normal) | 17-74557625 | cg19439071 | (normal vs normal) |
| 1-243638141 | cg06721712 | (cancer+normal vs cancer+normal) | 17-76380922 | cg09980384 | (normal vs normal) |
| 1-26107517 | cg03848004 | (cancer+normal vs cancer+normal) | 17-80804126 | cg07450698 | (normal vs normal) (cancer+normal vs cancer+normal) |
| 1-29446085 | cg26144567 | (cancer+normal vs cancer+normal) | 17-8771003 | cg16268473 | (normal vs normal) |
| 1-32826969 | cg08843283 | (cancer vs cancer) (cancer+normal vs cancer+normal) | 17-8771055 | cg06613765 | (normal vs normal) |
| 10-103420568 | cg15941413 | (cancer+normal vs cancer+normal) | 18-77277583 | cg16179938 | (normal vs normal) |
| 10-105210440 | cg09562683 | (normal vs normal) (cancer+normal vs | 19-17634172 | cg06305609 | (normal vs normal) |

| | | | | | | |
|---|---|---|---|---|---|---|
| | | cancer+normal) | 19-2085301 | cg1951931 9 | (normal vs normal) |
| 10-12853879 | cg20683445 | (cancer+normal vs cancer+normal) | 19-2085301 | cg1951931 9 | (normal vs normal) |
| 11-117075078 | cg11609668 | (cancer+normal vs cancer+normal) | 19-33699683 | cg0270903 2 | (normal vs normal) |
| 11-2735101 | cg07592519 | (cancer+normal vs cancer+normal) | 19-4916783 | cg1890110 4 | (normal vs normal) |
| 11-33081126 | cg17485717 | (cancer+normal vs cancer+normal) | 2-202125212 | cg2684280 2 | (normal vs normal) |
| 11-44243314 | cg05495611 | (normal vs normal) (cancer+normal vs cancer+normal) | 2-240174613 | cg0647850 4 | (normal vs normal) |
| 11-88032774 | cg12710531 | (normal vs normal) (cancer+normal vs cancer+normal) | 2-240174650 | cg1793988 9 | (normal vs normal) |
| 12-133414334 | cg26415787 | (cancer+normal vs cancer+normal) | 2-240174659 | cg1402005 2 | (normal vs normal) |
| 12-15907517 | cg01975858 | (cancer+normal vs cancer+normal) | 2-54697181 | cg1666725 1 | (normal vs normal) (cancer+normal vs cancer+normal) |
| 12-77156412 | cg09192801 | (cancer+normal vs cancer+normal) | 2-54823768 | cg1592868 0 | (normal vs normal) |
| 13-113407548 | cg04656015 | (cancer+normal vs cancer+normal) | 20-30176106 | cg0834075 5 | (normal vs normal) (cancer+normal vs cancer+normal) |
| 13-114150035 | cg23167246 | (cancer+normal vs cancer+normal) | 20-56073589 | cg1585848 3 | (normal vs normal) |
| 13-114203831 | cg12939777 | (cancer+normal vs cancer+normal) | 22-17956453 | cg0101021 1 | (normal vs normal) (cancer+normal vs cancer+normal) |
| 13-50190584 | cg03295274 | (cancer+normal vs cancer+normal) | 22-17956641 | cg0719723 0 | (normal vs normal) |
| 14-31858202 | cg01358620 | (cancer+normal vs cancer+normal) | 22-35940438 | cg1228438 2 | (normal vs normal) |
| 14-51854261 | cg00750630 | (normal vs normal) (cancer+normal vs cancer+normal) | 22-42828447 | cg0138395 5 | (normal vs normal) |
| 15-30163660 | cg00232832 | (cancer+normal vs cancer+normal) | 3-14853225 | cg0264378 2 | (normal vs normal) |
| 15-31355304 | cg20640374 | (normal vs normal) (cancer+normal vs cancer+normal) | 3-183309985 | cg0442561 7 | (normal vs normal) |
| 16-1583899 | cg07341220 | (normal vs normal) (cancer+normal vs cancer+normal) | 3-192675515 | cg2570265 1 | (normal vs normal) |
| 16-1583984 | cg00463982 | (normal vs normal) (cancer+normal vs cancer+normal) | 3-194901357 | cg1543165 9 | (normal vs normal) |
| 16-68378359 | cg05110241 | (cancer+normal vs cancer+normal) | 3-32993063 | cg2381798 1 | (normal vs normal) |
| 16-69958189 | cg26832686 | (cancer+normal vs cancer+normal) | 3-50280404 | cg0152058 6 | (normal vs normal) |
| 16-69958298 | cg01578632 | (cancer+normal vs cancer+normal) | 4-100244604 | cg2446753 5 | (normal vs normal) |
| 16-75328793 | cg16589807 | (cancer+normal vs cancer+normal) | 4-2748026 | cg0709429 8 | (normal vs normal) |
| 16-89468673 | cg04340114 | (cancer+normal vs cancer+normal) | 4-788117 | cg0152618 3 | (normal vs normal) |
| 17-17449166 | cg26177311 | (normal vs normal) (cancer+normal vs cancer+normal) | 5-100239919 | cg0040569 9 | (normal vs normal) |

155

| | | | | | |
|---|---|---|---|---|---|
| 17-18918410 | cg17876831 | (cancer+normal vs cancer+normal) | 5-141395447 | cg05979766 | (normal vs normal) |
| 17-1975245 | cg03025825 | (cancer+normal vs cancer+normal) | 5-142382188 | cg22184990 | (normal vs normal) |
| 17-40851514 | cg24808754 | (cancer+normal vs cancer+normal) | 5-142444988 | cg00997251 | (normal vs normal) |
| 17-42289980 | cg08499158 | (cancer+normal vs cancer+normal) | 5-176294068 | cg11139090 | (normal vs normal) |
| 17-48263173 | cg03743861 | (cancer+normal vs cancer+normal) | 5-40841315 | cg05305046 | (normal vs normal) |
| 17-48263189 | cg11615029 | (cancer+normal vs cancer+normal) | 5-56595244 | cg19985056 | (normal vs normal) |
| 17-48263204 | cg25735490 | (cancer+normal vs cancer+normal) | 5-6775922 | cg05625559 | (normal vs normal) |
| 17-61927036 | cg13332729 | (cancer vs cancer) (cancer+normal vs cancer+normal) | 5-72701818 | cg05868410 | (normal vs normal) |
| 17-78892850 | cg21000762 | (normal vs normal) (cancer+normal vs cancer+normal) | 5-81470056 | cg15153141 | (normal vs normal) |
| 17-78893363 | cg15331383 | (cancer+normal vs cancer+normal) | 6-132066129 | cg18334681 | (normal vs normal) |
| 17-78893429 | cg01525498 | (cancer+normal vs cancer+normal) | 6-155739835 | cg05797854 | (normal vs normal) |
| 17-78894364 | cg15694704 | (cancer+normal vs cancer+normal) | 6-158447883 | cg08918658 | (normal vs normal) |
| 17-78894477 | cg21818807 | (cancer+normal vs cancer+normal) | 6-160241360 | cg17481235 | (normal vs normal) |
| 17-80137075 | cg11935831 | (cancer+normal vs cancer+normal) | 6-170463397 | cg01622006 | (normal vs normal) |
| 17-821621 | cg02459042 | (normal vs normal) (cancer+normal vs cancer+normal) | 6-29856353 | cg09208740 | (normal vs normal) |
| 19-45150513 | cg07455685 | (cancer+normal vs cancer+normal) | 6-52173014 | cg01761966 | (normal vs normal) |
| 19-45866874 | cg01518138 | (cancer+normal vs cancer+normal) | 7-115995934 | cg18887458 | (normal vs normal) |
| 19-50392917 | cg23074703 | (cancer vs cancer) (cancer+normal vs cancer+normal) | 7-150147988 | cg07223253 | (normal vs normal) |
| 2-109203501 | cg01942372 | (cancer+normal vs cancer+normal) | 7-39629290 | cg13800005 | (normal vs normal) |
| 2-118700475 | cg13482326 | (cancer+normal vs cancer+normal) | 8-105342491 | cg04554929 | (normal vs normal) |
| 2-135203925 | cg15584989 | (normal vs normal) (cancer+normal vs cancer+normal) | 8-41168186 | cg03575666 | (normal vs normal) |
| 2-238647755 | cg17032848 | (normal vs normal) (cancer+normal vs cancer+normal) | 8-41168205 | cg09410389 | (normal vs normal) |
| 2-242835165 | cg19851200 | (cancer+normal vs cancer+normal) | 9-126135610 | cg13558371 | (normal vs normal) |
| 2-242988706 | cg06360820 | (cancer+normal vs cancer+normal) | 9-139557920 | cg08529852 | (normal vs normal) (cancer+normal vs cancer+normal) |
| 2-3452563 | cg11642891 | (cancer+normal vs cancer+normal) | **LuCaG (cancer vs cancer)** | | |
| 2-3453196 | cg16793199 | (cancer+normal vs cancer+normal) | **Status - Hyper** | | |
| 2-39188006 | cg03690080 | (cancer vs cancer) (cancer+normal vs | 1-10856707 | cg16523380 | (cancer vs cancer) |

156

| | | | | | |
|---|---|---|---|---|---|
| | | cancer+normal) | | | |
| 2-46747628 | cg02822958 | (cancer+normal vs cancer+normal) | 1-23668691 | cg00907427 | (cancer vs cancer) (cancer vs cancer) (normal vs normal) (cancer+normal vs cancer+normal) |
| 2-55393977 | cg19384241 | (normal vs normal) (cancer+normal vs cancer+normal) | 1-28764523 | cg08123074 | (cancer vs cancer) (cancer vs cancer) |
| 20-57463991 | cg09885502 | (normal vs normal) (cancer+normal vs cancer+normal) | 1-40609565 | cg04865976 | (cancer vs cancer) (cancer vs cancer) (cancer+normal vs cancer+normal) |
| 20-58508076 | cg21950459 | (cancer vs cancer) (cancer+normal vs cancer+normal) | 1-44884204 | cg12049688 | (cancer vs cancer) (cancer vs cancer) (cancer+normal vs cancer+normal) |
| 20-58508123 | cg12181372 | (cancer vs cancer) (cancer+normal vs cancer+normal) | 1-88928362 | cg09152582 | (cancer vs cancer) |
| 3-113458144 | cg15067606 | (cancer+normal vs cancer+normal) | 10-102489344 | cg22748290 | (cancer vs cancer) (cancer vs cancer) |
| 3-123418791 | cg20230305 | (cancer+normal vs cancer+normal) | 10-102982492 | cg18334881 | (cancer vs cancer) (cancer vs cancer) |
| 3-139353025 | cg00893348 | (cancer+normal vs cancer+normal) | 10-124910550 | cg02847649 | (cancer vs cancer) |
| 3-139353058 | cg15093769 | (normal vs normal) (cancer+normal vs cancer+normal) | 10-128593943 | cg22665692 | (cancer vs cancer) |
| 3-148804746 | cg02611507 | (cancer vs cancer) (cancer+normal vs cancer+normal) | 10-128593946 | cg23665778 | (cancer vs cancer) |
| 4-24745718 | cg01464067 | (cancer+normal vs cancer+normal) | 10-128594141 | cg18932726 | (cancer vs cancer) |
| 5-65506308 | cg06049297 | (cancer+normal vs cancer+normal) | 10-128594144 | cg05608777 | (cancer vs cancer) |
| 6-170065938 | cg21796935 | (normal vs normal) (cancer+normal vs cancer+normal) | 10-8091753 | cg22783180 | (cancer vs cancer) (cancer vs cancer) (cancer+normal vs cancer+normal) |
| 6-33228341 | cg13809627 | (cancer+normal vs cancer+normal) | 10-8092165 | cg15803869 | (cancer vs cancer) (cancer vs cancer) (cancer+normal vs cancer+normal) |
| 7-100421502 | cg21129976 | (cancer+normal vs cancer+normal) | 10-8092264 | cg16710894 | (cancer vs cancer) (cancer vs cancer) (cancer+normal vs cancer+normal) |
| 7-151833893 | cg11872373 | (cancer+normal vs cancer+normal) | 10-81206376 | cg16584688 | (cancer vs cancer) |
| 7-2144579 | cg18641463 | (cancer+normal vs cancer+normal) | 11-130030103 | cg10089145 | (cancer vs cancer) (cancer+normal vs cancer+normal) |
| 7-4763182 | cg18733230 | (cancer+normal vs cancer+normal) | 11-31831974 | cg18270629 | (cancer vs cancer) (cancer vs cancer) (cancer+normal vs cancer+normal) |
| 7-4806755 | cg06114605 | (cancer vs cancer) (cancer vs cancer) (cancer+normal vs cancer+normal) | 11-31832246 | cg26848086 | (cancer vs cancer) (cancer vs cancer) (cancer+normal vs cancer+normal) |

| | | | | | |
|---|---|---|---|---|---|
| 7-4806869 | cg05102770 | (cancer+normal vs cancer+normal) | 11-36615998 | cg1036418 2 | (cancer vs cancer) (cancer vs cancer) |
| 7-4806949 | cg25330264 | (cancer vs cancer) (cancer vs cancer) (cancer+normal vs cancer+normal) | 11-36616015 | cg2167160 7 | (cancer vs cancer) |
| 7-4807062 | cg06496454 | (cancer+normal vs cancer+normal) | 11-36616048 | cg2682132 7 | (cancer vs cancer) |
| 7-4807525 | cg17625535 | (cancer+normal vs cancer+normal) | 11-57529255 | cg2127299 6 | (cancer vs cancer) (cancer vs cancer) |
| 7-5389516 | cg20025824 | (cancer+normal vs cancer+normal) | 11-57529419 | cg1083121 2 | (cancer vs cancer) (cancer vs cancer) |
| 7-811786 | cg03184424 | (cancer+normal vs cancer+normal) | 11-57529465 | cg1921027 6 | (cancer vs cancer) (cancer vs cancer) |
| 7-837536 | cg21113846 | (cancer+normal vs cancer+normal) | 11-61739400 | cg1944699 0 | (cancer vs cancer) |
| 8-143782585 | cg10802543 | (cancer vs cancer) (cancer+normal vs cancer+normal) | 11-67777823 | cg0233130 3 | (cancer vs cancer) (cancer vs cancer) (cancer+normal vs cancer+normal) |
| 8-30300142 | cg03408619 | (cancer+normal vs cancer+normal) | 12-28128567 | cg0046760 0 | (cancer vs cancer) (cancer vs cancer) (cancer+normal vs cancer+normal) |
| 8-42659977 | cg06815361 | (cancer+normal vs cancer+normal) | 12-88974484 | cg2754995 2 | (cancer vs cancer) |
| **(normal vs normal)** | | | 13-114301996 | cg2593635 8 | (cancer vs cancer) |
| **Status - Hyper** | | | 13-114302021 | cg1221219 8 | (cancer vs cancer) |
| 1-101704504 | cg10020333 | (normal vs normal) | 14-34420415 | cg0812214 0 | (cancer vs cancer) |
| 1-233751191 | cg13110283 | (normal vs normal) | 15-40633194 | cg0198720 2 | (cancer vs cancer) |
| 10-73848915 | cg02595823 | (normal vs normal) | 15-67814880 | cg2270688 3 | (cancer vs cancer) (cancer vs cancer) |
| 10-82295723 | cg24440658 | (normal vs normal) | 15-90456248 | cg2241005 7 | (cancer vs cancer) |
| 10-82295864 | cg15642854 | (normal vs normal) | 15-98504668 | cg2275593 2 | (cancer vs cancer) |
| 10-82296072 | cg09981929 | (normal vs normal) | 16-30817058 | cg1102777 2 | (cancer vs cancer) |
| 11-10949337 | cg20815992 | (normal vs normal) | 16-55090946 | cg1118117 1 | (cancer vs cancer) (cancer vs cancer) (cancer+normal vs cancer+normal) |
| 11-30510489 | cg17320698 | (normal vs normal) | 16-70720149 | cg2766518 1 | (cancer vs cancer) |
| 11-316456 | cg27331665 | (normal vs normal) | 16-83842404 | cg0806829 6 | (cancer vs cancer) (cancer+normal vs cancer+normal) |
| 11-44325823 | cg04549333 | (normal vs normal) | 16-86478190 | cg0427608 4 | (cancer vs cancer) (cancer vs cancer) |
| 11-44327399 | cg04970352 | (normal vs normal) | 16-86609963 | cg0131390 4 | (cancer vs cancer) (cancer vs cancer) (cancer+normal vs cancer+normal) |
| 12-115105519 | cg22741264 | (normal vs normal) | 16-90089283 | cg0363499 7 | (cancer vs cancer) |
| 12-115861520 | cg02506008 | (normal vs normal) | 17-25707428 | cg0819400 9 | (cancer vs cancer) |

| | | | | | |
|---|---|---|---|---|---|
| 12-123383399 | cg11586448 | (normal vs normal) (cancer+normal vs cancer+normal) | 17-37183552 | cg16864825 | (cancer vs cancer) |
| 12-25055304 | cg08724310 | (normal vs normal) | 17-37183683 | cg02443072 | (cancer vs cancer) |
| 12-49485085 | cg15878670 | (normal vs normal) | 17-72427628 | cg15437528 | (cancer vs cancer) |
| 12-54389891 | cg24208826 | (normal vs normal) | 17-79370518 | cg06873452 | (cancer vs cancer) |
| 12-54396990 | cg22679316 | (normal vs normal) | 17-79533843 | cg25072666 | (cancer vs cancer) |
| 12-54420837 | cg03892356 | (normal vs normal) | 18-29304111 | cg07064495 | (cancer vs cancer) (cancer vs cancer) |
| 12-54424203 | cg13826247 | (normal vs normal) | 18-45688478 | cg17789102 | (cancer vs cancer) |
| 12-89749033 | cg22466678 | (normal vs normal) | 18-72124683 | cg06365984 | (cancer vs cancer) |
| 13-111193758 | cg07800658 | (normal vs normal) | 19-13107252 | cg06197482 | (cancer vs cancer) (cancer vs cancer) |
| 14-48148429 | cg13721907 | (normal vs normal) | 19-1676011 | cg08091147 | (cancer vs cancer) (cancer vs cancer) |
| 15-99987288 | cg20909380 | (normal vs normal) | 19-2169160 | cg27073066 | (cancer vs cancer) (cancer vs cancer) |
| 16-86547203 | cg06834912 | (normal vs normal) | 19-44556485 | cg06890553 | (cancer vs cancer) |
| 16-86547530 | cg00551679 | (normal vs normal) | 2-224467359 | cg21616047 | (cancer vs cancer) (cancer vs cancer) (cancer+normal vs cancer+normal) |
| 17-41522069 | cg03184776 | (normal vs normal) | 2-236402544 | cg06059032 | (cancer vs cancer) |
| 17-43663579 | cg22968622 | (normal vs normal) (cancer+normal vs cancer+normal) | 2-68073599 | cg01475649 | (cancer vs cancer) |
| 17-46651952 | cg24963001 | (normal vs normal) | 2-85999628 | cg08079596 | (cancer vs cancer) |
| 17-46652179 | cg13280788 | (normal vs normal) | 20-11871375 | cg14603345 | (cancer vs cancer) |
| 17-46656543 | cg05527785 | (normal vs normal) | 20-11871396 | cg12894649 | (cancer vs cancer) |
| 17-46659019 | cg14285150 | (normal vs normal) | 20-11872206 | cg20159775 | (cancer vs cancer) |
| 17-46679135 | cg14509967 | (normal vs normal) | 20-23618481 | cg05824225 | (cancer vs cancer) |
| 17-55444427 | cg04573500 | (normal vs normal) | 20-46413743 | cg21343919 | (cancer vs cancer) (cancer vs cancer) (cancer+normal vs cancer+normal) |
| 17-70536199 | cg23398508 | (normal vs normal) | 20-46414900 | cg11979959 | (cancer vs cancer) |
| 17-79011277 | cg15867652 | (normal vs normal) | 20-46414934 | cg22084611 | (cancer vs cancer) (cancer vs cancer) |
| 18-55714052 | cg20976694 | (normal vs normal) | 20-46414999 | cg19754861 | (cancer vs cancer) |
| 18-56888922 | cg19611392 | (normal vs normal) | 21-38071066 | cg05573262 | (cancer vs cancer) |
| 18-72166303 | cg14963724 | (normal vs normal) | 21-38071688 | cg12630953 | (cancer vs cancer) |
| 18-911318 | cg14479567 | (normal vs normal) | 21-38071807 | cg14769207 | (cancer vs cancer) (cancer vs cancer) |
| 19- | cg20725493 | (normal vs normal) | 21- | cg2435860 | (cancer vs cancer) |

| | | |
|---|---|---|
| 15740067 | | |
| 2-173601413 | cg14234152 | (normal vs normal) |
| 2-177054573 | cg02466815 | (normal vs normal) |
| 2-20865651 | cg23719318 | (normal vs normal) |
| 2-228093069 | cg27243623 | (normal vs normal) |
| 2-242892815 | cg18781835 | (normal vs normal) |
| 2-38342447 | cg26682499 | (normal vs normal) |
| 2-54900343 | cg16198930 | (normal vs normal) |
| 2-85805621 | cg08379897 | (normal vs normal) |
| 20-3730680 | cg17361896 | (normal vs normal) |
| 21-15352213 | cg18681837 | (normal vs normal) |
| 3-126007325 | cg03438417 | (normal vs normal) |
| 3-138655827 | cg02744524 | (normal vs normal) |
| 3-138656042 | cg02704158 | (normal vs normal) |
| 3-138656628 | cg05099537 | (normal vs normal) |
| 3-138657291 | cg01711746 | (normal vs normal) (cancer+normal vs cancer+normal) |
| 3-138657429 | cg07287724 | (normal vs normal) (cancer+normal vs cancer+normal) |
| 3-138657541 | cg17769084 | (normal vs normal) (cancer+normal vs cancer+normal) |
| 3-138657666 | cg11023900 | (normal vs normal) (cancer+normal vs cancer+normal) |
| 3-138657774 | cg21530266 | (normal vs normal) (cancer+normal vs cancer+normal) |
| 3-138658243 | cg06970190 | (normal vs normal) |
| 3-138658345 | cg15665928 | (normal vs normal) |
| 3- | cg02019774 | (normal vs normal) |

| | | |
|---|---|---|
| 40984553 | 3 | (cancer vs cancer) |
| 21-40984610 | cg20269359 | (cancer vs cancer) (cancer vs cancer) |
| 22-41763255 | cg23915792 | (cancer vs cancer) |
| 4-125633640 | cg00317648 | (cancer vs cancer) |
| 4-128553917 | cg13084458 | (cancer vs cancer) |
| 4-77134471 | cg19050061 | (cancer vs cancer) |
| 4-85888263 | cg04595835 | (cancer vs cancer) |
| 4-94749609 | cg02640260 | (cancer vs cancer) |
| 5-135528543 | cg00054702 | (cancer vs cancer) (cancer vs cancer) |
| 5-174178256 | cg24996482 | (cancer vs cancer) (cancer vs cancer) |
| 5-65018961 | cg17329494 | (cancer vs cancer) (cancer+normal vs cancer+normal) |
| 6-110736865 | cg06413398 | (cancer vs cancer) (cancer vs cancer) (cancer+normal vs cancer+normal) |
| 6-110736941 | cg00804078 | (cancer vs cancer) (cancer vs cancer) (cancer+normal vs cancer+normal) |
| 6-110736958 | cg07164639 | (cancer vs cancer) |
| 6-144608500 | cg15785898 | (cancer vs cancer) (cancer vs cancer) (cancer+normal vs cancer+normal) |
| 6-149775621 | cg07346187 | (cancer vs cancer) |
| 6-152623304 | cg05917732 | (cancer vs cancer) (cancer vs cancer) (normal vs normal) (cancer+normal vs cancer+normal) |
| 6-152623387 | cg02682457 | (cancer vs cancer) (cancer vs cancer) (normal vs normal) (cancer+normal vs cancer+normal) |
| 6-50680997 | cg17489695 | (cancer vs cancer) (cancer vs cancer) |
| 7-2271722 | cg03694580 | (cancer vs cancer) (cancer vs cancer) (cancer+normal vs cancer+normal) |
| 7-5467366 | cg05406868 | (cancer vs cancer) |
| 8-21988147 | cg26702929 | (cancer vs cancer) (cancer vs cancer) |
| 8-21988173 | cg1274825 | (cancer vs cancer) |

| | | | | | |
|---|---|---|---|---|---|
| 138658470 | | | | 8 | |
| 3-138658554 | cg24625136 | (normal vs normal) | 8-22102083 | cg0490521 0 | (cancer vs cancer) |
| 3-138658704 | cg21575509 | (normal vs normal) | 8-93977794 | cg1656071 1 | (cancer vs cancer) |
| 3-138658878 | cg22132508 | (normal vs normal) | 8-93978221 | cg1044058 3 | (cancer vs cancer) |
| 3-138658941 | cg14504269 | (normal vs normal) | 9-107827215 | cg1408506 0 | (cancer vs cancer) |
| 3-20085136 | cg15910502 | (normal vs normal) | 9-81871926 | cg1456087 2 | (cancer vs cancer) (cancer vs cancer) (cancer+normal vs cancer+normal) |
| 4-4871525 | cg02680341 | (cancer vs cancer) (normal vs normal) (cancer+normal vs cancer+normal) | **Status - Hypo** | | |
| 4-64223949 | cg25017774 | (normal vs normal) | 1-110923328 | cg2405836 5 | (cancer vs cancer) (cancer vs cancer) (cancer+normal vs cancer+normal) |
| 5-124461245 | cg22959827 | (normal vs normal) | 1-117151711 | cg1264350 2 | (cancer vs cancer) (cancer vs cancer) |
| 5-140018709 | cg15646382 | (normal vs normal) | 1-154179856 | cg1452095 7 | (cancer vs cancer) (cancer vs cancer) (cancer+normal vs cancer+normal) |
| 5-1594678 | cg24960960 | (cancer vs cancer) (cancer vs cancer) (normal vs normal) | 1-154179996 | cg0289676 8 | (cancer vs cancer) (cancer vs cancer) (cancer+normal vs cancer+normal) |
| 5-1594715 | cg21931717 | (cancer vs cancer) (cancer vs cancer) (normal vs normal) | 1-156711439 | cg0034644 6 | (cancer vs cancer) |
| 6-134213992 | cg19324940 | (normal vs normal) | 1-166806295 | cg0471916 6 | (cancer vs cancer) (cancer vs cancer) (cancer+normal vs cancer+normal) |
| 6-157254584 | cg03561416 | (normal vs normal) | 1-183204789 | cg2268496 9 | (cancer vs cancer) (cancer vs cancer) (cancer+normal vs cancer+normal) |
| 6-167508226 | cg05259303 | (normal vs normal) | 1-183204836 | cg1442023 0 | (cancer vs cancer) (cancer vs cancer) (cancer+normal vs cancer+normal) |
| 6-32064491 | cg20414186 | (normal vs normal) | 1-212671907 | cg0042608 9 | (cancer vs cancer) (cancer vs cancer) (cancer+normal vs cancer+normal) |
| 6-32064497 | cg03556669 | (normal vs normal) | 1-225697019 | cg0069039 2 | (cancer vs cancer) (cancer vs cancer) |
| 6-32064508 | cg24882324 | (normal vs normal) | 1-232761750 | cg2742894 8 | (cancer vs cancer) |
| 6-32064578 | cg13400512 | (normal vs normal) | 1-236162295 | cg1652481 8 | (cancer vs cancer) (cancer vs cancer) (cancer+normal vs cancer+normal) |
| 6-32522683 | cg25140213 | (cancer vs cancer) (normal vs normal) (cancer+normal vs | 1-43399327 | cg2147425 7 | (cancer vs cancer) |

| | | cancer+normal) | | | |
|---|---|---|---|---|---|
| 6-32549283 | cg12867728 | (cancer vs cancer) (normal vs normal) | 1-61750543 | cg00560072 | (cancer vs cancer) (cancer vs cancer) (cancer+normal vs cancer+normal) |
| 6-85484863 | cg06187770 | (normal vs normal) | 1-86052061 | cg25606842 | (cancer vs cancer) |
| 7-150498493 | cg03964111 | (normal vs normal) | 1-92414344 | cg06442199 | (cancer vs cancer) (cancer vs cancer) (cancer+normal vs cancer+normal) |
| 7-27160520 | cg24469729 | (normal vs normal) | 1-92414520 | cg03421440 | (cancer vs cancer) (cancer vs cancer) (cancer+normal vs cancer+normal) |
| 7-27160674 | cg20688917 | (normal vs normal) | 10-1120601 | cg15245292 | (cancer vs cancer) |
| 7-27167828 | cg11267546 | (normal vs normal) | 10-112444795 | cg26292895 | (cancer vs cancer) |
| 7-27182493 | cg11724970 | (normal vs normal) | 10-131592944 | cg06633615 | (cancer vs cancer) (cancer vs cancer) (cancer+normal vs cancer+normal) |
| 7-27183133 | cg23936031 | (normal vs normal) | 11-130089890 | cg03933026 | (cancer vs cancer) (cancer vs cancer) (cancer+normal vs cancer+normal) |
| 7-27183196 | cg02248486 | (normal vs normal) | 11-61125360 | cg14331899 | (cancer vs cancer) |
| 7-27183401 | cg19759481 | (normal vs normal) | 11-66515382 | cg19037291 | (cancer vs cancer) |
| 7-27183643 | cg17569124 | (normal vs normal) | 11-86224410 | cg27309871 | (cancer vs cancer) (cancer vs cancer) (cancer+normal vs cancer+normal) |
| 7-27183694 | cg25307665 | (normal vs normal) | 12-132259591 | cg15817163 | (cancer vs cancer) (cancer vs cancer) |
| 7-27194731 | cg16764637 | (normal vs normal) | 12-94145366 | cg23925994 | (cancer vs cancer) |
| 7-27236674 | cg01363170 | (normal vs normal) | 12-99029458 | cg06353075 | (cancer vs cancer) |
| 8-138135392 | cg08294772 | (normal vs normal) | 13-113420371 | cg08557188 | (normal vs normal) (cancer vs cancer) (cancer vs cancer) (cancer+normal vs cancer+normal) |
| 8-37699923 | cg01226742 | (normal vs normal) | 13-97787063 | cg03550548 | (cancer vs cancer) (cancer vs cancer) (cancer+normal vs cancer+normal) |
| 9-38069959 | cg13843266 | (normal vs normal) | 13-98869844 | cg16689193 | (cancer vs cancer) (cancer vs cancer) (cancer+normal vs cancer+normal) |
| 9-92291269 | cg13552373 | (normal vs normal) | 14-91691190 | cg14304073 | (cancer vs cancer) (cancer vs cancer) (cancer+normal vs cancer+normal) |
| **Status - Hypo** | | | 15-73889528 | cg06332339 | (cancer vs cancer) (cancer vs cancer) (cancer+normal vs |

| | | | | | |
|---|---|---|---|---|---|
| | | | | | cancer+normal) |
| 1-116311395 | cg21648376 | (normal vs normal) (cancer+normal vs cancer+normal) | 15-80217380 | cg22389121 | (normal vs normal) (cancer vs cancer) (cancer vs cancer) (cancer+normal vs cancer+normal) |
| 1-116311561 | cg17903306 | (normal vs normal) (cancer+normal vs cancer+normal) | 16-2544909 | cg00794055 | (cancer vs cancer) (cancer vs cancer) (cancer+normal vs cancer+normal) |
| 1-154238265 | cg14859874 | (normal vs normal) (normal vs normal) (cancer+normal vs cancer+normal) | 16-3139060 | cg20364187 | (cancer vs cancer) (cancer vs cancer) (cancer+normal vs cancer+normal) |
| 1-162641005 | cg21539842 | (normal vs normal) | 17-79953059 | cg14419393 | (cancer vs cancer) (cancer vs cancer) |
| 1-172113756 | cg25214966 | (normal vs normal) (cancer vs cancer) (cancer+normal vs cancer+normal) | 17-80970799 | cg03989711 | (cancer vs cancer) (cancer+normal vs cancer+normal) |
| 1-180917873 | cg15109744 | (normal vs normal) (cancer+normal vs cancer+normal) | 17-80970825 | cg11824564 | (cancer vs cancer) (cancer vs cancer) (cancer+normal vs cancer+normal) |
| 1-23474871 | cg12483005 | (normal vs normal) | 17-8928028 | cg01862363 | (cancer vs cancer) (cancer+normal vs cancer+normal) |
| 1-236147721 | cg03396152 | (normal vs normal) | 19-12880789 | cg01632288 | (cancer vs cancer) |
| 1-244327251 | cg15718528 | (normal vs normal) | 19-4099405 | cg18156417 | (cancer vs cancer) (cancer vs cancer) (cancer+normal vs cancer+normal) |
| 1-45103967 | cg09899564 | (normal vs normal) | 19-5789699 | cg22961623 | (cancer vs cancer) (cancer vs cancer) (cancer+normal vs cancer+normal) |
| 1-60171660 | cg10220544 | (normal vs normal) | 2-105857809 | cg11223196 | (cancer vs cancer) |
| 10-105210440 | cg09562683 | (normal vs normal) (cancer+normal vs cancer+normal) | 2-105858013 | cg09510339 | (cancer vs cancer) |
| 10-13380770 | cg02257172 | (normal vs normal) | 2-132348705 | cg11467141 | (cancer vs cancer) (cancer vs cancer) (cancer+normal vs cancer+normal) |
| 10-13987636 | cg23443098 | (normal vs normal) | 2-15830021 | cg09520554 | (cancer vs cancer) (cancer vs cancer) |
| 10-31901490 | cg10431652 | (normal vs normal) | 2-207987538 | cg05861291 | (cancer vs cancer) |
| 10-94594240 | cg13654884 | (normal vs normal) | 2-232113244 | cg27273946 | (normal vs normal) (cancer vs cancer) (cancer vs cancer) (cancer+normal vs cancer+normal) |
| 10-97552565 | cg03122427 | (normal vs normal) | 2-234373030 | cg08354835 | (cancer vs cancer) (cancer vs cancer) (cancer+normal vs cancer+normal) |
| 11-12128203 | cg14816825 | (normal vs normal) | 2-234394478 | cg16719865 | (cancer vs cancer) (cancer vs cancer) |

| | | | | | (cancer+normal vs cancer+normal) |
|---|---|---|---|---|---|
| 11-44243314 | cg05495611 | (normal vs normal) (cancer+normal vs cancer+normal) | 2-234394541 | cg17212304 | (cancer vs cancer) (cancer vs cancer) (cancer+normal vs cancer+normal) |
| 11-75130069 | cg19145320 | (normal vs normal) | 2-234394645 | cg25923162 | (cancer vs cancer) (cancer vs cancer) (cancer+normal vs cancer+normal) |
| 11-88032774 | cg12710531 | (normal vs normal) (cancer+normal vs cancer+normal) | 2-236672749 | cg01206017 | (cancer vs cancer) (cancer vs cancer) (cancer+normal vs cancer+normal) |
| 12-116864616 | cg19641325 | (normal vs normal) | 2-239970075 | cg26126749 | (cancer vs cancer) (cancer vs cancer) (cancer+normal vs cancer+normal) |
| 12-11912232 | cg06225767 | (normal vs normal) | 2-239970152 | cg08781549 | (cancer vs cancer) (cancer vs cancer) (cancer+normal vs cancer+normal) |
| 12-120688060 | cg07419011 | (normal vs normal) | 2-37883934 | cg13286582 | (cancer vs cancer) (cancer+normal vs cancer+normal) |
| 12-124912049 | cg11311053 | (normal vs normal) | 2-45557351 | cg12804104 | (cancer vs cancer) (cancer vs cancer) (cancer+normal vs cancer+normal) |
| 12-28283812 | cg15026265 | (normal vs normal) | 2-61352525 | cg13409174 | (normal vs normal) (cancer vs cancer) |
| 12-3280014 | cg07303577 | (normal vs normal) | 3-124885459 | cg17997976 | (cancer vs cancer) (cancer vs cancer) (cancer+normal vs cancer+normal) |
| 12-9268379 | cg12058490 | (normal vs normal) | 3-132077190 | cg25769412 | (cancer vs cancer) (cancer+normal vs cancer+normal) |
| 13-114065897 | cg26081974 | (normal vs normal) | 3-187960622 | cg02579706 | (cancer vs cancer) (cancer vs cancer) (cancer+normal vs cancer+normal) |
| 13-41114929 | cg19429300 | (normal vs normal) | 3-72537392 | cg15491385 | (cancer vs cancer) (cancer vs cancer) (cancer+normal vs cancer+normal) |
| 14-40142517 | cg11835955 | (normal vs normal) | 4-113210708 | cg15345393 | (cancer vs cancer) |
| 14-51854261 | cg00750630 | (normal vs normal) (cancer+normal vs cancer+normal) | 4-140829046 | cg20054111 | (cancer vs cancer) |
| 14-54815802 | cg02270243 | (normal vs normal) | 5-131408149 | cg16465129 | (cancer vs cancer) (cancer vs cancer) (cancer+normal vs cancer+normal) |
| 14-59198210 | cg02069592 | (normal vs normal) | 5-141722174 | cg02084814 | (cancer vs cancer) (cancer vs cancer) (cancer+normal vs cancer+normal) |
| 14-64912417 | cg14412134 | (normal vs normal) | 5-143026910 | cg23194776 | (cancer vs cancer) (cancer vs cancer) |

| | | | | | (cancer+normal vs cancer+normal) |
|---|---|---|---|---|---|
| 14-75478802 | cg18459040 | (normal vs normal) | 5-147781634 | cg24787443 | (cancer vs cancer) |
| 15-29928082 | cg03147324 | (normal vs normal) | 5-1546960 | cg02165151 | (cancer vs cancer) (cancer vs cancer) (cancer+normal vs cancer+normal) |
| 15-31355304 | cg20640374 | (normal vs normal) (cancer+normal vs cancer+normal) | 5-1596053 | cg02475547 | (cancer vs cancer) (cancer vs cancer) |
| 15-31355362 | cg24957518 | (normal vs normal) | 6-108615613 | cg17969853 | (cancer vs cancer) (cancer+normal vs cancer+normal) |
| 15-33143407 | cg14161369 | (normal vs normal) | 6-136436154 | cg10872815 | (cancer vs cancer) |
| 15-68719924 | cg08872353 | (normal vs normal) | 6-143575123 | cg08612271 | (cancer vs cancer) (cancer vs cancer) (cancer+normal vs cancer+normal) |
| 15-70546706 | cg12737588 | (normal vs normal) | 6-170047483 | cg11412527 | (cancer vs cancer) (cancer vs cancer) (cancer+normal vs cancer+normal) |
| 16-11271052 | cg08194933 | (normal vs normal) | 6-25726912 | cg07077277 | (cancer vs cancer) (cancer vs cancer) (cancer+normal vs cancer+normal) |
| 16-1583899 | cg07341220 | (normal vs normal) (cancer+normal vs cancer+normal) | 6-28875356 | cg14059339 | (cancer vs cancer) (cancer vs cancer) (cancer+normal vs cancer+normal) |
| 16-1583984 | cg00463982 | (normal vs normal) (cancer+normal vs cancer+normal) | 6-28875370 | cg08279189 | (cancer vs cancer) (cancer vs cancer) (cancer+normal vs cancer+normal) |
| 16-89647782 | cg10225951 | (normal vs normal) | 6-30900678 | cg10006979 | (cancer vs cancer) (cancer vs cancer) (cancer+normal vs cancer+normal) |
| 17-17449166 | cg26177311 | (normal vs normal) (cancer+normal vs cancer+normal) | 6-39072458 | cg21645089 | (cancer vs cancer) (cancer vs cancer) (cancer+normal vs cancer+normal) |
| 17-35853648 | cg20695611 | (normal vs normal) | 6-46890220 | cg03558010 | (cancer vs cancer) (cancer vs cancer) (cancer+normal vs cancer+normal) |
| 17-3623472 | cg02225720 | (normal vs normal) | 6-88851522 | cg20510988 | (cancer vs cancer) |
| 17-76876239 | cg00863893 | (normal vs normal) | 7-105254235 | cg26932690 | (cancer vs cancer) (cancer vs cancer) |
| 17-78892850 | cg21000762 | (normal vs normal) (cancer+normal vs cancer+normal) | 7-116779435 | cg26619790 | (cancer vs cancer) (cancer vs cancer) (cancer+normal vs cancer+normal) |
| 17-821621 | cg02459042 | (normal vs normal) (cancer+normal vs cancer+normal) | 7-128767280 | cg08281469 | (cancer vs cancer) (cancer vs cancer) (cancer+normal vs cancer+normal) |
| 18- | cg26367197 | (normal vs normal) | 7- | cg0433475 | (cancer vs cancer) |

| | | |
|---|---|---|
| 76307273 | | |
| 19-3671118 | cg10591771 | (normal vs normal) |
| 2-101434611 | cg02049833 | (normal vs normal) |
| 2-109781768 | cg01741688 | (normal vs normal) |
| 2-135203925 | cg15584989 | (normal vs normal) (cancer+normal vs cancer+normal) |
| 2-153415027 | cg23482132 | (normal vs normal) |
| 2-177003996 | cg11375458 | (normal vs normal) |
| 2-177004015 | cg18516557 | (normal vs normal) |
| 2-177004975 | cg21818470 | (normal vs normal) |
| 2-238292904 | cg25424742 | (normal vs normal) |
| 2-238647755 | cg17032848 | (normal vs normal) (cancer+normal vs cancer+normal) |
| 2-238647857 | cg13184448 | (normal vs normal) |
| 2-238777587 | cg04011338 | (normal vs normal) |
| 2-239544973 | cg10160614 | (normal vs normal) |
| 2-240230892 | cg19449565 | (normal vs normal) (cancer vs cancer) (cancer+normal vs cancer+normal) |
| 2-240697449 | cg07506560 | (normal vs normal) |
| 2-38397399 | cg02053818 | (normal vs normal) |
| 2-55393977 | cg19384241 | (normal vs normal) (cancer+normal vs cancer+normal) |
| 2-864706 | cg08242232 | (normal vs normal) |
| 20-57463991 | cg09885502 | (normal vs normal) (cancer+normal vs cancer+normal) |
| 21-46629219 | cg04233620 | (normal vs normal) |

| | | |
|---|---|---|
| 129778936 | 1 | (cancer vs cancer) (cancer+normal vs cancer+normal) |
| 7-143204891 | cg27377610 | (cancer vs cancer) |
| 7-1615334 | cg13724248 | (cancer vs cancer) (cancer vs cancer) |
| 7-2473529 | cg17907628 | (cancer vs cancer) (cancer vs cancer) (cancer+normal vs cancer+normal) |
| 7-2473859 | cg18259487 | (cancer vs cancer) (cancer vs cancer) (cancer+normal vs cancer+normal) |
| 7-5262452 | cg08310400 | (cancer vs cancer) (cancer vs cancer) (cancer+normal vs cancer+normal) |
| 7-5272275 | cg20099458 | (cancer vs cancer) (cancer+normal vs cancer+normal) |
| 7-77126994 | cg02166725 | (normal vs normal) (cancer vs cancer) (cancer vs cancer) |
| 7-98996656 | cg19420101 | (cancer vs cancer) (cancer vs cancer) (cancer+normal vs cancer+normal) |
| 8-12985953 | cg03068843 | (cancer vs cancer) |
| 8-144573510 | cg14650228 | (cancer vs cancer) |
| 8-28625366 | cg26333660 | (cancer vs cancer) (cancer+normal vs cancer+normal) |
| 8-71228213 | cg06083412 | (cancer vs cancer) |
| 9-14690531 | cg21185283 | (cancer vs cancer) (cancer vs cancer) (cancer+normal vs cancer+normal) |
| 9-34370894 | cg13746854 | (cancer vs cancer) |
| 9-99450036 | cg21174344 | (cancer vs cancer) (cancer vs cancer) |
| **LuPaACaG (cancer vs cancer)** | | |
| **Status - Hyper** | | |
| 1-13840381 | cg13788258 | (cancer vs cancer) (cancer vs cancer) |
| 1-13840480 | cg23071120 | (cancer vs cancer) (cancer vs cancer) |
| 1-13840494 | cg26536189 | (cancer vs cancer) (cancer vs cancer) |

| | | | | | |
|---|---|---|---|---|---|
| 3-107381326 | cg23733260 | (normal vs normal) (cancer vs cancer) (cancer+normal vs cancer+normal) | 1-13840612 | cg01144933 | (cancer vs cancer) (cancer vs cancer) |
| 3-123417080 | cg00574513 | (normal vs normal) | 1-167013332 | cg25755191 | (cancer vs cancer) |
| 3-139353058 | cg15093769 | (normal vs normal) (cancer+normal vs cancer+normal) | 1-178062853 | cg04894642 | (cancer vs cancer) |
| 4-119948542 | cg13042918 | (normal vs normal) (cancer+normal vs cancer+normal) | 1-182584341 | cg00153856 | (cancer vs cancer) (cancer vs cancer) (cancer+normal vs cancer+normal) |
| 4-37910273 | cg08936645 | (normal vs normal) | 1-23668792 | cg08718097 | (cancer vs cancer) (cancer vs cancer) (normal vs normal) (cancer+normal vs cancer+normal) |
| 5-131599890 | cg02484352 | (normal vs normal) | 10-133558786 | cg04492858 | (cancer vs cancer) (cancer vs cancer) |
| 5-135315386 | cg15141378 | (normal vs normal) | 10-133558971 | cg23460707 | (cancer vs cancer) (cancer vs cancer) |
| 5-15595186 | cg15540341 | (normal vs normal) | 10-8092036 | cg18072629 | (cancer vs cancer) (cancer vs cancer) (cancer+normal vs cancer+normal) |
| 5-171190008 | cg16525277 | (normal vs normal) | 10-8095288 | cg05721515 | (cancer vs cancer) (cancer vs cancer) (cancer+normal vs cancer+normal) |
| 5-172306136 | cg21204530 | (normal vs normal) | 10-8096305 | cg09728012 | (cancer vs cancer) (cancer vs cancer) |
| 5-64334489 | cg11190987 | (normal vs normal) (cancer+normal vs cancer+normal) | 10-8096311 | cg24647276 | (cancer vs cancer) (cancer vs cancer) |
| 6-112081538 | cg08061598 | (normal vs normal) (cancer vs cancer) (cancer+normal vs cancer+normal) | 10-8096370 | cg15187550 | (cancer vs cancer) (cancer vs cancer) (cancer+normal vs cancer+normal) |
| 6-148823251 | cg16640450 | (normal vs normal) | 10-8096372 | cg15852223 | (cancer vs cancer) |
| 6-170065938 | cg21796935 | (normal vs normal) (cancer+normal vs cancer+normal) | 10-8096386 | cg19894747 | (cancer vs cancer) |
| 6-34493129 | cg04941418 | (normal vs normal) | 10-8374293 | cg08860607 | (cancer vs cancer) |
| 6-3760116 | cg00380780 | (normal vs normal) | 11-113345325 | cg10777103 | (cancer vs cancer) |
| 6-52377740 | cg00593404 | (normal vs normal) (cancer+normal vs cancer+normal) | 11-123525237 | cg00648476 | (cancer vs cancer) (cancer+normal vs cancer+normal) |
| 7-115862891 | cg02561103 | (normal vs normal) | 11-22359333 | cg06517489 | (cancer vs cancer) |
| 7-157102769 | cg23590389 | (normal vs normal) | 11-22359345 | cg12778476 | (cancer vs cancer) |
| 7-48124921 | cg14241836 | (normal vs normal) | 11-279950 | cg02471658 | (cancer vs cancer) (cancer vs cancer) (cancer+normal vs cancer+normal) |
| 7-55553545 | cg16182302 | (normal vs normal) | 11-31831591 | cg16822387 | (cancer vs cancer) |

| | | | | | |
|---|---|---|---|---|---|
| 7-73264533 | cg16212453 | (normal vs normal) | 11-31832353 | cg0584003 1 | (cancer vs cancer) (cancer vs cancer) (cancer+normal vs cancer+normal) |
| 7-76129360 | cg15798862 | (normal vs normal) | 12-107487800 | cg1114688 4 | (cancer vs cancer) |
| 7-91322751 | cg22709100 | (normal vs normal) | 12-115120789 | cg1934637 1 | (cancer vs cancer) |
| 8-12988516 | cg10941185 | (normal vs normal) | 12-115120847 | cg2763031 1 | (cancer vs cancer) (cancer vs cancer) |
| 8-1460569 | cg15938529 | (normal vs normal) | 12-115121016 | cg1971303 8 | (cancer vs cancer) (cancer vs cancer) |
| 8-22133076 | cg07298985 | (normal vs normal) | 12-115121047 | cg1930361 9 | (cancer vs cancer) |
| **CHOL (cancer vs cancer)** | | | 12-115121215 | cg1033002 4 | (cancer vs cancer) |
| **Status - Hyper** | | | 12-115121307 | cg1857577 0 | (cancer vs cancer) |
| 1-100111949 | cg02080641 | (cancer vs cancer) | 12-115121354 | cg1253005 0 | (cancer vs cancer) |
| 1-113497932 | cg16251079 | (cancer vs cancer) | 12-118814505 | cg2152000 2 | (cancer vs cancer) |
| 1-113497999 | cg07423363 | (cancer vs cancer) | 12-19283425 | cg1067565 9 | (cancer vs cancer) |
| 1-113498106 | cg12551029 | (cancer vs cancer) | 12-3714598 | cg2041622 2 | (cancer vs cancer) |
| 1-113498174 | cg17020695 | (cancer vs cancer) | 12-4274034 | cg0680796 6 | (cancer vs cancer) (cancer vs cancer) (normal vs normal) (cancer+normal vs cancer+normal) |
| 1-113498193 | cg00058299 | (cancer vs cancer) | 13-100635002 | cg2462327 1 | (cancer vs cancer) |
| 1-161111090 | cg23981150 | (cancer vs cancer) (cancer+normal vs cancer+normal) | 13-32605406 | cg2221925 4 | (cancer vs cancer) (cancer vs cancer) (normal vs normal) (cancer+normal vs cancer+normal) |
| 1-198651141 | cg15626828 | (cancer vs cancer) | 13-32605611 | cg1694165 6 | (cancer vs cancer) (cancer vs cancer) (normal vs normal) (cancer+normal vs cancer+normal) |
| 1-198904195 | cg09517106 | (cancer vs cancer) (cancer+normal vs cancer+normal) | 14-105714589 | cg2303481 8 | (cancer vs cancer) (cancer vs cancer) (normal vs normal) (cancer+normal vs cancer+normal) |
| 1-207818036 | cg13234569 | (cancer vs cancer) | 14-105714833 | cg1547436 7 | (cancer vs cancer) (cancer vs cancer) (normal vs normal) (cancer+normal vs cancer+normal) |
| 1-82136224 | cg08110488 | (cancer vs cancer) (cancer+normal vs cancer+normal) | 14-105714843 | cg1656154 3 | (cancer vs cancer) (cancer vs cancer) (normal vs normal) (cancer+normal vs cancer+normal) |
| 10-102107666 | cg06400428 | (cancer vs cancer) | 14-105715025 | cg0123756 5 | (cancer vs cancer) (cancer vs cancer) (normal vs normal) |

| | | |
|---|---|---|
| 10-102107676 | cg24503796 | (cancer vs cancer) |
| 10-102502104 | cg24127310 | (cancer vs cancer) |
| 10-72218642 | cg02809409 | (cancer vs cancer) |
| 10-7859768 | cg09994553 | (cancer vs cancer) |
| 10-94459528 | cg08151063 | (cancer vs cancer) |
| 11-134336336 | cg14308313 | (cancer vs cancer) |
| 11-2154113 | cg13165070 | (cancer vs cancer) (cancer+normal vs cancer+normal) |
| 11-64993076 | cg19323951 | (cancer vs cancer) |
| 11-64993239 | cg02675946 | (cancer vs cancer) (cancer+normal vs cancer+normal) |
| 11-64993281 | cg27428304 | (cancer vs cancer) |
| 11-64993335 | cg23930334 | (cancer vs cancer) (normal vs normal) |
| 12-102872937 | cg11612617 | (cancer vs cancer) |
| 12-108733253 | cg25832824 | (cancer vs cancer) |
| 12-108733261 | cg03612522 | (cancer vs cancer) (cancer+normal vs cancer+normal) |
| 12-108733275 | cg12142354 | (cancer vs cancer) (cancer+normal vs cancer+normal) |
| 12-108733308 | cg15448445 | (cancer vs cancer) (cancer+normal vs cancer+normal) |
| 12-2041707 | cg03131366 | (cancer vs cancer) |
| 12-4381777 | cg25425078 | (cancer vs cancer) (cancer+normal vs cancer+normal) |
| 12-4381788 | cg15993083 | (cancer vs cancer) |
| 12-66216880 | cg05481991 | (cancer vs cancer) |
| 13-114238565 | cg15120341 | (cancer vs cancer) (cancer+normal vs cancer+normal) |
| 13-47471052 | cg06476131 | (cancer vs cancer) (cancer+normal vs |

| | | |
|---|---|---|
| | | (cancer+normal vs cancer+normal) |
| 14-22458634 | cg22816294 | (cancer vs cancer) |
| 14-37131555 | cg22968936 | (cancer vs cancer) (cancer vs cancer) |
| 14-99737609 | cg23347987 | (cancer vs cancer) |
| 15-71185242 | cg02260353 | (cancer vs cancer) |
| 15-74532450 | cg02430430 | (cancer vs cancer) (cancer vs cancer) |
| 15-98447618 | cg22456000 | (cancer vs cancer) |
| 16-1202819 | cg00609966 | (cancer vs cancer) (cancer vs cancer) |
| 16-30798048 | cg02463478 | (cancer vs cancer) |
| 16-86231948 | cg07989221 | (cancer vs cancer) (cancer vs cancer) (normal vs normal) (cancer+normal vs cancer+normal) |
| 16-89723364 | cg05441039 | (cancer vs cancer) (cancer+normal vs cancer+normal) |
| 17-18266135 | cg26763362 | (cancer vs cancer) (cancer vs cancer) |
| 17-26685074 | cg08076044 | (cancer vs cancer) (cancer+normal vs cancer+normal) |
| 19-49589626 | cg21587372 | (cancer vs cancer) |
| 19-51222459 | cg00741774 | (cancer vs cancer) |
| 19-55677414 | cg12018403 | (cancer vs cancer) |
| 19-55728628 | cg01434523 | (cancer vs cancer) |
| 2-102804405 | cg01376829 | (cancer vs cancer) (cancer vs cancer) (cancer+normal vs cancer+normal) |
| 2-102804576 | cg00674896 | (cancer vs cancer) (cancer vs cancer) (cancer+normal vs cancer+normal) |
| 2-200324879 | cg26792080 | (cancer vs cancer) (cancer+normal vs cancer+normal) |
| 2-215275783 | cg21223159 | (cancer vs cancer) |
| 2-215275838 | cg15125763 | (cancer vs cancer) (cancer vs cancer) |
| 2-8816914 | cg16345795 | (cancer vs cancer) |

| | | cancer+normal) | | | |
|---|---|---|---|---|---|
| 13-47471090 | cg16188532 | (cancer vs cancer) | 2-8816922 | cg12515366 | (cancer vs cancer) |
| 13-47471169 | cg09361691 | (cancer vs cancer) (cancer+normal vs cancer+normal) | 20-10200383 | cg16786007 | (cancer vs cancer) |
| 14-88789549 | cg08054244 | (cancer vs cancer) (cancer+normal vs cancer+normal) | 20-29994089 | cg23204384 | (cancer vs cancer) |
| 15-60298043 | cg04861263 | (cancer vs cancer) | 20-62793452 | cg17526301 | (cancer vs cancer) |
| 15-76136875 | cg04672495 | (cancer vs cancer) | 3-132843565 | cg00052578 | (cancer vs cancer) (cancer vs cancer) (cancer+normal vs cancer+normal) |
| 15-99193993 | cg18202336 | (cancer vs cancer) | 3-64008330 | cg15316508 | (cancer vs cancer) |
| 15-99194172 | cg01631855 | (cancer vs cancer) | 4-4858598 | cg05600350 | (cancer vs cancer) (cancer vs cancer) |
| 15-99194474 | cg17297216 | (cancer vs cancer) | 4-4861618 | cg00267751 | (cancer vs cancer) |
| 18-24130367 | cg21739658 | (cancer vs cancer) | 4-4861820 | cg11930592 | (cancer vs cancer) |
| 18-52989085 | cg03284649 | (cancer vs cancer) | 5-102898478 | cg13665998 | (cancer vs cancer) |
| 19-39687568 | cg24571673 | (cancer vs cancer) | 5-175298655 | cg16025094 | (cancer vs cancer) (cancer vs cancer) |
| 19-52646265 | cg24449629 | (cancer vs cancer) (cancer+normal vs cancer+normal) | 5-175298869 | cg13564889 | (cancer vs cancer) (cancer vs cancer) (cancer+normal vs cancer+normal) |
| 2-119077241 | cg16205490 | (cancer vs cancer) | 6-100064885 | cg01156070 | (cancer vs cancer) (cancer+normal vs cancer+normal) |
| 2-136743460 | cg18110444 | (cancer vs cancer) (cancer+normal vs cancer+normal) | 6-152623479 | cg12023625 | (cancer vs cancer) (cancer vs cancer) (normal vs normal) (cancer+normal vs cancer+normal) |
| 2-172950626 | cg27378835 | (cancer vs cancer) | 6-165341320 | cg23309836 | (cancer vs cancer) (cancer vs cancer) (cancer+normal vs cancer+normal) |
| 2-71504771 | cg04737950 | (cancer vs cancer) (cancer+normal vs cancer+normal) | 6-19804855 | cg02682789 | (cancer vs cancer) |
| 3-120627406 | cg05293820 | (cancer vs cancer) (normal vs normal) (cancer+normal vs cancer+normal) | 6-26204773 | cg17123534 | (cancer vs cancer) |
| 3-157824209 | cg14961949 | (cancer vs cancer) | 6-26204823 | cg16706631 | (cancer vs cancer) |
| 3-157824217 | cg17191178 | (cancer vs cancer) | 6-26216541 | cg27133864 | (cancer vs cancer) (cancer vs cancer) |
| 3-168864101 | cg06238409 | (cancer vs cancer) | 6-26224070 | cg03854865 | (cancer vs cancer) (cancer vs cancer) |
| 3-181431065 | cg25444208 | (cancer vs cancer) | 6-26224392 | cg16070018 | (cancer vs cancer) (cancer vs cancer) |
| 3-181431306 | cg03827626 | (cancer vs cancer) | 6-26224668 | cg24440941 | (cancer vs cancer) (cancer vs cancer) (cancer+normal vs |

| | | | | | |
|---|---|---|---|---|---|
| | | | | | (cancer+normal) |
| 4-147443095 | cg20910303 | (cancer vs cancer) (cancer+normal vs cancer+normal) | 7-139168481 | cg24956250 | (cancer vs cancer) (cancer vs cancer) (cancer+normal vs cancer+normal) |
| 4-43900946 | cg14094460 | (cancer vs cancer) (cancer+normal vs cancer+normal) | 7-156812104 | cg09595202 | (cancer vs cancer) (cancer vs cancer) (cancer+normal vs cancer+normal) |
| 4-43901084 | cg05899741 | (cancer vs cancer) (cancer+normal vs cancer+normal) | 7-156814845 | cg02570079 | (cancer vs cancer) (cancer vs cancer) (normal vs normal) (cancer+normal vs cancer+normal) |
| 4-89378460 | cg14660125 | (cancer vs cancer) | 7-25894793 | cg02067688 | (cancer vs cancer) (cancer vs cancer) |
| 4-9534313 | cg15442990 | (cancer vs cancer) | 7-75831678 | cg13767245 | (cancer vs cancer) (cancer+normal vs cancer+normal) |
| 5-111115809 | cg11657018 | (cancer vs cancer) (cancer+normal vs cancer+normal) | 7-83824255 | cg16346212 | (cancer vs cancer) |
| 5-115387951 | cg23311108 | (cancer vs cancer) (normal vs normal) (cancer+normal vs cancer+normal) | 7-99678668 | cg01043524 | (cancer vs cancer) |
| 5-115387984 | cg14690181 | (cancer vs cancer) (cancer+normal vs cancer+normal) | 8-135843385 | cg22904815 | (cancer vs cancer) (cancer+normal vs cancer+normal) |
| 5-151304409 | cg14319409 | (cancer vs cancer) | 8-145181395 | cg15640503 | (cancer vs cancer) |
| 5-162930610 | cg03059048 | (cancer vs cancer) | 8-21987861 | cg26045434 | (cancer vs cancer) (cancer vs cancer) |
| 5-162931471 | cg19597031 | (cancer vs cancer) | 8-57906837 | cg03732295 | (cancer vs cancer) |
| 5-162931633 | cg16417447 | (cancer vs cancer) | 9-89561175 | cg13848566 | (cancer vs cancer) (cancer vs cancer) |
| 5-173345670 | cg05947699 | (cancer vs cancer) | **Status - Hypo** | | |
| 5-174150712 | cg26092471 | (cancer vs cancer) | 1-11561908 | cg13863764 | (cancer vs cancer) (cancer vs cancer) |
| 5-36607377 | cg25010361 | (cancer vs cancer) | 1-1374310 | cg13897675 | (cancer vs cancer) (cancer vs cancer) |
| 5-36607390 | cg21245981 | (cancer vs cancer) | 1-1374669 | cg19639479 | (cancer vs cancer) (cancer vs cancer) |
| 6-151561447 | cg10414242 | (cancer vs cancer) | 1-203098067 | cg24408776 | (cancer vs cancer) (cancer vs cancer) (cancer+normal vs cancer+normal) |
| 6-26234819 | cg24036126 | (cancer vs cancer) | 1-223903862 | cg10100437 | (cancer vs cancer) |
| 6-26235224 | cg24855943 | (cancer vs cancer) | 1-230250326 | cg09847368 | (cancer vs cancer) |
| 6-26273182 | cg18465515 | (cancer vs cancer) | 1-230250356 | cg00566187 | (cancer vs cancer) |
| 6-27775877 | cg06926499 | (cancer vs cancer) (cancer+normal vs cancer+normal) | 1-44819838 | cg00717995 | (cancer vs cancer) (cancer+normal vs cancer+normal) |
| 6-27777858 | cg18943383 | (cancer vs cancer) (cancer+normal vs cancer+normal) | 1-65299902 | cg16250812 | (cancer vs cancer) |

171

| | | | | | |
|---|---|---|---|---|---|
| 6-27778076 | cg15410276 | (cancer vs cancer) | 10-131217895 | cg08398691 | (cancer vs cancer) |
| 6-27778236 | cg07178031 | (cancer vs cancer) | 10-134499505 | cg06139099 | (cancer vs cancer) (cancer vs cancer) |
| 6-27782176 | cg20478264 | (cancer vs cancer) | 10-134499528 | cg00458454 | (cancer vs cancer) (cancer vs cancer) (cancer+normal vs cancer+normal) |
| 6-27782484 | cg16347724 | (cancer vs cancer) (cancer+normal vs cancer+normal) | 10-134499734 | cg13441142 | (normal vs normal) (cancer vs cancer) (cancer vs cancer) (cancer+normal vs cancer+normal) |
| 6-27782628 | cg05444312 | (cancer vs cancer) (cancer+normal vs cancer+normal) | 10-134499752 | cg02541125 | (cancer vs cancer) (cancer vs cancer) (cancer+normal vs cancer+normal) |
| 6-27834966 | cg00667898 | (cancer vs cancer) | 10-294353 | cg18570800 | (cancer vs cancer) (cancer vs cancer) |
| 6-27835460 | cg18798248 | (cancer vs cancer) | 10-90644018 | cg07510282 | (cancer vs cancer) (cancer vs cancer) |
| 6-27840068 | cg12181621 | (cancer vs cancer) | 11-47372949 | cg06168166 | (cancer vs cancer) (cancer vs cancer) (cancer+normal vs cancer+normal) |
| 6-27840105 | cg05511483 | (cancer vs cancer) (cancer+normal vs cancer+normal) | 11-47372960 | cg10354134 | (cancer vs cancer) (cancer vs cancer) (cancer+normal vs cancer+normal) |
| 6-31237013 | cg09556042 | (cancer vs cancer) | 11-67220318 | cg27265307 | (cancer vs cancer) |
| 6-32522683 | cg25140213 | (cancer vs cancer) (normal vs normal) (cancer+normal vs cancer+normal) | 11-78614522 | cg13228701 | (cancer vs cancer) |
| 6-32547019 | cg07984380 | (cancer vs cancer) (cancer+normal vs cancer+normal) | 11-86142478 | cg18725375 | (cancer vs cancer) |
| 6-32549283 | cg12867728 | (cancer vs cancer) (normal vs normal) | 11-86142587 | cg15146462 | (cancer vs cancer) |
| 7-101398152 | cg14823389 | (cancer vs cancer) (cancer+normal vs cancer+normal) | 12-102216984 | cg17214456 | (cancer vs cancer) (cancer+normal vs cancer+normal) |
| 7-1250182 | cg07919840 | (cancer vs cancer) (cancer+normal vs cancer+normal) | 12-124913717 | cg05395187 | (cancer vs cancer) |
| 7-155264170 | cg11382477 | (cancer vs cancer) | 12-47583278 | cg24085426 | (cancer vs cancer) |
| 7-20833585 | cg24954668 | (cancer vs cancer) | 12-95711510 | cg04876905 | (cancer vs cancer) (cancer vs cancer) |
| 7-27136424 | cg09420439 | (cancer vs cancer) | 12-95941571 | cg03162823 | (cancer vs cancer) |
| 8-108510096 | cg03245734 | (cancer vs cancer) | 13-101167901 | cg13731800 | (cancer vs cancer) (cancer vs cancer) |
| 8-122653619 | cg19275050 | (cancer vs cancer) | 13-50702675 | cg15389749 | (cancer vs cancer) |
| 9-123639954 | cg13793166 | (cancer vs cancer) (cancer+normal vs cancer+normal) | 14-45365651 | cg01523474 | (normal vs normal) (cancer vs cancer) (cancer vs cancer) (cancer+normal vs cancer+normal) |

| | | | | | |
|---|---|---|---|---|---|
| 9-99962350 | cg00429618 | (cancer vs cancer) (cancer+normal vs cancer+normal) | 14-68040530 | cg2110415 5 | (cancer vs cancer) |
| **Status - Hypo** | | | 14-75747332 | cg2503476 6 | (cancer vs cancer) |
| 1-114414802 | cg26035817 | (cancer vs cancer) (cancer+normal vs cancer+normal) | 14-91720696 | cg0303488 0 | (cancer vs cancer) |
| 1-117489843 | cg23093116 | (cancer vs cancer) (cancer+normal vs cancer+normal) | 14-91770145 | cg2313860 8 | (cancer vs cancer) (cancer vs cancer) |
| 1-119520934 | cg15553612 | (cancer vs cancer) (cancer+normal vs cancer+normal) | 14-91770229 | cg1946560 5 | (cancer vs cancer) (cancer vs cancer) |
| 1-150693299 | cg09059988 | (cancer vs cancer) | 15-62516405 | cg0345531 6 | (cancer vs cancer) |
| 1-202559755 | cg11143857 | (cancer vs cancer) (cancer+normal vs cancer+normal) | 15-71092216 | cg2595426 9 | (cancer vs cancer) (cancer vs cancer) |
| 1-233817629 | cg10175859 | (normal vs normal) (cancer vs cancer) (cancer+normal vs cancer+normal) | 16-29857167 | cg0368115 0 | (cancer vs cancer) (cancer+normal vs cancer+normal) |
| 1-23418405 | cg15897613 | (cancer vs cancer) (cancer+normal vs cancer+normal) | 17-36997627 | cg1976025 0 | (cancer vs cancer) |
| 1-26000291 | cg08460387 | (cancer vs cancer) (cancer+normal vs cancer+normal) | 17-73360252 | cg1328206 8 | (cancer vs cancer) (cancer vs cancer) |
| 1-40346530 | cg10530613 | (cancer vs cancer) (cancer+normal vs cancer+normal) | 17-79869997 | cg2177014 5 | (cancer vs cancer) |
| 1-67266410 | cg11521979 | (cancer vs cancer) (cancer+normal vs cancer+normal) | 17-80848209 | cg0788519 1 | (cancer vs cancer) |
| 1-6855140 | cg04874074 | (normal vs normal) (cancer vs cancer) (cancer+normal vs cancer+normal) | 17-835927 | cg1638105 9 | (cancer vs cancer) |
| 10-102671221 | cg15448683 | (cancer vs cancer) (cancer+normal vs cancer+normal) | 19-16437789 | cg0266824 8 | (cancer vs cancer) |
| 10-119101310 | cg17182036 | (cancer vs cancer) (cancer+normal vs cancer+normal) | 19-51607432 | cg0887394 0 | (cancer vs cancer) (cancer vs cancer) |
| 10-123353500 | cg14856220 | (cancer vs cancer) | 19-6742907 | cg1833173 2 | (cancer vs cancer) |
| 10-134926810 | cg27405177 | (normal vs normal) (cancer vs cancer) | 2-18736132 | cg0593739 2 | (normal vs normal) (cancer vs cancer) (cancer vs cancer) (cancer+normal vs cancer+normal) |
| 10-135097477 | cg27633010 | (cancer vs cancer) (cancer+normal vs cancer+normal) | 2-217448253 | cg1990729 3 | (cancer vs cancer) |
| 10-5542941 | cg26552733 | (cancer vs cancer) (cancer+normal vs cancer+normal) | 2-241290895 | cg0400387 1 | (cancer vs cancer) (cancer vs cancer) (cancer+normal vs cancer+normal) |
| 10-76801843 | cg23077498 | (cancer vs cancer) (cancer+normal vs | 2-7164527 | cg0818297 5 | (normal vs normal) (cancer vs cancer) |

| | | | | | |
|---|---|---|---|---|---|
| | | cancer+normal) | | | (cancer vs cancer) (cancer+normal vs cancer+normal) |
| 10-92663320 | cg14882291 | (cancer vs cancer) | 2-7164586 | cg20995753 | (normal vs normal) (cancer vs cancer) (cancer vs cancer) (cancer+normal vs cancer+normal) |
| 10-93872745 | cg24984532 | (cancer vs cancer) (cancer+normal vs cancer+normal) | 2-9388337 | cg00230766 | (cancer vs cancer) (cancer vs cancer) (cancer+normal vs cancer+normal) |
| 11-124539453 | cg18743034 | (normal vs normal) (cancer vs cancer) (cancer+normal vs cancer+normal) | 21-43241683 | cg07183691 | (cancer vs cancer) |
| 11-126072352 | cg26351663 | (cancer vs cancer) (cancer+normal vs cancer+normal) | 22-29441760 | cg18661550 | (cancer vs cancer) (cancer vs cancer) (cancer+normal vs cancer+normal) |
| 11-130763724 | cg00458607 | (cancer vs cancer) (cancer+normal vs cancer+normal) | 3-109115356 | cg08277100 | (cancer vs cancer) |
| 11-134118834 | cg22756211 | (cancer vs cancer) (cancer+normal vs cancer+normal) | 3-118864895 | cg20675391 | (cancer vs cancer) |
| 11-15959637 | cg12489547 | (cancer vs cancer) | 3-128079984 | cg05801066 | (cancer vs cancer) (cancer vs cancer) (cancer+normal vs cancer+normal) |
| 11-232013 | cg14777131 | (cancer vs cancer) | 3-141161580 | cg06294561 | (cancer vs cancer) (cancer vs cancer) (cancer+normal vs cancer+normal) |
| 11-2985302 | cg13363640 | (cancer vs cancer) (cancer+normal vs cancer+normal) | 3-149126842 | cg11431723 | (cancer vs cancer) (cancer+normal vs cancer+normal) |
| 11-63883947 | cg24431193 | (cancer vs cancer) (cancer+normal vs cancer+normal) | 3-194868843 | cg19760965 | (cancer vs cancer) |
| 11-70281584 | cg27243121 | (cancer vs cancer) | 3-32408427 | cg24924051 | (cancer vs cancer) (cancer vs cancer) |
| 11-75115584 | cg12506582 | (cancer vs cancer) (cancer+normal vs cancer+normal) | 3-55508686 | cg06311863 | (cancer vs cancer) (cancer vs cancer) |
| 11-9448817 | cg11512797 | (cancer vs cancer) | 4-1646179 | cg27571057 | (cancer vs cancer) (cancer vs cancer) (cancer+normal vs cancer+normal) |
| 12-11005841 | cg26459499 | (cancer vs cancer) (cancer+normal vs cancer+normal) | 4-38732661 | cg26300517 | (cancer vs cancer) |
| 12-46753241 | cg23334507 | (cancer vs cancer) (cancer+normal vs cancer+normal) | 4-8386188 | cg08206079 | (cancer vs cancer) (cancer+normal vs cancer+normal) |
| 12-48163063 | cg10052368 | (cancer vs cancer) (cancer+normal vs cancer+normal) | 4-8386198 | cg18999185 | (cancer vs cancer) (cancer vs cancer) (cancer+normal vs cancer+normal) |
| 12-64317120 | cg17430032 | (normal vs normal) (cancer vs cancer) | 4-8477915 | cg22993937 | (cancer vs cancer) |

| | | | | | |
|---|---|---|---|---|---|
| | | (cancer+normal vs cancer+normal) | | | |
| 12-76102490 | cg15354877 | (cancer vs cancer) (cancer+normal vs cancer+normal) | 5-138474152 | cg06416831 | (cancer vs cancer) |
| 13-111553101 | cg23052386 | (cancer vs cancer) (cancer+normal vs cancer+normal) | 5-150131024 | cg25306198 | (cancer vs cancer) |
| 13-113977663 | cg25408055 | (cancer vs cancer) | 5-42756851 | cg23688479 | (cancer vs cancer) (cancer vs cancer) (cancer+normal vs cancer+normal) |
| 13-114303277 | cg21941987 | (normal vs normal) (cancer vs cancer) (cancer+normal vs cancer+normal) | 5-64103336 | cg15663823 | (cancer vs cancer) |
| 13-21547300 | cg15951545 | (cancer vs cancer) | 6-31760383 | cg00202479 | (cancer vs cancer) |
| 13-31905755 | cg26286098 | (cancer vs cancer) (cancer+normal vs cancer+normal) | 6-39159259 | cg00859574 | (cancer vs cancer) (cancer+normal vs cancer+normal) |
| 13-44824424 | cg13341441 | (cancer vs cancer) (cancer+normal vs cancer+normal) | 6-56298004 | cg10030658 | (cancer vs cancer) |
| 14-23375575 | cg24961406 | (cancer vs cancer) (cancer+normal vs cancer+normal) | 7-1117418 | cg20934096 | (cancer vs cancer) (cancer vs cancer) (cancer+normal vs cancer+normal) |
| 14-62021555 | cg18703243 | (cancer vs cancer) | 7-1117540 | cg08137085 | (cancer vs cancer) (cancer vs cancer) (cancer+normal vs cancer+normal) |
| 14-65210900 | cg11849213 | (cancer vs cancer) (cancer+normal vs cancer+normal) | 7-133083962 | cg09655887 | (cancer vs cancer) |
| 14-68162924 | cg10166090 | (cancer vs cancer) (cancer+normal vs cancer+normal) | 7-155599780 | cg20521696 | (cancer vs cancer) |
| 14-92198288 | cg19000611 | (cancer vs cancer) | 7-17582718 | cg22204915 | (cancer vs cancer) |
| 14-92493378 | cg19749445 | (cancer vs cancer) | 7-23719453 | cg21913591 | (cancer vs cancer) |
| 15-102261251 | cg14588686 | (cancer vs cancer) (cancer+normal vs cancer+normal) | 7-2609648 | cg13457496 | (cancer vs cancer) (cancer vs cancer) |
| 15-25436246 | cg25908613 | (cancer vs cancer) | 7-34026657 | cg11646259 | (cancer vs cancer) (cancer vs cancer) |
| 15-44088809 | cg09938490 | (normal vs normal) (cancer vs cancer) (cancer+normal vs cancer+normal) | 7-44526856 | cg13301931 | (cancer vs cancer) (cancer vs cancer) |
| 16-28915665 | cg27347003 | (cancer vs cancer) | 7-4751611 | cg08797598 | (cancer vs cancer) (cancer vs cancer) |
| 16-29268157 | cg00345270 | (cancer vs cancer) (cancer+normal vs cancer+normal) | 7-4752983 | cg27640064 | (cancer vs cancer) (cancer vs cancer) |
| 16-71748967 | cg07346700 | (cancer vs cancer) (cancer+normal vs cancer+normal) | 7-4753002 | cg04261496 | (cancer vs cancer) (cancer vs cancer) |
| 16-89190857 | cg06035645 | (cancer vs cancer) (cancer+normal vs | 7-4806755 | cg06114605 | (cancer vs cancer) (cancer vs cancer) |

175

| | | | | | |
|---|---|---|---|---|---|
| | | cancer+normal) | | | (cancer+normal vs cancer+normal) |
| 17-12924257 | cg01559375 | (cancer vs cancer) (cancer+normal vs cancer+normal) | 7-4806949 | cg25330264 | (cancer vs cancer) (cancer vs cancer) (cancer+normal vs cancer+normal) |
| 17-16838321 | cg26910001 | (cancer vs cancer) | 7-75357247 | cg18724912 | (cancer vs cancer) (cancer vs cancer) |
| 17-16934655 | cg26026558 | (cancer vs cancer) (cancer+normal vs cancer+normal) | 7-77584545 | cg19396253 | (normal vs normal) (cancer vs cancer) |
| 18-77673604 | cg20368463 | (cancer vs cancer) (cancer+normal vs cancer+normal) | 7-97875293 | cg18929814 | (cancer vs cancer) (cancer vs cancer) (cancer+normal vs cancer+normal) |
| 19-18120692 | cg12218406 | (cancer vs cancer) | 7-97875333 | cg18563899 | (cancer vs cancer) (cancer vs cancer) (cancer+normal vs cancer+normal) |
| 19-18120728 | cg03014882 | (cancer vs cancer) | 8-130976125 | cg16520519 | (cancer vs cancer) |
| 19-18120819 | cg00474840 | (cancer vs cancer) | 8-17655156 | cg18109537 | (cancer vs cancer) (cancer vs cancer) (cancer+normal vs cancer+normal) |
| 2-111883501 | cg14475035 | (cancer vs cancer) (cancer+normal vs cancer+normal) | 8-18245225 | cg23630442 | (cancer vs cancer) |
| 2-144698389 | cg26038022 | (cancer vs cancer) | 8-49729253 | cg03490289 | (cancer vs cancer) |
| 2-187451704 | cg08776913 | (cancer vs cancer) (cancer+normal vs cancer+normal) | 8-62412760 | cg16648094 | (normal vs normal) (cancer vs cancer) |
| 2-36638660 | cg11793005 | (cancer vs cancer) (cancer+normal vs cancer+normal) | 8-71016364 | cg05570783 | (cancer vs cancer) |
| 2-63834284 | cg14458626 | (cancer vs cancer) (cancer+normal vs cancer+normal) | 8-8560866 | cg19436804 | (cancer vs cancer) |
| 2-65314733 | cg14769556 | (cancer vs cancer) (cancer+normal vs cancer+normal) | 9-126128745 | cg14503395 | (cancer vs cancer) |
| 20-13798846 | cg15511401 | (cancer vs cancer) | **OV (cancer vs cancer)** | | |
| 20-36033352 | cg14637027 | (normal vs normal) (cancer vs cancer) (cancer+normal vs cancer+normal) | **Status - Hyper** | | |
| 20-36033357 | cg15574461 | (cancer vs cancer) (cancer+normal vs cancer+normal) | 1-1297576 | cg15492552 | (cancer vs cancer) (cancer+normal vs cancer+normal) |
| 21-43242428 | cg19408149 | (normal vs normal) (cancer vs cancer) (cancer+normal vs cancer+normal) | 1-205399945 | cg06849719 | (cancer vs cancer) (cancer+normal vs cancer+normal) |
| 22-36135459 | cg25564144 | (cancer vs cancer) (cancer+normal vs cancer+normal) | 1-205400120 | cg14221039 | (cancer vs cancer) (cancer+normal vs cancer+normal) |
| 3-120156942 | cg13331610 | (cancer vs cancer) (cancer+normal vs cancer+normal) | 1-205400197 | cg06968878 | (cancer vs cancer) (cancer+normal vs cancer+normal) |

| | | | | | |
|---|---|---|---|---|---|
| 3-122628121 | cg01051047 | (cancer vs cancer) | 1-219834537 | cg03599978 | (cancer vs cancer) (normal vs normal) (cancer+normal vs cancer+normal) |
| 3-24213704 | cg25087352 | (cancer vs cancer) (cancer+normal vs cancer+normal) | 1-219834581 | cg26682664 | (cancer vs cancer) (cancer+normal vs cancer+normal) |
| 3-36418645 | cg20832448 | (cancer vs cancer) | 1-2313373 | cg04286030 | (cancer vs cancer) (cancer+normal vs cancer+normal) |
| 3-37092193 | cg16863190 | (cancer vs cancer) (cancer+normal vs cancer+normal) | 1-45279638 | cg00902496 | (cancer vs cancer) (cancer+normal vs cancer+normal) |
| 4-4459401 | cg17360330 | (cancer vs cancer) (cancer+normal vs cancer+normal) | 1-45279684 | cg01059432 | (cancer vs cancer) (cancer+normal vs cancer+normal) |
| 4-77917883 | cg27624381 | (cancer vs cancer) (cancer+normal vs cancer+normal) | 1-8229825 | cg04908588 | (cancer vs cancer) (cancer+normal vs cancer+normal) |
| 5-138702273 | cg14660032 | (cancer vs cancer) (cancer+normal vs cancer+normal) | 10-104417977 | cg14255417 | (cancer vs cancer) (normal vs normal) (cancer+normal vs cancer+normal) |
| 5-1809371 | cg12153106 | (cancer vs cancer) (cancer+normal vs cancer+normal) | 10-119256257 | cg01250961 | (cancer vs cancer) (cancer+normal vs cancer+normal) |
| 6-105854768 | cg18879177 | (cancer vs cancer) (cancer+normal vs cancer+normal) | 10-24808256 | cg23196346 | (cancer vs cancer) (cancer+normal vs cancer+normal) |
| 6-144167951 | cg22290704 | (cancer vs cancer) | 10-48952423 | cg06088084 | (cancer vs cancer) (cancer+normal vs cancer+normal) |
| 6-16147669 | cg15404791 | (cancer vs cancer) (cancer+normal vs cancer+normal) | 10-74870240 | cg05898953 | (cancer vs cancer) (cancer+normal vs cancer+normal) |
| 6-167794374 | cg24134481 | (cancer vs cancer) (cancer+normal vs cancer+normal) | 10-74870311 | cg01701415 | (cancer vs cancer) (cancer+normal vs cancer+normal) |
| 6-31771646 | cg26922625 | (cancer vs cancer) (cancer+normal vs cancer+normal) | 10-76779305 | cg14219256 | (cancer vs cancer) (cancer+normal vs cancer+normal) |
| 6-34231501 | cg15495717 | (cancer vs cancer) (cancer+normal vs cancer+normal) | 10-94452554 | cg07616394 | (cancer vs cancer) (cancer+normal vs cancer+normal) |
| 6-36237037 | cg27319730 | (cancer vs cancer) (cancer+normal vs cancer+normal) | 10-95327292 | cg10905401 | (cancer vs cancer) (cancer+normal vs cancer+normal) |
| 7-106040839 | cg21114407 | (cancer vs cancer) | 10-95327305 | cg05613447 | (cancer vs cancer) (cancer+normal vs cancer+normal) |
| 7-133218612 | cg20973561 | (cancer vs cancer) (cancer+normal vs cancer+normal) | 10-95327325 | cg15133448 | (cancer vs cancer) (cancer+normal vs cancer+normal) |
| 7-157213736 | cg02717503 | (cancer vs cancer) (cancer+normal vs cancer+normal) | 11-129243219 | cg12439439 | (cancer vs cancer) (cancer+normal vs cancer+normal) |
| 7-1946447 | cg25786640 | (cancer vs cancer) (cancer+normal vs cancer+normal) | 11-133582402 | cg11368617 | (cancer vs cancer) (cancer+normal vs cancer+normal) |
| 7-30053497 | cg23492432 | (cancer vs cancer) (cancer+normal vs | 11-57568305 | cg11643991 | (cancer vs cancer) (cancer+normal vs |

| | | | | | |
|---|---|---|---|---|---|
| | | cancer+normal) | | | cancer+normal) |
| 7-40047674 | cg08832195 | (cancer vs cancer) | 11-70692032 | cg12198334 | (cancer vs cancer) (cancer+normal vs cancer+normal) |
| 8-129099476 | cg21896394 | (cancer vs cancer) | 11-70917283 | cg20170028 | (cancer vs cancer) (cancer+normal vs cancer+normal) |
| 8-135710113 | cg00267196 | (cancer vs cancer) (cancer+normal vs cancer+normal) | 11-76382149 | cg25717182 | (cancer vs cancer) (cancer+normal vs cancer+normal) |
| 9-131535850 | cg13919506 | (cancer vs cancer) (cancer+normal vs cancer+normal) | 12-130529514 | cg11334644 | (cancer vs cancer) (cancer+normal vs cancer+normal) |
| 9-134004811 | cg14295534 | (cancer vs cancer) (cancer+normal vs cancer+normal) | 12-130529849 | cg06153623 | (cancer vs cancer) (cancer+normal vs cancer+normal) |
| 9-33270634 | cg14641829 | (cancer vs cancer) (cancer+normal vs cancer+normal) | 12-130529951 | cg16236851 | (cancer vs cancer) (cancer+normal vs cancer+normal) |
| **(cancer+normal vs cancer+normal)** | | | 13-110899996 | cg15416440 | (cancer vs cancer) (cancer+normal vs cancer+normal) |
| **Status - Hyper** | | | 13-41187926 | cg09000385 | (cancer vs cancer) (cancer+normal vs cancer+normal) |
| 1-14075560 | cg09360458 | (cancer+normal vs cancer+normal) | 15-34260555 | cg07915206 | (cancer vs cancer) (cancer+normal vs cancer+normal) |
| 1-145096567 | cg08301542 | (cancer+normal vs cancer+normal) | 15-39283510 | cg23129342 | (cancer vs cancer) (cancer+normal vs cancer+normal) |
| 1-161111090 | cg23981150 | (cancer vs cancer) (cancer+normal vs cancer+normal) | 15-65197883 | cg03051384 | (cancer vs cancer) (cancer+normal vs cancer+normal) |
| 1-183440909 | cg25320221 | (cancer+normal vs cancer+normal) | 16-10274315 | cg04761722 | (cancer vs cancer) (cancer+normal vs cancer+normal) |
| 1-198904195 | cg09517106 | (cancer vs cancer) (cancer+normal vs cancer+normal) | 16-31821352 | cg01879726 | (cancer vs cancer) (cancer+normal vs cancer+normal) |
| 1-249120106 | cg24360513 | (normal vs normal) (cancer+normal vs cancer+normal) | 17-16284304 | cg01286555 | (cancer vs cancer) (cancer+normal vs cancer+normal) |
| 1-32802033 | cg00556029 | (normal vs normal) (cancer+normal vs cancer+normal) | 17-33542219 | cg24956176 | (cancer vs cancer) (cancer+normal vs cancer+normal) |
| 1-567501 | cg23917638 | (cancer+normal vs cancer+normal) | 17-35084975 | cg10547969 | (cancer vs cancer) (normal vs normal) (cancer+normal vs cancer+normal) |
| 1-82136224 | cg08110488 | (cancer vs cancer) (cancer+normal vs cancer+normal) | 17-36105064 | cg04433035 | (cancer vs cancer) (cancer+normal vs cancer+normal) |
| 10-38146577 | cg17187559 | (cancer+normal vs cancer+normal) | 17-36105117 | cg05222347 | (cancer vs cancer) (cancer+normal vs cancer+normal) |
| 10-8203261 | cg12515684 | (cancer+normal vs cancer+normal) | 17-36105239 | cg02335804 | (cancer vs cancer) (cancer+normal vs cancer+normal) |
| 11-18211133 | cg09333889 | (cancer+normal vs cancer+normal) | 17-36204463 | cg01550307 | (cancer vs cancer) (cancer+normal vs |

| | | | | | |
|---|---|---|---|---|---|
| | | | | | cancer+normal) |
| 11-2154113 | cg13165070 | (cancer vs cancer) (cancer+normal vs cancer+normal) | 17-40770823 | cg08323969 | (cancer vs cancer) (cancer+normal vs cancer+normal) |
| 11-2163174 | cg05323345 | (cancer+normal vs cancer+normal) | 19-58280801 | cg27046034 | (cancer vs cancer) (cancer+normal vs cancer+normal) |
| 11-2171478 | cg27331871 | (cancer+normal vs cancer+normal) | 19-58280832 | cg03584535 | (cancer vs cancer) (cancer+normal vs cancer+normal) |
| 11-27527925 | cg22830701 | (cancer+normal vs cancer+normal) | 19-58280891 | cg07685728 | (cancer vs cancer) (cancer+normal vs cancer+normal) |
| 11-31391482 | cg22333733 | (cancer+normal vs cancer+normal) | 19-58280927 | cg13636880 | (cancer vs cancer) (cancer+normal vs cancer+normal) |
| 11-47927148 | cg02294991 | (cancer+normal vs cancer+normal) | 19-58280994 | cg24298255 | (cancer vs cancer) (cancer+normal vs cancer+normal) |
| 11-64993239 | cg02675946 | (cancer vs cancer) (cancer+normal vs cancer+normal) | 19-58281016 | cg18575209 | (cancer vs cancer) (cancer+normal vs cancer+normal) |
| 12-108733261 | cg03612522 | (cancer vs cancer) (cancer+normal vs cancer+normal) | 19-58281019 | cg20751795 | (cancer vs cancer) (cancer+normal vs cancer+normal) |
| 12-108733275 | cg12142354 | (cancer vs cancer) (cancer+normal vs cancer+normal) | 19-58281117 | cg22956410 | (cancer vs cancer) (cancer+normal vs cancer+normal) |
| 12-108733308 | cg15448445 | (cancer vs cancer) (cancer+normal vs cancer+normal) | 19-58281450 | cg14038484 | (cancer vs cancer) (cancer+normal vs cancer+normal) |
| 12-4381777 | cg25425078 | (cancer vs cancer) (cancer+normal vs cancer+normal) | 2-112658759 | cg17354880 | (cancer vs cancer) (cancer+normal vs cancer+normal) |
| 12-48478849 | cg24232869 | (normal vs normal) (cancer+normal vs cancer+normal) | 2-134325873 | cg02715592 | (cancer vs cancer) (cancer+normal vs cancer+normal) |
| 13-114238565 | cg15120341 | (cancer vs cancer) (cancer+normal vs cancer+normal) | 2-134326055 | cg18123344 | (cancer vs cancer) (cancer+normal vs cancer+normal) |
| 13-47471052 | cg06476131 | (cancer vs cancer) (cancer+normal vs cancer+normal) | 2-134326172 | cg18984282 | (cancer vs cancer) (cancer+normal vs cancer+normal) |
| 13-47471169 | cg09361691 | (cancer vs cancer) (cancer+normal vs cancer+normal) | 2-170623948 | cg04072843 | (cancer vs cancer) (cancer+normal vs cancer+normal) |
| 14-20811563 | cg18899035 | (cancer+normal vs cancer+normal) | 2-201726139 | cg00982136 | (cancer vs cancer) (cancer+normal vs cancer+normal) |
| 14-23452111 | cg18073151 | (cancer+normal vs cancer+normal) | 2-212609384 | cg04784316 | (cancer vs cancer) (cancer+normal vs cancer+normal) |
| 14-73396628 | cg24043861 | (cancer+normal vs cancer+normal) | 2-219738732 | cg25242471 | (cancer vs cancer) (cancer+normal vs cancer+normal) |
| 14-88789549 | cg08054244 | (cancer vs cancer) (cancer+normal vs cancer+normal) | 2-71192445 | cg04751811 | (cancer vs cancer) (cancer+normal vs cancer+normal) |
| 15-65281866 | cg27542399 | (cancer+normal vs cancer+normal) | 20-56025557 | cg24831725 | (cancer vs cancer) (cancer+normal vs cancer+normal) |

| 16-2732203 | cg01709316 | (cancer+normal vs cancer+normal) | 21-46898137 | cg20383948 | (cancer vs cancer) (cancer+normal vs cancer+normal) |
|---|---|---|---|---|---|
| 16-75032758 | cg26627568 | (cancer+normal vs cancer+normal) | 21-47920571 | cg16636721 | (cancer vs cancer) (cancer+normal vs cancer+normal) |
| 16-89237997 | cg08839312 | (cancer+normal vs cancer+normal) | 22-18189443 | cg18166947 | (cancer vs cancer) (cancer+normal vs cancer+normal) |
| 17-28395749 | cg26114595 | (cancer+normal vs cancer+normal) | 22-24373322 | cg04234412 | (cancer vs cancer) (cancer+normal vs cancer+normal) |
| 17-29037254 | cg13341633 | (cancer+normal vs cancer+normal) | 22-36044151 | cg21205978 | (cancer vs cancer) (cancer+normal vs cancer+normal) |
| 17-46952555 | cg24958366 | (cancer+normal vs cancer+normal) | 22-42077939 | cg06781532 | (cancer vs cancer) (cancer+normal vs cancer+normal) |
| 17-46970413 | cg21346818 | (cancer+normal vs cancer+normal) | 22-42078567 | cg08686092 | (cancer vs cancer) (cancer+normal vs cancer+normal) |
| 19-37861707 | cg22460206 | (cancer+normal vs cancer+normal) | 3-15839627 | cg25572043 | (cancer vs cancer) (normal vs normal) (cancer+normal vs cancer+normal) |
| 19-40169912 | cg14377791 | (normal vs normal) (cancer+normal vs cancer+normal) | 3-160167768 | cg02723904 | (cancer vs cancer) (cancer+normal vs cancer+normal) |
| 19-40854718 | cg08533403 | (cancer+normal vs cancer+normal) | 3-160167789 | cg12479674 | (cancer vs cancer) (cancer+normal vs cancer+normal) |
| 19-40972683 | cg17847861 | (cancer+normal vs cancer+normal) | 3-47865946 | cg13052625 | (cancer vs cancer) (cancer+normal vs cancer+normal) |
| 19-52646265 | cg24449629 | (cancer vs cancer) (cancer+normal vs cancer+normal) | 3-4910253 | cg02388709 | (cancer vs cancer) (cancer+normal vs cancer+normal) |
| 19-9789027 | cg17371487 | (cancer+normal vs cancer+normal) | 3-50312389 | cg07629965 | (cancer vs cancer) (cancer+normal vs cancer+normal) |
| 2-136743460 | cg18110444 | (cancer vs cancer) (cancer+normal vs cancer+normal) | 3-71112437 | cg01511742 | (cancer vs cancer) (cancer+normal vs cancer+normal) |
| 2-230785697 | cg07864632 | (cancer+normal vs cancer+normal) | 4-1161247 | cg05257291 | (cancer vs cancer) (normal vs normal) (cancer+normal vs cancer+normal) |
| 2-63850056 | cg10828910 | (cancer+normal vs cancer+normal) | 4-174013995 | cg13099892 | (cancer vs cancer) (cancer+normal vs cancer+normal) |
| 2-71504771 | cg04737950 | (cancer vs cancer) (cancer+normal vs cancer+normal) | 4-38859728 | cg14665413 | (cancer vs cancer) (cancer+normal vs cancer+normal) |
| 2-86332777 | cg23655155 | (normal vs normal) (cancer+normal vs cancer+normal) | 4-4859772 | cg14602530 | (cancer vs cancer) (cancer+normal vs cancer+normal) |
| 20-30327429 | cg03352975 | (cancer+normal vs cancer+normal) | 4-649573 | cg20444990 | (cancer vs cancer) (cancer+normal vs cancer+normal) |
| 20-34359917 | cg27405321 | (cancer+normal vs cancer+normal) | 5-10307544 | cg17467873 | (cancer vs cancer) (cancer+normal vs |

| | | | | | |
|---|---|---|---|---|---|
| | | | | | (cancer+normal) |
| 20-443331 | cg04165120 | (cancer+normal vs cancer+normal) | 5-10307634 | cg1188225 2 | (cancer vs cancer) (cancer+normal vs cancer+normal) |
| 21-28516138 | cg10405333 | (cancer+normal vs cancer+normal) | 5-10307744 | cg0559334 9 | (cancer vs cancer) (cancer+normal vs cancer+normal) |
| 21-37504755 | cg17499729 | (cancer+normal vs cancer+normal) | 5-140502124 | cg1392597 5 | (cancer vs cancer) (cancer+normal vs cancer+normal) |
| 21-44985357 | cg16267304 | (cancer+normal vs cancer+normal) | 5-140739501 | cg0424614 4 | (cancer vs cancer) (cancer+normal vs cancer+normal) |
| 22-31556314 | cg01253545 | (normal vs normal) (cancer+normal vs cancer+normal) | 5-140739655 | cg1149902 5 | (cancer vs cancer) (cancer+normal vs cancer+normal) |
| 22-32435553 | cg05062854 | (cancer+normal vs cancer+normal) | 5-140753418 | cg0383560 9 | (cancer vs cancer) (cancer+normal vs cancer+normal) |
| 22-39796170 | cg07735571 | (cancer+normal vs cancer+normal) | 5-1479911 | cg0514557 3 | (cancer vs cancer) (cancer+normal vs cancer+normal) |
| 3-111579326 | cg05957736 | (normal vs normal) (cancer+normal vs cancer+normal) | 5-180469248 | cg0266630 2 | (cancer vs cancer) (cancer+normal vs cancer+normal) |
| 3-119217133 | cg19231082 | (cancer+normal vs cancer+normal) | 5-180486155 | cg2210268 8 | (cancer vs cancer) (cancer+normal vs cancer+normal) |
| 3-120627406 | cg05293820 | (cancer vs cancer) (normal vs normal) (cancer+normal vs cancer+normal) | 6-24205566 | cg0810796 0 | (cancer vs cancer) (cancer+normal vs cancer+normal) |
| 3-122694286 | cg07733481 | (normal vs normal) (cancer+normal vs cancer+normal) | 6-8192098 | cg2535455 1 | (cancer vs cancer) (cancer+normal vs cancer+normal) |
| 3-42641750 | cg05555409 | (cancer+normal vs cancer+normal) | 7-92262970 | cg0510143 7 | (cancer vs cancer) (cancer+normal vs cancer+normal) |
| 4-115520072 | cg10156125 | (normal vs normal) (cancer+normal vs cancer+normal) | 8-134511683 | cg0177899 4 | (cancer vs cancer) (cancer+normal vs cancer+normal) |
| 4-147443095 | cg20910303 | (cancer vs cancer) (cancer+normal vs cancer+normal) | 8-54569668 | cg0548302 1 | (cancer vs cancer) (cancer+normal vs cancer+normal) |
| 4-43900946 | cg14094460 | (cancer vs cancer) (cancer+normal vs cancer+normal) | 8-54569817 | cg2297346 8 | (cancer vs cancer) (cancer+normal vs cancer+normal) |
| 4-43901084 | cg05899741 | (cancer vs cancer) (cancer+normal vs cancer+normal) | 8-54569999 | cg0188799 5 | (cancer vs cancer) (cancer+normal vs cancer+normal) |
| 4-57844010 | cg16727027 | (cancer+normal vs cancer+normal) | **Status - Hypo** | | |
| 5-111115809 | cg11657018 | (cancer vs cancer) (cancer+normal vs cancer+normal) | 1-236156917 | cg0245436 4 | (cancer vs cancer) (cancer+normal vs cancer+normal) |
| 5-115387951 | cg23311108 | (cancer vs cancer) (normal vs normal) (cancer+normal vs cancer+normal) | 1-236157114 | cg0757821 5 | (cancer vs cancer) (cancer+normal vs cancer+normal) |
| 5-115387984 | cg14690181 | (cancer vs cancer) (cancer+normal vs | 1-236157142 | cg0420418 8 | (cancer vs cancer) (cancer+normal vs |

| | | | | | |
|---|---|---|---|---|---|
| | | cancer+normal) | | | cancer+normal) |
| 5-1174666 | cg02124887 | (normal vs normal) (cancer+normal vs cancer+normal) | 1-236157210 | cg06094815 | (cancer vs cancer) (cancer+normal vs cancer+normal) |
| 5-53606234 | cg22963133 | (cancer+normal vs cancer+normal) | 1-7210826 | cg24513014 | (cancer vs cancer) (cancer+normal vs cancer+normal) |
| 6-109804103 | cg20560186 | (cancer+normal vs cancer+normal) | 1-94560697 | cg09234454 | (normal vs normal) (cancer vs cancer) (cancer+normal vs cancer+normal) |
| 6-153304972 | cg14765172 | (cancer+normal vs cancer+normal) | 10-132365700 | cg13253210 | (cancer vs cancer) (cancer+normal vs cancer+normal) |
| 6-27775877 | cg06926499 | (cancer vs cancer) (cancer+normal vs cancer+normal) | 10-134246660 | cg19448497 | (cancer vs cancer) (cancer+normal vs cancer+normal) |
| 6-27777858 | cg18943383 | (cancer vs cancer) (cancer+normal vs cancer+normal) | 10-134593345 | cg11790551 | (cancer vs cancer) (cancer+normal vs cancer+normal) |
| 6-27782484 | cg16347724 | (cancer vs cancer) (cancer+normal vs cancer+normal) | 10-134593418 | cg13418804 | (cancer vs cancer) (cancer+normal vs cancer+normal) |
| 6-27782628 | cg05444312 | (cancer vs cancer) (cancer+normal vs cancer+normal) | 10-134593496 | cg19895894 | (cancer vs cancer) (cancer+normal vs cancer+normal) |
| 6-27832945 | cg05729499 | (cancer+normal vs cancer+normal) | 10-134593532 | cg16832975 | (cancer vs cancer) (cancer+normal vs cancer+normal) |
| 6-27840105 | cg05511483 | (cancer vs cancer) (cancer+normal vs cancer+normal) | 10-134593607 | cg07783889 | (cancer vs cancer) (cancer+normal vs cancer+normal) |
| 6-30539641 | cg20738959 | (cancer+normal vs cancer+normal) | 10-134996411 | cg04599298 | (cancer vs cancer) (cancer+normal vs cancer+normal) |
| 6-31779637 | cg03243683 | (cancer+normal vs cancer+normal) | 10-38240973 | cg26684581 | (cancer vs cancer) (cancer+normal vs cancer+normal) |
| 6-32522683 | cg25140213 | (cancer vs cancer) (normal vs normal) (cancer+normal vs cancer+normal) | 11-120998732 | cg26401732 | (cancer vs cancer) (cancer+normal vs cancer+normal) |
| 6-32547019 | cg07984380 | (cancer vs cancer) (cancer+normal vs cancer+normal) | 11-130781542 | cg05431684 | (cancer vs cancer) (cancer+normal vs cancer+normal) |
| 6-33291367 | cg03369465 | (cancer+normal vs cancer+normal) | 11-17591873 | cg07592809 | (cancer vs cancer) (cancer+normal vs cancer+normal) |
| 6-64289969 | cg12857652 | (cancer+normal vs cancer+normal) | 11-63885740 | cg06268327 | (cancer vs cancer) (cancer+normal vs cancer+normal) |
| 6-66804704 | cg19032326 | (cancer+normal vs cancer+normal) | 11-63886204 | cg17676132 | (cancer vs cancer) (cancer+normal vs cancer+normal) |
| 6-74171350 | cg13435101 | (cancer+normal vs cancer+normal) | 11-64108790 | cg16718999 | (cancer vs cancer) (cancer+normal vs cancer+normal) |
| 6-89769123 | cg12344600 | (cancer+normal vs cancer+normal) | 11-65124394 | cg10925420 | (cancer vs cancer) (cancer+normal vs cancer+normal) |
| 7- | cg14823389 | (cancer vs cancer) | 11- | cg2411209 | (cancer vs cancer) |

| Position | CpG | Comparison |
|---|---|---|
| 101398152 | | (cancer+normal vs cancer+normal) |
| 7-1250182 | cg07919840 | (cancer vs cancer) (cancer+normal vs cancer+normal) |
| 7-128046294 | cg27488370 | (cancer+normal vs cancer+normal) |
| 8-124408743 | cg07229027 | (cancer+normal vs cancer+normal) |
| 8-93156639 | cg09373786 | (normal vs normal) (cancer+normal vs cancer+normal) |
| 9-102582694 | cg13796392 | (normal vs normal) (cancer+normal vs cancer+normal) |
| 9-123639935 | cg11054680 | (cancer+normal vs cancer+normal) |
| 9-123639954 | cg13793166 | (cancer vs cancer) (cancer+normal vs cancer+normal) |
| 9-99962350 | cg00429618 | (cancer vs cancer) (cancer+normal vs cancer+normal) |
| **Status - Hypo** | | |
| 1-114414802 | cg26035817 | (cancer vs cancer) (cancer+normal vs cancer+normal) |
| 1-117489843 | cg23093116 | (cancer vs cancer) (cancer+normal vs cancer+normal) |
| 1-119520934 | cg15553612 | (cancer vs cancer) (cancer+normal vs cancer+normal) |
| 1-151607108 | cg26571535 | (cancer+normal vs cancer+normal) |
| 1-160314746 | cg22443054 | (cancer+normal vs cancer+normal) |
| 1-185380011 | cg26428191 | (normal vs normal) (cancer+normal vs cancer+normal) |
| 1-202559755 | cg11143857 | (cancer vs cancer) (cancer+normal vs cancer+normal) |
| 1-207977042 | cg22525895 | (cancer+normal vs cancer+normal) |
| 1-233817629 | cg10175859 | (normal vs normal) (cancer vs cancer) (cancer+normal vs cancer+normal) |
| 1-23418405 | cg15897613 | (cancer vs cancer) (cancer+normal vs cancer+normal) |
| 1- | cg08460387 | (cancer vs cancer) |

| Position | CpG | Comparison |
|---|---|---|
| 69683619 | 1 | (cancer+normal vs cancer+normal) |
| 11-70289549 | cg08777812 | (cancer vs cancer) (cancer+normal vs cancer+normal) |
| 11-70416309 | cg05401670 | (cancer vs cancer) (cancer+normal vs cancer+normal) |
| 11-70458782 | cg14232870 | (cancer vs cancer) (cancer+normal vs cancer+normal) |
| 11-73685776 | cg04807856 | (cancer vs cancer) (cancer+normal vs cancer+normal) |
| 13-112630656 | cg11423130 | (cancer vs cancer) (cancer+normal vs cancer+normal) |
| 13-113461500 | cg09975219 | (cancer vs cancer) (cancer+normal vs cancer+normal) |
| 13-113491766 | cg02490942 | (cancer vs cancer) (cancer+normal vs cancer+normal) |
| 13-113634407 | cg16341737 | (cancer vs cancer) (cancer+normal vs cancer+normal) |
| 13-113663206 | cg17928152 | (cancer vs cancer) (cancer+normal vs cancer+normal) |
| 13-113696937 | cg02909018 | (cancer vs cancer) (cancer+normal vs cancer+normal) |
| 13-113708289 | cg24746106 | (cancer vs cancer) (cancer+normal vs cancer+normal) |
| 13-113744072 | cg16043368 | (cancer vs cancer) (cancer+normal vs cancer+normal) |
| 13-113744320 | cg16277139 | (cancer vs cancer) (cancer+normal vs cancer+normal) |
| 13-113796215 | cg02617285 | (cancer vs cancer) (cancer+normal vs cancer+normal) |
| 14-101192634 | cg13684115 | (cancer vs cancer) (cancer+normal vs cancer+normal) |
| 14-91806002 | cg01021847 | (cancer vs cancer) (cancer+normal vs cancer+normal) |
| 15-22560713 | cg02759635 | (cancer vs cancer) (cancer+normal vs cancer+normal) |
| 15-45444508 | cg24006582 | (cancer vs cancer) (cancer+normal vs cancer+normal) |
| 15-58429751 | cg02450124 | (cancer vs cancer) (cancer+normal vs cancer+normal) |
| 16- | cg0747219 | (cancer vs cancer) |

| | | | | | |
|---|---|---|---|---|---|
| 26000291 | | (cancer+normal vs cancer+normal) | 31453477 | 9 | (cancer+normal vs cancer+normal) |
| 1-40346530 | cg10530613 | (cancer vs cancer) (cancer+normal vs cancer+normal) | 16-85465268 | cg02174252 | (cancer vs cancer) (cancer+normal vs cancer+normal) |
| 1-67266410 | cg11521979 | (cancer vs cancer) (cancer+normal vs cancer+normal) | 16-85465628 | cg16581085 | (cancer vs cancer) (cancer+normal vs cancer+normal) |
| 1-6855140 | cg04874074 | (normal vs normal) (cancer vs cancer) (cancer+normal vs cancer+normal) | 16-87856264 | cg01412419 | (cancer vs cancer) (cancer+normal vs cancer+normal) |
| 10-102671221 | cg15448683 | (cancer vs cancer) (cancer+normal vs cancer+normal) | 16-88534585 | cg07933078 | (cancer vs cancer) (cancer+normal vs cancer+normal) |
| 10-119101310 | cg17182036 | (cancer vs cancer) (cancer+normal vs cancer+normal) | 16-89219472 | cg05445348 | (cancer vs cancer) (cancer+normal vs cancer+normal) |
| 10-133748048 | cg13985270 | (cancer+normal vs cancer+normal) | 16-89219547 | cg05032150 | (cancer vs cancer) (cancer+normal vs cancer+normal) |
| 10-135097477 | cg27633010 | (cancer vs cancer) (cancer+normal vs cancer+normal) | 17-1069335 | cg12159837 | (cancer vs cancer) (cancer+normal vs cancer+normal) |
| 10-135206831 | cg05676341 | (cancer+normal vs cancer+normal) (cancer+normal vs cancer+normal) | 17-2593076 | cg19435859 | (cancer vs cancer) (cancer+normal vs cancer+normal) |
| 10-5542941 | cg26552733 | (cancer vs cancer) (cancer+normal vs cancer+normal) | 17-6024017 | cg06304518 | (cancer vs cancer) (cancer+normal vs cancer+normal) |
| 10-76801843 | cg23077498 | (cancer vs cancer) (cancer+normal vs cancer+normal) | 18-13263188 | cg19772897 | (cancer vs cancer) (cancer+normal vs cancer+normal) |
| 10-93872745 | cg24984532 | (cancer vs cancer) (cancer+normal vs cancer+normal) | 18-76725474 | cg01593857 | (cancer vs cancer) (cancer+normal vs cancer+normal) |
| 11-124539453 | cg18743034 | (normal vs normal) (cancer vs cancer) (cancer+normal vs cancer+normal) | 19-16466480 | cg17702388 | (cancer vs cancer) (cancer+normal vs cancer+normal) |
| 11-126072352 | cg26351663 | (cancer vs cancer) (cancer+normal vs cancer+normal) | 19-16466617 | cg04738220 | (cancer vs cancer) (cancer+normal vs cancer+normal) |
| 11-130763724 | cg00458607 | (cancer vs cancer) (cancer+normal vs cancer+normal) | 19-4371934 | cg24928938 | (cancer vs cancer) (cancer+normal vs cancer+normal) |
| 11-134118834 | cg22756211 | (cancer vs cancer) (cancer+normal vs cancer+normal) | 19-45262055 | cg11459773 | (cancer vs cancer) (cancer+normal vs cancer+normal) |
| 11-2985302 | cg13363640 | (cancer vs cancer) (cancer+normal vs cancer+normal) | 19-4544446 | cg02715602 | (cancer vs cancer) (cancer+normal vs cancer+normal) |
| 11-63883947 | cg24431193 | (cancer vs cancer) (cancer+normal vs cancer+normal) | 2-102867275 | cg05168012 | (cancer vs cancer) (cancer+normal vs cancer+normal) |
| 11-75115584 | cg12506582 | (cancer vs cancer) (cancer+normal vs cancer+normal) | 2-241896967 | cg08200420 | (cancer vs cancer) (cancer+normal vs cancer+normal) |
| 12-11005841 | cg26459499 | (cancer vs cancer) (cancer+normal vs | 2-72359477 | cg14657116 | (cancer vs cancer) (cancer+normal vs |

| | | cancer+normal) | | | cancer+normal) |
|---|---|---|---|---|---|
| 12-117593728 | cg22694318 | (cancer+normal vs cancer+normal) | 2-74375930 | cg09717809 | (normal vs normal) (cancer vs cancer) (cancer+normal vs cancer+normal) |
| 12-46753241 | cg23334507 | (cancer vs cancer) (cancer+normal vs cancer+normal) | 2-8343710 | cg23079012 | (cancer vs cancer) (cancer+normal vs cancer+normal) |
| 12-46945908 | cg00966357 | (cancer+normal vs cancer+normal) | 20-2308988 | cg20117447 | (cancer vs cancer) (cancer+normal vs cancer+normal) |
| 12-48163063 | cg10052368 | (cancer vs cancer) (cancer+normal vs cancer+normal) | 22-18277981 | cg12923994 | (cancer vs cancer) (cancer+normal vs cancer+normal) |
| 12-64317120 | cg17430032 | (normal vs normal) (cancer vs cancer) (cancer+normal vs cancer+normal) | 3-123813393 | cg25184248 | (cancer vs cancer) (cancer+normal vs cancer+normal) |
| 12-76102490 | cg15354877 | (cancer vs cancer) (cancer+normal vs cancer+normal) | 3-123813596 | cg10430690 | (cancer vs cancer) (cancer+normal vs cancer+normal) |
| 13-111553101 | cg23052386 | (cancer vs cancer) (cancer+normal vs cancer+normal) | 3-130368239 | cg08850509 | (cancer vs cancer) (cancer+normal vs cancer+normal) |
| 13-114303277 | cg21941987 | (normal vs normal) (cancer vs cancer) (cancer+normal vs cancer+normal) | 3-196318703 | cg03920544 | (cancer vs cancer) (cancer+normal vs cancer+normal) |
| 13-31905755 | cg26286098 | (cancer vs cancer) (cancer+normal vs cancer+normal) | 3-196318719 | cg12288267 | (cancer vs cancer) (cancer+normal vs cancer+normal) |
| 13-44824424 | cg13341441 | (cancer vs cancer) (cancer+normal vs cancer+normal) | 3-196387058 | cg16053281 | (cancer vs cancer) (cancer+normal vs cancer+normal) |
| 14-23375575 | cg24961406 | (cancer vs cancer) (cancer+normal vs cancer+normal) | 4-90168963 | cg12577942 | (cancer vs cancer) (cancer+normal vs cancer+normal) |
| 14-65210900 | cg11849213 | (cancer vs cancer) (cancer+normal vs cancer+normal) | 5-131658161 | cg19848599 | (cancer vs cancer) (cancer+normal vs cancer+normal) |
| 14-68162924 | cg10166090 | (cancer vs cancer) (cancer+normal vs cancer+normal) | 5-13810195 | cg20738735 | (cancer vs cancer) (cancer+normal vs cancer+normal) |
| 15-102261251 | cg14588686 | (cancer vs cancer) (cancer+normal vs cancer+normal) | 5-13810279 | cg08818829 | (cancer vs cancer) (cancer+normal vs cancer+normal) |
| 15-44088809 | cg09938490 | (normal vs normal) (cancer vs cancer) (cancer+normal vs cancer+normal) | 5-42468836 | cg08761396 | (cancer vs cancer) (cancer+normal vs cancer+normal) |
| 15-64683155 | cg21501234 | (cancer+normal vs cancer+normal) | 6-168378903 | cg00678938 | (cancer vs cancer) (cancer+normal vs cancer+normal) |
| 16-29268157 | cg00345270 | (cancer vs cancer) (cancer+normal vs cancer+normal) | 6-169825345 | cg11181693 | (cancer vs cancer) (cancer+normal vs cancer+normal) |
| 16-67276940 | cg27054887 | (cancer+normal vs cancer+normal) (cancer+normal vs cancer+normal) | 6-30708009 | cg05129568 | (cancer vs cancer) (cancer+normal vs cancer+normal) |
| 16- | cg07346700 | (cancer vs cancer) | 6-31592082 | cg0010791 | (cancer vs cancer) |

| | | | | | |
|---|---|---|---|---|---|
| 71748967 | | (cancer+normal vs cancer+normal) | | 4 | (cancer+normal vs cancer+normal) |
| 16-87470545 | cg16727006 | (cancer+normal vs cancer+normal) | 6-42231123 | cg13256491 | (cancer vs cancer) (cancer+normal vs cancer+normal) |
| 16-89190857 | cg06035645 | (cancer vs cancer) (cancer+normal vs cancer+normal) | 7-101755186 | cg06746318 | (cancer vs cancer) (cancer+normal vs cancer+normal) |
| 17-12924257 | cg01559375 | (cancer vs cancer) (cancer+normal vs cancer+normal) | 7-157449217 | cg07506599 | (cancer vs cancer) (cancer+normal vs cancer+normal) |
| 17-16934655 | cg26026558 | (cancer vs cancer) (cancer+normal vs cancer+normal) | 7-36339044 | cg02734419 | (cancer vs cancer) (cancer+normal vs cancer+normal) |
| 18-77673604 | cg20368463 | (cancer vs cancer) (cancer+normal vs cancer+normal) | 7-36585420 | cg12069132 | (cancer vs cancer) (cancer+normal vs cancer+normal) |
| 19-14171165 | cg11437328 | (normal vs normal) (cancer+normal vs cancer+normal) | 7-56242072 | cg03456771 | (cancer vs cancer) (cancer+normal vs cancer+normal) |
| 19-44342834 | cg14023449 | (cancer+normal vs cancer+normal) | 8-142841083 | cg17776033 | (cancer vs cancer) (cancer+normal vs cancer+normal) |
| 2-102978461 | cg01969473 | (cancer+normal vs cancer+normal) | 8-144872852 | cg09541794 | (cancer vs cancer) (cancer+normal vs cancer+normal) |
| 2-111883501 | cg14475035 | (cancer vs cancer) (cancer+normal vs cancer+normal) | 8-144873034 | cg10282319 | (cancer vs cancer) (cancer+normal vs cancer+normal) |
| 2-147933072 | cg20740102 | (cancer+normal vs cancer+normal) | 8-144873183 | cg18430411 | (cancer vs cancer) (cancer+normal vs cancer+normal) |
| 2-187451704 | cg08776913 | (cancer vs cancer) (cancer+normal vs cancer+normal) | 8-144958131 | cg00112048 | (cancer vs cancer) (cancer+normal vs cancer+normal) |
| 2-228176991 | cg12267933 | (normal vs normal) (cancer+normal vs cancer+normal) | 8-43131260 | cg19035254 | (cancer vs cancer) (cancer+normal vs cancer+normal) |
| 2-36638660 | cg11793005 | (cancer vs cancer) (cancer+normal vs cancer+normal) | 8-48676898 | cg11912754 | (cancer vs cancer) (cancer+normal vs cancer+normal) |
| 2-63834284 | cg14458626 | (cancer vs cancer) (cancer+normal vs cancer+normal) | 8-49335020 | cg14938360 | (cancer vs cancer) (cancer+normal vs cancer+normal) |
| 2-65314733 | cg14769556 | (cancer vs cancer) (cancer+normal vs cancer+normal) | 8-49335078 | cg00726029 | (cancer vs cancer) (cancer+normal vs cancer+normal) |
| 2-87420273 | cg12600631 | (cancer+normal vs cancer+normal) | 9-140703588 | cg14469972 | (cancer vs cancer) (cancer+normal vs cancer+normal) |
| 20-36033352 | cg14637027 | (normal vs normal) (cancer vs cancer) (cancer+normal vs cancer+normal) | 9-140703637 | cg12872489 | (cancer vs cancer) (cancer+normal vs cancer+normal) |
| 20-36033357 | cg15574461 | (cancer vs cancer) (cancer+normal vs cancer+normal) | 9-99258799 | cg20960456 | (cancer vs cancer) (cancer+normal vs cancer+normal) |
| 21-43242428 | cg19408149 | (normal vs normal) (cancer vs cancer) (cancer+normal vs cancer+normal) | 9-99259456 | cg14160020 | (cancer vs cancer) (cancer+normal vs cancer+normal) |

| | | | | | |
|---|---|---|---|---|---|
| 22-36135459 | cg25564144 | (cancer vs cancer) (cancer+normal vs cancer+normal) | **(cancer+normal vs cancer+normal)** | | |
| 3-113405689 | cg24599037 | (cancer+normal vs cancer+normal) | **Status - Hyper** | | |
| 3-120156942 | cg13331610 | (cancer vs cancer) (cancer+normal vs cancer+normal) | 1-1297576 | cg15492552 | (cancer vs cancer) (cancer+normal vs cancer+normal) |
| 3-138479392 | cg27500716 | (cancer+normal vs cancer+normal) | 1-205399945 | cg06849719 | (cancer vs cancer) (cancer+normal vs cancer+normal) |
| 3-140992338 | cg18802720 | (normal vs normal) (cancer+normal vs cancer+normal) | 1-205400120 | cg14221039 | (cancer vs cancer) (cancer+normal vs cancer+normal) |
| 3-24213704 | cg25087352 | (cancer vs cancer) (cancer+normal vs cancer+normal) | 1-205400197 | cg06968878 | (cancer vs cancer) (cancer+normal vs cancer+normal) |
| 3-37092193 | cg16863190 | (cancer vs cancer) (cancer+normal vs cancer+normal) | 1-219834537 | cg03599978 | (cancer vs cancer) (normal vs normal) (cancer+normal vs cancer+normal) |
| 4-4459401 | cg17360330 | (cancer vs cancer) (cancer+normal vs cancer+normal) | 1-219834581 | cg26682664 | (cancer vs cancer) (cancer+normal vs cancer+normal) |
| 4-77917883 | cg27624381 | (cancer vs cancer) (cancer+normal vs cancer+normal) | 1-2313373 | cg04286030 | (cancer vs cancer) (cancer+normal vs cancer+normal) |
| 5-138702273 | cg14660032 | (cancer vs cancer) (cancer+normal vs cancer+normal) | 1-45279638 | cg00902496 | (cancer vs cancer) (cancer+normal vs cancer+normal) |
| 5-1809371 | cg12153106 | (cancer vs cancer) (cancer+normal vs cancer+normal) | 1-45279684 | cg01059432 | (cancer vs cancer) (cancer+normal vs cancer+normal) |
| 6-105854768 | cg18879177 | (cancer vs cancer) (cancer+normal vs cancer+normal) | 1-8229825 | cg04908588 | (cancer vs cancer) (cancer+normal vs cancer+normal) |
| 6-16147669 | cg15404791 | (cancer vs cancer) (cancer+normal vs cancer+normal) | 10-104417977 | cg14255417 | (cancer vs cancer) (normal vs normal) (cancer+normal vs cancer+normal) |
| 6-167794374 | cg24134481 | (cancer vs cancer) (cancer+normal vs cancer+normal) | 10-119256257 | cg01250961 | (cancer vs cancer) (cancer+normal vs cancer+normal) |
| 6-31771646 | cg26922625 | (cancer vs cancer) (cancer+normal vs cancer+normal) | 10-24808256 | cg23196346 | (cancer vs cancer) (cancer+normal vs cancer+normal) |
| 6-34231501 | cg15495717 | (cancer vs cancer) (cancer+normal vs cancer+normal) | 10-48952423 | cg06088084 | (cancer vs cancer) (cancer+normal vs cancer+normal) |
| 6-36237037 | cg27319730 | (cancer vs cancer) (cancer+normal vs cancer+normal) | 10-74870240 | cg05898953 | (cancer vs cancer) (cancer+normal vs cancer+normal) |
| 6-44084209 | cg10049156 | (cancer+normal vs cancer+normal) | 10-74870311 | cg01701415 | (cancer vs cancer) (cancer+normal vs cancer+normal) |
| 6-7414671 | cg10066132 | (cancer+normal vs cancer+normal) | 10-76779305 | cg14219256 | (cancer vs cancer) (cancer+normal vs cancer+normal) |
| 7-133218612 | cg20973561 | (cancer vs cancer) (cancer+normal vs cancer+normal) | 10-94452554 | cg07616394 | (cancer vs cancer) (cancer+normal vs cancer+normal) |

| | | | | | |
|---|---|---|---|---|---|
| 7-134850443 | cg14329833 | (cancer+normal vs cancer+normal) | 10-95327292 | cg10905401 | (cancer vs cancer) (cancer+normal vs cancer+normal) |
| 7-156561790 | cg24952358 | (cancer+normal vs cancer+normal) | 10-95327305 | cg05613447 | (cancer vs cancer) (cancer+normal vs cancer+normal) |
| 7-157213736 | cg02717503 | (cancer vs cancer) (cancer+normal vs cancer+normal) | 10-95327325 | cg15133448 | (cancer vs cancer) (cancer+normal vs cancer+normal) |
| 7-158456404 | cg24564603 | (cancer+normal vs cancer+normal) | 11-129243219 | cg12439439 | (cancer vs cancer) (cancer+normal vs cancer+normal) |
| 7-1946447 | cg25786640 | (cancer vs cancer) (cancer+normal vs cancer+normal) | 11-133582402 | cg11368617 | (cancer vs cancer) (cancer+normal vs cancer+normal) |
| 7-23214241 | cg24984423 | (cancer+normal vs cancer+normal) | 11-57568305 | cg11643991 | (cancer vs cancer) (cancer+normal vs cancer+normal) |
| 7-30053497 | cg23492432 | (cancer vs cancer) (cancer+normal vs cancer+normal) | 11-70692032 | cg12198334 | (cancer vs cancer) (cancer+normal vs cancer+normal) |
| 7-90890086 | cg01196121 | (cancer+normal vs cancer+normal) | 11-70917283 | cg20170028 | (cancer vs cancer) (cancer+normal vs cancer+normal) |
| 8-135710113 | cg00267196 | (cancer vs cancer) (cancer+normal vs cancer+normal) | 11-76382149 | cg25717182 | (cancer vs cancer) (cancer+normal vs cancer+normal) |
| 9-131535850 | cg13919506 | (cancer vs cancer) (cancer+normal vs cancer+normal) | 12-130529514 | cg11334644 | (cancer vs cancer) (cancer+normal vs cancer+normal) |
| 9-134004811 | cg14295534 | (cancer vs cancer) (cancer+normal vs cancer+normal) | 12-130529849 | cg06153623 | (cancer vs cancer) (cancer+normal vs cancer+normal) |
| 9-33270634 | cg14641829 | (cancer vs cancer) (cancer+normal vs cancer+normal) | 12-130529951 | cg16236851 | (cancer vs cancer) (cancer+normal vs cancer+normal) |
| **(normal vs normal)** | | | 13-110899996 | cg15416440 | (cancer vs cancer) (cancer+normal vs cancer+normal) |
| **Status - Hyper** | | | 13-41187926 | cg09000385 | (cancer vs cancer) (cancer+normal vs cancer+normal) |
| 1-110210925 | cg03942855 | (normal vs normal) | 15-34260555 | cg07915206 | (cancer vs cancer) (cancer+normal vs cancer+normal) |
| 1-110254662 | cg25593510 | (normal vs normal) | 15-39283510 | cg23129342 | (cancer vs cancer) (cancer+normal vs cancer+normal) |
| 1-110254709 | cg20803293 | (normal vs normal) | 15-65197883 | cg03051384 | (cancer vs cancer) (cancer+normal vs cancer+normal) |
| 1-146544097 | cg01701555 | (normal vs normal) | 16-10274315 | cg04761722 | (cancer vs cancer) (cancer+normal vs cancer+normal) |
| 1-150121727 | cg12072973 | (normal vs normal) | 16-31821352 | cg01879726 | (cancer vs cancer) (cancer+normal vs cancer+normal) |
| 1-150670422 | cg09365446 | (normal vs normal) | 17-16284304 | cg01286555 | (cancer vs cancer) (cancer+normal vs cancer+normal) |
| 1- | cg03277515 | (normal vs normal) | 17- | cg2495617 | (cancer vs cancer) |

| | | | | | |
|---|---|---|---|---|---|
| 153321198 | | | 33542219 | 6 | (cancer+normal vs cancer+normal) |
| 1-153514442 | cg19808620 | (normal vs normal) | 17-35084975 | cg10547969 | (cancer vs cancer) (normal vs normal) (cancer+normal vs cancer+normal) |
| 1-16554558 | cg16312002 | (normal vs normal) | 17-36105064 | cg04433035 | (cancer vs cancer) (cancer+normal vs cancer+normal) |
| 1-17086061 | cg04365481 | (normal vs normal) | 17-36105117 | cg05222347 | (cancer vs cancer) (cancer+normal vs cancer+normal) |
| 1-17086071 | cg06346138 | (normal vs normal) | 17-36105239 | cg02335804 | (cancer vs cancer) (cancer+normal vs cancer+normal) |
| 1-249120106 | cg24360513 | (normal vs normal) (cancer+normal vs cancer+normal) | 17-36204463 | cg01550307 | (cancer vs cancer) (cancer+normal vs cancer+normal) |
| 1-26797619 | cg27425314 | (normal vs normal) | 17-40770823 | cg08323969 | (cancer vs cancer) (cancer+normal vs cancer+normal) |
| 1-32802033 | cg00556029 | (normal vs normal) (cancer+normal vs cancer+normal) | 19-58280801 | cg27046034 | (cancer vs cancer) (cancer+normal vs cancer+normal) |
| 1-6265220 | cg19180828 | (normal vs normal) | 19-58280832 | cg03584535 | (cancer vs cancer) (cancer+normal vs cancer+normal) |
| 10-113943723 | cg24965984 | (normal vs normal) | 19-58280891 | cg07685728 | (cancer vs cancer) (cancer+normal vs cancer+normal) |
| 10-135342218 | cg10862468 | (normal vs normal) | 19-58280927 | cg13636880 | (cancer vs cancer) (cancer+normal vs cancer+normal) |
| 10-29874759 | cg10261325 | (normal vs normal) | 19-58280994 | cg24298255 | (cancer vs cancer) (cancer+normal vs cancer+normal) |
| 10-30347670 | cg00479240 | (normal vs normal) | 19-58281016 | cg18575209 | (cancer vs cancer) (cancer+normal vs cancer+normal) |
| 10-43721445 | cg22757362 | (normal vs normal) | 19-58281019 | cg20751795 | (cancer vs cancer) (cancer+normal vs cancer+normal) |
| 11-2159122 | cg08986368 | (normal vs normal) | 19-58281117 | cg22956410 | (cancer vs cancer) (cancer+normal vs cancer+normal) |
| 11-2161473 | cg13756879 | (normal vs normal) | 19-58281450 | cg14038484 | (cancer vs cancer) (cancer+normal vs cancer+normal) |
| 11-33746426 | cg09864245 | (normal vs normal) | 2-112658759 | cg17354880 | (cancer vs cancer) (cancer+normal vs cancer+normal) |
| 11-4615540 | cg23153338 | (normal vs normal) | 2-134325873 | cg02715592 | (cancer vs cancer) (cancer+normal vs cancer+normal) |
| 11-64334217 | cg00270878 | (normal vs normal) | 2-134326055 | cg18123344 | (cancer vs cancer) (cancer+normal vs cancer+normal) |
| 11-64993335 | cg23930334 | (cancer vs cancer) (normal vs normal) | 2-134326172 | cg18984282 | (cancer vs cancer) (cancer+normal vs cancer+normal) |
| 11- | cg07873178 | (normal vs normal) | 2- | cg0407284 | (cancer vs cancer) |

| | | | | | |
|---|---|---|---|---|---|
| 65307616 | | | 170623948 | 3 | (cancer+normal vs cancer+normal) |
| 11-65307791 | cg04089426 | (normal vs normal) | 2-201726139 | cg00982136 | (cancer vs cancer) (cancer+normal vs cancer+normal) |
| 11-65308501 | cg00206168 | (normal vs normal) | 2-212609384 | cg04784316 | (cancer vs cancer) (cancer+normal vs cancer+normal) |
| 11-71276557 | cg17256240 | (normal vs normal) | 2-219738732 | cg25242471 | (cancer vs cancer) (cancer+normal vs cancer+normal) |
| 12-119446891 | cg15341350 | (normal vs normal) | 2-71192445 | cg04751811 | (cancer vs cancer) (cancer+normal vs cancer+normal) |
| 12-122277439 | cg06601685 | (normal vs normal) | 20-56025557 | cg24831725 | (cancer vs cancer) (cancer+normal vs cancer+normal) |
| 12-124017613 | cg02571819 | (normal vs normal) | 21-46898137 | cg20383948 | (cancer vs cancer) (cancer+normal vs cancer+normal) |
| 13-28498161 | cg21900495 | (normal vs normal) | 21-47920571 | cg16636721 | (cancer vs cancer) (cancer+normal vs cancer+normal) |
| 13-28498334 | cg16302790 | (normal vs normal) | 22-18189443 | cg18166947 | (cancer vs cancer) (cancer+normal vs cancer+normal) |
| 13-28501366 | cg16300300 | (normal vs normal) | 22-24373322 | cg04234412 | (cancer vs cancer) (cancer+normal vs cancer+normal) |
| 13-28502630 | cg08100687 | (normal vs normal) | 22-36044151 | cg21205978 | (cancer vs cancer) (cancer+normal vs cancer+normal) |
| 14-103396161 | cg10366062 | (normal vs normal) | 22-42077939 | cg06781532 | (cancer vs cancer) (cancer+normal vs cancer+normal) |
| 15-37390284 | cg13800209 | (normal vs normal) | 22-42078567 | cg08686092 | (cancer vs cancer) (cancer+normal vs cancer+normal) |
| 15-53087599 | cg22078710 | (normal vs normal) | 3-15839627 | cg25572043 | (cancer vs cancer) (normal vs normal) (cancer+normal vs cancer+normal) |
| 16-3068085 | cg08935125 | (normal vs normal) | 3-160167768 | cg02723904 | (cancer vs cancer) (cancer+normal vs cancer+normal) |
| 16-3201981 | cg06643150 | (normal vs normal) | 3-160167789 | cg12479674 | (cancer vs cancer) (cancer+normal vs cancer+normal) |
| 16-56669038 | cg00170620 | (normal vs normal) | 3-47865946 | cg13052625 | (cancer vs cancer) (cancer+normal vs cancer+normal) |
| 16-67695516 | cg26919100 | (normal vs normal) | 3-4910253 | cg02388709 | (cancer vs cancer) (cancer+normal vs cancer+normal) |
| 17-18061566 | cg25826404 | (normal vs normal) | 3-50312389 | cg07629965 | (cancer vs cancer) (cancer+normal vs cancer+normal) |
| 17-26634225 | cg06503831 | (normal vs normal) | 3-71112437 | cg01511742 | (cancer vs cancer) (cancer+normal vs cancer+normal) |
| 17- | cg03741619 | (normal vs normal) | 4-1161247 | cg0525729 | (cancer vs cancer) |

| | | | | | |
|---|---|---|---|---|---|
| 3438918 | | | | 1 | (normal vs normal) (cancer+normal vs cancer+normal) |
| 17-40705708 | cg17035997 | (normal vs normal) | 4-174013995 | cg13099892 | (cancer vs cancer) (cancer+normal vs cancer+normal) |
| 17-43037309 | cg02395846 | (normal vs normal) (normal vs normal) | 4-38859728 | cg14665413 | (cancer vs cancer) (cancer+normal vs cancer+normal) |
| 17-43037427 | cg00164949 | (normal vs normal) | 4-4859772 | cg14602530 | (cancer vs cancer) (cancer+normal vs cancer+normal) |
| 17-4642047 | cg18056600 | (normal vs normal) | 4-649573 | cg20444990 | (cancer vs cancer) (cancer+normal vs cancer+normal) |
| 17-61959416 | cg02856727 | (normal vs normal) | 5-10307544 | cg17467873 | (cancer vs cancer) (cancer+normal vs cancer+normal) |
| 19-16999768 | cg19006008 | (normal vs normal) | 5-10307634 | cg11882252 | (cancer vs cancer) (cancer+normal vs cancer+normal) |
| 19-2494587 | cg01515960 | (normal vs normal) | 5-10307744 | cg05593349 | (cancer vs cancer) (cancer+normal vs cancer+normal) |
| 19-40169912 | cg14377791 | (normal vs normal) (cancer+normal vs cancer+normal) | 5-140502124 | cg13925975 | (cancer vs cancer) (cancer+normal vs cancer+normal) |
| 19-47925425 | cg22933807 | (normal vs normal) | 5-140739501 | cg04246144 | (cancer vs cancer) (cancer+normal vs cancer+normal) |
| 19-49340574 | cg16594139 | (normal vs normal) | 5-140739655 | cg11499025 | (cancer vs cancer) (cancer+normal vs cancer+normal) |
| 19-56733045 | cg14941172 | (normal vs normal) | 5-140753418 | cg03835609 | (cancer vs cancer) (cancer+normal vs cancer+normal) |
| 2-20871002 | cg10687131 | (normal vs normal) | 5-1479911 | cg05145573 | (cancer vs cancer) (cancer+normal vs cancer+normal) |
| 2-74230310 | cg19147129 | (normal vs normal) | 5-180469248 | cg02666302 | (cancer vs cancer) (cancer+normal vs cancer+normal) |
| 2-86332777 | cg23655155 | (normal vs normal) (cancer+normal vs cancer+normal) | 5-180486155 | cg22102688 | (cancer vs cancer) (cancer+normal vs cancer+normal) |
| 20-3389329 | cg07023803 | (normal vs normal) | 6-24205566 | cg08107960 | (cancer vs cancer) (cancer+normal vs cancer+normal) |
| 22-17680545 | cg13248581 | (normal vs normal) | 6-8192098 | cg25354551 | (cancer vs cancer) (cancer+normal vs cancer+normal) |
| 3-111579326 | cg05957736 | (normal vs normal) (cancer+normal vs cancer+normal) | 7-92262970 | cg05101437 | (cancer vs cancer) (cancer+normal vs cancer+normal) |
| 3-119529158 | cg24129382 | (normal vs normal) | 8-134511683 | cg01778994 | (cancer vs cancer) (cancer+normal vs cancer+normal) |
| 3-120627406 | cg05293820 | (cancer vs cancer) (normal vs normal) (cancer+normal vs cancer+normal) | 8-54569668 | cg05483021 | (cancer vs cancer) (cancer+normal vs cancer+normal) |

| | | | | | |
|---|---|---|---|---|---|
| 3-122694286 | cg07733481 | (normal vs normal) (cancer+normal vs cancer+normal) | 8-54569817 | cg22973468 | (cancer vs cancer) (cancer+normal vs cancer+normal) |
| 3-147113726 | cg18082337 | (normal vs normal) | 8-54569999 | cg01887995 | (cancer vs cancer) (cancer+normal vs cancer+normal) |
| 3-147126403 | cg16636671 | (normal vs normal) | **Status - Hypo** | | |
| 3-147131178 | cg12204258 | (normal vs normal) | 1-236156917 | cg02454364 | (cancer vs cancer) (cancer+normal vs cancer+normal) |
| 3-183542914 | cg12727374 | (cancer vs cancer) (normal vs normal) (cancer+normal vs cancer+normal) | 1-236157114 | cg07578215 | (cancer vs cancer) (cancer+normal vs cancer+normal) |
| 3-183543587 | cg09738156 | (cancer vs cancer) (normal vs normal) (cancer+normal vs cancer+normal) | 1-236157142 | cg04204188 | (cancer vs cancer) (cancer+normal vs cancer+normal) |
| 3-196466494 | cg07061775 | (normal vs normal) | 1-236157210 | cg06094815 | (cancer vs cancer) (cancer+normal vs cancer+normal) |
| 3-38207809 | cg15789250 | (normal vs normal) | 1-7210826 | cg24513014 | (cancer vs cancer) (cancer+normal vs cancer+normal) |
| 3-45017297 | cg04432377 | (normal vs normal) | 1-94560697 | cg09234454 | (normal vs normal) (cancer vs cancer) (cancer+normal vs cancer+normal) |
| 3-49757134 | cg15865150 | (normal vs normal) | 10-132365700 | cg13253210 | (cancer vs cancer) (cancer+normal vs cancer+normal) |
| 4-115520072 | cg10156125 | (normal vs normal) (cancer+normal vs cancer+normal) | 10-134246660 | cg19448497 | (cancer vs cancer) (cancer+normal vs cancer+normal) |
| 4-2404284 | cg01601518 | (normal vs normal) | 10-134593345 | cg11790551 | (cancer vs cancer) (cancer+normal vs cancer+normal) |
| 4-41869175 | cg12929487 | (normal vs normal) | 10-134593418 | cg13418804 | (cancer vs cancer) (cancer+normal vs cancer+normal) |
| 4-41884669 | cg15264681 | (normal vs normal) | 10-134593496 | cg19895894 | (cancer vs cancer) (cancer+normal vs cancer+normal) |
| 5-112073570 | cg21634602 | (normal vs normal) | 10-134593532 | cg16832975 | (cancer vs cancer) (cancer+normal vs cancer+normal) |
| 5-1174646 | cg11100933 | (normal vs normal) | 10-134593607 | cg07783889 | (cancer vs cancer) (cancer+normal vs cancer+normal) |
| 5-1174666 | cg02124887 | (normal vs normal) (cancer+normal vs cancer+normal) | 10-134996411 | cg04599298 | (cancer vs cancer) (cancer+normal vs cancer+normal) |
| 5-127420616 | cg01159515 | (normal vs normal) | 10-38240973 | cg26684581 | (cancer vs cancer) (cancer+normal vs cancer+normal) |
| 5-140892677 | cg00076195 | (normal vs normal) | 11-120998732 | cg26401732 | (cancer vs cancer) (cancer+normal vs cancer+normal) |
| 5-170763967 | cg07682041 | (normal vs normal) | 11-130781542 | cg05431684 | (cancer vs cancer) (cancer+normal vs |

| | | |
|---|---|---|
| 5-65018933 | cg17679824 | (normal vs normal) |
| 6-27870695 | cg01177470 | (normal vs normal) |
| 6-31627678 | cg15415945 | (normal vs normal) |
| 6-33161328 | cg08481075 | (normal vs normal) |
| 6-33244752 | cg19156220 | (normal vs normal) |
| 6-33244755 | cg17225129 | (cancer vs cancer) (normal vs normal) |
| 7-127225891 | cg02945056 | (normal vs normal) |
| 7-158751184 | cg12744031 | (normal vs normal) |
| 7-27205262 | cg21001184 | (normal vs normal) |
| 8-1729607 | cg09410841 | (normal vs normal) |
| 8-74660125 | cg14195684 | (normal vs normal) |
| 9-102582694 | cg13796392 | (normal vs normal) (cancer+normal vs cancer+normal) |
| 9-112810402 | cg15083233 | (normal vs normal) |
| 9-124461640 | cg27650175 | (normal vs normal) |
| **Status - Hypo** | | |
| 1-147097078 | cg25004385 | (normal vs normal) |
| 1-185380011 | cg26428191 | (normal vs normal) (cancer+normal vs cancer+normal) |
| 1-228607587 | cg18918637 | (normal vs normal) |
| 1-233817629 | cg10175859 | (normal vs normal) (cancer vs cancer) (cancer+normal vs cancer+normal) |
| 1-53195759 | cg22166914 | (normal vs normal) |

| | | |
|---|---|---|
| | | cancer+normal) |
| 11-17591873 | cg07592809 | (cancer vs cancer) (cancer+normal vs cancer+normal) |
| 11-63885740 | cg06268327 | (cancer vs cancer) (cancer+normal vs cancer+normal) |
| 11-63886204 | cg17676132 | (cancer vs cancer) (cancer+normal vs cancer+normal) |
| 11-64108790 | cg16718999 | (cancer vs cancer) (cancer+normal vs cancer+normal) |
| 11-65124394 | cg10925420 | (cancer vs cancer) (cancer+normal vs cancer+normal) |
| 11-69683619 | cg24112091 | (cancer vs cancer) (cancer+normal vs cancer+normal) |
| 11-70289549 | cg08777812 | (cancer vs cancer) (cancer+normal vs cancer+normal) |
| 11-70416309 | cg05401670 | (cancer vs cancer) (cancer+normal vs cancer+normal) |
| 11-70458782 | cg14232870 | (cancer vs cancer) (cancer+normal vs cancer+normal) |
| 11-73685776 | cg04807856 | (cancer vs cancer) (cancer+normal vs cancer+normal) |
| 13-112630656 | cg11423130 | (cancer vs cancer) (cancer+normal vs cancer+normal) |
| 13-113461500 | cg09975219 | (cancer vs cancer) (cancer+normal vs cancer+normal) |
| 13-113491766 | cg02490942 | (cancer vs cancer) (cancer+normal vs cancer+normal) |
| 13-113634407 | cg16341737 | (cancer vs cancer) (cancer+normal vs cancer+normal) |
| 13-113663206 | cg17928152 | (cancer vs cancer) (cancer+normal vs cancer+normal) |
| 13-113696937 | cg02909018 | (cancer vs cancer) (cancer+normal vs cancer+normal) |
| 13-113708289 | cg24746106 | (cancer vs cancer) (cancer+normal vs cancer+normal) |
| 13-113744072 | cg16043368 | (cancer vs cancer) (cancer+normal vs cancer+normal) |
| 13-113744320 | cg16277139 | (cancer vs cancer) (cancer+normal vs cancer+normal) |
| 13-113796215 | cg02617285 | (cancer vs cancer) (cancer+normal vs |

| | | | | | |
|---|---|---|---|---|---|
| | | | | | (cancer+normal) |
| 1-55464084 | cg14969361 | (normal vs normal) | 14-101192634 | cg1368411 5 | (cancer vs cancer) (cancer+normal vs cancer+normal) |
| 1-55501145 | cg22029640 | (normal vs normal) | 14-91806002 | cg0102184 7 | (cancer vs cancer) (cancer+normal vs cancer+normal) |
| 1-6855140 | cg04874074 | (normal vs normal) (cancer vs cancer) (cancer+normal vs cancer+normal) | 15-22560713 | cg0275963 5 | (cancer vs cancer) (cancer+normal vs cancer+normal) |
| 10-134926810 | cg27405177 | (normal vs normal) (cancer vs cancer) | 15-45444508 | cg2400658 2 | (cancer vs cancer) (cancer+normal vs cancer+normal) |
| 10-16566625 | cg19197922 | (normal vs normal) (normal vs normal) (cancer+normal vs cancer+normal) | 15-58429751 | cg0245012 4 | (cancer vs cancer) (cancer+normal vs cancer+normal) |
| 10-22621481 | cg15708219 | (normal vs normal) | 16-31453477 | cg0747219 9 | (cancer vs cancer) (cancer+normal vs cancer+normal) |
| 10-32403029 | cg22534759 | (normal vs normal) | 16-85465268 | cg0217425 2 | (cancer vs cancer) (cancer+normal vs cancer+normal) |
| 10-8487762 | cg08738443 | (normal vs normal) | 16-85465628 | cg1658108 5 | (cancer vs cancer) (cancer+normal vs cancer+normal) |
| 10-88610737 | cg26593228 | (normal vs normal) | 16-87856264 | cg0141241 9 | (cancer vs cancer) (cancer+normal vs cancer+normal) |
| 10-97889373 | cg16289751 | (normal vs normal) | 16-88534585 | cg0793307 8 | (cancer vs cancer) (cancer+normal vs cancer+normal) |
| 11-124539453 | cg18743034 | (normal vs normal) (cancer vs cancer) (cancer+normal vs cancer+normal) | 16-89219472 | cg0544534 8 | (cancer vs cancer) (cancer+normal vs cancer+normal) |
| 11-126611424 | cg22205341 | (normal vs normal) | 16-89219547 | cg0503215 0 | (cancer vs cancer) (cancer+normal vs cancer+normal) |
| 11-22689281 | cg16363146 | (normal vs normal) | 17-1069335 | cg1215983 7 | (cancer vs cancer) (cancer+normal vs cancer+normal) |
| 11-57363669 | cg14311630 | (normal vs normal) | 17-2593076 | cg1943585 9 | (cancer vs cancer) (cancer+normal vs cancer+normal) |
| 11-91717555 | cg23360860 | (normal vs normal) | 17-6024017 | cg0630451 8 | (cancer vs cancer) (cancer+normal vs cancer+normal) |
| 12-132971019 | cg15856066 | (normal vs normal) | 18-13263188 | cg1977289 7 | (cancer vs cancer) (cancer+normal vs cancer+normal) |
| 12-150865 | cg26523196 | (normal vs normal) | 18-76725474 | cg0159385 7 | (cancer vs cancer) (cancer+normal vs cancer+normal) |
| 12-63117540 | cg11497017 | (normal vs normal) | 19-16466480 | cg1770238 8 | (cancer vs cancer) (cancer+normal vs cancer+normal) |
| 12-64317120 | cg17430032 | (normal vs normal) (cancer vs cancer) (cancer+normal vs | 19-16466617 | cg0473822 0 | (cancer vs cancer) (cancer+normal vs cancer+normal) |

| | | cancer+normal) | | | |
|---|---|---|---|---|---|
| 12-65385603 | cg22253541 | (normal vs normal) | 19-4371934 | cg24928938 | (cancer vs cancer) (cancer+normal vs cancer+normal) |
| 13-108087589 | cg23511851 | (normal vs normal) | 19-45262055 | cg11459773 | (cancer vs cancer) (cancer+normal vs cancer+normal) |
| 13-113674587 | cg14219365 | (normal vs normal) | 19-4544446 | cg02715602 | (cancer vs cancer) (cancer+normal vs cancer+normal) |
| 13-114303277 | cg21941987 | (normal vs normal) (cancer vs cancer) (cancer+normal vs cancer+normal) | 2-102867275 | cg05168012 | (cancer vs cancer) (cancer+normal vs cancer+normal) |
| 13-114776704 | cg14984162 | (normal vs normal) | 2-241896967 | cg08200420 | (cancer vs cancer) (cancer+normal vs cancer+normal) |
| 13-32497491 | cg17877237 | (normal vs normal) | 2-72359477 | cg14657116 | (cancer vs cancer) (cancer+normal vs cancer+normal) |
| 13-33754450 | cg22095128 | (normal vs normal) | 2-74375930 | cg09717809 | (normal vs normal) (cancer vs cancer) (cancer+normal vs cancer+normal) |
| 13-35212111 | cg22046318 | (normal vs normal) | 2-8343710 | cg23079012 | (cancer vs cancer) (cancer+normal vs cancer+normal) |
| 14-102481497 | cg14839386 | (normal vs normal) | 20-2308988 | cg20117447 | (cancer vs cancer) (cancer+normal vs cancer+normal) |
| 14-52897968 | cg10146780 | (normal vs normal) | 22-18277981 | cg12923994 | (cancer vs cancer) (cancer+normal vs cancer+normal) |
| 14-64746129 | cg09993711 | (normal vs normal) | 3-123813393 | cg25184248 | (cancer vs cancer) (cancer+normal vs cancer+normal) |
| 14-71250277 | cg26578476 | (normal vs normal) | 3-123813596 | cg10430690 | (cancer vs cancer) (cancer+normal vs cancer+normal) |
| 14-91592793 | cg12167284 | (normal vs normal) | 3-130368239 | cg08850509 | (cancer vs cancer) (cancer+normal vs cancer+normal) |
| 15-42061865 | cg15183258 | (normal vs normal) | 3-196318703 | cg03920544 | (cancer vs cancer) (cancer+normal vs cancer+normal) |
| 15-43560165 | cg14299369 | (normal vs normal) | 3-196318719 | cg12288267 | (cancer vs cancer) (cancer+normal vs cancer+normal) |
| 15-44088809 | cg09938490 | (normal vs normal) (cancer vs cancer) (cancer+normal vs cancer+normal) | 3-196387058 | cg16053281 | (cancer vs cancer) (cancer+normal vs cancer+normal) |
| 15-61889439 | cg23100955 | (normal vs normal) | 4-90168963 | cg12577942 | (cancer vs cancer) (cancer+normal vs cancer+normal) |
| 15-93114062 | cg26296941 | (normal vs normal) | 5-131658161 | cg19848599 | (cancer vs cancer) (cancer+normal vs cancer+normal) |
| 16-6067801 | cg08255211 | (normal vs normal) | 5-13810195 | cg20738735 | (cancer vs cancer) (cancer+normal vs cancer+normal) |

| | | | | | |
|---|---|---|---|---|---|
| 16-87422913 | cg08673532 | (normal vs normal) | 5-13810279 | cg0881882 9 | (cancer vs cancer) (cancer+normal vs cancer+normal) |
| 17-13399070 | cg27260984 | (normal vs normal) | 5-42468836 | cg0876139 6 | (cancer vs cancer) (cancer+normal vs cancer+normal) |
| 17-4225165 | cg23371655 | (normal vs normal) | 6-168378903 | cg0067893 8 | (cancer vs cancer) (cancer+normal vs cancer+normal) |
| 17-56246435 | cg14819820 | (normal vs normal) | 6-169825345 | cg1118169 3 | (cancer vs cancer) (cancer+normal vs cancer+normal) |
| 18-14747082 | cg15231809 | (normal vs normal) | 6-30708009 | cg0512956 8 | (cancer vs cancer) (cancer+normal vs cancer+normal) |
| 18-61368902 | cg14572895 | (normal vs normal) | 6-31592082 | cg0010791 4 | (cancer vs cancer) (cancer+normal vs cancer+normal) |
| 18-72925371 | cg16167782 | (normal vs normal) | 6-42231123 | cg1325649 1 | (cancer vs cancer) (cancer+normal vs cancer+normal) |
| 19-14171165 | cg11437328 | (normal vs normal) (cancer+normal vs cancer+normal) | 7-101755186 | cg0674631 8 | (cancer vs cancer) (cancer+normal vs cancer+normal) |
| 19-54208517 | cg09404289 | (normal vs normal) | 7-157449217 | cg0750659 9 | (cancer vs cancer) (cancer+normal vs cancer+normal) |
| 19-55417390 | cg13518366 | (normal vs normal) | 7-36339044 | cg0273441 9 | (cancer vs cancer) (cancer+normal vs cancer+normal) |
| 2-1051469 | cg14174583 | (normal vs normal) | 7-36585420 | cg1206913 2 | (cancer vs cancer) (cancer+normal vs cancer+normal) |
| 2-109889899 | cg25707877 | (normal vs normal) | 7-56242072 | cg0345677 1 | (cancer vs cancer) (cancer+normal vs cancer+normal) |
| 2-129100899 | cg25020810 | (normal vs normal) | 8-142841083 | cg1777603 3 | (cancer vs cancer) (cancer+normal vs cancer+normal) |
| 2-130831550 | cg27402713 | (normal vs normal) | 8-144872852 | cg0954179 4 | (cancer vs cancer) (cancer+normal vs cancer+normal) |
| 2-134368695 | cg18966810 | (normal vs normal) | 8-144873034 | cg1028231 9 | (cancer vs cancer) (cancer+normal vs cancer+normal) |
| 2-204737672 | cg14288266 | (normal vs normal) | 8-144873183 | cg1843041 1 | (cancer vs cancer) (cancer+normal vs cancer+normal) |
| 2-216252792 | cg19773547 | (normal vs normal) | 8-144958131 | cg0011204 8 | (cancer vs cancer) (cancer+normal vs cancer+normal) |
| 2-221491208 | cg13432007 | (normal vs normal) | 8-43131260 | cg1903525 4 | (cancer vs cancer) (cancer+normal vs cancer+normal) |
| 2-228176991 | cg12267933 | (normal vs normal) (cancer+normal vs cancer+normal) | 8-48676898 | cg1191275 4 | (cancer vs cancer) (cancer+normal vs cancer+normal) |
| 2-39209066 | cg26354721 | (normal vs normal) | 8-49335020 | cg1493836 0 | (cancer vs cancer) (cancer+normal vs cancer+normal) |
| 2- | cg16101804 | (normal vs normal) | 8-49335078 | cg0072602 | (cancer vs cancer) |

| | | |
|---|---|---|
| 71194638 | | |
| 2-81428108 | cg15454811 | (normal vs normal) |
| 2-8997977 | cg14820213 | (normal vs normal) |
| 20-36033352 | cg14637027 | (normal vs normal) (cancer vs cancer) (cancer+normal vs cancer+normal) |
| 21-43242428 | cg19408149 | (normal vs normal) (cancer vs cancer) (cancer+normal vs cancer+normal) |
| 22-41604442 | cg06838028 | (normal vs normal) |
| 3-140992338 | cg18802720 | (normal vs normal) (cancer+normal vs cancer+normal) |
| 3-155838032 | cg14218037 | (normal vs normal) |
| 3-42064950 | cg17224181 | (normal vs normal) |
| 3-53782863 | cg13414750 | (normal vs normal) |
| 3-73436384 | cg25855565 | (normal vs normal) |
| 4-186349449 | cg15185864 | (normal vs normal) |
| 4-567537 | cg14945316 | (normal vs normal) |
| 4-83273357 | cg17837792 | (normal vs normal) |
| 4-96102203 | cg17337917 | (normal vs normal) |
| 5-106606747 | cg26545167 | (normal vs normal) |
| 5-137523911 | cg26927150 | (normal vs normal) |
| 5-166582591 | cg18924771 | (normal vs normal) |
| 5-175545977 | cg09739850 | (normal vs normal) |
| 5-34005493 | cg18689929 | (normal vs normal) |
| 5-94623660 | cg18774611 | (normal vs normal) |
| 6-143891975 | cg25407410 | (normal vs normal) |
| 6-165747945 | cg25641223 | (normal vs normal) |
| 6-1836850 | cg21478123 | (normal vs normal) |

| | | |
|---|---|---|
| | 9 | (cancer+normal vs cancer+normal) |
| 9-140703588 | cg14469972 | (cancer vs cancer) (cancer+normal vs cancer+normal) |
| 9-140703637 | cg12872489 | (cancer vs cancer) (cancer+normal vs cancer+normal) |
| 9-99258799 | cg20960456 | (cancer vs cancer) (cancer+normal vs cancer+normal) |
| 9-99259456 | cg14160020 | (cancer vs cancer) (cancer+normal vs cancer+normal) |
| **PAAD (cancer vs cancer)** | | |
| **Status - Hyper** | | |
| 1-13840381 | cg13788258 | (cancer vs cancer) (cancer vs cancer) |
| 1-13840480 | cg23071120 | (cancer vs cancer) (cancer vs cancer) |
| 1-13840494 | cg26536189 | (cancer vs cancer) (cancer vs cancer) |
| 1-13840612 | cg01144933 | (cancer vs cancer) (cancer vs cancer) |
| 1-182584341 | cg00153856 | (cancer vs cancer) (cancer vs cancer) (cancer+normal vs cancer+normal) |
| 1-182584520 | cg07144296 | (cancer vs cancer) (normal vs normal) (cancer+normal vs cancer+normal) |
| 1-182584578 | cg24085895 | (cancer vs cancer) (normal vs normal) (cancer+normal vs cancer+normal) |
| 1-237207256 | cg01968657 | (cancer vs cancer) |
| 1-28176762 | cg22080472 | (cancer vs cancer) |
| 10-100994381 | cg08529260 | (cancer vs cancer) (cancer+normal vs cancer+normal) |
| 10-132284252 | cg19262683 | (cancer vs cancer) |
| 10-133558786 | cg04492858 | (cancer vs cancer) (cancer vs cancer) |
| 10-133558893 | cg20568497 | (cancer vs cancer) |
| 10-133558971 | cg23460707 | (cancer vs cancer) (cancer vs cancer) |
| 10-43572621 | cg17339505 | (cancer vs cancer) |
| 11-2291652 | cg18485844 | (cancer vs cancer) |
| 11-2291677 | cg2234612 | (cancer vs cancer) |

| | | | | | |
|---|---|---|---|---|---|
| | | | | 4 | |
| 6-33284748 | cg13598232 | (normal vs normal) | 11-35440525 | cg0901717 4 | (cancer vs cancer) (cancer+normal vs cancer+normal) |
| 7-134916876 | cg26502514 | (normal vs normal) | 11-35441088 | cg2698566 6 | (cancer vs cancer) |
| 7-147709862 | cg22807241 | (normal vs normal) | 11-49974622 | cg1638652 3 | (cancer vs cancer) (cancer+normal vs cancer+normal) |
| 7-33894296 | cg24249450 | (normal vs normal) | 11-55593465 | cg2166799 8 | (cancer vs cancer) |
| 7-33945948 | cg26347222 | (normal vs normal) | 11-615950 | cg1406777 0 | (cancer vs cancer) |
| 7-34193859 | cg15331578 | (normal vs normal) | 11-616089 | cg1337828 4 | (cancer vs cancer) (cancer+normal vs cancer+normal) |
| 7-56517941 | cg22296322 | (normal vs normal) | 12-115122028 | cg0833077 8 | (cancer vs cancer) |
| 7-98945891 | cg24791283 | (normal vs normal) | 12-115122157 | cg2279773 5 | (cancer vs cancer) |
| 8-11189650 | cg21484512 | (normal vs normal) | 12-115122304 | cg0049947 5 | (cancer vs cancer) |
| 8-121166405 | cg22335954 | (normal vs normal) | 12-125383138 | cg0630046 9 | (cancer vs cancer) |
| 8-139750054 | cg18842396 | (normal vs normal) | 12-130498701 | cg1465772 8 | (cancer vs cancer) |
| 8-35186363 | cg18760194 | (normal vs normal) | 12-4274034 | cg0680796 6 | (cancer vs cancer) (cancer vs cancer) (normal vs normal) (cancer+normal vs cancer+normal) |
| 8-56788346 | cg17217741 | (normal vs normal) | 12-4274070 | cg0887391 2 | (cancer vs cancer) (cancer+normal vs cancer+normal) |
| **COAD_READ (cancer vs cancer)** | | | 12-85430174 | cg2008007 9 | (cancer vs cancer) |
| **Status - Hyper** | | | 12-85430215 | cg2634597 1 | (cancer vs cancer) |
| 1-120837955 | cg02905065 | (cancer vs cancer) (cancer+normal vs cancer+normal) | 13-32605218 | cg1615886 3 | (cancer vs cancer) (cancer+normal vs cancer+normal) |
| 1-120838320 | cg07784526 | (cancer vs cancer) (cancer+normal vs cancer+normal) | 13-32605406 | cg2221925 4 | (cancer vs cancer) (cancer vs cancer) (normal vs normal) (cancer+normal vs cancer+normal) |
| 1-120838323 | cg18948743 | (cancer vs cancer) (cancer+normal vs cancer+normal) | 13-32605611 | cg1694165 6 | (cancer vs cancer) (cancer vs cancer) (normal vs normal) (cancer+normal vs cancer+normal) |
| 1-143913552 | cg22710840 | (cancer vs cancer) (cancer+normal vs cancer+normal) | 13-42032666 | cg1849568 2 | (cancer vs cancer) |
| 1-150254477 | cg11207983 | (cancer vs cancer) (cancer+normal vs cancer+normal) | 14-105714589 | cg2303481 8 | (cancer vs cancer) (cancer vs cancer) (normal vs normal) (cancer+normal vs cancer+normal) |
| 1-150254546 | cg06982544 | (cancer vs cancer) (cancer+normal vs | 14-105714833 | cg1547436 7 | (cancer vs cancer) (cancer vs cancer) |

| | | | | | |
|---|---|---|---|---|---|
| | | cancer+normal) | | | (normal vs normal) (cancer+normal vs cancer+normal) |
| 1-150254869 | cg10055771 | (cancer vs cancer) (cancer+normal vs cancer+normal) | 14-105714843 | cg16561543 | (cancer vs cancer) (cancer vs cancer) (normal vs normal) (cancer+normal vs cancer+normal) |
| 1-150266311 | cg18031008 | (cancer vs cancer) (cancer+normal vs cancer+normal) | 14-105715025 | cg01237565 | (cancer vs cancer) (cancer vs cancer) (normal vs normal) (cancer+normal vs cancer+normal) |
| 1-206137054 | cg00552973 | (cancer vs cancer) | 14-37131555 | cg22968936 | (cancer vs cancer) (cancer vs cancer) |
| 10-101088828 | cg13692476 | (cancer vs cancer) | 14-37131601 | cg19032066 | (cancer vs cancer) |
| 10-93392836 | cg00197495 | (cancer vs cancer) (cancer+normal vs cancer+normal) | 14-38057752 | cg26435698 | (cancer vs cancer) (cancer+normal vs cancer+normal) |
| 10-93393030 | cg12486537 | (cancer vs cancer) (cancer+normal vs cancer+normal) | 14-72943461 | cg18515872 | (cancer vs cancer) (cancer+normal vs cancer+normal) |
| 11-94473668 | cg26963029 | (cancer vs cancer) (cancer+normal vs cancer+normal) | 14-84173031 | cg24749311 | (cancer vs cancer) |
| 11-94501718 | cg11117364 | (cancer vs cancer) (cancer+normal vs cancer+normal) | 15-74532450 | cg02430430 | (cancer vs cancer) (cancer vs cancer) |
| 11-94502388 | cg23628232 | (cancer vs cancer) (cancer+normal vs cancer+normal) | 16-1202468 | cg16427096 | (cancer vs cancer) (normal vs normal) (cancer+normal vs cancer+normal) |
| 11-94502524 | cg20493960 | (cancer vs cancer) (cancer+normal vs cancer+normal) | 16-1202819 | cg00609966 | (cancer vs cancer) (cancer vs cancer) |
| 12-15374303 | cg24979348 | (cancer vs cancer) | 16-86231823 | cg27299647 | (cancer vs cancer) (cancer+normal vs cancer+normal) |
| 12-15374457 | cg25283609 | (cancer vs cancer) | 16-86231948 | cg07989221 | (cancer vs cancer) (cancer vs cancer) (normal vs normal) (cancer+normal vs cancer+normal) |
| 12-54333587 | cg15611413 | (cancer vs cancer) (cancer+normal vs cancer+normal) | 16-86232315 | cg07282058 | (cancer vs cancer) |
| 12-54519825 | cg21161136 | (cancer vs cancer) (cancer+normal vs cancer+normal) | 16-86232457 | cg26827987 | (cancer vs cancer) (cancer+normal vs cancer+normal) |
| 12-57998762 | cg11654179 | (cancer vs cancer) | 16-86321899 | cg08927145 | (cancer vs cancer) (cancer+normal vs cancer+normal) |
| 13-26626134 | cg20193324 | (cancer vs cancer) (cancer+normal vs cancer+normal) | 16-89789362 | cg04044418 | (cancer vs cancer) |
| 13-95620306 | cg25264081 | (cancer vs cancer) (cancer vs cancer) | 17-18266135 | cg26763362 | (cancer vs cancer) (cancer vs cancer) |
| 15-45670068 | cg05280133 | (cancer vs cancer) (cancer+normal vs cancer+normal) | 17-47210270 | cg16206138 | (cancer vs cancer) |

| | | | | | |
|---|---|---|---|---|---|
| 15-45670478 | cg11431346 | (cancer vs cancer) (cancer+normal vs cancer+normal) | 19-1766358 | cg15086113 | (cancer vs cancer) |
| 15-45670526 | cg01145430 | (cancer vs cancer) (cancer+normal vs cancer+normal) | 19-1768630 | cg22874255 | (cancer vs cancer) |
| 16-10276580 | cg09239744 | (cancer vs cancer) | 19-34359847 | cg20439030 | (cancer vs cancer) |
| 16-15528758 | cg03210180 | (cancer vs cancer) | 19-5294093 | cg14725952 | (cancer vs cancer) |
| 16-58019123 | cg04462627 | (cancer vs cancer) | 19-54850619 | cg20542190 | (cancer vs cancer) |
| 17-33775394 | cg22588307 | (cancer vs cancer) (cancer+normal vs cancer+normal) | 2-102804405 | cg01376829 | (cancer vs cancer) (cancer vs cancer) (cancer+normal vs cancer+normal) |
| 17-46124821 | cg06740897 | (cancer vs cancer) (cancer+normal vs cancer+normal) | 2-102804576 | cg00674896 | (cancer vs cancer) (cancer vs cancer) (cancer+normal vs cancer+normal) |
| 17-881949 | cg23513567 | (cancer vs cancer) (cancer+normal vs cancer+normal) | 2-215275838 | cg15125763 | (cancer vs cancer) (cancer vs cancer) |
| 17-882342 | cg13966567 | (cancer vs cancer) (cancer+normal vs cancer+normal) | 2-215276156 | cg06580419 | (cancer vs cancer) (cancer+normal vs cancer+normal) |
| 19-14016729 | cg09479389 | (cancer vs cancer) (normal vs normal) (cancer+normal vs cancer+normal) | 2-233368263 | cg05137450 | (cancer vs cancer) (cancer+normal vs cancer+normal) |
| 19-3869172 | cg15823845 | (cancer vs cancer) | 20-55204266 | cg24581326 | (cancer vs cancer) |
| 19-5293083 | cg16733654 | (cancer vs cancer) (cancer+normal vs cancer+normal) | 20-55204351 | cg19694200 | (cancer vs cancer) |
| 19-57831632 | cg00846300 | (cancer vs cancer) (cancer+normal vs cancer+normal) | 20-55204593 | cg12521353 | (cancer vs cancer) |
| 19-57831662 | cg19533530 | (cancer vs cancer) | 20-55204633 | cg05013064 | (cancer vs cancer) |
| 19-57831678 | cg23986470 | (cancer vs cancer) (cancer+normal vs cancer+normal) | 20-55204986 | cg26736540 | (cancer vs cancer) |
| 19-57831816 | cg14786398 | (cancer vs cancer) | 21-47063408 | cg19320275 | (cancer vs cancer) |
| 19-57831819 | cg01325460 | (cancer vs cancer) | 3-123603311 | cg07802917 | (cancer vs cancer) |
| 19-57862480 | cg17849956 | (cancer vs cancer) | 3-169489583 | cg27143246 | (cancer vs cancer) |
| 19-57862612 | cg13788592 | (cancer vs cancer) | 4-187002617 | cg14827929 | (cancer vs cancer) (cancer+normal vs cancer+normal) |
| 19-57862627 | cg16848116 | (cancer vs cancer) (cancer+normal vs cancer+normal) | 4-4858059 | cg08576208 | (cancer vs cancer) |
| 19-57862638 | cg21627760 | (cancer vs cancer) (cancer+normal vs cancer+normal) | 4-4858340 | cg06688323 | (cancer vs cancer) |
| 19-57862713 | cg07494047 | (cancer vs cancer) (cancer+normal vs cancer+normal) | 4-4858482 | cg13332538 | (cancer vs cancer) |

| | | | | | |
|---|---|---|---|---|---|
| 2-100938799 | cg23977631 | (cancer vs cancer) (cancer+normal vs cancer+normal) | 4-4858598 | cg05600350 | (cancer vs cancer) (cancer vs cancer) |
| 2-100938813 | cg14997226 | (cancer vs cancer) | 4-4858752 | cg03189184 | (cancer vs cancer) |
| 2-144695257 | cg09667303 | (cancer vs cancer) (cancer vs cancer) | 4-60001000 | cg11281224 | (cancer vs cancer) (cancer+normal vs cancer+normal) |
| 2-158454110 | cg18061904 | (cancer vs cancer) (normal vs normal) (cancer+normal vs cancer+normal) | 5-151304312 | cg12995800 | (cancer vs cancer) |
| 2-29339076 | cg21972382 | (cancer vs cancer) (cancer vs cancer) (cancer+normal vs cancer+normal) | 6-17600994 | cg10154826 | (cancer vs cancer) |
| 2-29339091 | cg05038216 | (cancer vs cancer) (cancer vs cancer) (cancer+normal vs cancer+normal) | 6-19838817 | cg24109612 | (cancer vs cancer) |
| 2-66803033 | cg02764245 | (cancer vs cancer) (cancer+normal vs cancer+normal) | 7-139167960 | cg26663651 | (cancer vs cancer) |
| 20-23029806 | cg24562819 | (cancer vs cancer) | 7-139168305 | cg08528826 | (cancer vs cancer) |
| 20-23030250 | cg09279949 | (cancer vs cancer) (cancer vs cancer) | 7-139168481 | cg24956250 | (cancer vs cancer) (cancer vs cancer) (cancer+normal vs cancer+normal) |
| 20-23030442 | cg25397597 | (cancer vs cancer) | 7-139168484 | cg15506157 | (cancer vs cancer) (normal vs normal) |
| 20-23030446 | cg22152407 | (cancer vs cancer) (cancer+normal vs cancer+normal) | 7-139168487 | cg00919016 | (cancer vs cancer) |
| 20-23031246 | cg10185099 | (cancer vs cancer) | 7-139168503 | cg00699934 | (cancer vs cancer) (cancer+normal vs cancer+normal) |
| 20-3389408 | cg13516746 | (cancer vs cancer) (cancer+normal vs cancer+normal) | 7-139168523 | cg05224190 | (cancer vs cancer) |
| 20-3389535 | cg20681068 | (cancer vs cancer) (cancer+normal vs cancer+normal) | 7-139168659 | cg25181507 | (cancer vs cancer) (normal vs normal) (cancer+normal vs cancer+normal) |
| 20-61050914 | cg16919517 | (cancer vs cancer) | 7-156811362 | cg10108372 | (cancer vs cancer) (cancer+normal vs cancer+normal) |
| 20-61051432 | cg08568720 | (cancer vs cancer) | 7-156812104 | cg09595202 | (cancer vs cancer) (cancer vs cancer) (cancer+normal vs cancer+normal) |
| 22-44258572 | cg15471953 | (cancer vs cancer) | 7-156814845 | cg02570079 | (cancer vs cancer) (cancer vs cancer) (normal vs normal) (cancer+normal vs cancer+normal) |
| 3-48700027 | cg15511738 | (cancer vs cancer) (cancer+normal vs cancer+normal) | 7-156815030 | cg20235658 | (cancer vs cancer) (cancer+normal vs cancer+normal) |
| 4-111553161 | cg19602081 | (cancer vs cancer) (cancer+normal vs | 7-157643037 | cg22216157 | (cancer vs cancer) |

| | | | | | | |
|---|---|---|---|---|---|---|
| | | cancer+normal) | 7-20825634 | cg16544956 | (cancer vs cancer) | |
| 4-30722781 | cg24291087 | (cancer vs cancer) | 7-25894793 | cg02067688 | (cancer vs cancer) (cancer vs cancer) | |
| 4-55991860 | cg00989765 | (cancer vs cancer) (cancer vs cancer) | 7-56553285 | cg21833728 | (cancer vs cancer) | |
| 5-142785258 | cg13648501 | (cancer vs cancer) | **Status – Hypo** | | | |
| 5-146258195 | cg09528265 | (cancer vs cancer) (cancer+normal vs cancer+normal) | 1-10699604 | cg01824138 | (cancer vs cancer) | |
| 5-146258237 | cg11826826 | (cancer vs cancer) | 1-11561908 | cg13863764 | (cancer vs cancer) (cancer vs cancer) | |
| 5-146258427 | cg25149751 | (cancer vs cancer) | 1-1374310 | cg13897675 | (cancer vs cancer) (cancer vs cancer) | |
| 5-31855185 | cg26880493 | (cancer vs cancer) (cancer+normal vs cancer+normal) | 1-1374669 | cg19639479 | (cancer vs cancer) (cancer vs cancer) | |
| 5-38556223 | cg08392199 | (cancer vs cancer) (cancer+normal vs cancer+normal) | 1-203098067 | cg24408776 | (cancer vs cancer) (cancer vs cancer) (cancer+normal vs cancer+normal) | |
| 5-38556435 | cg12587766 | (cancer vs cancer) (cancer+normal vs cancer+normal) | 1-2205902 | cg24579224 | (cancer vs cancer) | |
| 5-38557085 | cg18174928 | (cancer vs cancer) (cancer+normal vs cancer+normal) | 1-8292657 | cg07021405 | (cancer vs cancer) | |
| 5-38557143 | cg03723506 | (cancer vs cancer) (cancer+normal vs cancer+normal) | 10-134231517 | cg03860038 | (cancer vs cancer) | |
| 5-38557162 | cg12602374 | (cancer vs cancer) | 10-134499505 | cg06139099 | (cancer vs cancer) (cancer vs cancer) | |
| 5-38557386 | cg18848688 | (cancer vs cancer) | 10-134499528 | cg00458454 | (cancer vs cancer) (cancer vs cancer) (cancer+normal vs cancer+normal) | |
| 5-38846100 | cg17528648 | (cancer vs cancer) (cancer+normal vs cancer+normal) | 10-134499734 | cg13441142 | (normal vs normal) (cancer vs cancer) (cancer vs cancer) (cancer+normal vs cancer+normal) | |
| 6-72130209 | cg10039188 | (cancer vs cancer) (cancer+normal vs cancer+normal) | 10-134499752 | cg02541125 | (cancer vs cancer) (cancer vs cancer) (cancer+normal vs cancer+normal) | |
| 6-72130407 | cg25210451 | (cancer vs cancer) (cancer+normal vs cancer+normal) | 10-134567188 | cg15216078 | (cancer vs cancer) (cancer+normal vs cancer+normal) | |
| 6-72130432 | cg15045441 | (cancer vs cancer) (cancer+normal vs cancer+normal) | 10-27703336 | cg00632657 | (cancer vs cancer) (cancer+normal vs cancer+normal) | |
| 6-72130539 | cg26162616 | (cancer vs cancer) (cancer+normal vs cancer+normal) | 10-49647167 | cg08866557 | (cancer vs cancer) (cancer vs cancer) | |
| 6-72130553 | cg22300282 | (cancer vs cancer) (cancer+normal vs cancer+normal) | 10-712032 | cg26311782 | (cancer vs cancer) | |
| 6-72130689 | cg25141674 | (cancer vs cancer) (cancer+normal vs cancer+normal) | 11-46374124 | cg12087471 | (cancer vs cancer) (cancer+normal vs | |
| 6-72130742 | cg24772267 | (cancer vs cancer) | | | | |

202

| | | | | | |
|---|---|---|---|---|---|
| | | | | | cancer+normal) |
| 6-72130755 | cg00920327 | (cancer vs cancer) (cancer+normal vs cancer+normal) | 11-47372949 | cg06168166 | (cancer vs cancer) (cancer vs cancer) (cancer+normal vs cancer+normal) |
| 7-149411498 | cg23778596 | (cancer vs cancer) (cancer+normal vs cancer+normal) | 11-47372960 | cg10354134 | (cancer vs cancer) (cancer vs cancer) (cancer+normal vs cancer+normal) |
| 7-149411652 | cg08131100 | (cancer vs cancer) (cancer+normal vs cancer+normal) | 12-109889431 | cg07719172 | (cancer vs cancer) |
| 7-149412290 | cg03788131 | (cancer vs cancer) (cancer+normal vs cancer+normal) | 12-119592094 | cg26829101 | (cancer vs cancer) |
| 8-11204681 | cg03192020 | (cancer vs cancer) | 12-124718401 | cg02353048 | (cancer vs cancer) |
| 8-11205155 | cg13165109 | (cancer vs cancer) | 12-125034283 | cg24847366 | (cancer vs cancer) |
| 8-11205166 | cg05362548 | (cancer vs cancer) | 12-133159752 | cg25130710 | (cancer vs cancer) |
| 8-11205247 | cg14900984 | (cancer vs cancer) | 13-101167901 | cg13731800 | (cancer vs cancer) (cancer vs cancer) |
| 8-48100410 | cg05746795 | (cancer vs cancer) | 13-111330434 | cg20549079 | (cancer vs cancer) |
| 9-124132393 | cg14632002 | (cancer vs cancer) (cancer+normal vs cancer+normal) | 14-104190829 | cg14977938 | (cancer vs cancer) (cancer+normal vs cancer+normal) |
| 9-137299436 | cg14654324 | (cancer vs cancer) (cancer+normal vs cancer+normal) | 14-45365651 | cg01523474 | (normal vs normal) (cancer vs cancer) (cancer vs cancer) (cancer+normal vs cancer+normal) |
| 9-707166 | cg14399863 | (cancer vs cancer) (normal vs normal) (cancer+normal vs cancer+normal) | 14-91770145 | cg23138608 | (cancer vs cancer) (cancer vs cancer) |
| 9-707387 | cg13877502 | (cancer vs cancer) (cancer vs cancer) (normal vs normal) (cancer+normal vs cancer+normal) | 14-91770229 | cg19465605 | (cancer vs cancer) (cancer vs cancer) |
| 9-707410 | cg03358592 | (cancer vs cancer) (normal vs normal) (cancer+normal vs cancer+normal) | 15-102157726 | cg11484721 | (cancer vs cancer) (cancer+normal vs cancer+normal) |
| **Status - Hypo** | | | 15-34657364 | cg23441018 | (cancer vs cancer) (cancer vs cancer) |
| 1-1095607 | cg02971920 | (normal vs normal) (cancer vs cancer) (cancer vs cancer) (cancer+normal vs cancer+normal) | 16-12061715 | cg09319797 | (normal vs normal) (cancer vs cancer) (cancer+normal vs cancer+normal) |
| 1-167059365 | cg06665322 | (normal vs normal) (cancer vs cancer) (cancer+normal vs cancer+normal) | 16-1578807 | cg05308792 | (cancer vs cancer) (cancer vs cancer) |
| 1-17898874 | cg24600987 | (normal vs normal) (cancer vs cancer) (cancer vs cancer) (cancer+normal vs | 16-3345604 | cg08555866 | (cancer vs cancer) (cancer+normal vs cancer+normal) |

| | | | | | | |
|---|---|---|---|---|---|---|
| | | cancer+normal) | 16-47047897 | cg0300186 7 | (cancer vs cancer) |
| 1-2003605 | cg22332339 | (cancer vs cancer) | | | |
| 1-206593025 | cg13487476 | (cancer vs cancer) (cancer+normal vs cancer+normal) | 16-47048004 | cg0374367 8 | (cancer vs cancer) (cancer+normal vs cancer+normal) |
| 1-220132091 | cg05230389 | (cancer vs cancer) (cancer+normal vs cancer+normal) | 16-50707467 | cg2672407 0 | (cancer vs cancer) |
| 1-220132097 | cg10576232 | (cancer vs cancer) (cancer+normal vs cancer+normal) | 16-77952576 | cg0253522 3 | (cancer vs cancer) |
| 1-230257067 | cg01554316 | (cancer vs cancer) (cancer+normal vs cancer+normal) | 16-80654763 | cg0088833 6 | (cancer vs cancer) |
| 1-54723213 | cg22537280 | (cancer vs cancer) (cancer vs cancer) (cancer+normal vs cancer+normal) | 16-88538355 | cg1019572 6 | (cancer vs cancer) |
| 1-54723426 | cg00838829 | (cancer vs cancer) | 16-88540175 | cg1662735 8 | (cancer vs cancer) (cancer+normal vs cancer+normal) |
| 1-9339683 | cg20700740 | (cancer vs cancer) | 16-88540241 | cg0894016 9 | (cancer vs cancer) |
| 10-518370 | cg26718707 | (cancer vs cancer) (cancer vs cancer) (cancer+normal vs cancer+normal) | 16-88564366 | cg0501267 6 | (cancer vs cancer) |
| 11-119609303 | cg09112054 | (cancer vs cancer) | 16-89381904 | cg2718379 1 | (cancer vs cancer) |
| 11-130060459 | cg11069338 | (normal vs normal) (cancer vs cancer) (cancer vs cancer) (cancer+normal vs cancer+normal) | 17-40996565 | cg0878325 3 | (cancer vs cancer) (cancer+normal vs cancer+normal) |
| 11-2287734 | cg14907310 | (cancer vs cancer) (cancer vs cancer) (cancer+normal vs cancer+normal) | 17-55015826 | cg1167967 3 | (cancer vs cancer) |
| 11-76265321 | cg17373759 | (cancer vs cancer) (cancer+normal vs cancer+normal) | 17-62120439 | cg1717276 1 | (cancer vs cancer) (cancer+normal vs cancer+normal) |
| 11-94275751 | cg24256772 | (cancer vs cancer) (cancer vs cancer) | 17-77948884 | cg1979087 7 | (cancer vs cancer) (cancer+normal vs cancer+normal) |
| 12-109885015 | cg24961970 | (cancer vs cancer) (cancer+normal vs cancer+normal) | 18-77623598 | cg0368211 2 | (cancer vs cancer) (cancer+normal vs cancer+normal) |
| 12-121203948 | cg02419362 | (cancer vs cancer) (cancer+normal vs cancer+normal) | 19-17444421 | cg2004233 8 | (cancer vs cancer) |
| 12-14849268 | cg18754342 | (normal vs normal) (cancer vs cancer) (cancer vs cancer) | 19-50117794 | cg1405565 5 | (cancer vs cancer) |
| 12-50476169 | cg21235960 | (cancer vs cancer) | 19-51607432 | cg0887394 0 | (cancer vs cancer) (cancer vs cancer) |
| 13-52735075 | cg19524009 | (cancer vs cancer) (cancer+normal vs cancer+normal) | 2-201342647 | cg2474666 6 | (cancer vs cancer) |
| 16-71755733 | cg05028274 | (cancer vs cancer) (cancer+normal vs | 2-240234604 | cg0810730 8 | (cancer vs cancer) |

204

| | | cancer+normal) | | | |
|---|---|---|---|---|---|
| 16-78233495 | cg07747924 | (cancer vs cancer) (cancer+normal vs cancer+normal) | 2-241290895 | cg04003871 | (cancer vs cancer) (cancer vs cancer) (cancer+normal vs cancer+normal) |
| 16-89169289 | cg10398761 | (normal vs normal) (cancer vs cancer) (cancer vs cancer) (cancer+normal vs cancer+normal) | 2-43252424 | cg15865511 | (cancer vs cancer) |
| 17-1412449 | cg23346781 | (cancer vs cancer) (cancer vs cancer) (cancer+normal vs cancer+normal) | 2-692026 | cg13650504 | (cancer vs cancer) |
| 17-26127537 | cg07704981 | (cancer vs cancer) | 2-7164527 | cg08182975 | (normal vs normal) (cancer vs cancer) (cancer vs cancer) (cancer+normal vs cancer+normal) |
| 17-4042717 | cg11542336 | (cancer vs cancer) (cancer vs cancer) (cancer+normal vs cancer+normal) | 2-7164586 | cg20995753 | (normal vs normal) (cancer vs cancer) (cancer vs cancer) (cancer+normal vs cancer+normal) |
| 17-70256462 | cg06909248 | (normal vs normal) (cancer vs cancer) (cancer vs cancer) (cancer+normal vs cancer+normal) | 2-9388337 | cg00230766 | (cancer vs cancer) (cancer vs cancer) (cancer+normal vs cancer+normal) |
| 17-77075025 | cg21088108 | (cancer vs cancer) (cancer+normal vs cancer+normal) | 22-50616420 | cg07895657 | (cancer vs cancer) |
| 17-79224306 | cg22462714 | (normal vs normal) (cancer vs cancer) (cancer+normal vs cancer+normal) | 3-141161876 | cg10532384 | (cancer vs cancer) |
| 17-80535398 | cg16366685 | (normal vs normal) (cancer vs cancer) (cancer vs cancer) (cancer+normal vs cancer+normal) | 3-194868750 | cg21937377 | (normal vs normal) (cancer vs cancer) (cancer vs cancer) (cancer+normal vs cancer+normal) |
| 17-80535428 | cg03454705 | (normal vs normal) (cancer vs cancer) (cancer vs cancer) (cancer+normal vs cancer+normal) | 3-194868790 | cg16306870 | (cancer vs cancer) |
| 18-46288005 | cg00137385 | (normal vs normal) (cancer vs cancer) (cancer+normal vs cancer+normal) | 3-50287909 | cg21028562 | (cancer vs cancer) |
| 19-2811654 | cg17741192 | (cancer vs cancer) (cancer+normal vs cancer+normal) | 3-55508460 | cg17553300 | (cancer vs cancer) |
| 19-39306244 | cg12028674 | (normal vs normal) (cancer vs cancer) (cancer vs cancer) (cancer+normal vs cancer+normal) | 3-55508585 | cg10701252 | (cancer vs cancer) |
| 19-4174165 | cg01983454 | (cancer vs cancer) | 3-55508686 | cg06311863 | (cancer vs cancer) (cancer vs cancer) |
| 2- | cg24419520 | (normal vs normal) | 3-55517101 | cg0358899 | (cancer vs cancer) |

| | | | | | |
|---|---|---|---|---|---|
| 106959257 | | (cancer vs cancer)<br>(cancer vs cancer)<br>(cancer+normal vs cancer+normal) | | 8 | |
| 2-106959384 | cg13656404 | (cancer vs cancer)<br>(cancer+normal vs cancer+normal) | 4-1646179 | cg27571057 | (cancer vs cancer)<br>(cancer vs cancer)<br>(cancer+normal vs cancer+normal) |
| 2-11245407 | cg00049868 | (cancer vs cancer) | 4-1800284 | cg24979233 | (cancer vs cancer) |
| 2-112808038 | cg25890678 | (cancer vs cancer)<br>(cancer vs cancer) | 5-42756851 | cg23688479 | (cancer vs cancer)<br>(cancer vs cancer)<br>(cancer+normal vs cancer+normal) |
| 2-11917623 | cg10142874 | (cancer vs cancer) | 5-42756876 | cg12585923 | (cancer vs cancer)<br>(cancer+normal vs cancer+normal) |
| 2-17830624 | cg12793924 | (normal vs normal)<br>(cancer vs cancer)<br>(cancer vs cancer)<br>(cancer+normal vs cancer+normal) | 5-42757171 | cg21929564 | (cancer vs cancer)<br>(cancer+normal vs cancer+normal) |
| 2-227312417 | cg03426602 | (cancer vs cancer)<br>(cancer vs cancer)<br>(cancer+normal vs cancer+normal) | 5-72922957 | cg00081602 | (cancer vs cancer) |
| 2-234526077 | cg18313132 | (cancer vs cancer)<br>(cancer+normal vs cancer+normal) | 6-31111076 | cg02652369 | (cancer vs cancer) |
| 2-240181931 | cg19286774 | (cancer vs cancer) | 6-31111088 | cg03130962 | (cancer vs cancer) |
| 2-242735324 | cg00529371 | (normal vs normal)<br>(cancer vs cancer)<br>(cancer+normal vs cancer+normal) | 6-3324467 | cg16887070 | (cancer vs cancer) |
| 2-55366440 | cg21913376 | (cancer vs cancer) | 6-3723112 | cg19117070 | (cancer vs cancer) |
| 2-8850020 | cg00240378 | (normal vs normal)<br>(cancer vs cancer)<br>(cancer vs cancer)<br>(cancer+normal vs cancer+normal) | 7-101596218 | cg08116711 | (cancer vs cancer) |
| 2-97427975 | cg11464842 | (normal vs normal)<br>(cancer vs cancer)<br>(cancer vs cancer)<br>(cancer+normal vs cancer+normal) | 7-101596340 | cg23261443 | (cancer vs cancer) |
| 20-55959405 | cg25225756 | (normal vs normal)<br>(cancer vs cancer)<br>(cancer vs cancer)<br>(cancer+normal vs cancer+normal) | 7-101943788 | cg27195676 | (cancer vs cancer) |
| 20-55959549 | cg22618720 | (cancer vs cancer)<br>(cancer vs cancer)<br>(cancer+normal vs cancer+normal) | 7-1117418 | cg20934096 | (cancer vs cancer)<br>(cancer vs cancer)<br>(cancer+normal vs cancer+normal) |
| 21-27372387 | cg24168308 | (cancer vs cancer)<br>(cancer+normal vs cancer+normal) | 7-1117540 | cg08137085 | (cancer vs cancer)<br>(cancer vs cancer)<br>(cancer+normal vs cancer+normal) |
| 21- | cg11321156 | (cancer vs cancer) | 7-1970923 | cg2418991 | (cancer vs cancer) |

| | | | | | |
|---|---|---|---|---|---|
| 27372396 | | (cancer+normal vs cancer+normal) | | 7 | |
| 21-27372446 | cg23269692 | (cancer vs cancer) (cancer+normal vs cancer+normal) | 7-2609648 | cg13457496 | (cancer vs cancer) (cancer vs cancer) |
| 21-27372461 | cg18274664 | (cancer vs cancer) (cancer+normal vs cancer+normal) | 7-2609861 | cg09159452 | (cancer vs cancer) |
| 21-45360804 | cg05957544 | (cancer vs cancer) (cancer+normal vs cancer+normal) | 7-44187310 | cg23381646 | (cancer vs cancer) |
| 21-46269191 | cg08823554 | (cancer vs cancer) (cancer+normal vs cancer+normal) | 7-44526856 | cg13301931 | (cancer vs cancer) (cancer vs cancer) |
| 22-31062286 | cg01274916 | (cancer vs cancer) (cancer vs cancer) (cancer+normal vs cancer+normal) | 7-4806755 | cg06114605 | (cancer vs cancer) (cancer vs cancer) (cancer+normal vs cancer+normal) |
| 3-150238208 | cg27024386 | (cancer vs cancer) | 7-4806949 | cg25330264 | (cancer vs cancer) (cancer vs cancer) (cancer+normal vs cancer+normal) |
| 3-150238257 | cg02504622 | (cancer vs cancer) (cancer vs cancer) (cancer+normal vs cancer+normal) | 7-97841864 | cg22884541 | (normal vs normal) (cancer vs cancer) (cancer+normal vs cancer+normal) |
| 3-171412917 | cg09433558 | (cancer vs cancer) (cancer vs cancer) (cancer+normal vs cancer+normal) | 7-97875293 | cg18929814 | (cancer vs cancer) (cancer vs cancer) (cancer+normal vs cancer+normal) |
| 3-55525967 | cg24158324 | (cancer vs cancer) (cancer+normal vs cancer+normal) | 7-97875333 | cg18563899 | (cancer vs cancer) (cancer vs cancer) (cancer+normal vs cancer+normal) |
| 3-9820588 | cg15771477 | (cancer vs cancer) (cancer+normal vs cancer+normal) | 8-145163102 | cg14631276 | (cancer vs cancer) |
| 4-144275126 | cg12710519 | (cancer vs cancer) (cancer+normal vs cancer+normal) | 8-22079330 | cg04011470 | (cancer vs cancer) |
| 4-184017790 | cg00268149 | (cancer vs cancer) (cancer+normal vs cancer+normal) | 8-28929168 | cg19888654 | (cancer vs cancer) |
| 4-187126114 | cg23442198 | (normal vs normal) (cancer vs cancer) (cancer vs cancer) (cancer+normal vs cancer+normal) | 8-30334348 | cg09272186 | (cancer vs cancer) (cancer+normal vs cancer+normal) |
| 4-187629328 | cg21399040 | (normal vs normal) (cancer vs cancer) (cancer vs cancer) (cancer+normal vs cancer+normal) | 8-9040449 | cg03078239 | (cancer vs cancer) |
| 4-187629336 | cg24820270 | (normal vs normal) (cancer vs cancer) (cancer vs cancer) (cancer+normal vs cancer+normal) | **(cancer+normal vs cancer+normal)** | | |
| 4-187629339 | cg01040779 | (normal vs normal) (cancer vs cancer) (cancer+normal vs | **Status - Hyper** | | |

| | | | | | |
|---|---|---|---|---|---|
| | | cancer+normal) | | | |
| 4-187629387 | cg25352342 | (normal vs normal) (cancer vs cancer) (cancer vs cancer) (cancer+normal vs cancer+normal) | 1-12655992 | cg13314614 | (cancer+normal vs cancer+normal) |
| 4-187629400 | cg24833566 | (normal vs normal) (cancer vs cancer) (cancer+normal vs cancer+normal) | 1-12656114 | cg04339837 | (normal vs normal) (cancer+normal vs cancer+normal) |
| 4-187629427 | cg13454542 | (normal vs normal) (cancer vs cancer) (cancer+normal vs cancer+normal) | 1-12656315 | cg09517873 | (cancer+normal vs cancer+normal) |
| 4-6945702 | cg05069807 | (cancer vs cancer) (cancer vs cancer) (cancer+normal vs cancer+normal) | 1-168489851 | cg22393436 | (cancer+normal vs cancer+normal) |
| 4-6945745 | cg16574807 | (normal vs normal) (cancer vs cancer) (cancer vs cancer) (cancer+normal vs cancer+normal) | 1-17571896 | cg06772309 | (cancer+normal vs cancer+normal) |
| 4-7985238 | cg17630294 | (normal vs normal) (cancer vs cancer) (cancer+normal vs cancer+normal) | 1-182584341 | cg00153856 | (cancer vs cancer) (cancer vs cancer) (cancer+normal vs cancer+normal) |
| 4-99668618 | cg02008770 | (cancer vs cancer) (cancer+normal vs cancer+normal) | 1-182584520 | cg07144296 | (cancer vs cancer) (normal vs normal) (cancer+normal vs cancer+normal) |
| 5-149546073 | cg26531174 | (cancer vs cancer) (cancer vs cancer) (cancer+normal vs cancer+normal) | 1-182584578 | cg24085895 | (cancer vs cancer) (normal vs normal) (cancer+normal vs cancer+normal) |
| 5-149547566 | cg09690765 | (cancer vs cancer) (cancer vs cancer) (cancer+normal vs cancer+normal) | 1-223747670 | cg18391209 | (cancer+normal vs cancer+normal) |
| 5-179393917 | cg26722544 | (cancer vs cancer) (cancer vs cancer) (cancer+normal vs cancer+normal) | 1-2886868 | cg04080530 | (cancer+normal vs cancer+normal) |
| 6-136756541 | cg11114242 | (cancer vs cancer) | 1-44466601 | cg25605771 | (cancer+normal vs cancer+normal) |
| 6-136874706 | cg06093525 | (cancer vs cancer) | 1-6511878 | cg00014168 | (cancer+normal vs cancer+normal) |
| 6-31123044 | cg01086672 | (normal vs normal) (cancer vs cancer) (cancer vs cancer) (cancer+normal vs cancer+normal) | 10-100994381 | cg08529260 | (cancer vs cancer) (cancer+normal vs cancer+normal) |
| 7-150069026 | cg17677524 | (normal vs normal) (cancer vs cancer) (cancer vs cancer) (cancer+normal vs cancer+normal) | 10-28342666 | cg08505335 | (cancer+normal vs cancer+normal) |
| 7-150069577 | cg12613107 | (normal vs normal) (cancer vs cancer) (cancer+normal vs cancer+normal) | 11-119227328 | cg10904972 | (cancer+normal vs cancer+normal) |

| | | | | | |
|---|---|---|---|---|---|
| 7-150069890 | cg00835828 | (cancer vs cancer) (cancer+normal vs cancer+normal) | 11-119612342 | cg03432275 | (normal vs normal) (cancer+normal vs cancer+normal) |
| 7-1563610 | cg16784174 | (cancer vs cancer) | 11-119612475 | cg09194364 | (normal vs normal) (cancer+normal vs cancer+normal) |
| 7-24798855 | cg26712096 | (cancer vs cancer) | 11-18416535 | cg20429911 | (normal vs normal) (cancer+normal vs cancer+normal) |
| 7-33793736 | cg08085853 | (cancer vs cancer) (cancer+normal vs cancer+normal) | 11-49974622 | cg16386523 | (cancer vs cancer) (cancer+normal vs cancer+normal) |
| 7-50133100 | cg10349880 | (cancer vs cancer) | 11-55796572 | cg17060964 | (cancer+normal vs cancer+normal) |
| 7-50133131 | cg09921682 | (cancer vs cancer) (cancer vs cancer) | 11-615519 | cg16541031 | (cancer+normal vs cancer+normal) |
| 7-73408058 | cg16233515 | (normal vs normal) (cancer vs cancer) (cancer vs cancer) (cancer+normal vs cancer+normal) | 11-615732 | cg18131537 | (normal vs normal) (cancer+normal vs cancer+normal) |
| 7-73408060 | cg07243141 | (normal vs normal) (cancer vs cancer) (cancer vs cancer) (cancer+normal vs cancer+normal) | 11-616009 | cg07652350 | (cancer+normal vs cancer+normal) |
| 7-73408101 | cg18780627 | (normal vs normal) (cancer vs cancer) (cancer vs cancer) (cancer+normal vs cancer+normal) | 11-616089 | cg13378284 | (cancer vs cancer) (cancer+normal vs cancer+normal) |
| 8-140971959 | cg10454596 | (cancer vs cancer) | 12-130450777 | cg11737941 | (cancer+normal vs cancer+normal) |
| 8-18248453 | cg14494313 | (cancer vs cancer) (cancer+normal vs cancer+normal) | 12-133000830 | cg01577029 | (cancer+normal vs cancer+normal) |
| 9-126243651 | cg13847724 | (normal vs normal) (cancer vs cancer) (cancer+normal vs cancer+normal) | 12-31769252 | cg21882037 | (cancer+normal vs cancer+normal) |
| 9-128022358 | cg11557901 | (cancer vs cancer) | 12-4274034 | cg06807966 | (cancer vs cancer) (cancer vs cancer) (normal vs normal) (cancer+normal vs cancer+normal) |
| 9-140198751 | cg13601936 | (cancer vs cancer) (cancer+normal vs cancer+normal) | 12-4274070 | cg08873912 | (cancer vs cancer) (cancer+normal vs cancer+normal) |
| 9-140574551 | cg13710613 | (cancer vs cancer) (cancer+normal vs cancer+normal) | 13-32605218 | cg16158863 | (cancer vs cancer) (cancer+normal vs cancer+normal) |
| **(cancer+normal vs cancer+normal)** | | | 13-32605406 | cg22219254 | (cancer vs cancer) (cancer vs cancer) (normal vs normal) (cancer+normal vs cancer+normal) |
| **Status - Hyper** | | | 13-32605611 | cg16941656 | (cancer vs cancer) (cancer vs cancer) (normal vs normal) (cancer+normal vs cancer+normal) |

| | | | | | |
|---|---|---|---|---|---|
| 1-120837955 | cg02905065 | (cancer vs cancer) (cancer+normal vs cancer+normal) | 14-105714589 | cg23034818 | (cancer vs cancer) (cancer vs cancer) (normal vs normal) (cancer+normal vs cancer+normal) |
| 1-120838320 | cg07784526 | (cancer vs cancer) (cancer+normal vs cancer+normal) | 14-105714833 | cg15474367 | (cancer vs cancer) (cancer vs cancer) (normal vs normal) (cancer+normal vs cancer+normal) |
| 1-120838323 | cg18948743 | (cancer vs cancer) (cancer+normal vs cancer+normal) | 14-105714843 | cg16561543 | (cancer vs cancer) (cancer vs cancer) (normal vs normal) (cancer+normal vs cancer+normal) |
| 1-143913552 | cg22710840 | (cancer vs cancer) (cancer+normal vs cancer+normal) | 14-105715025 | cg01237565 | (cancer vs cancer) (cancer vs cancer) (normal vs normal) (cancer+normal vs cancer+normal) |
| 1-150254477 | cg11207983 | (cancer vs cancer) (cancer+normal vs cancer+normal) | 14-38057752 | cg26435698 | (cancer vs cancer) (cancer+normal vs cancer+normal) |
| 1-150254546 | cg06982544 | (cancer vs cancer) (cancer+normal vs cancer+normal) | 14-38068608 | cg10168722 | (cancer+normal vs cancer+normal) |
| 1-150254869 | cg10055771 | (cancer vs cancer) (cancer+normal vs cancer+normal) | 14-62331287 | cg04791162 | (normal vs normal) (cancer+normal vs cancer+normal) |
| 1-150266242 | cg01566242 | (cancer+normal vs cancer+normal) | 15-22798849 | cg13429424 | (cancer+normal vs cancer+normal) |
| 1-150266311 | cg18031008 | (cancer vs cancer) (cancer+normal vs cancer+normal) | 15-53090807 | cg11983576 | (cancer+normal vs cancer+normal) |
| 1-150266313 | cg26535484 | (cancer+normal vs cancer+normal) | 15-53096763 | cg22488745 | (normal vs normal) (cancer+normal vs cancer+normal) |
| 1-150266477 | cg08293086 | (cancer+normal vs cancer+normal) | 16-1202468 | cg16427096 | (cancer vs cancer) (normal vs normal) (cancer+normal vs cancer+normal) |
| 1-150266488 | cg23819679 | (cancer+normal vs cancer+normal) | 16-51183533 | cg02417084 | (cancer+normal vs cancer+normal) |
| 1-150266643 | cg09808343 | (cancer+normal vs cancer+normal) | 16-86231823 | cg27299647 | (cancer vs cancer) (cancer+normal vs cancer+normal) |
| 1-150266645 | cg15934674 | (cancer+normal vs cancer+normal) | 16-86231948 | cg07989221 | (cancer vs cancer) (cancer vs cancer) (normal vs normal) (cancer+normal vs cancer+normal) |
| 1-210111028 | cg15389528 | (cancer+normal vs cancer+normal) | 16-86232457 | cg26827987 | (cancer vs cancer) (cancer+normal vs cancer+normal) |
| 10-93392836 | cg00197495 | (cancer vs cancer) (cancer+normal vs cancer+normal) | 16-86331580 | cg26778630 | (cancer+normal vs cancer+normal) |
| 10-93393030 | cg12486537 | (cancer vs cancer) (cancer+normal vs cancer+normal) | 17-19656554 | cg08817867 | (cancer+normal vs cancer+normal) |
| 11- | cg01234063 | (normal vs normal) | 17- | cg1196113 | (cancer+normal vs |

| | | | | | |
|---|---|---|---|---|---|
| 126226007 | | (cancer+normal vs cancer+normal) | 38599366 | 8 | cancer+normal) |
| 11-94473668 | cg26963029 | (cancer vs cancer) (cancer+normal vs cancer+normal) | 17-42857157 | cg1164020 8 | (cancer+normal vs cancer+normal) |
| 11-94501718 | cg11117364 | (cancer vs cancer) (cancer+normal vs cancer+normal) | 19-5828172 | cg1469434 2 | (cancer+normal vs cancer+normal) |
| 11-94502388 | cg23628232 | (cancer vs cancer) (cancer+normal vs cancer+normal) | 19-5828301 | cg0991152 1 | (cancer+normal vs cancer+normal) |
| 11-94502524 | cg20493960 | (cancer vs cancer) (cancer+normal vs cancer+normal) | 2-102804405 | cg0137682 9 | (cancer vs cancer) (cancer vs cancer) (cancer+normal vs cancer+normal) |
| 12-54333587 | cg15611413 | (cancer vs cancer) (cancer+normal vs cancer+normal) | 2-102804576 | cg0067489 6 | (cancer vs cancer) (cancer vs cancer) (cancer+normal vs cancer+normal) |
| 12-54519825 | cg21161136 | (cancer vs cancer) (cancer+normal vs cancer+normal) | 2-215276156 | cg0658041 9 | (cancer vs cancer) (cancer+normal vs cancer+normal) |
| 12-91775247 | cg22524951 | (normal vs normal) (cancer+normal vs cancer+normal) | 2-233368263 | cg0513745 0 | (cancer vs cancer) (cancer+normal vs cancer+normal) |
| 12-91775501 | cg24338491 | (normal vs normal) (cancer+normal vs cancer+normal) | 2-56236435 | cg0970391 0 | (cancer+normal vs cancer+normal) |
| 13-26626134 | cg20193324 | (cancer vs cancer) (cancer+normal vs cancer+normal) | 2-8816608 | cg2560253 6 | (cancer+normal vs cancer+normal) |
| 13-27999177 | cg22138327 | (cancer+normal vs cancer+normal) | 2-8817010 | cg2194600 6 | (cancer+normal vs cancer+normal) |
| 14-21439243 | cg01655898 | (normal vs normal) (cancer+normal vs cancer+normal) | 3-12994877 | cg0332559 8 | (cancer+normal vs cancer+normal) |
| 15-45670068 | cg05280133 | (cancer vs cancer) (cancer+normal vs cancer+normal) | 3-27674461 | cg1370789 4 | (normal vs normal) (cancer+normal vs cancer+normal) |
| 15-45670478 | cg11431346 | (cancer vs cancer) (cancer+normal vs cancer+normal) | 3-63428840 | cg1128215 6 | (normal vs normal) (cancer+normal vs cancer+normal) |
| 15-45670526 | cg01145430 | (cancer vs cancer) (cancer+normal vs cancer+normal) | 4-1160783 | cg0846212 2 | (cancer+normal vs cancer+normal) |
| 15-83654517 | cg07109804 | (cancer+normal vs cancer+normal) | 4-1166270 | cg2275739 1 | (cancer+normal vs cancer+normal) |
| 15-83654524 | cg01323954 | (cancer+normal vs cancer+normal) | 4-1166272 | cg1815963 6 | (cancer+normal vs cancer+normal) |
| 15-98197914 | cg19707853 | (cancer+normal vs cancer+normal) | 4-174456504 | cg1518979 3 | (cancer+normal vs cancer+normal) |
| 17-33775394 | cg22588307 | (cancer vs cancer) (cancer+normal vs cancer+normal) | 4-175444482 | cg0108456 6 | (normal vs normal) (cancer+normal vs cancer+normal) |
| 17-37794080 | cg24526433 | (cancer+normal vs cancer+normal) | 4-187002617 | cg1482792 9 | (cancer vs cancer) (cancer+normal vs cancer+normal) |
| 17-46124821 | cg06740897 | (cancer vs cancer) (cancer+normal vs cancer+normal) | 4-60001000 | cg1128122 4 | (cancer vs cancer) (cancer+normal vs cancer+normal) |
| 17-881949 | cg23513567 | (cancer vs cancer) | 4-84869222 | cg2150116 | (cancer+normal vs |

| | | | | | |
|---|---|---|---|---|---|
| | | (cancer+normal vs cancer+normal) | | 7 | cancer+normal) |
| 17-882342 | cg13966567 | (cancer vs cancer) (cancer+normal vs cancer+normal) | 5-11385893 | cg19576843 | (cancer+normal vs cancer+normal) |
| 19-14016729 | cg09479389 | (cancer vs cancer) (normal vs normal) (cancer+normal vs cancer+normal) | 5-672872 | cg24082121 | (normal vs normal) (cancer+normal vs cancer+normal) |
| 19-36822442 | cg23044017 | (cancer+normal vs cancer+normal) | 5-75996951 | cg23256683 | (cancer+normal vs cancer+normal) |
| 19-36822564 | cg21808240 | (cancer+normal vs cancer+normal) | 6-166869078 | cg18404335 | (cancer+normal vs cancer+normal) |
| 19-5293083 | cg16733654 | (cancer vs cancer) (cancer+normal vs cancer+normal) | 6-170602486 | cg04050463 | (cancer+normal vs cancer+normal) |
| 19-57792200 | cg17412580 | (cancer+normal vs cancer+normal) | 6-170604454 | cg26446009 | (cancer+normal vs cancer+normal) |
| 19-57831632 | cg00846300 | (cancer vs cancer) (cancer+normal vs cancer+normal) | 6-43022109 | cg08834050 | (cancer+normal vs cancer+normal) |
| 19-57831678 | cg23986470 | (cancer vs cancer) (cancer+normal vs cancer+normal) | 7-123674510 | cg09777637 | (cancer+normal vs cancer+normal) |
| 19-57832339 | cg04174410 | (cancer+normal vs cancer+normal) | 7-139168481 | cg24956250 | (cancer vs cancer) (cancer vs cancer) (cancer+normal vs cancer+normal) |
| 19-57862627 | cg16848116 | (cancer vs cancer) (cancer+normal vs cancer+normal) | 7-139168503 | cg00699934 | (cancer vs cancer) (cancer+normal vs cancer+normal) |
| 19-57862638 | cg21627760 | (cancer vs cancer) (cancer+normal vs cancer+normal) | 7-139168659 | cg25181507 | (cancer vs cancer) (normal vs normal) (cancer+normal vs cancer+normal) |
| 19-57862713 | cg07494047 | (cancer vs cancer) (cancer+normal vs cancer+normal) | 7-149734481 | cg18356634 | (normal vs normal) (cancer+normal vs cancer+normal) |
| 19-6274208 | cg02987482 | (cancer+normal vs cancer+normal) | 7-156810507 | cg20316549 | (normal vs normal) (cancer+normal vs cancer+normal) |
| 2-100938799 | cg23977631 | (cancer vs cancer) (cancer+normal vs cancer+normal) | 7-156811362 | cg10108372 | (cancer vs cancer) (cancer+normal vs cancer+normal) |
| 2-158454110 | cg18061904 | (cancer vs cancer) (normal vs normal) (cancer+normal vs cancer+normal) | 7-156811422 | cg03413097 | (cancer+normal vs cancer+normal) |
| 2-29339076 | cg21972382 | (cancer vs cancer) (cancer vs cancer) (cancer+normal vs cancer+normal) | 7-156812104 | cg09595202 | (cancer vs cancer) (cancer vs cancer) (cancer+normal vs cancer+normal) |
| 2-29339091 | cg05038216 | (cancer vs cancer) (cancer vs cancer) (cancer+normal vs cancer+normal) | 7-156812806 | cg04867749 | (normal vs normal) (cancer+normal vs cancer+normal) |
| 2-66803033 | cg02764245 | (cancer vs cancer) (cancer+normal vs cancer+normal) | 7-156813480 | cg23393242 | (cancer+normal vs cancer+normal) |
| 2-66803345 | cg13895765 | (cancer+normal vs cancer+normal) | 7-156814480 | cg09858188 | (cancer+normal vs cancer+normal) |

| | | | | | |
|---|---|---|---|---|---|
| 20-23030446 | cg22152407 | (cancer vs cancer) (cancer+normal vs cancer+normal) | 7-156814491 | cg00024086 | (cancer+normal vs cancer+normal) |
| 20-3389408 | cg13516746 | (cancer vs cancer) (cancer+normal vs cancer+normal) | 7-156814845 | cg02570079 | (cancer vs cancer) (cancer vs cancer) (normal vs normal) (cancer+normal vs cancer+normal) |
| 20-3389535 | cg20681068 | (cancer vs cancer) (cancer+normal vs cancer+normal) | 7-156815030 | cg20235658 | (cancer vs cancer) (cancer+normal vs cancer+normal) |
| 20-42875708 | cg07003360 | (normal vs normal) (cancer+normal vs cancer+normal) | 7-157644555 | cg16561057 | (cancer+normal vs cancer+normal) |
| 20-42875721 | cg25799864 | (cancer+normal vs cancer+normal) | 7-157644665 | cg21280014 | (cancer+normal vs cancer+normal) |
| 20-42876091 | cg17810944 | (cancer+normal vs cancer+normal) | 7-157698543 | cg01329577 | (cancer+normal vs cancer+normal) |
| 21-43182367 | cg04893913 | (normal vs normal) (cancer+normal vs cancer+normal) | 7-157869970 | cg20705893 | (cancer+normal vs cancer+normal) |
| 22-20849196 | cg02527346 | (cancer+normal vs cancer+normal) | 7-157932951 | cg09066883 | (cancer+normal vs cancer+normal) |
| 3-48700027 | cg15511738 | (cancer vs cancer) (cancer+normal vs cancer+normal) | 7-2672400 | cg15934804 | (cancer+normal vs cancer+normal) |
| 4-111553161 | cg19602081 | (cancer vs cancer) (cancer+normal vs cancer+normal) | 8-8821019 | cg26711333 | (cancer+normal vs cancer+normal) |
| 5-146258195 | cg09528265 | (cancer vs cancer) (cancer+normal vs cancer+normal) | **Status - Hypo** | | |
| 5-31855185 | cg26880493 | (cancer vs cancer) (cancer+normal vs cancer+normal) | 1-118165662 | cg17181598 | (normal vs normal) (cancer+normal vs cancer+normal) |
| 5-38556223 | cg08392199 | (cancer vs cancer) (cancer+normal vs cancer+normal) | 1-118427435 | cg01778345 | (normal vs normal) (cancer+normal vs cancer+normal) |
| 5-38556435 | cg12587766 | (cancer vs cancer) (cancer+normal vs cancer+normal) | 1-155167337 | cg24457118 | (cancer+normal vs cancer+normal) |
| 5-38557085 | cg18174928 | (cancer vs cancer) (cancer+normal vs cancer+normal) | 1-15764869 | cg03096785 | (cancer+normal vs cancer+normal) |
| 5-38557143 | cg03723506 | (cancer vs cancer) (cancer+normal vs cancer+normal) | 1-17249877 | cg07319253 | (normal vs normal) (cancer+normal vs cancer+normal) |
| 5-38846034 | cg02390103 | (cancer+normal vs cancer+normal) | 1-1847864 | cg15527168 | (normal vs normal) (cancer+normal vs cancer+normal) |
| 5-38846100 | cg17528648 | (cancer vs cancer) (cancer+normal vs cancer+normal) | 1-203098067 | cg24408776 | (cancer vs cancer) (cancer vs cancer) (cancer+normal vs cancer+normal) |
| 5-38846475 | cg05485663 | (cancer+normal vs cancer+normal) | 1-22303542 | cg17517480 | (cancer+normal vs cancer+normal) |
| 6-56716884 | cg24970181 | (cancer+normal vs cancer+normal) | 1-27687074 | cg01730970 | (cancer+normal vs cancer+normal) |
| 6-72130209 | cg10039188 | (cancer vs cancer) (cancer+normal vs cancer+normal) | 1-44819838 | cg00717995 | (cancer vs cancer) (cancer+normal vs cancer+normal) |

| | | | | | |
|---|---|---|---|---|---|
| 6-72130407 | cg25210451 | (cancer vs cancer) (cancer+normal vs cancer+normal) | 1-47078581 | cg01417692 | (normal vs normal) (cancer+normal vs cancer+normal) |
| 6-72130432 | cg15045441 | (cancer vs cancer) (cancer+normal vs cancer+normal) | 1-47078752 | cg19866325 | (normal vs normal) (cancer+normal vs cancer+normal) |
| 6-72130539 | cg26162616 | (cancer vs cancer) (cancer+normal vs cancer+normal) | 10-124605905 | cg06584433 | (normal vs normal) (cancer+normal vs cancer+normal) |
| 6-72130547 | cg23281154 | (cancer+normal vs cancer+normal) | 10-134499528 | cg00458454 | (cancer vs cancer) (cancer vs cancer) (cancer+normal vs cancer+normal) |
| 6-72130553 | cg22300282 | (cancer vs cancer) (cancer+normal vs cancer+normal) | 10-134499734 | cg13441142 | (normal vs normal) (cancer vs cancer) (cancer vs cancer) (cancer+normal vs cancer+normal) |
| 6-72130689 | cg25141674 | (cancer vs cancer) (cancer+normal vs cancer+normal) | 10-134499752 | cg02541125 | (cancer vs cancer) (cancer vs cancer) (cancer+normal vs cancer+normal) |
| 6-72130755 | cg00920327 | (cancer vs cancer) (cancer+normal vs cancer+normal) | 10-134567188 | cg15216078 | (cancer vs cancer) (cancer+normal vs cancer+normal) |
| 7-149411498 | cg23778596 | (cancer vs cancer) (cancer+normal vs cancer+normal) | 10-27703336 | cg00632657 | (cancer vs cancer) (cancer+normal vs cancer+normal) |
| 7-149411652 | cg08131100 | (cancer vs cancer) (cancer+normal vs cancer+normal) | 11-1011281 | cg01261206 | (cancer+normal vs cancer+normal) |
| 7-149412290 | cg03788131 | (cancer vs cancer) (cancer+normal vs cancer+normal) | 11-1011315 | cg18319533 | (cancer+normal vs cancer+normal) |
| 7-73442353 | cg16735682 | (normal vs normal) (cancer+normal vs cancer+normal) | 11-46374124 | cg12087471 | (cancer vs cancer) (cancer+normal vs cancer+normal) |
| 8-27450047 | cg24592790 | (cancer+normal vs cancer+normal) | 11-46612744 | cg19244380 | (cancer+normal vs cancer+normal) |
| 9-124132393 | cg14632002 | (cancer vs cancer) (cancer+normal vs cancer+normal) | 11-47372949 | cg06168166 | (cancer vs cancer) (cancer vs cancer) (cancer+normal vs cancer+normal) |
| 9-124132520 | cg05939335 | (cancer+normal vs cancer+normal) | 11-47372960 | cg10354134 | (cancer vs cancer) (cancer vs cancer) (cancer+normal vs cancer+normal) |
| 9-131842938 | cg14599233 | (cancer+normal vs cancer+normal) | 11-57319872 | cg25492363 | (normal vs normal) (cancer+normal vs cancer+normal) |
| 9-137299285 | cg13865488 | (cancer+normal vs cancer+normal) | 11-62139710 | cg00473624 | (normal vs normal) (cancer+normal vs cancer+normal) |
| 9-137299436 | cg14654324 | (cancer vs cancer) (cancer+normal vs cancer+normal) | 11-67810268 | cg18456140 | (cancer+normal vs cancer+normal) |
| 9-140024858 | cg06657096 | (cancer+normal vs cancer+normal) | 11-888685 | cg07091346 | (normal vs normal) (cancer+normal vs cancer+normal) |
| 9-707166 | cg14399863 | (cancer vs cancer) (normal vs normal) | 12-117468110 | cg19528797 | (normal vs normal) (cancer+normal vs |

| | | | | | |
|---|---|---|---|---|---|
| | | (cancer+normal vs cancer+normal) | | | cancer+normal) |
| 9-707387 | cg13877502 | (cancer vs cancer) (cancer vs cancer) (normal vs normal) (cancer+normal vs cancer+normal) | 12-22602447 | cg23542449 | (cancer+normal vs cancer+normal) |
| 9-707410 | cg03358592 | (cancer vs cancer) (normal vs normal) (cancer+normal vs cancer+normal) | 12-33031216 | cg03762760 | (cancer+normal vs cancer+normal) |
| **Status - Hypo** | | | 13-111719003 | cg17121140 | (cancer+normal vs cancer+normal) |
| 1-1095607 | cg02971920 | (normal vs normal) (cancer vs cancer) (cancer vs cancer) (cancer+normal vs cancer+normal) | 13-113296192 | cg12351768 | (cancer+normal vs cancer+normal) |
| 1-167059365 | cg06665322 | (normal vs normal) (cancer vs cancer) (cancer+normal vs cancer+normal) | 14-104184575 | cg00690554 | (cancer+normal vs cancer+normal) |
| 1-17898874 | cg24600987 | (normal vs normal) (cancer vs cancer) (cancer vs cancer) (cancer+normal vs cancer+normal) | 14-104190829 | cg14977938 | (cancer vs cancer) (cancer+normal vs cancer+normal) |
| 1-206593025 | cg13487476 | (cancer vs cancer) (cancer+normal vs cancer+normal) | 14-105737848 | cg18374393 | (cancer+normal vs cancer+normal) |
| 1-220132091 | cg05230389 | (cancer vs cancer) (cancer+normal vs cancer+normal) | 14-24732234 | cg19797304 | (normal vs normal) (cancer+normal vs cancer+normal) |
| 1-220132097 | cg10576232 | (cancer vs cancer) (cancer+normal vs cancer+normal) | 14-45365651 | cg01523474 | (normal vs normal) (cancer vs cancer) (cancer vs cancer) (cancer+normal vs cancer+normal) |
| 1-226077287 | cg25552768 | (cancer+normal vs cancer+normal) | 14-61108227 | cg03032497 | (cancer+normal vs cancer+normal) |
| 1-226077303 | cg14264773 | (cancer+normal vs cancer+normal) | 14-64680996 | cg10498703 | (cancer+normal vs cancer+normal) |
| 1-230257067 | cg01554316 | (cancer vs cancer) (cancer+normal vs cancer+normal) | 14-64681225 | cg21174375 | (cancer+normal vs cancer+normal) |
| 1-23040723 | cg07952381 | (cancer+normal vs cancer+normal) | 15-102157726 | cg11484721 | (cancer vs cancer) (cancer+normal vs cancer+normal) |
| 1-54723213 | cg22537280 | (cancer vs cancer) (cancer vs cancer) (cancer+normal vs cancer+normal) | 15-22930613 | cg26344513 | (normal vs normal) (cancer+normal vs cancer+normal) |
| 10-518370 | cg26718707 | (cancer vs cancer) (cancer vs cancer) (cancer+normal vs cancer+normal) | 15-42211679 | cg09204108 | (cancer+normal vs cancer+normal) |
| 10-94891725 | cg25800753 | (cancer+normal vs cancer+normal) | 15-42211689 | cg04656171 | (cancer+normal vs cancer+normal) |
| 11-130060459 | cg11069338 | (normal vs normal) (cancer vs cancer) (cancer vs cancer) | 16-12061715 | cg09319797 | (normal vs normal) (cancer vs cancer) (cancer+normal vs |

| | | (cancer+normal vs cancer+normal) | | | cancer+normal) |
|---|---|---|---|---|---|
| 11-1847357 | cg03529598 | (normal vs normal) (cancer+normal vs cancer+normal) | 16-3345604 | cg08555866 | (cancer vs cancer) (cancer+normal vs cancer+normal) |
| 11-2287734 | cg14907310 | (cancer vs cancer) (cancer vs cancer) (cancer+normal vs cancer+normal) | 16-47048004 | cg03743678 | (cancer vs cancer) (cancer+normal vs cancer+normal) |
| 11-76265321 | cg17373759 | (cancer vs cancer) (cancer+normal vs cancer+normal) | 16-67965756 | cg04205655 | (cancer+normal vs cancer+normal) |
| 12-104218302 | cg04909529 | (cancer+normal vs cancer+normal) | 16-67965867 | cg04413148 | (cancer+normal vs cancer+normal) |
| 12-109885015 | cg24961970 | (cancer vs cancer) (cancer+normal vs cancer+normal) | 16-75241125 | cg27635470 | (cancer+normal vs cancer+normal) |
| 12-121203948 | cg02419362 | (cancer vs cancer) (cancer+normal vs cancer+normal) | 16-88540175 | cg16627358 | (cancer vs cancer) (cancer+normal vs cancer+normal) |
| 13-52735075 | cg19524009 | (cancer vs cancer) (cancer+normal vs cancer+normal) | 16-89472771 | cg05749855 | (normal vs normal) (cancer+normal vs cancer+normal) |
| 14-68759670 | cg04384914 | (cancer+normal vs cancer+normal) | 17-37322330 | cg07330481 | (cancer+normal vs cancer+normal) |
| 15-80866134 | cg27665327 | (cancer+normal vs cancer+normal) | 17-40996565 | cg08783253 | (cancer vs cancer) (cancer+normal vs cancer+normal) |
| 16-71755733 | cg05028274 | (cancer vs cancer) (cancer+normal vs cancer+normal) | 17-43578911 | cg02622647 | (cancer+normal vs cancer+normal) |
| 16-78233495 | cg07747924 | (cancer vs cancer) (cancer+normal vs cancer+normal) | 17-62120439 | cg17172761 | (cancer vs cancer) (cancer+normal vs cancer+normal) |
| 16-89165765 | cg08316009 | (normal vs normal) (cancer+normal vs cancer+normal) | 17-7755877 | cg25732961 | (cancer+normal vs cancer+normal) |
| 16-89169289 | cg10398761 | (normal vs normal) (cancer vs cancer) (cancer vs cancer) (cancer+normal vs cancer+normal) | 17-78337534 | cg22161784 | (normal vs normal) (cancer+normal vs cancer+normal) |
| 16-968682 | cg04454872 | (normal vs normal) (cancer+normal vs cancer+normal) | 17-78338052 | cg04011497 | (cancer+normal vs cancer+normal) |
| 17-1412449 | cg23346781 | (cancer vs cancer) (cancer vs cancer) (cancer+normal vs cancer+normal) | 17-80395405 | cg16535788 | (normal vs normal) (cancer+normal vs cancer+normal) |
| 17-4042717 | cg11542336 | (cancer vs cancer) (cancer vs cancer) (cancer+normal vs cancer+normal) | 18-77623598 | cg03682112 | (cancer vs cancer) (cancer+normal vs cancer+normal) |
| 17-5993785 | cg06421238 | (normal vs normal) (cancer+normal vs cancer+normal) | 19-2424793 | cg08569517 | (cancer+normal vs cancer+normal) |
| 17-70256462 | cg06909248 | (normal vs normal) (cancer vs cancer) (cancer vs cancer) (cancer+normal vs cancer+normal) | 19-50059027 | cg12149986 | (cancer+normal vs cancer+normal) |

| | | | | | |
|---|---|---|---|---|---|
| 17-77075025 | cg21088108 | (cancer vs cancer) (cancer+normal vs cancer+normal) | 19-50059937 | cg21292909 | (normal vs normal) (cancer+normal vs cancer+normal) |
| 17-79224178 | cg03971338 | (cancer+normal vs cancer+normal) | 19-50059946 | cg04355077 | (normal vs normal) (cancer+normal vs cancer+normal) |
| 17-79224306 | cg22462714 | (normal vs normal) (cancer vs cancer) (cancer+normal vs cancer+normal) | 19-50060174 | cg26274166 | (normal vs normal) (cancer+normal vs cancer+normal) |
| 17-79224770 | cg27529668 | (cancer+normal vs cancer+normal) | 2-7164527 | cg08182975 | (normal vs normal) (cancer vs cancer) (cancer vs cancer) (cancer+normal vs cancer+normal) |
| 17-80535398 | cg16366685 | (normal vs normal) (cancer vs cancer) (cancer vs cancer) (cancer+normal vs cancer+normal) | 2-7164586 | cg20995753 | (normal vs normal) (cancer vs cancer) (cancer vs cancer) (cancer+normal vs cancer+normal) |
| 17-80535428 | cg03454705 | (normal vs normal) (cancer vs cancer) (cancer vs cancer) (cancer+normal vs cancer+normal) | 2-9388337 | cg00230766 | (cancer vs cancer) (cancer vs cancer) (cancer+normal vs cancer+normal) |
| 17-96503 | cg09580249 | (cancer+normal vs cancer+normal) | 20-1165206 | cg06645286 | (cancer+normal vs cancer+normal) |
| 18-46288005 | cg00137385 | (normal vs normal) (cancer vs cancer) (cancer+normal vs cancer+normal) | 21-46726267 | cg18466173 | (normal vs normal) (cancer+normal vs cancer+normal) |
| 19-2811654 | cg17741192 | (cancer vs cancer) (cancer+normal vs cancer+normal) | 22-29660173 | cg05333149 | (cancer+normal vs cancer+normal) |
| 19-39306244 | cg12028674 | (normal vs normal) (cancer vs cancer) (cancer vs cancer) (cancer+normal vs cancer+normal) | 3-125077434 | cg01289769 | (cancer+normal vs cancer+normal) |
| 2-106959257 | cg24419520 | (normal vs normal) (cancer vs cancer) (cancer vs cancer) (cancer+normal vs cancer+normal) | 4-154373479 | cg08758887 | (cancer+normal vs cancer+normal) |
| 2-106959384 | cg13656404 | (cancer vs cancer) (cancer+normal vs cancer+normal) | 4-1646179 | cg27571057 | (cancer vs cancer) (cancer vs cancer) (cancer+normal vs cancer+normal) |
| 2-17830624 | cg12793924 | (normal vs normal) (cancer vs cancer) (cancer vs cancer) (cancer+normal vs cancer+normal) | 4-3241813 | cg25354617 | (cancer+normal vs cancer+normal) |
| 2-227312417 | cg03426602 | (cancer vs cancer) (cancer vs cancer) (cancer+normal vs cancer+normal) | 5-42756851 | cg23688479 | (cancer vs cancer) (cancer vs cancer) (cancer+normal vs cancer+normal) |
| 2-234526077 | cg18313132 | (cancer vs cancer) (cancer+normal vs cancer+normal) | 5-42756876 | cg12585923 | (cancer vs cancer) (cancer+normal vs cancer+normal) |
| 2- | cg00529371 | (normal vs normal) | 5-42757171 | cg2192956 | (cancer vs cancer) |

| | | | | | |
|---|---|---|---|---|---|
| 242735324 | | (cancer vs cancer) (cancer+normal vs cancer+normal) | | 4 | (cancer+normal vs cancer+normal) |
| 2-8850020 | cg00240378 | (normal vs normal) (cancer vs cancer) (cancer vs cancer) (cancer+normal vs cancer+normal) | 5-92443321 | cg1179495 6 | (cancer+normal vs cancer+normal) |
| 2-97427975 | cg11464842 | (normal vs normal) (cancer vs cancer) (cancer vs cancer) (cancer+normal vs cancer+normal) | 6-35766051 | cg1859668 9 | (cancer+normal vs cancer+normal) |
| 20-55959405 | cg25225756 | (normal vs normal) (cancer vs cancer) (cancer vs cancer) (cancer+normal vs cancer+normal) | 6-5087031 | cg1157474 5 | (normal vs normal) (cancer+normal vs cancer+normal) |
| 20-55959549 | cg22618720 | (cancer vs cancer) (cancer vs cancer) (cancer+normal vs cancer+normal) | 7-1066650 | cg0481631 1 | (normal vs normal) (cancer+normal vs cancer+normal) |
| 21-27372387 | cg24168308 | (cancer vs cancer) (cancer+normal vs cancer+normal) | 7-1117418 | cg2093409 6 | (cancer vs cancer) (cancer vs cancer) (cancer+normal vs cancer+normal) |
| 21-27372396 | cg11321156 | (cancer vs cancer) (cancer+normal vs cancer+normal) | 7-1117540 | cg0813708 5 | (cancer vs cancer) (cancer vs cancer) (cancer+normal vs cancer+normal) |
| 21-27372446 | cg23269692 | (cancer vs cancer) (cancer+normal vs cancer+normal) | 7-130021519 | cg0876519 9 | (normal vs normal) (cancer+normal vs cancer+normal) |
| 21-27372461 | cg18274664 | (cancer vs cancer) (cancer+normal vs cancer+normal) | 7-130021544 | cg2111534 6 | (normal vs normal) (cancer+normal vs cancer+normal) |
| 21-45360804 | cg05957544 | (cancer vs cancer) (cancer+normal vs cancer+normal) | 7-130021807 | cg0824418 1 | (normal vs normal) (cancer+normal vs cancer+normal) |
| 21-46269191 | cg08823554 | (cancer vs cancer) (cancer+normal vs cancer+normal) | 7-44187017 | cg1223778 4 | (cancer+normal vs cancer+normal) |
| 22-21320131 | cg00280270 | (cancer+normal vs cancer+normal) | 7-924964 | cg1698208 7 | (cancer+normal vs cancer+normal) |
| 22-31062286 | cg01274916 | (cancer vs cancer) (cancer vs cancer) (cancer+normal vs cancer+normal) | 7-97841864 | cg2288454 1 | (normal vs normal) (cancer vs cancer) (cancer+normal vs cancer+normal) |
| 3-150238257 | cg02504622 | (cancer vs cancer) (cancer vs cancer) (cancer+normal vs cancer+normal) | 7-97844473 | cg2109941 9 | (normal vs normal) (cancer+normal vs cancer+normal) |
| 3-171412917 | cg09433558 | (cancer vs cancer) (cancer vs cancer) (cancer+normal vs cancer+normal) | 7-97857492 | cg0102380 8 | (normal vs normal) (cancer+normal vs cancer+normal) |
| 3-197401639 | cg17304531 | (cancer+normal vs cancer+normal) | 7-97875293 | cg1892981 4 | (cancer vs cancer) (cancer vs cancer) (cancer+normal vs cancer+normal) |
| 3- | cg19463199 | (cancer+normal vs | 7-97875333 | cg1856389 | (cancer vs cancer) |

| Position | cg ID | Comparisons | Position | cg ID | Comparisons |
|---|---|---|---|---|---|
| 197401858 | | cancer+normal) | | 9 | (cancer vs cancer) (cancer+normal vs cancer+normal) |
| 3-31494178 | cg19817118 | (normal vs normal) (cancer+normal vs cancer+normal) | 8-145048363 | cg05141333 | (cancer+normal vs cancer+normal) |
| 3-31494215 | cg20927547 | (normal vs normal) (cancer+normal vs cancer+normal) | 8-30334348 | cg09272186 | (cancer vs cancer) (cancer+normal vs cancer+normal) |
| 3-55525967 | cg24158324 | (cancer vs cancer) (cancer+normal vs cancer+normal) | 9-130271989 | cg14601053 | (cancer+normal vs cancer+normal) |
| 3-9820588 | cg15771477 | (cancer vs cancer) (cancer+normal vs cancer+normal) | 9-140588019 | cg23538468 | (normal vs normal) (cancer+normal vs cancer+normal) |
| 4-144275126 | cg12710519 | (cancer vs cancer) (cancer+normal vs cancer+normal) | **(normal vs normal)** | | |
| 4-184017790 | cg00268149 | (cancer vs cancer) (cancer+normal vs cancer+normal) | **Status - Hyper** | | |
| 4-187126114 | cg23442198 | (normal vs normal) (cancer vs cancer) (cancer vs cancer) (cancer+normal vs cancer+normal) | 1-110626540 | cg23660154 | (normal vs normal) |
| 4-187629328 | cg21399040 | (normal vs normal) (cancer vs cancer) (cancer vs cancer) (cancer+normal vs cancer+normal) | 1-12656114 | cg04339837 | (normal vs normal) (cancer+normal vs cancer+normal) |
| 4-187629336 | cg24820270 | (normal vs normal) (cancer vs cancer) (cancer vs cancer) (cancer+normal vs cancer+normal) | 1-182584520 | cg07144296 | (cancer vs cancer) (normal vs normal) (cancer+normal vs cancer+normal) |
| 4-187629339 | cg01040779 | (normal vs normal) (cancer vs cancer) (cancer+normal vs cancer+normal) | 1-182584578 | cg24085895 | (cancer vs cancer) (normal vs normal) (cancer+normal vs cancer+normal) |
| 4-187629387 | cg25352342 | (normal vs normal) (cancer vs cancer) (cancer vs cancer) (cancer+normal vs cancer+normal) | 1-2163437 | cg15564619 | (normal vs normal) |
| 4-187629400 | cg24833566 | (normal vs normal) (cancer vs cancer) (cancer+normal vs cancer+normal) | 1-226925082 | cg14711690 | (normal vs normal) |
| 4-187629427 | cg13454542 | (normal vs normal) (cancer vs cancer) (cancer+normal vs cancer+normal) | 1-226925172 | cg16340268 | (normal vs normal) |
| 4-6945702 | cg05069807 | (cancer vs cancer) (cancer vs cancer) (cancer+normal vs cancer+normal) | 1-226925181 | cg04482794 | (normal vs normal) |
| 4-6945745 | cg16574807 | (normal vs normal) (cancer vs cancer) (cancer vs cancer) (cancer+normal vs | 10-100993583 | cg13737332 | (normal vs normal) |

| | | | | | |
|---|---|---|---|---|---|
| | | cancer+normal) | | | |
| 4-7985238 | cg17630294 | (normal vs normal)(cancer vs cancer)(cancer+normal vs cancer+normal) | 10-100993826 | cg16825290 | (normal vs normal) |
| 4-99668618 | cg02008770 | (cancer vs cancer)(cancer+normal vs cancer+normal) | 10-100993938 | cg13690864 | (normal vs normal) |
| 5-149546073 | cg26531174 | (cancer vs cancer)(cancer vs cancer)(cancer+normal vs cancer+normal) | 10-23484556 | cg04460771 | (normal vs normal) |
| 5-149547566 | cg09690765 | (cancer vs cancer)(cancer vs cancer)(cancer+normal vs cancer+normal) | 10-81158982 | cg25423573 | (normal vs normal) |
| 5-179393917 | cg26722544 | (cancer vs cancer)(cancer vs cancer)(cancer+normal vs cancer+normal) | 10-94822908 | cg05127821 | (normal vs normal) |
| 6-170038665 | cg21239439 | (cancer+normal vs cancer+normal) | 11-101990755 | cg15999356 | (cancer vs cancer)(normal vs normal) |
| 6-2682491 | cg00390784 | (cancer+normal vs cancer+normal) | 11-119227592 | cg14359435 | (normal vs normal) |
| 6-31123044 | cg01086672 | (normal vs normal)(cancer vs cancer)(cancer vs cancer)(cancer+normal vs cancer+normal) | 11-119612342 | cg03432275 | (normal vs normal)(cancer+normal vs cancer+normal) |
| 7-150069026 | cg17677524 | (normal vs normal)(cancer vs cancer)(cancer vs cancer)(cancer+normal vs cancer+normal) | 11-119612475 | cg09194364 | (normal vs normal)(cancer+normal vs cancer+normal) |
| 7-150069577 | cg12613107 | (normal vs normal)(cancer vs cancer)(cancer+normal vs cancer+normal) | 11-119613061 | cg15407505 | (normal vs normal) |
| 7-150069890 | cg00835828 | (cancer vs cancer)(cancer+normal vs cancer+normal) | 11-1463554 | cg14064268 | (normal vs normal) |
| 7-33793736 | cg08085853 | (cancer vs cancer)(cancer+normal vs cancer+normal) | 11-1463620 | cg10590925 | (normal vs normal) |
| 7-73408058 | cg16233515 | (normal vs normal)(cancer vs cancer)(cancer vs cancer)(cancer+normal vs cancer+normal) | 11-1463662 | cg17429870 | (normal vs normal) |
| 7-73408060 | cg07243141 | (normal vs normal)(cancer vs cancer)(cancer vs cancer)(cancer+normal vs cancer+normal) | 11-18416535 | cg20429911 | (normal vs normal)(cancer+normal vs cancer+normal) |
| 7-73408101 | cg18780627 | (normal vs normal)(cancer vs cancer)(cancer vs cancer)(cancer+normal vs cancer+normal) | 11-615732 | cg18131537 | (normal vs normal)(cancer+normal vs cancer+normal) |
| 7-74021938 | cg27099880 | (normal vs normal)(cancer+normal vs | 11-830190 | cg00420348 | (normal vs normal) |

| Position | Probe | Comparison | Position | Probe | Comparison |
|---|---|---|---|---|---|
| | | cancer+normal) | | | |
| 7-74021961 | cg12431188 | (cancer+normal vs cancer+normal) | 11-830320 | cg20801007 | (normal vs normal) |
| 8-18248453 | cg14494313 | (cancer vs cancer) (cancer+normal vs cancer+normal) | 11-830527 | cg06085985 | (normal vs normal) |
| 9-126243651 | cg13847724 | (normal vs normal) (cancer vs cancer) (cancer+normal vs cancer+normal) | 12-133000792 | cg26120813 | (normal vs normal) |
| 9-140198751 | cg13601936 | (cancer vs cancer) (cancer+normal vs cancer+normal) | 12-4274034 | cg06807966 | (cancer vs cancer) (cancer vs cancer) (normal vs normal) (cancer+normal vs cancer+normal) |
| 9-140574551 | cg13710613 | (cancer vs cancer) (cancer+normal vs cancer+normal) | 12-65218069 | cg03030717 | (normal vs normal) |
| **(normal vs normal)** | | | 12-65218258 | cg11832404 | (normal vs normal) |
| **Status - Hyper** | | | 12-65218260 | cg23684878 | (normal vs normal) |
| 1-111217194 | cg20302133 | (normal vs normal) | 12-65218413 | cg14019323 | (normal vs normal) |
| 1-121261077 | cg06970370 | (normal vs normal) | 12-65218841 | cg23013309 | (normal vs normal) |
| 1-156626749 | cg05888917 | (normal vs normal) | 12-65219392 | cg06234063 | (normal vs normal) |
| 1-3663339 | cg01793449 | (normal vs normal) | 13-28549698 | cg12938068 | (normal vs normal) |
| 1-3663531 | cg15472784 | (normal vs normal) | 13-28550400 | cg15427232 | (normal vs normal) |
| 1-43524347 | cg00778807 | (normal vs normal) | 13-28555387 | cg21950155 | (normal vs normal) |
| 11-126226007 | cg01234063 | (normal vs normal) (cancer+normal vs cancer+normal) | 13-32605406 | cg22219254 | (cancer vs cancer) (cancer vs cancer) (normal vs normal) (cancer+normal vs cancer+normal) |
| 11-14926738 | cg24304093 | (normal vs normal) | 13-32605611 | cg16941656 | (cancer vs cancer) (cancer vs cancer) (normal vs normal) (cancer+normal vs cancer+normal) |
| 11-64397734 | cg27466845 | (normal vs normal) | 14-105714589 | cg23034818 | (cancer vs cancer) (cancer vs cancer) (normal vs normal) (cancer+normal vs cancer+normal) |
| 12-125140105 | cg17322044 | (normal vs normal) | 14-105714833 | cg15474367 | (cancer vs cancer) (cancer vs cancer) (normal vs normal) (cancer+normal vs cancer+normal) |
| 12-91775247 | cg22524951 | (normal vs normal) (cancer+normal vs cancer+normal) | 14-105714843 | cg16561543 | (cancer vs cancer) (cancer vs cancer) (normal vs normal) (cancer+normal vs cancer+normal) |
| 12-91775501 | cg24338491 | (normal vs normal) (cancer+normal vs | 14-105715025 | cg01237565 | (cancer vs cancer) (cancer vs cancer) |

| | | | | | |
|---|---|---|---|---|---|
| | | cancer+normal) | | | (normal vs normal) (cancer+normal vs cancer+normal) |
| 13-37005566 | cg00282249 | (normal vs normal) | 14-23291897 | cg23708763 | (normal vs normal) |
| 13-37006063 | cg02478448 | (normal vs normal) | 14-38058243 | cg27143688 | (normal vs normal) |
| 14-103524899 | cg15827779 | (normal vs normal) | 14-38058263 | cg07465387 | (normal vs normal) |
| 14-103524901 | cg24779941 | (normal vs normal) | 14-38058639 | cg12212453 | (normal vs normal) |
| 14-21439243 | cg01655898 | (normal vs normal) (cancer+normal vs cancer+normal) | 14-38060564 | cg10988041 | (normal vs normal) |
| 15-39871876 | cg25499543 | (normal vs normal) | 15-29395932 | cg15605858 | (normal vs normal) |
| 15-39872186 | cg23849826 | (normal vs normal) | 15-29396200 | cg05790451 | (normal vs normal) |
| 15-45670865 | cg25908973 | (normal vs normal) | 15-53096763 | cg22488745 | (normal vs normal) (cancer+normal vs cancer+normal) |
| 15-45670872 | cg10088041 | (normal vs normal) | 15-66274709 | cg24500441 | (normal vs normal) |
| 15-45670875 | cg00645339 | (normal vs normal) | 15-66274767 | cg18422268 | (normal vs normal) |
| 16-5500503 | cg05364038 | (normal vs normal) | 15-68128025 | cg25903779 | (normal vs normal) |
| 17-27918338 | cg16755500 | (normal vs normal) | 15-68128175 | cg14210607 | (normal vs normal) |
| 17-35084975 | cg10547969 | (cancer vs cancer) (normal vs normal) (cancer+normal vs cancer+normal) | 16-1202468 | cg16427096 | (cancer vs cancer) (normal vs normal) (cancer+normal vs cancer+normal) |
| 17-46671293 | cg10153335 | (cancer vs cancer) (normal vs normal) | 16-68118754 | cg00402172 | (normal vs normal) |
| 17-46671298 | cg02293936 | (cancer vs cancer) (normal vs normal) | 16-86231948 | cg07989221 | (cancer vs cancer) (cancer vs cancer) (normal vs normal) (cancer+normal vs cancer+normal) |
| 17-70216332 | cg00482488 | (normal vs normal) | 17-3591377 | cg06468454 | (normal vs normal) |
| 17-7486770 | cg06931941 | (normal vs normal) | 17-40573764 | cg00971050 | (normal vs normal) |
| 19-14016729 | cg09479389 | (cancer vs cancer) (normal vs normal) (cancer+normal vs cancer+normal) | 17-61553938 | cg09920557 | (normal vs normal) |
| 19-37096148 | cg25397945 | (normal vs normal) | 17-61553954 | cg25054907 | (normal vs normal) |
| 19-37096323 | cg18630667 | (normal vs normal) | 17-61554106 | cg02131967 | (cancer vs cancer) (normal vs normal) |
| 19-37096329 | cg05020604 | (normal vs normal) | 17-79374327 | cg16779839 | (normal vs normal) |
| 19-58220370 | cg05661282 | (normal vs normal) | 18-47088780 | cg15677957 | (normal vs normal) |
| 19-58220494 | cg21790626 | (normal vs normal) | 19-51522894 | cg18437710 | (cancer vs cancer) (normal vs normal) |
| 19-58220516 | cg27049766 | (normal vs normal) | 19-5827811 | cg18517266 | (normal vs normal) |
| 19- | cg08668790 | (normal vs normal) | 2- | cg1507707 | (normal vs normal) |

| | | | | | |
|---|---|---|---|---|---|
| 58220662 | | | 102803290 | 0 | |
| 19-58220718 | cg12506930 | (normal vs normal) | 2-218843435 | cg20225999 | (normal vs normal) |
| 19-58220818 | cg01268824 | (normal vs normal) | 20-21492914 | cg22474464 | (normal vs normal) |
| 19-6272158 | cg06633438 | (normal vs normal) | 20-30193892 | cg09923107 | (cancer vs cancer) (normal vs normal) (cancer+normal vs cancer+normal) |
| 2-123289581 | cg18106923 | (normal vs normal) | 3-187455371 | cg00480331 | (normal vs normal) |
| 2-132152814 | cg26153054 | (normal vs normal) | 3-27674461 | cg13707894 | (normal vs normal) (cancer+normal vs cancer+normal) |
| 2-158454083 | cg16171838 | (normal vs normal) | 3-63428840 | cg11282156 | (normal vs normal) (cancer+normal vs cancer+normal) |
| 2-158454110 | cg18061904 | (cancer vs cancer) (normal vs normal) (cancer+normal vs cancer+normal) | 3-9993934 | cg06181784 | (normal vs normal) |
| 2-158454243 | cg21442003 | (normal vs normal) | 4-1161247 | cg05257291 | (cancer vs cancer) (normal vs normal) (cancer+normal vs cancer+normal) |
| 2-242743588 | cg15346191 | (normal vs normal) | 4-175444482 | cg01084566 | (normal vs normal) (cancer+normal vs cancer+normal) |
| 2-29338113 | cg23428985 | (normal vs normal) | 4-85423132 | cg23254393 | (normal vs normal) |
| 2-29338121 | cg23255835 | (normal vs normal) | 5-11385520 | cg10555307 | (normal vs normal) |
| 2-29338258 | cg03135351 | (normal vs normal) | 5-16179135 | cg06782035 | (normal vs normal) |
| 2-61371880 | cg26147678 | (normal vs normal) | 5-672872 | cg24082121 | (normal vs normal) (cancer+normal vs cancer+normal) |
| 2-61372066 | cg01648237 | (normal vs normal) | 6-100915191 | cg17760405 | (normal vs normal) |
| 2-61372117 | cg05037927 | (normal vs normal) | 6-26172397 | cg27209395 | (normal vs normal) |
| 2-61372138 | cg16328106 | (normal vs normal) | 6-711405 | cg23252902 | (normal vs normal) |
| 2-61372226 | cg24757310 | (normal vs normal) | 7-100224437 | cg19767562 | (normal vs normal) |
| 2-61372256 | cg18158151 | (normal vs normal) | 7-101579929 | cg06173663 | (normal vs normal) |
| 2-70313417 | cg09175843 | (normal vs normal) | 7-101579936 | cg06471596 | (normal vs normal) |
| 2-97193174 | cg17839721 | (normal vs normal) | 7-139168659 | cg25181507 | (cancer vs cancer) (normal vs normal) (cancer+normal vs cancer+normal) |
| 20-42875708 | cg07003360 | (normal vs normal) (cancer+normal vs cancer+normal) | 7-146332766 | cg22049038 | (normal vs normal) |
| 20-42875779 | cg09994356 | (normal vs normal) | 7-146351256 | cg15440158 | (normal vs normal) |
| 20-42875931 | cg13868604 | (normal vs normal) | 7-156810507 | cg20316549 | (normal vs normal) (cancer+normal vs cancer+normal) |

| | | | | | |
|---|---|---|---|---|---|
| 21-43181729 | cg17879722 | (normal vs normal) | 7-156812806 | cg04867749 | (normal vs normal) (cancer+normal vs cancer+normal) |
| 21-43182367 | cg04893913 | (normal vs normal) (cancer+normal vs cancer+normal) | 7-156814845 | cg02570079 | (cancer vs cancer) (cancer vs cancer) (normal vs normal) (cancer+normal vs cancer+normal) |
| 3-25469860 | cg24396624 | (normal vs normal) | 7-29605897 | cg10580293 | (normal vs normal) |
| 3-37033894 | cg11291081 | (normal vs normal) | 7-29606289 | cg18451588 | (normal vs normal) |
| 3-37033903 | cg05670953 | (normal vs normal) | 7-47576580 | cg11203616 | (normal vs normal) |
| 3-37034084 | cg21109167 | (normal vs normal) | 8-11540758 | cg22883125 | (normal vs normal) |
| 4-122632918 | cg03248158 | (normal vs normal) | 8-142216031 | cg16788286 | (normal vs normal) |
| 4-157692845 | cg19518666 | (normal vs normal) | 8-142216119 | cg02508881 | (normal vs normal) |
| 5-137225191 | cg21051519 | (normal vs normal) | **Status - Hypo** | | |
| 5-140797172 | cg13933262 | (normal vs normal) | 1-110530427 | cg01814898 | (normal vs normal) |
| 5-154230223 | cg18188717 | (normal vs normal) | 1-11711141 | cg19095000 | (normal vs normal) |
| 5-154230438 | cg18260397 | (normal vs normal) | 1-118165662 | cg17181598 | (normal vs normal) (cancer+normal vs cancer+normal) |
| 5-49736988 | cg18110165 | (normal vs normal) | 1-118427435 | cg01778345 | (normal vs normal) (cancer+normal vs cancer+normal) |
| 5-75919242 | cg22828110 | (normal vs normal) | 1-1533852 | cg05934012 | (normal vs normal) |
| 6-26240528 | cg22723502 | (normal vs normal) | 1-17249877 | cg07319253 | (normal vs normal) (cancer+normal vs cancer+normal) |
| 6-26240939 | cg21425842 | (normal vs normal) | 1-1847864 | cg15527168 | (normal vs normal) (cancer+normal vs cancer+normal) |
| 6-27173991 | cg06321998 | (normal vs normal) | 1-245607970 | cg24490133 | (normal vs normal) |
| 6-30095419 | cg18055610 | (normal vs normal) | 1-47078581 | cg01417692 | (normal vs normal) (cancer+normal vs cancer+normal) |
| 6-30095549 | cg08548396 | (normal vs normal) | 1-47078752 | cg19866325 | (normal vs normal) (cancer+normal vs cancer+normal) |
| 6-30095570 | cg12534216 | (normal vs normal) | 10-102637177 | cg11822175 | (normal vs normal) |
| 6-30095728 | cg01528052 | (normal vs normal) | 10-102637197 | cg22973459 | (normal vs normal) |
| 6-30095762 | cg27108419 | (normal vs normal) | 10-124605905 | cg06584433 | (normal vs normal) (cancer+normal vs cancer+normal) |
| 6-31831489 | cg22753340 | (normal vs normal) | 10-124605963 | cg02765177 | (normal vs normal) |
| 6-31831502 | cg23174932 | (normal vs normal) | 10-130546208 | cg06733394 | (normal vs normal) |
| 6-31831574 | cg19738463 | (normal vs normal) | 10-130546259 | cg10175249 | (normal vs normal) |

| | | | | | |
|---|---|---|---|---|---|
| 6-35479628 | cg15219228 | (normal vs normal) | 10-130621726 | cg19699317 | (normal vs normal) |
| 6-35569471 | cg07633853 | (normal vs normal) | 10-134499734 | cg13441142 | (normal vs normal) (cancer vs cancer) (cancer vs cancer) (cancer+normal vs cancer+normal) |
| 7-150652948 | cg20883831 | (normal vs normal) | 10-98425148 | cg27215768 | (normal vs normal) (cancer vs cancer) |
| 7-150653001 | cg18423852 | (normal vs normal) | 11-12263892 | cg27631389 | (normal vs normal) |
| 7-18535332 | cg19585556 | (normal vs normal) | 11-1430940 | cg20129113 | (normal vs normal) |
| 7-51538921 | cg07114310 | (normal vs normal) | 11-57319872 | cg25492363 | (normal vs normal) (cancer+normal vs cancer+normal) |
| 7-72838671 | cg12210255 | (normal vs normal) | 11-62139710 | cg00473624 | (normal vs normal) (cancer+normal vs cancer+normal) |
| 7-72838776 | cg13598010 | (normal vs normal) | 11-68972906 | cg02236949 | (normal vs normal) |
| 7-73442353 | cg16735682 | (normal vs normal) (cancer+normal vs cancer+normal) | 11-822402 | cg06072036 | (normal vs normal) |
| 7-73442531 | cg05822532 | (normal vs normal) | 11-823839 | cg07026560 | (normal vs normal) |
| 8-132918921 | cg06773023 | (normal vs normal) | 11-888685 | cg07091346 | (normal vs normal) (cancer+normal vs cancer+normal) |
| 8-70623620 | cg20211463 | (normal vs normal) | 12-111664600 | cg24309040 | (normal vs normal) |
| 9-707166 | cg14399863 | (cancer vs cancer) (normal vs normal) (cancer+normal vs cancer+normal) | 12-111664628 | cg05979433 | (normal vs normal) |
| 9-707387 | cg13877502 | (cancer vs cancer) (cancer vs cancer) (normal vs normal) (cancer+normal vs cancer+normal) | 12-117468110 | cg19528797 | (normal vs normal) (cancer+normal vs cancer+normal) |
| 9-707410 | cg03358592 | (cancer vs cancer) (normal vs normal) (cancer+normal vs cancer+normal) | 12-132381902 | cg15260428 | (normal vs normal) |
| **Status - Hypo** | | | 12-132381984 | cg08731435 | (normal vs normal) |
| 1-1061647 | cg25973293 | (normal vs normal) | 12-132882028 | cg24113243 | (normal vs normal) |
| 1-1095607 | cg02971920 | (normal vs normal) (cancer vs cancer) (cancer vs cancer) (cancer+normal vs cancer+normal) | 12-46776389 | cg19266635 | (normal vs normal) |
| 1-167059365 | cg06665322 | (normal vs normal) (cancer vs cancer) (cancer+normal vs cancer+normal) | 13-113242878 | cg19916129 | (normal vs normal) |
| 1-17898874 | cg24600987 | (normal vs normal) (cancer vs cancer) (cancer vs cancer) (cancer+normal vs | 13-113242997 | cg08602346 | (normal vs normal) |

| | | | | | |
|---|---|---|---|---|---|
| | | cancer+normal) | 13-113243141 | cg0059800 9 | (normal vs normal) |
| 1-36643743 | cg14285365 | (normal vs normal) | 13-113611873 | cg1808073 3 | (normal vs normal) |
| 1-47907054 | cg11393040 | (normal vs normal) | 13-41632640 | cg0242002 7 | (normal vs normal) |
| 10-3160926 | cg05394294 | (normal vs normal) | 14-23855309 | cg2295871 5 | (normal vs normal) |
| 10-3160982 | cg02579140 | (normal vs normal) | 14-24732234 | cg1979730 4 | (normal vs normal) (cancer+normal vs cancer+normal) |
| 10-45719880 | cg20447730 | (normal vs normal) | 14-45365651 | cg0152347 4 | (normal vs normal) (cancer vs cancer) (cancer vs cancer) (cancer+normal vs cancer+normal) |
| 11-1095156 | cg05877661 | (normal vs normal) | 14-62440685 | cg2091733 8 | (normal vs normal) |
| 11-1241885 | cg10964472 | (normal vs normal) | 14-64682912 | cg0823826 5 | (normal vs normal) |
| 11-130059549 | cg25892168 | (normal vs normal) (cancer vs cancer) | 15-22930613 | cg2634451 3 | (normal vs normal) (cancer+normal vs cancer+normal) |
| 11-130059700 | cg04823219 | (normal vs normal) | 16-12061715 | cg0931979 7 | (normal vs normal) (cancer vs cancer) (cancer+normal vs cancer+normal) |
| 11-130060459 | cg11069338 | (normal vs normal) (cancer vs cancer) (cancer vs cancer) (cancer+normal vs cancer+normal) | 16-50982437 | cg1030397 8 | (normal vs normal) |
| 11-1847357 | cg03529598 | (normal vs normal) (cancer+normal vs cancer+normal) | 16-75241216 | cg1347620 4 | (normal vs normal) |
| 11-2441442 | cg22081905 | (normal vs normal) | 16-88473713 | cg0537704 1 | (normal vs normal) |
| 12-130821530 | cg16244664 | (normal vs normal) | 16-89472771 | cg0574985 5 | (normal vs normal) (cancer+normal vs cancer+normal) |
| 12-132849404 | cg25179780 | (normal vs normal) | 17-185102 | cg0322684 4 | (normal vs normal) |
| 12-14849268 | cg18754342 | (normal vs normal) (cancer vs cancer) (cancer vs cancer) | 17-55828038 | cg0004198 9 | (normal vs normal) |
| 15-28339563 | cg20703122 | (normal vs normal) | 17-76497386 | cg1850100 1 | (normal vs normal) |
| 15-59568712 | cg13887966 | (normal vs normal) | 17-78337534 | cg2216178 4 | (normal vs normal) (cancer+normal vs cancer+normal) |
| 16-1808230 | cg01149677 | (normal vs normal) (cancer vs cancer) | 17-80395405 | cg1653578 8 | (normal vs normal) (cancer+normal vs cancer+normal) |
| 16-89165765 | cg08316009 | (normal vs normal) (cancer+normal vs cancer+normal) | 19-39691363 | cg2689771 7 | (normal vs normal) |
| 16-89169289 | cg10398761 | (normal vs normal) (cancer vs cancer) (cancer vs cancer) (cancer+normal vs cancer+normal) | 19-50059937 | cg2129290 9 | (normal vs normal) (cancer+normal vs cancer+normal) |
| 16-968682 | cg04454872 | (normal vs normal) (cancer+normal vs cancer+normal) | 19- | cg0435507 | (normal vs normal) |
| 17- | cg06421238 | (normal vs normal) | | | |

| | | | | | |
|---|---|---|---|---|---|
| 5993785 | | (cancer+normal vs cancer+normal) | 50059946 | 7 | (cancer+normal vs cancer+normal) |
| 17-70256462 | cg06909248 | (normal vs normal) (cancer vs cancer) (cancer vs cancer) (cancer+normal vs cancer+normal) | 19-50060174 | cg26274166 | (normal vs normal) (cancer+normal vs cancer+normal) |
| 17-79224306 | cg22462714 | (normal vs normal) (cancer vs cancer) (cancer+normal vs cancer+normal) | 2-242977619 | cg23056539 | (normal vs normal) |
| 17-80535398 | cg16366685 | (normal vs normal) (cancer vs cancer) (cancer vs cancer) (cancer+normal vs cancer+normal) | 2-7164527 | cg08182975 | (normal vs normal) (cancer vs cancer) (cancer vs cancer) (cancer+normal vs cancer+normal) |
| 17-80535428 | cg03454705 | (normal vs normal) (cancer vs cancer) (cancer vs cancer) (cancer+normal vs cancer+normal) | 2-7164586 | cg20995753 | (normal vs normal) (cancer vs cancer) (cancer vs cancer) (cancer+normal vs cancer+normal) |
| 18-46288005 | cg00137385 | (normal vs normal) (cancer vs cancer) (cancer+normal vs cancer+normal) | 2-74375930 | cg09717809 | (normal vs normal) (cancer vs cancer) (cancer+normal vs cancer+normal) |
| 18-8718597 | cg13287668 | (normal vs normal) | 20-61150489 | cg15010213 | (normal vs normal) |
| 19-1235003 | cg27594157 | (normal vs normal) | 20-61979563 | cg22348992 | (normal vs normal) |
| 19-1235093 | cg03284113 | (normal vs normal) | 21-46726267 | cg18466173 | (normal vs normal) (cancer+normal vs cancer+normal) |
| 19-12886980 | cg10810394 | (normal vs normal) | 3-13679574 | cg17378193 | (normal vs normal) |
| 19-2278618 | cg27341866 | (normal vs normal) | 3-4344661 | cg23182840 | (normal vs normal) |
| 19-2278708 | cg07595134 | (normal vs normal) | 3-48413707 | cg14888296 | (normal vs normal) |
| 19-2278773 | cg08399733 | (normal vs normal) | 3-48413738 | cg06616014 | (normal vs normal) |
| 19-39306244 | cg12028674 | (normal vs normal) (cancer vs cancer) (cancer vs cancer) (cancer+normal vs cancer+normal) | 3-64008007 | cg07662771 | (normal vs normal) |
| 19-4054253 | cg27196194 | (normal vs normal) | 3-64008041 | cg06286796 | (normal vs normal) |
| 19-42460930 | cg24478966 | (normal vs normal) | 4-164391778 | cg12311837 | (normal vs normal) |
| 19-4835716 | cg02330271 | (normal vs normal) | 4-177198411 | cg22115892 | (normal vs normal) |
| 2-106959257 | cg24419520 | (normal vs normal) (cancer vs cancer) (cancer vs cancer) (cancer+normal vs cancer+normal) | 5-1702381 | cg01075852 | (normal vs normal) |
| 2-106959853 | cg08093959 | (normal vs normal) | 5-1702470 | cg17470674 | (normal vs normal) |
| 2-106959868 | cg15139063 | (normal vs normal) | 5-179763857 | cg10962930 | (normal vs normal) |
| 2- | cg12606455 | (normal vs normal) | 6- | cg0460079 | (normal vs normal) |

| 106959878 | | |
|---|---|---|
| 2-131114432 | cg12815916 | (normal vs normal) |
| 2-17830624 | cg12793924 | (normal vs normal) (cancer vs cancer) (cancer vs cancer) (cancer+normal vs cancer+normal) |
| 2-242735324 | cg00529371 | (normal vs normal) (cancer vs cancer) (cancer+normal vs cancer+normal) |
| 2-8849962 | cg10900049 | (normal vs normal) |
| 2-8850020 | cg00240378 | (normal vs normal) (cancer vs cancer) (cancer vs cancer) (cancer+normal vs cancer+normal) |
| 2-97427733 | cg19953799 | (normal vs normal) |
| 2-97427895 | cg12942038 | (normal vs normal) (cancer vs cancer) |
| 2-97427975 | cg11464842 | (normal vs normal) (cancer vs cancer) (cancer vs cancer) (cancer+normal vs cancer+normal) |
| 20-55959405 | cg25225756 | (normal vs normal) (cancer vs cancer) (cancer vs cancer) (cancer+normal vs cancer+normal) |
| 20-57616191 | cg20726575 | (normal vs normal) (cancer vs cancer) |
| 20-62187126 | cg11523191 | (normal vs normal) |
| 22-46809688 | cg13814677 | (normal vs normal) |
| 3-31494178 | cg19817118 | (normal vs normal) (cancer+normal vs cancer+normal) |
| 3-31494215 | cg20927547 | (normal vs normal) (cancer+normal vs cancer+normal) |
| 4-187126114 | cg23442198 | (normal vs normal) (cancer vs cancer) (cancer vs cancer) (cancer+normal vs cancer+normal) |
| 4-187629328 | cg21399040 | (normal vs normal) (cancer vs cancer) (cancer vs cancer) (cancer+normal vs cancer+normal) |
| 4-187629336 | cg24820270 | (normal vs normal) (cancer vs cancer) (cancer vs cancer) |

| 130774523 | 5 | |
|---|---|---|
| 6-168970787 | cg13517866 | (normal vs normal) (cancer vs cancer) |
| 6-169286420 | cg01769392 | (normal vs normal) |
| 6-169286588 | cg10122103 | (normal vs normal) |
| 6-31322926 | cg27529346 | (normal vs normal) |
| 6-5087031 | cg11574745 | (normal vs normal) (cancer+normal vs cancer+normal) |
| 7-1066650 | cg04816311 | (normal vs normal) (cancer+normal vs cancer+normal) |
| 7-130021519 | cg08765199 | (normal vs normal) (cancer+normal vs cancer+normal) |
| 7-130021544 | cg21115346 | (normal vs normal) (cancer+normal vs cancer+normal) |
| 7-130021807 | cg08244181 | (normal vs normal) (cancer+normal vs cancer+normal) |
| 7-130025478 | cg17433998 | (normal vs normal) |
| 7-149979234 | cg07096883 | (normal vs normal) |
| 7-152524814 | cg04220881 | (normal vs normal) |
| 7-72738961 | cg18632634 | (normal vs normal) |
| 7-97841864 | cg22884541 | (normal vs normal) (cancer vs cancer) (cancer+normal vs cancer+normal) |
| 7-97844473 | cg21099419 | (normal vs normal) (cancer+normal vs cancer+normal) |
| 7-97857492 | cg01023808 | (normal vs normal) (cancer+normal vs cancer+normal) |
| 8-9790928 | cg04989185 | (normal vs normal) |

| | | | | | |
|---|---|---|---|---|---|
| | | (cancer+normal vs cancer+normal) | | | |
| 4-187629339 | cg01040779 | (normal vs normal) (cancer vs cancer) (cancer+normal vs cancer+normal) | 9-127602615 | cg21222350 | (normal vs normal) |
| 4-187629387 | cg25352342 | (normal vs normal) (cancer vs cancer) (cancer vs cancer) (cancer+normal vs cancer+normal) | 9-131857200 | cg13649330 | (normal vs normal) |
| 4-187629400 | cg24833566 | (normal vs normal) (cancer vs cancer) (cancer+normal vs cancer+normal) | 9-138437177 | cg13941436 | (normal vs normal) |
| 4-187629427 | cg13454542 | (normal vs normal) (cancer vs cancer) (cancer+normal vs cancer+normal) | 9-140588019 | cg23538468 | (normal vs normal) (cancer+normal vs cancer+normal) |
| 4-6945745 | cg16574807 | (normal vs normal) (cancer vs cancer) (cancer vs cancer) (cancer+normal vs cancer+normal) | 9-140770915 | cg14452299 | (normal vs normal) |
| 4-7736057 | cg27036376 | (normal vs normal) | **PRAD (cancer vs cancer)** | | |
| 4-7985238 | cg17630294 | (normal vs normal) (cancer vs cancer) (cancer+normal vs cancer+normal) | **Status - Hyper** | | |
| 4-8608486 | cg11951974 | (normal vs normal) | 1-116710943 | cg04151062 | (cancer vs cancer) |
| 5-1291888 | cg16750953 | (normal vs normal) | 1-116711075 | cg27527108 | (cancer vs cancer) (cancer+normal vs cancer+normal) |
| 5-1292339 | cg07285213 | (normal vs normal) | 1-116711077 | cg17933722 | (cancer vs cancer) (cancer+normal vs cancer+normal) |
| 5-142757312 | cg25535999 | (normal vs normal) | 1-146556479 | cg16277128 | (cancer vs cancer) (cancer+normal vs cancer+normal) |
| 5-14405747 | cg17603288 | (normal vs normal) | 1-154971922 | cg10445911 | (cancer vs cancer) |
| 5-14405920 | cg15143268 | (normal vs normal) | 1-19283761 | cg10582648 | (cancer vs cancer) (cancer+normal vs cancer+normal) |
| 6-30616468 | cg01402746 | (normal vs normal) | 1-228566718 | cg03669282 | (cancer vs cancer) (cancer+normal vs cancer+normal) |
| 6-30616472 | cg07889936 | (normal vs normal) | 1-228566824 | cg19025525 | (cancer vs cancer) (cancer+normal vs cancer+normal) |
| 6-31123044 | cg01086672 | (normal vs normal) (cancer vs cancer) (cancer vs cancer) (cancer+normal vs cancer+normal) | 1-2461820 | cg20430847 | (cancer vs cancer) (cancer+normal vs cancer+normal) |
| 6-35109435 | cg12090052 | (normal vs normal) | 1-2461823 | cg09827752 | (cancer vs cancer) (cancer+normal vs cancer+normal) |
| 6- | cg12835524 | (normal vs normal) | 1-2461834 | cg1775596 | (cancer vs cancer) |

| | | | | | |
|---|---|---|---|---|---|
| 35109485 | | | | 4 | |
| 6-38141763 | cg14151158 | (normal vs normal) | 1-24648696 | cg14616251 | (cancer vs cancer) (cancer+normal vs cancer+normal) |
| 6-99842351 | cg18751133 | (normal vs normal) | 1-24648851 | cg22176324 | (cancer vs cancer) |
| 7-1037343 | cg20449048 | (normal vs normal) | 1-33938224 | cg18085998 | (cancer vs cancer) (cancer+normal vs cancer+normal) |
| 7-150069026 | cg17677524 | (normal vs normal) (cancer vs cancer) (cancer vs cancer) (cancer+normal vs cancer+normal) | 1-33938226 | cg13303553 | (cancer vs cancer) |
| 7-150069577 | cg12613107 | (normal vs normal) (cancer vs cancer) (cancer+normal vs cancer+normal) | 1-45250181 | cg04108939 | (cancer vs cancer) |
| 7-4862349 | cg17334762 | (normal vs normal) | 1-48937404 | cg01710865 | (cancer vs cancer) |
| 7-73223852 | cg23460250 | (normal vs normal) | 10-111767379 | cg05098590 | (cancer vs cancer) (cancer+normal vs cancer+normal) |
| 7-73408058 | cg16233515 | (normal vs normal) (cancer vs cancer) (cancer vs cancer) (cancer+normal vs cancer+normal) | 10-52178225 | cg09425247 | (cancer vs cancer) |
| 7-73408060 | cg07243141 | (normal vs normal) (cancer vs cancer) (cancer vs cancer) (cancer+normal vs cancer+normal) | 10-74020976 | cg12799885 | (cancer vs cancer) (cancer+normal vs cancer+normal) |
| 7-73408101 | cg18780627 | (normal vs normal) (cancer vs cancer) (cancer vs cancer) (cancer+normal vs cancer+normal) | 10-74021022 | cg25104124 | (cancer vs cancer) |
| 7-74021938 | cg27099880 | (normal vs normal) (cancer+normal vs cancer+normal) | 11-116897558 | cg23684410 | (cancer vs cancer) (cancer+normal vs cancer+normal) |
| 7-960115 | cg18092351 | (normal vs normal) | 11-129244236 | cg13874739 | (cancer vs cancer) (cancer+normal vs cancer+normal) |
| 7-960411 | cg20928945 | (normal vs normal) | 11-35639656 | cg23083315 | (cancer vs cancer) (cancer+normal vs cancer+normal) |
| 8-141574207 | cg07466463 | (normal vs normal) | 11-58940866 | cg05415131 | (cancer vs cancer) (cancer+normal vs cancer+normal) |
| 8-142984467 | cg07295586 | (normal vs normal) | 11-78131601 | cg08481002 | (cancer vs cancer) (cancer+normal vs cancer+normal) |
| 8-22673141 | cg11847597 | (normal vs normal) | 12-89744701 | cg05769889 | (cancer vs cancer) (cancer+normal vs cancer+normal) |
| 8-61789727 | cg23625106 | (normal vs normal) (cancer vs cancer) | 13-20735532 | cg11521404 | (cancer vs cancer) |
| 8-7308492 | cg20324687 | (normal vs normal) | 14-24837892 | cg12177793 | (cancer vs cancer) |
| 9- | cg13847724 | (normal vs normal) | 14- | cg2192717 | (cancer vs cancer) |

| | | | | | |
|---|---|---|---|---|---|
| 126243651 | | (cancer vs cancer) (cancer+normal vs cancer+normal) | 65172436 | 7 | (cancer+normal vs cancer+normal) |
| **ESCA_HNSC (cancer vs cancer)** | | | 15-35014270 | cg24922143 | (cancer vs cancer) (cancer+normal vs cancer+normal) |
| **Status - Hyper** | | | 15-56536865 | cg08857201 | (cancer vs cancer) |
| 1-109939840 | cg13912858 | (cancer vs cancer) (cancer+normal vs cancer+normal) | 15-68724065 | cg14188232 | (cancer vs cancer) (cancer+normal vs cancer+normal) |
| 1-152085136 | cg20795569 | (cancer vs cancer) (cancer vs cancer) (cancer+normal vs cancer+normal) | 17-1131878 | cg11424456 | (cancer vs cancer) |
| 1-207627941 | cg10993336 | (cancer vs cancer) (cancer+normal vs cancer+normal) | 17-38465489 | cg00236832 | (cancer vs cancer) |
| 1-244213358 | cg02497700 | (cancer vs cancer) | 17-46830260 | cg13132370 | (cancer vs cancer) |
| 1-244213451 | cg14075221 | (cancer vs cancer) | 17-61554106 | cg02131967 | (cancer vs cancer) (normal vs normal) |
| 1-44401857 | cg26114324 | (cancer vs cancer) (cancer vs cancer) (cancer+normal vs cancer+normal) | 17-74706551 | cg11122493 | (cancer vs cancer) |
| 1-44401979 | cg18115627 | (cancer vs cancer) (cancer vs cancer) (cancer+normal vs cancer+normal) | 17-74706567 | cg04880618 | (cancer vs cancer) |
| 1-44402353 | cg23954153 | (cancer vs cancer) (cancer vs cancer) (cancer+normal vs cancer+normal) | 18-59560289 | cg01037637 | (cancer vs cancer) |
| 1-6546574 | cg24535622 | (cancer vs cancer) (cancer vs cancer) (cancer+normal vs cancer+normal) | 19-16187364 | cg25593560 | (cancer vs cancer) (cancer+normal vs cancer+normal) |
| 1-84767878 | cg03204322 | (cancer vs cancer) | 19-16187631 | cg16232979 | (cancer vs cancer) (cancer+normal vs cancer+normal) |
| 10-126136435 | cg05291069 | (cancer vs cancer) (cancer vs cancer) | 19-16187889 | cg26149167 | (cancer vs cancer) (cancer+normal vs cancer+normal) |
| 10-126136709 | cg06299833 | (cancer vs cancer) (cancer vs cancer) | 19-1857434 | cg15045802 | (cancer vs cancer) (cancer+normal vs cancer+normal) |
| 10-95326801 | cg13262165 | (cancer vs cancer) (cancer vs cancer) | 19-34397628 | cg06882591 | (cancer vs cancer) |
| 11-22850866 | cg01948390 | (cancer vs cancer) (cancer vs cancer) (cancer+normal vs cancer+normal) | 19-46800467 | cg02879662 | (cancer vs cancer) (cancer+normal vs cancer+normal) |
| 11-22850891 | cg03988778 | (cancer vs cancer) (cancer vs cancer) (cancer+normal vs cancer+normal) | 19-51416098 | cg17355294 | (cancer vs cancer) (cancer+normal vs cancer+normal) |
| 11-35441558 | cg18494399 | (cancer vs cancer) | 19-51416202 | cg09953499 | (cancer vs cancer) (cancer+normal vs cancer+normal) |
| 11- | cg15917625 | (cancer vs cancer) | 19- | cg1428356 | (cancer vs cancer) |

| | | | | | |
|---|---|---|---|---|---|
| 46299900 | | | 51416213 | 9 | (cancer+normal vs cancer+normal) |
| 12-113661270 | cg24281668 | (cancer vs cancer)<br>(cancer vs cancer)<br>(cancer+normal vs cancer+normal) | 19-51416517 | cg20115266 | (cancer vs cancer)<br>(cancer+normal vs cancer+normal) |
| 12-24717001 | cg17405840 | (cancer vs cancer)<br>(cancer vs cancer) | 19-51522561 | cg24512400 | (cancer vs cancer)<br>(cancer+normal vs cancer+normal) |
| 14-59931110 | cg16322262 | (cancer vs cancer)<br>(cancer vs cancer)<br>(cancer+normal vs cancer+normal) | 19-51522588 | cg15910208 | (cancer vs cancer) |
| 14-59931541 | cg22821834 | (cancer vs cancer)<br>(cancer vs cancer)<br>(cancer+normal vs cancer+normal) | 19-51522641 | cg04772683 | (cancer vs cancer)<br>(cancer+normal vs cancer+normal) |
| 14-59931922 | cg15436096 | (cancer vs cancer)<br>(cancer vs cancer)<br>(cancer+normal vs cancer+normal) | 19-51522894 | cg18437710 | (cancer vs cancer)<br>(normal vs normal) |
| 14-59932033 | cg09859398 | (cancer vs cancer)<br>(cancer vs cancer)<br>(cancer+normal vs cancer+normal) | 19-51568260 | cg03307401 | (cancer vs cancer) |
| 14-59932101 | cg04186360 | (cancer vs cancer)<br>(cancer vs cancer)<br>(cancer+normal vs cancer+normal) | 19-51568523 | cg20104456 | (cancer vs cancer) |
| 14-59932113 | cg14847688 | (cancer vs cancer)<br>(cancer vs cancer)<br>(cancer+normal vs cancer+normal) | 2-122042345 | cg14557510 | (cancer vs cancer) |
| 14-59932128 | cg05217962 | (cancer vs cancer)<br>(cancer vs cancer)<br>(cancer+normal vs cancer+normal) | 2-191045309 | cg08350814 | (cancer vs cancer)<br>(cancer+normal vs cancer+normal) |
| 14-59932146 | cg01678172 | (cancer vs cancer)<br>(cancer vs cancer)<br>(cancer+normal vs cancer+normal) | 2-191045430 | cg06532037 | (cancer vs cancer)<br>(cancer+normal vs cancer+normal) |
| 14-59932153 | cg21893185 | (cancer vs cancer)<br>(cancer vs cancer)<br>(cancer+normal vs cancer+normal) | 2-191045668 | cg15313226 | (cancer vs cancer)<br>(cancer+normal vs cancer+normal) |
| 14-59932250 | cg16411251 | (cancer vs cancer)<br>(cancer vs cancer)<br>(cancer+normal vs cancer+normal) | 2-191045697 | cg19906093 | (cancer vs cancer) |
| 14-59932273 | cg10014563 | (cancer vs cancer)<br>(cancer vs cancer)<br>(cancer+normal vs cancer+normal) | 2-43451842 | cg16876647 | (cancer vs cancer)<br>(normal vs normal)<br>(cancer+normal vs cancer+normal) |
| 14-59932289 | cg08229199 | (cancer vs cancer)<br>(cancer+normal vs cancer+normal) | 20-30458708 | cg06102403 | (cancer vs cancer) |
| 14-59932321 | cg04329105 | (cancer vs cancer) | 21-46143498 | cg04285033 | (cancer vs cancer) |
| 16-55514378 | cg09350341 | (cancer vs cancer)<br>(cancer vs cancer) | 21-47402144 | cg25020459 | (cancer vs cancer) |
| 16- | cg10026203 | (cancer vs cancer) | 3- | cg2287862 | (cancer vs cancer) |

| | | | | | |
|---|---|---|---|---|---|
| 66878654 | | (cancer vs cancer) | 169379010 | 7 | (normal vs normal) (cancer+normal vs cancer+normal) |
| 16-66878676 | cg06524479 | (cancer vs cancer) (cancer vs cancer) | 3-187455718 | cg07091593 | (cancer vs cancer) |
| 16-66878685 | cg10373196 | (cancer vs cancer) (cancer vs cancer) | 3-193776240 | cg08825929 | (cancer vs cancer) |
| 16-66878687 | cg05440435 | (cancer vs cancer) (cancer vs cancer) | 3-25469392 | cg20899354 | (cancer vs cancer) |
| 16-85932214 | cg14986962 | (cancer vs cancer) (cancer+normal vs cancer+normal) | 3-25469402 | cg26124016 | (cancer vs cancer) |
| 16-85932383 | cg06672120 | (cancer vs cancer) | 3-25469573 | cg12479047 | (cancer vs cancer) |
| 17-26554610 | cg20947476 | (cancer vs cancer) (cancer+normal vs cancer+normal) | 3-25469708 | cg03481274 | (cancer vs cancer) |
| 17-38498914 | cg16276850 | (cancer vs cancer) (cancer vs cancer) (cancer+normal vs cancer+normal) | 3-25469720 | cg02499249 | (cancer vs cancer) (cancer+normal vs cancer+normal) |
| 17-5404330 | cg22298430 | (cancer vs cancer) (cancer vs cancer) | 3-50243260 | cg14957718 | (cancer vs cancer) |
| 17-5404337 | cg27230784 | (cancer vs cancer) (cancer vs cancer) | 5-176875120 | cg14098951 | (cancer vs cancer) (cancer+normal vs cancer+normal) |
| 17-5404581 | cg18671949 | (cancer vs cancer) (cancer vs cancer) (cancer+normal vs cancer+normal) | 5-179246594 | cg15126957 | (cancer vs cancer) (cancer+normal vs cancer+normal) |
| 17-72920362 | cg25306087 | (cancer vs cancer) (cancer vs cancer) | 5-43193681 | cg10641714 | (cancer vs cancer) (cancer+normal vs cancer+normal) |
| 17-72920598 | cg09461395 | (cancer vs cancer) (cancer vs cancer) | 5-43603643 | cg26233374 | (cancer vs cancer) (cancer+normal vs cancer+normal) |
| 17-79316841 | cg00036408 | (cancer vs cancer) | 5-43604149 | cg09489281 | (cancer vs cancer) (cancer+normal vs cancer+normal) |
| 18-47087419 | cg17012181 | (cancer vs cancer) (cancer vs cancer) | 5-55776721 | cg14135025 | (cancer vs cancer) |
| 19-18335182 | cg18816098 | (cancer vs cancer) (cancer vs cancer) (cancer+normal vs cancer+normal) | 6-10426588 | cg16754467 | (cancer vs cancer) (cancer+normal vs cancer+normal) |
| 19-40314899 | cg06528584 | (cancer vs cancer) (cancer vs cancer) | 6-146136371 | cg13319975 | (cancer vs cancer) |
| 19-40314927 | cg06936564 | (cancer vs cancer) (cancer vs cancer) | 6-146136563 | cg23095615 | (cancer vs cancer) (cancer+normal vs cancer+normal) |
| 19-45595006 | cg15046935 | (cancer vs cancer) (cancer+normal vs cancer+normal) | 6-146136749 | cg09094393 | (cancer vs cancer) (cancer+normal vs cancer+normal) |
| 19-46974886 | cg10788735 | (cancer vs cancer) (cancer vs cancer) | 6-2876494 | cg17298973 | (cancer vs cancer) (cancer+normal vs cancer+normal) |
| 2-121499363 | cg06576873 | (cancer vs cancer) (cancer+normal vs cancer+normal) | 6-32223076 | cg01738650 | (cancer vs cancer) |
| 2-121499577 | cg18006328 | (cancer vs cancer) (cancer+normal vs | 7-120496827 | cg03913921 | (cancer vs cancer) (cancer+normal vs |

| | | | | | |
|---|---|---|---|---|---|
| | | cancer+normal) | | | cancer+normal) |
| 2-171387064 | cg19702675 | (cancer vs cancer) (cancer vs cancer) (cancer+normal vs cancer+normal) | 7-120628874 | cg01029638 | (cancer vs cancer) (cancer+normal vs cancer+normal) |
| 2-233285289 | cg13193840 | (cancer vs cancer) | 7-120629051 | cg15945129 | (cancer vs cancer) (cancer+normal vs cancer+normal) |
| 2-238395852 | cg24603464 | (cancer vs cancer) (cancer vs cancer) | 7-25935147 | cg13332474 | (cancer vs cancer) (normal vs normal) (cancer+normal vs cancer+normal) |
| 20-60642115 | cg03117005 | (cancer vs cancer) (cancer+normal vs cancer+normal) | 7-25935223 | cg09339462 | (cancer vs cancer) (normal vs normal) (cancer+normal vs cancer+normal) |
| 22-28010528 | cg19343611 | (cancer vs cancer) (cancer vs cancer) | 7-29185965 | cg22187722 | (cancer vs cancer) |
| 3-182834217 | cg16876162 | (cancer vs cancer) | 7-32981826 | cg08946731 | (cancer vs cancer) (cancer+normal vs cancer+normal) |
| 3-42814854 | cg09008417 | (cancer vs cancer) (cancer vs cancer) (cancer+normal vs cancer+normal) | 7-75535491 | cg03069204 | (cancer vs cancer) |
| 3-75834335 | cg27409478 | (cancer vs cancer) (cancer vs cancer) (cancer+normal vs cancer+normal) | 8-38323238 | cg13123964 | (cancer vs cancer) (cancer+normal vs cancer+normal) |
| 3-75834525 | cg02621032 | (cancer vs cancer) (cancer+normal vs cancer+normal) | 8-38323291 | cg00400221 | (cancer vs cancer) (cancer+normal vs cancer+normal) |
| 4-143766916 | cg21943416 | (cancer vs cancer) (cancer vs cancer) | 8-94713190 | cg17696629 | (cancer vs cancer) |
| 4-143767118 | cg00183107 | (cancer vs cancer) (cancer vs cancer) | 9-27528999 | cg22262168 | (cancer vs cancer) (cancer+normal vs cancer+normal) |
| 4-173961344 | cg06814616 | (cancer vs cancer) (normal vs normal) | 9-27529339 | cg21244846 | (cancer vs cancer) (cancer+normal vs cancer+normal) |
| 4-42399703 | cg16405637 | (cancer vs cancer) | 9-27529519 | cg17727989 | (cancer vs cancer) |
| 5-139030390 | cg00426310 | (cancer vs cancer) (cancer+normal vs cancer+normal) | **Status - Hypo** | | |
| 5-1594579 | cg08778598 | (cancer vs cancer) | 1-1190137 | cg19081571 | (cancer vs cancer) |
| 5-1594676 | cg21167402 | (cancer vs cancer) (cancer vs cancer) | 1-151375993 | cg14520360 | (cancer vs cancer) (cancer+normal vs cancer+normal) |
| 5-1594678 | cg24960960 | (cancer vs cancer) (cancer vs cancer) (normal vs normal) | 1-204057900 | cg04918708 | (cancer vs cancer) (cancer+normal vs cancer+normal) |
| 5-1594715 | cg21931717 | (cancer vs cancer) (cancer vs cancer) (normal vs normal) | 1-205603040 | cg19028997 | (cancer vs cancer) (cancer+normal vs cancer+normal) |
| 5-1594733 | cg08422420 | (cancer vs cancer) | 1-2342094 | cg19676238 | (cancer vs cancer) (cancer+normal vs cancer+normal) |
| 5-1594808 | cg06721546 | (cancer vs cancer) (cancer+normal vs | 1-246939216 | cg24640577 | (normal vs normal) (cancer vs cancer) |

| | | | | | |
|---|---|---|---|---|---|
| | | cancer+normal) | | | (cancer+normal vs cancer+normal) |
| 5-1594863 | cg27378537 | (cancer vs cancer) (cancer+normal vs cancer+normal) | 1-246939249 | cg06826710 | (cancer vs cancer) |
| 5-170289021 | cg20780811 | (cancer vs cancer) (cancer vs cancer) | 1-246939407 | cg23255020 | (cancer vs cancer) |
| 5-170289070 | cg03555227 | (cancer vs cancer) (cancer+normal vs cancer+normal) | 1-246939453 | cg06790053 | (cancer vs cancer) |
| 5-170289421 | cg23873669 | (cancer vs cancer) (cancer+normal vs cancer+normal) | 1-246939499 | cg08906442 | (cancer vs cancer) |
| 5-170289430 | cg07848601 | (cancer vs cancer) (cancer+normal vs cancer+normal) | 1-66747598 | cg20169766 | (cancer vs cancer) (cancer+normal vs cancer+normal) |
| 6-166722053 | cg27369401 | (cancer vs cancer) (cancer vs cancer) (cancer+normal vs cancer+normal) | 10-114591733 | cg02571204 | (normal vs normal) (cancer vs cancer) (cancer+normal vs cancer+normal) |
| 6-166722060 | cg02151625 | (cancer vs cancer) (cancer vs cancer) (cancer+normal vs cancer+normal) | 10-123781602 | cg26606947 | (cancer vs cancer) (cancer+normal vs cancer+normal) |
| 6-28092048 | cg13045351 | (cancer vs cancer) (cancer vs cancer) (cancer+normal vs cancer+normal) | 10-133783182 | cg11356398 | (cancer vs cancer) (cancer+normal vs cancer+normal) |
| 6-28304185 | cg17968647 | (cancer vs cancer) | 10-133783302 | cg18679487 | (cancer vs cancer) (cancer+normal vs cancer+normal) |
| 6-28304194 | cg13997124 | (cancer vs cancer) | 10-15174924 | cg26080545 | (cancer vs cancer) (cancer+normal vs cancer+normal) |
| 6-28304216 | cg19013262 | (cancer vs cancer) | 10-65344030 | cg11937768 | (cancer vs cancer) |
| 6-28304249 | cg09996797 | (cancer vs cancer) | 11-36652206 | cg18554866 | (normal vs normal) (cancer vs cancer) (cancer+normal vs cancer+normal) |
| 6-28304451 | cg26031541 | (cancer vs cancer) (cancer+normal vs cancer+normal) | 11-62677251 | cg22031964 | (cancer vs cancer) (cancer+normal vs cancer+normal) |
| 6-46097671 | cg23634318 | (cancer vs cancer) (cancer vs cancer) (cancer+normal vs cancer+normal) | 11-70282745 | cg04410987 | (normal vs normal) (cancer vs cancer) (cancer+normal vs cancer+normal) |
| 6-46097695 | cg06768203 | (cancer vs cancer) (cancer vs cancer) (cancer+normal vs cancer+normal) | 11-70282774 | cg14498227 | (normal vs normal) (cancer vs cancer) (cancer+normal vs cancer+normal) |
| 6-46097784 | cg08211306 | (cancer vs cancer) (cancer vs cancer) (cancer+normal vs cancer+normal) | 11-70282789 | cg14041701 | (normal vs normal) (cancer vs cancer) (cancer+normal vs cancer+normal) |
| 8-145027948 | cg24507266 | (cancer vs cancer) (cancer vs cancer) (cancer+normal vs cancer+normal) | 11-77320503 | cg16227072 | (cancer vs cancer) (cancer+normal vs cancer+normal) |
| 8-145028093 | cg06452769 | (cancer vs cancer) (cancer vs cancer) | 12-117429362 | cg03389789 | (normal vs normal) (cancer vs cancer) |

| | | | | | |
|---|---|---|---|---|---|
| | | (cancer+normal vs cancer+normal) | | | (cancer+normal vs cancer+normal) |
| 8-145028103 | cg14234406 | (cancer vs cancer) (cancer vs cancer) (cancer+normal vs cancer+normal) | 12-121841372 | cg06657560 | (cancer vs cancer) (cancer+normal vs cancer+normal) |
| 8-145028170 | cg00329447 | (cancer vs cancer) (normal vs normal) (cancer+normal vs cancer+normal) | 12-124832755 | cg27653901 | (cancer vs cancer) (cancer+normal vs cancer+normal) |
| 8-145028222 | cg02110858 | (cancer vs cancer) (normal vs normal) | 12-133354410 | cg15721142 | (cancer vs cancer) |
| 9-35757158 | cg13439181 | (cancer vs cancer) (cancer vs cancer) (cancer+normal vs cancer+normal) | 12-3930923 | cg02474926 | (normal vs normal) (cancer vs cancer) (cancer+normal vs cancer+normal) |
| 9-35757314 | cg20227976 | (cancer vs cancer) (cancer vs cancer) (cancer+normal vs cancer+normal) | 12-53995154 | cg02019508 | (cancer vs cancer) (cancer+normal vs cancer+normal) |
| 9-4299830 | cg13513288 | (cancer vs cancer) (cancer vs cancer) (cancer+normal vs cancer+normal) | 13-111935412 | cg10130374 | (cancer vs cancer) (cancer+normal vs cancer+normal) |
| 9-96713643 | cg07852402 | (cancer vs cancer) (cancer vs cancer) (normal vs normal) (cancer+normal vs cancer+normal) | 13-111935521 | cg14270002 | (normal vs normal) (cancer vs cancer) (cancer+normal vs cancer+normal) |
| **Status - Hypo** | | | 13-111936071 | cg21613549 | (cancer vs cancer) (cancer+normal vs cancer+normal) |
| 1-10704886 | cg09118017 | (cancer vs cancer) | 14-103430286 | cg26975587 | (cancer vs cancer) (cancer+normal vs cancer+normal) |
| 1-10704953 | cg16401490 | (cancer vs cancer) (cancer vs cancer) | 14-32866773 | cg21156756 | (normal vs normal) (cancer vs cancer) (cancer+normal vs cancer+normal) |
| 1-147101904 | cg07790752 | (cancer vs cancer) (cancer vs cancer) | 14-70235567 | cg16709294 | (cancer vs cancer) |
| 1-156232301 | cg10199857 | (cancer vs cancer) | 15-39742079 | cg00306851 | (normal vs normal) (cancer vs cancer) (cancer+normal vs cancer+normal) |
| 1-1564422 | cg00078456 | (cancer vs cancer) (cancer vs cancer) | 15-45803341 | cg08057475 | (normal vs normal) (cancer vs cancer) (cancer+normal vs cancer+normal) |
| 1-161928549 | cg10230257 | (cancer vs cancer) (cancer vs cancer) (cancer+normal vs cancer+normal) | 16-15154082 | cg06547203 | (cancer vs cancer) (cancer+normal vs cancer+normal) |
| 1-162337134 | cg26663636 | (cancer vs cancer) | 16-49766785 | cg00535785 | (normal vs normal) (cancer vs cancer) (cancer+normal vs cancer+normal) |
| 1-162337320 | cg12486795 | (cancer vs cancer) (cancer vs cancer) (cancer+normal vs cancer+normal) | 17-3922863 | cg26591066 | (cancer vs cancer) (cancer+normal vs cancer+normal) |

| | | | | | |
|---|---|---|---|---|---|
| 1-162337375 | cg26813162 | (cancer vs cancer) | 17-47436584 | cg07164631 | (cancer vs cancer) |
| 1-167436541 | cg01833122 | (cancer vs cancer) | 17-5392363 | cg03228407 | (cancer vs cancer) (cancer+normal vs cancer+normal) |
| 1-206958014 | cg12073436 | (cancer vs cancer) | 17-76989965 | cg23225637 | (cancer vs cancer) |
| 1-228362509 | cg18277682 | (cancer vs cancer) (cancer vs cancer) | 17-76993394 | cg00902147 | (cancer vs cancer) |
| 1-2303963 | cg18106803 | (cancer vs cancer) (cancer vs cancer) | 17-77953016 | cg14193007 | (cancer vs cancer) (cancer+normal vs cancer+normal) |
| 1-43971496 | cg25211006 | (cancer vs cancer) (cancer vs cancer) (cancer+normal vs cancer+normal) | 17-77953088 | cg04194055 | (cancer vs cancer) (cancer+normal vs cancer+normal) |
| 1-6355008 | cg25029657 | (cancer vs cancer) | 17-78006238 | cg11830876 | (normal vs normal) (cancer vs cancer) (cancer+normal vs cancer+normal) |
| 10-105845238 | cg13448625 | (cancer vs cancer) | 17-79058333 | cg02080909 | (cancer vs cancer) |
| 10-1156482 | cg03444838 | (cancer vs cancer) (cancer vs cancer) | 17-80019526 | cg20651080 | (cancer vs cancer) (cancer+normal vs cancer+normal) |
| 10-116097581 | cg04784635 | (cancer vs cancer) (cancer vs cancer) (cancer+normal vs cancer+normal) | 17-80560098 | cg19277182 | (cancer vs cancer) (cancer+normal vs cancer+normal) |
| 10-29578013 | cg24075136 | (cancer vs cancer) (cancer vs cancer) (cancer+normal vs cancer+normal) | 19-13112283 | cg22824635 | (cancer vs cancer) (cancer+normal vs cancer+normal) |
| 10-34399605 | cg11103296 | (cancer vs cancer) | 19-3381526 | cg18528593 | (cancer vs cancer) |
| 10-95847671 | cg11322432 | (cancer vs cancer) (cancer vs cancer) | 19-34718795 | cg16740364 | (cancer vs cancer) (cancer+normal vs cancer+normal) |
| 11-1543206 | cg02376282 | (cancer vs cancer) (cancer vs cancer) (cancer+normal vs cancer+normal) | 19-49703932 | cg26522368 | (cancer vs cancer) |
| 11-1543275 | cg08373904 | (cancer vs cancer) (cancer vs cancer) (cancer+normal vs cancer+normal) | 19-56608958 | cg07642822 | (cancer vs cancer) (cancer+normal vs cancer+normal) |
| 11-3187784 | cg15718663 | (normal vs normal) (cancer vs cancer) (cancer+normal vs cancer+normal) | 2-206773293 | cg26303339 | (cancer vs cancer) (cancer+normal vs cancer+normal) |
| 11-47213196 | cg05584439 | (cancer vs cancer) (cancer vs cancer) (cancer+normal vs cancer+normal) | 2-236964187 | cg01951459 | (cancer vs cancer) (cancer+normal vs cancer+normal) |
| 11-69089119 | cg24121967 | (cancer vs cancer) (cancer vs cancer) | 2-237490808 | cg27529004 | (normal vs normal) (cancer vs cancer) (cancer+normal vs cancer+normal) |
| 11-70440395 | cg01397507 | (cancer vs cancer) | 2-239079215 | cg16205389 | (cancer vs cancer) |
| 11- | cg08849509 | (cancer vs cancer) | 2- | cg1339380 | (cancer vs cancer) |

| | | | | | |
|---|---|---|---|---|---|
| 9837208 | | (cancer+normal vs cancer+normal) | 239974467 | 9 | |
| 12-118293169 | cg05652090 | (cancer vs cancer) | 2-239974554 | cg10067182 | (cancer vs cancer) (cancer+normal vs cancer+normal) |
| 12-1726076 | cg07896667 | (cancer vs cancer) (cancer vs cancer) (cancer+normal vs cancer+normal) | 2-242617935 | cg21478921 | (cancer vs cancer) (cancer+normal vs cancer+normal) |
| 13-33743647 | cg13370010 | (cancer vs cancer) (cancer vs cancer) | 2-242617946 | cg03301433 | (cancer vs cancer) (cancer+normal vs cancer+normal) |
| 14-103574566 | cg16051352 | (cancer vs cancer) | 20-5080743 | cg05097899 | (normal vs normal) (cancer vs cancer) (cancer+normal vs cancer+normal) |
| 15-45449437 | cg13570892 | (cancer vs cancer) (cancer+normal vs cancer+normal) | 20-62550734 | cg15580304 | (cancer vs cancer) |
| 15-63062631 | cg14101284 | (cancer vs cancer) (cancer vs cancer) | 20-62550740 | cg14523475 | (normal vs normal) (cancer vs cancer) |
| 15-83544284 | cg16040341 | (cancer vs cancer) | 20-62562848 | cg15457037 | (normal vs normal) (cancer vs cancer) |
| 16-1424607 | cg03998655 | (cancer vs cancer) | 22-39709853 | cg22041137 | (cancer vs cancer) (cancer+normal vs cancer+normal) |
| 16-18482212 | cg09556148 | (cancer vs cancer) | 3-128005180 | cg18192419 | (cancer vs cancer) |
| 16-84446919 | cg00838040 | (cancer vs cancer) (cancer vs cancer) (cancer+normal vs cancer+normal) | 3-57903821 | cg18712231 | (normal vs normal) (cancer vs cancer) (cancer+normal vs cancer+normal) |
| 16-84871952 | cg00308659 | (cancer vs cancer) (cancer vs cancer) | 3-69220980 | cg14155446 | (cancer vs cancer) (cancer+normal vs cancer+normal) |
| 16-928338 | cg09080920 | (cancer vs cancer) (cancer vs cancer) | 3-81604026 | cg02395434 | (cancer vs cancer) (cancer+normal vs cancer+normal) |
| 17-78801851 | cg23715732 | (cancer vs cancer) | 4-1205359 | cg07928191 | (cancer vs cancer) (cancer+normal vs cancer+normal) |
| 17-78802104 | cg08219486 | (cancer vs cancer) | 4-1205447 | cg06295238 | (cancer vs cancer) (cancer+normal vs cancer+normal) |
| 17-78820391 | cg23019125 | (cancer vs cancer) (cancer vs cancer) | 4-1206150 | cg03955568 | (cancer vs cancer) (cancer+normal vs cancer+normal) |
| 19-14552177 | cg06564132 | (cancer vs cancer) | 4-79531297 | cg10366878 | (cancer vs cancer) (cancer+normal vs cancer+normal) |
| 19-14552350 | cg13964781 | (cancer vs cancer) (cancer vs cancer) | 5-80060102 | cg07324116 | (normal vs normal) (cancer vs cancer) (cancer+normal vs cancer+normal) |
| 19-31640711 | cg01120132 | (cancer vs cancer) | 6-127781063 | cg05798147 | (normal vs normal) (cancer vs cancer) (cancer+normal vs cancer+normal) |
| 2-10395189 | cg05963605 | (cancer vs cancer) | 6-137495354 | cg22004774 | (normal vs normal) (cancer vs cancer) (cancer+normal vs |

| | | | | | |
|---|---|---|---|---|---|
| | | | | | (cancer+normal) |
| 2-19320928 | cg18011760 | (cancer vs cancer) | 6-157910065 | cg20564351 | (cancer vs cancer) (cancer+normal vs cancer+normal) |
| 2-240008082 | cg12973017 | (cancer vs cancer) | 6-30552010 | cg23104816 | (cancer vs cancer) (cancer+normal vs cancer+normal) |
| 2-240008233 | cg09468399 | (cancer vs cancer) (cancer vs cancer) (cancer+normal vs cancer+normal) | 6-3150267 | cg19927661 | (normal vs normal) (cancer vs cancer) (cancer+normal vs cancer+normal) |
| 2-25600752 | cg17774001 | (cancer vs cancer) (cancer vs cancer) (cancer+normal vs cancer+normal) | 6-34569886 | cg16526755 | (cancer vs cancer) |
| 2-97423319 | cg10155101 | (cancer vs cancer) | 7-135183939 | cg25871890 | (cancer vs cancer) (cancer+normal vs cancer+normal) |
| 2-97652489 | cg12055459 | (cancer vs cancer) (cancer vs cancer) | 7-157053014 | cg17609887 | (cancer vs cancer) |
| 2-97652491 | cg13571852 | (cancer vs cancer) (cancer vs cancer) | 7-27782764 | cg17844448 | (cancer vs cancer) (cancer+normal vs cancer+normal) |
| 20-56888536 | cg15032960 | (cancer vs cancer) (cancer vs cancer) | 7-39747662 | cg15423092 | (normal vs normal) (cancer vs cancer) (cancer+normal vs cancer+normal) |
| 22-50981121 | cg08425796 | (cancer vs cancer) (cancer vs cancer) | 7-89842741 | cg26521402 | (cancer vs cancer) (cancer+normal vs cancer+normal) |
| 3-123452994 | cg17787502 | (cancer vs cancer) | 8-103254722 | cg19365151 | (cancer vs cancer) (cancer+normal vs cancer+normal) |
| 3-195947062 | cg17572056 | (cancer vs cancer) (cancer vs cancer) | 8-144884056 | cg14733867 | (cancer vs cancer) |
| 4-1212539 | cg17158584 | (cancer vs cancer) | 8-144884124 | cg05637265 | (cancer vs cancer) (cancer+normal vs cancer+normal) |
| 4-129643491 | cg01266375 | (cancer vs cancer) (cancer vs cancer) | 8-23538610 | cg12529432 | (cancer vs cancer) (cancer+normal vs cancer+normal) |
| 4-151179898 | cg06676006 | (cancer vs cancer) (cancer vs cancer) | 8-49561756 | cg08951452 | (cancer vs cancer) (cancer+normal vs cancer+normal) |
| 5-140868130 | cg03898832 | (cancer vs cancer) (cancer vs cancer) | 8-53499558 | cg20202760 | (cancer vs cancer) (cancer+normal vs cancer+normal) |
| 5-149675916 | cg14105467 | (cancer vs cancer) (cancer vs cancer) | 8-53499582 | cg11936809 | (cancer vs cancer) (cancer+normal vs cancer+normal) |
| 5-465330 | cg15026243 | (cancer vs cancer) (cancer vs cancer) (cancer+normal vs cancer+normal) | 9-100822040 | cg14451771 | (cancer vs cancer) |
| 6-134911061 | cg05368910 | (cancer vs cancer) (cancer vs cancer) | 9-116355097 | cg14284187 | (cancer vs cancer) |
| 6-14305310 | cg00273754 | (cancer vs cancer) (cancer vs cancer) | 9-116812263 | cg13934015 | (cancer vs cancer) (cancer+normal vs cancer+normal) |
| 6-157939700 | cg09484852 | (cancer vs cancer) (cancer vs cancer) | 9-116812388 | cg26127166 | (cancer vs cancer) |

| | | | | | |
|---|---|---|---|---|---|
| 6-158478872 | cg12002139 | (cancer vs cancer) | 9-16204159 | cg14427528 | (normal vs normal) (cancer vs cancer) (cancer+normal vs cancer+normal) |
| 6-163730283 | cg10584587 | (cancer vs cancer) (cancer vs cancer) (cancer+normal vs cancer+normal) | **(cancer+normal vs cancer+normal)** | | |
| 6-168393930 | cg00664730 | (cancer vs cancer) (cancer vs cancer) | **Status - Hyper** | | |
| 6-168393963 | cg14157549 | (cancer vs cancer) | 1-116711075 | cg27527108 | (cancer vs cancer) (cancer+normal vs cancer+normal) |
| 6-168394160 | cg16301975 | (cancer vs cancer) | 1-116711077 | cg17933722 | (cancer vs cancer) (cancer+normal vs cancer+normal) |
| 6-168394191 | cg19527959 | (cancer vs cancer) (cancer vs cancer) (cancer+normal vs cancer+normal) | 1-146556479 | cg16277128 | (cancer vs cancer) (cancer+normal vs cancer+normal) |
| 6-168394198 | cg14041603 | (cancer vs cancer) (cancer vs cancer) (cancer+normal vs cancer+normal) | 1-19283761 | cg10582648 | (cancer vs cancer) (cancer+normal vs cancer+normal) |
| 6-168394477 | cg10367069 | (cancer vs cancer) (cancer vs cancer) | 1-228566718 | cg03669282 | (cancer vs cancer) (cancer+normal vs cancer+normal) |
| 6-168684623 | cg06251420 | (cancer vs cancer) (cancer vs cancer) (cancer+normal vs cancer+normal) | 1-228566824 | cg19025525 | (cancer vs cancer) (cancer+normal vs cancer+normal) |
| 6-168684757 | cg08062387 | (cancer vs cancer) (cancer vs cancer) | 1-241695164 | cg04880751 | (cancer+normal vs cancer+normal) |
| 6-6971315 | cg18679635 | (cancer vs cancer) (cancer vs cancer) (cancer+normal vs cancer+normal) | 1-2461820 | cg20430847 | (cancer vs cancer) (cancer+normal vs cancer+normal) |
| 6-6971371 | cg19751937 | (normal vs normal) (cancer vs cancer) (cancer vs cancer) (cancer+normal vs cancer+normal) | 1-2461823 | cg09827752 | (cancer vs cancer) (cancer+normal vs cancer+normal) |
| 7-1102344 | cg12692727 | (cancer vs cancer) (cancer vs cancer) (cancer+normal vs cancer+normal) | 1-24648696 | cg14616251 | (cancer vs cancer) (cancer+normal vs cancer+normal) |
| 7-120644149 | cg22092876 | (cancer vs cancer) | 1-33938224 | cg18085998 | (cancer vs cancer) (cancer+normal vs cancer+normal) |
| 7-157204364 | cg18362281 | (cancer vs cancer) (cancer vs cancer) (cancer+normal vs cancer+normal) | 10-100227782 | cg26329178 | (cancer+normal vs cancer+normal) |
| 7-157204960 | cg07993586 | (cancer vs cancer) (cancer vs cancer) | 10-111767379 | cg05098590 | (cancer vs cancer) (cancer+normal vs cancer+normal) |
| 7-1937120 | cg04326741 | (cancer vs cancer) (cancer vs cancer) | 10-74020976 | cg12799885 | (cancer vs cancer) (cancer+normal vs cancer+normal) |
| 7-94927713 | cg22798737 | (cancer vs cancer) (cancer vs cancer) | 11-116897558 | cg23684410 | (cancer vs cancer) (cancer+normal vs |

| | | | | | |
|---|---|---|---|---|---|
| | | | | | cancer+normal) |
| 7-94928249 | cg09416203 | (cancer vs cancer) | 11-116967348 | cg16858415 | (normal vs normal) (cancer+normal vs cancer+normal) |
| 8-100708621 | cg06258648 | (cancer vs cancer) (cancer vs cancer) | 11-129244105 | cg09643340 | (cancer+normal vs cancer+normal) |
| 8-118912508 | cg11829680 | (cancer vs cancer) | 11-129244122 | cg10068989 | (cancer+normal vs cancer+normal) |
| 8-119344904 | cg22533992 | (cancer vs cancer) (cancer vs cancer) | 11-129244236 | cg13874739 | (cancer vs cancer) (cancer+normal vs cancer+normal) |
| 8-121768955 | cg27039420 | (cancer vs cancer) | 11-129244505 | cg17692125 | (cancer+normal vs cancer+normal) |
| 8-141588165 | cg23287005 | (cancer vs cancer) (cancer+normal vs cancer+normal) | 11-32916120 | cg19238415 | (cancer+normal vs cancer+normal) |
| 8-143404187 | cg01785719 | (cancer vs cancer) | 11-35639656 | cg23083315 | (cancer vs cancer) (cancer+normal vs cancer+normal) |
| 8-143463488 | cg09605693 | (cancer vs cancer) (cancer+normal vs cancer+normal) | 11-56345354 | cg12738765 | (cancer+normal vs cancer+normal) |
| 8-144788492 | cg16530595 | (cancer vs cancer) (cancer vs cancer) | 11-58940866 | cg05415131 | (cancer vs cancer) (cancer+normal vs cancer+normal) |
| 8-145599544 | cg24513449 | (cancer vs cancer) (cancer vs cancer) (cancer+normal vs cancer+normal) | 11-63381144 | cg22380508 | (cancer+normal vs cancer+normal) |
| 8-145758588 | cg18811093 | (cancer vs cancer) (cancer vs cancer) | 11-78131601 | cg08481002 | (cancer vs cancer) (cancer+normal vs cancer+normal) |
| 9-139907691 | cg14173587 | (cancer vs cancer) (cancer vs cancer) (cancer+normal vs cancer+normal) | 12-89744701 | cg05769889 | (cancer vs cancer) (cancer+normal vs cancer+normal) |
| 9-86149293 | cg13858974 | (cancer vs cancer) (cancer vs cancer) | 14-37117758 | cg12051980 | (normal vs normal) (cancer+normal vs cancer+normal) |
| 9-96069142 | cg19989242 | (cancer vs cancer) (cancer vs cancer) | 14-37117947 | cg20910490 | (normal vs normal) (cancer+normal vs cancer+normal) |
| 9-96069195 | cg14505733 | (cancer vs cancer) (cancer vs cancer) | 14-65172436 | cg21927177 | (cancer vs cancer) (cancer+normal vs cancer+normal) |
| (cancer+normal vs cancer+normal) | | | 14-94917627 | cg21007342 | (cancer+normal vs cancer+normal) |
| Status - Hyper | | | 15-35014270 | cg24922143 | (cancer vs cancer) (cancer+normal vs cancer+normal) |
| 1-109939840 | cg13912858 | (cancer vs cancer) (cancer+normal vs cancer+normal) | 15-68724065 | cg14188232 | (cancer vs cancer) (cancer+normal vs cancer+normal) |
| 1-113683521 | cg21576728 | (cancer+normal vs cancer+normal) | 15-77924281 | cg06014092 | (cancer+normal vs cancer+normal) |
| 1-152085136 | cg20795569 | (cancer vs cancer) (cancer vs cancer) (cancer+normal vs cancer+normal) | 17-46659939 | cg22005990 | (normal vs normal) (cancer+normal vs cancer+normal) |
| 1-152161521 | cg26320663 | (cancer+normal vs cancer+normal) | 17-46659993 | cg21082028 | (normal vs normal) (cancer+normal vs |

| | | | | | |
|---|---|---|---|---|---|
| | | | | | (cancer+normal) |
| 1-152850698 | cg13575682 | (cancer+normal vs cancer+normal) | 17-47516312 | cg14467181 | (cancer+normal vs cancer+normal) |
| 1-207627941 | cg10993336 | (cancer vs cancer) (cancer+normal vs cancer+normal) | 19-16187364 | cg25593560 | (cancer vs cancer) (cancer+normal vs cancer+normal) |
| 1-220959931 | cg11245333 | (cancer+normal vs cancer+normal) | 19-16187631 | cg16232979 | (cancer vs cancer) (cancer+normal vs cancer+normal) |
| 1-223306857 | cg12095594 | (cancer+normal vs cancer+normal) | 19-16187889 | cg26149167 | (cancer vs cancer) (cancer+normal vs cancer+normal) |
| 1-44401857 | cg26114324 | (cancer vs cancer) (cancer vs cancer) (cancer+normal vs cancer+normal) | 19-1857434 | cg15045802 | (cancer vs cancer) (cancer+normal vs cancer+normal) |
| 1-44401979 | cg18115627 | (cancer vs cancer) (cancer vs cancer) (cancer+normal vs cancer+normal) | 19-46800467 | cg02879662 | (cancer vs cancer) (cancer+normal vs cancer+normal) |
| 1-44402353 | cg23954153 | (cancer vs cancer) (cancer vs cancer) (cancer+normal vs cancer+normal) | 19-51416098 | cg17355294 | (cancer vs cancer) (cancer+normal vs cancer+normal) |
| 1-6546574 | cg24535622 | (cancer vs cancer) (cancer vs cancer) (cancer+normal vs cancer+normal) | 19-51416202 | cg09953499 | (cancer vs cancer) (cancer+normal vs cancer+normal) |
| 10-27389908 | cg08856118 | (cancer+normal vs cancer+normal) | 19-51416213 | cg14283569 | (cancer vs cancer) (cancer+normal vs cancer+normal) |
| 10-43857904 | cg26255807 | (cancer+normal vs cancer+normal) | 19-51416517 | cg20115266 | (cancer vs cancer) (cancer+normal vs cancer+normal) |
| 10-98480124 | cg04115659 | (cancer vs cancer) (cancer+normal vs cancer+normal) | 19-51522561 | cg24512400 | (cancer vs cancer) (cancer+normal vs cancer+normal) |
| 11-11862867 | cg12492653 | (cancer+normal vs cancer+normal) | 19-51522641 | cg04772683 | (cancer vs cancer) (cancer+normal vs cancer+normal) |
| 11-123985650 | cg03673190 | (cancer+normal vs cancer+normal) | 2-176937957 | cg25180342 | (normal vs normal) (cancer+normal vs cancer+normal) |
| 11-22850866 | cg01948390 | (cancer vs cancer) (cancer vs cancer) (cancer+normal vs cancer+normal) | 2-176937971 | cg07694390 | (normal vs normal) (cancer+normal vs cancer+normal) |
| 11-22850891 | cg03988778 | (cancer vs cancer) (cancer vs cancer) (cancer+normal vs cancer+normal) | 2-176983949 | cg08992581 | (cancer+normal vs cancer+normal) |
| 11-67777823 | cg02331303 | (cancer vs cancer) (cancer vs cancer) (cancer+normal vs cancer+normal) | 2-177003280 | cg10503083 | (normal vs normal) (cancer+normal vs cancer+normal) |
| 12-113661270 | cg24281668 | (cancer vs cancer) (cancer vs cancer) (cancer+normal vs cancer+normal) | 2-191045309 | cg08350814 | (cancer vs cancer) (cancer+normal vs cancer+normal) |
| 12-39837660 | cg06416491 | (cancer+normal vs cancer+normal) | 2-191045430 | cg06532037 | (cancer vs cancer) (cancer+normal vs |

| | | | | | |
|---|---|---|---|---|---|
| | | | | | cancer+normal) |
| 14-105943425 | cg00684283 | (cancer+normal vs cancer+normal) | 2-191045668 | cg15313226 | (cancer vs cancer) (cancer+normal vs cancer+normal) |
| 14-59931110 | cg16322262 | (cancer vs cancer) (cancer vs cancer) (cancer+normal vs cancer+normal) | 2-43451842 | cg16876647 | (cancer vs cancer) (normal vs normal) (cancer+normal vs cancer+normal) |
| 14-59931541 | cg22821834 | (cancer vs cancer) (cancer vs cancer) (cancer+normal vs cancer+normal) | 20-32899806 | cg12002708 | (cancer+normal vs cancer+normal) |
| 14-59931922 | cg15436096 | (cancer vs cancer) (cancer vs cancer) (cancer+normal vs cancer+normal) | 20-55203840 | cg06630799 | (cancer+normal vs cancer+normal) |
| 14-59932033 | cg09859398 | (cancer vs cancer) (cancer vs cancer) (cancer+normal vs cancer+normal) | 20-55203849 | cg15536490 | (cancer+normal vs cancer+normal) |
| 14-59932101 | cg04186360 | (cancer vs cancer) (cancer vs cancer) (cancer+normal vs cancer+normal) | 20-56724999 | cg11800608 | (cancer+normal vs cancer+normal) |
| 14-59932113 | cg14847688 | (cancer vs cancer) (cancer vs cancer) (cancer+normal vs cancer+normal) | 3-132930478 | cg15961993 | (cancer+normal vs cancer+normal) |
| 14-59932128 | cg05217962 | (cancer vs cancer) (cancer vs cancer) (cancer+normal vs cancer+normal) | 3-169378835 | cg06201393 | (normal vs normal) (cancer+normal vs cancer+normal) |
| 14-59932146 | cg01678172 | (cancer vs cancer) (cancer vs cancer) (cancer+normal vs cancer+normal) | 3-169379010 | cg22878627 | (cancer vs cancer) (normal vs normal) (cancer+normal vs cancer+normal) |
| 14-59932153 | cg21893185 | (cancer vs cancer) (cancer vs cancer) (cancer+normal vs cancer+normal) | 3-181428046 | cg24513480 | (normal vs normal) (cancer+normal vs cancer+normal) |
| 14-59932250 | cg16411251 | (cancer vs cancer) (cancer vs cancer) (cancer+normal vs cancer+normal) | 3-181428280 | cg19258425 | (cancer+normal vs cancer+normal) |
| 14-59932273 | cg10014563 | (cancer vs cancer) (cancer vs cancer) (cancer+normal vs cancer+normal) | 3-25469720 | cg02499249 | (cancer vs cancer) (cancer+normal vs cancer+normal) |
| 14-59932289 | cg08229199 | (cancer vs cancer) (cancer+normal vs cancer+normal) | 3-46250024 | cg07115110 | (cancer+normal vs cancer+normal) |
| 14-61117067 | cg02029441 | (cancer+normal vs cancer+normal) | 4-25033126 | cg27177983 | (cancer+normal vs cancer+normal) |
| 14-99943086 | cg10923313 | (cancer+normal vs cancer+normal) | 5-176875120 | cg14098951 | (cancer vs cancer) (cancer+normal vs cancer+normal) |
| 16-83374884 | cg01093138 | (cancer+normal vs cancer+normal) | 5-177423311 | cg11916054 | (cancer+normal vs cancer+normal) |
| 16-85932214 | cg14986962 | (cancer vs cancer) (cancer+normal vs cancer+normal) | 5-179246575 | cg01152073 | (cancer+normal vs cancer+normal) |

| | | | | | |
|---|---|---|---|---|---|
| 17-26554610 | cg20947476 | (cancer vs cancer) (cancer+normal vs cancer+normal) | 5-179246594 | cg15126957 | (cancer vs cancer) (cancer+normal vs cancer+normal) |
| 17-38498914 | cg16276850 | (cancer vs cancer) (cancer vs cancer) (cancer+normal vs cancer+normal) | 5-1877309 | cg04441405 | (cancer+normal vs cancer+normal) |
| 17-53341659 | cg04219321 | (cancer+normal vs cancer+normal) | 5-43193681 | cg10641714 | (cancer vs cancer) (cancer+normal vs cancer+normal) |
| 17-5404581 | cg18671949 | (cancer vs cancer) (cancer vs cancer) (cancer+normal vs cancer+normal) | 5-43603643 | cg26233374 | (cancer vs cancer) (cancer+normal vs cancer+normal) |
| 17-72920378 | cg06353318 | (cancer vs cancer) (cancer+normal vs cancer+normal) | 5-43604149 | cg09489281 | (cancer vs cancer) (cancer+normal vs cancer+normal) |
| 17-77805596 | cg13073699 | (cancer+normal vs cancer+normal) | 6-101839808 | cg01242976 | (cancer+normal vs cancer+normal) |
| 19-10124152 | cg03720611 | (cancer+normal vs cancer+normal) | 6-10426588 | cg16754467 | (cancer vs cancer) (cancer+normal vs cancer+normal) |
| 19-18335182 | cg18816098 | (cancer vs cancer) (cancer vs cancer) (cancer+normal vs cancer+normal) | 6-136173085 | cg23090824 | (cancer+normal vs cancer+normal) |
| 19-45595006 | cg15046935 | (cancer vs cancer) (cancer+normal vs cancer+normal) | 6-146136563 | cg23095615 | (cancer vs cancer) (cancer+normal vs cancer+normal) |
| 19-53400545 | cg19660239 | (cancer vs cancer) (cancer+normal vs cancer+normal) | 6-146136749 | cg09094393 | (cancer vs cancer) (cancer+normal vs cancer+normal) |
| 2-121499363 | cg06576873 | (cancer vs cancer) (cancer+normal vs cancer+normal) | 6-168225923 | cg22321572 | (cancer+normal vs cancer+normal) |
| 2-121499577 | cg18006328 | (cancer vs cancer) (cancer+normal vs cancer+normal) | 6-2876494 | cg17298973 | (cancer vs cancer) (cancer+normal vs cancer+normal) |
| 2-171387064 | cg19702675 | (cancer vs cancer) (cancer vs cancer) (cancer+normal vs cancer+normal) | 6-30687511 | cg01340312 | (cancer+normal vs cancer+normal) |
| 2-238395125 | cg01387102 | (cancer+normal vs cancer+normal) | 7-120496827 | cg03913921 | (cancer vs cancer) (cancer+normal vs cancer+normal) |
| 20-2928233 | cg21278103 | (normal vs normal) (cancer+normal vs cancer+normal) | 7-120628113 | cg21626523 | (cancer+normal vs cancer+normal) |
| 20-42142766 | cg10360552 | (cancer+normal vs cancer+normal) | 7-120628658 | cg22126440 | (cancer+normal vs cancer+normal) |
| 20-42143174 | cg15330298 | (cancer+normal vs cancer+normal) | 7-120628874 | cg01029638 | (cancer vs cancer) (cancer+normal vs cancer+normal) |
| 20-42143399 | cg15302378 | (cancer+normal vs cancer+normal) | 7-120629051 | cg15945129 | (cancer vs cancer) (cancer+normal vs cancer+normal) |
| 20-42143502 | cg02472486 | (cancer+normal vs cancer+normal) | 7-25935147 | cg13332474 | (cancer vs cancer) (normal vs normal) (cancer+normal vs cancer+normal) |
| 20- | cg03117005 | (cancer vs cancer) | 7-25935223 | cg0933946 | (cancer vs cancer) |

244

| | | |
|---|---|---|
| 60642115 | | (cancer+normal vs cancer+normal) |
| 22-19744051 | cg15487105 | (normal vs normal) (cancer+normal vs cancer+normal) |
| 22-19744062 | cg21910543 | (normal vs normal) (cancer+normal vs cancer+normal) |
| 22-31091262 | cg23331322 | (cancer+normal vs cancer+normal) |
| 3-170077111 | cg08348121 | (cancer+normal vs cancer+normal) |
| 3-42814854 | cg09008417 | (cancer vs cancer) (cancer vs cancer) (cancer+normal vs cancer+normal) |
| 3-75834335 | cg27409478 | (cancer vs cancer) (cancer vs cancer) (cancer+normal vs cancer+normal) |
| 3-75834525 | cg02621032 | (cancer vs cancer) (cancer+normal vs cancer+normal) |
| 4-27050410 | cg17934894 | (cancer+normal vs cancer+normal) |
| 5-126852998 | cg02762363 | (cancer+normal vs cancer+normal) |
| 5-126853024 | cg04431054 | (cancer+normal vs cancer+normal) |
| 5-137475177 | cg22656956 | (normal vs normal) (cancer+normal vs cancer+normal) |
| 5-139030390 | cg00426310 | (cancer vs cancer) (cancer+normal vs cancer+normal) |
| 5-1594808 | cg06721546 | (cancer vs cancer) (cancer+normal vs cancer+normal) |
| 5-1594863 | cg27378537 | (cancer vs cancer) (cancer+normal vs cancer+normal) |
| 5-170289070 | cg03555227 | (cancer vs cancer) (cancer+normal vs cancer+normal) |
| 5-170289421 | cg23873669 | (cancer vs cancer) (cancer+normal vs cancer+normal) |
| 5-170289430 | cg07848601 | (cancer vs cancer) (cancer+normal vs cancer+normal) |
| 5-2253129 | cg22626548 | (cancer+normal vs cancer+normal) |
| 6-1311165 | cg17153122 | (cancer+normal vs cancer+normal) |

| | | |
|---|---|---|
| | 2 | (normal vs normal) (cancer+normal vs cancer+normal) |
| 7-27130090 | cg20307885 | (cancer+normal vs cancer+normal) |
| 7-32981826 | cg08946731 | (cancer vs cancer) (cancer+normal vs cancer+normal) |
| 7-83097669 | cg14519356 | (cancer+normal vs cancer+normal) |
| 8-124529273 | cg12897164 | (cancer+normal vs cancer+normal) |
| 8-23553798 | cg11823496 | (cancer+normal vs cancer+normal) |
| 8-38323238 | cg13123964 | (cancer vs cancer) (cancer+normal vs cancer+normal) |
| 8-38323291 | cg00400221 | (cancer vs cancer) (cancer+normal vs cancer+normal) |
| 9-27528999 | cg22262168 | (cancer vs cancer) (cancer+normal vs cancer+normal) |
| 9-27529339 | cg21244846 | (cancer vs cancer) (cancer+normal vs cancer+normal) |
| **Status - Hypo** | | |
| 1-151375993 | cg14520360 | (cancer vs cancer) (cancer+normal vs cancer+normal) |
| 1-204057900 | cg04918708 | (cancer vs cancer) (cancer+normal vs cancer+normal) |
| 1-205603040 | cg19028997 | (cancer vs cancer) (cancer+normal vs cancer+normal) |
| 1-2342094 | cg19676238 | (cancer vs cancer) (cancer+normal vs cancer+normal) |
| 1-246939216 | cg24640577 | (normal vs normal) (cancer vs cancer) (cancer+normal vs cancer+normal) |
| 1-66747598 | cg20169766 | (cancer vs cancer) (cancer+normal vs cancer+normal) |
| 10-112655570 | cg05999324 | (cancer+normal vs cancer+normal) |
| 10-114591733 | cg02571204 | (normal vs normal) (cancer vs cancer) (cancer+normal vs cancer+normal) |
| 10-123781602 | cg26606947 | (cancer vs cancer) (cancer+normal vs cancer+normal) |

| | | | | | |
|---|---|---|---|---|---|
| 6-166722053 | cg27369401 | (cancer vs cancer)<br>(cancer vs cancer)<br>(cancer+normal vs cancer+normal) | 10-133783182 | cg11356398 | (cancer vs cancer)<br>(cancer+normal vs cancer+normal) |
| 6-166722060 | cg02151625 | (cancer vs cancer)<br>(cancer vs cancer)<br>(cancer+normal vs cancer+normal) | 10-133783302 | cg18679487 | (cancer vs cancer)<br>(cancer+normal vs cancer+normal) |
| 6-27280423 | cg11262757 | (cancer+normal vs cancer+normal) | 10-15174924 | cg26080545 | (cancer vs cancer)<br>(cancer+normal vs cancer+normal) |
| 6-28092048 | cg13045351 | (cancer vs cancer)<br>(cancer vs cancer)<br>(cancer+normal vs cancer+normal) | 11-130746300 | cg07740705 | (cancer+normal vs cancer+normal) |
| 6-28304451 | cg26031541 | (cancer vs cancer)<br>(cancer+normal vs cancer+normal) | 11-36652206 | cg18554866 | (normal vs normal)<br>(cancer vs cancer)<br>(cancer+normal vs cancer+normal) |
| 6-46097521 | cg00567696 | (cancer vs cancer)<br>(cancer+normal vs cancer+normal) | 11-62677251 | cg22031964 | (cancer vs cancer)<br>(cancer+normal vs cancer+normal) |
| 6-46097671 | cg23634318 | (cancer vs cancer)<br>(cancer vs cancer)<br>(cancer+normal vs cancer+normal) | 11-70282745 | cg04410987 | (normal vs normal)<br>(cancer vs cancer)<br>(cancer+normal vs cancer+normal) |
| 6-46097695 | cg06768203 | (cancer vs cancer)<br>(cancer vs cancer)<br>(cancer+normal vs cancer+normal) | 11-70282774 | cg14498227 | (normal vs normal)<br>(cancer vs cancer)<br>(cancer+normal vs cancer+normal) |
| 6-46097784 | cg08211306 | (cancer vs cancer)<br>(cancer vs cancer)<br>(cancer+normal vs cancer+normal) | 11-70282789 | cg14041701 | (normal vs normal)<br>(cancer vs cancer)<br>(cancer+normal vs cancer+normal) |
| 7-55225183 | cg20706768 | (cancer+normal vs cancer+normal) | 11-77320503 | cg16227072 | (cancer vs cancer)<br>(cancer+normal vs cancer+normal) |
| 8-140847721 | cg23681110 | (cancer+normal vs cancer+normal) | 12-117429362 | cg03389789 | (normal vs normal)<br>(cancer vs cancer)<br>(cancer+normal vs cancer+normal) |
| 8-145027948 | cg24507266 | (cancer vs cancer)<br>(cancer vs cancer)<br>(cancer+normal vs cancer+normal) | 12-121841372 | cg06657560 | (cancer vs cancer)<br>(cancer+normal vs cancer+normal) |
| 8-145028093 | cg06452769 | (cancer vs cancer)<br>(cancer vs cancer)<br>(cancer+normal vs cancer+normal) | 12-124832755 | cg27653901 | (cancer vs cancer)<br>(cancer+normal vs cancer+normal) |
| 8-145028103 | cg14234406 | (cancer vs cancer)<br>(cancer vs cancer)<br>(cancer+normal vs cancer+normal) | 12-131390894 | cg18793198 | (cancer+normal vs cancer+normal) |
| 8-145028170 | cg00329447 | (cancer vs cancer)<br>(normal vs normal)<br>(cancer+normal vs cancer+normal) | 12-3930923 | cg02474926 | (normal vs normal)<br>(cancer vs cancer)<br>(cancer+normal vs cancer+normal) |
| 9-131872542 | cg26805528 | (cancer+normal vs cancer+normal) | 12-53995154 | cg02019508 | (cancer vs cancer)<br>(cancer+normal vs cancer+normal) |
| 9- | cg13439181 | (cancer vs cancer) | 13- | cg1013037 | (cancer vs cancer) |

246

| Position | CpG | Comparison | Position | CpG | Comparison |
|---|---|---|---|---|---|
| 35757158 | | (cancer vs cancer) (cancer+normal vs cancer+normal) | 111935412 | 4 | (cancer+normal vs cancer+normal) |
| 9-35757314 | cg20227976 | (cancer vs cancer) (cancer vs cancer) (cancer+normal vs cancer+normal) | 13-111935508 | cg22795345 | (normal vs normal) (cancer+normal vs cancer+normal) |
| 9-4299830 | cg13513288 | (cancer vs cancer) (cancer vs cancer) (cancer+normal vs cancer+normal) | 13-111935521 | cg14270002 | (normal vs normal) (cancer vs cancer) (cancer+normal vs cancer+normal) |
| 9-96713643 | cg07852402 | (cancer vs cancer) (cancer vs cancer) (normal vs normal) (cancer+normal vs cancer+normal) | 13-111936071 | cg21613549 | (cancer vs cancer) (cancer+normal vs cancer+normal) |
| 9-96714295 | cg14374754 | (normal vs normal) (cancer+normal vs cancer+normal) | 14-103430286 | cg26975587 | (cancer vs cancer) (cancer+normal vs cancer+normal) |
| 9-96714313 | cg22902266 | (normal vs normal) (cancer+normal vs cancer+normal) | 14-105213100 | cg27312798 | (cancer+normal vs cancer+normal) |
| **Status - Hypo** | | | 14-32866773 | cg21156756 | (normal vs normal) (cancer vs cancer) (cancer+normal vs cancer+normal) |
| 1-10707917 | cg20318235 | (cancer+normal vs cancer+normal) | 15-39742079 | cg00306851 | (normal vs normal) (cancer vs cancer) (cancer+normal vs cancer+normal) |
| 1-10707953 | cg03962214 | (normal vs normal) (cancer+normal vs cancer+normal) | 15-45803341 | cg08057475 | (normal vs normal) (cancer vs cancer) (cancer+normal vs cancer+normal) |
| 1-1564920 | cg17122213 | (cancer vs cancer) (cancer+normal vs cancer+normal) | 15-75104987 | cg02395127 | (cancer+normal vs cancer+normal) |
| 1-161928549 | cg10230257 | (cancer vs cancer) (cancer vs cancer) (cancer+normal vs cancer+normal) | 15-79723173 | cg15189715 | (normal vs normal) (cancer+normal vs cancer+normal) |
| 1-162337320 | cg12486795 | (cancer vs cancer) (cancer vs cancer) (cancer+normal vs cancer+normal) | 16-15154082 | cg06547203 | (cancer vs cancer) (cancer+normal vs cancer+normal) |
| 1-18010807 | cg11742472 | (cancer+normal vs cancer+normal) | 16-49766785 | cg00535785 | (normal vs normal) (cancer vs cancer) (cancer+normal vs cancer+normal) |
| 1-204124158 | cg21101726 | (normal vs normal) (cancer+normal vs cancer+normal) | 17-35480754 | cg08013737 | (cancer+normal vs cancer+normal) |
| 1-26190114 | cg00465927 | (cancer+normal vs cancer+normal) | 17-3922863 | cg26591066 | (cancer vs cancer) (cancer+normal vs cancer+normal) |
| 1-3623605 | cg21418707 | (cancer+normal vs cancer+normal) | 17-5392363 | cg03228407 | (cancer vs cancer) (cancer+normal vs cancer+normal) |
| 1-43202523 | cg06654076 | (cancer+normal vs cancer+normal) | 17-663064 | cg20353884 | (normal vs normal) (cancer+normal vs cancer+normal) |

| | | | | | |
|---|---|---|---|---|---|
| 1-43971496 | cg25211006 | (cancer vs cancer) (cancer vs cancer) (cancer+normal vs cancer+normal) | 17-77953016 | cg14193007 | (cancer vs cancer) (cancer+normal vs cancer+normal) |
| 10-105216262 | cg19853077 | (cancer+normal vs cancer+normal) | 17-77953088 | cg04194055 | (cancer vs cancer) (cancer+normal vs cancer+normal) |
| 10-116097581 | cg04784635 | (cancer vs cancer) (cancer vs cancer) (cancer+normal vs cancer+normal) | 17-78006238 | cg11830876 | (normal vs normal) (cancer vs cancer) (cancer+normal vs cancer+normal) |
| 10-123493091 | cg25724837 | (cancer vs cancer) (cancer+normal vs cancer+normal) | 17-80019526 | cg20651080 | (cancer vs cancer) (cancer+normal vs cancer+normal) |
| 10-134458209 | cg26472690 | (cancer+normal vs cancer+normal) | 17-80560098 | cg19277182 | (cancer vs cancer) (cancer+normal vs cancer+normal) |
| 10-29578013 | cg24075136 | (cancer vs cancer) (cancer vs cancer) (cancer+normal vs cancer+normal) | 17-80560124 | cg07891774 | (cancer+normal vs cancer+normal) |
| 10-88445516 | cg16862260 | (cancer vs cancer) (cancer+normal vs cancer+normal) | 17-9088206 | cg09305503 | (cancer+normal vs cancer+normal) |
| 11-1543206 | cg02376282 | (cancer vs cancer) (cancer vs cancer) (cancer+normal vs cancer+normal) | 19-13110842 | cg22983694 | (cancer+normal vs cancer+normal) |
| 11-1543275 | cg08373904 | (cancer vs cancer) (cancer vs cancer) (cancer+normal vs cancer+normal) | 19-13112283 | cg22824635 | (cancer vs cancer) (cancer+normal vs cancer+normal) |
| 11-17803389 | cg08129093 | (cancer vs cancer) (cancer+normal vs cancer+normal) | 19-34718795 | cg16740364 | (cancer vs cancer) (cancer+normal vs cancer+normal) |
| 11-3187784 | cg15718663 | (normal vs normal) (cancer vs cancer) (cancer+normal vs cancer+normal) | 19-5587909 | cg22022797 | (cancer+normal vs cancer+normal) |
| 11-47213196 | cg05584439 | (cancer vs cancer) (cancer vs cancer) (cancer+normal vs cancer+normal) | 19-56608958 | cg07642822 | (cancer vs cancer) (cancer+normal vs cancer+normal) |
| 11-69151092 | cg24568082 | (normal vs normal) (cancer+normal vs cancer+normal) | 2-151112658 | cg11032640 | (cancer+normal vs cancer+normal) |
| 11-9837208 | cg08849509 | (cancer vs cancer) (cancer+normal vs cancer+normal) | 2-206773293 | cg26303339 | (cancer vs cancer) (cancer+normal vs cancer+normal) |
| 12-117190080 | cg02528386 | (cancer+normal vs cancer+normal) | 2-236964187 | cg01951459 | (cancer vs cancer) (cancer+normal vs cancer+normal) |
| 12-1726033 | cg24854010 | (cancer+normal vs cancer+normal) | 2-237490808 | cg27529004 | (normal vs normal) (cancer vs cancer) (cancer+normal vs cancer+normal) |
| 12-1726076 | cg07896667 | (cancer vs cancer) (cancer vs cancer) (cancer+normal vs cancer+normal) | 2-239974554 | cg10067182 | (cancer vs cancer) (cancer+normal vs cancer+normal) |
| 13- | cg04769514 | (cancer+normal vs | 2- | cg0808148 | (cancer+normal vs |

| | | | | | |
|---|---|---|---|---|---|
| 30088640 | | cancer+normal) | 242617771 | 5 | cancer+normal) |
| 13-30088771 | cg02381064 | (normal vs normal) (cancer+normal vs cancer+normal) | 2-242617935 | cg21478921 | (cancer vs cancer) (cancer+normal vs cancer+normal) |
| 14-105851883 | cg10348368 | (normal vs normal) (cancer+normal vs cancer+normal) | 2-242617946 | cg03301433 | (cancer vs cancer) (cancer+normal vs cancer+normal) |
| 14-55138530 | cg10888242 | (cancer+normal vs cancer+normal) | 2-46113875 | cg23478225 | (cancer+normal vs cancer+normal) |
| 14-91744445 | cg15313956 | (cancer+normal vs cancer+normal) | 20-5080743 | cg05097899 | (normal vs normal) (cancer vs cancer) (cancer+normal vs cancer+normal) |
| 15-37379321 | cg09970590 | (cancer+normal vs cancer+normal) | 22-39709853 | cg22041137 | (cancer vs cancer) (cancer+normal vs cancer+normal) |
| 15-42234343 | cg20935245 | (cancer+normal vs cancer+normal) | 3-57903821 | cg18712231 | (normal vs normal) (cancer vs cancer) (cancer+normal vs cancer+normal) |
| 15-45449437 | cg13570892 | (cancer vs cancer) (cancer+normal vs cancer+normal) | 3-69220980 | cg14155446 | (cancer vs cancer) (cancer+normal vs cancer+normal) |
| 16-4587038 | cg05396425 | (normal vs normal) (cancer+normal vs cancer+normal) | 3-81604026 | cg02395434 | (cancer vs cancer) (cancer+normal vs cancer+normal) |
| 16-84446919 | cg00838040 | (cancer vs cancer) (cancer vs cancer) (cancer+normal vs cancer+normal) | 4-1205359 | cg07928191 | (cancer vs cancer) (cancer+normal vs cancer+normal) |
| 16-87857222 | cg01779679 | (normal vs normal) (cancer+normal vs cancer+normal) | 4-1205447 | cg06295238 | (cancer vs cancer) (cancer+normal vs cancer+normal) |
| 16-87871160 | cg01829163 | (cancer+normal vs cancer+normal) | 4-1206150 | cg03955568 | (cancer vs cancer) (cancer+normal vs cancer+normal) |
| 16-87878292 | cg02614661 | (cancer+normal vs cancer+normal) | 4-54667491 | cg14833626 | (normal vs normal) (cancer+normal vs cancer+normal) |
| 17-11872861 | cg24945092 | (cancer+normal vs cancer+normal) | 4-79531297 | cg10366878 | (cancer vs cancer) (cancer+normal vs cancer+normal) |
| 17-47629873 | cg26026296 | (cancer+normal vs cancer+normal) | 5-80060102 | cg07324116 | (normal vs normal) (cancer vs cancer) (cancer+normal vs cancer+normal) |
| 17-78968450 | cg17714010 | (cancer+normal vs cancer+normal) | 6-127781063 | cg05798147 | (normal vs normal) (cancer vs cancer) (cancer+normal vs cancer+normal) |
| 17-79032487 | cg03916756 | (cancer+normal vs cancer+normal) | 6-137495354 | cg22004774 | (normal vs normal) (cancer vs cancer) (cancer+normal vs cancer+normal) |
| 17-79056117 | cg18173351 | (cancer+normal vs cancer+normal) | 6-157910065 | cg20564351 | (cancer vs cancer) (cancer+normal vs cancer+normal) |
| 17-927457 | cg22006392 | (cancer+normal vs cancer+normal) | 6-160201987 | cg24280851 | (cancer+normal vs cancer+normal) |
| 18-28572450 | cg24245840 | (cancer+normal vs cancer+normal) | 6-30552010 | cg23104816 | (cancer vs cancer) (cancer+normal vs |

| | | | | | |
|---|---|---|---|---|---|
| | | | | | cancer+normal) |
| 18-77230665 | cg16536399 | (cancer+normal vs cancer+normal) | 6-30552025 | cg20635067 | (cancer+normal vs cancer+normal) |
| 19-1117289 | cg07962882 | (cancer+normal vs cancer+normal) | 6-3150267 | cg19927661 | (normal vs normal) (cancer vs cancer) (cancer+normal vs cancer+normal) |
| 19-1117453 | cg09432775 | (cancer+normal vs cancer+normal) | 6-36020012 | cg16145324 | (cancer+normal vs cancer+normal) |
| 19-18418056 | cg08006956 | (cancer+normal vs cancer+normal) | 7-135183939 | cg25871890 | (cancer vs cancer) (cancer+normal vs cancer+normal) |
| 19-7580275 | cg00351537 | (cancer+normal vs cancer+normal) | 7-27782764 | cg17844448 | (cancer vs cancer) (cancer+normal vs cancer+normal) |
| 2-235242882 | cg21517506 | (normal vs normal) (cancer+normal vs cancer+normal) | 7-39747662 | cg15423092 | (normal vs normal) (cancer vs cancer) (cancer+normal vs cancer+normal) |
| 2-240008233 | cg09468399 | (cancer vs cancer) (cancer vs cancer) (cancer+normal vs cancer+normal) | 7-7474236 | cg13879411 | (cancer+normal vs cancer+normal) |
| 2-25600752 | cg17774001 | (cancer vs cancer) (cancer vs cancer) (cancer+normal vs cancer+normal) | 7-778962 | cg00207279 | (cancer+normal vs cancer+normal) |
| 2-54315563 | cg17091056 | (cancer+normal vs cancer+normal) | 7-89842741 | cg26521402 | (cancer vs cancer) (cancer+normal vs cancer+normal) |
| 2-55382186 | cg23574298 | (normal vs normal) (cancer+normal vs cancer+normal) | 7-99515080 | cg05832509 | (cancer+normal vs cancer+normal) |
| 20-36151120 | cg11252765 | (cancer+normal vs cancer+normal) | 8-103254722 | cg19365151 | (cancer vs cancer) (cancer+normal vs cancer+normal) |
| 20-60926800 | cg01572549 | (normal vs normal) (cancer+normal vs cancer+normal) | 8-12594834 | cg23719367 | (normal vs normal) (cancer+normal vs cancer+normal) |
| 22-18531447 | cg08819022 | (cancer+normal vs cancer+normal) | 8-144884124 | cg05637265 | (cancer vs cancer) (cancer+normal vs cancer+normal) |
| 22-30230951 | cg18253910 | (cancer+normal vs cancer+normal) | 8-23538610 | cg12529432 | (cancer vs cancer) (cancer+normal vs cancer+normal) |
| 22-40417538 | cg26241650 | (cancer+normal vs cancer+normal) | 8-49561756 | cg08951452 | (cancer vs cancer) (cancer+normal vs cancer+normal) |
| 3-184349562 | cg22607164 | (normal vs normal) (cancer+normal vs cancer+normal) | 8-53499558 | cg20202760 | (cancer vs cancer) (cancer+normal vs cancer+normal) |
| 3-50361554 | cg24335984 | (cancer+normal vs cancer+normal) | 8-53499582 | cg11936809 | (cancer vs cancer) (cancer+normal vs cancer+normal) |
| 3-64338594 | cg22475071 | (cancer+normal vs cancer+normal) | 9-116812263 | cg13934015 | (cancer vs cancer) (cancer+normal vs cancer+normal) |
| 4-166428151 | cg15403758 | (cancer+normal vs cancer+normal) | 9-130027116 | cg13802789 | (cancer+normal vs cancer+normal) |
| 5-175955353 | cg19416326 | (cancer+normal vs cancer+normal) | 9-16204159 | cg14427528 | (normal vs normal) (cancer vs cancer) |

| | | | | | (cancer+normal vs cancer+normal) |
|---|---|---|---|---|---|
| 5-175955925 | cg20748242 | (cancer vs cancer) (cancer+normal vs cancer+normal) | 9-93943683 | cg21245718 | (cancer+normal vs cancer+normal) |
| 5-393347 | cg17287155 | (normal vs normal) (cancer+normal vs cancer+normal) | **(normal vs normal)** | | |
| 5-465330 | cg15026243 | (cancer vs cancer) (cancer vs cancer) (cancer+normal vs cancer+normal) | **Status - Hyper** | | |
| 5-65528850 | cg08853399 | (normal vs normal) (cancer+normal vs cancer+normal) | 1-18956947 | cg25020286 | (normal vs normal) |
| 6-12355642 | cg19147483 | (cancer+normal vs cancer+normal) | 1-20512944 | cg11651220 | (normal vs normal) |
| 6-158464296 | cg00407393 | (cancer+normal vs cancer+normal) | 1-228195779 | cg18642177 | (normal vs normal) |
| 6-163730283 | cg10584587 | (cancer vs cancer) (cancer vs cancer) (cancer+normal vs cancer+normal) | 1-86043795 | cg20431191 | (normal vs normal) |
| 6-168147420 | cg06389605 | (cancer+normal vs cancer+normal) | 10-8089182 | cg16952963 | (normal vs normal) |
| 6-168394191 | cg19527959 | (cancer vs cancer) (cancer vs cancer) (cancer+normal vs cancer+normal) | 10-8089733 | cg20281962 | (normal vs normal) |
| 6-168394198 | cg14041603 | (cancer vs cancer) (cancer vs cancer) (cancer+normal vs cancer+normal) | 10-8094860 | cg00407546 | (normal vs normal) |
| 6-168684623 | cg06251420 | (cancer vs cancer) (cancer vs cancer) (cancer+normal vs cancer+normal) | 11-116967348 | cg16858415 | (normal vs normal) (cancer+normal vs cancer+normal) |
| 6-30166164 | cg09579704 | (cancer+normal vs cancer+normal) | 11-13300592 | cg15603424 | (normal vs normal) |
| 6-6971315 | cg18679635 | (cancer vs cancer) (cancer vs cancer) (cancer+normal vs cancer+normal) | 12-54339653 | cg03239552 | (normal vs normal) |
| 6-6971335 | cg09146892 | (cancer vs cancer) (cancer+normal vs cancer+normal) | 13-20735819 | cg25524962 | (normal vs normal) |
| 6-6971371 | cg19751937 | (normal vs normal) (cancer vs cancer) (cancer vs cancer) (cancer+normal vs cancer+normal) | 14-37116121 | cg26397549 | (normal vs normal) |
| 7-1102344 | cg12692727 | (cancer vs cancer) (cancer vs cancer) (cancer+normal vs cancer+normal) | 14-37117758 | cg12051980 | (normal vs normal) (cancer+normal vs cancer+normal) |
| 7-1102375 | cg02733842 | (cancer+normal vs cancer+normal) | 14-37117862 | cg05897809 | (normal vs normal) |
| 7-130630462 | cg16852756 | (cancer+normal vs cancer+normal) | 14-37117947 | cg20910490 | (normal vs normal) (cancer+normal vs cancer+normal) |
| 7- | cg18362281 | (cancer vs cancer) | 14- | cg0184394 | (normal vs normal) |

| | | | | | |
|---|---|---|---|---|---|
| 157204364 | | (cancer vs cancer) (cancer+normal vs cancer+normal) | 37124434 | 6 | |
| 7-2094977 | cg22717323 | (normal vs normal) (cancer+normal vs cancer+normal) | 14-37125914 | cg00970313 | (normal vs normal) |
| 7-4231054 | cg03976326 | (normal vs normal) (cancer+normal vs cancer+normal) | 14-37126582 | cg23655970 | (normal vs normal) |
| 7-6192053 | cg22136051 | (cancer+normal vs cancer+normal) | 14-37128586 | cg04415798 | (normal vs normal) |
| 8-141566084 | cg11007190 | (normal vs normal) (cancer+normal vs cancer+normal) | 14-37130212 | cg07459252 | (normal vs normal) |
| 8-141588165 | cg23287005 | (cancer vs cancer) (cancer+normal vs cancer+normal) | 14-37136391 | cg18361098 | (normal vs normal) |
| 8-141588242 | cg05827089 | (cancer+normal vs cancer+normal) | 14-60972853 | cg12811449 | (normal vs normal) |
| 8-143463488 | cg09605693 | (cancer vs cancer) (cancer+normal vs cancer+normal) | 14-61118751 | cg15425541 | (normal vs normal) |
| 8-144696651 | cg16888603 | (normal vs normal) (cancer+normal vs cancer+normal) | 14-61120662 | cg13007843 | (normal vs normal) |
| 8-145009137 | cg10299941 | (normal vs normal) (cancer+normal vs cancer+normal) | 15-83953880 | cg17124224 | (normal vs normal) |
| 8-145009241 | cg08159271 | (normal vs normal) (cancer+normal vs cancer+normal) | 15-83953883 | cg06523224 | (normal vs normal) |
| 8-145599544 | cg24513449 | (cancer vs cancer) (cancer vs cancer) (cancer+normal vs cancer+normal) | 15-83953929 | cg07016258 | (normal vs normal) |
| 8-99179021 | cg22623649 | (cancer+normal vs cancer+normal) | 17-46620347 | cg09328349 | (normal vs normal) |
| 9-116337285 | cg14327394 | (cancer+normal vs cancer+normal) | 17-46659939 | cg22005990 | (normal vs normal) (cancer+normal vs cancer+normal) |
| 9-139907691 | cg14173587 | (cancer vs cancer) (cancer vs cancer) (cancer+normal vs cancer+normal) | 17-46659993 | cg21082028 | (normal vs normal) (cancer+normal vs cancer+normal) |
| **(normal vs normal)** | | | 17-46831623 | cg27204671 | (normal vs normal) |
| **Status - Hyper** | | | 18-43355414 | cg25416153 | (normal vs normal) |
| 1-207996459 | cg15393490 | (normal vs normal) | 18-43355547 | cg18088442 | (normal vs normal) |
| 1-229406711 | cg16909962 | (normal vs normal) | 2-176931680 | cg00674679 | (normal vs normal) |
| 1-2391317 | cg07700843 | (normal vs normal) | 2-176936581 | cg00459623 | (normal vs normal) |
| 1-2391347 | cg02850689 | (normal vs normal) | 2-176937957 | cg25180342 | (normal vs normal) (cancer+normal vs cancer+normal) |
| 1-42921582 | cg06520846 | (normal vs normal) | 2-176937971 | cg07694390 | (normal vs normal) (cancer+normal vs cancer+normal) |
| 1- | cg06346081 | (normal vs normal) | 2- | cg0780235 | (normal vs normal) |

| | | | | | |
|---|---|---|---|---|---|
| 42921584 | | | 176956573 | 0 | |
| 1-66999753 | cg02994349 | (normal vs normal) | 2-176983815 | cg26708100 | (normal vs normal) |
| 1-66999813 | cg25203031 | (normal vs normal) | 2-176987404 | cg14991487 | (normal vs normal) |
| 10-102896640 | cg06382559 | (normal vs normal) | 2-176987411 | cg09578028 | (normal vs normal) |
| 10-102896869 | cg20120208 | (normal vs normal) | 2-176987462 | cg04739647 | (normal vs normal) |
| 10-102899262 | cg14383658 | (normal vs normal) | 2-176988203 | cg22541735 | (normal vs normal) |
| 10-102899285 | cg05482942 | (normal vs normal) | 2-176988284 | cg14236662 | (normal vs normal) |
| 10-1336103 | cg27336068 | (normal vs normal) | 2-177001909 | cg25820279 | (normal vs normal) |
| 11-12699661 | cg08537660 | (normal vs normal) | 2-177003280 | cg10503083 | (normal vs normal) (cancer+normal vs cancer+normal) |
| 11-35441777 | cg21163960 | (normal vs normal) | 2-200329872 | cg00586817 | (normal vs normal) |
| 11-35441881 | cg10159951 | (normal vs normal) | 2-200330782 | cg09250678 | (normal vs normal) |
| 11-69785905 | cg18220961 | (normal vs normal) | 2-200330851 | cg09240763 | (normal vs normal) |
| 11-76839217 | cg14708990 | (normal vs normal) | 2-200332025 | cg27109600 | (normal vs normal) |
| 12-32654992 | cg22431093 | (normal vs normal) | 2-200332380 | cg07444473 | (normal vs normal) |
| 13-111212174 | cg16728323 | (normal vs normal) | 2-200334439 | cg24345062 | (normal vs normal) |
| 13-51086835 | cg06255601 | (normal vs normal) | 2-200334901 | cg07913197 | (normal vs normal) |
| 13-80706924 | cg20100768 | (normal vs normal) | 2-200335101 | cg25740565 | (normal vs normal) |
| 13-98796790 | cg18252102 | (normal vs normal) | 2-234847769 | cg00793935 | (normal vs normal) |
| 14-50584005 | cg17923947 | (normal vs normal) | 2-64836449 | cg09950256 | (normal vs normal) |
| 16-67433458 | cg09451235 | (normal vs normal) | 2-64836764 | cg27470373 | (normal vs normal) |
| 16-86598792 | cg05390968 | (normal vs normal) | 3-169378835 | cg06201393 | (normal vs normal) (cancer+normal vs cancer+normal) |
| 16-86598824 | cg09814487 | (normal vs normal) | 3-169379010 | cg22878627 | (cancer vs cancer) (normal vs normal) (cancer+normal vs cancer+normal) |
| 17-32404422 | cg14826891 | (normal vs normal) | 3-169384410 | cg04120686 | (normal vs normal) |
| 17-48042779 | cg21342910 | (normal vs normal) | 3-181428046 | cg24513480 | (normal vs normal) (cancer+normal vs cancer+normal) |
| 17-48049952 | cg23634124 | (normal vs normal) | 4-1037615 | cg09680447 | (normal vs normal) |
| 17-48628397 | cg16312900 | (normal vs normal) | 4-109092769 | cg03149565 | (normal vs normal) |
| 17-48628455 | cg12765405 | (normal vs normal) | 5-134363877 | cg04223420 | (normal vs normal) |
| 17-66194706 | cg05247809 | (normal vs normal) | 5-134364387 | cg00396667 | (normal vs normal) |
| 17- | cg13674575 | (normal vs normal) | 5- | cg1267310 | (normal vs normal) |

| | | | | | |
|---|---|---|---|---|---|
| 66195134 | | | 134364717 | 3 | |
| 17-66196681 | cg09396295 | (normal vs normal) | 5-134376247 | cg09839170 | (normal vs normal) |
| 17-70611386 | cg14431361 | (normal vs normal) | 5-134376442 | cg13441766 | (normal vs normal) |
| 17-9110369 | cg07114544 | (normal vs normal) | 5-2741047 | cg16072192 | (normal vs normal) |
| 19-11998651 | cg26578856 | (normal vs normal) | 5-2748369 | cg24630195 | (normal vs normal) |
| 19-18714552 | cg25383503 | (normal vs normal) | 5-2753746 | cg02509058 | (normal vs normal) |
| 19-45975990 | cg02360514 | (normal vs normal) | 5-2753852 | cg13944468 | (normal vs normal) |
| 19-45976195 | cg12542255 | (normal vs normal) | 6-10382828 | cg20168806 | (normal vs normal) |
| 2-131722274 | cg22572908 | (normal vs normal) | 6-10420696 | cg17557766 | (normal vs normal) |
| 2-131722307 | cg10004780 | (normal vs normal) | 6-10420798 | cg00689580 | (normal vs normal) |
| 2-158694670 | cg16682903 | (normal vs normal) | 6-10421001 | cg10129408 | (normal vs normal) |
| 2-238394933 | cg14070755 | (normal vs normal) | 6-10421069 | cg14993900 | (normal vs normal) |
| 2-238395007 | cg17187163 | (normal vs normal) | 6-10421372 | cg02407720 | (normal vs normal) |
| 2-28456184 | cg00470044 | (normal vs normal) | 6-28956577 | cg02766845 | (normal vs normal) |
| 2-63284066 | cg23229261 | (normal vs normal) | 7-158824108 | cg23058037 | (normal vs normal) |
| 2-63285365 | cg05092308 | (normal vs normal) | 7-25935147 | cg13332474 | (cancer vs cancer) (normal vs normal) (cancer+normal vs cancer+normal) |
| 2-63285425 | cg19351026 | (normal vs normal) | 7-25935223 | cg09339462 | (cancer vs cancer) (normal vs normal) (cancer+normal vs cancer+normal) |
| 20-20352002 | cg24752267 | (normal vs normal) | 7-27196759 | cg08934785 | (normal vs normal) |
| 20-2928233 | cg21278103 | (normal vs normal) (cancer+normal vs cancer+normal) | 7-27286075 | cg22964918 | (normal vs normal) |
| 21-19258040 | cg14288673 | (normal vs normal) | 7-96639411 | cg22505086 | (normal vs normal) |
| 22-19743336 | cg06782697 | (normal vs normal) | 7-96641477 | cg09388140 | (normal vs normal) |
| 22-19743892 | cg26520942 | (normal vs normal) | 7-96643539 | cg25707676 | (normal vs normal) |
| 22-19744051 | cg15487105 | (normal vs normal) (cancer+normal vs cancer+normal) | 7-96647021 | cg23262036 | (normal vs normal) |
| 22-19744062 | cg21910543 | (normal vs normal) (cancer+normal vs cancer+normal) | 7-96647079 | cg25928986 | (normal vs normal) |
| 22-19744078 | cg16076038 | (normal vs normal) | 7-96647117 | cg01680010 | (normal vs normal) |
| 22-22862884 | cg20361154 | (normal vs normal) | 7-96647323 | cg00388195 | (normal vs normal) |
| 22-38635773 | cg02100011 | (normal vs normal) | 7-96650096 | cg09359114 | (normal vs normal) |
| 3- | cg13621410 | (normal vs normal) | 7-96650171 | cg1204138 | (normal vs normal) |

| Position | cg | Comparison |
|---|---|---|
| 136537694 | | |
| 3-194408516 | cg11862731 | (normal vs normal) |
| 3-194408845 | cg09652652 | (normal vs normal) |
| 3-194408901 | cg00352417 | (normal vs normal) |
| 3-194408965 | cg10623840 | (normal vs normal) |
| 3-42094749 | cg20372886 | (normal vs normal) |
| 3-58045340 | cg26295272 | (normal vs normal) |
| 4-86851411 | cg14376467 | (normal vs normal) |
| 5-137474700 | cg13707560 | (normal vs normal) |
| 5-137474830 | cg16354091 | (normal vs normal) |
| 5-137474905 | cg09047573 | (normal vs normal) |
| 5-137475002 | cg04233770 | (normal vs normal) |
| 5-137475177 | cg22656956 | (normal vs normal) (cancer+normal vs cancer+normal) |
| 5-137475379 | cg20452230 | (normal vs normal) |
| 5-72529100 | cg23340350 | (normal vs normal) |
| 5-72529127 | cg17791977 | (normal vs normal) |
| 5-72530472 | cg23517208 | (normal vs normal) |
| 5-72739768 | cg19092317 | (normal vs normal) |
| 6-12365899 | cg08015382 | (normal vs normal) |
| 6-50786369 | cg01555614 | (normal vs normal) |
| 6-50786511 | cg17507887 | (normal vs normal) |
| 6-50792868 | cg18886245 | (normal vs normal) |
| 7-29186889 | cg14525310 | (normal vs normal) |
| 7-63768139 | cg04636841 | (normal vs normal) |
| 7-97680731 | cg18676790 | (normal vs normal) |
| 8-145028170 | cg00329447 | (cancer vs cancer) (normal vs normal) (cancer+normal vs cancer+normal) |
| 8-22447068 | cg09125300 | (normal vs normal) |
| 7-96650192 | cg08835113 | (normal vs normal) |
| 8-99951420 | cg14888916 | (normal vs normal) |
| 8-99951730 | cg03020554 | (normal vs normal) |
| 8-99952047 | cg06992285 | (normal vs normal) |
| 8-99955610 | cg22920700 | (normal vs normal) |
| 8-99960148 | cg04768602 | (normal vs normal) |
| 8-99960498 | cg02409878 | (normal vs normal) |
| **Status - Hypo** | | |
| 1-17760589 | cg06356708 | (normal vs normal) |
| 1-180911763 | cg05638165 | (normal vs normal) |
| 1-193077840 | cg03750903 | (normal vs normal) |
| 1-246939216 | cg24640577 | (normal vs normal) (cancer vs cancer) (cancer+normal vs cancer+normal) |
| 1-37973652 | cg17226042 | (normal vs normal) |
| 10-114591733 | cg02571204 | (normal vs normal) (cancer vs cancer) (cancer+normal vs cancer+normal) |
| 10-133529939 | cg15003570 | (normal vs normal) |
| 10-26678169 | cg15728146 | (normal vs normal) |
| 11-102206714 | cg08847737 | (normal vs normal) |
| 11-36652206 | cg18554866 | (normal vs normal) (cancer vs cancer) (cancer+normal vs cancer+normal) |
| 11-63776653 | cg19960891 | (normal vs normal) |
| 11-64120728 | cg05233289 | (normal vs normal) |
| 11-66556756 | cg03094226 | (normal vs normal) |
| 11-69468789 | cg04717045 | (normal vs normal) |
| 11-69468991 | cg02723533 | (normal vs normal) |
| 11-69469113 | cg00347938 | (normal vs normal) |
| 11-70282745 | cg04410987 | (normal vs normal) (cancer vs cancer) (cancer+normal vs cancer+normal) |
| 11-70282774 | cg14498227 | (normal vs normal) (cancer vs cancer) |

| | | | | | |
|---|---|---|---|---|---|
| | | | | | (cancer+normal vs cancer+normal) |
| 9-4300203 | cg10836809 | (normal vs normal) | 11-70282789 | cg14041701 | (normal vs normal) (cancer vs cancer) (cancer+normal vs cancer+normal) |
| 9-96709791 | cg13985688 | (normal vs normal) | 12-10958919 | cg21156620 | (normal vs normal) |
| 9-96712772 | cg06194070 | (normal vs normal) | 12-110785080 | cg00752047 | (normal vs normal) |
| 9-96713643 | cg07852402 | (cancer vs cancer) (cancer vs cancer) (normal vs normal) (cancer+normal vs cancer+normal) | 12-117429362 | cg03389789 | (normal vs normal) (cancer vs cancer) (cancer+normal vs cancer+normal) |
| 9-96714295 | cg14374754 | (normal vs normal) (cancer+normal vs cancer+normal) | 12-131493258 | cg18725348 | (normal vs normal) |
| 9-96714313 | cg22902266 | (normal vs normal) (cancer+normal vs cancer+normal) | 12-3930923 | cg02474926 | (normal vs normal) (cancer vs cancer) (cancer+normal vs cancer+normal) |
| 9-96715047 | cg10454937 | (normal vs normal) | 12-75961704 | cg24032056 | (normal vs normal) |
| 9-96715256 | cg13398482 | (cancer vs cancer) (normal vs normal) | 12-98986887 | cg16871561 | (normal vs normal) |
| 9-96715687 | cg00927495 | (normal vs normal) | 13-111935508 | cg22795345 | (normal vs normal) (cancer+normal vs cancer+normal) |
| 9-96721817 | cg14316431 | (normal vs normal) | 13-111935521 | cg14270002 | (normal vs normal) (cancer vs cancer) (cancer+normal vs cancer+normal) |
| 9-96722329 | cg07184658 | (normal vs normal) | 13-112602415 | cg03616722 | (normal vs normal) |
| 9-96723014 | cg14428080 | (normal vs normal) | 13-112927190 | cg09473364 | (normal vs normal) |
| 9-96723033 | cg09155774 | (normal vs normal) | 14-32866773 | cg21156756 | (normal vs normal) (cancer vs cancer) (cancer+normal vs cancer+normal) |
| **Status - Hypo** | | | 14-51230329 | cg20512044 | (normal vs normal) |
| 1-10707953 | cg03962214 | (normal vs normal) (cancer+normal vs cancer+normal) | 14-82028411 | cg12069158 | (normal vs normal) |
| 1-153536528 | cg00647881 | (normal vs normal) | 15-39742079 | cg00306851 | (normal vs normal) (cancer vs cancer) (cancer+normal vs cancer+normal) |
| 1-153536563 | cg27310485 | (normal vs normal) | 15-45803341 | cg08057475 | (normal vs normal) (cancer vs cancer) (cancer+normal vs cancer+normal) |
| 1-156211415 | cg11580676 | (normal vs normal) | 15-56643419 | cg05907398 | (normal vs normal) |
| 1-1563500 | cg09159050 | (normal vs normal) | 15-79723173 | cg15189715 | (normal vs normal) (cancer+normal vs cancer+normal) |
| 1-204124158 | cg21101726 | (normal vs normal) (cancer+normal vs | 16-1599072 | cg08214329 | (normal vs normal) |

| | | | | | |
|---|---|---|---|---|---|
| | | cancer+normal) | | | |
| 1-2142534 | cg04597658 | (normal vs normal) | 16-49766785 | cg00535785 | (normal vs normal) (cancer vs cancer) (cancer+normal vs cancer+normal) |
| 1-2304315 | cg09129839 | (normal vs normal) | 16-65708104 | cg03880673 | (normal vs normal) |
| 1-3626994 | cg17441982 | (normal vs normal) | 16-73247500 | cg06684296 | (normal vs normal) |
| 1-898852 | cg03148373 | (normal vs normal) | 16-88111009 | cg08250921 | (normal vs normal) |
| 10-5638431 | cg16993911 | (normal vs normal) | 16-88111362 | cg04747693 | (normal vs normal) |
| 11-119993751 | cg04745079 | (normal vs normal) | 17-58966213 | cg02372144 | (normal vs normal) |
| 11-119994722 | cg26247168 | (normal vs normal) | 17-663064 | cg20353884 | (normal vs normal) (cancer+normal vs cancer+normal) |
| 11-128813183 | cg13962286 | (normal vs normal) | 17-76798203 | cg17278466 | (normal vs normal) |
| 11-28422367 | cg17589284 | (normal vs normal) | 17-78006238 | cg11830876 | (normal vs normal) (cancer vs cancer) (cancer+normal vs cancer+normal) |
| 11-3187777 | cg03857384 | (normal vs normal) | 18-12437948 | cg01295785 | (normal vs normal) |
| 11-3187784 | cg15718663 | (normal vs normal) (cancer vs cancer) (cancer+normal vs cancer+normal) | 2-12907913 | cg18763247 | (normal vs normal) |
| 11-3187803 | cg05982473 | (normal vs normal) | 2-144995067 | cg18406255 | (normal vs normal) |
| 11-69151092 | cg24568082 | (normal vs normal) (cancer+normal vs cancer+normal) | 2-19376860 | cg16439608 | (normal vs normal) |
| 12-20193597 | cg07274333 | (normal vs normal) | 2-217301204 | cg05311180 | (normal vs normal) |
| 12-57559901 | cg27419119 | (normal vs normal) | 2-219994830 | cg26152051 | (normal vs normal) |
| 12-66050928 | cg07677157 | (normal vs normal) | 2-237490808 | cg27529004 | (normal vs normal) (cancer vs cancer) (cancer+normal vs cancer+normal) |
| 13-109803528 | cg24719241 | (normal vs normal) | 2-24816070 | cg27015161 | (normal vs normal) |
| 13-110591252 | cg02355680 | (normal vs normal) | 2-39627269 | cg24008893 | (normal vs normal) |
| 13-113672808 | cg12762449 | (normal vs normal) | 2-39952015 | cg02965283 | (normal vs normal) |
| 13-30088771 | cg02381064 | (normal vs normal) (cancer+normal vs cancer+normal) | 2-98507477 | cg17016175 | (normal vs normal) |
| 13-31747897 | cg19968890 | (normal vs normal) | 20-13971811 | cg10640284 | (normal vs normal) |
| 14-105851883 | cg10348368 | (normal vs normal) (cancer+normal vs cancer+normal) | 20-5080743 | cg05097899 | (normal vs normal) (cancer vs cancer) (cancer+normal vs cancer+normal) |
| 14-34247003 | cg13496915 | (normal vs normal) | 20-55072651 | cg23037777 | (normal vs normal) |
| 14- | cg23660866 | (normal vs normal) | 22- | cg0956136 | (normal vs normal) |

| | | | | | |
|---|---|---|---|---|---|
| 95009914 | | | 45676755 | 5 | |
| 15-58797839 | cg27307975 | (normal vs normal) | 22-50445676 | cg16744710 | (normal vs normal) |
| 15-61097246 | cg14222307 | (normal vs normal) | 3-100270300 | cg24157982 | (normal vs normal) |
| 16-1742021 | cg10458734 | (normal vs normal) | 3-126554736 | cg22572820 | (normal vs normal) |
| 16-1742245 | cg06775420 | (normal vs normal) | 3-45190814 | cg10929614 | (normal vs normal) |
| 16-1813796 | cg05711506 | (normal vs normal) | 3-57903821 | cg18712231 | (normal vs normal) (cancer vs cancer) (cancer+normal vs cancer+normal) |
| 16-31447114 | cg06095047 | (normal vs normal) | 3-66798910 | cg09377980 | (normal vs normal) |
| 16-4587038 | cg05396425 | (normal vs normal) (cancer+normal vs cancer+normal) | 3-73503670 | cg13050115 | (normal vs normal) |
| 16-7807001 | cg04736751 | (normal vs normal) | 4-114679761 | cg16503611 | (normal vs normal) |
| 16-87857222 | cg01779679 | (normal vs normal) (cancer+normal vs cancer+normal) | 4-177804777 | cg03582419 | (normal vs normal) |
| 17-8128471 | cg09361941 | (normal vs normal) | 4-54667491 | cg14833626 | (normal vs normal) (cancer+normal vs cancer+normal) |
| 18-20811408 | cg01288184 | (normal vs normal) | 4-74487609 | cg05516499 | (normal vs normal) |
| 2-216708370 | cg12535109 | (normal vs normal) | 5-107450780 | cg23022206 | (normal vs normal) |
| 2-235242882 | cg21517506 | (normal vs normal) (cancer+normal vs cancer+normal) | 5-137365576 | cg24566595 | (normal vs normal) |
| 2-241383517 | cg27214687 | (normal vs normal) | 5-13945611 | cg19540371 | (normal vs normal) |
| 2-55382186 | cg23574298 | (normal vs normal) (cancer+normal vs cancer+normal) | 5-14374397 | cg15689969 | (normal vs normal) |
| 2-65839377 | cg13659933 | (normal vs normal) | 5-177853481 | cg07858195 | (normal vs normal) |
| 20-60926800 | cg01572549 | (normal vs normal) (cancer+normal vs cancer+normal) | 5-177853574 | cg18009093 | (normal vs normal) |
| 22-20050298 | cg03175417 | (normal vs normal) | 5-5320334 | cg03824768 | (normal vs normal) |
| 3-110110880 | cg23471798 | (normal vs normal) | 5-58412295 | cg18425032 | (normal vs normal) |
| 3-184349562 | cg22607164 | (normal vs normal) (cancer+normal vs cancer+normal) | 5-80060102 | cg07324116 | (normal vs normal) (cancer vs cancer) (cancer+normal vs cancer+normal) |
| 3-196065357 | cg21090033 | (normal vs normal) | 6-127781063 | cg05798147 | (normal vs normal) (cancer vs cancer) (cancer+normal vs cancer+normal) |
| 3-66002927 | cg00779602 | (normal vs normal) | 6-127977312 | cg27232401 | (normal vs normal) |
| 3-66002991 | cg06109867 | (normal vs normal) | 6-137495354 | cg22004774 | (normal vs normal) (cancer vs cancer) (cancer+normal vs cancer+normal) |

| | | | | | |
|---|---|---|---|---|---|
| 4-183769227 | cg25209938 | (normal vs normal) | 6-3150267 | cg19927661 | (normal vs normal) (cancer vs cancer) (cancer+normal vs cancer+normal) |
| 4-188256637 | cg26221941 | (normal vs normal) | 6-32933013 | cg12033125 | (normal vs normal) |
| 4-26095438 | cg16847428 | (normal vs normal) | 6-34566245 | cg02827572 | (normal vs normal) |
| 4-70861449 | cg00436282 | (normal vs normal) | 6-44629403 | cg11412703 | (normal vs normal) |
| 5-131725325 | cg23475112 | (normal vs normal) | 6-65214835 | cg23028841 | (normal vs normal) |
| 5-393347 | cg17287155 | (normal vs normal) (cancer+normal vs cancer+normal) | 6-9116428 | cg00734931 | (normal vs normal) |
| 5-65528850 | cg08853399 | (normal vs normal) (cancer+normal vs cancer+normal) | 7-148730143 | cg05518778 | (normal vs normal) |
| 5-912599 | cg14499678 | (normal vs normal) | 7-27565815 | cg20567361 | (normal vs normal) |
| 5-912821 | cg17510385 | (normal vs normal) | 7-27595446 | cg23208678 | (normal vs normal) |
| 6-107926037 | cg00944666 | (normal vs normal) | 7-37086890 | cg18425586 | (normal vs normal) |
| 6-11771473 | cg26130669 | (normal vs normal) | 7-39747662 | cg15423092 | (normal vs normal) (cancer vs cancer) (cancer+normal vs cancer+normal) |
| 6-11771680 | cg10301879 | (normal vs normal) | 8-12594834 | cg23719367 | (normal vs normal) (cancer+normal vs cancer+normal) |
| 6-160463767 | cg04837722 | (normal vs normal) | 8-60537981 | cg25335258 | (normal vs normal) |
| 6-168748492 | cg05923105 | (normal vs normal) | 8-80819477 | cg20035127 | (normal vs normal) |
| 6-168748530 | cg02840092 | (normal vs normal) (cancer vs cancer) | 9-16204159 | cg14427528 | (normal vs normal) (cancer vs cancer) (cancer+normal vs cancer+normal) |
| 6-170560097 | cg24852883 | (normal vs normal) | **ReGiCaG (cancer vs cancer)** | | |
| 6-31823344 | cg12651173 | (normal vs normal) | **Status - Hyper** | | |
| 6-33131450 | cg26867987 | (normal vs normal) | 1-11539599 | cg09018444 | (cancer vs cancer) |
| 6-33131560 | cg04249605 | (normal vs normal) | 1-165325673 | cg04351049 | (cancer vs cancer) |
| 6-3517953 | cg22745369 | (normal vs normal) | 1-29586580 | cg21929771 | (cancer vs cancer) (normal vs normal) |
| 6-6894163 | cg02096172 | (normal vs normal) | 1-47691160 | cg10810752 | (cancer vs cancer) |
| 6-6971371 | cg19751937 | (normal vs normal) (cancer vs cancer) (cancer vs cancer) (cancer+normal vs cancer+normal) | 1-62784651 | cg09588271 | (cancer vs cancer) |
| 7-131720532 | cg13223721 | (normal vs normal) | 10-11059577 | cg23858040 | (cancer vs cancer) |
| 7-1604448 | cg01726448 | (normal vs normal) | 10-124895441 | cg03908015 | (cancer vs cancer) |
| 7-2094977 | cg22717323 | (normal vs normal) | 10- | cg1868540 | (cancer vs cancer) |

| | | | 124895448 | 8 | |
|---|---|---|---|---|---|
| 7-4231054 | cg03976326 | (normal vs normal) (cancer+normal vs cancer+normal) | 10-124895461 | cg14663510 | (cancer vs cancer) |
| 7-4802535 | cg15247247 | (normal vs normal) | 10-131762052 | cg11757357 | (cancer vs cancer) |
| 7-5647786 | cg01687558 | (normal vs normal) | 10-20104497 | cg24022375 | (cancer vs cancer) (cancer vs cancer) (cancer+normal vs cancer+normal) |
| 7-6290148 | cg23728359 | (normal vs normal) | 10-20104935 | cg17603206 | (cancer vs cancer) (cancer+normal vs cancer+normal) |
| 8-102019870 | cg21341817 | (normal vs normal) | 10-20104948 | cg08625590 | (cancer vs cancer) (cancer vs cancer) (cancer+normal vs cancer+normal) |
| 8-103817950 | cg11313780 | (normal vs normal) | 10-71812612 | cg26166804 | (cancer vs cancer) |
| 8-1274678 | cg15654779 | (normal vs normal) | 10-71813286 | cg03349922 | (cancer vs cancer) |
| 8-1274686 | cg22152500 | (normal vs normal) | 10-79398055 | cg24113782 | (cancer vs cancer) |
| 8-141566084 | cg11007190 | (normal vs normal) (cancer+normal vs cancer+normal) | 10-85954316 | cg11591516 | (cancer vs cancer) |
| 8-143463227 | cg00293626 | (normal vs normal) | 10-85954331 | cg16678602 | (cancer vs cancer) |
| 8-144590015 | cg25834632 | (normal vs normal) | 10-85954336 | cg03611007 | (cancer vs cancer) |
| 8-144696651 | cg16888603 | (normal vs normal) (cancer+normal vs cancer+normal) | 11-117667862 | cg16764494 | (cancer vs cancer) |
| 8-145009137 | cg10299941 | (normal vs normal) (cancer+normal vs cancer+normal) | 12-2161661 | cg01637551 | (cancer vs cancer) |
| 8-145009241 | cg08159271 | (normal vs normal) (cancer+normal vs cancer+normal) | 12-21927433 | cg09125430 | (cancer vs cancer) |
| 8-30556362 | cg27498434 | (normal vs normal) | 12-48398370 | cg20255775 | (cancer vs cancer) (cancer vs cancer) |
| 8-337539 | cg15469184 | (normal vs normal) | 13-110437093 | cg05404236 | (cancer vs cancer) |
| 8-41628552 | cg05885688 | (normal vs normal) | 15-29862517 | cg04283162 | (cancer vs cancer) (normal vs normal) |
| 8-79713763 | cg25465344 | (normal vs normal) | 17-17685407 | cg25927164 | (cancer vs cancer) (cancer vs cancer) (cancer+normal vs cancer+normal) |
| 8-99180671 | cg01822827 | (normal vs normal) | 17-17685582 | cg15906409 | (cancer vs cancer) (cancer vs cancer) (cancer+normal vs cancer+normal) |
| 8-99180740 | cg03381604 | (normal vs normal) | 17-17686141 | cg19872299 | (cancer vs cancer) (cancer vs cancer) (normal vs normal) (cancer+normal vs cancer+normal) |
| 9-138289504 | cg13794641 | (normal vs normal) | 17-43972806 | cg19670923 | (cancer vs cancer) |

| | | | | | |
|---|---|---|---|---|---|
| 9-139530300 | cg14520832 | (normal vs normal) | 17-43972978 | cg18368019 | (cancer vs cancer) (cancer vs cancer) |
| **GBM_LGG (cancer vs cancer)** | | | 17-48619672 | cg27437806 | (cancer vs cancer) (cancer vs cancer) (cancer+normal vs cancer+normal) |
| **Status - Hyper** | | | 17-5974064 | cg18898440 | (cancer vs cancer) |
| 1-11919464 | cg18533883 | (cancer vs cancer) | 17-75447478 | cg14517217 | (cancer vs cancer) (cancer vs cancer) (normal vs normal) (cancer+normal vs cancer+normal) |
| 1-150522121 | cg16559275 | (cancer vs cancer) | 17-75447504 | cg18104645 | (cancer vs cancer) (cancer vs cancer) (normal vs normal) (cancer+normal vs cancer+normal) |
| 1-27894955 | cg12610832 | (cancer vs cancer) (cancer vs cancer) (cancer+normal vs cancer+normal) | 17-79373624 | cg00712390 | (cancer vs cancer) (cancer+normal vs cancer+normal) |
| 10-75172806 | cg17276002 | (cancer vs cancer) (cancer+normal vs cancer+normal) | 17-8055756 | cg23363911 | (cancer vs cancer) (cancer vs cancer) (cancer+normal vs cancer+normal) |
| 11-119191974 | cg06285590 | (cancer vs cancer) | 18-42260234 | cg20127035 | (cancer vs cancer) |
| 11-126456052 | cg16628183 | (cancer vs cancer) (cancer+normal vs cancer+normal) | 18-7566656 | cg22141456 | (cancer vs cancer) |
| 11-65190999 | cg07985890 | (cancer vs cancer) | 18-7566781 | cg16474170 | (cancer vs cancer) (cancer+normal vs cancer+normal) |
| 11-67272636 | cg15676015 | (cancer vs cancer) | 19-31841161 | cg23641852 | (cancer vs cancer) |
| 11-72463673 | cg20513950 | (cancer vs cancer) | 19-31841391 | cg00545804 | (cancer vs cancer) |
| 12-107297551 | cg16848054 | (cancer vs cancer) | 19-31841555 | cg15208375 | (cancer vs cancer) (cancer vs cancer) |
| 12-117316490 | cg21334598 | (cancer vs cancer) | 19-31841559 | cg12986271 | (cancer vs cancer) |
| 12-125670513 | cg06880239 | (cancer vs cancer) (cancer vs cancer) | 19-31841663 | cg11199770 | (cancer vs cancer) (cancer vs cancer) (normal vs normal) (cancer+normal vs cancer+normal) |
| 12-57504948 | cg01063813 | (cancer vs cancer) (cancer vs cancer) (normal vs normal) (cancer+normal vs cancer+normal) | 19-31841952 | cg15711268 | (cancer vs cancer) (cancer vs cancer) (cancer+normal vs cancer+normal) |
| 12-6493003 | cg23079808 | (cancer vs cancer) (cancer vs cancer) (cancer+normal vs cancer+normal) | 19-45656942 | cg21574675 | (cancer vs cancer) (cancer vs cancer) (cancer+normal vs cancer+normal) |
| 12-6493278 | cg10452531 | (cancer vs cancer) (cancer vs cancer) | 19-51228831 | cg18723560 | (cancer vs cancer) |
| 12-94136549 | cg21266616 | (cancer vs cancer) | 19-58661833 | cg24884572 | (cancer vs cancer) (cancer vs cancer) |
| 12- | cg03861966 | (cancer vs cancer) | 2- | cg2154443 | (cancer vs cancer) |

| | | | | | |
|---|---|---|---|---|---|
| 94136602 | | | 114260879 | 7 | |
| 13-114927854 | cg11997708 | (cancer vs cancer) (cancer vs cancer) (normal vs normal) (cancer+normal vs cancer+normal) | 2-177053345 | cg19001226 | (cancer vs cancer) |
| 13-27557503 | cg03268893 | (cancer vs cancer) (normal vs normal) (cancer+normal vs cancer+normal) | 2-177502854 | cg04739123 | (cancer vs cancer) |
| 14-24835006 | cg12930714 | (cancer vs cancer) (cancer vs cancer) | 2-45395956 | cg25206134 | (cancer vs cancer) |
| 14-24898992 | cg10859966 | (cancer vs cancer) | 21-40984780 | cg08448665 | (cancer vs cancer) (cancer vs cancer) (cancer+normal vs cancer+normal) |
| 14-32670315 | cg01174771 | (cancer vs cancer) (cancer vs cancer) | 22-17849639 | cg26796679 | (cancer vs cancer) |
| 14-70038925 | cg02380334 | (cancer vs cancer) (cancer vs cancer) (normal vs normal) (cancer+normal vs cancer+normal) | 22-21319245 | cg16590005 | (cancer vs cancer) (cancer vs cancer) (cancer+normal vs cancer+normal) |
| 15-34611057 | cg15586327 | (cancer vs cancer) | 22-21319661 | cg07960806 | (cancer vs cancer) (cancer vs cancer) (cancer+normal vs cancer+normal) |
| 15-65127973 | cg25489524 | (cancer vs cancer) | 3-128337033 | cg10691592 | (cancer vs cancer) |
| 15-65128112 | cg24977462 | (cancer vs cancer) | 3-170136489 | cg21474897 | (cancer vs cancer) |
| 15-67143691 | cg12317470 | (cancer vs cancer) | 3-170136499 | cg21389743 | (cancer vs cancer) |
| 15-93198495 | cg18558647 | (cancer vs cancer) (cancer vs cancer) | 3-77089110 | cg17189465 | (cancer vs cancer) (cancer+normal vs cancer+normal) |
| 16-25078095 | cg04153495 | (cancer vs cancer) | 4-13546151 | cg27438798 | (cancer vs cancer) (cancer+normal vs cancer+normal) |
| 16-4233246 | cg05006755 | (cancer vs cancer) (cancer vs cancer) (cancer+normal vs cancer+normal) | 4-13546169 | cg20393324 | (cancer vs cancer) |
| 16-56390811 | cg03300177 | (cancer vs cancer) | 4-141294016 | cg17333211 | (cancer vs cancer) |
| 16-56390830 | cg10504751 | (cancer vs cancer) | 4-166300290 | cg02886284 | (cancer vs cancer) (cancer vs cancer) (cancer+normal vs cancer+normal) |
| 17-1553202 | cg04313875 | (cancer vs cancer) (cancer vs cancer) (normal vs normal) (cancer+normal vs cancer+normal) | 4-166300445 | cg00599280 | (cancer vs cancer) |
| 17-16256990 | cg13180678 | (cancer vs cancer) (cancer vs cancer) | 4-170947241 | cg10469329 | (cancer vs cancer) |
| 17-49199381 | cg05207834 | (cancer vs cancer) | 4-48988066 | cg18280362 | (cancer vs cancer) |
| 17-55938748 | cg00023001 | (cancer vs cancer) (cancer vs cancer) (normal vs normal) | 4-55524400 | cg17891820 | (cancer vs cancer) (cancer+normal vs cancer+normal) |

| | | | | | |
|---|---|---|---|---|---|
| | | (cancer+normal vs cancer+normal) | | | |
| 17-55938871 | cg22451358 | (cancer vs cancer) (cancer vs cancer) (normal vs normal) (cancer+normal vs cancer+normal) | 4-57522094 | cg12791492 | (cancer vs cancer) (cancer vs cancer) |
| 17-57915665 | cg16936953 | (cancer vs cancer) (cancer+normal vs cancer+normal) | 4-81951953 | cg26105156 | (cancer vs cancer) |
| 17-57915717 | cg12054453 | (cancer vs cancer) (cancer+normal vs cancer+normal) | 4-81952024 | cg26917673 | (cancer vs cancer) (cancer vs cancer) |
| 17-67323953 | cg06409153 | (cancer vs cancer) | 4-81952653 | cg26699569 | (cancer vs cancer) |
| 17-74497306 | cg25038060 | (cancer vs cancer) | 5-121647858 | cg04684516 | (cancer vs cancer) (cancer+normal vs cancer+normal) |
| 17-74497418 | cg12565681 | (cancer vs cancer) | 5-121648022 | cg21984150 | (cancer vs cancer) |
| 19-13266390 | cg02097309 | (cancer vs cancer) | 5-134871778 | cg07781035 | (cancer vs cancer) |
| 19-1468943 | cg10565187 | (cancer vs cancer) (cancer vs cancer) (normal vs normal) (cancer+normal vs cancer+normal) | 5-134871805 | cg04620091 | (cancer vs cancer) |
| 19-17717530 | cg06303772 | (cancer vs cancer) | 5-134871807 | cg22630755 | (cancer vs cancer) |
| 19-19336240 | cg25814383 | (cancer vs cancer) (cancer vs cancer) (normal vs normal) (cancer+normal vs cancer+normal) | 5-134871962 | cg14747563 | (cancer vs cancer) |
| 19-2614086 | cg19250790 | (cancer vs cancer) (cancer vs cancer) (cancer+normal vs cancer+normal) | 5-134871966 | cg07035503 | (cancer vs cancer) |
| 19-3275913 | cg26825934 | (cancer vs cancer) | 5-134871973 | cg04063589 | (cancer vs cancer) |
| 19-3275998 | cg19814309 | (cancer vs cancer) | 5-159343468 | cg05963618 | (cancer vs cancer) |
| 19-35531817 | cg24139898 | (cancer vs cancer) (cancer vs cancer) (cancer+normal vs cancer+normal) | 5-159343481 | cg09049672 | (cancer vs cancer) |
| 19-35531859 | cg21484586 | (cancer vs cancer) (cancer vs cancer) | 5-159343549 | cg07011172 | (cancer vs cancer) (cancer vs cancer) |
| 19-45257305 | cg11597497 | (cancer vs cancer) (cancer+normal vs cancer+normal) | 5-172756550 | cg08711858 | (cancer vs cancer) |
| 19-46142673 | cg12536502 | (cancer vs cancer) (cancer+normal vs cancer+normal) | 5-172756558 | cg16443866 | (cancer vs cancer) |
| 19-46142757 | cg01544351 | (cancer vs cancer) | 5-175223982 | cg07295964 | (cancer vs cancer) (cancer+normal vs cancer+normal) |
| 19-48824622 | cg07925085 | (cancer vs cancer) (cancer vs cancer) | 5-42423718 | cg14868574 | (cancer vs cancer) |
| 19-49315663 | cg18873530 | (cancer vs cancer) | 5-42423947 | cg12042587 | (cancer vs cancer) (cancer vs cancer) |

| | | | | | |
|---|---|---|---|---|---|
| | | | | | (normal vs normal) (cancer+normal vs cancer+normal) |
| 2-201982310 | cg12944530 | (cancer vs cancer) | 5-42424356 | cg16292016 | (cancer vs cancer) (cancer+normal vs cancer+normal) |
| 2-220159595 | cg23869335 | (cancer vs cancer) | 5-42424525 | cg24773720 | (cancer vs cancer) |
| 2-231280907 | cg11702456 | (cancer vs cancer) (normal vs normal) (cancer+normal vs cancer+normal) | 6-17281838 | cg10096145 | (cancer vs cancer) (cancer+normal vs cancer+normal) |
| 2-231281264 | cg02001779 | (cancer vs cancer) | 6-21664652 | cg24428877 | (cancer vs cancer) (cancer vs cancer) (normal vs normal) (cancer+normal vs cancer+normal) |
| 2-232571157 | cg07158434 | (cancer vs cancer) | 7-155249648 | cg20767111 | (cancer vs cancer) |
| 2-47169299 | cg19622755 | (cancer vs cancer) | 7-20817530 | cg08650910 | (cancer vs cancer) |
| 2-58273552 | cg18998670 | (cancer vs cancer) (cancer vs cancer) | 7-20825216 | cg15427906 | (cancer vs cancer) |
| 20-10652787 | cg26705720 | (cancer vs cancer) | 7-6655711 | cg07728479 | (cancer vs cancer) |
| 3-127176163 | cg10678427 | (cancer vs cancer) | 8-105601615 | cg04413895 | (cancer vs cancer) (normal vs normal) (cancer+normal vs cancer+normal) |
| 3-13008800 | cg22481960 | (cancer vs cancer) (cancer vs cancer) | 8-6691879 | cg21493703 | (cancer vs cancer) |
| 3-182970833 | cg17701886 | (cancer vs cancer) | 9-126773656 | cg18904346 | (cancer vs cancer) |
| 3-46735335 | cg19455322 | (cancer vs cancer) | 9-126773879 | cg10755973 | (cancer vs cancer) |
| 3-46735454 | cg25104512 | (cancer vs cancer) | **Status - Hypo** | | |
| 4-1006237 | cg17105609 | (cancer vs cancer) | 1-172628020 | cg10161121 | (cancer vs cancer) |
| 4-1006270 | cg20564913 | (cancer vs cancer) | 1-178061518 | cg15007813 | (cancer vs cancer) |
| 4-1006278 | cg05866411 | (cancer vs cancer) | 1-184211934 | cg21606956 | (cancer vs cancer) |
| 4-141173867 | cg04212922 | (cancer vs cancer) | 1-184721222 | cg07817967 | (cancer vs cancer) |
| 4-3464874 | cg25237365 | (cancer vs cancer) | 1-28111716 | cg16903618 | (cancer vs cancer) |
| 5-138897363 | cg04597532 | (cancer vs cancer) | 10-3984975 | cg14575790 | (cancer vs cancer) |
| 5-139126512 | cg10642240 | (cancer vs cancer) | 10-49647167 | cg08866557 | (cancer vs cancer) (cancer vs cancer) |
| 5-175560449 | cg25412017 | (cancer vs cancer) | 10-60588674 | cg23799393 | (cancer vs cancer) |
| 6-157041096 | cg04671145 | (cancer vs cancer) (cancer vs cancer) | 10-88719848 | cg05498539 | (cancer vs cancer) |
| 6-25962987 | cg08501292 | (cancer vs cancer) (cancer vs cancer) (normal vs normal) (cancer+normal vs cancer+normal) | 10-88719950 | cg20459238 | (cancer vs cancer) (cancer vs cancer) (cancer+normal vs cancer+normal) |
| 6- | cg05552183 | (cancer vs cancer) | 10- | cg0709209 | (normal vs normal) |

| | | | | | |
|---|---|---|---|---|---|
| 42928497 | | (normal vs normal) | 88720025 | 7 | (cancer vs cancer)(cancer+normal vs cancer+normal) |
| 6-43237330 | cg23514619 | (cancer vs cancer)(cancer vs cancer)(normal vs normal)(cancer+normal vs cancer+normal) | 10-97145431 | cg04248373 | (cancer vs cancer) |
| 6-43237511 | cg20434586 | (cancer vs cancer)(cancer vs cancer)(normal vs normal)(cancer+normal vs cancer+normal) | 10-98425148 | cg27215768 | (normal vs normal)(cancer vs cancer) |
| 6-74233510 | cg09681335 | (cancer vs cancer)(cancer vs cancer) | 11-115088907 | cg16061656 | (cancer vs cancer)(cancer vs cancer) |
| 7-150020925 | cg11347411 | (cancer vs cancer)(cancer vs cancer) | 11-72982996 | cg26890621 | (cancer vs cancer) |
| 7-22894557 | cg06980547 | (cancer vs cancer)(cancer vs cancer) | 11-94248604 | cg24881472 | (cancer vs cancer) |
| 7-74020924 | cg19089073 | (cancer vs cancer) | 12-10324843 | cg21249659 | (cancer vs cancer) |
| 7-75896889 | cg19105122 | (cancer vs cancer) | 12-10324918 | cg21685770 | (cancer vs cancer) |
| 7-77648567 | cg17222941 | (cancer vs cancer) | 12-24228975 | cg16095464 | (cancer vs cancer) |
| 7-77648600 | cg10271408 | (cancer vs cancer) | 12-32831428 | cg20215290 | (cancer vs cancer) |
| 7-77649540 | cg04976780 | (cancer vs cancer) | 12-32831504 | cg27387222 | (cancer vs cancer) |
| 7-77649564 | cg01914455 | (cancer vs cancer) | 12-47471907 | cg18436898 | (cancer vs cancer)(cancer vs cancer)(cancer+normal vs cancer+normal) |
| 8-104310713 | cg24115571 | (cancer vs cancer)(cancer vs cancer) | 12-52182759 | cg15618336 | (cancer vs cancer) |
| 8-110656442 | cg25834419 | (cancer vs cancer) | 12-54985731 | cg03782220 | (cancer vs cancer) |
| 8-145556707 | cg00447052 | (cancer vs cancer) | 13-113544125 | cg25252255 | (cancer vs cancer) |
| 8-21906349 | cg06829538 | (cancer vs cancer) | 13-113544293 | cg22286732 | (cancer vs cancer) |
| 8-23082789 | cg01725531 | (cancer vs cancer)(cancer vs cancer) | 13-113544658 | cg00141447 | (cancer vs cancer) |
| 8-30580804 | cg08305799 | (cancer vs cancer)(cancer vs cancer)(cancer+normal vs cancer+normal) | 14-23285365 | cg18960218 | (cancer vs cancer) |
| 9-132646821 | cg13668544 | (cancer vs cancer) | 14-67881521 | cg12199376 | (cancer vs cancer) |
| 9-33166541 | cg14242528 | (cancer vs cancer) | 14-75747961 | cg16701133 | (cancer vs cancer) |
| 9-88714961 | cg20263942 | (cancer vs cancer) | 15-28263701 | cg23908019 | (cancer vs cancer) |
| **Status - Hypo** | | | 15-28263706 | cg19890533 | (cancer vs cancer) |
| 1-10240017 | cg27508545 | (normal vs normal)(cancer vs cancer)(cancer vs cancer)(cancer+normal vs cancer+normal) | 15-34657364 | cg23441018 | (cancer vs cancer)(cancer vs cancer) |
| 1- | cg23661000 | (normal vs normal) | 15- | cg0345387 | (cancer vs cancer) |

| | | | | | |
|---|---|---|---|---|---|
| 10240025 | | (cancer vs cancer) (cancer vs cancer) (cancer+normal vs cancer+normal) | 39563291 | 0 | |
| 1-2006465 | cg06791083 | (cancer vs cancer) (cancer+normal vs cancer+normal) | 15-43822292 | cg23987491 | (cancer vs cancer) |
| 1-3128606 | cg22031783 | (cancer vs cancer) (cancer vs cancer) | 15-57626444 | cg13580002 | (cancer vs cancer) |
| 1-36559506 | cg09506001 | (cancer vs cancer) (cancer vs cancer) (cancer+normal vs cancer+normal) | 15-65042722 | cg22423363 | (normal vs normal) (cancer vs cancer) (cancer vs cancer) (cancer+normal vs cancer+normal) |
| 1-6390793 | cg00081799 | (cancer vs cancer) (cancer vs cancer) | 15-69563432 | cg14864999 | (cancer vs cancer) |
| 1-66458803 | cg26912671 | (cancer vs cancer) (cancer+normal vs cancer+normal) | 15-70786271 | cg09164206 | (cancer vs cancer) |
| 1-9448660 | cg12658495 | (cancer vs cancer) (cancer vs cancer) (cancer+normal vs cancer+normal) | 15-99408636 | cg19322380 | (cancer vs cancer) (cancer vs cancer) |
| 10-115738883 | cg01467532 | (cancer vs cancer) (cancer vs cancer) (cancer+normal vs cancer+normal) | 16-1578807 | cg05308792 | (cancer vs cancer) (cancer vs cancer) |
| 10-405225 | cg20595750 | (normal vs normal) (cancer vs cancer) (cancer vs cancer) (cancer+normal vs cancer+normal) | 16-88564247 | cg04362711 | (cancer vs cancer) |
| 10-405362 | cg18074403 | (cancer vs cancer) (cancer+normal vs cancer+normal) | 16-88575349 | cg05242494 | (cancer vs cancer) |
| 10-661009 | cg19926144 | (cancer vs cancer) (cancer vs cancer) (cancer+normal vs cancer+normal) | 17-38609504 | cg11860777 | (cancer vs cancer) |
| 11-113577434 | cg23871994 | (cancer vs cancer) (cancer vs cancer) (cancer+normal vs cancer+normal) | 17-38609634 | cg19878076 | (cancer vs cancer) |
| 11-1486341 | cg07737063 | (cancer vs cancer) (cancer vs cancer) (cancer+normal vs cancer+normal) | 17-767301 | cg13476078 | (normal vs normal) (cancer vs cancer) (cancer vs cancer) (cancer+normal vs cancer+normal) |
| 12-123738291 | cg03061518 | (cancer vs cancer) (cancer vs cancer) | 18-56296496 | cg27614534 | (cancer vs cancer) |
| 12-124905230 | cg17429587 | (cancer vs cancer) (cancer vs cancer) | 18-56296544 | cg25407077 | (cancer vs cancer) |
| 12-131452297 | cg23617848 | (cancer vs cancer) (cancer vs cancer) (cancer+normal vs cancer+normal) | 19-39052032 | cg19338222 | (cancer vs cancer) (cancer vs cancer) (cancer+normal vs cancer+normal) |
| 12-4596426 | cg24211304 | (cancer vs cancer) (cancer vs cancer) (cancer+normal vs cancer+normal) | 19-55721180 | cg19673376 | (cancer vs cancer) |
| 12-656703 | cg03539204 | (cancer vs cancer) | 19- | cg1964183 | (cancer vs cancer) |

| | | | | | |
|---|---|---|---|---|---|
| | | (cancer vs cancer)(cancer+normal vs cancer+normal) | 57105832 | 9 | |
| 13-103531040 | cg10628699 | (cancer vs cancer) | 2-106960893 | cg14730449 | (cancer vs cancer) |
| 13-103531058 | cg22954906 | (cancer vs cancer) | 2-135532566 | cg16200531 | (cancer vs cancer)(cancer+normal vs cancer+normal) |
| 13-111893537 | cg26951340 | (normal vs normal)(cancer vs cancer)(cancer vs cancer)(cancer+normal vs cancer+normal) | 2-219256018 | cg23212579 | (cancer vs cancer)(cancer vs cancer)(cancer+normal vs cancer+normal) |
| 13-113436564 | cg06206670 | (cancer vs cancer)(cancer vs cancer)(cancer+normal vs cancer+normal) | 2-224993012 | cg27366888 | (cancer vs cancer) |
| 13-113436568 | cg14737571 | (cancer vs cancer)(cancer vs cancer)(cancer+normal vs cancer+normal) | 2-75078286 | cg11672772 | (cancer vs cancer)(cancer vs cancer)(cancer+normal vs cancer+normal) |
| 13-80585265 | cg03010009 | (cancer vs cancer)(cancer vs cancer)(cancer+normal vs cancer+normal) | 2-8596908 | cg26784300 | (cancer vs cancer)(cancer vs cancer) |
| 14-32798387 | cg03403507 | (cancer vs cancer)(cancer vs cancer)(cancer+normal vs cancer+normal) | 2-9427798 | cg11444072 | (cancer vs cancer) |
| 14-32798762 | cg24812523 | (cancer vs cancer)(cancer vs cancer)(cancer+normal vs cancer+normal) | 21-27015734 | cg01975706 | (cancer vs cancer) |
| 14-51293793 | cg18459806 | (cancer vs cancer)(cancer vs cancer) | 21-46323665 | cg27019201 | (cancer vs cancer)(cancer vs cancer) |
| 14-95599667 | cg24158307 | (cancer vs cancer)(cancer vs cancer)(cancer+normal vs cancer+normal) | 22-23519916 | cg11229267 | (cancer vs cancer) |
| 15-71685353 | cg13191951 | (cancer vs cancer)(cancer vs cancer) | 3-150484093 | cg11816838 | (cancer vs cancer) |
| 16-50897271 | cg00900231 | (normal vs normal)(cancer vs cancer)(cancer vs cancer)(cancer+normal vs cancer+normal) | 3-175987813 | cg12479512 | (cancer vs cancer) |
| 16-72460083 | cg02619656 | (normal vs normal)(cancer vs cancer)(cancer vs cancer)(cancer+normal vs cancer+normal) | 3-194573301 | cg10985281 | (cancer vs cancer)(cancer vs cancer)(cancer+normal vs cancer+normal) |
| 16-72460114 | cg05347108 | (normal vs normal)(cancer vs cancer)(cancer vs cancer)(cancer+normal vs cancer+normal) | 3-194868750 | cg21937377 | (normal vs normal)(cancer vs cancer)(cancer vs cancer)(cancer+normal vs cancer+normal) |
| 16-75300861 | cg00319595 | (cancer vs cancer)(cancer vs cancer)(cancer+normal vs cancer+normal) | 3-196754664 | cg21856334 | (cancer vs cancer) |
| 16- | cg03551062 | (cancer vs cancer) | 3- | cg0606945 | (cancer vs cancer) |

| | | | | | |
|---|---|---|---|---|---|
| 88041289 | | (cancer vs cancer) (cancer+normal vs cancer+normal) | 196754722 | 7 | |
| 16-89623374 | cg01279418 | (normal vs normal) (cancer vs cancer) (cancer+normal vs cancer+normal) | 3-59980762 | cg25921543 | (cancer vs cancer) |
| 17-1308616 | cg13691567 | (normal vs normal) (cancer vs cancer) (cancer+normal vs cancer+normal) | 3-61517101 | cg07627317 | (cancer vs cancer) |
| 17-2319906 | cg00145352 | (normal vs normal) (cancer vs cancer) (cancer vs cancer) (cancer+normal vs cancer+normal) | 4-154391944 | cg20367304 | (cancer vs cancer) |
| 17-29624396 | cg18232597 | (normal vs normal) (cancer vs cancer) (cancer+normal vs cancer+normal) | 4-2439731 | cg26605046 | (normal vs normal) (cancer vs cancer) (cancer+normal vs cancer+normal) |
| 17-30815034 | cg18473137 | (cancer vs cancer) | 5-179442399 | cg03369432 | (cancer vs cancer) |
| 17-63535223 | cg06405341 | (cancer vs cancer) (cancer vs cancer) | 5-37772265 | cg22808435 | (cancer vs cancer) |
| 17-71345538 | cg00711291 | (cancer vs cancer) (cancer vs cancer) (cancer+normal vs cancer+normal) | 5-77072789 | cg25455811 | (cancer vs cancer) |
| 17-76220898 | cg19272238 | (cancer vs cancer) (cancer+normal vs cancer+normal) | 6-106582669 | cg25581448 | (cancer vs cancer) (cancer vs cancer) (cancer+normal vs cancer+normal) |
| 17-76220929 | cg07366188 | (cancer vs cancer) (cancer vs cancer) (cancer+normal vs cancer+normal) | 6-1080885 | cg03887092 | (cancer vs cancer) |
| 17-76220955 | cg10070788 | (cancer vs cancer) (cancer vs cancer) (cancer+normal vs cancer+normal) | 6-161636502 | cg14840850 | (cancer vs cancer) |
| 17-9151012 | cg12130672 | (cancer vs cancer) (cancer vs cancer) (cancer+normal vs cancer+normal) | 6-167189272 | cg03329755 | (cancer vs cancer) (cancer vs cancer) (cancer+normal vs cancer+normal) |
| 18-32398181 | cg22291942 | (normal vs normal) (cancer vs cancer) (cancer vs cancer) (cancer+normal vs cancer+normal) | 6-168562318 | cg23492327 | (cancer vs cancer) |
| 18-42324965 | cg03722909 | (normal vs normal) (cancer vs cancer) (cancer vs cancer) (cancer+normal vs cancer+normal) | 6-168594903 | cg10896609 | (cancer vs cancer) (cancer vs cancer) |
| 18-42325086 | cg07015525 | (normal vs normal) (cancer vs cancer) (cancer vs cancer) (cancer+normal vs cancer+normal) | 6-168684757 | cg08062387 | (cancer vs cancer) (cancer vs cancer) |
| 19-1255283 | cg27172769 | (cancer vs cancer) (cancer+normal vs | 6-168748530 | cg02840092 | (normal vs normal) (cancer vs cancer) |

| | | | | | |
|---|---|---|---|---|---|
| | | cancer+normal) | | | |
| 19-1255516 | cg24300446 | (cancer vs cancer) (cancer vs cancer) (cancer+normal vs cancer+normal) | 6-30956397 | cg24067150 | (cancer vs cancer) |
| 19-47770746 | cg15080870 | (cancer vs cancer) (cancer vs cancer) (cancer+normal vs cancer+normal) | 6-47202254 | cg18015809 | (cancer vs cancer) |
| 2-236716219 | cg22764497 | (cancer vs cancer) (cancer vs cancer) (cancer+normal vs cancer+normal) | 6-47202394 | cg21552001 | (cancer vs cancer) (cancer vs cancer) (cancer+normal vs cancer+normal) |
| 2-236716285 | cg06694561 | (cancer vs cancer) (cancer vs cancer) (cancer+normal vs cancer+normal) | 6-7541066 | cg03814872 | (cancer vs cancer) |
| 2-236716372 | cg06747919 | (cancer vs cancer) (cancer vs cancer) (cancer+normal vs cancer+normal) | 7-210075 | cg12458039 | (cancer vs cancer) (cancer vs cancer) (cancer+normal vs cancer+normal) |
| 2-23879137 | cg00746446 | (cancer vs cancer) (cancer vs cancer) (cancer+normal vs cancer+normal) | 7-33765409 | cg23635728 | (cancer vs cancer) |
| 2-239306169 | cg09675853 | (cancer vs cancer) (cancer vs cancer) | 7-36607476 | cg22695711 | (cancer vs cancer) |
| 2-240117222 | cg06257052 | (normal vs normal) (cancer vs cancer) (cancer vs cancer) (cancer+normal vs cancer+normal) | 7-40566521 | cg04099158 | (cancer vs cancer) |
| 2-240117640 | cg20853370 | (cancer vs cancer) (cancer vs cancer) (cancer+normal vs cancer+normal) | 7-40788535 | cg13392320 | (cancer vs cancer) |
| 2-30041338 | cg00073794 | (cancer vs cancer) (cancer vs cancer) (cancer+normal vs cancer+normal) | 7-41741671 | cg15291905 | (cancer vs cancer) |
| 2-3292757 | cg03025986 | (normal vs normal) (cancer vs cancer) (cancer vs cancer) (cancer+normal vs cancer+normal) | 7-98722470 | cg14769121 | (cancer vs cancer) |
| 2-3480658 | cg05777919 | (cancer vs cancer) (cancer vs cancer) (cancer+normal vs cancer+normal) | 8-102381050 | cg23963995 | (cancer vs cancer) |
| 2-55174473 | cg04768463 | (cancer vs cancer) (cancer vs cancer) (cancer+normal vs cancer+normal) | 8-104185332 | cg00934232 | (cancer vs cancer) |
| 2-97213642 | cg00820405 | (cancer vs cancer) (cancer vs cancer) | 8-123966105 | cg01886570 | (normal vs normal) (cancer vs cancer) (cancer vs cancer) (cancer+normal vs cancer+normal) |
| 20-5985495 | cg04316905 | (cancer vs cancer) (cancer vs cancer) | 8-142189543 | cg18165707 | (cancer vs cancer) |
| 22- | cg12894883 | (normal vs normal) | 8-2037954 | cg1273800 | (cancer vs cancer) |

| | | |
|---|---|---|
| 22288473 | | (cancer vs cancer) (cancer vs cancer) (cancer+normal vs cancer+normal) |
| 22-24041104 | cg00489795 | (cancer vs cancer) (cancer vs cancer) (cancer+normal vs cancer+normal) |
| 22-42611379 | cg26799416 | (cancer vs cancer) (cancer vs cancer) (cancer+normal vs cancer+normal) |
| 3-127327369 | cg12678686 | (cancer vs cancer) (cancer vs cancer) (cancer+normal vs cancer+normal) |
| 3-129408521 | cg19272592 | (cancer vs cancer) (cancer vs cancer) (cancer+normal vs cancer+normal) |
| 3-147657586 | cg03320743 | (cancer vs cancer) (cancer vs cancer) (cancer+normal vs cancer+normal) |
| 3-195616361 | cg20224111 | (cancer vs cancer) (cancer vs cancer) (cancer+normal vs cancer+normal) |
| 3-33188872 | cg25680629 | (cancer vs cancer) |
| 4-146804010 | cg01578875 | (normal vs normal) (cancer vs cancer) (cancer vs cancer) (cancer+normal vs cancer+normal) |
| 4-147187180 | cg05701706 | (cancer vs cancer) (cancer vs cancer) |
| 4-24982540 | cg10569820 | (normal vs normal) (cancer vs cancer) (cancer vs cancer) (cancer+normal vs cancer+normal) |
| 4-39064477 | cg26784682 | (cancer vs cancer) (cancer vs cancer) |
| 5-102834858 | cg06612355 | (cancer vs cancer) (cancer vs cancer) (cancer+normal vs cancer+normal) |
| 5-138211184 | cg01156295 | (cancer vs cancer) |
| 5-140865433 | cg11830096 | (cancer vs cancer) (cancer vs cancer) |
| 5-179494929 | cg10519882 | (cancer vs cancer) (cancer+normal vs cancer+normal) |
| 5-77289836 | cg09645993 | (normal vs normal) (cancer vs cancer) (cancer vs cancer) (cancer+normal vs cancer+normal) |

| | | |
|---|---|---|
| | 8 | |
| 8-81441781 | cg11609156 | (cancer vs cancer) |
| 8-98868006 | cg15195097 | (cancer vs cancer) |
| **SARC (cancer vs cancer)** | | |
| **Status - Hyper** | | |
| 1-165322606 | cg27636676 | (cancer vs cancer) (cancer+normal vs cancer+normal) |
| 1-165322784 | cg10315533 | (cancer vs cancer) (cancer+normal vs cancer+normal) |
| 1-165323232 | cg26522592 | (cancer vs cancer) (cancer+normal vs cancer+normal) |
| 1-169079632 | cg24304617 | (cancer vs cancer) (cancer+normal vs cancer+normal) |
| 1-183155097 | cg24146518 | (cancer vs cancer) |
| 1-183155116 | cg01949993 | (cancer vs cancer) |
| 1-183155154 | cg05929019 | (cancer vs cancer) |
| 1-183155366 | cg04218899 | (cancer vs cancer) |
| 1-183155573 | cg22346461 | (cancer vs cancer) |
| 10-119310964 | cg08451832 | (cancer vs cancer) |
| 10-77163983 | cg25006231 | (cancer vs cancer) |
| 11-35439994 | cg27457284 | (cancer vs cancer) (cancer+normal vs cancer+normal) |

| | | | | | |
|---|---|---|---|---|---|
| 6-102224868 | cg24574819 | (normal vs normal)<br>(cancer vs cancer)<br>(cancer vs cancer)<br>(cancer+normal vs cancer+normal) | 11-35440062 | cg2418332 4 | (cancer vs cancer)<br>(cancer+normal vs cancer+normal) |
| 6-125341187 | cg01080927 | (cancer vs cancer)<br>(cancer vs cancer) | 11-35440136 | cg2494410 9 | (cancer vs cancer) |
| 6-157493773 | cg17596249 | (cancer vs cancer)<br>(cancer vs cancer)<br>(cancer+normal vs cancer+normal) | 11-35440252 | cg2350196 2 | (cancer vs cancer) |
| 6-24140822 | cg14549524 | (cancer vs cancer)<br>(cancer vs cancer)<br>(cancer+normal vs cancer+normal) | 11-87908631 | cg0465352 2 | (cancer vs cancer) |
| 6-24140899 | cg08908855 | (cancer vs cancer)<br>(cancer vs cancer)<br>(cancer+normal vs cancer+normal) | 12-70758965 | cg0857329 5 | (cancer vs cancer) |
| 7-101849120 | cg06010390 | (normal vs normal)<br>(cancer vs cancer)<br>(cancer vs cancer)<br>(cancer+normal vs cancer+normal) | 13-37838311 | cg2601516 3 | (cancer vs cancer)<br>(cancer+normal vs cancer+normal) |
| 7-101849473 | cg01234984 | (cancer vs cancer)<br>(cancer vs cancer)<br>(cancer+normal vs cancer+normal) | 13-92002454 | cg0229783 8 | (cancer vs cancer)<br>(cancer+normal vs cancer+normal) |
| 7-105283982 | cg11895696 | (cancer vs cancer)<br>(cancer vs cancer)<br>(cancer+normal vs cancer+normal) | 15-99559002 | cg1982313 8 | (cancer vs cancer)<br>(cancer+normal vs cancer+normal) |
| 7-4747216 | cg01275887 | (cancer vs cancer)<br>(cancer vs cancer)<br>(cancer+normal vs cancer+normal) | 16-29296693 | cg0171917 9 | (cancer vs cancer) |
| 7-4747261 | cg07076109 | (cancer vs cancer)<br>(cancer vs cancer) | 16-29296704 | cg2351512 5 | (cancer vs cancer) |
| 7-5535692 | cg09286367 | (cancer vs cancer)<br>(cancer vs cancer) | 16-85648263 | cg0730132 9 | (cancer vs cancer)<br>(cancer+normal vs cancer+normal) |
| 8-26440349 | cg00470636 | (cancer vs cancer)<br>(cancer+normal vs cancer+normal) | 16-88292973 | cg0709964 0 | (cancer vs cancer) |
| 8-87570714 | cg02712949 | (cancer vs cancer)<br>(cancer vs cancer)<br>(cancer+normal vs cancer+normal) | 16-88292996 | cg1470507 9 | (cancer vs cancer)<br>(cancer+normal vs cancer+normal) |
| 8-9260354 | cg10359460 | (normal vs normal)<br>(cancer vs cancer)<br>(cancer vs cancer)<br>(cancer+normal vs cancer+normal) | 17-77810742 | cg0697602 5 | (cancer vs cancer)<br>(cancer+normal vs cancer+normal) |
| 8-9537322 | cg10419849 | (cancer vs cancer)<br>(cancer vs cancer)<br>(cancer+normal vs cancer+normal) | 18-597111 | cg1121488 9 | (cancer vs cancer) |
| 9-130854713 | cg14554539 | (cancer vs cancer)<br>(cancer vs cancer) | 19-44270892 | cg2696089 6 | (cancer vs cancer) |
| 9-139410910 | cg14494733 | (cancer vs cancer)<br>(cancer vs cancer) | 19-48833410 | cg1033883 0 | (cancer vs cancer)<br>(cancer+normal vs |

271

| | | | | | |
|---|---|---|---|---|---|
| | | | | | cancer+normal) |
| 9-99264127 | cg14155501 | (normal vs normal) (cancer vs cancer) (cancer vs cancer) (cancer+normal vs cancer+normal) | 19-48833535 | cg06350544 | (cancer vs cancer) |
| **(cancer+normal vs cancer+normal)** | | | 19-48833559 | cg17315219 | (cancer vs cancer) (cancer+normal vs cancer+normal) |
| **Status - Hyper** | | | 2-119608121 | cg25317460 | (cancer vs cancer) (normal vs normal) |
| 1-100231682 | cg04308687 | (normal vs normal) (cancer+normal vs cancer+normal) | 2-128173685 | cg24430189 | (cancer vs cancer) (cancer+normal vs cancer+normal) |
| 1-12678641 | cg17935811 | (cancer vs cancer) (normal vs normal) (cancer+normal vs cancer+normal) | 2-128173750 | cg02473600 | (cancer vs cancer) (cancer+normal vs cancer+normal) |
| 1-12678761 | cg01346152 | (cancer vs cancer) (normal vs normal) (cancer+normal vs cancer+normal) | 2-177021899 | cg10107434 | (cancer vs cancer) (cancer+normal vs cancer+normal) |
| 1-12679132 | cg02902865 | (cancer+normal vs cancer+normal) | 2-177022056 | cg25708328 | (cancer vs cancer) (cancer+normal vs cancer+normal) |
| 1-175161069 | cg03317045 | (normal vs normal) (cancer+normal vs cancer+normal) | 2-177022121 | cg18338775 | (cancer vs cancer) (cancer+normal vs cancer+normal) |
| 1-181003203 | cg23037321 | (cancer+normal vs cancer+normal) | 2-177022962 | cg05669418 | (cancer vs cancer) (cancer+normal vs cancer+normal) |
| 1-203320506 | cg22705746 | (normal vs normal) (cancer+normal vs cancer+normal) | 2-177023062 | cg09784934 | (cancer vs cancer) (cancer+normal vs cancer+normal) |
| 1-203320541 | cg27387030 | (cancer+normal vs cancer+normal) | 2-227665799 | cg10098888 | (cancer vs cancer) |
| 1-20698023 | cg12624197 | (cancer+normal vs cancer+normal) | 2-227665840 | cg13572511 | (cancer vs cancer) |
| 1-212769641 | cg10985914 | (cancer vs cancer) (normal vs normal) (cancer+normal vs cancer+normal) | 2-227665895 | cg23601397 | (cancer vs cancer) |
| 1-23854847 | cg11526198 | (normal vs normal) (cancer+normal vs cancer+normal) | 20-3653555 | cg06023791 | (cancer vs cancer) (cancer+normal vs cancer+normal) |
| 1-24867657 | cg17094249 | (cancer+normal vs cancer+normal) | 20-45986173 | cg02767841 | (cancer vs cancer) (normal vs normal) |
| 1-27894955 | cg12610832 | (cancer vs cancer) (cancer vs cancer) (cancer+normal vs cancer+normal) | 20-50159160 | cg23085343 | (cancer vs cancer) |
| 1-37413867 | cg23715749 | (cancer+normal vs cancer+normal) | 22-20785765 | cg17119930 | (cancer vs cancer) |
| 1-37943106 | cg19680850 | (cancer+normal vs cancer+normal) | 3-157815672 | cg03458172 | (cancer vs cancer) (cancer+normal vs cancer+normal) |
| 1-37945057 | cg17888086 | (cancer+normal vs cancer+normal) | 3-157815808 | cg05401764 | (cancer vs cancer) (cancer+normal vs cancer+normal) |
| 1-59280952 | cg15687600 | (cancer vs cancer) (normal vs normal) | 3-157816094 | cg09095222 | (cancer vs cancer) (normal vs normal) |

| | | (cancer+normal vs cancer+normal) | | | (cancer+normal vs cancer+normal) |
|---|---|---|---|---|---|
| 1-59281067 | cg03611060 | (cancer vs cancer) (normal vs normal) (cancer+normal vs cancer+normal) | 3-157816330 | cg0008948 6 | (cancer vs cancer) (cancer+normal vs cancer+normal) |
| 1-59281540 | cg23075983 | (cancer+normal vs cancer+normal) | 3-157817678 | cg1572615 4 | (cancer vs cancer) |
| 1-61039539 | cg16711650 | (cancer+normal vs cancer+normal) | 4-174457371 | cg0174786 2 | (cancer vs cancer) |
| 1-89873226 | cg13938909 | (normal vs normal) (cancer+normal vs cancer+normal) | 4-174457944 | cg2495023 3 | (cancer vs cancer) |
| 10-13749193 | cg16485048 | (normal vs normal) (cancer+normal vs cancer+normal) | 4-174458819 | cg1909592 0 | (cancer vs cancer) |
| 10-3894968 | cg00524374 | (cancer+normal vs cancer+normal) | 4-186732926 | cg0439208 2 | (cancer vs cancer) (cancer+normal vs cancer+normal) |
| 10-45474372 | cg02076355 | (normal vs normal) (cancer+normal vs cancer+normal) | 4-186732932 | cg0279030 5 | (cancer vs cancer) (cancer+normal vs cancer+normal) |
| 10-75172806 | cg17276002 | (cancer vs cancer) (cancer+normal vs cancer+normal) | 4-186732936 | cg2678534 6 | (cancer vs cancer) (cancer+normal vs cancer+normal) |
| 11-126456052 | cg16628183 | (cancer vs cancer) (cancer+normal vs cancer+normal) | 4-186732942 | cg0193307 3 | (cancer vs cancer) |
| 12-49659619 | cg16881676 | (cancer+normal vs cancer+normal) | 4-186733024 | cg1392144 4 | (cancer vs cancer) (cancer+normal vs cancer+normal) |
| 12-57504948 | cg01063813 | (cancer vs cancer) (cancer vs cancer) (normal vs normal) (cancer+normal vs cancer+normal) | 4-186733060 | cg0434826 5 | (cancer vs cancer) |
| 12-6493003 | cg23079808 | (cancer vs cancer) (cancer vs cancer) (cancer+normal vs cancer+normal) | 5-122422500 | cg1882982 7 | (cancer vs cancer) |
| 12-6493495 | cg14947547 | (cancer vs cancer) (cancer+normal vs cancer+normal) | 5-122423615 | cg0230058 4 | (cancer vs cancer) |
| 12-96441714 | cg25671716 | (normal vs normal) (cancer+normal vs cancer+normal) | 5-122434544 | cg1063220 9 | (cancer vs cancer) |
| 13-114927854 | cg11997708 | (cancer vs cancer) (cancer vs cancer) (normal vs normal) (cancer+normal vs cancer+normal) | 5-122434853 | cg0012956 3 | (cancer vs cancer) |
| 13-27557503 | cg03268893 | (cancer vs cancer) (normal vs normal) (cancer+normal vs cancer+normal) | 5-122434944 | cg0136005 4 | (cancer vs cancer) (cancer+normal vs cancer+normal) |
| 13-41593416 | cg02632314 | (cancer vs cancer) (cancer+normal vs cancer+normal) | 5-149669414 | cg0180466 4 | (cancer vs cancer) |
| 14-62004353 | cg13564459 | (cancer+normal vs cancer+normal) | 5-90575459 | cg0033571 5 | (cancer vs cancer) |
| 14- | cg02380334 | (cancer vs cancer) | 6-1607922 | cg1839805 | (cancer vs cancer) |

273

| | | | | | |
|---|---|---|---|---|---|
| 70038925 | | (cancer vs cancer) (normal vs normal) (cancer+normal vs cancer+normal) | | 6 | (cancer+normal vs cancer+normal) |
| 14-70038949 | cg14539393 | (cancer vs cancer) (normal vs normal) (cancer+normal vs cancer+normal) | 6-170598215 | cg17586256 | (cancer vs cancer) (cancer+normal vs cancer+normal) |
| 15-33360195 | cg19635897 | (cancer+normal vs cancer+normal) | 6-32063835 | cg01337207 | (cancer vs cancer) (cancer+normal vs cancer+normal) |
| 15-34610829 | cg12476968 | (cancer+normal vs cancer+normal) | 6-32063838 | cg26266427 | (cancer vs cancer) (cancer+normal vs cancer+normal) |
| 15-93722499 | cg21610221 | (cancer vs cancer) (cancer+normal vs cancer+normal) | 6-32063901 | cg07524919 | (cancer vs cancer) (cancer+normal vs cancer+normal) |
| 16-20774960 | cg10060338 | (cancer vs cancer) (cancer+normal vs cancer+normal) | 6-32063991 | cg00872984 | (cancer vs cancer) (cancer+normal vs cancer+normal) |
| 16-20775011 | cg10078415 | (cancer+normal vs cancer+normal) | 6-32064206 | cg08516507 | (cancer vs cancer) |
| 16-20775166 | cg00394823 | (cancer+normal vs cancer+normal) | 6-32064677 | cg01485117 | (cancer vs cancer) (cancer+normal vs cancer+normal) |
| 16-4233246 | cg05006755 | (cancer vs cancer) (cancer vs cancer) (cancer+normal vs cancer+normal) | 6-32064692 | cg27081346 | (cancer vs cancer) (cancer+normal vs cancer+normal) |
| 16-50730737 | cg08554257 | (cancer vs cancer) (cancer+normal vs cancer+normal) | 6-32064749 | cg15793329 | (cancer vs cancer) (cancer+normal vs cancer+normal) |
| 16-744328 | cg05542681 | (cancer vs cancer) (cancer+normal vs cancer+normal) | 6-32064764 | cg21289669 | (cancer vs cancer) |
| 16-744939 | cg04005677 | (cancer vs cancer) (cancer+normal vs cancer+normal) | 6-45387347 | cg03474350 | (cancer vs cancer) (cancer+normal vs cancer+normal) |
| 16-745663 | cg02958327 | (cancer vs cancer) (normal vs normal) (cancer+normal vs cancer+normal) | 6-45387395 | cg26358286 | (cancer vs cancer) (cancer+normal vs cancer+normal) |
| 16-745687 | cg07482202 | (cancer vs cancer) (normal vs normal) (cancer+normal vs cancer+normal) | 6-84221752 | cg08687825 | (cancer vs cancer) |
| 16-86002958 | cg27507473 | (cancer+normal vs cancer+normal) | 6-85481532 | cg25560696 | (cancer vs cancer) |
| 17-1553202 | cg04313875 | (cancer vs cancer) (cancer vs cancer) (normal vs normal) (cancer+normal vs cancer+normal) | 7-100202882 | cg26100986 | (cancer vs cancer) (cancer+normal vs cancer+normal) |
| 17-36715787 | cg03026373 | (normal vs normal) (cancer+normal vs cancer+normal) | 7-100203114 | cg25680486 | (cancer vs cancer) (cancer+normal vs cancer+normal) |
| 17-42392983 | cg10712561 | (normal vs normal) (cancer+normal vs cancer+normal) | 7-143106114 | cg06161630 | (cancer vs cancer) |
| 17-55938748 | cg00023001 | (cancer vs cancer) (cancer vs cancer) | 7-143106126 | cg27426220 | (cancer vs cancer) |

| | | | | | |
|---|---|---|---|---|---|
| | | (normal vs normal)<br>(cancer+normal vs cancer+normal) | | | |
| 17-55938871 | cg22451358 | (cancer vs cancer)<br>(cancer vs cancer)<br>(normal vs normal)<br>(cancer+normal vs cancer+normal) | 7-143106130 | cg02376703 | (cancer vs cancer) |
| 17-55938940 | cg06452419 | (cancer+normal vs cancer+normal) | 8-102503892 | cg05458052 | (cancer vs cancer)<br>(cancer+normal vs cancer+normal) |
| 17-57915665 | cg16936953 | (cancer vs cancer)<br>(cancer+normal vs cancer+normal) | 8-102503970 | cg20342184 | (cancer vs cancer)<br>(cancer+normal vs cancer+normal) |
| 17-57915717 | cg12054453 | (cancer vs cancer)<br>(cancer+normal vs cancer+normal) | 8-102504447 | cg16435779 | (cancer vs cancer) |
| 17-57915740 | cg01409343 | (cancer+normal vs cancer+normal) | 8-102504621 | cg25639415 | (cancer vs cancer) |
| 17-57915773 | cg18942579 | (cancer+normal vs cancer+normal) | 8-25907314 | cg09430976 | (cancer vs cancer) |
| 17-72439044 | cg11963912 | (cancer vs cancer)<br>(cancer+normal vs cancer+normal) | 8-25908279 | cg04244354 | (cancer vs cancer) |
| 19-1455513 | cg06615719 | (cancer vs cancer)<br>(normal vs normal)<br>(cancer+normal vs cancer+normal) | 8-26047996 | cg19889626 | (cancer vs cancer) |
| 19-1468943 | cg10565187 | (cancer vs cancer)<br>(cancer vs cancer)<br>(normal vs normal)<br>(cancer+normal vs cancer+normal) | 8-37699239 | cg05552035 | (cancer vs cancer) |
| 19-19336240 | cg25814383 | (cancer vs cancer)<br>(cancer vs cancer)<br>(normal vs normal)<br>(cancer+normal vs cancer+normal) | 8-37699360 | cg12869334 | (cancer vs cancer) |
| 19-2614086 | cg19250790 | (cancer vs cancer)<br>(cancer vs cancer)<br>(cancer+normal vs cancer+normal) | 8-72916620 | cg24523948 | (cancer vs cancer)<br>(cancer+normal vs cancer+normal) |
| 19-35531817 | cg24139898 | (cancer vs cancer)<br>(cancer vs cancer)<br>(cancer+normal vs cancer+normal) | 9-133308594 | cg00070042 | (cancer vs cancer) |
| 19-39898283 | cg10369688 | (cancer vs cancer)<br>(normal vs normal)<br>(cancer+normal vs cancer+normal) | 9-133308669 | cg14362630 | (cancer vs cancer) |
| 19-45257305 | cg11597497 | (cancer vs cancer)<br>(cancer+normal vs cancer+normal) | 9-133308888 | cg13496979 | (cancer vs cancer) |
| 19-46142212 | cg08831348 | (cancer+normal vs cancer+normal) | **Status - Hypo** | | |
| 19-46142574 | cg03096975 | (normal vs normal)<br>(cancer+normal vs cancer+normal) | 1-116610397 | cg24311182 | (cancer vs cancer) |
| 19-46142647 | cg07804711 | (normal vs normal)<br>(cancer+normal vs cancer+normal) | 1-118239410 | cg00544032 | (cancer vs cancer) |

| Position | Probe | Comparison | Position | Probe | Comparison |
|---|---|---|---|---|---|
| 19-46142673 | cg12536502 | (cancer vs cancer) (cancer+normal vs cancer+normal) | 1-155291224 | cg08264805 | (cancer vs cancer) (cancer+normal vs cancer+normal) |
| 19-46144774 | cg23533683 | (cancer vs cancer) (cancer+normal vs cancer+normal) | 1-1566687 | cg08231710 | (cancer vs cancer) (cancer+normal vs cancer+normal) |
| 2-231280878 | cg09456094 | (normal vs normal) (cancer+normal vs cancer+normal) | 1-161168957 | cg10493436 | (cancer vs cancer) (cancer+normal vs cancer+normal) |
| 2-231280907 | cg11702456 | (cancer vs cancer) (normal vs normal) (cancer+normal vs cancer+normal) | 1-161676266 | cg24664855 | (cancer vs cancer) |
| 2-231281474 | cg05091653 | (normal vs normal) (cancer+normal vs cancer+normal) | 1-168698463 | cg09922481 | (cancer vs cancer) (cancer+normal vs cancer+normal) |
| 2-28618831 | cg08836542 | (cancer+normal vs cancer+normal) | 1-168698479 | cg21266845 | (cancer vs cancer) (cancer+normal vs cancer+normal) |
| 20-31262314 | cg24467337 | (cancer vs cancer) (cancer+normal vs cancer+normal) | 1-172113506 | cg02633729 | (cancer vs cancer) (cancer+normal vs cancer+normal) |
| 20-43883307 | cg07668993 | (normal vs normal) (cancer+normal vs cancer+normal) | 1-172113756 | cg25214966 | (normal vs normal) (cancer vs cancer) (cancer+normal vs cancer+normal) |
| 20-61447490 | cg11368502 | (cancer+normal vs cancer+normal) | 1-172113920 | cg24002149 | (cancer vs cancer) (cancer+normal vs cancer+normal) |
| 22-36649144 | cg07713361 | (normal vs normal) (cancer+normal vs cancer+normal) | 1-172114047 | cg21450888 | (cancer vs cancer) (cancer+normal vs cancer+normal) |
| 3-28283857 | cg15038811 | (cancer vs cancer) (cancer+normal vs cancer+normal) | 1-172114068 | cg17178220 | (cancer vs cancer) (cancer+normal vs cancer+normal) |
| 3-50639079 | cg13519902 | (normal vs normal) (cancer+normal vs cancer+normal) | 1-172114419 | cg03322057 | (cancer vs cancer) (cancer+normal vs cancer+normal) |
| 4-129474594 | cg07174665 | (normal vs normal) (cancer+normal vs cancer+normal) | 1-201511610 | cg22534374 | (cancer vs cancer) |
| 4-129474618 | cg17521583 | (normal vs normal) (cancer+normal vs cancer+normal) | 1-2028213 | cg00312809 | (cancer vs cancer) |
| 4-809452 | cg24464265 | (normal vs normal) (cancer+normal vs cancer+normal) | 1-206879950 | cg06419268 | (cancer vs cancer) |
| 4-87857418 | cg11468363 | (cancer+normal vs cancer+normal) | 1-229733305 | cg04128065 | (cancer vs cancer) |
| 5-52404911 | cg01065586 | (normal vs normal) (cancer+normal vs cancer+normal) | 1-753376 | cg09989996 | (cancer vs cancer) |
| 6-150393535 | cg07336872 | (cancer+normal vs cancer+normal) | 1-86044230 | cg17108141 | (cancer vs cancer) |
| 6-20211654 | cg21119074 | (cancer+normal vs cancer+normal) | 10-124254860 | cg15719652 | (cancer vs cancer) |
| 6-25962987 | cg08501292 | (cancer vs cancer) (cancer vs cancer) (normal vs normal) (cancer+normal vs cancer+normal) | 10-14551049 | cg03654668 | (cancer vs cancer) |

| | | | | | |
|---|---|---|---|---|---|
| 6-25963109 | cg06153893 | (cancer vs cancer) (normal vs normal) (cancer+normal vs cancer+normal) | 10-14551091 | cg27304519 | (cancer vs cancer) |
| 6-25963442 | cg21844956 | (cancer vs cancer) (normal vs normal) (cancer+normal vs cancer+normal) | 10-14551178 | cg01253386 | (cancer vs cancer) |
| 6-31705988 | cg10563645 | (cancer+normal vs cancer+normal) | 10-90712478 | cg10894512 | (cancer vs cancer) (cancer+normal vs cancer+normal) |
| 6-43237330 | cg23514619 | (cancer vs cancer) (cancer vs cancer) (normal vs normal) (cancer+normal vs cancer+normal) | 11-2730129 | cg03512098 | (cancer vs cancer) (cancer+normal vs cancer+normal) |
| 6-43237511 | cg20434586 | (cancer vs cancer) (cancer vs cancer) (normal vs normal) (cancer+normal vs cancer+normal) | 11-68779817 | cg13734833 | (cancer vs cancer) (cancer+normal vs cancer+normal) |
| 7-101500303 | cg22202558 | (cancer+normal vs cancer+normal) | 11-70505972 | cg18080819 | (cancer vs cancer) |
| 8-30580804 | cg08305799 | (cancer vs cancer) (cancer vs cancer) (cancer+normal vs cancer+normal) | 12-2289797 | cg11830694 | (cancer vs cancer) (cancer+normal vs cancer+normal) |
| 8-65293126 | cg07773510 | (cancer+normal vs cancer+normal) | 13-30009956 | cg10490577 | (cancer vs cancer) |
| 8-80803365 | cg06107816 | (cancer vs cancer) (cancer+normal vs cancer+normal) | 13-30096184 | cg11760099 | (cancer vs cancer) |
| **Status - Hypo** | | | 13-73833939 | cg12762039 | (cancer vs cancer) (cancer+normal vs cancer+normal) |
| 1-10240017 | cg27508545 | (normal vs normal) (cancer vs cancer) (cancer vs cancer) (cancer+normal vs cancer+normal) | 14-102394448 | cg14225665 | (cancer vs cancer) (cancer+normal vs cancer+normal) |
| 1-10240025 | cg23661000 | (normal vs normal) (cancer vs cancer) (cancer vs cancer) (cancer+normal vs cancer+normal) | 14-23624788 | cg23168192 | (cancer vs cancer) (cancer+normal vs cancer+normal) |
| 1-2006465 | cg06791083 | (cancer vs cancer) (cancer+normal vs cancer+normal) | 14-76445988 | cg10782206 | (cancer vs cancer) (cancer+normal vs cancer+normal) |
| 1-36559506 | cg09506001 | (cancer vs cancer) (cancer vs cancer) (cancer+normal vs cancer+normal) | 14-76446161 | cg13121428 | (cancer vs cancer) (cancer+normal vs cancer+normal) |
| 1-66458803 | cg26912671 | (cancer vs cancer) (cancer+normal vs cancer+normal) | 14-76461805 | cg19530831 | (cancer vs cancer) (cancer+normal vs cancer+normal) |
| 1-9448660 | cg12658495 | (cancer vs cancer) (cancer vs cancer) (cancer+normal vs cancer+normal) | 15-49563246 | cg11935459 | (cancer vs cancer) |
| 10-115738883 | cg01467532 | (cancer vs cancer) (cancer vs cancer) | 15-50170642 | cg20651644 | (cancer vs cancer) (cancer+normal vs |

EP 3 692 164 B1

| | | | | | |
|---|---|---|---|---|---|
| | | (cancer+normal vs cancer+normal) | | | cancer+normal) |
| 10-134552168 | cg05760937 | (cancer+normal vs cancer+normal) | 15-50316779 | cg21204588 | (cancer vs cancer) (cancer+normal vs cancer+normal) |
| 10-405225 | cg20595750 | (normal vs normal) (cancer vs cancer) (cancer vs cancer) (cancer+normal vs cancer+normal) | 15-65143918 | cg13941978 | (cancer vs cancer) (cancer+normal vs cancer+normal) |
| 10-405362 | cg18074403 | (cancer vs cancer) (cancer+normal vs cancer+normal) | 15-86160762 | cg00691586 | (cancer vs cancer) (cancer+normal vs cancer+normal) |
| 10-661009 | cg19926144 | (cancer vs cancer) (cancer vs cancer) (cancer+normal vs cancer+normal) | 16-69310261 | cg02550308 | (cancer vs cancer) (cancer+normal vs cancer+normal) |
| 11-113577434 | cg23871994 | (cancer vs cancer) (cancer vs cancer) (cancer+normal vs cancer+normal) | 17-19287677 | cg06997997 | (cancer vs cancer) (cancer+normal vs cancer+normal) |
| 11-1486341 | cg07737063 | (cancer vs cancer) (cancer vs cancer) (cancer+normal vs cancer+normal) | 17-4108148 | cg21653149 | (cancer vs cancer) (cancer+normal vs cancer+normal) |
| 12-125500634 | cg01589353 | (normal vs normal) (cancer+normal vs cancer+normal) | 17-6658706 | cg06195280 | (cancer vs cancer) (cancer+normal vs cancer+normal) |
| 12-131452297 | cg23617848 | (cancer vs cancer) (cancer vs cancer) (cancer+normal vs cancer+normal) | 17-6658728 | cg03419151 | (cancer vs cancer) (cancer+normal vs cancer+normal) |
| 12-4596426 | cg24211304 | (cancer vs cancer) (cancer vs cancer) (cancer+normal vs cancer+normal) | 17-79439144 | cg05115862 | (cancer vs cancer) |
| 12-656703 | cg03539204 | (cancer vs cancer) (cancer vs cancer) (cancer+normal vs cancer+normal) | 17-8584536 | cg15905656 | (cancer vs cancer) (cancer+normal vs cancer+normal) |
| 13-111893537 | cg26951340 | (normal vs normal) (cancer vs cancer) (cancer vs cancer) (cancer+normal vs cancer+normal) | 19-10928172 | cg27648270 | (cancer vs cancer) (cancer+normal vs cancer+normal) |
| 13-113436564 | cg06206670 | (cancer vs cancer) (cancer vs cancer) (cancer+normal vs cancer+normal) | 19-10928178 | cg18544365 | (cancer vs cancer) (cancer+normal vs cancer+normal) |
| 13-113436568 | cg14737571 | (cancer vs cancer) (cancer vs cancer) (cancer+normal vs cancer+normal) | 19-10928211 | cg23047544 | (cancer vs cancer) (cancer+normal vs cancer+normal) |
| 13-36944471 | cg00084432 | (cancer+normal vs cancer+normal) | 19-10928233 | cg02660440 | (cancer vs cancer) (cancer+normal vs cancer+normal) |
| 13-80585265 | cg03010009 | (cancer vs cancer) (cancer vs cancer) (cancer+normal vs cancer+normal) | 19-10928322 | cg06754197 | (cancer vs cancer) (cancer+normal vs cancer+normal) |
| 14- | cg03403507 | (cancer vs cancer) | 19- | cg0290706 | (cancer vs cancer) |

278

| | | | | | |
|---|---|---|---|---|---|
| 32798387 | | (cancer vs cancer) (cancer+normal vs cancer+normal) | 10928327 | 4 | (cancer+normal vs cancer+normal) |
| 14-32798409 | cg16683572 | (cancer+normal vs cancer+normal) | 19-10928549 | cg23068797 | (cancer vs cancer) |
| 14-32798762 | cg24812523 | (cancer vs cancer) (cancer vs cancer) (cancer+normal vs cancer+normal) | 19-10928639 | cg03216043 | (cancer vs cancer) (cancer+normal vs cancer+normal) |
| 14-95599667 | cg24158307 | (cancer vs cancer) (cancer vs cancer) (cancer+normal vs cancer+normal) | 19-10928696 | cg13965612 | (cancer vs cancer) (cancer+normal vs cancer+normal) |
| 16-2177269 | cg03494430 | (cancer vs cancer) (cancer+normal vs cancer+normal) | 2-110064019 | cg15993723 | (cancer vs cancer) |
| 16-50897271 | cg00900231 | (normal vs normal) (cancer vs cancer) (cancer vs cancer) (cancer+normal vs cancer+normal) | 2-153252785 | cg01624229 | (cancer vs cancer) |
| 16-69854895 | cg03346415 | (cancer vs cancer) (cancer+normal vs cancer+normal) | 2-23840739 | cg14067761 | (cancer vs cancer) (cancer+normal vs cancer+normal) |
| 16-72460083 | cg02619656 | (normal vs normal) (cancer vs cancer) (cancer vs cancer) (cancer+normal vs cancer+normal) | 2-23840763 | cg02338778 | (cancer vs cancer) (cancer+normal vs cancer+normal) |
| 16-72460114 | cg05347108 | (normal vs normal) (cancer vs cancer) (cancer vs cancer) (cancer+normal vs cancer+normal) | 2-240230587 | cg17356718 | (cancer vs cancer) (cancer+normal vs cancer+normal) |
| 16-75300795 | cg00898447 | (cancer+normal vs cancer+normal) | 2-240230663 | cg20298668 | (cancer vs cancer) (cancer+normal vs cancer+normal) |
| 16-75300861 | cg00319595 | (cancer vs cancer) (cancer vs cancer) (cancer+normal vs cancer+normal) | 2-240230850 | cg05171197 | (cancer vs cancer) (cancer+normal vs cancer+normal) |
| 16-85618206 | cg07585257 | (normal vs normal) (cancer+normal vs cancer+normal) | 2-240230892 | cg19449565 | (normal vs normal) (cancer vs cancer) (cancer+normal vs cancer+normal) |
| 16-88041289 | cg03551062 | (cancer vs cancer) (cancer vs cancer) (cancer+normal vs cancer+normal) | 21-44089014 | cg21639922 | (cancer vs cancer) (cancer+normal vs cancer+normal) |
| 16-89603606 | cg02207944 | (cancer vs cancer) (cancer+normal vs cancer+normal) | 21-44089142 | cg27160952 | (cancer vs cancer) (cancer+normal vs cancer+normal) |
| 16-89623374 | cg01279418 | (normal vs normal) (cancer vs cancer) (cancer+normal vs cancer+normal) | 3-107381326 | cg23733260 | (normal vs normal) (cancer vs cancer) (cancer+normal vs cancer+normal) |
| 17-1308616 | cg13691567 | (normal vs normal) (cancer vs cancer) (cancer+normal vs cancer+normal) | 3-124531371 | cg00171092 | (cancer vs cancer) (cancer+normal vs cancer+normal) |
| 17- | cg00145352 | (normal vs normal) | 3-12980202 | cg1608109 | (cancer vs cancer) |

| | | | | | |
|---|---|---|---|---|---|
| 2319906 | | (cancer vs cancer) (cancer vs cancer) (cancer+normal vs cancer+normal) | | 6 | (cancer+normal vs cancer+normal) |
| 17-29624396 | cg18232597 | (normal vs normal) (cancer vs cancer) (cancer+normal vs cancer+normal) | 3-13420826 | cg01562813 | (cancer vs cancer) |
| 17-71345538 | cg00711291 | (cancer vs cancer) (cancer vs cancer) (cancer+normal vs cancer+normal) | 3-13420961 | cg18313416 | (cancer vs cancer) (cancer+normal vs cancer+normal) |
| 17-76220672 | cg10140240 | (cancer+normal vs cancer+normal) | 3-4625188 | cg02105211 | (cancer vs cancer) (cancer+normal vs cancer+normal) |
| 17-76220898 | cg19272238 | (cancer vs cancer) (cancer+normal vs cancer+normal) | 4-38121914 | cg17508905 | (cancer vs cancer) |
| 17-76220929 | cg07366188 | (cancer vs cancer) (cancer vs cancer) (cancer+normal vs cancer+normal) | 4-38871320 | cg20016631 | (cancer vs cancer) (cancer+normal vs cancer+normal) |
| 17-76220955 | cg10070788 | (cancer vs cancer) (cancer vs cancer) (cancer+normal vs cancer+normal) | 4-53862521 | cg05813673 | (cancer vs cancer) (cancer+normal vs cancer+normal) |
| 17-9151012 | cg12130672 | (cancer vs cancer) (cancer vs cancer) (cancer+normal vs cancer+normal) | 4-54765826 | cg11984971 | (cancer vs cancer) (cancer+normal vs cancer+normal) |
| 18-32398181 | cg22291942 | (normal vs normal) (cancer vs cancer) (cancer vs cancer) (cancer+normal vs cancer+normal) | 4-89152212 | cg23706819 | (cancer vs cancer) (cancer+normal vs cancer+normal) |
| 18-42324965 | cg03722909 | (normal vs normal) (cancer vs cancer) (cancer vs cancer) (cancer+normal vs cancer+normal) | 5-156988400 | cg14104626 | (cancer vs cancer) |
| 18-42325086 | cg07015525 | (normal vs normal) (cancer vs cancer) (cancer vs cancer) (cancer+normal vs cancer+normal) | 5-171320720 | cg26112871 | (cancer vs cancer) (cancer+normal vs cancer+normal) |
| 18-686346 | cg01037496 | (normal vs normal) (cancer vs cancer) (cancer+normal vs cancer+normal) | 5-172306112 | cg16345114 | (cancer vs cancer) |
| 19-1255283 | cg27172769 | (cancer vs cancer) (cancer+normal vs cancer+normal) | 5-53814891 | cg21995347 | (cancer vs cancer) |
| 19-1255516 | cg24300446 | (cancer vs cancer) (cancer vs cancer) (cancer+normal vs cancer+normal) | 6-112081538 | cg08061598 | (normal vs normal) (cancer vs cancer) (cancer+normal vs cancer+normal) |
| 19-47158242 | cg06454084 | (cancer+normal vs cancer+normal) | 6-145527462 | cg07198369 | (cancer vs cancer) |
| 19-47770746 | cg15080870 | (cancer vs cancer) (cancer vs cancer) (cancer+normal vs | 6-1702693 | cg13253856 | (cancer vs cancer) |

| | | | | | |
|---|---|---|---|---|---|
| | | cancer+normal) | | | |
| 2-145196972 | cg03303633 | (normal vs normal) (cancer+normal vs cancer+normal) | 6-28557765 | cg17017591 | (cancer vs cancer) (cancer+normal vs cancer+normal) |
| 2-182850683 | cg13218739 | (normal vs normal) (cancer+normal vs cancer+normal) | 6-45914282 | cg01330316 | (cancer vs cancer) (cancer+normal vs cancer+normal) |
| 2-236716219 | cg22764497 | (cancer vs cancer) (cancer vs cancer) (cancer+normal vs cancer+normal) | 7-130033247 | cg06917763 | (cancer vs cancer) (cancer+normal vs cancer+normal) |
| 2-236716285 | cg06694561 | (cancer vs cancer) (cancer vs cancer) (cancer+normal vs cancer+normal) | 7-151297347 | cg14481469 | (cancer vs cancer) (cancer+normal vs cancer+normal) |
| 2-236716372 | cg06747919 | (cancer vs cancer) (cancer vs cancer) (cancer+normal vs cancer+normal) | 7-17691191 | cg03947384 | (cancer vs cancer) |
| 2-23879137 | cg00746446 | (cancer vs cancer) (cancer vs cancer) (cancer+normal vs cancer+normal) | 8-100770219 | cg19609438 | (cancer vs cancer) |
| 2-240117222 | cg06257052 | (normal vs normal) (cancer vs cancer) (cancer vs cancer) (cancer+normal vs cancer+normal) | 8-107937633 | cg25545598 | (cancer vs cancer) (cancer+normal vs cancer+normal) |
| 2-240117640 | cg20853370 | (cancer vs cancer) (cancer vs cancer) (cancer+normal vs cancer+normal) | 8-19034837 | cg23089425 | (cancer vs cancer) |
| 2-24443636 | cg05139187 | (normal vs normal) (cancer+normal vs cancer+normal) | 8-38322629 | cg02043791 | (cancer vs cancer) (cancer+normal vs cancer+normal) |
| 2-30041338 | cg00073794 | (cancer vs cancer) (cancer vs cancer) (cancer+normal vs cancer+normal) | 8-49467831 | cg06043908 | (cancer vs cancer) (cancer+normal vs cancer+normal) |
| 2-3292757 | cg03025986 | (normal vs normal) (cancer vs cancer) (cancer vs cancer) (cancer+normal vs cancer+normal) | 8-74363469 | cg11064454 | (cancer vs cancer) (cancer+normal vs cancer+normal) |
| 2-3480658 | cg05777919 | (cancer vs cancer) (cancer vs cancer) (cancer+normal vs cancer+normal) | 9-113100053 | cg05154454 | (cancer vs cancer) (cancer+normal vs cancer+normal) |
| 2-55174473 | cg04768463 | (cancer vs cancer) (cancer vs cancer) (cancer+normal vs cancer+normal) | 9-114812406 | cg14308311 | (cancer vs cancer) (cancer+normal vs cancer+normal) |
| 22-22288473 | cg12894883 | (normal vs normal) (cancer vs cancer) (cancer vs cancer) (cancer+normal vs cancer+normal) | 9-85020050 | cg14299115 | (cancer vs cancer) (cancer+normal vs cancer+normal) |
| 22-24041104 | cg00489795 | (cancer vs cancer) (cancer vs cancer) (cancer+normal vs cancer+normal) | **(cancer+normal vs cancer+normal)** | | |

| | | |
|---|---|---|
| 22-42611379 | cg26799416 | (cancer vs cancer) (cancer vs cancer) (cancer+normal vs cancer+normal) |
| 3-127327369 | cg12678686 | (cancer vs cancer) (cancer vs cancer) (cancer+normal vs cancer+normal) |
| 3-129408521 | cg19272592 | (cancer vs cancer) (cancer vs cancer) (cancer+normal vs cancer+normal) |
| 3-147657586 | cg03320743 | (cancer vs cancer) (cancer vs cancer) (cancer+normal vs cancer+normal) |
| 3-195616361 | cg20224111 | (cancer vs cancer) (cancer vs cancer) (cancer+normal vs cancer+normal) |
| 4-146804010 | cg01578875 | (normal vs normal) (cancer vs cancer) (cancer vs cancer) (cancer+normal vs cancer+normal) |
| 4-24982540 | cg10569820 | (normal vs normal) (cancer vs cancer) (cancer vs cancer) (cancer+normal vs cancer+normal) |
| 5-102834858 | cg06612355 | (cancer vs cancer) (cancer vs cancer) (cancer+normal vs cancer+normal) |
| 5-179494929 | cg10519882 | (cancer vs cancer) (cancer+normal vs cancer+normal) |
| 5-77289836 | cg09645993 | (normal vs normal) (cancer vs cancer) (cancer vs cancer) (cancer+normal vs cancer+normal) |
| 6-102224868 | cg24574819 | (normal vs normal) (cancer vs cancer) (cancer vs cancer) (cancer+normal vs cancer+normal) |
| 6-143130570 | cg12616114 | (cancer+normal vs cancer+normal) |
| 6-157493773 | cg17596249 | (cancer vs cancer) (cancer vs cancer) (cancer+normal vs cancer+normal) |
| 6-24140822 | cg14549524 | (cancer vs cancer) (cancer vs cancer) (cancer+normal vs cancer+normal) |
| 6-24140899 | cg08908855 | (cancer vs cancer) (cancer vs cancer) (cancer+normal vs |

| Status - Hyper | | |
|---|---|---|
| 1-165322606 | cg27636676 | (cancer vs cancer) (cancer+normal vs cancer+normal) |
| 1-165322784 | cg10315533 | (cancer vs cancer) (cancer+normal vs cancer+normal) |
| 1-165323232 | cg26522592 | (cancer vs cancer) (cancer+normal vs cancer+normal) |
| 1-169079632 | cg24304617 | (cancer vs cancer) (cancer+normal vs cancer+normal) |
| 1-214435182 | cg16682225 | (normal vs normal) (cancer+normal vs cancer+normal) |
| 10-130832268 | cg11589800 | (cancer+normal vs cancer+normal) |
| 10-130832393 | cg08881175 | (cancer+normal vs cancer+normal) |
| 10-70748274 | cg18947032 | (cancer+normal vs cancer+normal) |
| 10-831247 | cg01169559 | (cancer+normal vs cancer+normal) |
| 11-35439994 | cg27457284 | (cancer vs cancer) (cancer+normal vs cancer+normal) |
| 11-35440062 | cg24183324 | (cancer vs cancer) (cancer+normal vs cancer+normal) |
| 11-44335060 | cg02443626 | (cancer+normal vs cancer+normal) |
| 11-44338782 | cg19935945 | (cancer+normal vs cancer+normal) |
| 12-111835267 | cg27002516 | (cancer+normal vs cancer+normal) |

| | | | | | |
|---|---|---|---|---|---|
| | | cancer+normal) | | | |
| 7-101723568 | cg17713751 | (normal vs normal) (cancer+normal vs cancer+normal) | 12-123383399 | cg11586448 | (normal vs normal) (cancer+normal vs cancer+normal) |
| 7-101723947 | cg14203426 | (normal vs normal) (cancer+normal vs cancer+normal) | 12-49783222 | cg00560284 | (cancer+normal vs cancer+normal) |
| 7-101849120 | cg06010390 | (normal vs normal) (cancer vs cancer) (cancer vs cancer) (cancer+normal vs cancer+normal) | 12-53264540 | cg03510486 | (cancer+normal vs cancer+normal) |
| 7-101849473 | cg01234984 | (cancer vs cancer) (cancer vs cancer) (cancer+normal vs cancer+normal) | 12-89744471 | cg00772407 | (cancer+normal vs cancer+normal) |
| 7-105283982 | cg11895696 | (cancer vs cancer) (cancer vs cancer) (cancer+normal vs cancer+normal) | 12-89744524 | cg01814191 | (cancer+normal vs cancer+normal) |
| 7-140034703 | cg17102674 | (cancer vs cancer) (cancer+normal vs cancer+normal) | 12-89744609 | cg17740822 | (cancer+normal vs cancer+normal) |
| 7-2148642 | cg16329842 | (normal vs normal) (cancer vs cancer) (cancer+normal vs cancer+normal) | 13-37838311 | cg26015163 | (cancer vs cancer) (cancer+normal vs cancer+normal) |
| 7-4747216 | cg01275887 | (cancer vs cancer) (cancer vs cancer) (cancer+normal vs cancer+normal) | 13-92002454 | cg02297838 | (cancer vs cancer) (cancer+normal vs cancer+normal) |
| 7-6465799 | cg18642425 | (cancer+normal vs cancer+normal) | 14-72943461 | cg18515872 | (cancer vs cancer) (cancer+normal vs cancer+normal) |
| 8-131011240 | cg24525461 | (normal vs normal) (cancer+normal vs cancer+normal) | 15-63894296 | cg23737062 | (cancer+normal vs cancer+normal) |
| 8-26440349 | cg00470636 | (cancer vs cancer) (cancer+normal vs cancer+normal) | 15-90735148 | cg24924577 | (cancer+normal vs cancer+normal) |
| 8-87570714 | cg02712949 | (cancer vs cancer) (cancer vs cancer) (cancer+normal vs cancer+normal) | 15-99559002 | cg19823138 | (cancer vs cancer) (cancer+normal vs cancer+normal) |
| 8-9260354 | cg10359460 | (normal vs normal) (cancer vs cancer) (cancer vs cancer) (cancer+normal vs cancer+normal) | 15-99791282 | cg08096291 | (normal vs normal) (cancer+normal vs cancer+normal) |
| 8-9537322 | cg10419849 | (cancer vs cancer) (cancer vs cancer) (cancer+normal vs cancer+normal) | 16-2198075 | cg00664609 | (cancer+normal vs cancer+normal) |
| 9-96846674 | cg14467276 | (cancer+normal vs cancer+normal) | 16-85648263 | cg07301329 | (cancer vs cancer) (cancer+normal vs cancer+normal) |
| 9-99264127 | cg14155501 | (normal vs normal) (cancer vs cancer) (cancer vs cancer) (cancer+normal vs cancer+normal) | 16-86371248 | cg03936135 | (cancer+normal vs cancer+normal) |

| | | | | | |
|---|---|---|---|---|---|
| (normal vs normal) | | | 16-88292996 | cg14705079 | (cancer vs cancer) (cancer+normal vs cancer+normal) |
| Status - Hyper | | | 16-88729968 | cg10330885 | (normal vs normal) (cancer+normal vs cancer+normal) |
| 1-100231682 | cg04308687 | (normal vs normal) (cancer+normal vs cancer+normal) | 17-6347142 | cg25288085 | (normal vs normal) (cancer+normal vs cancer+normal) |
| 1-12678641 | cg17935811 | (cancer vs cancer) (normal vs normal) (cancer+normal vs cancer+normal) | 17-77810742 | cg06976025 | (cancer vs cancer) (cancer+normal vs cancer+normal) |
| 1-12678761 | cg01346152 | (cancer vs cancer) (normal vs normal) (cancer+normal vs cancer+normal) | 17-77810912 | cg19715771 | (cancer+normal vs cancer+normal) |
| 1-175161069 | cg03317045 | (normal vs normal) (cancer+normal vs cancer+normal) | 18-45934595 | cg07933552 | (cancer+normal vs cancer+normal) |
| 1-203320506 | cg22705746 | (normal vs normal) (cancer+normal vs cancer+normal) | 19-48833410 | cg10338830 | (cancer vs cancer) (cancer+normal vs cancer+normal) |
| 1-212769641 | cg10985914 | (cancer vs cancer) (normal vs normal) (cancer+normal vs cancer+normal) | 19-48833559 | cg17315219 | (cancer vs cancer) (cancer+normal vs cancer+normal) |
| 1-23854847 | cg11526198 | (normal vs normal) (cancer+normal vs cancer+normal) | 19-940724 | cg12713583 | (cancer+normal vs cancer+normal) |
| 1-31655009 | cg18232347 | (normal vs normal) | 2-128173685 | cg24430189 | (cancer vs cancer) (cancer+normal vs cancer+normal) |
| 1-59280952 | cg15687600 | (cancer vs cancer) (normal vs normal) (cancer+normal vs cancer+normal) | 2-128173750 | cg02473600 | (cancer vs cancer) (cancer+normal vs cancer+normal) |
| 1-59281067 | cg03611060 | (cancer vs cancer) (normal vs normal) (cancer+normal vs cancer+normal) | 2-162364264 | cg24695950 | (normal vs normal) (cancer+normal vs cancer+normal) |
| 1-89873226 | cg13938909 | (normal vs normal) (cancer+normal vs cancer+normal) | 2-177021899 | cg10107434 | (cancer vs cancer) (cancer+normal vs cancer+normal) |
| 10-13749193 | cg16485048 | (normal vs normal) (cancer+normal vs cancer+normal) | 2-177022056 | cg25708328 | (cancer vs cancer) (cancer+normal vs cancer+normal) |
| 10-22606053 | cg14014799 | (normal vs normal) | 2-177022121 | cg18338775 | (cancer vs cancer) (cancer+normal vs cancer+normal) |
| 10-45474372 | cg02076355 | (normal vs normal) (cancer+normal vs cancer+normal) | 2-177022962 | cg05669418 | (cancer vs cancer) (cancer+normal vs cancer+normal) |
| 11-126456119 | cg06230206 | (normal vs normal) | 2-177023062 | cg09784934 | (cancer vs cancer) (cancer+normal vs cancer+normal) |
| 11-31819464 | cg06666008 | (normal vs normal) | 2-231729487 | cg15127563 | (cancer+normal vs cancer+normal) |
| 11-31822302 | cg16865446 | (normal vs normal) | 2-23604202 | cg14893576 | (cancer+normal vs cancer+normal) |
| 11- | cg23287710 | (normal vs normal) | 2- | cg1579641 | (cancer+normal vs |

| | | | | | |
|---|---|---|---|---|---|
| 31822526 | | | 241923082 | 7 | cancer+normal) |
| 11-31825166 | cg09224689 | (normal vs normal) | 20-3653555 | cg06023791 | (cancer vs cancer) (cancer+normal vs cancer+normal) |
| 11-31825214 | cg04938549 | (normal vs normal) | 21-40721130 | cg24641125 | (normal vs normal) (cancer+normal vs cancer+normal) |
| 11-31829582 | cg09217215 | (normal vs normal) | 3-118753005 | cg01845041 | (cancer+normal vs cancer+normal) |
| 11-31837104 | cg11162118 | (normal vs normal) | 3-157815672 | cg03458172 | (cancer vs cancer) (cancer+normal vs cancer+normal) |
| 11-31840628 | cg06312283 | (normal vs normal) | 3-157815808 | cg05401764 | (cancer vs cancer) (cancer+normal vs cancer+normal) |
| 11-31841524 | cg20512711 | (normal vs normal) | 3-157816094 | cg09095222 | (cancer vs cancer) (normal vs normal) (cancer+normal vs cancer+normal) |
| 11-31845389 | cg24701575 | (normal vs normal) | 3-157816330 | cg00089486 | (cancer vs cancer) (cancer+normal vs cancer+normal) |
| 11-31849262 | cg08370146 | (normal vs normal) | 3-28616404 | cg00380860 | (cancer+normal vs cancer+normal) |
| 12-124247223 | cg11026555 | (normal vs normal) | 4-105411756 | cg17473165 | (cancer+normal vs cancer+normal) |
| 12-56523270 | cg23588713 | (normal vs normal) | 4-175635801 | cg22747507 | (cancer+normal vs cancer+normal) |
| 12-57504948 | cg01063813 | (cancer vs cancer) (cancer vs cancer) (normal vs normal) (cancer+normal vs cancer+normal) | 4-186732926 | cg04392082 | (cancer vs cancer) (cancer+normal vs cancer+normal) |
| 12-57504988 | cg27320213 | (normal vs normal) | 4-186732932 | cg02790305 | (cancer vs cancer) (cancer+normal vs cancer+normal) |
| 12-96441714 | cg25671716 | (normal vs normal) (cancer+normal vs cancer+normal) | 4-186732936 | cg26785346 | (cancer vs cancer) (cancer+normal vs cancer+normal) |
| 13-100624855 | cg16033633 | (normal vs normal) | 4-186733024 | cg13921444 | (cancer vs cancer) (cancer+normal vs cancer+normal) |
| 13-100626995 | cg01792403 | (normal vs normal) | 4-43901032 | cg16838729 | (normal vs normal) (cancer+normal vs cancer+normal) |
| 13-100627175 | cg25845367 | (normal vs normal) | 5-122434944 | cg01360054 | (cancer vs cancer) (cancer+normal vs cancer+normal) |
| 13-100641243 | cg25527494 | (normal vs normal) | 5-158533081 | cg27047836 | (cancer+normal vs cancer+normal) |
| 13-100641867 | cg19385331 | (normal vs normal) | 5-178563145 | cg09533145 | (cancer+normal vs cancer+normal) |
| 13-100643858 | cg05072470 | (normal vs normal) | 5-179740743 | cg23221052 | (cancer+normal vs cancer+normal) |
| 13-100644420 | cg10445449 | (normal vs normal) | 5-180700651 | cg11330843 | (normal vs normal) (cancer+normal vs cancer+normal) |
| 13-100644473 | cg03786327 | (normal vs normal) | 5-44332757 | cg16204420 | (cancer+normal vs cancer+normal) |
| 13-100644657 | cg11694262 | (normal vs normal) | 5-74965039 | cg26621088 | (cancer+normal vs cancer+normal) |

| | | | | | |
|---|---|---|---|---|---|
| 13-100646453 | cg16762841 | (normal vs normal) | 6-134217613 | cg25843115 | (cancer+normal vs cancer+normal) |
| 13-114927854 | cg11997708 | (cancer vs cancer) (cancer vs cancer) (normal vs normal) (cancer+normal vs cancer+normal) | 6-143380966 | cg11314042 | (cancer+normal vs cancer+normal) |
| 13-27557503 | cg03268893 | (cancer vs cancer) (normal vs normal) (cancer+normal vs cancer+normal) | 6-1607922 | cg18398056 | (cancer vs cancer) (cancer+normal vs cancer+normal) |
| 14-70038505 | cg23920953 | (normal vs normal) | 6-170598215 | cg17586256 | (cancer vs cancer) (cancer+normal vs cancer+normal) |
| 14-70038884 | cg18477204 | (normal vs normal) | 6-30851624 | cg16537676 | (cancer+normal vs cancer+normal) |
| 14-70038925 | cg02380334 | (cancer vs cancer) (cancer vs cancer) (normal vs normal) (cancer+normal vs cancer+normal) | 6-31461853 | cg25212457 | (normal vs normal) (cancer+normal vs cancer+normal) |
| 14-70038949 | cg14539393 | (cancer vs cancer) (normal vs normal) (cancer+normal vs cancer+normal) | 6-32063835 | cg01337207 | (cancer vs cancer) (cancer+normal vs cancer+normal) |
| 16-745663 | cg02958327 | (cancer vs cancer) (normal vs normal) (cancer+normal vs cancer+normal) | 6-32063838 | cg26266427 | (cancer vs cancer) (cancer+normal vs cancer+normal) |
| 16-745685 | cg01003448 | (normal vs normal) | 6-32063901 | cg07524919 | (cancer vs cancer) (cancer+normal vs cancer+normal) |
| 16-745687 | cg07482202 | (cancer vs cancer) (normal vs normal) (cancer+normal vs cancer+normal) | 6-32063991 | cg00872984 | (cancer vs cancer) (cancer+normal vs cancer+normal) |
| 17-1553202 | cg04313875 | (cancer vs cancer) (cancer vs cancer) (normal vs normal) (cancer+normal vs cancer+normal) | 6-32064677 | cg01485117 | (cancer vs cancer) (cancer+normal vs cancer+normal) |
| 17-36715787 | cg03026373 | (normal vs normal) (cancer+normal vs cancer+normal) | 6-32064692 | cg27081346 | (cancer vs cancer) (cancer+normal vs cancer+normal) |
| 17-36715798 | cg14862207 | (normal vs normal) | 6-32064749 | cg15793329 | (cancer vs cancer) (cancer+normal vs cancer+normal) |
| 17-36715953 | cg04194674 | (normal vs normal) | 6-32064810 | cg18102337 | (cancer+normal vs cancer+normal) |
| 17-42392668 | cg01122302 | (normal vs normal) | 6-32765109 | cg16516959 | (cancer+normal vs cancer+normal) |
| 17-42392697 | cg11088422 | (cancer vs cancer) (normal vs normal) | 6-34206084 | cg01745499 | (cancer+normal vs cancer+normal) |
| 17-42392983 | cg10712561 | (normal vs normal) (cancer+normal vs cancer+normal) | 6-45387347 | cg03474350 | (cancer vs cancer) (cancer+normal vs cancer+normal) |
| 17-55938748 | cg00023001 | (cancer vs cancer) (cancer vs cancer) (normal vs normal) (cancer+normal vs cancer+normal) | 6-45387395 | cg26358286 | (cancer vs cancer) (cancer+normal vs cancer+normal) |

| Position | cg ID | Comparison | Position | cg ID | Comparison |
|---|---|---|---|---|---|
| 17-55938871 | cg22451358 | (cancer vs cancer) (cancer vs cancer) (normal vs normal) (cancer+normal vs cancer+normal) | 7-100202882 | cg26100986 | (cancer vs cancer) (cancer+normal vs cancer+normal) |
| 19-1455513 | cg06615719 | (cancer vs cancer) (normal vs normal) (cancer+normal vs cancer+normal) | 7-100203114 | cg25680486 | (cancer vs cancer) (cancer+normal vs cancer+normal) |
| 19-1467952 | cg03306486 | (normal vs normal) | 7-151136940 | cg21035142 | (cancer+normal vs cancer+normal) |
| 19-1467979 | cg19333963 | (normal vs normal) | 7-26416987 | cg24755163 | (normal vs normal) (cancer+normal vs cancer+normal) |
| 19-1468943 | cg10565187 | (cancer vs cancer) (cancer vs cancer) (normal vs normal) (cancer+normal vs cancer+normal) | 7-42834498 | cg08568561 | (normal vs normal) (cancer+normal vs cancer+normal) |
| 19-1469891 | cg08958549 | (normal vs normal) | 7-77426814 | cg24669602 | (cancer+normal vs cancer+normal) |
| 19-19336240 | cg25814383 | (cancer vs cancer) (cancer vs cancer) (normal vs normal) (cancer+normal vs cancer+normal) | 8-102503892 | cg05458052 | (cancer vs cancer) (cancer+normal vs cancer+normal) |
| 19-39898283 | cg10369688 | (cancer vs cancer) (normal vs normal) (cancer+normal vs cancer+normal) | 8-102503970 | cg20342184 | (cancer vs cancer) (cancer+normal vs cancer+normal) |
| 19-46142574 | cg03096975 | (normal vs normal) (cancer+normal vs cancer+normal) | 8-49636840 | cg12743248 | (cancer+normal vs cancer+normal) |
| 19-46142647 | cg07804711 | (normal vs normal) (cancer+normal vs cancer+normal) | 8-72916620 | cg24523948 | (cancer vs cancer) (cancer+normal vs cancer+normal) |
| 2-231280698 | cg23539753 | (normal vs normal) | **Status - Hypo** | | |
| 2-231280878 | cg09456094 | (normal vs normal) (cancer+normal vs cancer+normal) | 1-114522670 | cg17253459 | (cancer+normal vs cancer+normal) |
| 2-231280907 | cg11702456 | (cancer vs cancer) (normal vs normal) (cancer+normal vs cancer+normal) | 1-155291224 | cg08264805 | (cancer vs cancer) (cancer+normal vs cancer+normal) |
| 2-231281474 | cg05091653 | (normal vs normal) (cancer+normal vs cancer+normal) | 1-1566687 | cg08231710 | (cancer vs cancer) (cancer+normal vs cancer+normal) |
| 2-48920986 | cg08299783 | (normal vs normal) | 1-161168957 | cg10493436 | (cancer vs cancer) (cancer+normal vs cancer+normal) |
| 20-20346426 | cg22610655 | (normal vs normal) | 1-16344977 | cg16110455 | (cancer+normal vs cancer+normal) |
| 20-21497429 | cg08183724 | (normal vs normal) | 1-168698463 | cg09922481 | (cancer vs cancer) (cancer+normal vs cancer+normal) |
| 20-43883307 | cg07668993 | (normal vs normal) (cancer+normal vs cancer+normal) | 1-168698479 | cg21266845 | (cancer vs cancer) (cancer+normal vs cancer+normal) |
| 22-36649144 | cg07713361 | (normal vs normal) (cancer+normal vs | 1-172113506 | cg02633729 | (cancer vs cancer) (cancer+normal vs |

| | | | | | |
|---|---|---|---|---|---|
| | | cancer+normal) | | | cancer+normal) |
| 3-50639079 | cg13519902 | (normal vs normal) (cancer+normal vs cancer+normal) | 1-172113756 | cg25214966 | (normal vs normal) (cancer vs cancer) (cancer+normal vs cancer+normal) |
| 3-62363642 | cg08994384 | (normal vs normal) | 1-172113920 | cg24002149 | (cancer vs cancer) (cancer+normal vs cancer+normal) |
| 4-129474594 | cg07174665 | (normal vs normal) (cancer+normal vs cancer+normal) | 1-172114047 | cg21450888 | (cancer vs cancer) (cancer+normal vs cancer+normal) |
| 4-129474618 | cg17521583 | (normal vs normal) (cancer+normal vs cancer+normal) | 1-172114068 | cg17178220 | (cancer vs cancer) (cancer+normal vs cancer+normal) |
| 4-809452 | cg24464265 | (normal vs normal) (cancer+normal vs cancer+normal) | 1-172114419 | cg03322057 | (cancer vs cancer) (cancer+normal vs cancer+normal) |
| 5-134880353 | cg21233688 | (normal vs normal) | 1-214477063 | cg22796363 | (cancer+normal vs cancer+normal) |
| 5-52404911 | cg01065586 | (normal vs normal) (cancer+normal vs cancer+normal) | 1-27881600 | cg26161057 | (cancer+normal vs cancer+normal) |
| 6-108437051 | cg19095187 | (normal vs normal) | 10-126782357 | cg15205441 | (cancer+normal vs cancer+normal) |
| 6-108496716 | cg06103642 | (normal vs normal) | 10-134334283 | cg11116274 | (cancer+normal vs cancer+normal) |
| 6-25962987 | cg08501292 | (cancer vs cancer) (cancer vs cancer) (normal vs normal) (cancer+normal vs cancer+normal) | 10-31872296 | cg26953469 | (cancer+normal vs cancer+normal) |
| 6-25963109 | cg06153893 | (cancer vs cancer) (normal vs normal) (cancer+normal vs cancer+normal) | 10-90712478 | cg10894512 | (cancer vs cancer) (cancer+normal vs cancer+normal) |
| 6-25963442 | cg21844956 | (cancer vs cancer) (normal vs normal) (cancer+normal vs cancer+normal) | 10-90712684 | cg09990481 | (cancer+normal vs cancer+normal) |
| 6-30711680 | cg08384314 | (normal vs normal) | 10-90712739 | cg07216529 | (cancer+normal vs cancer+normal) |
| 6-43237330 | cg23514619 | (cancer vs cancer) (cancer vs cancer) (normal vs normal) (cancer+normal vs cancer+normal) | 11-2730129 | cg03512098 | (cancer vs cancer) (cancer+normal vs cancer+normal) |
| 6-43237511 | cg20434586 | (cancer vs cancer) (cancer vs cancer) (normal vs normal) (cancer+normal vs cancer+normal) | 11-68779817 | cg13734833 | (cancer vs cancer) (cancer+normal vs cancer+normal) |
| 6-43424018 | cg00482398 | (normal vs normal) | 11-68779826 | cg23882131 | (cancer+normal vs cancer+normal) |
| 7-157479272 | cg04937416 | (normal vs normal) | 11-93262607 | cg18076842 | (cancer+normal vs cancer+normal) |
| 7-157487066 | cg05124021 | (normal vs normal) | 12-2289797 | cg11830694 | (cancer vs cancer) (cancer+normal vs cancer+normal) |
| 8-126447096 | cg14156441 | (normal vs normal) | 12-96628738 | cg19737746 | (cancer+normal vs cancer+normal) |
| 8- | cg02226645 | (normal vs normal) | 13- | cg1093822 | (cancer+normal vs |

| | | |
|---|---|---|
| 65291287 | | |
| 8-65291990 | cg01517740 | (normal vs normal) |
| 8-65292321 | cg11626236 | (normal vs normal) |
| 8-80803196 | cg20577468 | (cancer vs cancer) (normal vs normal) |
| **Status - Hypo** | | |
| 1-10240017 | cg27508545 | (normal vs normal) (cancer vs cancer) (cancer vs cancer) (cancer+normal vs cancer+normal) |
| 1-10240025 | cg23661000 | (normal vs normal) (cancer vs cancer) (cancer vs cancer) (cancer+normal vs cancer+normal) |
| 1-1361077 | cg00305774 | (normal vs normal) |
| 1-230975106 | cg12486498 | (normal vs normal) |
| 1-243651920 | cg22950111 | (normal vs normal) |
| 1-244217562 | cg01983216 | (normal vs normal) |
| 1-244318867 | cg15393025 | (normal vs normal) |
| 10-131694689 | cg26275858 | (normal vs normal) |
| 10-134058796 | cg10084213 | (normal vs normal) |
| 10-134548998 | cg11151314 | (normal vs normal) |
| 10-3313302 | cg13793681 | (normal vs normal) |
| 10-405225 | cg20595750 | (normal vs normal) (cancer vs cancer) (cancer vs cancer) (cancer+normal vs cancer+normal) |
| 10-7486981 | cg09137125 | (normal vs normal) |
| 11-70563677 | cg08718050 | (normal vs normal) |
| 11-70563792 | cg26429499 | (normal vs normal) |
| 12-125500634 | cg01589353 | (normal vs normal) (cancer+normal vs |

| | | |
|---|---|---|
| 42197109 | 1 | cancer+normal) |
| 13-73833939 | cg12762039 | (cancer vs cancer) (cancer+normal vs cancer+normal) |
| 14-102394448 | cg14225665 | (cancer vs cancer) (cancer+normal vs cancer+normal) |
| 14-23624788 | cg23168192 | (cancer vs cancer) (cancer+normal vs cancer+normal) |
| 14-76445988 | cg10782206 | (cancer vs cancer) (cancer+normal vs cancer+normal) |
| 14-76446161 | cg13121428 | (cancer vs cancer) (cancer+normal vs cancer+normal) |
| 14-76461805 | cg19530831 | (cancer vs cancer) (cancer+normal vs cancer+normal) |
| 15-50170642 | cg20651644 | (cancer vs cancer) (cancer+normal vs cancer+normal) |
| 15-50316779 | cg21204588 | (cancer vs cancer) (cancer+normal vs cancer+normal) |
| 15-51464013 | cg18154567 | (cancer+normal vs cancer+normal) |
| 15-62767926 | cg26987928 | (cancer+normal vs cancer+normal) |
| 15-65143918 | cg13941978 | (cancer vs cancer) (cancer+normal vs cancer+normal) |
| 15-86160762 | cg00691586 | (cancer vs cancer) (cancer+normal vs cancer+normal) |
| 16-23144103 | cg06271652 | (cancer+normal vs cancer+normal) |
| 16-69310261 | cg02550308 | (cancer vs cancer) (cancer+normal vs cancer+normal) |
| 16-70812814 | cg06357685 | (cancer+normal vs cancer+normal) |
| 16-84829007 | cg05720733 | (cancer+normal vs cancer+normal) |
| 17-19287677 | cg06997997 | (cancer vs cancer) (cancer+normal vs cancer+normal) |
| 17-37216821 | cg24670063 | (cancer+normal vs cancer+normal) |
| 17-4108148 | cg21653149 | (cancer vs cancer) (cancer+normal vs cancer+normal) |
| 17-6658706 | cg06195280 | (cancer vs cancer) (cancer+normal vs |

| | | | | | |
|---|---|---|---|---|---|
| | | cancer+normal) | | | cancer+normal) |
| 12-130434412 | cg23592392 | (normal vs normal) | 17-6658728 | cg03419151 | (cancer vs cancer) (cancer+normal vs cancer+normal) |
| 12-50400225 | cg16614527 | (normal vs normal) | 17-8584536 | cg15905656 | (cancer vs cancer) (cancer+normal vs cancer+normal) |
| 13-111893537 | cg26951340 | (normal vs normal) (cancer vs cancer) (cancer vs cancer) (cancer+normal vs cancer+normal) | 19-10928172 | cg27648270 | (cancer vs cancer) (cancer+normal vs cancer+normal) |
| 13-25743608 | cg11030811 | (normal vs normal) | 19-10928178 | cg18544365 | (cancer vs cancer) (cancer+normal vs cancer+normal) |
| 13-25743999 | cg13055385 | (normal vs normal) | 19-10928211 | cg23047544 | (cancer vs cancer) (cancer+normal vs cancer+normal) |
| 14-62550978 | cg19961800 | (normal vs normal) | 19-10928233 | cg02660440 | (cancer vs cancer) (cancer+normal vs cancer+normal) |
| 14-78023429 | cg18872420 | (normal vs normal) | 19-10928322 | cg06754197 | (cancer vs cancer) (cancer+normal vs cancer+normal) |
| 14-93735925 | cg03505817 | (normal vs normal) | 19-10928327 | cg02907064 | (cancer vs cancer) (cancer+normal vs cancer+normal) |
| 14-95562368 | cg10948630 | (normal vs normal) | 19-10928639 | cg03216043 | (cancer vs cancer) (cancer+normal vs cancer+normal) |
| 14-99655593 | cg07015803 | (normal vs normal) | 19-10928696 | cg13965612 | (cancer vs cancer) (cancer+normal vs cancer+normal) |
| 14-99655676 | cg16452866 | (normal vs normal) | 2-23840739 | cg14067761 | (cancer vs cancer) (cancer+normal vs cancer+normal) |
| 15-79357160 | cg23047203 | (normal vs normal) | 2-23840763 | cg02338778 | (cancer vs cancer) (cancer+normal vs cancer+normal) |
| 16-50897271 | cg00900231 | (normal vs normal) (cancer vs cancer) (cancer vs cancer) (cancer+normal vs cancer+normal) | 2-240230587 | cg17356718 | (cancer vs cancer) (cancer+normal vs cancer+normal) |
| 16-58329456 | cg09462089 | (normal vs normal) | 2-240230663 | cg20298668 | (cancer vs cancer) (cancer+normal vs cancer+normal) |
| 16-72459595 | cg07864976 | (normal vs normal) | 2-240230850 | cg05171197 | (cancer vs cancer) (cancer+normal vs cancer+normal) |
| 16-72459796 | cg27088725 | (normal vs normal) | 2-240230892 | cg19449565 | (normal vs normal) (cancer vs cancer) (cancer+normal vs cancer+normal) |
| 16-72459825 | cg07209034 | (normal vs normal) | 2-241719655 | cg03554736 | (cancer+normal vs cancer+normal) |
| 16-72460083 | cg02619656 | (normal vs normal) (cancer vs cancer) (cancer vs cancer) (cancer+normal vs cancer+normal) | 21-44089014 | cg21639922 | (cancer vs cancer) (cancer+normal vs cancer+normal) |

| | | | | | |
|---|---|---|---|---|---|
| 16-72460114 | cg05347108 | (normal vs normal) (cancer vs cancer) (cancer vs cancer) (cancer+normal vs cancer+normal) | 21-44089142 | cg2716095 2 | (cancer vs cancer) (cancer+normal vs cancer+normal) |
| 16-84940830 | cg06250127 | (normal vs normal) | 3-107381326 | cg2373326 0 | (normal vs normal) (cancer vs cancer) (cancer+normal vs cancer+normal) |
| 16-85618206 | cg07585257 | (normal vs normal) (cancer+normal vs cancer+normal) | 3-124531371 | cg0017109 2 | (cancer vs cancer) (cancer+normal vs cancer+normal) |
| 16-89623374 | cg01279418 | (normal vs normal) (cancer vs cancer) (cancer+normal vs cancer+normal) | 3-12980202 | cg1608109 6 | (cancer vs cancer) (cancer+normal vs cancer+normal) |
| 17-1308616 | cg13691567 | (normal vs normal) (cancer vs cancer) (cancer+normal vs cancer+normal) | 3-13420961 | cg1831341 6 | (cancer vs cancer) (cancer+normal vs cancer+normal) |
| 17-2319906 | cg00145352 | (normal vs normal) (cancer vs cancer) (cancer vs cancer) (cancer+normal vs cancer+normal) | 3-171523157 | cg0284722 0 | (cancer+normal vs cancer+normal) |
| 17-29624396 | cg18232597 | (normal vs normal) (cancer vs cancer) (cancer+normal vs cancer+normal) | 3-24083947 | cg2630992 9 | (cancer+normal vs cancer+normal) |
| 17-65471303 | cg15536947 | (normal vs normal) | 3-4625188 | cg0210521 1 | (cancer vs cancer) (cancer+normal vs cancer+normal) |
| 17-71533286 | cg26758486 | (normal vs normal) | 4-119948360 | cg0838415 5 | (cancer+normal vs cancer+normal) |
| 17-75208807 | cg11348106 | (normal vs normal) | 4-119948505 | cg0975785 9 | (cancer+normal vs cancer+normal) |
| 17-76455102 | cg05561386 | (normal vs normal) | 4-119948542 | cg1304291 8 | (normal vs normal) (cancer+normal vs cancer+normal) |
| 17-79098558 | cg09591867 | (normal vs normal) | 4-38871320 | cg2001663 1 | (cancer vs cancer) (cancer+normal vs cancer+normal) |
| 17-8958039 | cg10665961 | (normal vs normal) | 4-53862521 | cg0581367 3 | (cancer vs cancer) (cancer+normal vs cancer+normal) |
| 18-32398181 | cg22291942 | (normal vs normal) (cancer vs cancer) (cancer vs cancer) (cancer+normal vs cancer+normal) | 4-54765826 | cg1198497 1 | (cancer vs cancer) (cancer+normal vs cancer+normal) |
| 18-42324965 | cg03722909 | (normal vs normal) (cancer vs cancer) (cancer vs cancer) (cancer+normal vs cancer+normal) | 4-89152212 | cg2370681 9 | (cancer vs cancer) (cancer+normal vs cancer+normal) |
| 18-42325086 | cg07015525 | (normal vs normal) (cancer vs cancer) (cancer vs cancer) (cancer+normal vs cancer+normal) | 5-171320720 | cg2611287 1 | (cancer vs cancer) (cancer+normal vs cancer+normal) |
| 18-686346 | cg01037496 | (normal vs normal) | 5- | cg1302253 | (cancer+normal vs |

| | | | | | |
|---|---|---|---|---|---|
| | | (cancer vs cancer) (cancer+normal vs cancer+normal) | 171831028 | 4 | cancer+normal) |
| 18-72201665 | cg03148084 | (normal vs normal) | 6-112081538 | cg08061598 | (normal vs normal) (cancer vs cancer) (cancer+normal vs cancer+normal) |
| 18-74691423 | cg15391531 | (normal vs normal) | 6-28557765 | cg17017591 | (cancer vs cancer) (cancer+normal vs cancer+normal) |
| 2-145196972 | cg03303633 | (normal vs normal) (cancer+normal vs cancer+normal) | 6-43997388 | cg02598430 | (cancer+normal vs cancer+normal) |
| 2-182850683 | cg13218739 | (normal vs normal) (cancer+normal vs cancer+normal) | 6-45914282 | cg01330316 | (cancer vs cancer) (cancer+normal vs cancer+normal) |
| 2-236761021 | cg22373519 | (normal vs normal) | 6-52377740 | cg00593404 | (normal vs normal) (cancer+normal vs cancer+normal) |
| 2-236826207 | cg16096176 | (normal vs normal) | 6-52440589 | cg03024301 | (cancer+normal vs cancer+normal) |
| 2-236826245 | cg06526980 | (normal vs normal) | 6-6737080 | cg22684370 | (cancer+normal vs cancer+normal) |
| 2-240117222 | cg06257052 | (normal vs normal) (cancer vs cancer) (cancer vs cancer) (cancer+normal vs cancer+normal) | 7-130033247 | cg06917763 | (cancer vs cancer) (cancer+normal vs cancer+normal) |
| 2-24443636 | cg05139187 | (normal vs normal) (cancer+normal vs cancer+normal) | 7-130040067 | cg01539484 | (cancer+normal vs cancer+normal) |
| 2-3292757 | cg03025986 | (normal vs normal) (cancer vs cancer) (cancer vs cancer) (cancer+normal vs cancer+normal) | 7-151297347 | cg14481469 | (cancer vs cancer) (cancer+normal vs cancer+normal) |
| 2-43295389 | cg17681516 | (normal vs normal) | 8-107937633 | cg25545598 | (cancer vs cancer) (cancer+normal vs cancer+normal) |
| 2-95978534 | cg11418389 | (normal vs normal) | 8-38322629 | cg02043791 | (cancer vs cancer) (cancer+normal vs cancer+normal) |
| 20-43530216 | cg16718537 | (normal vs normal) | 8-49467831 | cg06043908 | (cancer vs cancer) (cancer+normal vs cancer+normal) |
| 20-62550740 | cg14523475 | (normal vs normal) (cancer vs cancer) | 8-74363469 | cg11064454 | (cancer vs cancer) (cancer+normal vs cancer+normal) |
| 22-22288473 | cg12894883 | (normal vs normal) (cancer vs cancer) (cancer vs cancer) (cancer+normal vs cancer+normal) | 9-113100053 | cg05154454 | (cancer vs cancer) (cancer+normal vs cancer+normal) |
| 3-17082105 | cg21767191 | (normal vs normal) | 9-114812406 | cg14308311 | (cancer vs cancer) (cancer+normal vs cancer+normal) |
| 3-44140250 | cg23180636 | (normal vs normal) | 9-85020050 | cg14299115 | (cancer vs cancer) (cancer+normal vs cancer+normal) |
| 4-146804010 | cg01578875 | (normal vs normal) (cancer vs cancer) | **(normal vs normal)** | | |

| | | | | | |
|---|---|---|---|---|---|
| | | (cancer vs cancer) (cancer+normal vs cancer+normal) | | | |
| 4-24982540 | cg10569820 | (normal vs normal) (cancer vs cancer) (cancer vs cancer) (cancer+normal vs cancer+normal) | **Status - Hyper** | | |
| 5-2515708 | cg17870484 | (normal vs normal) | 1-154238265 | cg14859874 | (normal vs normal) (normal vs normal) (cancer+normal vs cancer+normal) |
| 5-36662804 | cg21050001 | (normal vs normal) | 1-21348906 | cg15519065 | (normal vs normal) |
| 5-67484008 | cg11861970 | (normal vs normal) | 1-214435182 | cg16682225 | (normal vs normal) (cancer+normal vs cancer+normal) |
| 5-77289836 | cg09645993 | (normal vs normal) (cancer vs cancer) (cancer vs cancer) (cancer+normal vs cancer+normal) | 1-54359778 | cg20932440 | (normal vs normal) |
| 6-102224868 | cg24574819 | (normal vs normal) (cancer vs cancer) (cancer vs cancer) (cancer+normal vs cancer+normal) | 1-566570 | cg08477687 | (normal vs normal) |
| 6-113639635 | cg07259165 | (normal vs normal) | 1-6309343 | cg15892785 | (normal vs normal) |
| 6-134637428 | cg05966641 | (normal vs normal) | 10-131761302 | cg26137940 | (normal vs normal) |
| 6-163558575 | cg24145613 | (normal vs normal) | 10-21814538 | cg26056831 | (normal vs normal) |
| 6-163558823 | cg11724750 | (normal vs normal) | 10-70748261 | cg26082690 | (normal vs normal) |
| 6-166908811 | cg09233791 | (normal vs normal) | 10-89621862 | cg03236184 | (normal vs normal) |
| 6-17032431 | cg19076258 | (normal vs normal) | 10-98156179 | cg04983289 | (normal vs normal) |
| 6-17032522 | cg10807105 | (normal vs normal) | 11-115372874 | cg03910065 | (normal vs normal) |
| 6-29627296 | cg25017994 | (normal vs normal) | 11-126310409 | cg08572513 | (normal vs normal) |
| 6-33158503 | cg19628686 | (normal vs normal) | 11-2211939 | cg19586845 | (normal vs normal) |
| 7-101723568 | cg17713751 | (normal vs normal) (cancer+normal vs cancer+normal) | 11-63382085 | cg03627015 | (normal vs normal) |
| 7-101723947 | cg14203426 | (normal vs normal) (cancer+normal vs cancer+normal) | 11-68171070 | cg07209141 | (normal vs normal) |
| 7-101849120 | cg06010390 | (normal vs normal) (cancer vs cancer) (cancer vs cancer) (cancer+normal vs cancer+normal) | 12-27273951 | cg02368737 | (normal vs normal) |
| 7-154035993 | cg15264162 | (normal vs normal) | 12-34317569 | cg25155766 | (normal vs normal) |
| 7-156190025 | cg19130610 | (normal vs normal) | 12-48478849 | cg24232869 | (normal vs normal) (cancer+normal vs cancer+normal) |

| | | | | | |
|---|---|---|---|---|---|
| 7-157281251 | cg10784273 | (normal vs normal) | 12-54144921 | cg06858186 | (normal vs normal) |
| 7-157394633 | cg14289852 | (normal vs normal) | 12-54354590 | cg05573844 | (normal vs normal) |
| 7-1650459 | cg03489907 | (normal vs normal) | 12-54393971 | cg12071536 | (normal vs normal) |
| 7-2148642 | cg16329842 | (normal vs normal) (cancer vs cancer) (cancer+normal vs cancer+normal) | 12-54474036 | cg1764709 1 | (normal vs normal) |
| 7-6198703 | cg21828663 | (normal vs normal) | 12-9227209 | cg27166707 | (normal vs normal) |
| 8-131011240 | cg24525461 | (normal vs normal) (cancer+normal vs cancer+normal) | 13-19299584 | cg11597969 | (normal vs normal) |
| 8-9260354 | cg10359460 | (normal vs normal) (cancer vs cancer) (cancer vs cancer) (cancer+normal vs cancer+normal) | 13-23423308 | cg09677938 | (normal vs normal) |
| 8-9952926 | cg02192555 | (normal vs normal) | 13-39612920 | cg09179485 | (normal vs normal) |
| 8-9953022 | cg11824826 | (normal vs normal) | 13-50705383 | cg00476054 | (normal vs normal) |
| 9-138774526 | cg17792192 | (normal vs normal) | 13-65533500 | cg21106946 | (normal vs normal) |
| 9-99264127 | cg14155501 | (normal vs normal) (cancer vs cancer) (cancer vs cancer) (cancer+normal vs cancer+normal) | 14-106113192 | cg06892141 | (normal vs normal) |
| **GECaG (cancer vs cancer)** | | | 14-62523700 | cg23641964 | (normal vs normal) |
| **Status - Hyper** | | | 14-72399846 | cg15448616 | (normal vs normal) |
| 1-115880865 | cg09008705 | (cancer vs cancer) | 14-81879375 | cg02996355 | (normal vs normal) |
| 1-234350051 | cg00662647 | (cancer vs cancer) | 15-25200490 | cg26875073 | (normal vs normal) |
| 1-34629559 | cg26150071 | (cancer vs cancer) | 15-66795737 | cg12660445 | (normal vs normal) |
| 1-35395821 | cg05046525 | (cancer vs cancer) | 15-99791282 | cg08096291 | (normal vs normal) (cancer+normal vs cancer+normal) |
| 1-49242513 | cg11666087 | (cancer vs cancer) | 16-18995157 | cg06773604 | (normal vs normal) |
| 1-49242519 | cg16573178 | (cancer vs cancer) | 16-30429837 | cg03991297 | (normal vs normal) |
| 10-82117089 | cg03701427 | (cancer vs cancer) | 16-31085579 | cg00221525 | (normal vs normal) |
| 10-88126853 | cg01229798 | (cancer vs cancer) | 16-32857007 | cg06944472 | (normal vs normal) |
| 11-110583377 | cg26578621 | (cancer vs cancer) | 16-33935427 | cg26469379 | (normal vs normal) |
| 11-128564106 | cg06072021 | (cancer vs cancer) | 16-3493440 | cg03896694 | (normal vs normal) |
| 11-128564180 | cg17872757 | (cancer vs cancer) | 16-46461571 | cg07494914 | (normal vs normal) |
| 11-134146132 | cg03323292 | (cancer vs cancer) | 16-88729968 | cg10330885 | (normal vs normal) (cancer+normal vs cancer+normal) |

| | | | | | |
|---|---|---|---|---|---|
| 11-30607068 | cg11855526 | (cancer vs cancer) | 17-16593601 | cg03049249 | (normal vs normal) |
| 11-8284312 | cg26121782 | (cancer vs cancer) | 17-6347142 | cg25288085 | (normal vs normal) (cancer+normal vs cancer+normal) |
| 11-8289974 | cg12740527 | (cancer vs cancer) | 18-40695592 | cg21320334 | (normal vs normal) |
| 12-15475690 | cg09736112 | (cancer vs cancer) | 19-30363203 | cg08587775 | (normal vs normal) |
| 12-24715478 | cg08972130 | (cancer vs cancer) | 19-3360732 | cg07165811 | (normal vs normal) |
| 12-8548978 | cg06797925 | (cancer vs cancer) | 19-54041308 | cg15607708 | (normal vs normal) |
| 13-95620306 | cg25264081 | (cancer vs cancer) (cancer vs cancer) | 2-106192732 | cg03581262 | (normal vs normal) |
| 14-100438647 | cg15698066 | (cancer vs cancer) | 2-132182417 | cg11003984 | (normal vs normal) |
| 14-100438737 | cg21164440 | (cancer vs cancer) | 2-162364264 | cg24695950 | (normal vs normal) (cancer+normal vs cancer+normal) |
| 14-70041283 | cg05218346 | (cancer vs cancer) | 2-177065985 | cg08814736 | (normal vs normal) |
| 15-28351619 | cg09155997 | (cancer vs cancer) | 2-190788356 | cg22157255 | (normal vs normal) |
| 15-28351906 | cg04803843 | (cancer vs cancer) | 2-27665139 | cg12000995 | (normal vs normal) |
| 15-28352098 | cg03061682 | (cancer vs cancer) | 2-88316847 | cg07669260 | (normal vs normal) |
| 15-93631754 | cg25968835 | (cancer vs cancer) | 20-34680964 | cg02672530 | (normal vs normal) |
| 16-10276799 | cg16368442 | (cancer vs cancer) | 20-57426743 | cg04677683 | (normal vs normal) |
| 16-55690130 | cg09746736 | (cancer vs cancer) | 20-57426749 | cg15160445 | (normal vs normal) |
| 19-34973341 | cg15409931 | (cancer vs cancer) | 20-57427472 | cg07105596 | (normal vs normal) |
| 19-34973644 | cg04497176 | (cancer vs cancer) | 21-40721130 | cg24641125 | (normal vs normal) (cancer+normal vs cancer+normal) |
| 2-144695257 | cg09667303 | (cancer vs cancer) (cancer vs cancer) | 22-31556314 | cg01253545 | (normal vs normal) (cancer+normal vs cancer+normal) |
| 2-164593221 | cg20593611 | (cancer vs cancer) | 22-45148218 | cg27321750 | (normal vs normal) |
| 2-29338432 | cg08808128 | (cancer vs cancer) | 22-45148223 | cg06516947 | (normal vs normal) |
| 2-29339076 | cg21972382 | (cancer vs cancer) (cancer vs cancer) (cancer+normal vs cancer+normal) | 3-113822317 | cg22505778 | (normal vs normal) |
| 2-29339091 | cg05038216 | (cancer vs cancer) (cancer vs cancer) (cancer+normal vs cancer+normal) | 3-178865678 | cg00658016 | (normal vs normal) |
| 2-31457043 | cg25840208 | (cancer vs cancer) | 3-25728371 | cg27572068 | (normal vs normal) |
| 2-56150925 | cg08130988 | (cancer vs cancer) | 3-55523099 | cg06678678 | (normal vs normal) |
| 2-66653254 | cg10089527 | (cancer vs cancer) | 4-135865584 | cg01789846 | (normal vs normal) |
| 2- | cg24171907 | (cancer vs cancer) | 4- | cg2359624 | (normal vs normal) |

| | | | | | |
|---|---|---|---|---|---|
| 68546579 | | | 165109834 | 9 | |
| 2-7058390 | cg05648183 | (cancer vs cancer) | 4-165118938 | cg24132580 | (normal vs normal) |
| 2-7058432 | cg04174211 | (cancer vs cancer) | 4-174438312 | cg19403541 | (normal vs normal) |
| 20-23030250 | cg09279949 | (cancer vs cancer) (cancer vs cancer) | 4-43901032 | cg16838729 | (normal vs normal) (cancer+normal vs cancer+normal) |
| 20-37434950 | cg25932164 | (cancer vs cancer) | 4-77069770 | cg26767154 | (normal vs normal) |
| 20-61051032 | cg20265733 | (cancer vs cancer) (normal vs normal) | 5-115387951 | cg23311108 | (cancer vs cancer) (normal vs normal) (cancer+normal vs cancer+normal) |
| 20-61051039 | cg14980983 | (cancer vs cancer) (normal vs normal) | 5-122430153 | cg27087956 | (normal vs normal) |
| 21-27011788 | cg02485200 | (cancer vs cancer) | 5-134259545 | cg12949141 | (normal vs normal) |
| 21-28217060 | cg24262066 | (cancer vs cancer) | 5-168728586 | cg10165167 | (normal vs normal) |
| 21-28217676 | cg00472814 | (cancer vs cancer) | 5-172199460 | cg09799633 | (normal vs normal) |
| 3-120169783 | cg03424342 | (cancer vs cancer) | 5-180700651 | cg11330843 | (normal vs normal) (cancer+normal vs cancer+normal) |
| 3-120169821 | cg15109851 | (cancer vs cancer) | 6-147124881 | cg13876481 | (normal vs normal) |
| 3-128720433 | cg20506715 | (cancer vs cancer) | 6-168227261 | cg00407457 | (normal vs normal) |
| 3-238392 | cg01295518 | (cancer vs cancer) | 6-27511112 | cg26105725 | (normal vs normal) |
| 3-44596770 | cg25805709 | (cancer vs cancer) | 6-31461853 | cg25212457 | (normal vs normal) (cancer+normal vs cancer+normal) |
| 4-126237371 | cg05118638 | (cancer vs cancer) | 6-31697988 | cg09684381 | (normal vs normal) |
| 4-126237373 | cg10731073 | (cancer vs cancer) | 6-32765117 | cg23314071 | (normal vs normal) |
| 4-142054254 | cg18120376 | (cancer vs cancer) | 6-33043138 | cg08405587 | (normal vs normal) |
| 4-142054329 | cg06340552 | (cancer vs cancer) | 6-34113908 | cg18735956 | (normal vs normal) |
| 4-142054446 | cg20599022 | (cancer vs cancer) | 7-1409216 | cg22646311 | (normal vs normal) |
| 4-154680909 | cg04478179 | (cancer vs cancer) | 7-26416987 | cg24755163 | (normal vs normal) (cancer+normal vs cancer+normal) |
| 4-156680400 | cg27272220 | (cancer vs cancer) | 7-42834498 | cg08568561 | (normal vs normal) (cancer+normal vs cancer+normal) |
| 4-156680518 | cg22664296 | (cancer vs cancer) | 7-63924407 | cg17282584 | (normal vs normal) |
| 4-156680769 | cg06217494 | (cancer vs cancer) | 7-94284396 | cg03209103 | (normal vs normal) |
| 4-156681047 | cg25185881 | (cancer vs cancer) | 7-96642037 | cg22281697 | (normal vs normal) |
| 4-157997360 | cg22065614 | (cancer vs cancer) | 8-125313885 | cg15739904 | (normal vs normal) |
| 4-157997367 | cg16269431 | (cancer vs cancer) | 8-216578 | cg17920646 | (normal vs normal) |
| 4- | cg08712866 | (cancer vs cancer) | 8-54625046 | cg1986470 | (normal vs normal) |

| | | | | | |
|---|---|---|---|---|---|
| 157997526 | | | | 5 | |
| 4-157997554 | cg11163620 | (cancer vs cancer) | 8-6875661 | cg22909083 | (normal vs normal) |
| 4-166794786 | cg24521633 | (cancer vs cancer) | 8-93156639 | cg09373786 | (normal vs normal) (cancer+normal vs cancer+normal) |
| 4-166794971 | cg19898128 | (cancer vs cancer) | **Status - Hypo** | | |
| 4-55991782 | cg07893544 | (cancer vs cancer) | 1-111832109 | cg08129490 | (normal vs normal) |
| 4-55991818 | cg21161891 | (cancer vs cancer) | 1-151040405 | cg03258749 | (normal vs normal) |
| 4-55991850 | cg11505338 | (cancer vs cancer) | 1-162630153 | cg19264028 | (normal vs normal) (cancer+normal vs cancer+normal) |
| 4-55991860 | cg00989765 | (cancer vs cancer) (cancer vs cancer) | 1-19202951 | cg11406134 | (normal vs normal) |
| 4-81187798 | cg02002231 | (cancer vs cancer) | 1-197124878 | cg16109012 | (normal vs normal) |
| 5-127874478 | cg01939274 | (cancer vs cancer) | 1-2040968 | cg02138098 | (normal vs normal) |
| 6-11044541 | cg22143569 | (cancer vs cancer) | 1-248437212 | cg01631408 | (normal vs normal) |
| 6-127440000 | cg19365062 | (cancer vs cancer) | 1-39347776 | cg10234007 | (normal vs normal) |
| 6-127440510 | cg01043019 | (cancer vs cancer) | 1-76537463 | cg17812253 | (normal vs normal) |
| 6-151562026 | cg01555431 | (cancer vs cancer) | 1-86011409 | cg06033321 | (normal vs normal) |
| 6-152957999 | cg23931421 | (cancer vs cancer) | 1-94006866 | cg04395462 | (normal vs normal) |
| 6-161352113 | cg11492102 | (cancer vs cancer) | 10-133960386 | cg14760884 | (normal vs normal) |
| 6-163836718 | cg24583770 | (cancer vs cancer) | 10-30982443 | cg02075434 | (normal vs normal) |
| 7-103630549 | cg03203223 | (cancer vs cancer) | 10-72578346 | cg02908912 | (normal vs normal) |
| 7-151107257 | cg17362861 | (cancer vs cancer) | 10-96991505 | cg09036531 | (normal vs normal) |
| 7-28449474 | cg01534145 | (cancer vs cancer) | 11-105448797 | cg22994018 | (normal vs normal) |
| 7-28449847 | cg11831043 | (cancer vs cancer) | 11-130099296 | cg06990529 | (normal vs normal) |
| 8-15397729 | cg18145877 | (cancer vs cancer) | 11-18617206 | cg15824435 | (normal vs normal) |
| 8-32405792 | cg25230074 | (cancer vs cancer) | 11-19395666 | cg21848671 | (normal vs normal) |
| 8-32406900 | cg08032135 | (cancer vs cancer) | 11-3822594 | cg16095660 | (normal vs normal) |
| 8-32406928 | cg06926782 | (cancer vs cancer) | 11-9482594 | cg14037413 | (normal vs normal) |
| 8-494721 | cg03294066 | (cancer vs cancer) | 12-14420864 | cg17061004 | (normal vs normal) |
| 8-54164310 | cg09999109 | (cancer vs cancer) | 12-44949198 | cg12378939 | (normal vs normal) |
| 8-97505764 | cg13096260 | (cancer vs cancer) | 12-55613318 | cg26442740 | (normal vs normal) |
| 8-97506251 | cg16935295 | (cancer vs cancer) | 12-6858549 | cg10617763 | (normal vs normal) |
| 8-97506675 | cg04261408 | (cancer vs cancer) | 12-88428011 | cg02981078 | (normal vs normal) |

| | | | | | |
|---|---|---|---|---|---|
| 9-112403301 | cg02560967 | (cancer vs cancer) | 12-95509731 | cg25036456 | (normal vs normal) (cancer vs cancer) (cancer+normal vs cancer+normal) |
| 9-133536345 | cg14236735 | (cancer vs cancer) | 12-97346526 | cg14315341 | (normal vs normal) |
| 9-6645468 | cg11747771 | (cancer vs cancer) | 13-21010146 | cg03244494 | (normal vs normal) |
| 9-707387 | cg13877502 | (cancer vs cancer) (cancer vs cancer) (normal vs normal) (cancer+normal vs cancer+normal) | 13-90015774 | cg00622516 | (normal vs normal) |
| 9-77112896 | cg14170313 | (cancer vs cancer) | 14-101455260 | cg05187247 | (normal vs normal) |
| **Status - Hypo** | | | 14-38455798 | cg11122170 | (normal vs normal) |
| 1-1062793 | cg03520879 | (cancer vs cancer) | 14-53109146 | cg13827458 | (normal vs normal) |
| 1-1095607 | cg02971920 | (normal vs normal) (cancer vs cancer) (cancer vs cancer) (cancer+normal vs cancer+normal) | 14-69254626 | cg19586027 | (normal vs normal) |
| 1-1095737 | cg06767191 | (cancer vs cancer) | 16-87251839 | cg22510362 | (normal vs normal) |
| 1-17898874 | cg24600987 | (normal vs normal) (cancer vs cancer) (cancer vs cancer) (cancer+normal vs cancer+normal) | 17-18965556 | cg01534423 | (normal vs normal) |
| 1-23038925 | cg00831127 | (cancer vs cancer) | 17-5990470 | cg09045360 | (normal vs normal) |
| 1-23763081 | cg24580199 | (cancer vs cancer) | 18-78001859 | cg14906638 | (normal vs normal) |
| 1-23763279 | cg27447006 | (cancer vs cancer) | 19-57679590 | cg02775223 | (normal vs normal) |
| 1-54723213 | cg22537280 | (cancer vs cancer) (cancer vs cancer) (cancer+normal vs cancer+normal) | 2-100939477 | cg21926883 | (normal vs normal) |
| 1-68283821 | cg16693012 | (cancer vs cancer) | 2-113953981 | cg27425262 | (normal vs normal) |
| 10-114798107 | cg18464364 | (cancer vs cancer) | 2-114866027 | cg17145559 | (normal vs normal) |
| 10-518370 | cg26718707 | (cancer vs cancer) (cancer vs cancer) (cancer+normal vs cancer+normal) | 2-238006557 | cg10809094 | (normal vs normal) |
| 10-73849626 | cg01861509 | (cancer vs cancer) | 2-240039617 | cg14550559 | (normal vs normal) |
| 10-81154181 | cg19265913 | (cancer vs cancer) | 2-27630550 | cg01611223 | (normal vs normal) |
| 10-81154192 | cg02953007 | (cancer vs cancer) | 2-63268631 | cg19446589 | (normal vs normal) |
| 11-126083901 | cg23240479 | (cancer vs cancer) | 2-63606136 | cg24726965 | (normal vs normal) |
| 11-130059549 | cg25892168 | (normal vs normal) (cancer vs cancer) | 21-34601762 | cg17977197 | (normal vs normal) |
| 11-130060459 | cg11069338 | (normal vs normal) (cancer vs cancer) | 22-38449367 | cg19171272 | (normal vs normal) |

| Position | cg | Comparison | Position | cg | Comparison |
|---|---|---|---|---|---|
| | | (cancer vs cancer) (cancer+normal vs cancer+normal) | | | |
| 11-2287734 | cg14907310 | (cancer vs cancer) (cancer vs cancer) (cancer+normal vs cancer+normal) | 3-100970896 | cg27318481 | (normal vs normal) |
| 11-67764320 | cg24974729 | (cancer vs cancer) | 3-141329863 | cg00789793 | (normal vs normal) |
| 11-9142260 | cg27270565 | (cancer vs cancer) | 3-142790398 | cg01520402 | (normal vs normal) |
| 11-94275751 | cg24256772 | (cancer vs cancer) (cancer vs cancer) | 3-151985433 | cg14423778 | (normal vs normal) |
| 12-122515332 | cg06610259 | (cancer vs cancer) | 3-158990856 | cg17374958 | (normal vs normal) |
| 12-14849268 | cg18754342 | (normal vs normal) (cancer vs cancer) (cancer vs cancer) | 3-4856199 | cg08998950 | (normal vs normal) |
| 12-33048259 | cg19677302 | (cancer vs cancer) | 3-7340148 | cg19930620 | (normal vs normal) |
| 12-96052514 | cg19013339 | (cancer vs cancer) | 3-73434814 | cg25688523 | (normal vs normal) |
| 13-44953908 | cg01154353 | (cancer vs cancer) | 4-147393712 | cg11313951 | (normal vs normal) |
| 16-1808230 | cg01149677 | (normal vs normal) (cancer vs cancer) | 4-152424074 | cg17479576 | (normal vs normal) |
| 16-29300673 | cg27133583 | (cancer vs cancer) | 4-175239475 | cg06748934 | (normal vs normal) |
| 16-89169289 | cg10398761 | (normal vs normal) (cancer vs cancer) (cancer vs cancer) (cancer+normal vs cancer+normal) | 4-2402046 | cg25726224 | (normal vs normal) |
| 17-1412449 | cg23346781 | (cancer vs cancer) (cancer vs cancer) (cancer+normal vs cancer+normal) | 4-89084266 | cg17756398 | (normal vs normal) |
| 17-4042717 | cg11542336 | (cancer vs cancer) (cancer vs cancer) (cancer+normal vs cancer+normal) | 5-102465432 | cg05770626 | (normal vs normal) |
| 17-48845009 | cg25518968 | (cancer vs cancer) | 5-115696151 | cg01419670 | (normal vs normal) |
| 17-48845052 | cg12235260 | (cancer vs cancer) | 5-118176275 | cg13927247 | (normal vs normal) |
| 17-48845064 | cg07443932 | (cancer vs cancer) | 5-13664584 | cg27586797 | (normal vs normal) |
| 17-70256462 | cg06909248 | (normal vs normal) (cancer vs cancer) (cancer vs cancer) (cancer+normal vs cancer+normal) | 5-173386190 | cg18533392 | (normal vs normal) |
| 17-7163136 | cg21762727 | (cancer vs cancer) | 5-43045555 | cg17427675 | (normal vs normal) |
| 17-80535398 | cg16366685 | (normal vs normal) (cancer vs cancer) (cancer vs cancer) (cancer+normal vs cancer+normal) | 6-101120796 | cg06539434 | (normal vs normal) |
| 17-80535428 | cg03454705 | (normal vs normal) (cancer vs cancer) | 6-104920530 | cg07484191 | (normal vs normal) |

| | | | | | |
|---|---|---|---|---|---|
| | | (cancer vs cancer) (cancer+normal vs cancer+normal) | | | |
| 19-39306244 | cg12028674 | (normal vs normal) (cancer vs cancer) (cancer vs cancer) (cancer+normal vs cancer+normal) | 6-110057745 | cg16441347 | (normal vs normal) |
| 19-44128109 | cg23263641 | (cancer vs cancer) | 6-112668163 | cg17428185 | (normal vs normal) |
| 19-46807660 | cg07684068 | (cancer vs cancer) | 6-117201841 | cg06022698 | (normal vs normal) |
| 2-106959257 | cg24419520 | (normal vs normal) (cancer vs cancer) (cancer vs cancer) (cancer+normal vs cancer+normal) | 6-150245071 | cg01145232 | (normal vs normal) |
| 2-112808038 | cg25890678 | (cancer vs cancer) (cancer vs cancer) | 6-170487311 | cg02546416 | (normal vs normal) |
| 2-164594200 | cg08280341 | (cancer vs cancer) | 6-31478822 | cg09320595 | (normal vs normal) |
| 2-17830624 | cg12793924 | (normal vs normal) (cancer vs cancer) (cancer vs cancer) (cancer+normal vs cancer+normal) | 6-31929046 | cg18035435 | (normal vs normal) |
| 2-210517441 | cg01218350 | (cancer vs cancer) | 6-32712093 | cg00334532 | (normal vs normal) |
| 2-227312417 | cg03426602 | (cancer vs cancer) (cancer vs cancer) (cancer+normal vs cancer+normal) | 6-39692382 | cg01090026 | (normal vs normal) |
| 2-241543460 | cg25599161 | (cancer vs cancer) | 6-9139720 | cg06134678 | (normal vs normal) |
| 2-85920000 | cg13603214 | (cancer vs cancer) | 7-123181238 | cg06294630 | (normal vs normal) |
| 2-8850020 | cg00240378 | (normal vs normal) (cancer vs cancer) (cancer vs cancer) (cancer+normal vs cancer+normal) | 7-12410026 | cg23238719 | (normal vs normal) |
| 2-97427895 | cg12942038 | (normal vs normal) (cancer vs cancer) | 7-133137334 | cg02749073 | (normal vs normal) |
| 2-97427975 | cg11464842 | (normal vs normal) (cancer vs cancer) (cancer vs cancer) (cancer+normal vs cancer+normal) | 7-157526947 | cg11757124 | (normal vs normal) |
| 20-35504064 | cg19448292 | (cancer vs cancer) | 7-43678966 | cg09949666 | (normal vs normal) |
| 20-43926551 | cg01268752 | (cancer vs cancer) | 7-50529359 | cg14593290 | (normal vs normal) |
| 20-55959405 | cg25225756 | (normal vs normal) (cancer vs cancer) (cancer vs cancer) (cancer+normal vs cancer+normal) | 7-56434951 | cg00933263 | (normal vs normal) |
| 20-55959549 | cg22618720 | (cancer vs cancer) (cancer vs cancer) (cancer+normal vs cancer+normal) | 7-57072233 | cg12405121 | (normal vs normal) |

| | | | | | |
|---|---|---|---|---|---|
| 20-57616191 | cg20726575 | (normal vs normal) (cancer vs cancer) | 7-57076741 | cg07352967 | (normal vs normal) |
| 22-31062286 | cg01274916 | (cancer vs cancer) (cancer vs cancer) (cancer+normal vs cancer+normal) | 7-63494639 | cg02254255 | (normal vs normal) |
| 3-124652790 | cg09081544 | (cancer vs cancer) | 7-6501749 | cg07144666 | (normal vs normal) |
| 3-139195319 | cg17778120 | (cancer vs cancer) | 7-65447489 | cg11367038 | (normal vs normal) |
| 3-150238257 | cg02504622 | (cancer vs cancer) (cancer vs cancer) (cancer+normal vs cancer+normal) | 7-6614945 | cg00014786 | (normal vs normal) |
| 3-171412917 | cg09433558 | (cancer vs cancer) (cancer vs cancer) (cancer+normal vs cancer+normal) | 7-69149951 | cg10619644 | (normal vs normal) |
| 3-30730226 | cg03420580 | (cancer vs cancer) | 7-83860330 | cg02106155 | (normal vs normal) |
| 3-58653593 | cg02658330 | (cancer vs cancer) | 8-131349729 | cg16277922 | (normal vs normal) |
| 3-72435597 | cg03173942 | (cancer vs cancer) | 8-142182166 | cg11716073 | (normal vs normal) |
| 4-1016081 | cg27309282 | (cancer vs cancer) | 8-71267300 | cg12724384 | (normal vs normal) |
| 4-187126114 | cg23442198 | (normal vs normal) (cancer vs cancer) (cancer vs cancer) (cancer+normal vs cancer+normal) | 9-137736684 | cg13910822 | (normal vs normal) |
| 4-187629328 | cg21399040 | (normal vs normal) (cancer vs cancer) (cancer vs cancer) (cancer+normal vs cancer+normal) | 9-140143642 | cg04476286 | (normal vs normal) |
| 4-187629336 | cg24820270 | (normal vs normal) (cancer vs cancer) (cancer vs cancer) (cancer+normal vs cancer+normal) | **STAD (cancer vs cancer)** | | |
| 4-187629387 | cg25352342 | (normal vs normal) (cancer vs cancer) (cancer vs cancer) (cancer+normal vs cancer+normal) | **Status - Hyper** | | |
| 4-41270335 | cg02359506 | (cancer vs cancer) | 1-220702225 | cg07420190 | (cancer vs cancer) |
| 4-6945702 | cg05069807 | (cancer vs cancer) (cancer vs cancer) (cancer+normal vs cancer+normal) | 1-244013006 | cg19712965 | (cancer vs cancer) |
| 4-6945745 | cg16574807 | (normal vs normal) (cancer vs cancer) (cancer vs cancer) (cancer+normal vs cancer+normal) | 1-29138936 | cg08366446 | (cancer vs cancer) (cancer+normal vs cancer+normal) |
| 5-10759383 | cg00143606 | (cancer vs cancer) | 1-57111117 | cg26068551 | (cancer vs cancer) (normal vs normal) (cancer+normal vs cancer+normal) |

301

| | | | | | |
|---|---|---|---|---|---|
| 5-149546073 | cg26531174 | (cancer vs cancer) (cancer vs cancer) (cancer+normal vs cancer+normal) | 1-57111122 | cg09686317 | (cancer vs cancer) (cancer+normal vs cancer+normal) |
| 5-149547566 | cg09690765 | (cancer vs cancer) (cancer vs cancer) (cancer+normal vs cancer+normal) | 10-20104497 | cg24022375 | (cancer vs cancer) (cancer vs cancer) (cancer+normal vs cancer+normal) |
| 5-179393917 | cg26722544 | (cancer vs cancer) (cancer vs cancer) (cancer+normal vs cancer+normal) | 10-20104806 | cg16747247 | (cancer vs cancer) (cancer+normal vs cancer+normal) |
| 6-126503271 | cg10846969 | (cancer vs cancer) | 10-20104948 | cg08625590 | (cancer vs cancer) (cancer vs cancer) (cancer+normal vs cancer+normal) |
| 6-151671694 | cg10134496 | (cancer vs cancer) | 10-20105641 | cg12563178 | (cancer vs cancer) (cancer+normal vs cancer+normal) |
| 6-155649609 | cg24212240 | (cancer vs cancer) | 10-28032282 | cg16397794 | (cancer vs cancer) (cancer+normal vs cancer+normal) |
| 6-160511937 | cg07214314 | (cancer vs cancer) | 10-90342899 | cg19235339 | (cancer vs cancer) (cancer+normal vs cancer+normal) |
| 6-160512064 | cg02079963 | (cancer vs cancer) | 10-90343204 | cg18192294 | (cancer vs cancer) |
| 6-161561121 | cg11342941 | (cancer vs cancer) | 12-20521463 | cg17535647 | (cancer vs cancer) (cancer+normal vs cancer+normal) |
| 6-30079662 | cg24880997 | (cancer vs cancer) | 12-48398370 | cg20255775 | (cancer vs cancer) (cancer vs cancer) |
| 6-31123044 | cg01086672 | (normal vs normal) (cancer vs cancer) (cancer vs cancer) (cancer+normal vs cancer+normal) | 12-48398374 | cg17054969 | (cancer vs cancer) |
| 6-36304456 | cg13319417 | (cancer vs cancer) | 12-95267307 | cg21533806 | (cancer vs cancer) |
| 6-36304794 | cg10550074 | (cancer vs cancer) | 13-110961069 | cg24909466 | (cancer vs cancer) (cancer+normal vs cancer+normal) |
| 6-36305071 | cg04434994 | (cancer vs cancer) | 13-28674720 | cg14660839 | (cancer vs cancer) (cancer+normal vs cancer+normal) |
| 7-102792783 | cg14504536 | (cancer vs cancer) | 13-28674876 | cg05598562 | (cancer vs cancer) |
| 7-150069026 | cg17677524 | (normal vs normal) (cancer vs cancer) (cancer vs cancer) (cancer+normal vs cancer+normal) | 14-23822017 | cg01557297 | (cancer vs cancer) (cancer+normal vs cancer+normal) |
| 7-23749667 | cg05407490 | (cancer vs cancer) | 14-96505874 | cg04184208 | (cancer vs cancer) (cancer+normal vs cancer+normal) |
| 7-23749740 | cg05000488 | (cancer vs cancer) | 15-23208349 | cg24583766 | (cancer vs cancer) |
| 7-50133131 | cg09921682 | (cancer vs cancer) (cancer vs cancer) | 15-28983117 | cg11794877 | (cancer vs cancer) |
| 7-73408058 | cg16233515 | (normal vs normal) (cancer vs cancer) | 15-28983120 | cg26825751 | (cancer vs cancer) |

EP 3 692 164 B1

| | | | | | |
|---|---|---|---|---|---|
| | | (cancer vs cancer)<br>(cancer+normal vs cancer+normal) | | | |
| 7-73408060 | cg07243141 | (normal vs normal)<br>(cancer vs cancer)<br>(cancer vs cancer)<br>(cancer+normal vs cancer+normal) | 15-28983122 | cg07124017 | (cancer vs cancer) |
| 7-73408101 | cg18780627 | (normal vs normal)<br>(cancer vs cancer)<br>(cancer vs cancer)<br>(cancer+normal vs cancer+normal) | 15-93632619 | cg20899253 | (cancer vs cancer)<br>(cancer+normal vs cancer+normal) |
| 7-90670577 | cg20411384 | (cancer vs cancer) | 17-43972969 | cg02804087 | (cancer vs cancer) |
| 8-61789727 | cg23625106 | (normal vs normal)<br>(cancer vs cancer) | 17-43972978 | cg18368019 | (cancer vs cancer)<br>(cancer vs cancer) |
| 9-97369302 | cg14434198 | (cancer vs cancer) | 17-45810865 | cg09775582 | (cancer vs cancer) |
| 9-97369791 | cg13494954 | (cancer vs cancer) | 17-59476790 | cg26932779 | (cancer vs cancer)<br>(cancer+normal vs cancer+normal) |
| **KICH (cancer vs cancer)** | | | 17-59477557 | cg19949441 | (cancer vs cancer)<br>(cancer+normal vs cancer+normal) |
| **Status - Hyper** | | | 17-59477564 | cg09365557 | (cancer vs cancer)<br>(cancer+normal vs cancer+normal) |
| 1-152538426 | cg07476201 | (cancer vs cancer)<br>(cancer+normal vs cancer+normal) | 18-57364587 | cg02774186 | (cancer vs cancer)<br>(cancer+normal vs cancer+normal) |
| 1-152538857 | cg24000437 | (cancer vs cancer)<br>(cancer vs cancer)<br>(cancer+normal vs cancer+normal) | 19-31841555 | cg15208375 | (cancer vs cancer)<br>(cancer vs cancer) |
| 1-166139996 | cg11979420 | (cancer vs cancer)<br>(cancer+normal vs cancer+normal) | 19-31841663 | cg11199770 | (cancer vs cancer)<br>(cancer vs cancer)<br>(normal vs normal)<br>(cancer+normal vs cancer+normal) |
| 1-17337346 | cg22045253 | (cancer vs cancer)<br>(cancer+normal vs cancer+normal) | 19-31841952 | cg15711268 | (cancer vs cancer)<br>(cancer vs cancer)<br>(cancer+normal vs cancer+normal) |
| 1-184357556 | cg10061342 | (cancer vs cancer)<br>(cancer vs cancer)<br>(cancer+normal vs cancer+normal) | 19-37957995 | cg14142713 | (cancer vs cancer) |
| 1-186504948 | cg05398036 | (cancer vs cancer)<br>(cancer+normal vs cancer+normal) | 2-109745812 | cg12518410 | (cancer vs cancer)<br>(cancer+normal vs cancer+normal) |
| 1-19109544 | cg03315346 | (cancer vs cancer)<br>(cancer+normal vs cancer+normal) | 2-109745828 | cg09392940 | (cancer vs cancer)<br>(cancer+normal vs cancer+normal) |
| 1-222816858 | cg00089945 | (cancer vs cancer)<br>(cancer+normal vs cancer+normal) | 2-180725907 | cg22657780 | (cancer vs cancer)<br>(cancer+normal vs cancer+normal) |
| 1-226112534 | cg06939851 | (cancer vs cancer)<br>(cancer+normal vs cancer+normal) | 2-180726035 | cg24575676 | (cancer vs cancer)<br>(cancer+normal vs cancer+normal) |
| 1- | cg09119494 | (cancer vs cancer) | 2- | cg1111376 | (cancer vs cancer) |

303

| | | | | | |
|---|---|---|---|---|---|
| 23851471 | | (cancer+normal vs cancer+normal) | 180726247 | 0 | (cancer+normal vs cancer+normal) |
| 1-39491459 | cg07388493 | (cancer vs cancer) (cancer+normal vs cancer+normal) | 2-180726249 | cg2627189 1 | (cancer vs cancer) (cancer+normal vs cancer+normal) |
| 1-40887988 | cg26042300 | (cancer vs cancer) (cancer vs cancer) (cancer+normal vs cancer+normal) | 2-180726252 | cg0927924 0 | (cancer vs cancer) (cancer+normal vs cancer+normal) |
| 1-46859774 | cg12671744 | (cancer vs cancer) (cancer+normal vs cancer+normal) | 2-180726328 | cg0669561 1 | (cancer vs cancer) (cancer+normal vs cancer+normal) |
| 1-90309410 | cg24720717 | (cancer vs cancer) (cancer+normal vs cancer+normal) | 2-197457425 | cg2609498 4 | (cancer vs cancer) |
| 1-9224198 | cg22373770 | (cancer vs cancer) (cancer+normal vs cancer+normal) | 2-197457430 | cg0785375 8 | (cancer vs cancer) (cancer+normal vs cancer+normal) |
| 10-102588959 | cg17480467 | (cancer vs cancer) (cancer vs cancer) (normal vs normal) (cancer+normal vs cancer+normal) | 2-197458046 | cg2461347 9 | (cancer vs cancer) (cancer+normal vs cancer+normal) |
| 10-102589102 | cg14778235 | (cancer vs cancer) (cancer vs cancer) (normal vs normal) (cancer+normal vs cancer+normal) | 2-197458093 | cg0138167 8 | (cancer vs cancer) (cancer+normal vs cancer+normal) |
| 10-102589250 | cg08217716 | (cancer vs cancer) (cancer vs cancer) (normal vs normal) (cancer+normal vs cancer+normal) | 2-197458104 | cg1264469 6 | (cancer vs cancer) (cancer+normal vs cancer+normal) |
| 10-120154512 | cg23160142 | (cancer vs cancer) (cancer+normal vs cancer+normal) | 2-213403929 | cg1632965 0 | (cancer vs cancer) (cancer+normal vs cancer+normal) |
| 10-128360149 | cg06624146 | (cancer vs cancer) (cancer+normal vs cancer+normal) | 2-88751786 | cg2474610 0 | (cancer vs cancer) |
| 10-129350922 | cg19321696 | (cancer vs cancer) (cancer+normal vs cancer+normal) | 20-13200969 | cg0214593 2 | (cancer vs cancer) (cancer+normal vs cancer+normal) |
| 10-129706313 | cg16750801 | (cancer vs cancer) (cancer+normal vs cancer+normal) | 20-13200973 | cg0626243 6 | (cancer vs cancer) (cancer+normal vs cancer+normal) |
| 10-17273187 | cg26063719 | (cancer vs cancer) (normal vs normal) (cancer+normal vs cancer+normal) | 20-13200980 | cg0949199 1 | (cancer vs cancer) (normal vs normal) (cancer+normal vs cancer+normal) |
| 10-31101116 | cg03127349 | (cancer vs cancer) (cancer+normal vs cancer+normal) | 20-13200992 | cg2156197 0 | (cancer vs cancer) (cancer+normal vs cancer+normal) |
| 10-34320548 | cg10772185 | (cancer vs cancer) (cancer+normal vs cancer+normal) | 20-13201456 | cg1899391 8 | (cancer vs cancer) (cancer+normal vs cancer+normal) |
| 10-375391 | cg06193766 | (cancer vs cancer) (cancer vs cancer) (cancer+normal vs cancer+normal) | 22-24551820 | cg0864991 9 | (cancer vs cancer) |
| 10-375830 | cg08410533 | (cancer vs cancer) (cancer+normal vs | 22-24552132 | cg0270611 0 | (cancer vs cancer) |

| | | cancer+normal) | | | |
|---|---|---|---|---|---|
| 10-99822472 | cg07232642 | (cancer vs cancer) (cancer+normal vs cancer+normal) | 22-24552379 | cg0561177 9 | (cancer vs cancer) |
| 11-59950525 | cg00673646 | (cancer vs cancer) (cancer+normal vs cancer+normal) | 3-139654245 | cg0885365 9 | (cancer vs cancer) |
| 11-7728814 | cg13563074 | (cancer vs cancer) (cancer vs cancer) (cancer+normal vs cancer+normal) | 3-16554910 | cg2262396 7 | (cancer vs cancer) |
| 11-7960899 | cg09140665 | (cancer vs cancer) (cancer+normal vs cancer+normal) | 3-55521351 | cg2261515 8 | (cancer vs cancer) |
| 12-10022682 | cg08752459 | (cancer vs cancer) (cancer vs cancer) (normal vs normal) (cancer+normal vs cancer+normal) | 3-56502265 | cg0806524 1 | (cancer vs cancer) (cancer+normal vs cancer+normal) |
| 12-10022688 | cg12591315 | (cancer vs cancer) (cancer vs cancer) (normal vs normal) (cancer+normal vs cancer+normal) | 3-56502293 | cg0667827 9 | (cancer vs cancer) (cancer+normal vs cancer+normal) |
| 12-105089508 | cg07696842 | (cancer vs cancer) (cancer+normal vs cancer+normal) | 3-87040286 | cg1261513 7 | (cancer vs cancer) (cancer+normal vs cancer+normal) |
| 12-113229319 | cg01573562 | (cancer vs cancer) (cancer+normal vs cancer+normal) | 3-96532043 | cg1109035 2 | (cancer vs cancer) (cancer+normal vs cancer+normal) |
| 12-116997535 | cg03822198 | (cancer vs cancer) (cancer+normal vs cancer+normal) | 4-123748386 | cg2558317 4 | (cancer vs cancer) |
| 12-131198873 | cg27080194 | (cancer vs cancer) (cancer+normal vs cancer+normal) | 4-141295029 | cg2198622 5 | (cancer vs cancer) |
| 12-70215816 | cg23907053 | (cancer vs cancer) (cancer+normal vs cancer+normal) | 4-166300252 | cg1653410 3 | (cancer vs cancer) (cancer+normal vs cancer+normal) |
| 13-46870454 | cg11203397 | (cancer vs cancer) (cancer+normal vs cancer+normal) | 4-166300290 | cg0288628 4 | (cancer vs cancer) (cancer vs cancer) (cancer+normal vs cancer+normal) |
| 15-25330341 | cg08051964 | (cancer vs cancer) (cancer+normal vs cancer+normal) | 4-183370138 | cg0976760 2 | (cancer vs cancer) (normal vs normal) (cancer+normal vs cancer+normal) |
| 15-70767570 | cg16973527 | (cancer vs cancer) (cancer vs cancer) (cancer+normal vs cancer+normal) | 4-20253514 | cg1375579 6 | (cancer vs cancer) (cancer+normal vs cancer+normal) |
| 15-70767649 | cg13427748 | (cancer vs cancer) (cancer vs cancer) (cancer+normal vs cancer+normal) | 4-20253556 | cg1032272 4 | (cancer vs cancer) (cancer+normal vs cancer+normal) |
| 16-2391081 | cg02331561 | (cancer vs cancer) (cancer+normal vs cancer+normal) | 4-20253595 | cg0128177 6 | (cancer vs cancer) (cancer+normal vs cancer+normal) |
| 16-4103492 | cg10177032 | (cancer vs cancer) (cancer+normal vs cancer+normal) | 4-57687839 | cg1319740 6 | (cancer vs cancer) |

305

| | | | | | |
|---|---|---|---|---|---|
| 16-4103533 | cg01352090 | (cancer vs cancer) (cancer+normal vs cancer+normal) | 4-81952024 | cg26917673 | (cancer vs cancer) (cancer vs cancer) |
| 16-474271 | cg09555736 | (cancer vs cancer) (cancer+normal vs cancer+normal) | 5-132948251 | cg00586365 | (cancer vs cancer) |
| 16-474430 | cg02102075 | (cancer vs cancer) (cancer+normal vs cancer+normal) | 5-157002793 | cg14607812 | (cancer vs cancer) |
| 16-84390897 | cg05964640 | (cancer vs cancer) (cancer+normal vs cancer+normal) | 5-157002805 | cg23851860 | (cancer vs cancer) |
| 17-19967600 | cg02656560 | (cancer vs cancer) (cancer+normal vs cancer+normal) | 5-157002817 | cg02725069 | (cancer vs cancer) |
| 17-33473881 | cg10174170 | (cancer vs cancer) (cancer+normal vs cancer+normal) | 5-159343549 | cg07011172 | (cancer vs cancer) (cancer vs cancer) |
| 17-48989061 | cg11235451 | (cancer vs cancer) (cancer+normal vs cancer+normal) | 5-176057427 | cg17996329 | (cancer vs cancer) |
| 17-74100132 | cg08122232 | (cancer vs cancer) (cancer+normal vs cancer+normal) | 5-42423615 | cg08217227 | (cancer vs cancer) (cancer+normal vs cancer+normal) |
| 19-14785849 | cg15746620 | (cancer vs cancer) (cancer vs cancer) (cancer+normal vs cancer+normal) | 5-42423947 | cg12042587 | (cancer vs cancer) (cancer vs cancer) (normal vs normal) (cancer+normal vs cancer+normal) |
| 19-49164383 | cg09341448 | (cancer vs cancer) (cancer+normal vs cancer+normal) | 5-79866099 | cg02466321 | (cancer vs cancer) |
| 19-51018365 | cg15655714 | (cancer vs cancer) (cancer+normal vs cancer+normal) | 6-125284359 | cg06993703 | (cancer vs cancer) |
| 2-121496875 | cg20219891 | (cancer vs cancer) (cancer vs cancer) (cancer+normal vs cancer+normal) | 6-21664652 | cg24428877 | (cancer vs cancer) (cancer vs cancer) (normal vs normal) (cancer+normal vs cancer+normal) |
| 2-138019701 | cg05853130 | (cancer vs cancer) (cancer+normal vs cancer+normal) | 6-88875840 | cg20622089 | (cancer vs cancer) |
| 2-176970064 | cg06354392 | (cancer vs cancer) (cancer+normal vs cancer+normal) | 7-114562847 | cg11256364 | (cancer vs cancer) |
| 2-176970508 | cg18351329 | (cancer vs cancer) (cancer+normal vs cancer+normal) | 7-128828460 | cg13971154 | (cancer vs cancer) |
| 2-236172440 | cg09406107 | (cancer vs cancer) (cancer+normal vs cancer+normal) | 7-128828575 | cg00405843 | (cancer vs cancer) |
| 2-97202260 | cg02085953 | (cancer vs cancer) (cancer+normal vs cancer+normal) | 7-42277375 | cg07191743 | (cancer vs cancer) (cancer+normal vs cancer+normal) |
| 20-57975832 | cg12649683 | (cancer vs cancer) (cancer+normal vs cancer+normal) | 8-133492476 | cg27016990 | (cancer vs cancer) (cancer+normal vs cancer+normal) |
| 22-46934981 | cg11533712 | (cancer vs cancer) (cancer+normal vs cancer+normal) | 8-21646662 | cg03453638 | (cancer vs cancer) (cancer+normal vs cancer+normal) |

| | | | | | | |
|---|---|---|---|---|---|
| 3-143567492 | cg15360181 | (cancer vs cancer) (cancer+normal vs cancer+normal) | 8-38325950 | cg27063138 | (cancer vs cancer) |
| 3-149095573 | cg23246821 | (cancer vs cancer) (cancer+normal vs cancer+normal) | 8-41504078 | cg16174680 | (cancer vs cancer) |
| 3-194030706 | cg07141504 | (cancer vs cancer) (cancer+normal vs cancer+normal) | 8-6692148 | cg17763188 | (cancer vs cancer) (cancer+normal vs cancer+normal) |
| 4-16628713 | cg06818159 | (cancer vs cancer) (cancer+normal vs cancer+normal) | 8-6692470 | cg05234760 | (cancer vs cancer) |
| 4-40057241 | cg01492656 | (cancer vs cancer) (cancer+normal vs cancer+normal) | 8-6693103 | cg12482557 | (cancer vs cancer) (cancer+normal vs cancer+normal) |
| 4-74570525 | cg23099587 | (cancer vs cancer) (cancer+normal vs cancer+normal) | **Status - Hypo** | | |
| 4-77119206 | cg01624414 | (cancer vs cancer) (cancer+normal vs cancer+normal) | 1-111023201 | cg16592897 | (cancer vs cancer) (cancer+normal vs cancer+normal) |
| 5-162516650 | cg18086386 | (cancer vs cancer) (cancer vs cancer) (cancer+normal vs cancer+normal) | 1-119860821 | cg19275091 | (cancer vs cancer) |
| 5-171870875 | cg19979108 | (cancer vs cancer) (cancer+normal vs cancer+normal) | 1-12078821 | cg15770654 | (cancer vs cancer) |
| 5-173852421 | cg18040788 | (cancer vs cancer) (cancer+normal vs cancer+normal) | 1-1228990 | cg02506501 | (cancer vs cancer) |
| 5-55881858 | cg00329411 | (cancer vs cancer) (cancer+normal vs cancer+normal) | 1-15783239 | cg07381141 | (cancer vs cancer) |
| 5-97645526 | cg14972576 | (cancer vs cancer) (cancer+normal vs cancer+normal) | 1-22615900 | cg02053477 | (cancer vs cancer) |
| 6-100650602 | cg01889956 | (cancer vs cancer) (cancer+normal vs cancer+normal) | 1-232692910 | cg14260004 | (cancer vs cancer) |
| 6-106773949 | cg14951955 | (cancer vs cancer) (cancer+normal vs cancer+normal) | 1-43228316 | cg00917471 | (cancer vs cancer) |
| 6-110736772 | cg02872426 | (cancer vs cancer) (cancer+normal vs cancer+normal) | 10-103349097 | cg18834029 | (cancer vs cancer) |
| 6-134216081 | cg07571531 | (cancer vs cancer) (cancer vs cancer) (cancer+normal vs cancer+normal) | 10-12709305 | cg26966582 | (cancer vs cancer) |
| 6-166970727 | cg11387340 | (cancer vs cancer) (cancer+normal vs cancer+normal) | 10-12874395 | cg22813891 | (cancer vs cancer) |
| 6-168134760 | cg01797450 | (cancer vs cancer) (cancer+normal vs cancer+normal) | 10-131631850 | cg23391090 | (cancer vs cancer) |
| 6-28892465 | cg23756442 | (cancer vs cancer) (cancer vs cancer) (cancer+normal vs cancer+normal) | 10-43898033 | cg08682036 | (cancer vs cancer) |
| 6- | cg07053841 | (cancer vs cancer) | 10- | cg1208364 | (cancer vs cancer) |

| | | | | | |
|---|---|---|---|---|---|
| 29012588 | | (cancer+normal vs cancer+normal) | 51549656 | 4 | |
| 6-32172871 | cg18244508 | (cancer vs cancer) (cancer+normal vs cancer+normal) | 10-77142527 | cg15284082 | (cancer vs cancer) |
| 6-33256216 | cg17131579 | (cancer vs cancer) (cancer+normal vs cancer+normal) | 11-115088907 | cg16061656 | (cancer vs cancer) (cancer vs cancer) |
| 6-35309867 | cg21748751 | (cancer vs cancer) (cancer+normal vs cancer+normal) | 11-15692519 | cg11966998 | (cancer vs cancer) (cancer+normal vs cancer+normal) |
| 6-50785325 | cg06653004 | (cancer vs cancer) (cancer+normal vs cancer+normal) | 12-1748816 | cg02935351 | (cancer vs cancer) (cancer+normal vs cancer+normal) |
| 6-50812112 | cg17153727 | (cancer vs cancer) (cancer+normal vs cancer+normal) | 12-59319720 | cg10283871 | (cancer vs cancer) (cancer+normal vs cancer+normal) |
| 6-97010903 | cg03043157 | (cancer vs cancer) (cancer+normal vs cancer+normal) | 13-26761337 | cg11787508 | (cancer vs cancer) |
| 7-27404187 | cg05729490 | (cancer vs cancer) (cancer+normal vs cancer+normal) | 13-99336246 | cg02319149 | (cancer vs cancer) |
| 7-28726216 | cg05083033 | (cancer vs cancer) (cancer+normal vs cancer+normal) | 14-71291951 | cg06937653 | (cancer vs cancer) |
| 7-5277188 | cg10229594 | (cancer vs cancer) (cancer+normal vs cancer+normal) | 15-99408636 | cg19322380 | (cancer vs cancer) (cancer vs cancer) |
| 7-69289866 | cg21557473 | (cancer vs cancer) (cancer+normal vs cancer+normal) | 15-99408804 | cg21746425 | (cancer vs cancer) |
| 7-852714 | cg19239199 | (cancer vs cancer) (cancer+normal vs cancer+normal) | 15-99409411 | cg13812291 | (cancer vs cancer) |
| 7-99517509 | cg25591451 | (cancer vs cancer) (cancer+normal vs cancer+normal) | 16-29913162 | cg09328356 | (cancer vs cancer) (cancer+normal vs cancer+normal) |
| 8-21900314 | cg26069514 | (cancer vs cancer) (cancer+normal vs cancer+normal) | 16-81254010 | cg00908271 | (cancer vs cancer) |
| 8-40602363 | cg02458516 | (cancer vs cancer) (cancer+normal vs cancer+normal) | 17-39024690 | cg12437115 | (cancer vs cancer) |
| 9-110310795 | cg13430755 | (cancer vs cancer) (cancer+normal vs cancer+normal) | 17-45934681 | cg13457515 | (cancer vs cancer) |
| 9-138605049 | cg14341131 | (cancer vs cancer) (cancer vs cancer) (cancer+normal vs cancer+normal) | 17-70384485 | cg03828329 | (cancer vs cancer) |
| 9-91267411 | cg03578548 | (cancer vs cancer) (cancer+normal vs cancer+normal) | 17-79431331 | cg19536407 | (cancer vs cancer) (cancer+normal vs cancer+normal) |
| **Status - Hypo** | | | 19-17405195 | cg13352717 | (cancer vs cancer) |
| 1-223472245 | cg22158547 | (cancer vs cancer) (cancer+normal vs cancer+normal) | 19-42873953 | cg14481689 | (cancer vs cancer) |
| 1-3129633 | cg05265611 | (cancer vs cancer) (cancer+normal vs | 2-102398839 | cg00747372 | (cancer vs cancer) |

| | | | | | |
|---|---|---|---|---|---|
| | | cancer+normal) | | | |
| 10-126314680 | cg04426802 | (cancer vs cancer) (cancer+normal vs cancer+normal) | 2-134004614 | cg03671556 | (cancer vs cancer) |
| 10-77165905 | cg06262069 | (cancer vs cancer) (cancer+normal vs cancer+normal) | 2-206545776 | cg04768970 | (cancer vs cancer) |
| 11-10462924 | cg06814191 | (cancer vs cancer) (cancer+normal vs cancer+normal) | 2-33596547 | cg07119076 | (cancer vs cancer) |
| 11-108365051 | cg04295172 | (cancer vs cancer) (cancer+normal vs cancer+normal) | 2-45234140 | cg04364219 | (cancer vs cancer) |
| 11-456063 | cg16124920 | (cancer vs cancer) (cancer+normal vs cancer+normal) | 2-8596908 | cg26784300 | (cancer vs cancer) (cancer vs cancer) |
| 11-63906084 | cg04725041 | (cancer vs cancer) (cancer+normal vs cancer+normal) | 2-86037857 | cg08183425 | (cancer vs cancer) (cancer+normal vs cancer+normal) |
| 11-66494177 | cg22015277 | (cancer vs cancer) (cancer+normal vs cancer+normal) | 20-30866501 | cg21073212 | (cancer vs cancer) |
| 12-110986864 | cg15698107 | (cancer vs cancer) (cancer+normal vs cancer+normal) | 22-33195343 | cg07641497 | (cancer vs cancer) |
| 12-111883369 | cg13116789 | (cancer vs cancer) (cancer+normal vs cancer+normal) | 3-127394884 | cg01123353 | (cancer vs cancer) |
| 12-124812999 | cg11601197 | (cancer vs cancer) (cancer+normal vs cancer+normal) | 3-127394912 | cg24768999 | (cancer vs cancer) |
| 12-4762206 | cg13719771 | (cancer vs cancer) (cancer+normal vs cancer+normal) | 3-127395387 | cg25929731 | (cancer vs cancer) |
| 12-63088234 | cg11311280 | (cancer vs cancer) (cancer+normal vs cancer+normal) | 3-137717665 | cg24894531 | (normal vs normal) (cancer vs cancer) |
| 12-64240787 | cg26214431 | (cancer vs cancer) (cancer+normal vs cancer+normal) | 3-137892432 | cg00933443 | (normal vs normal) (cancer vs cancer) (cancer+normal vs cancer+normal) |
| 12-72083379 | cg12552402 | (cancer vs cancer) (cancer+normal vs cancer+normal) | 3-155656364 | cg02625968 | (cancer vs cancer) |
| 12-76428445 | cg12202370 | (cancer vs cancer) (cancer+normal vs cancer+normal) | 3-170138205 | cg20794824 | (cancer vs cancer) |
| 12-76927207 | cg16165258 | (cancer vs cancer) (cancer+normal vs cancer+normal) | 3-52048820 | cg22291974 | (cancer vs cancer) (cancer+normal vs cancer+normal) |
| 12-80309007 | cg05381383 | (cancer vs cancer) (cancer+normal vs cancer+normal) | 3-79026069 | cg01375651 | (cancer vs cancer) |
| 12-95509731 | cg25036456 | (normal vs normal) (cancer vs cancer) (cancer+normal vs cancer+normal) | 4-2275994 | cg19771469 | (cancer vs cancer) |
| 12-966396 | cg06817737 | (cancer vs cancer) (cancer+normal vs cancer+normal) | 4-2276003 | cg22029856 | (cancer vs cancer) |
| 13- | cg02898500 | (cancer vs cancer) | 5- | cg0087668 | (cancer vs cancer) |

| | | | | | |
|---|---|---|---|---|---|
| 113826554 | | (cancer+normal vs cancer+normal) | 121646014 | 1 | |
| 14-102483074 | cg04310833 | (cancer vs cancer) (cancer+normal vs cancer+normal) | 5-132009352 | cg25368824 | (cancer vs cancer) (cancer+normal vs cancer+normal) |
| 14-102669785 | cg09980114 | (cancer vs cancer) (cancer+normal vs cancer+normal) | 5-42422138 | cg08357895 | (cancer vs cancer) |
| 14-102968497 | cg19687152 | (cancer vs cancer) (cancer+normal vs cancer+normal) | 6-105935915 | cg10111335 | (cancer vs cancer) (cancer+normal vs cancer+normal) |
| 14-67822707 | cg03487553 | (cancer vs cancer) (cancer+normal vs cancer+normal) | 6-107388925 | cg01963573 | (cancer vs cancer) |
| 14-71239883 | cg12850822 | (cancer vs cancer) (cancer+normal vs cancer+normal) | 6-11778902 | cg14178895 | (cancer vs cancer) (cancer+normal vs cancer+normal) |
| 14-76236901 | cg10395252 | (cancer vs cancer) (cancer+normal vs cancer+normal) | 6-125331312 | cg20433382 | (cancer vs cancer) |
| 14-95585023 | cg23488578 | (cancer vs cancer) (cancer+normal vs cancer+normal) | 6-125364093 | cg17993419 | (cancer vs cancer) (cancer+normal vs cancer+normal) |
| 15-57404080 | cg05141289 | (cancer vs cancer) (cancer+normal vs cancer+normal) | 6-125534847 | cg02807882 | (cancer vs cancer) |
| 15-70364327 | cg01666796 | (normal vs normal) (cancer vs cancer) (cancer+normal vs cancer+normal) | 6-126384600 | cg17620039 | (cancer vs cancer) |
| 16-29578336 | cg06184547 | (cancer vs cancer) (cancer+normal vs cancer+normal) | 6-167536063 | cg21794222 | (cancer vs cancer) |
| 16-479994 | cg10357909 | (cancer vs cancer) (cancer+normal vs cancer+normal) | 6-167536086 | cg01646461 | (cancer vs cancer) |
| 16-50351302 | cg01187464 | (cancer vs cancer) (cancer+normal vs cancer+normal) | 6-32810833 | cg16186435 | (cancer vs cancer) |
| 16-56394104 | cg00500540 | (cancer vs cancer) (cancer+normal vs cancer+normal) | 6-33715942 | cg18708504 | (cancer vs cancer) |
| 16-56398105 | cg01912578 | (cancer vs cancer) (cancer+normal vs cancer+normal) | 6-41892347 | cg15745273 | (cancer vs cancer) |
| 16-56899183 | cg03455024 | (cancer vs cancer) (cancer+normal vs cancer+normal) | 6-53181687 | cg16946445 | (cancer vs cancer) |
| 16-87873837 | cg06665333 | (cancer vs cancer) (cancer+normal vs cancer+normal) | 6-75960530 | cg02106279 | (cancer vs cancer) |
| 17-11985185 | cg18156845 | (cancer vs cancer) (cancer+normal vs cancer+normal) | 7-100663185 | cg13906098 | (cancer vs cancer) |
| 17-81040663 | cg04819655 | (cancer vs cancer) (cancer+normal vs cancer+normal) | 7-100877583 | cg05630192 | (cancer vs cancer) |
| 17-81045501 | cg14521546 | (cancer vs cancer) (cancer+normal vs cancer+normal) | 7-1896154 | cg22460466 | (cancer vs cancer) |
| 18- | cg21116087 | (normal vs normal) | 7-1896220 | cg2539763 | (cancer vs cancer) |

| | | | | | |
|---|---|---|---|---|---|
| 74832171 | | (cancer vs cancer) (cancer+normal vs cancer+normal) | | 6 | |
| 18-74832261 | cg16990945 | (normal vs normal) (cancer vs cancer) (cancer+normal vs cancer+normal) | 7-2686405 | cg02393470 | (cancer vs cancer) |
| 19-19104064 | cg06508379 | (cancer vs cancer) (cancer+normal vs cancer+normal) | 7-2686947 | cg21257581 | (cancer vs cancer) |
| 19-45316493 | cg12249345 | (normal vs normal) (cancer vs cancer) (cancer+normal vs cancer+normal) | 7-41351539 | cg00071241 | (cancer vs cancer) |
| 19-45316509 | cg21978694 | (normal vs normal) (cancer vs cancer) (cancer+normal vs cancer+normal) | 7-44438216 | cg23886101 | (cancer vs cancer) |
| 19-45316789 | cg22640961 | (cancer vs cancer) (cancer+normal vs cancer+normal) | 7-97834623 | cg02972941 | (cancer vs cancer) |
| 19-46404031 | cg07453407 | (cancer vs cancer) (cancer+normal vs cancer+normal) | 7-99773786 | cg01942654 | (cancer vs cancer) |
| 2-100061911 | cg09500565 | (cancer vs cancer) (cancer+normal vs cancer+normal) | 8-102083111 | cg03483314 | (cancer vs cancer) (cancer+normal vs cancer+normal) |
| 2-105695949 | cg25114693 | (cancer vs cancer) (cancer+normal vs cancer+normal) | 8-102699486 | cg25989783 | (cancer vs cancer) |
| 2-231582109 | cg06852824 | (cancer vs cancer) (cancer+normal vs cancer+normal) | 8-103491087 | cg16268315 | (cancer vs cancer) |
| 2-233766002 | cg09336899 | (cancer vs cancer) (cancer+normal vs cancer+normal) | 8-125928410 | cg18510311 | (cancer vs cancer) |
| 2-238617188 | cg16900589 | (cancer vs cancer) (cancer+normal vs cancer+normal) | 8-131368433 | cg16154713 | (cancer vs cancer) |
| 20-3128566 | cg27556861 | (cancer vs cancer) (cancer+normal vs cancer+normal) | 8-134546052 | cg00736681 | (cancer vs cancer) |
| 20-44439932 | cg01661001 | (cancer vs cancer) (cancer+normal vs cancer+normal) | 8-145626588 | cg20555305 | (cancer vs cancer) |
| 22-35694370 | cg27436324 | (cancer vs cancer) (cancer+normal vs cancer+normal) | 8-145721667 | cg21518261 | (cancer vs cancer) |
| 3-11634536 | cg08559364 | (cancer vs cancer) (cancer+normal vs cancer+normal) | 8-145721896 | cg26078439 | (cancer vs cancer) |
| 3-11666825 | cg07643000 | (cancer vs cancer) (cancer+normal vs cancer+normal) | 8-27516631 | cg27512316 | (cancer vs cancer) |
| 3-132373398 | cg07460095 | (cancer vs cancer) (cancer+normal vs cancer+normal) | 8-37591379 | cg06415302 | (cancer vs cancer) (cancer+normal vs cancer+normal) |
| 3-156955923 | cg10538433 | (cancer vs cancer) (cancer+normal vs cancer+normal) | 8-41507621 | cg11363615 | (cancer vs cancer) |
| 3- | cg13017471 | (cancer vs cancer) | 8-41507844 | cg2146863 | (cancer vs cancer) |

| | | | | | |
|---|---|---|---|---|---|
| 171832308 | | (cancer+normal vs cancer+normal) | | 4 | |
| 3-188745304 | cg13237147 | (cancer vs cancer) (cancer+normal vs cancer+normal) | 8-41507852 | cg15649474 | (cancer vs cancer) |
| 3-196693809 | cg21429725 | (cancer vs cancer) (cancer+normal vs cancer+normal) | 8-41508618 | cg07161603 | (cancer vs cancer) |
| 3-30691493 | cg24719910 | (cancer vs cancer) (cancer+normal vs cancer+normal) | 8-41508779 | cg19945060 | (cancer vs cancer) |
| 3-61889337 | cg24295890 | (cancer vs cancer) (cancer+normal vs cancer+normal) | 8-49307089 | cg02467602 | (cancer vs cancer) |
| 4-140745554 | cg16650096 | (cancer vs cancer) (cancer+normal vs cancer+normal) | 8-8239485 | cg18303581 | (cancer vs cancer) |
| 4-149360981 | cg23329208 | (cancer vs cancer) (cancer+normal vs cancer+normal) | 9-111616366 | cg11272244 | (cancer vs cancer) |
| 4-15659864 | cg18334727 | (cancer vs cancer) (cancer+normal vs cancer+normal) | 9-3899055 | cg14269813 | (cancer vs cancer) (cancer+normal vs cancer+normal) |
| 4-169480345 | cg16373817 | (cancer vs cancer) (cancer+normal vs cancer+normal) | **(cancer+normal vs cancer+normal)** | | |
| 4-185317438 | cg00762029 | (cancer vs cancer) (cancer+normal vs cancer+normal) | **Status - Hyper** | | |
| 4-185362534 | cg02354388 | (cancer vs cancer) (cancer+normal vs cancer+normal) | 1-26372887 | cg07766612 | (cancer+normal vs cancer+normal) |
| 4-38006787 | cg23289854 | (cancer vs cancer) (cancer+normal vs cancer+normal) | 1-29138936 | cg08366446 | (cancer vs cancer) (cancer+normal vs cancer+normal) |
| 4-73941755 | cg09428766 | (cancer vs cancer) (cancer+normal vs cancer+normal) | 1-57111099 | cg04876835 | (cancer+normal vs cancer+normal) |
| 4-77953569 | cg14118160 | (cancer vs cancer) (cancer+normal vs cancer+normal) | 1-57111108 | cg12167830 | (cancer+normal vs cancer+normal) |
| 5-139260468 | cg25228995 | (cancer vs cancer) (cancer+normal vs cancer+normal) | 1-57111117 | cg26068551 | (cancer vs cancer) (normal vs normal) (cancer+normal vs cancer+normal) |
| 5-173307471 | cg13384849 | (cancer vs cancer) (cancer+normal vs cancer+normal) | 1-57111122 | cg09686317 | (cancer vs cancer) (cancer+normal vs cancer+normal) |
| 5-80530213 | cg16230060 | (cancer vs cancer) (cancer+normal vs cancer+normal) | 10-135050779 | cg18002909 | (normal vs normal) (cancer+normal vs cancer+normal) |
| 5-96304288 | cg19691163 | (cancer vs cancer) | 10-20104497 | cg24022375 | (cancer vs cancer) (cancer vs cancer) (cancer+normal vs cancer+normal) |
| 6-100917427 | cg25351606 | (cancer vs cancer) (cancer+normal vs cancer+normal) | 10-20104806 | cg16747247 | (cancer vs cancer) (cancer+normal vs cancer+normal) |
| 6-11185142 | cg18202405 | (cancer vs cancer) (cancer+normal vs cancer+normal) | 10-20104935 | cg17603206 | (cancer vs cancer) (cancer+normal vs cancer+normal) |

| | | | | | |
|---|---|---|---|---|---|
| 6-170686956 | cg00489213 | (cancer vs cancer) (cancer+normal vs cancer+normal) | 10-20104948 | cg08625590 | (cancer vs cancer) (cancer vs cancer) (cancer+normal vs cancer+normal) |
| 7-107771148 | cg01550161 | (cancer vs cancer) (cancer+normal vs cancer+normal) | 10-20105641 | cg12563178 | (cancer vs cancer) (cancer+normal vs cancer+normal) |
| 7-116272127 | cg23886314 | (cancer vs cancer) (cancer+normal vs cancer+normal) | 10-28032282 | cg16397794 | (cancer vs cancer) (cancer+normal vs cancer+normal) |
| 7-127574992 | cg18495781 | (cancer vs cancer) (cancer+normal vs cancer+normal) | 10-90342899 | cg19235339 | (cancer vs cancer) (cancer+normal vs cancer+normal) |
| 7-135624343 | cg05600688 | (cancer vs cancer) (cancer+normal vs cancer+normal) | 11-113930430 | cg09719477 | (cancer+normal vs cancer+normal) |
| 7-151335190 | cg11344729 | (cancer vs cancer) (cancer+normal vs cancer+normal) | 12-107713740 | cg24966575 | (cancer+normal vs cancer+normal) |
| 7-23723014 | cg20200811 | (cancer vs cancer) (cancer+normal vs cancer+normal) | 12-20521463 | cg17535647 | (cancer vs cancer) (cancer+normal vs cancer+normal) |
| 7-5270512 | cg21585409 | (cancer vs cancer) (cancer+normal vs cancer+normal) | 13-110961069 | cg24909466 | (cancer vs cancer) (cancer+normal vs cancer+normal) |
| 7-5270553 | cg21764632 | (cancer vs cancer) (cancer+normal vs cancer+normal) | 13-28674720 | cg14660839 | (cancer vs cancer) (cancer+normal vs cancer+normal) |
| 7-98667581 | cg09221667 | (cancer vs cancer) (cancer+normal vs cancer+normal) | 13-44360259 | cg10482596 | (cancer+normal vs cancer+normal) |
| 8-10454154 | cg08775375 | (cancer vs cancer) (cancer+normal vs cancer+normal) | 14-23822017 | cg01557297 | (cancer vs cancer) (cancer+normal vs cancer+normal) |
| 8-109206912 | cg13734860 | (cancer vs cancer) (cancer+normal vs cancer+normal) | 14-36989550 | cg14005139 | (cancer+normal vs cancer+normal) |
| 8-124515502 | cg01955203 | (cancer vs cancer) (cancer+normal vs cancer+normal) | 14-36989936 | cg10999312 | (cancer+normal vs cancer+normal) |
| 8-129073877 | cg24146714 | (cancer vs cancer) (cancer+normal vs cancer+normal) | 14-96505874 | cg04184208 | (cancer vs cancer) (cancer+normal vs cancer+normal) |
| 8-135817117 | cg01954305 | (cancer vs cancer) (cancer+normal vs cancer+normal) | 15-93632619 | cg20899253 | (cancer vs cancer) (cancer+normal vs cancer+normal) |
| 8-142309582 | cg25043496 | (cancer vs cancer) (cancer+normal vs cancer+normal) | 16-88450241 | cg06184647 | (normal vs normal) (cancer+normal vs cancer+normal) |
| 8-142309652 | cg10090414 | (cancer vs cancer) (cancer+normal vs cancer+normal) | 17-17399405 | cg18050291 | (normal vs normal) (cancer+normal vs cancer+normal) |
| 8-145685564 | cg00718444 | (cancer vs cancer) (cancer+normal vs cancer+normal) | 17-19437157 | cg13004927 | (cancer+normal vs cancer+normal) |
| 8-6616240 | cg24485696 | (cancer vs cancer) (cancer+normal vs cancer+normal) | 17-45056008 | cg22674412 | (cancer+normal vs cancer+normal) |
| 9-103062981 | cg14091473 | (cancer vs cancer) (cancer+normal vs cancer+normal) | 17-59476790 | cg26932779 | (cancer vs cancer) (cancer+normal vs cancer+normal) |

| | | (cancer+normal vs cancer+normal) | | 17-59477557 | cg19949441 | (cancer vs cancer) (cancer+normal vs cancer+normal) |
|---|---|---|---|---|---|---|
| | | **Status - Hyper** | | 17-59477564 | cg09365557 | (cancer vs cancer) (cancer+normal vs cancer+normal) |
| 1-152538426 | cg07476201 | (cancer vs cancer) (cancer+normal vs cancer+normal) | | 17-79373381 | cg16560774 | (normal vs normal) (cancer+normal vs cancer+normal) |
| 1-152538857 | cg24000437 | (cancer vs cancer) (cancer vs cancer) (cancer+normal vs cancer+normal) | | 17-79373624 | cg00712390 | (cancer vs cancer) (cancer+normal vs cancer+normal) |
| 1-166139996 | cg11979420 | (cancer vs cancer) (cancer+normal vs cancer+normal) | | 17-79373673 | cg07219667 | (normal vs normal) (cancer+normal vs cancer+normal) |
| 1-17337346 | cg22045253 | (cancer vs cancer) (cancer+normal vs cancer+normal) | | 18-57364587 | cg02774186 | (cancer vs cancer) (cancer+normal vs cancer+normal) |
| 1-184357556 | cg10061342 | (cancer vs cancer) (cancer vs cancer) (cancer+normal vs cancer+normal) | | 18-6414978 | cg17688525 | (normal vs normal) (cancer+normal vs cancer+normal) |
| 1-186504948 | cg05398036 | (cancer vs cancer) (cancer+normal vs cancer+normal) | | 18-7566781 | cg16474170 | (cancer vs cancer) (cancer+normal vs cancer+normal) |
| 1-19109544 | cg03315346 | (cancer vs cancer) (cancer+normal vs cancer+normal) | | 19-31841663 | cg11199770 | (cancer vs cancer) (cancer vs cancer) (normal vs normal) (cancer+normal vs cancer+normal) |
| 1-222816858 | cg00089945 | (cancer vs cancer) (cancer+normal vs cancer+normal) | | 19-31841952 | cg15711268 | (cancer vs cancer) (cancer vs cancer) (cancer+normal vs cancer+normal) |
| 1-226112534 | cg06939851 | (cancer vs cancer) (cancer+normal vs cancer+normal) | | 19-58514022 | cg08397818 | (cancer+normal vs cancer+normal) |
| 1-23851471 | cg09119494 | (cancer vs cancer) (cancer+normal vs cancer+normal) | | 2-109745812 | cg12518410 | (cancer vs cancer) (cancer+normal vs cancer+normal) |
| 1-39491459 | cg07388493 | (cancer vs cancer) (cancer+normal vs cancer+normal) | | 2-109745828 | cg09392940 | (cancer vs cancer) (cancer+normal vs cancer+normal) |
| 1-40887988 | cg26042300 | (cancer vs cancer) (cancer vs cancer) (cancer+normal vs cancer+normal) | | 2-180725907 | cg22657780 | (cancer vs cancer) (cancer+normal vs cancer+normal) |
| 1-46859774 | cg12671744 | (cancer vs cancer) (cancer+normal vs cancer+normal) | | 2-180726035 | cg24575676 | (cancer vs cancer) (cancer+normal vs cancer+normal) |
| 1-90309410 | cg24720717 | (cancer vs cancer) (cancer+normal vs cancer+normal) | | 2-180726247 | cg11113760 | (cancer vs cancer) (cancer+normal vs cancer+normal) |
| 1-9224198 | cg22373770 | (cancer vs cancer) (cancer+normal vs cancer+normal) | | 2-180726249 | cg26271891 | (cancer vs cancer) (cancer+normal vs cancer+normal) |
| 10-102588959 | cg17480467 | (cancer vs cancer) (cancer vs cancer) (normal vs normal) (cancer+normal vs | | 2-180726252 | cg09279240 | (cancer vs cancer) (cancer+normal vs cancer+normal) |

| | | | | | |
|---|---|---|---|---|---|
| | | cancer+normal) | | | |
| 10-102589102 | cg14778235 | (cancer vs cancer) (cancer vs cancer) (normal vs normal) (cancer+normal vs cancer+normal) | 2-180726328 | cg06695611 | (cancer vs cancer) (cancer+normal vs cancer+normal) |
| 10-102589250 | cg08217716 | (cancer vs cancer) (cancer vs cancer) (normal vs normal) (cancer+normal vs cancer+normal) | 2-197457430 | cg07853758 | (cancer vs cancer) (cancer+normal vs cancer+normal) |
| 10-120154512 | cg23160142 | (cancer vs cancer) (cancer+normal vs cancer+normal) | 2-197458046 | cg24613479 | (cancer vs cancer) (cancer+normal vs cancer+normal) |
| 10-128360149 | cg06624146 | (cancer vs cancer) (cancer+normal vs cancer+normal) | 2-197458093 | cg01381678 | (cancer vs cancer) (cancer+normal vs cancer+normal) |
| 10-129350922 | cg19321696 | (cancer vs cancer) (cancer+normal vs cancer+normal) | 2-197458104 | cg12644696 | (cancer vs cancer) (cancer+normal vs cancer+normal) |
| 10-129706313 | cg16750801 | (cancer vs cancer) (cancer+normal vs cancer+normal) | 2-213403929 | cg16329650 | (cancer vs cancer) (cancer+normal vs cancer+normal) |
| 10-17273187 | cg26063719 | (cancer vs cancer) (normal vs normal) (cancer+normal vs cancer+normal) | 2-213403960 | cg20637307 | (cancer+normal vs cancer+normal) |
| 10-31101116 | cg03127349 | (cancer vs cancer) (cancer+normal vs cancer+normal) | 2-68870499 | cg19452535 | (cancer+normal vs cancer+normal) |
| 10-34320548 | cg10772185 | (cancer vs cancer) (cancer+normal vs cancer+normal) | 2-68870812 | cg13451280 | (cancer+normal vs cancer+normal) |
| 10-375391 | cg06193766 | (cancer vs cancer) (cancer vs cancer) (cancer+normal vs cancer+normal) | 20-13200944 | cg14060111 | (normal vs normal) (cancer+normal vs cancer+normal) |
| 10-375830 | cg08410533 | (cancer vs cancer) (cancer+normal vs cancer+normal) | 20-13200954 | cg12664209 | (normal vs normal) (cancer+normal vs cancer+normal) |
| 10-99822472 | cg07232642 | (cancer vs cancer) (cancer+normal vs cancer+normal) | 20-13200969 | cg02145932 | (cancer vs cancer) (cancer+normal vs cancer+normal) |
| 11-59950525 | cg00673646 | (cancer vs cancer) (cancer+normal vs cancer+normal) | 20-13200973 | cg06262436 | (cancer vs cancer) (cancer+normal vs cancer+normal) |
| 11-7728814 | cg13563074 | (cancer vs cancer) (cancer vs cancer) (cancer+normal vs cancer+normal) | 20-13200980 | cg09491991 | (cancer vs cancer) (normal vs normal) (cancer+normal vs cancer+normal) |
| 11-7960899 | cg09140665 | (cancer vs cancer) (cancer+normal vs cancer+normal) | 20-13200992 | cg21561970 | (cancer vs cancer) (cancer+normal vs cancer+normal) |
| 12-10022682 | cg08752459 | (cancer vs cancer) (cancer vs cancer) (normal vs normal) (cancer+normal vs cancer+normal) | 20-13201456 | cg18993918 | (cancer vs cancer) (cancer+normal vs cancer+normal) |
| 12-10022688 | cg12591315 | (cancer vs cancer) (cancer vs cancer) (normal vs normal) | 20-19193060 | cg15501281 | (cancer+normal vs cancer+normal) |

| | | | | | |
|---|---|---|---|---|---|
| | | (cancer+normal vs cancer+normal) | | | |
| 12-105089508 | cg07696842 | (cancer vs cancer) (cancer+normal vs cancer+normal) | 22-45403493 | cg09273641 | (cancer+normal vs cancer+normal) |
| 12-113229319 | cg01573562 | (cancer vs cancer) (cancer+normal vs cancer+normal) | 22-45403700 | cg02869649 | (cancer+normal vs cancer+normal) |
| 12-116997535 | cg03822198 | (cancer vs cancer) (cancer+normal vs cancer+normal) | 3-56502265 | cg08065241 | (cancer vs cancer) (cancer+normal vs cancer+normal) |
| 12-131198873 | cg27080194 | (cancer vs cancer) (cancer+normal vs cancer+normal) | 3-56502293 | cg06678279 | (cancer vs cancer) (cancer+normal vs cancer+normal) |
| 12-70215816 | cg23907053 | (cancer vs cancer) (cancer+normal vs cancer+normal) | 3-77089110 | cg17189465 | (cancer vs cancer) (cancer+normal vs cancer+normal) |
| 13-46870454 | cg11203397 | (cancer vs cancer) (cancer+normal vs cancer+normal) | 3-87040286 | cg12615137 | (cancer vs cancer) (cancer+normal vs cancer+normal) |
| 15-25330341 | cg08051964 | (cancer vs cancer) (cancer+normal vs cancer+normal) | 3-87040426 | cg09376680 | (cancer+normal vs cancer+normal) |
| 15-70767570 | cg16973527 | (cancer vs cancer) (cancer vs cancer) (cancer+normal vs cancer+normal) | 3-96532043 | cg11090352 | (cancer vs cancer) (cancer+normal vs cancer+normal) |
| 15-70767649 | cg13427748 | (cancer vs cancer) (cancer vs cancer) (cancer+normal vs cancer+normal) | 4-121843785 | cg13155001 | (cancer+normal vs cancer+normal) |
| 16-2391081 | cg02331561 | (cancer vs cancer) (cancer+normal vs cancer+normal) | 4-13546151 | cg27438798 | (cancer vs cancer) (cancer+normal vs cancer+normal) |
| 16-4103492 | cg10177032 | (cancer vs cancer) (cancer+normal vs cancer+normal) | 4-166300252 | cg16534103 | (cancer vs cancer) (cancer+normal vs cancer+normal) |
| 16-4103533 | cg01352090 | (cancer vs cancer) (cancer+normal vs cancer+normal) | 4-166300290 | cg02886284 | (cancer vs cancer) (cancer vs cancer) (cancer+normal vs cancer+normal) |
| 16-474271 | cg09555736 | (cancer vs cancer) (cancer+normal vs cancer+normal) | 4-183370059 | cg02422426 | (normal vs normal) (cancer+normal vs cancer+normal) |
| 16-474430 | cg02102075 | (cancer vs cancer) (cancer+normal vs cancer+normal) | 4-183370138 | cg09767602 | (cancer vs cancer) (normal vs normal) (cancer+normal vs cancer+normal) |
| 16-84390897 | cg05964640 | (cancer vs cancer) (cancer+normal vs cancer+normal) | 4-20253514 | cg13755796 | (cancer vs cancer) (cancer+normal vs cancer+normal) |
| 17-19967600 | cg02656560 | (cancer vs cancer) (cancer+normal vs cancer+normal) | 4-20253556 | cg10322724 | (cancer vs cancer) (cancer+normal vs cancer+normal) |
| 17-33473881 | cg10174170 | (cancer vs cancer) (cancer+normal vs cancer+normal) | 4-20253595 | cg01281776 | (cancer vs cancer) (cancer+normal vs cancer+normal) |
| 17-48989061 | cg11235451 | (cancer vs cancer) (cancer+normal vs cancer+normal) | 4-55524400 | cg17891820 | (cancer vs cancer) (cancer+normal vs cancer+normal) |
| 17- | cg08122232 | (cancer vs cancer) | 4-87515467 | cg1554356 | (cancer+normal vs |

316

| | | | | | |
|---|---|---|---|---|---|
| 74100132 | | (cancer+normal vs cancer+normal) | | 6 | cancer+normal) |
| 19-14785849 | cg15746620 | (cancer vs cancer) (cancer vs cancer) (cancer+normal vs cancer+normal) | 5-121647858 | cg0468451 6 | (cancer vs cancer) (cancer+normal vs cancer+normal) |
| 19-49164383 | cg09341448 | (cancer vs cancer) (cancer+normal vs cancer+normal) | 5-175223982 | cg0729596 4 | (cancer vs cancer) (cancer+normal vs cancer+normal) |
| 19-51018365 | cg15655714 | (cancer vs cancer) (cancer+normal vs cancer+normal) | 5-42423615 | cg0821722 7 | (cancer vs cancer) (cancer+normal vs cancer+normal) |
| 2-121496875 | cg20219891 | (cancer vs cancer) (cancer vs cancer) (cancer+normal vs cancer+normal) | 5-42423947 | cg1204258 7 | (cancer vs cancer) (cancer vs cancer) (normal vs normal) (cancer+normal vs cancer+normal) |
| 2-138019701 | cg05853130 | (cancer vs cancer) (cancer+normal vs cancer+normal) | 5-42424356 | cg1629201 6 | (cancer vs cancer) (cancer+normal vs cancer+normal) |
| 2-176970064 | cg06354392 | (cancer vs cancer) (cancer+normal vs cancer+normal) | 6-125283245 | cg2482269 6 | (cancer+normal vs cancer+normal) |
| 2-176970508 | cg18351329 | (cancer vs cancer) (cancer+normal vs cancer+normal) | 6-146864781 | cg1076647 5 | (normal vs normal) (cancer+normal vs cancer+normal) |
| 2-236172440 | cg09406107 | (cancer vs cancer) (cancer+normal vs cancer+normal) | 6-17281838 | cg1009614 5 | (cancer vs cancer) (cancer+normal vs cancer+normal) |
| 2-97202260 | cg02085953 | (cancer vs cancer) (cancer+normal vs cancer+normal) | 6-21664652 | cg2442887 7 | (cancer vs cancer) (cancer vs cancer) (normal vs normal) (cancer+normal vs cancer+normal) |
| 20-57975832 | cg12649683 | (cancer vs cancer) (cancer+normal vs cancer+normal) | 7-42277375 | cg0719174 3 | (cancer vs cancer) (cancer+normal vs cancer+normal) |
| 22-46934981 | cg11533712 | (cancer vs cancer) (cancer+normal vs cancer+normal) | 8-105601615 | cg0441389 5 | (cancer vs cancer) (normal vs normal) (cancer+normal vs cancer+normal) |
| 3-143567492 | cg15360181 | (cancer vs cancer) (cancer+normal vs cancer+normal) | 8-105601641 | cg0167581 4 | (cancer+normal vs cancer+normal) |
| 3-149095573 | cg23246821 | (cancer vs cancer) (cancer+normal vs cancer+normal) | 8-133492476 | cg2701699 0 | (cancer vs cancer) (cancer+normal vs cancer+normal) |
| 3-194030706 | cg07141504 | (cancer vs cancer) (cancer+normal vs cancer+normal) | 8-21646662 | cg0345363 8 | (cancer vs cancer) (cancer+normal vs cancer+normal) |
| 4-16628713 | cg06818159 | (cancer vs cancer) (cancer+normal vs cancer+normal) | 8-6692148 | cg1776318 8 | (cancer vs cancer) (cancer+normal vs cancer+normal) |
| 4-40057241 | cg01492656 | (cancer vs cancer) (cancer+normal vs cancer+normal) | 8-6693103 | cg1248255 7 | (cancer vs cancer) (cancer+normal vs cancer+normal) |
| 4-74570525 | cg23099587 | (cancer vs cancer) (cancer+normal vs cancer+normal) | **Status - Hypo** | | |
| 4-77119206 | cg01624414 | (cancer vs cancer) (cancer+normal vs | 1-111023201 | cg1659289 7 | (cancer vs cancer) (cancer+normal vs |

| | | cancer+normal) | | | cancer+normal) |
|---|---|---|---|---|---|
| 5-162516650 | cg18086386 | (cancer vs cancer) (cancer vs cancer) (cancer+normal vs cancer+normal) | 1-119957380 | cg20414217 | (cancer+normal vs cancer+normal) |
| 5-171870875 | cg19979108 | (cancer vs cancer) (cancer+normal vs cancer+normal) | 1-162219640 | cg21692434 | (normal vs normal) (cancer+normal vs cancer+normal) |
| 5-173852421 | cg18040788 | (cancer vs cancer) (cancer+normal vs cancer+normal) | 1-176147105 | cg07205154 | (cancer+normal vs cancer+normal) |
| 5-55881858 | cg00329411 | (cancer vs cancer) (cancer+normal vs cancer+normal) | 1-204230657 | cg23234811 | (cancer+normal vs cancer+normal) |
| 5-97645526 | cg14972576 | (cancer vs cancer) (cancer+normal vs cancer+normal) | 1-207991780 | cg03885098 | (cancer+normal vs cancer+normal) |
| 6-100650602 | cg01889956 | (cancer vs cancer) (cancer+normal vs cancer+normal) | 1-24833311 | cg15519096 | (cancer+normal vs cancer+normal) |
| 6-106773949 | cg14951955 | (cancer vs cancer) (cancer+normal vs cancer+normal) | 1-68209155 | cg11209521 | (normal vs normal) (cancer+normal vs cancer+normal) |
| 6-110736772 | cg02872426 | (cancer vs cancer) (cancer+normal vs cancer+normal) | 1-95639346 | cg16000017 | (cancer+normal vs cancer+normal) |
| 6-134216081 | cg07571531 | (cancer vs cancer) (cancer vs cancer) (cancer+normal vs cancer+normal) | 10-101194096 | cg17295053 | (cancer+normal vs cancer+normal) |
| 6-166970727 | cg11387340 | (cancer vs cancer) (cancer+normal vs cancer+normal) | 10-126714382 | cg27660388 | (cancer+normal vs cancer+normal) |
| 6-168134760 | cg01797450 | (cancer vs cancer) (cancer+normal vs cancer+normal) | 10-127569494 | cg06507244 | (cancer+normal vs cancer+normal) |
| 6-28892465 | cg23756442 | (cancer vs cancer) (cancer vs cancer) (cancer+normal vs cancer+normal) | 10-129105057 | cg03458096 | (cancer+normal vs cancer+normal) |
| 6-29012588 | cg07053841 | (cancer vs cancer) (cancer+normal vs cancer+normal) | 10-34604934 | cg13976324 | (normal vs normal) (cancer+normal vs cancer+normal) |
| 6-32172871 | cg18244508 | (cancer vs cancer) (cancer+normal vs cancer+normal) | 11-100661648 | cg15880016 | (cancer+normal vs cancer+normal) |
| 6-33256216 | cg17131579 | (cancer vs cancer) (cancer+normal vs cancer+normal) | 11-15692519 | cg11966998 | (cancer vs cancer) (cancer+normal vs cancer+normal) |
| 6-35309867 | cg21748751 | (cancer vs cancer) (cancer+normal vs cancer+normal) | 11-69464012 | cg15974867 | (cancer+normal vs cancer+normal) |
| 6-50785325 | cg06653004 | (cancer vs cancer) (cancer+normal vs cancer+normal) | 11-75528534 | cg13641591 | (cancer+normal vs cancer+normal) |
| 6-50812112 | cg17153727 | (cancer vs cancer) (cancer+normal vs cancer+normal) | 11-811669 | cg25872852 | (normal vs normal) (cancer+normal vs cancer+normal) |
| 6-97010903 | cg03043157 | (cancer vs cancer) (cancer+normal vs cancer+normal) | 12-110368234 | cg18926450 | (cancer+normal vs cancer+normal) |

318

| | | | | | |
|---|---|---|---|---|---|
| 7-27404187 | cg05729490 | (cancer vs cancer) (cancer+normal vs cancer+normal) | 12-1748816 | cg02935351 | (cancer vs cancer) (cancer+normal vs cancer+normal) |
| 7-28726216 | cg05083033 | (cancer vs cancer) (cancer+normal vs cancer+normal) | 12-32711698 | cg16820973 | (cancer+normal vs cancer+normal) |
| 7-5277188 | cg10229594 | (cancer vs cancer) (cancer+normal vs cancer+normal) | 12-52610273 | cg19959477 | (normal vs normal) (cancer+normal vs cancer+normal) |
| 7-69289866 | cg21557473 | (cancer vs cancer) (cancer+normal vs cancer+normal) | 12-59319720 | cg10283871 | (cancer vs cancer) (cancer+normal vs cancer+normal) |
| 7-852714 | cg19239199 | (cancer vs cancer) (cancer+normal vs cancer+normal) | 13-111277611 | cg21664614 | (cancer+normal vs cancer+normal) |
| 7-99517509 | cg25591451 | (cancer vs cancer) (cancer+normal vs cancer+normal) | 13-114014065 | cg01961211 | (cancer+normal vs cancer+normal) |
| 8-21900314 | cg26069514 | (cancer vs cancer) (cancer+normal vs cancer+normal) | 13-45911764 | cg11218434 | (cancer+normal vs cancer+normal) |
| 8-40602363 | cg02458516 | (cancer vs cancer) (cancer+normal vs cancer+normal) | 13-45912168 | cg04766834 | (cancer+normal vs cancer+normal) |
| 9-110310795 | cg13430755 | (cancer vs cancer) (cancer+normal vs cancer+normal) | 13-46093146 | cg22419414 | (cancer+normal vs cancer+normal) |
| 9-138605049 | cg14341131 | (cancer vs cancer) (cancer vs cancer) (cancer+normal vs cancer+normal) | 15-75915192 | cg19013753 | (cancer+normal vs cancer+normal) |
| 9-91267411 | cg03578548 | (cancer vs cancer) (cancer+normal vs cancer+normal) | 15-77456283 | cg10577241 | (cancer+normal vs cancer+normal) |
| **Status - Hypo** | | | 16-1673702 | cg27624753 | (cancer+normal vs cancer+normal) |
| 1-223472245 | cg22158547 | (cancer vs cancer) (cancer+normal vs cancer+normal) | 16-29913162 | cg09328356 | (cancer vs cancer) (cancer+normal vs cancer+normal) |
| 1-3129633 | cg05265611 | (cancer vs cancer) (cancer+normal vs cancer+normal) | 16-88564747 | cg01750783 | (cancer+normal vs cancer+normal) |
| 10-126314680 | cg04426802 | (cancer vs cancer) (cancer+normal vs cancer+normal) | 16-89367323 | cg09607566 | (cancer+normal vs cancer+normal) |
| 10-77165905 | cg06262069 | (cancer vs cancer) (cancer+normal vs cancer+normal) | 17-33864734 | cg03526142 | (cancer+normal vs cancer+normal) |
| 11-10462924 | cg06814191 | (cancer vs cancer) (cancer+normal vs cancer+normal) | 17-5265254 | cg25136408 | (cancer+normal vs cancer+normal) |
| 11-108365051 | cg04295172 | (cancer vs cancer) (cancer+normal vs cancer+normal) | 17-77923971 | cg03296204 | (cancer+normal vs cancer+normal) |
| 11-456063 | cg16124920 | (cancer vs cancer) (cancer+normal vs cancer+normal) | 17-77948884 | cg19790877 | (cancer vs cancer) (cancer+normal vs cancer+normal) |
| 11-63906084 | cg04725041 | (cancer vs cancer) (cancer+normal vs cancer+normal) | 17-79146774 | cg16466065 | (cancer+normal vs cancer+normal) |
| 11- | cg22015277 | (cancer vs cancer) | 17- | cg1953640 | (cancer vs cancer) |

| | | | | | |
|---|---|---|---|---|---|
| 66494177 | | (cancer+normal vs cancer+normal) | 79431331 | 7 | (cancer+normal vs cancer+normal) |
| 12-110986864 | cg15698107 | (cancer vs cancer) (cancer+normal vs cancer+normal) | 17-79623626 | cg16690753 | (cancer+normal vs cancer+normal) |
| 12-111883369 | cg13116789 | (cancer vs cancer) (cancer+normal vs cancer+normal) | 18-19780878 | cg00148892 | (cancer+normal vs cancer+normal) |
| 12-116808922 | cg18623355 | (cancer+normal vs cancer+normal) | 18-67997425 | cg13360215 | (cancer+normal vs cancer+normal) |
| 12-124812999 | cg11601197 | (cancer vs cancer) (cancer+normal vs cancer+normal) | 18-77283493 | cg22324981 | (normal vs normal) (cancer+normal vs cancer+normal) |
| 12-4762206 | cg13719771 | (cancer vs cancer) (cancer+normal vs cancer+normal) | 19-49253931 | cg22518433 | (cancer+normal vs cancer+normal) |
| 12-63088234 | cg11311280 | (cancer vs cancer) (cancer+normal vs cancer+normal) | 2-103095638 | cg03405781 | (normal vs normal) (cancer+normal vs cancer+normal) |
| 12-64240787 | cg26214431 | (cancer vs cancer) (cancer+normal vs cancer+normal) | 2-181235115 | cg19218067 | (cancer+normal vs cancer+normal) |
| 12-72083379 | cg12552402 | (cancer vs cancer) (cancer+normal vs cancer+normal) | 2-23852064 | cg04937609 | (cancer+normal vs cancer+normal) |
| 12-76428445 | cg12202370 | (cancer vs cancer) (cancer+normal vs cancer+normal) | 2-239983929 | cg08825699 | (cancer+normal vs cancer+normal) |
| 12-76927207 | cg16165258 | (cancer vs cancer) (cancer+normal vs cancer+normal) | 2-69399575 | cg10803714 | (cancer+normal vs cancer+normal) |
| 12-80309007 | cg05381383 | (cancer vs cancer) (cancer+normal vs cancer+normal) | 2-86037857 | cg08183425 | (cancer vs cancer) (cancer+normal vs cancer+normal) |
| 12-95509731 | cg25036456 | (normal vs normal) (cancer vs cancer) (cancer+normal vs cancer+normal) | 3-124284219 | cg06218523 | (cancer+normal vs cancer+normal) |
| 12-966396 | cg06817737 | (cancer vs cancer) (cancer+normal vs cancer+normal) | 3-132070595 | cg05409131 | (cancer+normal vs cancer+normal) |
| 13-113826554 | cg02898500 | (cancer vs cancer) (cancer+normal vs cancer+normal) | 3-134124275 | cg18130622 | (cancer+normal vs cancer+normal) |
| 14-102483074 | cg04310833 | (cancer vs cancer) (cancer+normal vs cancer+normal) | 3-137892432 | cg00933443 | (normal vs normal) (cancer vs cancer) (cancer+normal vs cancer+normal) |
| 14-102669785 | cg09980114 | (cancer vs cancer) (cancer+normal vs cancer+normal) | 3-138078989 | cg08932381 | (cancer+normal vs cancer+normal) |
| 14-102968497 | cg19687152 | (cancer vs cancer) (cancer+normal vs cancer+normal) | 3-149832476 | cg27316567 | (normal vs normal) (cancer+normal vs cancer+normal) |
| 14-67822707 | cg03487553 | (cancer vs cancer) (cancer+normal vs cancer+normal) | 3-194868750 | cg21937377 | (normal vs normal) (cancer vs cancer) (cancer vs cancer) (cancer+normal vs cancer+normal) |
| 14-71239883 | cg12850822 | (cancer vs cancer) (cancer+normal vs | 3-52048820 | cg22291974 | (cancer vs cancer) (cancer+normal vs |

| | | | | | |
|---|---|---|---|---|---|
| | | cancer+normal) | | | cancer+normal) |
| 14-76236901 | cg10395252 | (cancer vs cancer) (cancer+normal vs cancer+normal) | 3-67691965 | cg27533999 | (cancer+normal vs cancer+normal) |
| 14-95585023 | cg23488578 | (cancer vs cancer) (cancer+normal vs cancer+normal) | 4-2439397 | cg05347334 | (cancer+normal vs cancer+normal) |
| 15-57404080 | cg05141289 | (cancer vs cancer) (cancer+normal vs cancer+normal) | 4-2439731 | cg26605046 | (normal vs normal) (cancer vs cancer) (cancer+normal vs cancer+normal) |
| 15-70364327 | cg01666796 | (normal vs normal) (cancer vs cancer) (cancer+normal vs cancer+normal) | 4-37686268 | cg06562964 | (cancer+normal vs cancer+normal) |
| 16-29578336 | cg06184547 | (cancer vs cancer) (cancer+normal vs cancer+normal) | 4-95335239 | cg24557027 | (normal vs normal) (cancer+normal vs cancer+normal) |
| 16-479994 | cg10357909 | (cancer vs cancer) (cancer+normal vs cancer+normal) | 5-132009352 | cg25368824 | (cancer vs cancer) (cancer+normal vs cancer+normal) |
| 16-50351302 | cg01187464 | (cancer vs cancer) (cancer+normal vs cancer+normal) | 5-1342170 | cg13325231 | (cancer+normal vs cancer+normal) |
| 16-56394104 | cg00500540 | (cancer vs cancer) (cancer+normal vs cancer+normal) | 5-1342172 | cg07493874 | (cancer+normal vs cancer+normal) |
| 16-56398105 | cg01912578 | (cancer vs cancer) (cancer+normal vs cancer+normal) | 5-1342200 | cg14733637 | (normal vs normal) (cancer+normal vs cancer+normal) |
| 16-56899183 | cg03455024 | (cancer vs cancer) (cancer+normal vs cancer+normal) | 5-139079288 | cg25551358 | (normal vs normal) (cancer+normal vs cancer+normal) |
| 16-87873837 | cg06665333 | (cancer vs cancer) (cancer+normal vs cancer+normal) | 5-1489889 | cg16272981 | (normal vs normal) (cancer+normal vs cancer+normal) |
| 17-11985185 | cg18156845 | (cancer vs cancer) (cancer+normal vs cancer+normal) | 5-172117322 | cg15552370 | (cancer+normal vs cancer+normal) |
| 17-81040663 | cg04819655 | (cancer vs cancer) (cancer+normal vs cancer+normal) | 6-105935915 | cg10111335 | (cancer vs cancer) (cancer+normal vs cancer+normal) |
| 17-81045501 | cg14521546 | (cancer vs cancer) (cancer+normal vs cancer+normal) | 6-11778902 | cg14178895 | (cancer vs cancer) (cancer+normal vs cancer+normal) |
| 18-74832171 | cg21116087 | (normal vs normal) (cancer vs cancer) (cancer+normal vs cancer+normal) | 6-125364093 | cg17993419 | (cancer vs cancer) (cancer+normal vs cancer+normal) |
| 18-74832261 | cg16990945 | (normal vs normal) (cancer vs cancer) (cancer+normal vs cancer+normal) | 6-136679108 | cg25289569 | (normal vs normal) (cancer+normal vs cancer+normal) |
| 19-19104064 | cg06508379 | (cancer vs cancer) (cancer+normal vs cancer+normal) | 6-138747752 | cg21411705 | (cancer+normal vs cancer+normal) |
| 19-45316493 | cg12249345 | (normal vs normal) (cancer vs cancer) (cancer+normal vs cancer+normal) | 6-143132934 | cg10295609 | (cancer+normal vs cancer+normal) |
| 19- | cg21978694 | (normal vs normal) | 6- | cg0552601 | (cancer+normal vs |

| | | | | | |
|---|---|---|---|---|---|
| 45316509 | | (cancer vs cancer) (cancer+normal vs cancer+normal) | 170553845 | 5 | cancer+normal) |
| 19-45316789 | cg22640961 | (cancer vs cancer) (cancer+normal vs cancer+normal) | 6-21754525 | cg00955808 | (normal vs normal) (cancer+normal vs cancer+normal) |
| 19-46404031 | cg07453407 | (cancer vs cancer) (cancer+normal vs cancer+normal) | 6-33384179 | cg08462194 | (normal vs normal) (cancer+normal vs cancer+normal) |
| 2-100061911 | cg09500565 | (cancer vs cancer) (cancer+normal vs cancer+normal) | 6-41714182 | cg17391877 | (normal vs normal) (cancer+normal vs cancer+normal) |
| 2-105695949 | cg25114693 | (cancer vs cancer) (cancer+normal vs cancer+normal) | 7-100883552 | cg14361969 | (cancer+normal vs cancer+normal) |
| 2-231582109 | cg06852824 | (cancer vs cancer) (cancer+normal vs cancer+normal) | 7-1142643 | cg07202610 | (normal vs normal) (cancer+normal vs cancer+normal) |
| 2-233766002 | cg09336899 | (cancer vs cancer) (cancer+normal vs cancer+normal) | 7-1142765 | cg18297960 | (normal vs normal) (cancer+normal vs cancer+normal) |
| 2-238617188 | cg16900589 | (cancer vs cancer) (cancer+normal vs cancer+normal) | 7-1143524 | cg20054412 | (cancer+normal vs cancer+normal) |
| 20-3128566 | cg27556861 | (cancer vs cancer) (cancer+normal vs cancer+normal) | 7-139332087 | cg15535471 | (cancer+normal vs cancer+normal) |
| 20-44439932 | cg01661001 | (cancer vs cancer) (cancer+normal vs cancer+normal) | 7-77552789 | cg05036615 | (normal vs normal) (cancer+normal vs cancer+normal) |
| 22-35694370 | cg27436324 | (cancer vs cancer) (cancer+normal vs cancer+normal) | 7-97935756 | cg15211734 | (normal vs normal) (cancer+normal vs cancer+normal) |
| 3-11634536 | cg08559364 | (cancer vs cancer) (cancer+normal vs cancer+normal) | 7-97935764 | cg10592494 | (normal vs normal) (cancer+normal vs cancer+normal) |
| 3-11666825 | cg07643000 | (cancer vs cancer) (cancer+normal vs cancer+normal) | 8-102083111 | cg03483314 | (cancer vs cancer) (cancer+normal vs cancer+normal) |
| 3-132373398 | cg07460095 | (cancer vs cancer) (cancer+normal vs cancer+normal) | 8-30383628 | cg03796224 | (cancer+normal vs cancer+normal) |
| 3-156955923 | cg10538433 | (cancer vs cancer) (cancer+normal vs cancer+normal) | 8-37518651 | cg26977759 | (cancer+normal vs cancer+normal) |
| 3-171832308 | cg13017471 | (cancer vs cancer) (cancer+normal vs cancer+normal) | 8-37591379 | cg06415302 | (cancer vs cancer) (cancer+normal vs cancer+normal) |
| 3-188745304 | cg13237147 | (cancer vs cancer) (cancer+normal vs cancer+normal) | 8-71279864 | cg10456591 | (cancer+normal vs cancer+normal) |
| 3-196693809 | cg21429725 | (cancer vs cancer) (cancer+normal vs cancer+normal) | 8-86112931 | cg12112870 | (cancer+normal vs cancer+normal) |
| 3-30691493 | cg24719910 | (cancer vs cancer) (cancer+normal vs cancer+normal) | 8-86112946 | cg14145632 | (cancer+normal vs cancer+normal) |
| 3-61889337 | cg24295890 | (cancer vs cancer) (cancer+normal vs cancer+normal) | 9-137255666 | cg14468605 | (cancer+normal vs cancer+normal) |
| 4- | cg16650096 | (cancer vs cancer) | 9-3899055 | cg1426981 | (cancer vs cancer) |

| | | | | | |
|---|---|---|---|---|---|
| 140745554 | | (cancer+normal vs cancer+normal) | | 3 | (cancer+normal vs cancer+normal) |
| 4-149360981 | cg23329208 | (cancer vs cancer) (cancer+normal vs cancer+normal) | 9-96940848 | cg0327564 8 | (cancer+normal vs cancer+normal) |
| 4-15659864 | cg18334727 | (cancer vs cancer) (cancer+normal vs cancer+normal) | **(normal vs normal)** | | |
| 4-169480345 | cg16373817 | (cancer vs cancer) (cancer+normal vs cancer+normal) | **Status - Hyper** | | |
| 4-185317438 | cg00762029 | (cancer vs cancer) (cancer+normal vs cancer+normal) | 1-116381475 | cg2410663 6 | (normal vs normal) |
| 4-185362534 | cg02354388 | (cancer vs cancer) (cancer+normal vs cancer+normal) | 1-236227686 | cg0747967 0 | (normal vs normal) |
| 4-38006787 | cg23289854 | (cancer vs cancer) (cancer+normal vs cancer+normal) | 1-57111117 | cg2606855 1 | (cancer vs cancer) (normal vs normal) (cancer+normal vs cancer+normal) |
| 4-73941755 | cg09428766 | (cancer vs cancer) (cancer+normal vs cancer+normal) | 1-91182777 | cg1832256 9 | (normal vs normal) |
| 4-77953569 | cg14118160 | (cancer vs cancer) (cancer+normal vs cancer+normal) | 10-119000638 | cg1517313 4 | (normal vs normal) |
| 5-139260468 | cg25228995 | (cancer vs cancer) (cancer+normal vs cancer+normal) | 10-131988874 | cg2749175 9 | (normal vs normal) |
| 5-173307471 | cg13384849 | (cancer vs cancer) (cancer+normal vs cancer+normal) | 10-134600998 | cg2548587 5 | (normal vs normal) |
| 5-80530213 | cg16230060 | (cancer vs cancer) (cancer+normal vs cancer+normal) | 10-134901294 | cg0963972 5 | (normal vs normal) |
| 6-100917427 | cg25351606 | (cancer vs cancer) (cancer+normal vs cancer+normal) | 10-134901297 | cg1582578 6 | (normal vs normal) |
| 6-11185142 | cg18202405 | (cancer vs cancer) (cancer+normal vs cancer+normal) | 10-135050779 | cg1800290 9 | (normal vs normal) (cancer+normal vs cancer+normal) |
| 6-170686956 | cg00489213 | (cancer vs cancer) (cancer+normal vs cancer+normal) | 10-46970625 | cg0937303 7 | (normal vs normal) |
| 7-107771148 | cg01550161 | (cancer vs cancer) (cancer+normal vs cancer+normal) | 11-112833773 | cg1204083 0 | (normal vs normal) |
| 7-116272127 | cg23886314 | (cancer vs cancer) (cancer+normal vs cancer+normal) | 11-118023765 | cg1871863 4 | (normal vs normal) |
| 7-127574992 | cg18495781 | (cancer vs cancer) (cancer+normal vs cancer+normal) | 11-7273046 | cg1643772 8 | (normal vs normal) |
| 7-135624343 | cg05600688 | (cancer vs cancer) (cancer+normal vs cancer+normal) | 11-7273148 | cg1856032 8 | (normal vs normal) |
| 7-151335190 | cg11344729 | (cancer vs cancer) (cancer+normal vs cancer+normal) | 12-50297793 | cg0694791 3 | (normal vs normal) |
| 7- | cg20200811 | (cancer vs cancer) | 13- | cg0410196 | (normal vs normal) |

| | | | | | |
|---|---|---|---|---|---|
| 23723014 | | (cancer+normal vs cancer+normal) | 20806477 | 9 | |
| 7-5270512 | cg21585409 | (cancer vs cancer) (cancer+normal vs cancer+normal) | 13-20806539 | cg07148716 | (normal vs normal) |
| 7-5270553 | cg21764632 | (cancer vs cancer) (cancer+normal vs cancer+normal) | 13-20806543 | cg25369560 | (normal vs normal) |
| 7-98667581 | cg09221667 | (cancer vs cancer) (cancer+normal vs cancer+normal) | 14-52734525 | cg05302386 | (normal vs normal) |
| 8-10454154 | cg08775375 | (cancer vs cancer) (cancer+normal vs cancer+normal) | 14-90528983 | cg25225073 | (normal vs normal) |
| 8-109206912 | cg13734860 | (cancer vs cancer) (cancer+normal vs cancer+normal) | 15-29862517 | cg04283162 | (cancer vs cancer) (normal vs normal) |
| 8-124515502 | cg01955203 | (cancer vs cancer) (cancer+normal vs cancer+normal) | 15-98503952 | cg00012522 | (normal vs normal) |
| 8-129073877 | cg24146714 | (cancer vs cancer) (cancer+normal vs cancer+normal) | 15-99193247 | cg03437748 | (normal vs normal) |
| 8-135817117 | cg01954305 | (cancer vs cancer) (cancer+normal vs cancer+normal) | 15-99193929 | cg15460872 | (normal vs normal) |
| 8-142309582 | cg25043496 | (cancer vs cancer) (cancer+normal vs cancer+normal) | 16-1968709 | cg05417127 | (normal vs normal) |
| 8-142309652 | cg10090414 | (cancer vs cancer) (cancer+normal vs cancer+normal) | 16-25703528 | cg27014135 | (normal vs normal) |
| 8-145685564 | cg00718444 | (cancer vs cancer) (cancer+normal vs cancer+normal) | 16-56666334 | cg00916884 | (normal vs normal) |
| 8-6616240 | cg24485696 | (cancer vs cancer) (cancer+normal vs cancer+normal) | 16-67427339 | cg07355841 | (normal vs normal) |
| 9-103062981 | cg14091473 | (cancer vs cancer) (cancer+normal vs cancer+normal) | 16-88450241 | cg06184647 | (normal vs normal) (cancer+normal vs cancer+normal) |
| **KIRC_KIRP (cancer vs cancer)** | | | 16-90114063 | cg01898661 | (normal vs normal) |
| **Status - Hyper** | | | 16-90114173 | cg09012633 | (normal vs normal) |
| 1-1072642 | cg22433276 | (cancer vs cancer) | 17-17399405 | cg18050291 | (normal vs normal) (cancer+normal vs cancer+normal) |
| 1-110026001 | cg08911820 | (cancer vs cancer) (cancer vs cancer) (cancer+normal vs cancer+normal) | 17-31528 | cg18765366 | (normal vs normal) |
| 1-151030981 | cg07139440 | (cancer vs cancer) | 17-42061328 | cg24704730 | (cancer vs cancer) (normal vs normal) |
| 1-54952901 | cg00868131 | (cancer vs cancer) (cancer+normal vs cancer+normal) | 17-44928516 | cg20769177 | (normal vs normal) |
| 1-64558474 | cg21850722 | (cancer vs cancer) (cancer+normal vs cancer+normal) | 17-44928967 | cg08067721 | (normal vs normal) |
| 10- | cg11579421 | (cancer vs cancer) | 17- | cg1463090 | (normal vs normal) |

| | | | | | |
|---|---|---|---|---|---|
| 134211857 | | | 44928992 | 5 | |
| 10-134211908 | cg24085039 | (cancer vs cancer) (cancer+normal vs cancer+normal) | 17-79373381 | cg16560774 | (normal vs normal) (cancer+normal vs cancer+normal) |
| 10-16563821 | cg10982433 | (cancer vs cancer) | 17-79373673 | cg07219667 | (normal vs normal) (cancer+normal vs cancer+normal) |
| 11-101990755 | cg15999356 | (cancer vs cancer) (normal vs normal) | 18-6414978 | cg17688525 | (normal vs normal) (cancer+normal vs cancer+normal) |
| 11-123431162 | cg17637327 | (cancer vs cancer) | 18-74962133 | cg03502002 | (normal vs normal) |
| 11-45918197 | cg15005677 | (cancer vs cancer) | 19-1567517 | cg13647706 | (normal vs normal) |
| 11-84148821 | cg02692785 | (cancer vs cancer) | 19-31841663 | cg11199770 | (cancer vs cancer) (cancer vs cancer) (normal vs normal) (cancer+normal vs cancer+normal) |
| 12-120525338 | cg03800721 | (cancer vs cancer) (cancer vs cancer) (normal vs normal) (cancer+normal vs cancer+normal) | 19-33685817 | cg10276227 | (normal vs normal) |
| 12-121088408 | cg25969992 | (cancer vs cancer) (cancer vs cancer) (cancer+normal vs cancer+normal) | 19-460934 | cg04148794 | (normal vs normal) |
| 12-52214119 | cg19495013 | (cancer vs cancer) (cancer vs cancer) (cancer+normal vs cancer+normal) | 19-822086 | cg21928701 | (normal vs normal) |
| 12-52214320 | cg07375978 | (cancer vs cancer) (cancer vs cancer) (cancer+normal vs cancer+normal) | 2-115919009 | cg21678377 | (normal vs normal) |
| 12-52214758 | cg05238741 | (cancer vs cancer) (cancer+normal vs cancer+normal) | 2-176994764 | cg24416513 | (normal vs normal) |
| 12-54422127 | cg12232783 | (cancer vs cancer) (cancer vs cancer) (cancer+normal vs cancer+normal) | 2-177053292 | cg05099387 | (normal vs normal) |
| 12-54422239 | cg04576672 | (cancer vs cancer) (normal vs normal) (cancer+normal vs cancer+normal) | 2-27341795 | cg10660909 | (normal vs normal) |
| 12-54422350 | cg27441225 | (cancer vs cancer) (cancer vs cancer) (cancer+normal vs cancer+normal) | 20-13200944 | cg14060111 | (normal vs normal) (cancer+normal vs cancer+normal) |
| 12-54422447 | cg11941633 | (cancer vs cancer) | 20-13200954 | cg12664209 | (normal vs normal) (cancer+normal vs cancer+normal) |
| 12-54422488 | cg22378817 | (cancer vs cancer) (cancer vs cancer) (cancer+normal vs cancer+normal) | 20-13200980 | cg09491991 | (cancer vs cancer) (normal vs normal) (cancer+normal vs cancer+normal) |
| 12-54427293 | cg00576279 | (cancer vs cancer) | 20-25566178 | cg12358000 | (normal vs normal) |
| 12- | cg16983211 | (cancer vs cancer) | 20- | cg1266446 | (normal vs normal) |

| | | | | | |
|---|---|---|---|---|---|
| 54427636 | | (cancer vs cancer) (cancer+normal vs cancer+normal) | 61051021 | 4 | |
| 12-54427700 | cg15700739 | (cancer vs cancer) (cancer vs cancer) (cancer+normal vs cancer+normal) | 20-61051032 | cg20265733 | (cancer vs cancer) (normal vs normal) |
| 12-54446505 | cg03416628 | (cancer vs cancer) (cancer+normal vs cancer+normal) | 20-61051036 | cg02484469 | (normal vs normal) |
| 12-54446537 | cg25975485 | (cancer vs cancer) | 20-61051039 | cg14980983 | (cancer vs cancer) (normal vs normal) |
| 12-54446576 | cg08712054 | (cancer vs cancer) (cancer+normal vs cancer+normal) | 21-34442377 | cg20340508 | (normal vs normal) |
| 12-97857511 | cg03782861 | (cancer vs cancer) (cancer+normal vs cancer+normal) | 3-128720884 | cg02604524 | (normal vs normal) |
| 12-99287876 | cg01473955 | (cancer vs cancer) | 3-158450251 | cg14226182 | (normal vs normal) |
| 13-113598068 | cg01866427 | (cancer vs cancer) (cancer vs cancer) (cancer+normal vs cancer+normal) | 3-181414237 | cg21453451 | (normal vs normal) |
| 13-113623233 | cg02223067 | (cancer vs cancer) (cancer vs cancer) (cancer+normal vs cancer+normal) | 3-184287196 | cg17292583 | (normal vs normal) |
| 13-113623300 | cg16737670 | (cancer vs cancer) (cancer vs cancer) (cancer+normal vs cancer+normal) | 4-11430717 | cg20075156 | (normal vs normal) |
| 13-113623639 | cg00127894 | (cancer vs cancer) (cancer vs cancer) (normal vs normal) (cancer+normal vs cancer+normal) | 4-118006619 | cg21599230 | (normal vs normal) |
| 13-113623659 | cg15179805 | (cancer vs cancer) (cancer vs cancer) (cancer+normal vs cancer+normal) | 4-119274013 | cg17632579 | (normal vs normal) |
| 13-113623844 | cg09678836 | (cancer vs cancer) (cancer vs cancer) (normal vs normal) (cancer+normal vs cancer+normal) | 4-141490037 | cg02268229 | (normal vs normal) |
| 17-18908220 | cg05090106 | (cancer vs cancer) (cancer+normal vs cancer+normal) | 4-170947395 | cg20710394 | (normal vs normal) |
| 17-18908235 | cg14807365 | (cancer vs cancer) (cancer vs cancer) (cancer+normal vs cancer+normal) | 4-183370059 | cg02422426 | (normal vs normal) (cancer+normal vs cancer+normal) |
| 17-18908242 | cg22220165 | (cancer vs cancer) (cancer vs cancer) (cancer+normal vs cancer+normal) | 4-183370138 | cg09767602 | (cancer vs cancer) (normal vs normal) (cancer+normal vs cancer+normal) |
| 17-35289543 | cg16293732 | (cancer vs cancer) (cancer vs cancer) (cancer+normal vs cancer+normal) | 4-25032556 | cg15379354 | (normal vs normal) |

326

| | | | | | |
|---|---|---|---|---|---|
| 17-35290307 | cg06094642 | (cancer vs cancer) | 4-37455485 | cg26306909 | (normal vs normal) |
| 17-35290458 | cg05279092 | (cancer vs cancer) (cancer+normal vs cancer+normal) | 5-11904114 | cg07195011 | (normal vs normal) |
| 17-35290523 | cg12655190 | (cancer vs cancer) | 5-132947725 | cg24908814 | (normal vs normal) |
| 17-35290635 | cg19502920 | (cancer vs cancer) (cancer+normal vs cancer+normal) | 5-148033708 | cg12825070 | (normal vs normal) |
| 17-35290822 | cg17231347 | (cancer vs cancer) | 5-42423947 | cg12042587 | (cancer vs cancer) (cancer vs cancer) (normal vs normal) (cancer+normal vs cancer+normal) |
| 17-35297752 | cg07103093 | (cancer vs cancer) (cancer+normal vs cancer+normal) | 5-50694985 | cg15814504 | (normal vs normal) |
| 17-35300874 | cg04588165 | (cancer vs cancer) (normal vs normal) (cancer+normal vs cancer+normal) | 5-72512115 | cg26590003 | (normal vs normal) |
| 17-46697115 | cg23349301 | (cancer vs cancer) | 6-146864781 | cg10766475 | (normal vs normal) (cancer+normal vs cancer+normal) |
| 17-46704410 | cg24042517 | (cancer vs cancer) (cancer+normal vs cancer+normal) | 6-21664652 | cg24428877 | (cancer vs cancer) (cancer vs cancer) (normal vs normal) (cancer+normal vs cancer+normal) |
| 17-55056066 | cg10342722 | (cancer vs cancer) (cancer+normal vs cancer+normal) | 6-30043194 | cg23027574 | (normal vs normal) |
| 17-5673550 | cg06683719 | (cancer vs cancer) (cancer vs cancer) | 6-42928497 | cg05552183 | (cancer vs cancer) (normal vs normal) |
| 17-70498417 | cg25753722 | (cancer vs cancer) | 6-42928500 | cg14338887 | (normal vs normal) |
| 17-7790179 | cg21068610 | (cancer vs cancer) (cancer vs cancer) (cancer+normal vs cancer+normal) | 7-121946036 | cg10485664 | (normal vs normal) |
| 19-11199917 | cg18596381 | (cancer vs cancer) (cancer vs cancer) (cancer+normal vs cancer+normal) | 7-123673331 | cg17025835 | (normal vs normal) |
| 19-11199944 | cg19751789 | (cancer vs cancer) (cancer vs cancer) (cancer+normal vs cancer+normal) | 7-139168484 | cg15506157 | (cancer vs cancer) (normal vs normal) |
| 19-11199965 | cg12013591 | (cancer vs cancer) (cancer vs cancer) (cancer+normal vs cancer+normal) | 7-38671001 | cg26122980 | (normal vs normal) |
| 2-10263480 | cg00506866 | (cancer vs cancer) (cancer vs cancer) (cancer+normal vs cancer+normal) | 7-89748686 | cg03098837 | (normal vs normal) |
| 2-105374995 | cg22365240 | (cancer vs cancer) | 8-105601615 | cg04413895 | (cancer vs cancer) (normal vs normal) (cancer+normal vs cancer+normal) |

| | | | | | |
|---|---|---|---|---|---|
| 2-134024618 | cg09597470 | (cancer vs cancer) | 8-120220799 | cg14338425 | (normal vs normal) |
| 2-21856440 | cg01598144 | (cancer vs cancer) (cancer+normal vs cancer+normal) | 8-13134166 | cg20536983 | (cancer vs cancer) (normal vs normal) (cancer+normal vs cancer+normal) |
| 2-238769060 | cg17845266 | (cancer vs cancer) | 8-26371551 | cg24410546 | (normal vs normal) |
| 2-72372965 | cg08863459 | (cancer vs cancer) (cancer+normal vs cancer+normal) | 8-38965252 | cg02637318 | (normal vs normal) |
| 2-73151201 | cg04965934 | (cancer vs cancer) | 8-74005562 | cg18454916 | (normal vs normal) |
| 2-73151595 | cg16781647 | (cancer vs cancer) (normal vs normal) | 8-76320150 | cg26220018 | (normal vs normal) |
| 2-73151879 | cg00486564 | (cancer vs cancer) (cancer+normal vs cancer+normal) | 9-139085579 | cg04455058 | (normal vs normal) |
| 2-73151966 | cg15270150 | (cancer vs cancer) | 9-90112711 | cg20401521 | (normal vs normal) |
| 3-132036382 | cg00589251 | (cancer vs cancer) (cancer vs cancer) (cancer+normal vs cancer+normal) | 9-90589530 | cg13685727 | (normal vs normal) |
| 4-111530471 | cg15903185 | (cancer vs cancer) (cancer vs cancer) (cancer+normal vs cancer+normal) | 9-95947146 | cg10272601 | (normal vs normal) |
| 4-14113465 | cg01612366 | (cancer vs cancer) (cancer+normal vs cancer+normal) | **Status - Hypo** | | |
| 5-149682233 | cg25780607 | (cancer vs cancer) | 1-155881944 | cg24337215 | (normal vs normal) (cancer vs cancer) |
| 5-475085 | cg15084390 | (cancer vs cancer) (cancer+normal vs cancer+normal) | 1-162219640 | cg21692434 | (normal vs normal) (cancer+normal vs cancer+normal) |
| 5-475205 | cg21743597 | (cancer vs cancer) (cancer+normal vs cancer+normal) | 1-192544716 | cg10861751 | (normal vs normal) |
| 5-58335271 | cg18242030 | (cancer vs cancer) (normal vs normal) | 1-204329775 | cg18865257 | (normal vs normal) |
| 5-58335681 | cg22078451 | (cancer vs cancer) (normal vs normal) | 1-206317854 | cg02290880 | (normal vs normal) |
| 5-66299786 | cg02632185 | (cancer vs cancer) (cancer vs cancer) (normal vs normal) (cancer+normal vs cancer+normal) | 1-211384247 | cg16140794 | (normal vs normal) |
| 5-66299875 | cg18118497 | (cancer vs cancer) (cancer vs cancer) (cancer+normal vs cancer+normal) | 1-248852044 | cg15133201 | (normal vs normal) |
| 5-66300406 | cg17449851 | (cancer vs cancer) (cancer vs cancer) (normal vs normal) (cancer+normal vs cancer+normal) | 1-25291905 | cg09993145 | (normal vs normal) |
| 5-66300433 | cg11831286 | (cancer vs cancer) (cancer vs cancer) (normal vs normal) (cancer+normal vs | 1-26083702 | cg19897113 | (normal vs normal) |

| | | | | | |
|---|---|---|---|---|---|
| | | cancer+normal) | | | |
| 5-66300503 | cg27116842 | (cancer vs cancer)<br>(cancer vs cancer)<br>(normal vs normal)<br>(cancer+normal vs cancer+normal) | 1-27480668 | cg22481448 | (normal vs normal) |
| 6-26440101 | cg01269795 | (cancer vs cancer)<br>(cancer+normal vs cancer+normal) | 1-68209155 | cg11209521 | (normal vs normal)<br>(cancer+normal vs cancer+normal) |
| 7-1271604 | cg07074316 | (cancer vs cancer) | 1-7201539 | cg21443133 | (normal vs normal) |
| 7-1288184 | cg16275882 | (cancer vs cancer)<br>(cancer+normal vs cancer+normal) | 10-14603607 | cg14884828 | (normal vs normal) |
| 7-151328093 | cg19412964 | (cancer vs cancer)<br>(cancer+normal vs cancer+normal) | 10-34604934 | cg13976324 | (normal vs normal)<br>(cancer+normal vs cancer+normal) |
| 7-1708823 | cg17202717 | (cancer vs cancer)<br>(cancer+normal vs cancer+normal) | 11-110198965 | cg10264003 | (normal vs normal) |
| 7-92747308 | cg08986767 | (cancer vs cancer)<br>(cancer vs cancer)<br>(cancer+normal vs cancer+normal) | 11-3240068 | cg24702253 | (normal vs normal) |
| 8-107669778 | cg07554329 | (cancer vs cancer)<br>(cancer vs cancer)<br>(cancer+normal vs cancer+normal) | 11-48041783 | cg01372366 | (normal vs normal) |
| 8-107669783 | cg17136799 | (cancer vs cancer)<br>(cancer vs cancer)<br>(cancer+normal vs cancer+normal) | 11-59634276 | cg03928812 | (normal vs normal) |
| 8-107669787 | cg26622232 | (cancer vs cancer)<br>(cancer vs cancer)<br>(cancer+normal vs cancer+normal) | 11-6767826 | cg24135977 | (normal vs normal) |
| 8-107669856 | cg17176732 | (cancer vs cancer)<br>(cancer vs cancer)<br>(cancer+normal vs cancer+normal) | 11-72759293 | cg13912027 | (normal vs normal) |
| 8-107669906 | cg17212289 | (cancer vs cancer)<br>(cancer vs cancer)<br>(cancer+normal vs cancer+normal) | 11-76818702 | cg19164997 | (normal vs normal) |
| 8-107670161 | cg16326979 | (cancer vs cancer)<br>(cancer vs cancer)<br>(cancer+normal vs cancer+normal) | 11-811669 | cg25872852 | (normal vs normal)<br>(cancer+normal vs cancer+normal) |
| 8-107670358 | cg00298796 | (cancer vs cancer)<br>(cancer+normal vs cancer+normal) | 12-131576088 | cg15278374 | (normal vs normal) |
| 8-55294536 | cg19413417 | (cancer vs cancer) | 12-15114904 | cg08251901 | (normal vs normal) |
| 8-55294610 | cg13242160 | (cancer vs cancer)<br>(cancer vs cancer)<br>(cancer+normal vs cancer+normal) | 12-4023078 | cg21129006 | (normal vs normal) |
| 8-55294721 | cg07664990 | (cancer vs cancer)<br>(cancer vs cancer)<br>(cancer+normal vs cancer+normal) | 12-52610273 | cg19959477 | (normal vs normal)<br>(cancer+normal vs cancer+normal) |

| | | | | | |
|---|---|---|---|---|---|
| 8-59714165 | cg25766247 | (cancer vs cancer) (cancer+normal vs cancer+normal) | 12-70763703 | cg04609875 | (normal vs normal) |
| 8-9760427 | cg15526246 | (cancer vs cancer) | 14-75988747 | cg15645309 | (normal vs normal) |
| 8-9760528 | cg00528902 | (cancer vs cancer) (cancer+normal vs cancer+normal) | 15-31781955 | cg04967982 | (normal vs normal) |
| 8-9761905 | cg06292304 | (cancer vs cancer) | 15-95803603 | cg07695058 | (normal vs normal) |
| 9-79320026 | cg13914324 | (cancer vs cancer) (cancer+normal vs cancer+normal) | 16-84628969 | cg03738384 | (normal vs normal) |
| **Status - Hypo** | | | 16-85009995 | cg16671652 | (normal vs normal) |
| 1-101094117 | cg00902464 | (cancer vs cancer) (cancer+normal vs cancer+normal) | 17-684490 | cg17560340 | (normal vs normal) |
| 1-180278787 | cg17475583 | (cancer vs cancer) | 17-81006524 | cg10344477 | (normal vs normal) |
| 1-2026193 | cg22712840 | (cancer vs cancer) (cancer+normal vs cancer+normal) | 18-77283493 | cg22324981 | (normal vs normal) (cancer+normal vs cancer+normal) |
| 1-205114634 | cg15542880 | (cancer vs cancer) | 18-8364304 | cg14579769 | (normal vs normal) |
| 1-217724849 | cg10325088 | (cancer vs cancer) (cancer vs cancer) (cancer+normal vs cancer+normal) | 2-103095638 | cg03405781 | (normal vs normal) (cancer+normal vs cancer+normal) |
| 1-2427655 | cg10219816 | (cancer vs cancer) (cancer+normal vs cancer+normal) | 2-10686975 | cg13851334 | (normal vs normal) |
| 1-25069665 | cg12081359 | (cancer vs cancer) (cancer+normal vs cancer+normal) | 2-10687042 | cg09761224 | (normal vs normal) |
| 1-2996949 | cg22506548 | (cancer vs cancer) (cancer+normal vs cancer+normal) | 2-152991620 | cg20625334 | (normal vs normal) |
| 1-3162454 | cg12800122 | (cancer vs cancer) (cancer+normal vs cancer+normal) | 2-158300475 | cg12177677 | (normal vs normal) |
| 1-47656853 | cg15187606 | (cancer vs cancer) (cancer+normal vs cancer+normal) | 2-158300485 | cg10559416 | (normal vs normal) |
| 1-54195800 | cg17904129 | (cancer vs cancer) (cancer+normal vs cancer+normal) | 2-158301005 | cg03915055 | (normal vs normal) |
| 1-54195830 | cg27311970 | (cancer vs cancer) (cancer+normal vs cancer+normal) | 2-203044287 | cg21074959 | (normal vs normal) |
| 1-55317286 | cg25011368 | (cancer vs cancer) (cancer+normal vs cancer+normal) | 2-242483467 | cg06469726 | (normal vs normal) |
| 1-81001617 | cg11832870 | (cancer vs cancer) (cancer+normal vs cancer+normal) | 2-33500889 | cg21846949 | (normal vs normal) |
| 11-64323816 | cg21039563 | (cancer vs cancer) (cancer vs cancer) (cancer+normal vs cancer+normal) | 2-68348796 | cg10099758 | (normal vs normal) |
| 12- | cg14400030 | (cancer vs cancer) | 20- | cg0584053 | (normal vs normal) |

| | | | | | |
|---|---|---|---|---|---|
| 122498111 | | (cancer+normal vs cancer+normal) | 31755943 | 3 | |
| 12-129298690 | cg23521905 | (cancer vs cancer) | 20-62562848 | cg15457037 | (normal vs normal) (cancer vs cancer) |
| 12-1609346 | cg00230120 | (cancer vs cancer) (cancer+normal vs cancer+normal) | 3-124304960 | cg04915618 | (normal vs normal) |
| 12-40622688 | cg12664938 | (cancer vs cancer) | 3-137717665 | cg24894531 | (normal vs normal) (cancer vs cancer) |
| 12-7121914 | cg24859375 | (cancer vs cancer) (cancer vs cancer) (cancer+normal vs cancer+normal) | 3-137728329 | cg00405112 | (normal vs normal) |
| 13-111880998 | cg21980364 | (cancer vs cancer) (cancer vs cancer) (cancer+normal vs cancer+normal) | 3-137892432 | cg00933443 | (normal vs normal) (cancer vs cancer) (cancer+normal vs cancer+normal) |
| 13-114138019 | cg05508408 | (cancer vs cancer) (cancer+normal vs cancer+normal) | 3-149832476 | cg27316567 | (normal vs normal) (cancer+normal vs cancer+normal) |
| 13-114138260 | cg21238257 | (cancer vs cancer) (cancer vs cancer) (cancer+normal vs cancer+normal) | 3-169896616 | cg03603669 | (normal vs normal) |
| 13-30074671 | cg24215635 | (cancer vs cancer) | 3-194868750 | cg21937377 | (normal vs normal) (cancer vs cancer) (cancer vs cancer) (cancer+normal vs cancer+normal) |
| 13-96086098 | cg20278383 | (normal vs normal) (cancer vs cancer) (cancer vs cancer) (cancer+normal vs cancer+normal) | 3-41121968 | cg24901488 | (normal vs normal) |
| 13-96086249 | cg26557005 | (cancer vs cancer) (cancer+normal vs cancer+normal) | 3-54962954 | cg02810692 | (normal vs normal) |
| 13-97980712 | cg11862665 | (cancer vs cancer) (cancer+normal vs cancer+normal) | 3-65755382 | cg21550580 | (normal vs normal) |
| 14-73709663 | cg01655958 | (cancer vs cancer) | 4-22476976 | cg05029312 | (normal vs normal) |
| 14-89628141 | cg02212698 | (cancer vs cancer) (cancer+normal vs cancer+normal) | 4-2439731 | cg26605046 | (normal vs normal) (cancer vs cancer) (cancer+normal vs cancer+normal) |
| 14-89628169 | cg21898844 | (cancer vs cancer) | 4-2814064 | cg12086464 | (normal vs normal) |
| 14-91275103 | cg18518804 | (cancer vs cancer) (cancer+normal vs cancer+normal) | 4-2814122 | cg15569052 | (normal vs normal) |
| 14-99893325 | cg24180006 | (cancer vs cancer) (cancer+normal vs cancer+normal) | 4-76596784 | cg03862987 | (normal vs normal) |
| 15-64525039 | cg21617870 | (cancer vs cancer) | 4-95335239 | cg24557027 | (normal vs normal) (cancer+normal vs cancer+normal) |
| 15-85520317 | cg01156844 | (cancer vs cancer) | 5-118609467 | cg04855216 | (normal vs normal) |
| 16-15127121 | cg03928410 | (cancer vs cancer) | 5-132698273 | cg21176603 | (normal vs normal) |

| | | | | | |
|---|---|---|---|---|---|
| 16-15127172 | cg03245889 | (cancer vs cancer) (cancer+normal vs cancer+normal) | 5-1342200 | cg1473363 7 | (normal vs normal) (cancer+normal vs cancer+normal) |
| 16-15127193 | cg06978461 | (cancer vs cancer) (cancer vs cancer) (cancer+normal vs cancer+normal) | 5-139079288 | cg2555135 8 | (normal vs normal) (cancer+normal vs cancer+normal) |
| 16-54146616 | cg05967340 | (cancer vs cancer) | 5-1489889 | cg1627298 1 | (normal vs normal) (cancer+normal vs cancer+normal) |
| 16-78323024 | cg00879840 | (cancer vs cancer) (cancer+normal vs cancer+normal) | 5-169617918 | cg2696772 3 | (normal vs normal) |
| 16-81565917 | cg03212183 | (cancer vs cancer) (cancer vs cancer) (cancer+normal vs cancer+normal) | 5-38607402 | cg1397698 8 | (normal vs normal) |
| 17-18854760 | cg18449048 | (cancer vs cancer) (cancer vs cancer) (cancer+normal vs cancer+normal) | 5-57787167 | cg2645316 9 | (normal vs normal) |
| 17-18855313 | cg00306284 | (normal vs normal) (cancer vs cancer) (cancer+normal vs cancer+normal) | 6-105750581 | cg0535766 0 | (normal vs normal) |
| 17-18855506 | cg09632029 | (normal vs normal) (cancer vs cancer) (cancer+normal vs cancer+normal) | 6-119032202 | cg1016439 3 | (normal vs normal) |
| 17-40064155 | cg03950716 | (cancer vs cancer) (cancer+normal vs cancer+normal) | 6-13274314 | cg0791292 2 | (normal vs normal) |
| 17-4510743 | cg20818528 | (cancer vs cancer) (cancer+normal vs cancer+normal) | 6-136679108 | cg2528956 9 | (normal vs normal) (cancer+normal vs cancer+normal) |
| 17-4520467 | cg00349608 | (cancer vs cancer) (cancer+normal vs cancer+normal) | 6-148020881 | cg2077819 9 | (normal vs normal) |
| 17-46871905 | cg08137716 | (cancer vs cancer) (cancer+normal vs cancer+normal) | 6-167508041 | cg2183921 1 | (normal vs normal) |
| 17-7225013 | cg09777416 | (cancer vs cancer) (cancer+normal vs cancer+normal) | 6-167508053 | cg1615136 2 | (normal vs normal) |
| 17-78700928 | cg06975080 | (cancer vs cancer) (cancer vs cancer) (cancer+normal vs cancer+normal) | 6-168107203 | cg0886437 5 | (normal vs normal) |
| 17-78701084 | cg10790704 | (cancer vs cancer) | 6-21754525 | cg0095580 8 | (normal vs normal) (cancer+normal vs cancer+normal) |
| 19-13198960 | cg11883009 | (cancer vs cancer) (cancer+normal vs cancer+normal) | 6-32028946 | cg0954889 3 | (normal vs normal) |
| 19-1424658 | cg11656553 | (cancer vs cancer) (cancer+normal vs cancer+normal) | 6-32029152 | cg0089810 2 | (normal vs normal) |
| 2-127811853 | cg01441308 | (cancer vs cancer) (cancer+normal vs cancer+normal) | 6-33384179 | cg0846219 4 | (normal vs normal) (cancer+normal vs cancer+normal) |
| 2- | cg26015513 | (cancer vs cancer) | 6-41703872 | cg1449002 | (normal vs normal) |

| | | | | | |
|---|---|---|---|---|---|
| 201218948 | | (cancer vs cancer) (cancer+normal vs cancer+normal) | | 5 | |
| 2-213950646 | cg05148385 | (cancer vs cancer) (cancer+normal vs cancer+normal) | 6-41714182 | cg17391877 | (normal vs normal) (cancer+normal vs cancer+normal) |
| 2-217403190 | cg09473180 | (cancer vs cancer) (cancer vs cancer) (cancer+normal vs cancer+normal) | 6-49833873 | cg10632722 | (normal vs normal) |
| 2-223806495 | cg02116856 | (cancer vs cancer) | 6-56711206 | cg10784258 | (normal vs normal) |
| 2-224824723 | cg17963548 | (cancer vs cancer) (cancer+normal vs cancer+normal) | 7-1142643 | cg07202610 | (normal vs normal) (cancer+normal vs cancer+normal) |
| 2-75786714 | cg16720807 | (cancer vs cancer) | 7-1142765 | cg18297960 | (normal vs normal) (cancer+normal vs cancer+normal) |
| 20-16289592 | cg15821605 | (cancer vs cancer) | 7-23827492 | cg15920472 | (normal vs normal) |
| 20-6025272 | cg21209310 | (cancer vs cancer) (cancer vs cancer) (cancer+normal vs cancer+normal) | 7-77552789 | cg05036615 | (normal vs normal) (cancer+normal vs cancer+normal) |
| 22-24119331 | cg27598806 | (cancer vs cancer) (cancer+normal vs cancer+normal) | 7-97935756 | cg15211734 | (normal vs normal) (cancer+normal vs cancer+normal) |
| 3-126010451 | cg11423909 | (cancer vs cancer) (cancer+normal vs cancer+normal) | 7-97935764 | cg10592494 | (normal vs normal) (cancer+normal vs cancer+normal) |
| 3-149051031 | cg11761535 | (cancer vs cancer) | 8-20350779 | cg10145533 | (normal vs normal) |
| 3-149051159 | cg11011913 | (cancer vs cancer) (cancer vs cancer) (cancer+normal vs cancer+normal) | 8-67587258 | cg17253785 | (normal vs normal) |
| 3-171976122 | cg13050884 | (cancer vs cancer) (cancer+normal vs cancer+normal) | 8-82021210 | cg02520639 | (normal vs normal) |
| 3-183894727 | cg16301768 | (cancer vs cancer) (cancer+normal vs cancer+normal) | 8-82784211 | cg01068332 | (normal vs normal) |
| 3-65342843 | cg14533390 | (cancer vs cancer) | 9-40536407 | cg14443606 | (normal vs normal) |
| 4-72205104 | cg19850370 | (cancer vs cancer) (cancer+normal vs cancer+normal) | **THCA (cancer vs cancer)** | | |
| 4-87993109 | cg27000934 | (cancer vs cancer) (cancer vs cancer) (cancer+normal vs cancer+normal) | **Status - Hyper** | | |
| 5-111017556 | cg01885839 | (cancer vs cancer) (cancer+normal vs cancer+normal) | 1-109634248 | cg12080882 | (cancer vs cancer) |
| 5-131607888 | cg20547724 | (cancer vs cancer) (cancer+normal vs cancer+normal) | 1-155828926 | cg06440960 | (cancer vs cancer) (cancer+normal vs cancer+normal) |
| 5-1337747 | cg10641823 | (cancer vs cancer) (cancer+normal vs cancer+normal) | 1-16160360 | cg05960182 | (cancer vs cancer) (normal vs normal) (cancer+normal vs cancer+normal) |

| | | | | | |
|---|---|---|---|---|---|
| 5-135479136 | cg07033790 | (cancer vs cancer) | 1-190446756 | cg19632206 | (cancer vs cancer) (cancer+normal vs cancer+normal) |
| 5-142527658 | cg26547816 | (cancer vs cancer) (cancer+normal vs cancer+normal) | 1-228644750 | cg00146645 | (cancer vs cancer) |
| 5-156485233 | cg07320595 | (cancer vs cancer) | 1-7830855 | cg12072178 | (cancer vs cancer) (cancer+normal vs cancer+normal) |
| 5-7873542 | cg15108958 | (cancer vs cancer) | 10-11239482 | cg19548859 | (cancer vs cancer) |
| 6-160680162 | cg25545503 | (normal vs normal) (cancer vs cancer) (cancer+normal vs cancer+normal) | 10-3514783 | cg13687834 | (cancer vs cancer) (cancer+normal vs cancer+normal) |
| 7-101556684 | cg15600935 | (cancer vs cancer) (cancer vs cancer) (cancer+normal vs cancer+normal) | 10-5136782 | cg12530994 | (cancer vs cancer) (cancer+normal vs cancer+normal) |
| 7-101566857 | cg02307880 | (cancer vs cancer) (cancer+normal vs cancer+normal) | 11-102401616 | cg01813071 | (cancer vs cancer) (normal vs normal) (cancer+normal vs cancer+normal) |
| 7-1120141 | cg02112681 | (cancer vs cancer) (cancer+normal vs cancer+normal) | 11-118437129 | cg26954138 | (cancer vs cancer) (cancer+normal vs cancer+normal) |
| 7-1141341 | cg07242429 | (cancer vs cancer) | 11-118437133 | cg20874052 | (cancer vs cancer) (cancer+normal vs cancer+normal) |
| 7-1142433 | cg00364696 | (cancer vs cancer) (cancer+normal vs cancer+normal) | 11-121567477 | cg01195276 | (cancer vs cancer) (cancer+normal vs cancer+normal) |
| 7-2679148 | cg22788953 | (cancer vs cancer) | 11-126173548 | cg22911867 | (cancer vs cancer) (cancer+normal vs cancer+normal) |
| 7-2679726 | cg22239213 | (cancer vs cancer) (cancer+normal vs cancer+normal) | 11-131941989 | cg16763885 | (cancer vs cancer) (cancer+normal vs cancer+normal) |
| 7-2679971 | cg16184737 | (cancer vs cancer) (cancer+normal vs cancer+normal) | 11-17251956 | cg26906217 | (cancer vs cancer) (cancer+normal vs cancer+normal) |
| 7-4786364 | cg01808969 | (cancer vs cancer) | 11-407180 | cg08017880 | (cancer vs cancer) |
| 8-141147612 | cg02225988 | (cancer vs cancer) (cancer+normal vs cancer+normal) | 11-45433358 | cg15355111 | (cancer vs cancer) (cancer+normal vs cancer+normal) |
| 8-141147661 | cg22504031 | (cancer vs cancer) | 11-57424773 | cg27258272 | (cancer vs cancer) (cancer+normal vs cancer+normal) |
| 8-141147681 | cg17018549 | (cancer vs cancer) (cancer vs cancer) (cancer+normal vs cancer+normal) | 11-65487505 | cg06038201 | (cancer vs cancer) (normal vs normal) (cancer+normal vs cancer+normal) |
| 8-142311072 | cg18818267 | (cancer vs cancer) (cancer+normal vs cancer+normal) | 11-96123810 | cg03075605 | (cancer vs cancer) (cancer+normal vs cancer+normal) |
| 8-144601734 | cg19598713 | (cancer vs cancer) (cancer+normal vs cancer+normal) | 12-48100286 | cg05731122 | (cancer vs cancer) (cancer+normal vs cancer+normal) |
| 8-144601800 | cg09580859 | (cancer vs cancer) (cancer+normal vs | 12-51818049 | cg18077304 | (cancer vs cancer) |

| | | | | | |
|---|---|---|---|---|---|
| 8-144601851 | cg25900150 | (cancer vs cancer) (cancer+normal vs cancer+normal) | 12-65066768 | cg13042575 | (cancer vs cancer) (cancer+normal vs cancer+normal) |
| 8-144601883 | cg00783706 | (cancer vs cancer) (cancer+normal vs cancer+normal) | 12-78224480 | cg20217872 | (cancer vs cancer) (cancer+normal vs cancer+normal) |
| 8-30209371 | cg02723975 | (cancer vs cancer) (cancer+normal vs cancer+normal) | 12-93834066 | cg11084718 | (cancer vs cancer) |
| 8-30209386 | cg24296569 | (cancer vs cancer) | 13-20699452 | cg05585947 | (cancer vs cancer) (normal vs normal) (cancer+normal vs cancer+normal) |
| 8-30209391 | cg14895559 | (cancer vs cancer) (cancer vs cancer) (cancer+normal vs cancer+normal) | 13-53191046 | cg02215357 | (cancer vs cancer) (cancer+normal vs cancer+normal) |
| 8-66701194 | cg26266308 | (cancer vs cancer) (cancer+normal vs cancer+normal) | 13-92001764 | cg17799287 | (cancer vs cancer) (cancer+normal vs cancer+normal) |
| 9-4541807 | cg13599596 | (cancer vs cancer) | 13-92001959 | cg07641807 | (cancer vs cancer) (cancer+normal vs cancer+normal) |
| **(cancer+normal vs cancer+normal)** | | | 14-31698967 | cg10348234 | (cancer vs cancer) (cancer+normal vs cancer+normal) |
| **Status - Hyper** | | | 14-31699236 | cg18437792 | (cancer vs cancer) (cancer+normal vs cancer+normal) |
| 1-110026001 | cg08911820 | (cancer vs cancer) (cancer vs cancer) (cancer+normal vs cancer+normal) | 14-51327604 | cg19590421 | (cancer vs cancer) (cancer+normal vs cancer+normal) |
| 1-201083145 | cg22815214 | (cancer+normal vs cancer+normal) | 14-56046001 | cg27312312 | (cancer vs cancer) (normal vs normal) |
| 1-29448425 | cg03315764 | (cancer+normal vs cancer+normal) | 15-56286575 | cg13160251 | (cancer vs cancer) |
| 1-54952901 | cg00868131 | (cancer vs cancer) (cancer+normal vs cancer+normal) | 15-60883225 | cg00200653 | (cancer vs cancer) (normal vs normal) (cancer+normal vs cancer+normal) |
| 1-64558474 | cg21850722 | (cancer vs cancer) (cancer+normal vs cancer+normal) | 16-2058572 | cg00852033 | (cancer vs cancer) (cancer+normal vs cancer+normal) |
| 10-134211908 | cg24085039 | (cancer vs cancer) (cancer+normal vs cancer+normal) | 17-47072339 | cg22434594 | (cancer vs cancer) (cancer+normal vs cancer+normal) |
| 10-44148437 | cg07121693 | (cancer+normal vs cancer+normal) | 17-48785992 | cg06204730 | (cancer vs cancer) (cancer+normal vs cancer+normal) |
| 11-114832606 | cg07026904 | (cancer+normal vs cancer+normal) | 17-66452343 | cg25161129 | (cancer vs cancer) (cancer+normal vs cancer+normal) |
| 11-59015338 | cg27471192 | (cancer+normal vs cancer+normal) | 17-74248466 | cg24166450 | (cancer vs cancer) (cancer+normal vs cancer+normal) |
| 11-66821665 | cg01717649 | (cancer+normal vs cancer+normal) | 19-16189693 | cg27377863 | (cancer vs cancer) (cancer+normal vs cancer+normal) |
| 12- | cg03800721 | (cancer vs cancer) | 2-16790314 | cg1948284 | (cancer vs cancer) |

335

| | | | | | |
|---|---|---|---|---|---|
| 120525338 | | (cancer vs cancer) (normal vs normal) (cancer+normal vs cancer+normal) | | 2 | (cancer+normal vs cancer+normal) |
| 12-121088408 | cg25969992 | (cancer vs cancer) (cancer vs cancer) (cancer+normal vs cancer+normal) | 2-172968684 | cg20434178 | (cancer vs cancer) |
| 12-125831167 | cg19322788 | (cancer+normal vs cancer+normal) | 2-178104794 | cg21382890 | (cancer vs cancer) (cancer+normal vs cancer+normal) |
| 12-52214119 | cg19495013 | (cancer vs cancer) (cancer vs cancer) (cancer+normal vs cancer+normal) | 2-242824433 | cg03532030 | (cancer vs cancer) (cancer+normal vs cancer+normal) |
| 12-52214320 | cg07375978 | (cancer vs cancer) (cancer vs cancer) (cancer+normal vs cancer+normal) | 2-24715213 | cg11380327 | (cancer vs cancer) (normal vs normal) (cancer+normal vs cancer+normal) |
| 12-52214758 | cg05238741 | (cancer vs cancer) (cancer+normal vs cancer+normal) | 2-394115 | cg19039481 | (cancer vs cancer) (cancer+normal vs cancer+normal) |
| 12-53932355 | cg03998264 | (cancer+normal vs cancer+normal) | 2-85553841 | cg21513352 | (cancer vs cancer) (cancer+normal vs cancer+normal) |
| 12-54422127 | cg12232783 | (cancer vs cancer) (cancer vs cancer) (cancer+normal vs cancer+normal) | 20-21105829 | cg26827893 | (cancer vs cancer) (cancer+normal vs cancer+normal) |
| 12-54422239 | cg04576672 | (cancer vs cancer) (normal vs normal) (cancer+normal vs cancer+normal) | 21-33031392 | cg03173570 | (cancer vs cancer) (cancer+normal vs cancer+normal) |
| 12-54422350 | cg27441225 | (cancer vs cancer) (cancer vs cancer) (cancer+normal vs cancer+normal) | 21-41032396 | cg26390598 | (cancer vs cancer) (normal vs normal) (cancer+normal vs cancer+normal) |
| 12-54422488 | cg22378817 | (cancer vs cancer) (cancer vs cancer) (cancer+normal vs cancer+normal) | 22-46471129 | cg27338353 | (cancer vs cancer) (normal vs normal) (cancer+normal vs cancer+normal) |
| 12-54427636 | cg16983211 | (cancer vs cancer) (cancer vs cancer) (cancer+normal vs cancer+normal) | 22-50524541 | cg03677952 | (cancer vs cancer) (cancer+normal vs cancer+normal) |
| 12-54427700 | cg15700739 | (cancer vs cancer) (cancer vs cancer) (cancer+normal vs cancer+normal) | 3-155570275 | cg25375916 | (cancer vs cancer) |
| 12-54446505 | cg03416628 | (cancer vs cancer) (cancer+normal vs cancer+normal) | 3-173129681 | cg05058204 | (cancer vs cancer) (cancer+normal vs cancer+normal) |
| 12-54446576 | cg08712054 | (cancer vs cancer) (cancer+normal vs cancer+normal) | 3-182208536 | cg01465177 | (cancer vs cancer) (normal vs normal) (cancer+normal vs cancer+normal) |
| 12-97857511 | cg03782861 | (cancer vs cancer) (cancer+normal vs cancer+normal) | 3-24725365 | cg13279811 | (cancer vs cancer) (cancer+normal vs cancer+normal) |
| 13-113598068 | cg01866427 | (cancer vs cancer) (cancer vs cancer) | 3-44039204 | cg19444609 | (cancer vs cancer) (normal vs normal) |

| | | | | | |
|---|---|---|---|---|---|
| | | (cancer+normal vs cancer+normal) | | | (cancer+normal vs cancer+normal) |
| 13-113623233 | cg02223067 | (cancer vs cancer) (cancer vs cancer) (cancer+normal vs cancer+normal) | 3-44039357 | cg07289306 | (cancer vs cancer) (normal vs normal) (cancer+normal vs cancer+normal) |
| 13-113623300 | cg16737670 | (cancer vs cancer) (cancer vs cancer) (cancer+normal vs cancer+normal) | 3-44039509 | cg01856525 | (cancer vs cancer) (normal vs normal) |
| 13-113623639 | cg00127894 | (cancer vs cancer) (cancer vs cancer) (normal vs normal) (cancer+normal vs cancer+normal) | 3-44039578 | cg03889542 | (cancer vs cancer) (normal vs normal) |
| 13-113623659 | cg15179805 | (cancer vs cancer) (cancer vs cancer) (cancer+normal vs cancer+normal) | 3-98452940 | cg10128511 | (cancer vs cancer) (cancer+normal vs cancer+normal) |
| 13-113623844 | cg09678836 | (cancer vs cancer) (cancer vs cancer) (normal vs normal) (cancer+normal vs cancer+normal) | 3-98453086 | cg07390647 | (cancer vs cancer) (cancer+normal vs cancer+normal) |
| 14-52739256 | cg26168772 | (cancer+normal vs cancer+normal) | 4-169402298 | cg03364683 | (cancer vs cancer) (cancer+normal vs cancer+normal) |
| 15-88945783 | cg00200755 | (cancer+normal vs cancer+normal) | 4-184828386 | cg23965931 | (cancer vs cancer) (normal vs normal) |
| 15-97312064 | cg12306333 | (cancer+normal vs cancer+normal) | 4-186316674 | cg06277849 | (cancer vs cancer) (normal vs normal) (cancer+normal vs cancer+normal) |
| 15-97316699 | cg25271525 | (cancer+normal vs cancer+normal) | 4-78742376 | cg24877510 | (cancer vs cancer) (cancer+normal vs cancer+normal) |
| 16-20370531 | cg08284233 | (cancer+normal vs cancer+normal) | 5-115175001 | cg17519280 | (cancer vs cancer) (normal vs normal) (cancer+normal vs cancer+normal) |
| 17-18908220 | cg05090106 | (cancer vs cancer) (cancer+normal vs cancer+normal) | 5-121648993 | cg07195301 | (cancer vs cancer) (normal vs normal) (cancer+normal vs cancer+normal) |
| 17-18908235 | cg14807365 | (cancer vs cancer) (cancer vs cancer) (cancer+normal vs cancer+normal) | 5-134075279 | cg05998153 | (cancer vs cancer) (normal vs normal) (cancer+normal vs cancer+normal) |
| 17-18908242 | cg22220165 | (cancer vs cancer) (cancer vs cancer) (cancer+normal vs cancer+normal) | 5-54527190 | cg01230355 | (cancer vs cancer) (cancer+normal vs cancer+normal) |
| 17-35289543 | cg16293732 | (cancer vs cancer) (cancer vs cancer) (cancer+normal vs cancer+normal) | 5-83677872 | cg19613968 | (cancer vs cancer) (cancer+normal vs cancer+normal) |
| 17-35290458 | cg05279092 | (cancer vs cancer) (cancer+normal vs cancer+normal) | 6-108145420 | cg27353361 | (cancer vs cancer) (cancer+normal vs cancer+normal) |
| 17-35290635 | cg19502920 | (cancer vs cancer) (cancer+normal vs | 6-108145539 | cg27659622 | (cancer vs cancer) (cancer+normal vs |

| | | | | | |
|---|---|---|---|---|---|
| | | cancer+normal) | | | cancer+normal) |
| 17-35297752 | cg07103093 | (cancer vs cancer) (cancer+normal vs cancer+normal) | 6-111738477 | cg09365147 | (cancer vs cancer) (cancer+normal vs cancer+normal) |
| 17-35300874 | cg04588165 | (cancer vs cancer) (normal vs normal) (cancer+normal vs cancer+normal) | 6-113012601 | cg25441141 | (cancer vs cancer) (normal vs normal) (cancer+normal vs cancer+normal) |
| 17-46702028 | cg00846995 | (cancer+normal vs cancer+normal) | 6-31707151 | cg07801569 | (cancer vs cancer) |
| 17-46704410 | cg24042517 | (cancer vs cancer) (cancer+normal vs cancer+normal) | 6-7909642 | cg08880817 | (cancer vs cancer) (cancer+normal vs cancer+normal) |
| 17-55056066 | cg10342722 | (cancer vs cancer) (cancer+normal vs cancer+normal) | 6-88407390 | cg15991104 | (cancer vs cancer) (cancer+normal vs cancer+normal) |
| 17-7790179 | cg21068610 | (cancer vs cancer) (cancer vs cancer) (cancer+normal vs cancer+normal) | 7-101457175 | cg01401327 | (cancer vs cancer) (cancer+normal vs cancer+normal) |
| 18-49865488 | cg21263710 | (cancer+normal vs cancer+normal) | 7-1572252 | cg04358131 | (cancer vs cancer) (cancer+normal vs cancer+normal) |
| 19-11199917 | cg18596381 | (cancer vs cancer) (cancer vs cancer) (cancer+normal vs cancer+normal) | 7-1572327 | cg05279413 | (cancer vs cancer) (cancer+normal vs cancer+normal) |
| 19-11199944 | cg19751789 | (cancer vs cancer) (cancer vs cancer) (cancer+normal vs cancer+normal) | 7-1572571 | cg26468430 | (cancer vs cancer) (normal vs normal) (cancer+normal vs cancer+normal) |
| 19-11199965 | cg12013591 | (cancer vs cancer) (cancer vs cancer) (cancer+normal vs cancer+normal) | 7-18329916 | cg08725366 | (cancer vs cancer) |
| 19-2639424 | cg26988138 | (cancer vs cancer) (normal vs normal) (cancer+normal vs cancer+normal) | 7-68983823 | cg22203237 | (cancer vs cancer) (cancer+normal vs cancer+normal) |
| 2-10263480 | cg00506866 | (cancer vs cancer) (cancer vs cancer) (cancer+normal vs cancer+normal) | 7-79780310 | cg25418001 | (cancer vs cancer) (normal vs normal) (cancer+normal vs cancer+normal) |
| 2-10263696 | cg19516340 | (cancer vs cancer) (cancer+normal vs cancer+normal) | 7-92461971 | cg00907204 | (cancer vs cancer) (cancer+normal vs cancer+normal) |
| 2-21856440 | cg01598144 | (cancer vs cancer) (cancer+normal vs cancer+normal) | 8-127568650 | cg06023345 | (cancer vs cancer) (normal vs normal) (cancer+normal vs cancer+normal) |
| 2-72372965 | cg08863459 | (cancer vs cancer) (cancer+normal vs cancer+normal) | 8-127568677 | cg01923999 | (cancer vs cancer) (normal vs normal) (cancer+normal vs cancer+normal) |
| 2-73151879 | cg00486564 | (cancer vs cancer) (cancer+normal vs cancer+normal) | 8-127866047 | cg03294424 | (cancer vs cancer) (cancer+normal vs cancer+normal) |
| 2-91930138 | cg11775291 | (cancer+normal vs cancer+normal) | 8-67342600 | cg00007076 | (cancer vs cancer) (cancer+normal vs cancer+normal) |
| 22- | cg14742445 | (cancer vs cancer) | 8-72268832 | cg2570068 | (cancer vs cancer) |

| | | | | | |
|---|---|---|---|---|---|
| 30195299 | | (cancer+normal vs cancer+normal) | | 6 | |
| 3-132036382 | cg00589251 | (cancer vs cancer) (cancer vs cancer) (cancer+normal vs cancer+normal) | 8-731697 | cg18788284 | (cancer vs cancer) (cancer+normal vs cancer+normal) |
| 4-111530471 | cg15903185 | (cancer vs cancer) (cancer vs cancer) (cancer+normal vs cancer+normal) | 8-80679314 | cg03301084 | (cancer vs cancer) (normal vs normal) (cancer+normal vs cancer+normal) |
| 4-14113465 | cg01612366 | (cancer vs cancer) (cancer+normal vs cancer+normal) | 8-93107298 | cg07538339 | (cancer vs cancer) (normal vs normal) (cancer+normal vs cancer+normal) |
| 4-189548707 | cg19084053 | (cancer+normal vs cancer+normal) | 9-101019042 | cg13563728 | (cancer vs cancer) (cancer+normal vs cancer+normal) |
| 4-49204696 | cg14500801 | (cancer+normal vs cancer+normal) | 9-19378679 | cg14014731 | (cancer vs cancer) (cancer+normal vs cancer+normal) |
| 5-135536668 | cg18105361 | (cancer+normal vs cancer+normal) | 9-35603605 | cg13596208 | (cancer vs cancer) (cancer+normal vs cancer+normal) |
| 5-1406177 | cg24756227 | (cancer vs cancer) (cancer+normal vs cancer+normal) | 9-89560185 | cg02501714 | (cancer vs cancer) (normal vs normal) (cancer+normal vs cancer+normal) |
| 5-40833236 | cg08754067 | (cancer+normal vs cancer+normal) | **Status - Hypo** | | |
| 5-475085 | cg15084390 | (cancer vs cancer) (cancer+normal vs cancer+normal) | 1-10676290 | cg26919387 | (normal vs normal) (cancer vs cancer) (cancer+normal vs cancer+normal) |
| 5-475205 | cg21743597 | (cancer vs cancer) (cancer+normal vs cancer+normal) | 1-113819171 | cg23232873 | (normal vs normal) (cancer vs cancer) (cancer+normal vs cancer+normal) |
| 5-66299786 | cg02632185 | (cancer vs cancer) (cancer vs cancer) (normal vs normal) (cancer+normal vs cancer+normal) | 1-116925613 | cg17107017 | (cancer vs cancer) (cancer+normal vs cancer+normal) |
| 5-66299875 | cg18118497 | (cancer vs cancer) (cancer vs cancer) (cancer+normal vs cancer+normal) | 1-143663862 | cg14014098 | (cancer vs cancer) (cancer+normal vs cancer+normal) |
| 5-66300406 | cg17449851 | (cancer vs cancer) (cancer vs cancer) (normal vs normal) (cancer+normal vs cancer+normal) | 1-147851370 | cg22395335 | (cancer vs cancer) (cancer+normal vs cancer+normal) |
| 5-66300433 | cg11831286 | (cancer vs cancer) (cancer vs cancer) (normal vs normal) (cancer+normal vs cancer+normal) | 1-153514482 | cg08823182 | (cancer vs cancer) (cancer+normal vs cancer+normal) |
| 5-66300503 | cg27116842 | (cancer vs cancer) (cancer vs cancer) (normal vs normal) (cancer+normal vs cancer+normal) | 1-182570870 | cg08113628 | (normal vs normal) (cancer vs cancer) (cancer+normal vs cancer+normal) |

| | | | | | |
|---|---|---|---|---|---|
| 6-167508170 | cg10661263 | (cancer+normal vs cancer+normal) | 1-206765088 | cg13245983 | (cancer vs cancer) (cancer+normal vs cancer+normal) |
| 6-26440101 | cg01269795 | (cancer vs cancer) (cancer+normal vs cancer+normal) | 1-51910707 | cg20698942 | (cancer vs cancer) (cancer+normal vs cancer+normal) |
| 6-30690376 | cg24146125 | (cancer+normal vs cancer+normal) | 1-57040807 | cg17902947 | (cancer vs cancer) (cancer+normal vs cancer+normal) |
| 6-84570388 | cg06677013 | (cancer+normal vs cancer+normal) | 1-58622038 | cg18229457 | (normal vs normal) (cancer vs cancer) (cancer+normal vs cancer+normal) |
| 7-1288184 | cg16275882 | (cancer vs cancer) (cancer+normal vs cancer+normal) | 1-8442507 | cg06634717 | (cancer vs cancer) (cancer+normal vs cancer+normal) |
| 7-151328093 | cg19412964 | (cancer vs cancer) (cancer+normal vs cancer+normal) | 10-120804863 | cg03948139 | (cancer vs cancer) (cancer+normal vs cancer+normal) |
| 7-1687852 | cg02412346 | (cancer+normal vs cancer+normal) | 10-131200784 | cg16960573 | (normal vs normal) (cancer vs cancer) (cancer+normal vs cancer+normal) |
| 7-1708823 | cg17202717 | (cancer vs cancer) (cancer+normal vs cancer+normal) | 10-131200802 | cg26328291 | (normal vs normal) (cancer vs cancer) |
| 7-46972700 | cg25983901 | (normal vs normal) (cancer+normal vs cancer+normal) | 10-134559553 | cg11642140 | (normal vs normal) (cancer vs cancer) (cancer+normal vs cancer+normal) |
| 7-92747308 | cg08986767 | (cancer vs cancer) (cancer vs cancer) (cancer+normal vs cancer+normal) | 10-134559637 | cg17750946 | (normal vs normal) (cancer vs cancer) (cancer+normal vs cancer+normal) |
| 8-107669778 | cg07554329 | (cancer vs cancer) (cancer vs cancer) (cancer+normal vs cancer+normal) | 10-134559696 | cg07927953 | (normal vs normal) (cancer vs cancer) (cancer+normal vs cancer+normal) |
| 8-107669783 | cg17136799 | (cancer vs cancer) (cancer vs cancer) (cancer+normal vs cancer+normal) | 10-134559939 | cg17054783 | (normal vs normal) (cancer vs cancer) (cancer+normal vs cancer+normal) |
| 8-107669787 | cg26622232 | (cancer vs cancer) (cancer vs cancer) (cancer+normal vs cancer+normal) | 10-134561116 | cg12449716 | (cancer vs cancer) |
| 8-107669856 | cg17176732 | (cancer vs cancer) (cancer vs cancer) (cancer+normal vs cancer+normal) | 10-412257 | cg04336905 | (cancer vs cancer) (cancer+normal vs cancer+normal) |
| 8-107669906 | cg17212289 | (cancer vs cancer) (cancer vs cancer) (cancer+normal vs cancer+normal) | 11-111167086 | cg10131836 | (normal vs normal) (cancer vs cancer) (cancer+normal vs cancer+normal) |
| 8-107670161 | cg16326979 | (cancer vs cancer) (cancer vs cancer) (cancer+normal vs cancer+normal) | 11-35252384 | cg15427520 | (normal vs normal) (cancer vs cancer) (cancer+normal vs cancer+normal) |
| 8-107670358 | cg00298796 | (cancer vs cancer) (cancer+normal vs cancer+normal) | 11-57105500 | cg18997075 | (cancer vs cancer) (cancer+normal vs cancer+normal) |

| | | | | | |
|---|---|---|---|---|---|
| 8-55294610 | cg13242160 | (cancer vs cancer) (cancer vs cancer) (cancer+normal vs cancer+normal) | 11-75852389 | cg2463051 6 | (normal vs normal) (cancer vs cancer) (cancer+normal vs cancer+normal) |
| 8-55294721 | cg07664990 | (cancer vs cancer) (cancer vs cancer) (cancer+normal vs cancer+normal) | 11-78638670 | cg1923441 2 | (normal vs normal) (cancer vs cancer) (cancer+normal vs cancer+normal) |
| 8-59714165 | cg25766247 | (cancer vs cancer) (cancer+normal vs cancer+normal) | 12-109911508 | cg2271494 2 | (normal vs normal) (cancer vs cancer) (cancer+normal vs cancer+normal) |
| 8-9760528 | cg00528902 | (cancer vs cancer) (cancer+normal vs cancer+normal) | 12-120173011 | cg0295291 8 | (cancer vs cancer) (cancer+normal vs cancer+normal) |
| 9-136472927 | cg21223423 | (cancer+normal vs cancer+normal) | 12-120173057 | cg1457824 7 | (cancer vs cancer) (cancer+normal vs cancer+normal) |
| 9-35079141 | cg04961911 | (cancer+normal vs cancer+normal) | 12-120173114 | cg1733859 4 | (cancer vs cancer) (cancer+normal vs cancer+normal) |
| 9-79320026 | cg13914324 | (cancer vs cancer) (cancer+normal vs cancer+normal) | 12-123636717 | cg1517560 8 | (cancer vs cancer) (cancer+normal vs cancer+normal) |
| **Status - Hypo** | | | 12-16067423 | cg1661659 9 | (cancer vs cancer) |
| 1-101094117 | cg00902464 | (cancer vs cancer) (cancer+normal vs cancer+normal) | 12-1890922 | cg0384178 4 | (cancer vs cancer) (cancer+normal vs cancer+normal) |
| 1-2026193 | cg22712840 | (cancer vs cancer) (cancer+normal vs cancer+normal) | 12-3030602 | cg1313027 4 | (normal vs normal) (cancer vs cancer) (cancer+normal vs cancer+normal) |
| 1-217724849 | cg10325088 | (cancer vs cancer) (cancer vs cancer) (cancer+normal vs cancer+normal) | 14-103369717 | cg0181387 7 | (cancer vs cancer) (cancer+normal vs cancer+normal) |
| 1-2427655 | cg10219816 | (cancer vs cancer) (cancer+normal vs cancer+normal) | 14-103369786 | cg2277976 5 | (cancer vs cancer) (cancer+normal vs cancer+normal) |
| 1-25069665 | cg12081359 | (cancer vs cancer) (cancer+normal vs cancer+normal) | 14-103369816 | cg0354864 5 | (cancer vs cancer) |
| 1-2996949 | cg22506548 | (cancer vs cancer) (cancer+normal vs cancer+normal) | 14-64909119 | cg1831583 4 | (cancer vs cancer) (cancer+normal vs cancer+normal) |
| 1-3162454 | cg12800122 | (cancer vs cancer) (cancer+normal vs cancer+normal) | 15-40677854 | cg0803628 9 | (normal vs normal) (cancer vs cancer) (cancer+normal vs cancer+normal) |
| 1-47656853 | cg15187606 | (cancer vs cancer) (cancer+normal vs cancer+normal) | 15-55562773 | cg0404230 5 | (cancer vs cancer) |
| 1-54195800 | cg17904129 | (cancer vs cancer) (cancer+normal vs cancer+normal) | 16-1677047 | cg0969235 5 | (cancer vs cancer) |
| 1-54195830 | cg27311970 | (cancer vs cancer) (cancer+normal vs cancer+normal) | 16-2282372 | cg0807978 7 | (cancer vs cancer) |
| 1-55317286 | cg25011368 | (cancer vs cancer) (cancer+normal vs | 16-2282618 | cg0392974 7 | (normal vs normal) (cancer vs cancer) |

| | | | | | |
|---|---|---|---|---|---|
| | | (cancer+normal) | | | (cancer+normal vs cancer+normal) |
| 1-81001617 | cg11832870 | (cancer vs cancer) (cancer+normal vs cancer+normal) | 16-2282957 | cg04152767 | (cancer vs cancer) (cancer+normal vs cancer+normal) |
| 10-12940536 | cg17141577 | (cancer+normal vs cancer+normal) | 16-23410986 | cg03856411 | (cancer vs cancer) (cancer+normal vs cancer+normal) |
| 10-130831421 | cg02220418 | (cancer+normal vs cancer+normal) | 16-4164087 | cg06170425 | (cancer vs cancer) (cancer+normal vs cancer+normal) |
| 10-130844899 | cg07646467 | (normal vs normal) (cancer+normal vs cancer+normal) | 16-4802848 | cg10278046 | (normal vs normal) (cancer vs cancer) (cancer+normal vs cancer+normal) |
| 11-62248459 | cg08160224 | (cancer+normal vs cancer+normal) | 16-4802990 | cg16755475 | (normal vs normal) (cancer vs cancer) (cancer+normal vs cancer+normal) |
| 11-64323816 | cg21039563 | (cancer vs cancer) (cancer vs cancer) (cancer+normal vs cancer+normal) | 16-57147300 | cg06880930 | (cancer vs cancer) (cancer+normal vs cancer+normal) |
| 12-122498111 | cg14400030 | (cancer vs cancer) (cancer+normal vs cancer+normal) | 17-452707 | cg06871344 | (cancer vs cancer) (cancer+normal vs cancer+normal) |
| 12-1609346 | cg00230120 | (cancer vs cancer) (cancer+normal vs cancer+normal) | 17-48856029 | cg12280798 | (normal vs normal) (cancer vs cancer) (cancer+normal vs cancer+normal) |
| 12-7121914 | cg24859375 | (cancer vs cancer) (cancer vs cancer) (cancer+normal vs cancer+normal) | 17-80541649 | cg24220178 | (cancer vs cancer) |
| 13-111880998 | cg21980364 | (cancer vs cancer) (cancer vs cancer) (cancer+normal vs cancer+normal) | 18-8660164 | cg25899954 | (normal vs normal) (cancer vs cancer) (cancer+normal vs cancer+normal) |
| 13-113498450 | cg01233786 | (cancer+normal vs cancer+normal) | 19-4676202 | cg13513411 | (normal vs normal) (cancer vs cancer) (cancer+normal vs cancer+normal) |
| 13-114138019 | cg05508408 | (cancer vs cancer) (cancer+normal vs cancer+normal) | 19-4676623 | cg18328612 | (normal vs normal) (cancer vs cancer) (cancer+normal vs cancer+normal) |
| 13-114138260 | cg21238257 | (cancer vs cancer) (cancer vs cancer) (cancer+normal vs cancer+normal) | 2-11774852 | cg06856155 | (normal vs normal) (cancer vs cancer) (cancer+normal vs cancer+normal) |
| 13-47259150 | cg27591016 | (cancer+normal vs cancer+normal) | 2-193013929 | cg24923920 | (cancer vs cancer) |
| 13-96086098 | cg20278383 | (normal vs normal) (cancer vs cancer) (cancer vs cancer) (cancer+normal vs cancer+normal) | 2-236897639 | cg09589951 | (cancer vs cancer) |
| 13-96086249 | cg26557005 | (cancer vs cancer) (cancer+normal vs cancer+normal) | 2-3325158 | cg00027081 | (normal vs normal) (cancer vs cancer) (cancer+normal vs cancer+normal) |

| | | | | | |
|---|---|---|---|---|---|
| 13-97893311 | cg06155414 | (cancer+normal vs cancer+normal) | 2-69857465 | cg04694389 | (normal vs normal) (cancer vs cancer) (cancer+normal vs cancer+normal) |
| 13-97980712 | cg11862665 | (cancer vs cancer) (cancer+normal vs cancer+normal) | 2-98409192 | cg16901821 | (cancer vs cancer) |
| 14-64357795 | cg19987354 | (cancer+normal vs cancer+normal) | 2-98409290 | cg02489228 | (cancer vs cancer) |
| 14-89628141 | cg02212698 | (cancer vs cancer) (cancer+normal vs cancer+normal) | 2-98409368 | cg23812119 | (cancer vs cancer) |
| 14-91275103 | cg18518804 | (cancer vs cancer) (cancer+normal vs cancer+normal) | 2-98409379 | cg06969479 | (cancer vs cancer) |
| 14-99893325 | cg24180006 | (cancer vs cancer) (cancer+normal vs cancer+normal) | 20-32436418 | cg17894293 | (cancer vs cancer) (cancer+normal vs cancer+normal) |
| 16-15127172 | cg03245889 | (cancer vs cancer) (cancer+normal vs cancer+normal) | 20-35399961 | cg06313775 | (cancer vs cancer) |
| 16-15127193 | cg06978461 | (cancer vs cancer) (cancer vs cancer) (cancer+normal vs cancer+normal) | 20-35444275 | cg00171166 | (cancer vs cancer) (cancer+normal vs cancer+normal) |
| 16-78323024 | cg00879840 | (cancer vs cancer) (cancer+normal vs cancer+normal) | 20-35444364 | cg06814048 | (normal vs normal) (cancer vs cancer) (cancer+normal vs cancer+normal) |
| 16-81565917 | cg03212183 | (cancer vs cancer) (cancer vs cancer) (cancer+normal vs cancer+normal) | 20-35444560 | cg08209184 | (cancer vs cancer) (cancer+normal vs cancer+normal) |
| 17-18854760 | cg18449048 | (cancer vs cancer) (cancer vs cancer) (cancer+normal vs cancer+normal) | 22-42563204 | cg27157050 | (cancer vs cancer) (cancer+normal vs cancer+normal) |
| 17-18854799 | cg25567048 | (normal vs normal) (cancer vs cancer) (cancer+normal vs cancer+normal) | 22-45737514 | cg13318129 | (cancer vs cancer) (cancer+normal vs cancer+normal) |
| 17-18855313 | cg00306284 | (normal vs normal) (cancer vs cancer) (cancer+normal vs cancer+normal) | 3-113413263 | cg00395951 | (cancer vs cancer) (cancer+normal vs cancer+normal) |
| 17-18855506 | cg09632029 | (normal vs normal) (cancer vs cancer) (cancer+normal vs cancer+normal) | 3-11597868 | cg02131130 | (cancer vs cancer) (cancer+normal vs cancer+normal) |
| 17-40064155 | cg03950716 | (cancer vs cancer) (cancer+normal vs cancer+normal) | 3-11597941 | cg00668519 | (cancer vs cancer) |
| 17-4510743 | cg20818528 | (cancer vs cancer) (cancer+normal vs cancer+normal) | 3-11655466 | cg21396517 | (cancer vs cancer) (cancer+normal vs cancer+normal) |
| 17-4520467 | cg00349608 | (cancer vs cancer) (cancer+normal vs cancer+normal) | 3-184042708 | cg27315348 | (cancer vs cancer) |
| 17-46871905 | cg08137716 | (cancer vs cancer) (cancer+normal vs cancer+normal) | 3-195709698 | cg25931138 | (normal vs normal) (cancer vs cancer) (cancer+normal vs |

343

| | | | | | cancer+normal) |
|---|---|---|---|---|---|
| 17-7225013 | cg09777416 | (cancer vs cancer) (cancer+normal vs cancer+normal) | 3-52737693 | cg13723011 | (normal vs normal) (cancer vs cancer) (cancer+normal vs cancer+normal) |
| 17-78305896 | cg12648893 | (cancer+normal vs cancer+normal) | 4-169573255 | cg07979388 | (normal vs normal) (cancer vs cancer) (cancer+normal vs cancer+normal) |
| 17-78700928 | cg06975080 | (cancer vs cancer) (cancer vs cancer) (cancer+normal vs cancer+normal) | 4-183816164 | cg09557149 | (normal vs normal) (cancer vs cancer) (cancer+normal vs cancer+normal) |
| 17-78817938 | cg25899969 | (cancer+normal vs cancer+normal) | 4-184683218 | cg07396827 | (cancer vs cancer) (cancer+normal vs cancer+normal) |
| 19-13198960 | cg11883009 | (cancer vs cancer) (cancer+normal vs cancer+normal) | 4-186945339 | cg09227533 | (normal vs normal) (cancer vs cancer) (cancer+normal vs cancer+normal) |
| 19-1424658 | cg11656553 | (cancer vs cancer) (cancer+normal vs cancer+normal) | 4-2687547 | cg11692181 | (cancer vs cancer) |
| 19-18888081 | cg17759252 | (cancer+normal vs cancer+normal) | 4-83323585 | cg18579761 | (normal vs normal) (cancer vs cancer) (cancer+normal vs cancer+normal) |
| 2-127811853 | cg01441308 | (cancer vs cancer) (cancer+normal vs cancer+normal) | 5-134814626 | cg01018110 | (normal vs normal) (cancer vs cancer) (cancer+normal vs cancer+normal) |
| 2-201218948 | cg26015513 | (cancer vs cancer) (cancer vs cancer) (cancer+normal vs cancer+normal) | 5-78766902 | cg06646622 | (normal vs normal) (cancer vs cancer) (cancer+normal vs cancer+normal) |
| 2-213950646 | cg05148385 | (cancer vs cancer) (cancer+normal vs cancer+normal) | 6-138200309 | cg01981433 | (cancer vs cancer) (cancer+normal vs cancer+normal) |
| 2-217403190 | cg09473180 | (cancer vs cancer) (cancer vs cancer) (cancer+normal vs cancer+normal) | 6-169978311 | cg05066503 | (cancer vs cancer) (cancer+normal vs cancer+normal) |
| 2-224824723 | cg17963548 | (cancer vs cancer) (cancer+normal vs cancer+normal) | 6-169978387 | cg13211832 | (cancer vs cancer) (cancer+normal vs cancer+normal) |
| 2-42461109 | cg15425670 | (cancer+normal vs cancer+normal) | 6-170028109 | cg25373438 | (cancer vs cancer) (cancer+normal vs cancer+normal) |
| 2-96940284 | cg00336220 | (cancer+normal vs cancer+normal) | 6-30094960 | cg21376799 | (normal vs normal) (cancer vs cancer) (cancer+normal vs cancer+normal) |
| 20-6025272 | cg21209310 | (cancer vs cancer) (cancer vs cancer) (cancer+normal vs cancer+normal) | 7-101522290 | cg00982974 | (normal vs normal) (cancer vs cancer) (cancer+normal vs cancer+normal) |
| 22-24119331 | cg27598806 | (cancer vs cancer) (cancer+normal vs cancer+normal) | 7-134613526 | cg03032816 | (cancer vs cancer) (cancer+normal vs cancer+normal) |
| 3-126010451 | cg11423909 | (cancer vs cancer) (cancer+normal vs | 7-33725819 | cg18365383 | (normal vs normal) (cancer vs cancer) |

| | | | | | |
|---|---|---|---|---|---|
| | | cancer+normal) | | | (cancer+normal vs cancer+normal) |
| 3-149051159 | cg11011913 | (cancer vs cancer) (cancer vs cancer) (cancer+normal vs cancer+normal) | 7-5529868 | cg22399555 | (cancer vs cancer) (cancer+normal vs cancer+normal) |
| 3-171976122 | cg13050884 | (cancer vs cancer) (cancer+normal vs cancer+normal) | 8-126202653 | cg05445772 | (cancer vs cancer) (cancer+normal vs cancer+normal) |
| 3-183894727 | cg16301768 | (cancer vs cancer) (cancer+normal vs cancer+normal) | 8-12860315 | cg25515982 | (normal vs normal) (cancer vs cancer) (cancer+normal vs cancer+normal) |
| 3-64547310 | cg22118297 | (cancer+normal vs cancer+normal) | 9-123204555 | cg14003627 | (cancer vs cancer) |
| 3-64547346 | cg14187266 | (cancer+normal vs cancer+normal) | 9-126153067 | cg14177289 | (cancer vs cancer) (cancer+normal vs cancer+normal) |
| 4-1844807 | cg16500874 | (cancer+normal vs cancer+normal) | 9-129648322 | cg13595556 | (normal vs normal) (cancer vs cancer) (cancer+normal vs cancer+normal) |
| 4-72205104 | cg19850370 | (cancer vs cancer) (cancer+normal vs cancer+normal) | **(cancer+normal vs cancer+normal)** | | |
| 4-87993109 | cg27000934 | (cancer vs cancer) (cancer vs cancer) (cancer+normal vs cancer+normal) | **Status - Hyper** | | |
| 5-1100209 | cg05978154 | (cancer+normal vs cancer+normal) | 1-155828926 | cg06440960 | (cancer vs cancer) (cancer+normal vs cancer+normal) |
| 5-111017556 | cg01885839 | (cancer vs cancer) (cancer+normal vs cancer+normal) | 1-16160360 | cg05960182 | (cancer vs cancer) (normal vs normal) (cancer+normal vs cancer+normal) |
| 5-131607888 | cg20547724 | (cancer vs cancer) (cancer+normal vs cancer+normal) | 1-167690438 | cg04846203 | (normal vs normal) (cancer+normal vs cancer+normal) |
| 5-1337747 | cg10641823 | (cancer vs cancer) (cancer+normal vs cancer+normal) | 1-190446756 | cg19632206 | (cancer vs cancer) (cancer+normal vs cancer+normal) |
| 5-1339114 | cg20700235 | (cancer+normal vs cancer+normal) | 1-7830855 | cg12072178 | (cancer vs cancer) (cancer+normal vs cancer+normal) |
| 5-142527658 | cg26547816 | (cancer vs cancer) (cancer+normal vs cancer+normal) | 10-127622996 | cg06777902 | (cancer+normal vs cancer+normal) |
| 5-1514511 | cg00281273 | (cancer+normal vs cancer+normal) | 10-3514783 | cg13687834 | (cancer vs cancer) (cancer+normal vs cancer+normal) |
| 5-172097064 | cg24667575 | (cancer+normal vs cancer+normal) | 10-5136782 | cg12530994 | (cancer vs cancer) (cancer+normal vs cancer+normal) |
| 6-160680115 | cg02666489 | (cancer+normal vs cancer+normal) | 11-102401616 | cg01813071 | (cancer vs cancer) (normal vs normal) (cancer+normal vs cancer+normal) |
| 6-160680162 | cg25545503 | (normal vs normal) (cancer vs cancer) (cancer+normal vs | 11-118437129 | cg26954138 | (cancer vs cancer) (cancer+normal vs cancer+normal) |

| | | | | | |
|---|---|---|---|---|---|
| | | cancer+normal) | | | |
| 6-37896180 | cg24249695 | (cancer+normal vs cancer+normal) | 11-118437133 | cg20874052 | (cancer vs cancer) (cancer+normal vs cancer+normal) |
| 7-101556684 | cg15600935 | (cancer vs cancer) (cancer vs cancer) (cancer+normal vs cancer+normal) | 11-121567477 | cg01195276 | (cancer vs cancer) (cancer+normal vs cancer+normal) |
| 7-101566857 | cg02307880 | (cancer vs cancer) (cancer+normal vs cancer+normal) | 11-126173548 | cg22911867 | (cancer vs cancer) (cancer+normal vs cancer+normal) |
| 7-1120141 | cg02112681 | (cancer vs cancer) (cancer+normal vs cancer+normal) | 11-131941989 | cg16763885 | (cancer vs cancer) (cancer+normal vs cancer+normal) |
| 7-1142433 | cg00364696 | (cancer vs cancer) (cancer+normal vs cancer+normal) | 11-17251956 | cg26906217 | (cancer vs cancer) (cancer+normal vs cancer+normal) |
| 7-2679546 | cg14893935 | (cancer+normal vs cancer+normal) | 11-1974984 | cg14289294 | (normal vs normal) (cancer+normal vs cancer+normal) |
| 7-2679726 | cg22239213 | (cancer vs cancer) (cancer+normal vs cancer+normal) | 11-45433358 | cg15355111 | (cancer vs cancer) (cancer+normal vs cancer+normal) |
| 7-2679971 | cg16184737 | (cancer vs cancer) (cancer+normal vs cancer+normal) | 11-57424773 | cg27258272 | (cancer vs cancer) (cancer+normal vs cancer+normal) |
| 7-47580148 | cg05425699 | (cancer+normal vs cancer+normal) | 11-65487505 | cg06038201 | (cancer vs cancer) (normal vs normal) (cancer+normal vs cancer+normal) |
| 8-141147612 | cg02225988 | (cancer vs cancer) (cancer+normal vs cancer+normal) | 11-96123810 | cg03075605 | (cancer vs cancer) (cancer+normal vs cancer+normal) |
| 8-141147681 | cg17018549 | (cancer vs cancer) (cancer vs cancer) (cancer+normal vs cancer+normal) | 12-48100286 | cg05731122 | (cancer vs cancer) (cancer+normal vs cancer+normal) |
| 8-141220177 | cg12865888 | (cancer+normal vs cancer+normal) | 12-65066768 | cg13042575 | (cancer vs cancer) (cancer+normal vs cancer+normal) |
| 8-142311072 | cg18818267 | (cancer vs cancer) (cancer+normal vs cancer+normal) | 12-78224480 | cg20217872 | (cancer vs cancer) (cancer+normal vs cancer+normal) |
| 8-144601734 | cg19598713 | (cancer vs cancer) (cancer+normal vs cancer+normal) | 13-20699452 | cg05585947 | (cancer vs cancer) (normal vs normal) (cancer+normal vs cancer+normal) |
| 8-144601800 | cg09580859 | (cancer vs cancer) (cancer+normal vs cancer+normal) | 13-53191046 | cg02215357 | (cancer vs cancer) (cancer+normal vs cancer+normal) |
| 8-144601851 | cg25900150 | (cancer vs cancer) (cancer+normal vs cancer+normal) | 13-92001764 | cg17799287 | (cancer vs cancer) (cancer+normal vs cancer+normal) |
| 8-144601883 | cg00783706 | (cancer vs cancer) (cancer+normal vs cancer+normal) | 13-92001959 | cg07641807 | (cancer vs cancer) (cancer+normal vs cancer+normal) |
| 8-30209371 | cg02723975 | (cancer vs cancer) (cancer+normal vs cancer+normal) | 14-102094618 | cg06770163 | (cancer+normal vs cancer+normal) |
| 8-30209391 | cg14895559 | (cancer vs cancer) (cancer vs cancer) | 14-104666047 | cg26076119 | (cancer+normal vs cancer+normal) |

| | | | | | |
|---|---|---|---|---|---|
| | | (cancer+normal vs cancer+normal) | | | |
| 8-66701194 | cg26266308 | (cancer vs cancer) (cancer+normal vs cancer+normal) | 14-31698967 | cg10348234 | (cancer vs cancer) (cancer+normal vs cancer+normal) |
| **(normal vs normal)** | | | 14-31699236 | cg18437792 | (cancer vs cancer) (cancer+normal vs cancer+normal) |
| **Status - Hyper** | | | 14-51327604 | cg19590421 | (cancer vs cancer) (cancer+normal vs cancer+normal) |
| 1-147790458 | cg25200152 | (normal vs normal) | 15-60883225 | cg00200653 | (cancer vs cancer) (normal vs normal) (cancer+normal vs cancer+normal) |
| 1-21376633 | cg26561681 | (normal vs normal) | 16-2058572 | cg00852033 | (cancer vs cancer) (cancer+normal vs cancer+normal) |
| 1-47911336 | cg13936452 | (normal vs normal) | 17-47072339 | cg22434594 | (cancer vs cancer) (cancer+normal vs cancer+normal) |
| 1-47911449 | cg06531379 | (normal vs normal) | 17-48785992 | cg06204730 | (cancer vs cancer) (cancer+normal vs cancer+normal) |
| 1-47911508 | cg26864230 | (normal vs normal) | 17-66452343 | cg25161129 | (cancer vs cancer) (cancer+normal vs cancer+normal) |
| 1-47911940 | cg21535606 | (normal vs normal) | 17-74248466 | cg24166450 | (cancer vs cancer) (cancer+normal vs cancer+normal) |
| 1-6507693 | cg05068146 | (normal vs normal) | 18-21976748 | cg20528338 | (cancer+normal vs cancer+normal) |
| 1-6507804 | cg23919534 | (normal vs normal) | 19-11484842 | cg26495839 | (cancer+normal vs cancer+normal) |
| 10-102475324 | cg18279326 | (normal vs normal) | 19-16189693 | cg27377863 | (cancer vs cancer) (cancer+normal vs cancer+normal) |
| 10-102586168 | cg26156687 | (normal vs normal) | 2-16790314 | cg19482842 | (cancer vs cancer) (cancer+normal vs cancer+normal) |
| 10-102586254 | cg07046369 | (normal vs normal) | 2-178104794 | cg21382890 | (cancer vs cancer) (cancer+normal vs cancer+normal) |
| 10-102586454 | cg02770534 | (normal vs normal) | 2-242824433 | cg03532030 | (cancer vs cancer) (cancer+normal vs cancer+normal) |
| 10-102587110 | cg26439963 | (normal vs normal) | 2-24715213 | cg11380327 | (cancer vs cancer) (normal vs normal) (cancer+normal vs cancer+normal) |
| 10-102587322 | cg04943368 | (normal vs normal) | 2-394115 | cg19039481 | (cancer vs cancer) (cancer+normal vs cancer+normal) |
| 10-102587844 | cg23206032 | (normal vs normal) | 2-85553759 | cg23404877 | (cancer+normal vs cancer+normal) |
| 10-102588315 | cg16574134 | (normal vs normal) | 2-85553841 | cg21513352 | (cancer vs cancer) (cancer+normal vs cancer+normal) |
| 10-102588691 | cg20095124 | (cancer vs cancer) (normal vs normal) | 20-10200617 | cg02071463 | (cancer+normal vs cancer+normal) |
| 10- | cg17480467 | (cancer vs cancer) | 20- | cg2682789 | (cancer vs cancer) |

| | | | | | |
|---|---|---|---|---|---|
| 102588959 | | (cancer vs cancer) (normal vs normal) (cancer+normal vs cancer+normal) | 21105829 | 3 | (cancer+normal vs cancer+normal) |
| 10-102589102 | cg14778235 | (cancer vs cancer) (cancer vs cancer) (normal vs normal) (cancer+normal vs cancer+normal) | 21-33031392 | cg03173570 | (cancer vs cancer) (cancer+normal vs cancer+normal) |
| 10-102589250 | cg08217716 | (cancer vs cancer) (cancer vs cancer) (normal vs normal) (cancer+normal vs cancer+normal) | 21-41032396 | cg26390598 | (cancer vs cancer) (normal vs normal) (cancer+normal vs cancer+normal) |
| 10-102589467 | cg06921011 | (normal vs normal) | 22-46471129 | cg27338353 | (cancer vs cancer) (normal vs normal) (cancer+normal vs cancer+normal) |
| 10-102589532 | cg18494190 | (normal vs normal) | 22-50524541 | cg03677952 | (cancer vs cancer) (cancer+normal vs cancer+normal) |
| 10-102589545 | cg04436818 | (normal vs normal) | 3-173129681 | cg05058204 | (cancer vs cancer) (cancer+normal vs cancer+normal) |
| 10-102589840 | cg08823110 | (normal vs normal) | 3-182208536 | cg01465177 | (cancer vs cancer) (normal vs normal) (cancer+normal vs cancer+normal) |
| 10-102590401 | cg06379721 | (normal vs normal) | 3-24725365 | cg13279811 | (cancer vs cancer) (cancer+normal vs cancer+normal) |
| 10-12917286 | cg10684486 | (normal vs normal) | 3-44039204 | cg19444609 | (cancer vs cancer) (normal vs normal) (cancer+normal vs cancer+normal) |
| 10-16562998 | cg25104555 | (normal vs normal) | 3-44039357 | cg07289306 | (cancer vs cancer) (normal vs normal) (cancer+normal vs cancer+normal) |
| 10-17273187 | cg26063719 | (cancer vs cancer) (normal vs normal) (cancer+normal vs cancer+normal) | 3-98452940 | cg10128511 | (cancer vs cancer) (cancer+normal vs cancer+normal) |
| 11-123300839 | cg05451852 | (normal vs normal) | 3-98453086 | cg07390647 | (cancer vs cancer) (cancer+normal vs cancer+normal) |
| 11-123300849 | cg14917395 | (normal vs normal) | 4-169402298 | cg03364683 | (cancer vs cancer) (cancer+normal vs cancer+normal) |
| 11-123300867 | cg19419279 | (normal vs normal) | 4-174290520 | cg01214061 | (cancer+normal vs cancer+normal) |
| 11-123301171 | cg03401096 | (normal vs normal) | 4-186316674 | cg06277849 | (cancer vs cancer) (normal vs normal) (cancer+normal vs cancer+normal) |
| 11-123301490 | cg10904699 | (normal vs normal) | 4-39461721 | cg07027075 | (cancer+normal vs cancer+normal) |
| 11-123301674 | cg05289253 | (normal vs normal) | 4-78742376 | cg24877510 | (cancer vs cancer) (cancer+normal vs cancer+normal) |
| 11- | cg14085017 | (normal vs normal) | 5- | cg1751928 | (cancer vs cancer) |

| | | | | | |
|---|---|---|---|---|---|
| 63775226 | | | 115175001 | 0 | (normal vs normal) (cancer+normal vs cancer+normal) |
| 12-10022682 | cg08752459 | (cancer vs cancer) (cancer vs cancer) (normal vs normal) (cancer+normal vs cancer+normal) | 5-121648993 | cg07195301 | (cancer vs cancer) (normal vs normal) (cancer+normal vs cancer+normal) |
| 12-10022688 | cg12591315 | (cancer vs cancer) (cancer vs cancer) (normal vs normal) (cancer+normal vs cancer+normal) | 5-134075279 | cg05998153 | (cancer vs cancer) (normal vs normal) (cancer+normal vs cancer+normal) |
| 12-120525338 | cg03800721 | (cancer vs cancer) (cancer vs cancer) (normal vs normal) (cancer+normal vs cancer+normal) | 5-42858327 | cg14697657 | (normal vs normal) (cancer+normal vs cancer+normal) |
| 12-54366760 | cg03132556 | (normal vs normal) | 5-54527190 | cg01230355 | (cancer vs cancer) (cancer+normal vs cancer+normal) |
| 12-54422239 | cg04576672 | (cancer vs cancer) (normal vs normal) (cancer+normal vs cancer+normal) | 5-83677872 | cg19613968 | (cancer vs cancer) (cancer+normal vs cancer+normal) |
| 12-54446944 | cg18695839 | (normal vs normal) | 6-108145420 | cg27353361 | (cancer vs cancer) (cancer+normal vs cancer+normal) |
| 12-54447220 | cg03923561 | (normal vs normal) | 6-108145539 | cg27659622 | (cancer vs cancer) (cancer+normal vs cancer+normal) |
| 12-54447283 | cg09720701 | (normal vs normal) | 6-111738477 | cg09365147 | (cancer vs cancer) (cancer+normal vs cancer+normal) |
| 12-54447349 | cg15233062 | (normal vs normal) | 6-113012601 | cg25441141 | (cancer vs cancer) (normal vs normal) (cancer+normal vs cancer+normal) |
| 12-54447430 | cg11585893 | (normal vs normal) | 6-160112632 | cg13221458 | (normal vs normal) (cancer+normal vs cancer+normal) |
| 12-54447584 | cg07266404 | (normal vs normal) | 6-7909642 | cg08880817 | (cancer vs cancer) (cancer+normal vs cancer+normal) |
| 12-54447873 | cg22747076 | (normal vs normal) | 6-88407390 | cg15991104 | (cancer vs cancer) (cancer+normal vs cancer+normal) |
| 12-54448090 | cg11746813 | (normal vs normal) | 7-101457175 | cg01401327 | (cancer vs cancer) (cancer+normal vs cancer+normal) |
| 12-97857304 | cg02716635 | (normal vs normal) | 7-150785000 | cg07847101 | (cancer+normal vs cancer+normal) |
| 13-113623639 | cg00127894 | (cancer vs cancer) (cancer vs cancer) (normal vs normal) (cancer+normal vs cancer+normal) | 7-1572252 | cg04358131 | (cancer vs cancer) (cancer+normal vs cancer+normal) |
| 13-113623844 | cg09678836 | (cancer vs cancer) (cancer vs cancer) (normal vs normal) (cancer+normal vs | 7-1572327 | cg05279413 | (cancer vs cancer) (cancer+normal vs cancer+normal) |

| | | | | | |
|---|---|---|---|---|---|
| | | cancer+normal) | | | |
| 15-48705662 | cg12180319 | (normal vs normal) | 7-1572571 | cg26468430 | (cancer vs cancer) (normal vs normal) (cancer+normal vs cancer+normal) |
| 15-69887053 | cg24340572 | (cancer vs cancer) (normal vs normal) | 7-68983823 | cg22203237 | (cancer vs cancer) (cancer+normal vs cancer+normal) |
| 17-35300868 | cg04636269 | (normal vs normal) | 7-79780310 | cg25418001 | (cancer vs cancer) (normal vs normal) (cancer+normal vs cancer+normal) |
| 17-35300874 | cg04588165 | (cancer vs cancer) (normal vs normal) (cancer+normal vs cancer+normal) | 7-92461971 | cg00907204 | (cancer vs cancer) (cancer+normal vs cancer+normal) |
| 17-35302352 | cg12656291 | (normal vs normal) | 8-127568650 | cg06023345 | (cancer vs cancer) (normal vs normal) (cancer+normal vs cancer+normal) |
| 17-35303330 | cg04264908 | (normal vs normal) | 8-127568677 | cg01923999 | (cancer vs cancer) (normal vs normal) (cancer+normal vs cancer+normal) |
| 17-46705018 | cg14240300 | (normal vs normal) | 8-127866047 | cg03294424 | (cancer vs cancer) (cancer+normal vs cancer+normal) |
| 17-46705577 | cg20710730 | (normal vs normal) | 8-27323467 | cg04035728 | (cancer+normal vs cancer+normal) |
| 19-2639424 | cg26988138 | (cancer vs cancer) (normal vs normal) (cancer+normal vs cancer+normal) | 8-67342600 | cg00007076 | (cancer vs cancer) (cancer+normal vs cancer+normal) |
| 2-151061125 | cg27494897 | (normal vs normal) | 8-731697 | cg18788284 | (cancer vs cancer) (cancer+normal vs cancer+normal) |
| 2-161901182 | cg23244226 | (normal vs normal) | 8-80679314 | cg03301084 | (cancer vs cancer) (normal vs normal) (cancer+normal vs cancer+normal) |
| 2-177503596 | cg08474164 | (normal vs normal) | 8-93107298 | cg07538339 | (cancer vs cancer) (normal vs normal) (cancer+normal vs cancer+normal) |
| 2-220942540 | cg10612128 | (normal vs normal) | 9-101019042 | cg13563728 | (cancer vs cancer) (cancer+normal vs cancer+normal) |
| 2-228331319 | cg25996077 | (normal vs normal) | 9-130212550 | cg24046474 | (cancer+normal vs cancer+normal) |
| 2-237080535 | cg21019043 | (normal vs normal) | 9-19378679 | cg14014731 | (cancer vs cancer) (cancer+normal vs cancer+normal) |
| 2-73151595 | cg16781647 | (cancer vs cancer) (normal vs normal) | 9-35603605 | cg13596208 | (cancer vs cancer) (cancer+normal vs cancer+normal) |
| 3-151488006 | cg13696490 | (normal vs normal) | 9-89560185 | cg02501714 | (cancer vs cancer) (normal vs normal) (cancer+normal vs cancer+normal) |
| 3-156807379 | cg11931463 | (normal vs normal) | **Status - Hypo** | | |

| | | | | | |
|---|---|---|---|---|---|
| 4-140661865 | cg23206160 | (normal vs normal) | 1-10676290 | cg26919387 | (normal vs normal) (cancer vs cancer) (cancer+normal vs cancer+normal) |
| 4-26994740 | cg18777299 | (normal vs normal) | 1-10720111 | cg04830124 | (cancer+normal vs cancer+normal) |
| 5-131593218 | cg18301423 | (normal vs normal) | 1-113819171 | cg23232873 | (normal vs normal) (cancer vs cancer) (cancer+normal vs cancer+normal) |
| 5-472564 | cg00049323 | (normal vs normal) | 1-116925613 | cg17107017 | (cancer vs cancer) (cancer+normal vs cancer+normal) |
| 5-58335180 | cg17633020 | (normal vs normal) | 1-143663862 | cg14014098 | (cancer vs cancer) (cancer+normal vs cancer+normal) |
| 5-58335271 | cg18242030 | (cancer vs cancer) (normal vs normal) | 1-147851370 | cg22395335 | (cancer vs cancer) (cancer+normal vs cancer+normal) |
| 5-58335681 | cg22078451 | (cancer vs cancer) (normal vs normal) | 1-153514482 | cg08823182 | (cancer vs cancer) (cancer+normal vs cancer+normal) |
| 5-66299786 | cg02632185 | (cancer vs cancer) (cancer vs cancer) (normal vs normal) (cancer+normal vs cancer+normal) | 1-182570870 | cg08113628 | (normal vs normal) (cancer vs cancer) (cancer+normal vs cancer+normal) |
| 5-66300406 | cg17449851 | (cancer vs cancer) (cancer vs cancer) (normal vs normal) (cancer+normal vs cancer+normal) | 1-206765088 | cg13245983 | (cancer vs cancer) (cancer+normal vs cancer+normal) |
| 5-66300433 | cg11831286 | (cancer vs cancer) (cancer vs cancer) (normal vs normal) (cancer+normal vs cancer+normal) | 1-51910707 | cg20698942 | (cancer vs cancer) (cancer+normal vs cancer+normal) |
| 5-66300503 | cg27116842 | (cancer vs cancer) (cancer vs cancer) (normal vs normal) (cancer+normal vs cancer+normal) | 1-57040807 | cg17902947 | (cancer vs cancer) (cancer+normal vs cancer+normal) |
| 5-72596188 | cg22120063 | (normal vs normal) | 1-58622038 | cg18229457 | (normal vs normal) (cancer vs cancer) (cancer+normal vs cancer+normal) |
| 5-72597015 | cg09553242 | (normal vs normal) | 1-8442507 | cg06634717 | (cancer vs cancer) (cancer+normal vs cancer+normal) |
| 5-72597126 | cg18595065 | (normal vs normal) | 10-120804863 | cg03948139 | (cancer vs cancer) (cancer+normal vs cancer+normal) |
| 5-72597231 | cg09835740 | (normal vs normal) | 10-121271746 | cg16596052 | (normal vs normal) (cancer+normal vs cancer+normal) |
| 5-72597316 | cg16667631 | (normal vs normal) | 10-131200784 | cg16960573 | (normal vs normal) (cancer vs cancer) (cancer+normal vs cancer+normal) |
| 5-72597715 | cg04221521 | (normal vs normal) | 10-134559553 | cg11642140 | (normal vs normal) (cancer vs cancer) |

| | | | | | |
|---|---|---|---|---|---|
| | | | | | (cancer+normal vs cancer+normal) |
| 5-72598192 | cg25113089 | (cancer vs cancer) (normal vs normal) | 10-134559637 | cg17750946 | (normal vs normal) (cancer vs cancer) (cancer+normal vs cancer+normal) |
| 5-72598865 | cg00766220 | (normal vs normal) | 10-134559696 | cg07927953 | (normal vs normal) (cancer vs cancer) (cancer+normal vs cancer+normal) |
| 5-72598965 | cg11795854 | (normal vs normal) | 10-134559939 | cg17054783 | (normal vs normal) (cancer vs cancer) (cancer+normal vs cancer+normal) |
| 5-72677230 | cg00462107 | (normal vs normal) | 10-412257 | cg04336905 | (cancer vs cancer) (cancer+normal vs cancer+normal) |
| 5-72677723 | cg01927745 | (normal vs normal) | 11-111167086 | cg10131836 | (normal vs normal) (cancer vs cancer) (cancer+normal vs cancer+normal) |
| 6-43118059 | cg01302136 | (normal vs normal) | 11-35252384 | cg15427520 | (normal vs normal) (cancer vs cancer) (cancer+normal vs cancer+normal) |
| 6-45500999 | cg04110902 | (normal vs normal) | 11-3638458 | cg02453348 | (normal vs normal) (cancer+normal vs cancer+normal) |
| 6-50488809 | cg12976463 | (normal vs normal) | 11-57105500 | cg18997075 | (cancer vs cancer) (cancer+normal vs cancer+normal) |
| 7-128531106 | cg07039423 | (cancer vs cancer) (normal vs normal) | 11-75852355 | cg07783183 | (normal vs normal) (cancer+normal vs cancer+normal) |
| 7-1286023 | cg07296447 | (normal vs normal) | 11-75852389 | cg24630516 | (normal vs normal) (cancer vs cancer) (cancer+normal vs cancer+normal) |
| 7-24323840 | cg25884711 | (normal vs normal) | 11-78638670 | cg19234412 | (normal vs normal) (cancer vs cancer) (cancer+normal vs cancer+normal) |
| 7-46972700 | cg25983901 | (normal vs normal) (cancer+normal vs cancer+normal) | 12-109911508 | cg22714942 | (normal vs normal) (cancer vs cancer) (cancer+normal vs cancer+normal) |
| 8-105899330 | cg06033789 | (normal vs normal) | 12-120173011 | cg02952918 | (cancer vs cancer) (cancer+normal vs cancer+normal) |
| 8-27730485 | cg12116192 | (normal vs normal) | 12-120173057 | cg14578247 | (cancer vs cancer) (cancer+normal vs cancer+normal) |
| **Status - Hypo** | | | 12-120173114 | cg17338594 | (cancer vs cancer) (cancer+normal vs cancer+normal) |
| 1-164652019 | cg09168939 | (normal vs normal) | 12-123636717 | cg15175608 | (cancer vs cancer) (cancer+normal vs cancer+normal) |
| 1-201195051 | cg14095720 | (normal vs normal) | 12-1890922 | cg03841784 | (cancer vs cancer) (cancer+normal vs cancer+normal) |

| | | | | | |
|---|---|---|---|---|---|
| 1-87774698 | cg10985956 | (normal vs normal) | 12-3030602 | cg13130274 | (normal vs normal) (cancer vs cancer) (cancer+normal vs cancer+normal) |
| 10-130757223 | cg22605290 | (normal vs normal) | 14-103369717 | cg01813877 | (cancer vs cancer) (cancer+normal vs cancer+normal) |
| 10-130757242 | cg16621196 | (normal vs normal) | 14-103369786 | cg22779765 | (cancer vs cancer) (cancer+normal vs cancer+normal) |
| 10-130757617 | cg14701597 | (normal vs normal) | 14-64909119 | cg18315834 | (cancer vs cancer) (cancer+normal vs cancer+normal) |
| 10-130844121 | cg24098131 | (normal vs normal) | 14-68214742 | cg09350497 | (cancer+normal vs cancer+normal) |
| 10-130844209 | cg13303464 | (normal vs normal) | 15-40677854 | cg08036289 | (normal vs normal) (cancer vs cancer) (cancer+normal vs cancer+normal) |
| 10-130844321 | cg19825759 | (normal vs normal) | 15-45900639 | cg26410540 | (cancer+normal vs cancer+normal) |
| 10-130844727 | cg15030949 | (normal vs normal) | 15-90611758 | cg07353895 | (cancer+normal vs cancer+normal) |
| 10-130844860 | cg10652915 | (normal vs normal) | 16-2282618 | cg03929747 | (normal vs normal) (cancer vs cancer) (cancer+normal vs cancer+normal) |
| 10-130844884 | cg27596297 | (normal vs normal) | 16-2282957 | cg04152767 | (cancer vs cancer) (cancer+normal vs cancer+normal) |
| 10-130844899 | cg07646467 | (normal vs normal) (cancer+normal vs cancer+normal) | 16-23410986 | cg03856411 | (cancer vs cancer) (cancer+normal vs cancer+normal) |
| 10-132891319 | cg03625662 | (normal vs normal) | 16-27526259 | cg27372015 | (cancer+normal vs cancer+normal) |
| 10-132891341 | cg10589396 | (normal vs normal) | 16-4164087 | cg06170425 | (cancer vs cancer) (cancer+normal vs cancer+normal) |
| 10-132891371 | cg15167871 | (normal vs normal) | 16-4802848 | cg10278046 | (normal vs normal) (cancer vs cancer) (cancer+normal vs cancer+normal) |
| 10-133950900 | cg06326667 | (normal vs normal) | 16-4802990 | cg16755475 | (normal vs normal) (cancer vs cancer) (cancer+normal vs cancer+normal) |
| 10-135120603 | cg17851392 | (normal vs normal) (cancer vs cancer) | 16-57147300 | cg06880930 | (cancer vs cancer) (cancer+normal vs cancer+normal) |
| 10-2699291 | cg04633922 | (normal vs normal) | 16-87744948 | cg08734237 | (cancer+normal vs cancer+normal) |
| 10-2978687 | cg09115646 | (normal vs normal) | 16-89445503 | cg00488747 | (cancer+normal vs cancer+normal) |
| 10-77352287 | cg15969149 | (normal vs normal) | 17-30539553 | cg09774854 | (cancer+normal vs cancer+normal) |
| 10-77352376 | cg08542351 | (normal vs normal) | 17-44248233 | cg21214508 | (cancer+normal vs cancer+normal) |
| 11-110067118 | cg03893663 | (normal vs normal) | 17-452707 | cg06871344 | (cancer vs cancer) (cancer+normal vs cancer+normal) |
| 11- | cg25859670 | (normal vs normal) | 17- | cg1228079 | (normal vs normal) |

| | | | | | |
|---|---|---|---|---|---|
| 132031417 | | | 48856029 | 8 | (cancer vs cancer) (cancer+normal vs cancer+normal) |
| 11-132031665 | cg02516944 | (normal vs normal) | 17-75208309 | cg12840847 | (cancer+normal vs cancer+normal) |
| 11-133233200 | cg01140660 | (normal vs normal) | 18-77485710 | cg25249670 | (cancer+normal vs cancer+normal) |
| 11-19372014 | cg24137774 | (normal vs normal) (cancer vs cancer) | 18-8660164 | cg25899954 | (normal vs normal) (cancer vs cancer) (cancer+normal vs cancer+normal) |
| 11-7728104 | cg01229760 | (normal vs normal) | 19-4676202 | cg13513411 | (normal vs normal) (cancer vs cancer) (cancer+normal vs cancer+normal) |
| 12-105503674 | cg24630957 | (normal vs normal) | 19-4676623 | cg18328612 | (normal vs normal) (cancer vs cancer) (cancer+normal vs cancer+normal) |
| 12-132900464 | cg07385694 | (normal vs normal) | 2-10924176 | cg07921008 | (cancer+normal vs cancer+normal) |
| 12-132900480 | cg15866393 | (normal vs normal) | 2-11774852 | cg06856155 | (normal vs normal) (cancer vs cancer) (cancer+normal vs cancer+normal) |
| 12-4554940 | cg24965248 | (normal vs normal) | 2-152108040 | cg22109787 | (cancer+normal vs cancer+normal) |
| 12-4554962 | cg19018201 | (normal vs normal) | 2-239074497 | cg17459225 | (normal vs normal) (cancer+normal vs cancer+normal) |
| 13-113533425 | cg14807553 | (normal vs normal) | 2-3325158 | cg00027081 | (normal vs normal) (cancer vs cancer) (cancer+normal vs cancer+normal) |
| 13-23422746 | cg06509223 | (normal vs normal) | 2-69857465 | cg04694389 | (normal vs normal) (cancer vs cancer) (cancer+normal vs cancer+normal) |
| 13-23422801 | cg20196215 | (normal vs normal) | 20-32436418 | cg17894293 | (cancer vs cancer) (cancer+normal vs cancer+normal) |
| 13-23422850 | cg10416814 | (normal vs normal) | 20-35444275 | cg00171166 | (cancer vs cancer) (cancer+normal vs cancer+normal) |
| 13-96085969 | cg08418978 | (normal vs normal) (cancer vs cancer) | 20-35444364 | cg06814048 | (normal vs normal) (cancer vs cancer) (cancer+normal vs cancer+normal) |
| 13-96086023 | cg22122715 | (normal vs normal) (cancer vs cancer) | 20-35444560 | cg08209184 | (cancer vs cancer) (cancer+normal vs cancer+normal) |
| 13-96086098 | cg20278383 | (normal vs normal) (cancer vs cancer) (cancer vs cancer) (cancer+normal vs cancer+normal) | 21-40559999 | cg27011829 | (normal vs normal) (cancer+normal vs cancer+normal) |
| 15-25951163 | cg24602704 | (normal vs normal) | 22-42563204 | cg27157050 | (cancer vs cancer) (cancer+normal vs cancer+normal) |
| 15-70364327 | cg01666796 | (normal vs normal) (cancer vs cancer) | 22-45737514 | cg13318129 | (cancer vs cancer) (cancer+normal vs |

| | | | | | |
|---|---|---|---|---|---|
| | | (cancer+normal vs cancer+normal) | | | cancer+normal) |
| 16-12088037 | cg02498072 | (normal vs normal) (cancer vs cancer) | 3-113413263 | cg00395951 | (cancer vs cancer) (cancer+normal vs cancer+normal) |
| 16-88361441 | cg10278915 | (normal vs normal) | 3-11597868 | cg02131130 | (cancer vs cancer) (cancer+normal vs cancer+normal) |
| 16-88826522 | cg27092995 | (normal vs normal) | 3-11655466 | cg21396517 | (cancer vs cancer) (cancer+normal vs cancer+normal) |
| 16-88826569 | cg02154436 | (normal vs normal) | 3-195709698 | cg25931138 | (normal vs normal) (cancer vs cancer) (cancer+normal vs cancer+normal) |
| 16-981766 | cg04170174 | (normal vs normal) (cancer vs cancer) | 3-52737693 | cg13723011 | (normal vs normal) (cancer vs cancer) (cancer+normal vs cancer+normal) |
| 16-982031 | cg06993367 | (normal vs normal) (cancer vs cancer) | 4-169573255 | cg07979388 | (normal vs normal) (cancer vs cancer) (cancer+normal vs cancer+normal) |
| 17-18854799 | cg25567048 | (normal vs normal) (cancer vs cancer) (cancer+normal vs cancer+normal) | 4-183816164 | cg09557149 | (normal vs normal) (cancer vs cancer) (cancer+normal vs cancer+normal) |
| 17-18855313 | cg00306284 | (normal vs normal) (cancer vs cancer) (cancer+normal vs cancer+normal) | 4-184683218 | cg07396827 | (cancer vs cancer) (cancer+normal vs cancer+normal) |
| 17-18855506 | cg09632029 | (normal vs normal) (cancer vs cancer) (cancer+normal vs cancer+normal) | 4-186945339 | cg09227533 | (normal vs normal) (cancer vs cancer) (cancer+normal vs cancer+normal) |
| 17-39471907 | cg26885619 | (normal vs normal) | 4-83323585 | cg18579761 | (normal vs normal) (cancer vs cancer) (cancer+normal vs cancer+normal) |
| 17-4489504 | cg11486359 | (normal vs normal) | 5-134814626 | cg01018110 | (normal vs normal) (cancer vs cancer) (cancer+normal vs cancer+normal) |
| 17-55673870 | cg03156170 | (normal vs normal) | 5-78766902 | cg06646622 | (normal vs normal) (cancer vs cancer) (cancer+normal vs cancer+normal) |
| 17-71744924 | cg07992457 | (normal vs normal) | 6-138200309 | cg01981433 | (cancer vs cancer) (cancer+normal vs cancer+normal) |
| 17-74908714 | cg19267213 | (normal vs normal) | 6-138200387 | cg18287768 | (cancer+normal vs cancer+normal) |
| 17-75695378 | cg16145211 | (normal vs normal) | 6-169978311 | cg05066503 | (cancer vs cancer) (cancer+normal vs cancer+normal) |
| 18-74832115 | cg00989538 | (normal vs normal) | 6-169978387 | cg13211832 | (cancer vs cancer) (cancer+normal vs cancer+normal) |
| 18-74832171 | cg21116087 | (normal vs normal) (cancer vs cancer) (cancer+normal vs | 6-170028109 | cg25373438 | (cancer vs cancer) (cancer+normal vs cancer+normal) |

| | | |
|---|---|---|
| | | cancer+normal) |
| 18-74832261 | cg16990945 | (normal vs normal) (cancer vs cancer) (cancer+normal vs cancer+normal) |
| 18-76151203 | cg19355932 | (normal vs normal) |
| 19-45316493 | cg12249345 | (normal vs normal) (cancer vs cancer) (cancer+normal vs cancer+normal) |
| 19-45316509 | cg21978694 | (normal vs normal) (cancer vs cancer) (cancer+normal vs cancer+normal) |
| 19-54174606 | cg24128045 | (normal vs normal) |
| 19-55874500 | cg11225056 | (normal vs normal) |
| 2-178973079 | cg25207147 | (normal vs normal) |
| 2-27601647 | cg24655222 | (normal vs normal) |
| 2-3826980 | cg14989714 | (normal vs normal) |
| 21-46845160 | cg07529658 | (normal vs normal) |
| 3-12328740 | cg18063278 | (normal vs normal) |
| 4-100574455 | cg08577953 | (normal vs normal) |
| 4-25621615 | cg15375311 | (normal vs normal) |
| 4-40437086 | cg05617027 | (normal vs normal) (cancer vs cancer) |
| 5-2866465 | cg13945749 | (normal vs normal) |
| 5-6491884 | cg16612423 | (normal vs normal) |
| 5-73112528 | cg10572670 | (normal vs normal) (cancer vs cancer) |
| 6-160680162 | cg25545503 | (normal vs normal) (cancer vs cancer) (cancer+normal vs cancer+normal) |
| 6-170564889 | cg10832035 | (normal vs normal) |

| | | |
|---|---|---|
| 6-30094960 | cg21376799 | (normal vs normal) (cancer vs cancer) (cancer+normal vs cancer+normal) |
| 7-101522290 | cg00982974 | (normal vs normal) (cancer vs cancer) (cancer+normal vs cancer+normal) |
| 7-134613526 | cg03032816 | (cancer vs cancer) (cancer+normal vs cancer+normal) |
| 7-33725819 | cg18365383 | (normal vs normal) (cancer vs cancer) (cancer+normal vs cancer+normal) |
| 7-5529868 | cg22399555 | (cancer vs cancer) (cancer+normal vs cancer+normal) |
| 8-126202653 | cg05445772 | (cancer vs cancer) (cancer+normal vs cancer+normal) |
| 8-12860315 | cg25515982 | (normal vs normal) (cancer vs cancer) (cancer+normal vs cancer+normal) |
| 9-126153067 | cg14177289 | (cancer vs cancer) (cancer+normal vs cancer+normal) |
| 9-129648322 | cg13595556 | (normal vs normal) (cancer vs cancer) (cancer+normal vs cancer+normal) |
| **(normal vs normal)** | | |
| **Status - Hyper** | | |
| 1-16160360 | cg05960182 | (cancer vs cancer) (normal vs normal) (cancer+normal vs cancer+normal) |
| 1-167690438 | cg04846203 | (normal vs normal) (cancer+normal vs cancer+normal) |
| 1-226069189 | cg21710324 | (normal vs normal) |
| 1-227504721 | cg03890680 | (normal vs normal) |
| 1-232840764 | cg09548403 | (normal vs normal) |
| 1-59043576 | cg05346878 | (normal vs normal) |
| 1-59043873 | cg27398499 | (normal vs normal) |
| 10-104417977 | cg14255417 | (cancer vs cancer) (normal vs normal) (cancer+normal vs cancer+normal) |

| | | | | | | |
|---|---|---|---|---|---|---|
| 6-170565117 | cg08623515 | (normal vs normal) | 10-133793646 | cg25233900 | (normal vs normal) |
| 7-128550862 | cg07302677 | (normal vs normal) | 10-91061115 | cg27478224 | (normal vs normal) |
| 7-128550898 | cg17533927 | (normal vs normal) | 10-99185264 | cg25603927 | (normal vs normal) |
| 7-130130155 | cg05222671 | (normal vs normal) | 11-102401523 | cg17707274 | (normal vs normal) |
| 7-132500307 | cg13476800 | (normal vs normal) | 11-102401616 | cg01813071 | (cancer vs cancer) (normal vs normal) (cancer+normal vs cancer+normal) |
| 7-150745709 | cg03734783 | (normal vs normal) | 11-124628888 | cg02431260 | (normal vs normal) |
| 7-157280331 | cg14840782 | (normal vs normal) | 11-1974984 | cg14289294 | (normal vs normal) (cancer+normal vs cancer+normal) |
| 7-157444235 | cg15689733 | (normal vs normal) | 11-19799429 | cg06218688 | (normal vs normal) |
| 7-157444239 | cg14462553 | (normal vs normal) | 11-46582058 | cg24374161 | (normal vs normal) |
| 7-157447244 | cg16796151 | (normal vs normal) | 11-57243865 | cg12822074 | (normal vs normal) |
| 7-157655436 | cg10249637 | (normal vs normal) | 11-65487505 | cg06038201 | (cancer vs cancer) (normal vs normal) (cancer+normal vs cancer+normal) |
| 7-157688504 | cg09941017 | (normal vs normal) | 11-68607737 | cg17058475 | (normal vs normal) |
| 7-36124562 | cg02650121 | (normal vs normal) | 11-82871065 | cg04740205 | (normal vs normal) |
| 7-36124944 | cg05754377 | (normal vs normal) | 12-63026212 | cg01804193 | (normal vs normal) |
| 7-5161165 | cg05058150 | (normal vs normal) | 13-20699452 | cg05585947 | (cancer vs cancer) (normal vs normal) (cancer+normal vs cancer+normal) |
| 7-70096458 | cg18966688 | (normal vs normal) (cancer vs cancer) | 13-37249481 | cg25072733 | (normal vs normal) |
| 7-75864075 | cg16443009 | (normal vs normal) | 13-46425866 | cg08018585 | (normal vs normal) |
| 7-94289851 | cg19312058 | (normal vs normal) | 13-46425999 | cg02017534 | (normal vs normal) |
| 8-49747620 | cg03385495 | (normal vs normal) | 14-56046001 | cg27312312 | (cancer vs cancer) (normal vs normal) |
| 8-709576 | cg04027411 | (normal vs normal) | 14-56046098 | cg16907514 | (normal vs normal) |
| 8-709624 | cg09959150 | (normal vs normal) | 14-95780344 | cg01611017 | (normal vs normal) |
| 9-37340211 | cg13595739 | (normal vs normal) (cancer vs cancer) | 15-60883225 | cg00200653 | (cancer vs cancer) (normal vs normal) (cancer+normal vs cancer+normal) |
| **KiCaG (cancer vs cancer)** | | | 15-75075998 | cg05984096 | (normal vs normal) |
| **Status - Hyper** | | | 15-75076251 | cg02566978 | (normal vs normal) |
| 1-110026001 | cg08911820 | (cancer vs cancer) (cancer vs cancer) (cancer+normal vs cancer+normal) | 17-11142923 | cg07832738 | (normal vs normal) |

| | | | | | |
|---|---|---|---|---|---|
| 1-152538857 | cg24000437 | (cancer vs cancer) (cancer vs cancer) (cancer+normal vs cancer+normal) | 17-1903022 | cg23630423 | (normal vs normal) |
| 1-158324331 | cg12200412 | (cancer vs cancer) | 17-43392165 | cg00897875 | (normal vs normal) |
| 1-184357556 | cg10061342 | (cancer vs cancer) (cancer vs cancer) (cancer+normal vs cancer+normal) | 17-53809564 | cg25683268 | (normal vs normal) |
| 1-205181237 | cg24159697 | (cancer vs cancer) | 17-79503859 | cg02090762 | (normal vs normal) |
| 1-29448625 | cg08706732 | (cancer vs cancer) | 17-79503877 | cg12252135 | (normal vs normal) |
| 1-40887988 | cg26042300 | (cancer vs cancer) (cancer vs cancer) (cancer+normal vs cancer+normal) | 17-8056967 | cg04658021 | (normal vs normal) |
| 10-102588691 | cg20095124 | (cancer vs cancer) (normal vs normal) | 18-11752904 | cg15069823 | (normal vs normal) |
| 10-102588959 | cg17480467 | (cancer vs cancer) (cancer vs cancer) (normal vs normal) (cancer+normal vs cancer+normal) | 18-76002290 | cg24228532 | (normal vs normal) |
| 10-102589102 | cg14778235 | (cancer vs cancer) (cancer vs cancer) (normal vs normal) (cancer+normal vs cancer+normal) | 2-178013040 | cg04320629 | (normal vs normal) |
| 10-102589250 | cg08217716 | (cancer vs cancer) (cancer vs cancer) (normal vs normal) (cancer+normal vs cancer+normal) | 2-238583504 | cg20218460 | (normal vs normal) |
| 10-129534217 | cg18047662 | (cancer vs cancer) | 2-24715213 | cg11380327 | (cancer vs cancer) (normal vs normal) (cancer+normal vs cancer+normal) |
| 10-134801824 | cg10492836 | (cancer vs cancer) | 21-41032396 | cg26390598 | (cancer vs cancer) (normal vs normal) (cancer+normal vs cancer+normal) |
| 10-375391 | cg06193766 | (cancer vs cancer) (cancer vs cancer) (cancer+normal vs cancer+normal) | 21-46126104 | cg11250081 | (normal vs normal) |
| 11-16023708 | cg10825530 | (cancer vs cancer) | 21-46129392 | cg19683410 | (normal vs normal) |
| 11-33951810 | cg09951637 | (cancer vs cancer) | 22-46451214 | cg14425733 | (normal vs normal) |
| 11-7728814 | cg13563074 | (cancer vs cancer) (cancer vs cancer) (cancer+normal vs cancer+normal) | 22-46454012 | cg22982767 | (normal vs normal) |
| 12-10022682 | cg08752459 | (cancer vs cancer) (cancer vs cancer) (normal vs normal) (cancer+normal vs cancer+normal) | 22-46471129 | cg27338353 | (cancer vs cancer) (normal vs normal) (cancer+normal vs cancer+normal) |
| 12- | cg12591315 | (cancer vs cancer) | 3- | cg1014828 | (normal vs normal) |

| 10022688 | | (cancer vs cancer) (normal vs normal) (cancer+normal vs cancer+normal) | 148846750 | 0 | |
| --- | --- | --- | --- | --- | --- |
| 12-120525338 | cg03800721 | (cancer vs cancer) (cancer vs cancer) (normal vs normal) (cancer+normal vs cancer+normal) | 3-15839627 | cg25572043 | (cancer vs cancer) (normal vs normal) (cancer+normal vs cancer+normal) |
| 12-121088408 | cg25969992 | (cancer vs cancer) (cancer vs cancer) (cancer+normal vs cancer+normal) | 3-172467326 | cg21643939 | (normal vs normal) |
| 12-124418532 | cg14808040 | (cancer vs cancer) | 3-181045270 | cg25436634 | (normal vs normal) |
| 12-131417943 | cg23795633 | (cancer vs cancer) | 3-182208536 | cg01465177 | (cancer vs cancer) (normal vs normal) (cancer+normal vs cancer+normal) |
| 12-52214119 | cg19495013 | (cancer vs cancer) (cancer vs cancer) (cancer+normal vs cancer+normal) | 3-3219781 | cg00294109 | (normal vs normal) |
| 12-52214320 | cg07375978 | (cancer vs cancer) (cancer vs cancer) (cancer+normal vs cancer+normal) | 3-44039204 | cg19444609 | (cancer vs cancer) (normal vs normal) (cancer+normal vs cancer+normal) |
| 12-54371246 | cg27178293 | (cancer vs cancer) | 3-44039289 | cg19890879 | (normal vs normal) |
| 12-54422127 | cg12232783 | (cancer vs cancer) (cancer vs cancer) (cancer+normal vs cancer+normal) | 3-44039357 | cg07289306 | (cancer vs cancer) (normal vs normal) (cancer+normal vs cancer+normal) |
| 12-54422350 | cg27441225 | (cancer vs cancer) (cancer vs cancer) (cancer+normal vs cancer+normal) | 3-44039509 | cg01856525 | (cancer vs cancer) (normal vs normal) |
| 12-54422488 | cg22378817 | (cancer vs cancer) (cancer vs cancer) (cancer+normal vs cancer+normal) | 3-44039578 | cg03889542 | (cancer vs cancer) (normal vs normal) |
| 12-54427636 | cg16983211 | (cancer vs cancer) (cancer vs cancer) (cancer+normal vs cancer+normal) | 3-44039919 | cg17614974 | (normal vs normal) |
| 12-54427700 | cg15700739 | (cancer vs cancer) (cancer vs cancer) (cancer+normal vs cancer+normal) | 3-44517846 | cg17219326 | (normal vs normal) |
| 12-55725991 | cg11794356 | (cancer vs cancer) | 3-73671377 | cg07146912 | (normal vs normal) |
| 13-113598068 | cg01866427 | (cancer vs cancer) (cancer vs cancer) (cancer+normal vs cancer+normal) | 3-78084695 | cg04931126 | (normal vs normal) |
| 13-113623233 | cg02223067 | (cancer vs cancer) (cancer vs cancer) (cancer+normal vs cancer+normal) | 4-184828386 | cg23965931 | (cancer vs cancer) (normal vs normal) |
| 13-113623300 | cg16737670 | (cancer vs cancer) (cancer vs cancer) | 4-186316674 | cg06277849 | (cancer vs cancer) (normal vs normal) |

| | | | | | |
|---|---|---|---|---|---|
| | | (cancer+normal vs cancer+normal) | | | (cancer+normal vs cancer+normal) |
| 13-113623639 | cg00127894 | (cancer vs cancer) (cancer vs cancer) (normal vs normal) (cancer+normal vs cancer+normal) | 5-100237986 | cg0186006 3 | (normal vs normal) |
| 13-113623659 | cg15179805 | (cancer vs cancer) (cancer vs cancer) (cancer+normal vs cancer+normal) | 5-115175001 | cg1751928 0 | (cancer vs cancer) (normal vs normal) (cancer+normal vs cancer+normal) |
| 13-113623844 | cg09678836 | (cancer vs cancer) (cancer vs cancer) (normal vs normal) (cancer+normal vs cancer+normal) | 5-121648993 | cg0719530 1 | (cancer vs cancer) (normal vs normal) (cancer+normal vs cancer+normal) |
| 13-22836354 | cg26087651 | (cancer vs cancer) | 5-134075279 | cg0599815 3 | (cancer vs cancer) (normal vs normal) (cancer+normal vs cancer+normal) |
| 15-37111929 | cg07398661 | (cancer vs cancer) | 5-139016709 | cg1747376 5 | (normal vs normal) |
| 15-69887053 | cg24340572 | (cancer vs cancer) (normal vs normal) | 5-42858327 | cg1469765 7 | (normal vs normal) (cancer+normal vs cancer+normal) |
| 15-70767570 | cg16973527 | (cancer vs cancer) (cancer vs cancer) (cancer+normal vs cancer+normal) | 5-54932693 | cg2654000 4 | (normal vs normal) |
| 15-70767649 | cg13427748 | (cancer vs cancer) (cancer vs cancer) (cancer+normal vs cancer+normal) | 5-8461832 | cg0783277 2 | (normal vs normal) |
| 16-86387131 | cg26757472 | (cancer vs cancer) | 6-113012601 | cg2544114 1 | (cancer vs cancer) (normal vs normal) (cancer+normal vs cancer+normal) |
| 17-18908235 | cg14807365 | (cancer vs cancer) (cancer vs cancer) (cancer+normal vs cancer+normal) | 6-116381976 | cg1522627 5 | (normal vs normal) |
| 17-18908242 | cg22220165 | (cancer vs cancer) (cancer vs cancer) (cancer+normal vs cancer+normal) | 6-160112632 | cg1322145 8 | (normal vs normal) (cancer+normal vs cancer+normal) |
| 17-35289543 | cg16293732 | (cancer vs cancer) (cancer vs cancer) (cancer+normal vs cancer+normal) | 6-170125491 | cg0765223 7 | (normal vs normal) |
| 17-5673550 | cg06683719 | (cancer vs cancer) (cancer vs cancer) | 6-25035073 | cg1317532 9 | (normal vs normal) |
| 17-7080538 | cg20752878 | (cancer vs cancer) | 7-116592968 | cg1573085 7 | (normal vs normal) |
| 17-7790179 | cg21068610 | (cancer vs cancer) (cancer vs cancer) (cancer+normal vs cancer+normal) | 7-1293933 | cg1015543 2 | (normal vs normal) |
| 19-11199917 | cg18596381 | (cancer vs cancer) (cancer vs cancer) (cancer+normal vs cancer+normal) | 7-134832850 | cg0645603 1 | (normal vs normal) |

| | | | | | |
|---|---|---|---|---|---|
| 19-11199944 | cg19751789 | (cancer vs cancer) (cancer vs cancer) (cancer+normal vs cancer+normal) | 7-1572571 | cg26468430 | (cancer vs cancer) (normal vs normal) (cancer+normal vs cancer+normal) |
| 19-11199965 | cg12013591 | (cancer vs cancer) (cancer vs cancer) (cancer+normal vs cancer+normal) | 7-75956931 | cg03707599 | (normal vs normal) |
| 19-14785849 | cg15746620 | (cancer vs cancer) (cancer vs cancer) (cancer+normal vs cancer+normal) | 7-79780310 | cg25418001 | (cancer vs cancer) (normal vs normal) (cancer+normal vs cancer+normal) |
| 19-2639424 | cg26988138 | (cancer vs cancer) (normal vs normal) (cancer+normal vs cancer+normal) | 7-94284258 | cg27504782 | (normal vs normal) |
| 2-10263480 | cg00506866 | (cancer vs cancer) (cancer vs cancer) (cancer+normal vs cancer+normal) | 7-98739698 | cg17146738 | (normal vs normal) |
| 2-10263696 | cg19516340 | (cancer vs cancer) (cancer+normal vs cancer+normal) | 8-1068250 | cg14041855 | (normal vs normal) |
| 2-121496875 | cg20219891 | (cancer vs cancer) (cancer vs cancer) (cancer+normal vs cancer+normal) | 8-116660311 | cg13270873 | (normal vs normal) |
| 2-34633637 | cg18968409 | (cancer vs cancer) | 8-127568650 | cg06023345 | (cancer vs cancer) (normal vs normal) (cancer+normal vs cancer+normal) |
| 2-50718584 | cg12884814 | (cancer vs cancer) | 8-127568677 | cg01923999 | (cancer vs cancer) (normal vs normal) (cancer+normal vs cancer+normal) |
| 2-85648512 | cg16256243 | (cancer vs cancer) | 8-17519419 | cg14709253 | (normal vs normal) |
| 20-29993663 | cg08292485 | (cancer vs cancer) | 8-29210790 | cg15944766 | (normal vs normal) |
| 20-42879935 | cg08460399 | (cancer vs cancer) | 8-29210800 | cg00510870 | (normal vs normal) |
| 20-58983676 | cg01360605 | (cancer vs cancer) | 8-37595363 | cg14036776 | (normal vs normal) |
| 20-62084626 | cg01575590 | (cancer vs cancer) | 8-80679314 | cg03301084 | (cancer vs cancer) (normal vs normal) (cancer+normal vs cancer+normal) |
| 22-30195299 | cg14742445 | (cancer vs cancer) (cancer+normal vs cancer+normal) | 8-81491905 | cg20629254 | (normal vs normal) |
| 3-132036382 | cg00589251 | (cancer vs cancer) (cancer vs cancer) (cancer+normal vs cancer+normal) | 8-93107298 | cg07538339 | (cancer vs cancer) (normal vs normal) (cancer+normal vs cancer+normal) |
| 4-111530471 | cg15903185 | (cancer vs cancer) (cancer vs cancer) (cancer+normal vs cancer+normal) | 9-89560185 | cg02501714 | (cancer vs cancer) (normal vs normal) (cancer+normal vs cancer+normal) |
| 4-9917839 | cg14959260 | (cancer vs cancer) | **Status - Hypo** | | |
| 5-1406177 | cg24756227 | (cancer vs cancer) (cancer+normal vs | 1-10676290 | cg26919387 | (normal vs normal) (cancer vs cancer) |

| | | | | | |
|---|---|---|---|---|---|
| | | cancer+normal) | | | (cancer+normal vs cancer+normal) |
| 5-162516650 | cg18086386 | (cancer vs cancer) (cancer vs cancer) (cancer+normal vs cancer+normal) | 1-113819171 | cg23232873 | (normal vs normal) (cancer vs cancer) (cancer+normal vs cancer+normal) |
| 5-5033993 | cg08596532 | (cancer vs cancer) | 1-182570870 | cg08113628 | (normal vs normal) (cancer vs cancer) (cancer+normal vs cancer+normal) |
| 5-66299786 | cg02632185 | (cancer vs cancer) (cancer vs cancer) (normal vs normal) (cancer+normal vs cancer+normal) | 1-28504285 | cg07821160 | (normal vs normal) |
| 5-66299875 | cg18118497 | (cancer vs cancer) (cancer vs cancer) (cancer+normal vs cancer+normal) | 1-3159645 | cg13867039 | (normal vs normal) |
| 5-66300406 | cg17449851 | (cancer vs cancer) (cancer vs cancer) (normal vs normal) (cancer+normal vs cancer+normal) | 1-58622038 | cg18229457 | (normal vs normal) (cancer vs cancer) (cancer+normal vs cancer+normal) |
| 5-66300433 | cg11831286 | (cancer vs cancer) (cancer vs cancer) (normal vs normal) (cancer+normal vs cancer+normal) | 1-8045077 | cg14531038 | (normal vs normal) |
| 5-66300503 | cg27116842 | (cancer vs cancer) (cancer vs cancer) (normal vs normal) (cancer+normal vs cancer+normal) | 10-121271746 | cg16596052 | (normal vs normal) (cancer+normal vs cancer+normal) |
| 5-72598192 | cg25113089 | (cancer vs cancer) (normal vs normal) | 10-121271840 | cg00644351 | (normal vs normal) |
| 5-75753776 | cg22987011 | (cancer vs cancer) | 10-131200784 | cg16960573 | (normal vs normal) (cancer vs cancer) (cancer+normal vs cancer+normal) |
| 6-134216081 | cg07571531 | (cancer vs cancer) (cancer vs cancer) (cancer+normal vs cancer+normal) | 10-131200802 | cg26328291 | (normal vs normal) (cancer vs cancer) |
| 6-28892465 | cg23756442 | (cancer vs cancer) (cancer vs cancer) (cancer+normal vs cancer+normal) | 10-131200834 | cg27014522 | (normal vs normal) |
| 6-39393690 | cg04321126 | (cancer vs cancer) | 10-134559553 | cg11642140 | (normal vs normal) (cancer vs cancer) (cancer+normal vs cancer+normal) |
| 7-128530795 | cg18323749 | (cancer vs cancer) | 10-134559637 | cg17750946 | (normal vs normal) (cancer vs cancer) (cancer+normal vs cancer+normal) |
| 7-128530800 | cg08922068 | (cancer vs cancer) | 10-134559696 | cg07927953 | (normal vs normal) (cancer vs cancer) (cancer+normal vs cancer+normal) |
| 7- | cg07039423 | (cancer vs cancer) | 10- | cg1705478 | (normal vs normal) |

| | | | | | |
|---|---|---|---|---|---|
| 128531106 | | (normal vs normal) | 134559939 | 3 | (cancer vs cancer) (cancer+normal vs cancer+normal) |
| 7-128531165 | cg22939733 | (cancer vs cancer) | 10-76154015 | cg09299075 | (normal vs normal) |
| 7-128531479 | cg00682002 | (cancer vs cancer) | 11-111167086 | cg10131836 | (normal vs normal) (cancer vs cancer) (cancer+normal vs cancer+normal) |
| 7-92747308 | cg08986767 | (cancer vs cancer) (cancer vs cancer) (cancer+normal vs cancer+normal) | 11-129722477 | cg11985341 | (normal vs normal) |
| 8-107669778 | cg07554329 | (cancer vs cancer) (cancer vs cancer) (cancer+normal vs cancer+normal) | 11-134335356 | cg05052869 | (normal vs normal) |
| 8-107669783 | cg17136799 | (cancer vs cancer) (cancer vs cancer) (cancer+normal vs cancer+normal) | 11-35252384 | cg15427520 | (normal vs normal) (cancer vs cancer) (cancer+normal vs cancer+normal) |
| 8-107669787 | cg26622232 | (cancer vs cancer) (cancer vs cancer) (cancer+normal vs cancer+normal) | 11-3638458 | cg02453348 | (normal vs normal) (cancer+normal vs cancer+normal) |
| 8-107669856 | cg17176732 | (cancer vs cancer) (cancer vs cancer) (cancer+normal vs cancer+normal) | 11-63870619 | cg08788438 | (normal vs normal) |
| 8-107669906 | cg17212289 | (cancer vs cancer) (cancer vs cancer) (cancer+normal vs cancer+normal) | 11-75852355 | cg07783183 | (normal vs normal) (cancer+normal vs cancer+normal) |
| 8-107670161 | cg16326979 | (cancer vs cancer) (cancer vs cancer) (cancer+normal vs cancer+normal) | 11-75852389 | cg24630516 | (normal vs normal) (cancer vs cancer) (cancer+normal vs cancer+normal) |
| 8-25546737 | cg06162872 | (cancer vs cancer) | 11-78638670 | cg19234412 | (normal vs normal) (cancer vs cancer) (cancer+normal vs cancer+normal) |
| 8-55294610 | cg13242160 | (cancer vs cancer) (cancer vs cancer) (cancer+normal vs cancer+normal) | 12-109911508 | cg22714942 | (normal vs normal) (cancer vs cancer) (cancer+normal vs cancer+normal) |
| 8-55294721 | cg07664990 | (cancer vs cancer) (cancer vs cancer) (cancer+normal vs cancer+normal) | 12-130961949 | cg26491818 | (normal vs normal) |
| 8-55294823 | cg24039394 | (cancer vs cancer) | 12-132901741 | cg14288890 | (normal vs normal) |
| 8-55294882 | cg10239077 | (cancer vs cancer) | 12-3030602 | cg13130274 | (normal vs normal) (cancer vs cancer) (cancer+normal vs cancer+normal) |
| 9-138605049 | cg14341131 | (cancer vs cancer) (cancer vs cancer) (cancer+normal vs cancer+normal) | 12-7025947 | cg27528330 | (normal vs normal) |
| **Status - Hypo** | | | 13-105987461 | cg04089637 | (normal vs normal) |

| | | | | | |
|---|---|---|---|---|---|
| 1-120174801 | cg17501774 | (cancer vs cancer) | 13-113488452 | cg25678150 | (normal vs normal) |
| 1-217724849 | cg10325088 | (cancer vs cancer) (cancer vs cancer) (cancer+normal vs cancer+normal) | 14-51171931 | cg18508389 | (normal vs normal) |
| 1-233497903 | cg02452586 | (cancer vs cancer) | 14-68985557 | cg11064738 | (normal vs normal) |
| 1-233497916 | cg09347582 | (cancer vs cancer) | 14-80676940 | cg09005221 | (normal vs normal) |
| 1-8931135 | cg13785123 | (cancer vs cancer) | 15-40677854 | cg08036289 | (normal vs normal) (cancer vs cancer) (cancer+normal vs cancer+normal) |
| 10-135120603 | cg17851392 | (normal vs normal) (cancer vs cancer) | 15-66541975 | cg07575407 | (normal vs normal) |
| 10-135120640 | cg04070692 | (cancer vs cancer) | 16-2282618 | cg03929747 | (normal vs normal) (cancer vs cancer) (cancer+normal vs cancer+normal) |
| 10-29811563 | cg11801524 | (cancer vs cancer) | 16-4802848 | cg10278046 | (normal vs normal) (cancer vs cancer) (cancer+normal vs cancer+normal) |
| 10-94820376 | cg07225966 | (cancer vs cancer) | 16-4802990 | cg16755475 | (normal vs normal) (cancer vs cancer) (cancer+normal vs cancer+normal) |
| 11-108018824 | cg07364154 | (cancer vs cancer) | 16-58031050 | cg21757440 | (normal vs normal) |
| 11-118477406 | cg11286122 | (cancer vs cancer) | 16-87744906 | cg27459530 | (normal vs normal) |
| 11-1781557 | cg00509007 | (cancer vs cancer) | 17-48856029 | cg12280798 | (normal vs normal) (cancer vs cancer) (cancer+normal vs cancer+normal) |
| 11-19372014 | cg24137774 | (normal vs normal) (cancer vs cancer) | 17-74671934 | cg04608811 | (normal vs normal) |
| 11-2705153 | cg01693193 | (cancer vs cancer) | 18-30347555 | cg03477049 | (normal vs normal) |
| 11-64323816 | cg21039563 | (cancer vs cancer) (cancer vs cancer) (cancer+normal vs cancer+normal) | 18-8659964 | cg19305718 | (normal vs normal) |
| 11-945202 | cg03278299 | (cancer vs cancer) | 18-8660164 | cg25899954 | (normal vs normal) (cancer vs cancer) (cancer+normal vs cancer+normal) |
| 12-111909145 | cg07058694 | (cancer vs cancer) | 19-46033285 | cg15147841 | (normal vs normal) |
| 12-14617656 | cg06912420 | (cancer vs cancer) | 19-46307734 | cg11276053 | (normal vs normal) |
| 12-64403751 | cg11362183 | (cancer vs cancer) | 19-46307846 | cg02359773 | (normal vs normal) |
| 12-69417830 | cg11681462 | (cancer vs cancer) | 19-4676202 | cg13513411 | (normal vs normal) (cancer vs cancer) (cancer+normal vs cancer+normal) |
| 12-7121914 | cg24859375 | (cancer vs cancer) (cancer vs cancer) (cancer+normal vs | 19-4676623 | cg18328612 | (normal vs normal) (cancer vs cancer) (cancer+normal vs |

| | | | | | |
|---|---|---|---|---|---|
| | | cancer+normal) | | | cancer+normal) |
| 13-111880998 | cg21980364 | (cancer vs cancer) (cancer vs cancer) (cancer+normal vs cancer+normal) | 2-11774474 | cg17595386 | (normal vs normal) |
| 13-113977332 | cg12557626 | (cancer vs cancer) | 2-11774852 | cg06856155 | (normal vs normal) (cancer vs cancer) (cancer+normal vs cancer+normal) |
| 13-114138225 | cg21708867 | (cancer vs cancer) | 2-1417109 | cg16016036 | (normal vs normal) |
| 13-114138260 | cg21238257 | (cancer vs cancer) (cancer vs cancer) (cancer+normal vs cancer+normal) | 2-1417153 | cg12680131 | (normal vs normal) |
| 13-96085969 | cg08418978 | (normal vs normal) (cancer vs cancer) | 2-1417164 | cg06500727 | (normal vs normal) |
| 13-96086023 | cg22122715 | (normal vs normal) (cancer vs cancer) | 2-1417431 | cg10370591 | (normal vs normal) |
| 13-96086098 | cg20278383 | (normal vs normal) (cancer vs cancer) (cancer vs cancer) (cancer+normal vs cancer+normal) | 2-1418073 | cg23136645 | (normal vs normal) |
| 13-96086182 | cg27586249 | (cancer vs cancer) | 2-160917029 | cg03641300 | (normal vs normal) |
| 13-96086275 | cg04246521 | (cancer vs cancer) | 2-175431800 | cg04789761 | (normal vs normal) |
| 14-68592625 | cg07067667 | (cancer vs cancer) | 2-208331979 | cg08826345 | (normal vs normal) |
| 14-75549272 | cg04246305 | (cancer vs cancer) | 2-239074497 | cg17459225 | (normal vs normal) (cancer+normal vs cancer+normal) |
| 15-101728611 | cg04861869 | (cancer vs cancer) | 2-242004824 | cg17257554 | (normal vs normal) |
| 15-101728653 | cg14397171 | (cancer vs cancer) | 2-26986379 | cg24248317 | (normal vs normal) |
| 15-31280513 | cg24405716 | (cancer vs cancer) | 2-3325158 | cg00027081 | (normal vs normal) (cancer vs cancer) (cancer+normal vs cancer+normal) |
| 15-63610813 | cg25634457 | (cancer vs cancer) | 2-39876715 | cg06149733 | (normal vs normal) |
| 15-63875268 | cg24310913 | (cancer vs cancer) | 2-61809639 | cg03427577 | (normal vs normal) |
| 15-69590255 | cg20082641 | (cancer vs cancer) | 2-69857465 | cg04694389 | (normal vs normal) (cancer vs cancer) (cancer+normal vs cancer+normal) |
| 16-12088037 | cg02498072 | (normal vs normal) (cancer vs cancer) | 20-35444364 | cg06814048 | (normal vs normal) (cancer vs cancer) (cancer+normal vs cancer+normal) |
| 16-15127193 | cg06978461 | (cancer vs cancer) (cancer vs cancer) (cancer+normal vs cancer+normal) | 21-40559999 | cg27011829 | (normal vs normal) (cancer+normal vs cancer+normal) |
| 16-30425679 | cg04908960 | (cancer vs cancer) | 3-195709698 | cg25931138 | (normal vs normal) (cancer vs cancer) (cancer+normal vs cancer+normal) |

| | | | | | |
|---|---|---|---|---|---|
| 16-533228 | cg08591538 | (cancer vs cancer) | 3-46605616 | cg00285902 | (normal vs normal) |
| 16-81565917 | cg03212183 | (cancer vs cancer)<br>(cancer vs cancer)<br>(cancer+normal vs cancer+normal) | 3-52737693 | cg13723011 | (normal vs normal)<br>(cancer vs cancer)<br>(cancer+normal vs cancer+normal) |
| 16-86704130 | cg27391627 | (cancer vs cancer) | 4-169573255 | cg07979388 | (normal vs normal)<br>(cancer vs cancer)<br>(cancer+normal vs cancer+normal) |
| 16-981766 | cg04170174 | (normal vs normal)<br>(cancer vs cancer) | 4-183816164 | cg09557149 | (normal vs normal)<br>(cancer vs cancer)<br>(cancer+normal vs cancer+normal) |
| 16-982031 | cg06993367 | (normal vs normal)<br>(cancer vs cancer) | 4-186945215 | cg19945912 | (normal vs normal) |
| 17-18854760 | cg18449048 | (cancer vs cancer)<br>(cancer vs cancer)<br>(cancer+normal vs cancer+normal) | 4-186945339 | cg09227533 | (normal vs normal)<br>(cancer vs cancer)<br>(cancer+normal vs cancer+normal) |
| 17-18854799 | cg25567048 | (normal vs normal)<br>(cancer vs cancer)<br>(cancer+normal vs cancer+normal) | 4-83323585 | cg18579761 | (normal vs normal)<br>(cancer vs cancer)<br>(cancer+normal vs cancer+normal) |
| 17-25819112 | cg08041350 | (cancer vs cancer) | 5-134814626 | cg01018110 | (normal vs normal)<br>(cancer vs cancer)<br>(cancer+normal vs cancer+normal) |
| 17-37853032 | cg08585669 | (cancer vs cancer) | 5-177964757 | cg26994283 | (normal vs normal) |
| 17-78700928 | cg06975080 | (cancer vs cancer)<br>(cancer vs cancer)<br>(cancer+normal vs cancer+normal) | 5-1927225 | cg14670938 | (normal vs normal) |
| 19-10463831 | cg21480173 | (cancer vs cancer) | 5-53915380 | cg05664938 | (normal vs normal) |
| 19-10464320 | cg18038269 | (cancer vs cancer) | 5-78766902 | cg06646622 | (normal vs normal)<br>(cancer vs cancer)<br>(cancer+normal vs cancer+normal) |
| 19-10464711 | cg11575644 | (cancer vs cancer) | 6-126465314 | cg05421965 | (normal vs normal) |
| 19-1961185 | cg25367139 | (cancer vs cancer) | 6-151258451 | cg00619628 | (normal vs normal) |
| 19-1961290 | cg22278901 | (cancer vs cancer) | 6-1523823 | cg22247136 | (normal vs normal) |
| 2-121412005 | cg13283952 | (cancer vs cancer) | 6-30094960 | cg21376799 | (normal vs normal)<br>(cancer vs cancer)<br>(cancer+normal vs cancer+normal) |
| 2-121412139 | cg04864807 | (cancer vs cancer) | 7-101522290 | cg00982974 | (normal vs normal)<br>(cancer vs cancer)<br>(cancer+normal vs cancer+normal) |
| 2-180125932 | cg11432488 | (cancer vs cancer) | 7-126165242 | cg14691364 | (normal vs normal) |
| 2-201218948 | cg26015513 | (cancer vs cancer)<br>(cancer vs cancer)<br>(cancer+normal vs cancer+normal) | 7-157778531 | cg20985897 | (normal vs normal) |
| 2- | cg02371631 | (cancer vs cancer) | 7-33725819 | cg1836538 | (normal vs normal) |

| | | | | | |
|---|---|---|---|---|---|
| 202748718 | | | | 3 | (cancer vs cancer) (cancer+normal vs cancer+normal) |
| 2-217403190 | cg09473180 | (cancer vs cancer) (cancer vs cancer) (cancer+normal vs cancer+normal) | 7-47679990 | cg14201717 | (normal vs normal) |
| 2-223538875 | cg26186249 | (cancer vs cancer) | 7-6682784 | cg11026987 | (normal vs normal) |
| 2-44552508 | cg19752143 | (cancer vs cancer) | 8-12860315 | cg25515982 | (normal vs normal) (cancer vs cancer) (cancer+normal vs cancer+normal) |
| 2-73338081 | cg16936561 | (cancer vs cancer) | 8-2112392 | cg23935616 | (normal vs normal) |
| 2-99917669 | cg14245791 | (cancer vs cancer) | 9-129648322 | cg13595556 | (normal vs normal) (cancer vs cancer) (cancer+normal vs cancer+normal) |
| 20-33100246 | cg07733918 | (cancer vs cancer) | 9-139847558 | cg19534945 | (normal vs normal) |
| 20-34734501 | cg22423984 | (cancer vs cancer) | 9-16214627 | cg14612303 | (normal vs normal) |
| 20-6025272 | cg21209310 | (cancer vs cancer) (cancer vs cancer) (cancer+normal vs cancer+normal) | **UCEC (cancer vs cancer)** | | |
| 22-19751654 | cg19092981 | (cancer vs cancer) | **Status - Hyper** | | |
| 3-149051159 | cg11011913 | (cancer vs cancer) (cancer vs cancer) (cancer+normal vs cancer+normal) | 1-156815274 | cg13574390 | (cancer vs cancer) |
| 3-183722448 | cg09230938 | (cancer vs cancer) | 1-219347021 | cg26757053 | (cancer vs cancer) |
| 4-148936704 | cg26017679 | (cancer vs cancer) | 1-232940990 | cg02191044 | (cancer vs cancer) (cancer+normal vs cancer+normal) |
| 4-187729190 | cg14022183 | (cancer vs cancer) | 1-232941055 | cg02903907 | (cancer vs cancer) |
| 4-40437086 | cg05617027 | (normal vs normal) (cancer vs cancer) | 1-232941253 | cg15542798 | (cancer vs cancer) |
| 4-77877357 | cg17864469 | (cancer vs cancer) | 1-67218080 | cg11824639 | (cancer vs cancer) |
| 4-77891275 | cg21520613 | (cancer vs cancer) | 1-67218090 | cg09318375 | (cancer vs cancer) |
| 4-87993109 | cg27000934 | (cancer vs cancer) (cancer vs cancer) (cancer+normal vs cancer+normal) | 10-122708532 | cg05627029 | (cancer vs cancer) |
| 5-106859865 | cg22862094 | (cancer vs cancer) | 10-124639132 | cg12474023 | (cancer vs cancer) (cancer+normal vs cancer+normal) |
| 5-148822491 | cg11159417 | (cancer vs cancer) | 12-93966317 | cg11225410 | (cancer vs cancer) (cancer+normal vs cancer+normal) |
| 5-73112528 | cg10572670 | (normal vs normal) (cancer vs cancer) | 12-93966485 | cg22125370 | (cancer vs cancer) (cancer+normal vs cancer+normal) |
| 6-108928210 | cg03944099 | (cancer vs cancer) | 14-103655424 | cg23418339 | (cancer vs cancer) |

| | | | | | |
|---|---|---|---|---|---|
| 6-30885008 | cg16036022 | (cancer vs cancer) | 14-45432309 | cg05194618 | (cancer vs cancer) |
| 6-30885481 | cg15673491 | (cancer vs cancer) | 16-23724485 | cg16364709 | (cancer vs cancer) (cancer+normal vs cancer+normal) |
| 7-101556684 | cg15600935 | (cancer vs cancer) (cancer vs cancer) (cancer+normal vs cancer+normal) | 17-42030287 | cg01703581 | (cancer vs cancer) (cancer+normal vs cancer+normal) |
| 7-101789639 | cg06294954 | (cancer vs cancer) | 17-42030479 | cg25830696 | (cancer vs cancer) |
| 7-101789685 | cg00567872 | (cancer vs cancer) | 17-42091713 | cg12373208 | (cancer vs cancer) (cancer+normal vs cancer+normal) |
| 7-1055600 | cg20821713 | (cancer vs cancer) | 17-42091909 | cg04879755 | (cancer vs cancer) (cancer+normal vs cancer+normal) |
| 7-130129946 | cg17706086 | (cancer vs cancer) | 17-42092187 | cg18801599 | (cancer vs cancer) (cancer+normal vs cancer+normal) |
| 7-133451621 | cg00148025 | (cancer vs cancer) | 17-42092247 | cg24638647 | (cancer vs cancer) (cancer+normal vs cancer+normal) |
| 7-47193871 | cg16564132 | (cancer vs cancer) | 17-42092315 | cg27116819 | (cancer vs cancer) (cancer+normal vs cancer+normal) |
| 7-47579217 | cg04089246 | (cancer vs cancer) | 17-42092370 | cg08372947 | (cancer vs cancer) (cancer+normal vs cancer+normal) |
| 7-70096458 | cg18966688 | (normal vs normal) (cancer vs cancer) | 17-42092403 | cg06511389 | (cancer vs cancer) (cancer+normal vs cancer+normal) |
| 7-807608 | cg09533868 | (cancer vs cancer) | 17-42092431 | cg12259256 | (cancer vs cancer) (cancer+normal vs cancer+normal) |
| 7-811206 | cg07803375 | (cancer vs cancer) | 17-42092450 | cg13847066 | (cancer vs cancer) (cancer+normal vs cancer+normal) |
| 8-141147681 | cg17018549 | (cancer vs cancer) (cancer vs cancer) (cancer+normal vs cancer+normal) | 17-42092472 | cg01635193 | (cancer vs cancer) (cancer+normal vs cancer+normal) |
| 8-142310710 | cg26714129 | (cancer vs cancer) | 17-42092488 | cg25868286 | (cancer vs cancer) (cancer+normal vs cancer+normal) |
| 8-30209391 | cg14895559 | (cancer vs cancer) (cancer vs cancer) (cancer+normal vs cancer+normal) | 17-42092588 | cg01986131 | (cancer vs cancer) (cancer+normal vs cancer+normal) |
| 8-30341765 | cg13172905 | (cancer vs cancer) | 17-58498871 | cg10179315 | (cancer vs cancer) (cancer+normal vs cancer+normal) |
| 9-37340211 | cg13595739 | (normal vs normal) (cancer vs cancer) | 17-9479910 | cg25964007 | (cancer vs cancer) (cancer+normal vs cancer+normal) |
| **LAML (cancer vs cancer)** | | | 17-9479912 | cg12610887 | (cancer vs cancer) (cancer+normal vs cancer+normal) |
| **Status - Hyper** | | | 18-5238183 | cg05390530 | (cancer vs cancer) (cancer+normal vs cancer+normal) |

| | | | | | |
|---|---|---|---|---|---|
| 1-149908313 | cg13476072 | (cancer vs cancer) (cancer+normal vs cancer+normal) | 18-5238200 | cg14291066 | (cancer vs cancer) (cancer+normal vs cancer+normal) |
| 1-149908524 | cg00777271 | (cancer vs cancer) (cancer+normal vs cancer+normal) | 18-5238219 | cg15258847 | (cancer vs cancer) (cancer+normal vs cancer+normal) |
| 1-149908781 | cg24620905 | (cancer vs cancer) (cancer+normal vs cancer+normal) | 18-5238310 | cg20757519 | (cancer vs cancer) (cancer+normal vs cancer+normal) |
| 1-164581169 | cg20812370 | (cancer vs cancer) (cancer+normal vs cancer+normal) | 18-5238589 | cg10885961 | (cancer vs cancer) (cancer+normal vs cancer+normal) |
| 1-205270559 | cg03466326 | (cancer vs cancer) (cancer+normal vs cancer+normal) | 18-5238608 | cg21311023 | (cancer vs cancer) (cancer+normal vs cancer+normal) |
| 1-211643515 | cg05352688 | (cancer vs cancer) (cancer+normal vs cancer+normal) | 18-5238708 | cg20134241 | (cancer vs cancer) (cancer+normal vs cancer+normal) |
| 1-221613658 | cg24686918 | (cancer vs cancer) (cancer+normal vs cancer+normal) | 19-44325008 | cg17711567 | (cancer vs cancer) |
| 1-247611448 | cg05396897 | (cancer vs cancer) (cancer+normal vs cancer+normal) | 19-50836391 | cg13861524 | (cancer vs cancer) (cancer+normal vs cancer+normal) |
| 1-247611502 | cg09226051 | (cancer vs cancer) (cancer+normal vs cancer+normal) | 19-50836561 | cg01017355 | (cancer vs cancer) (cancer+normal vs cancer+normal) |
| 1-27709850 | cg16400794 | (cancer vs cancer) (cancer+normal vs cancer+normal) | 19-50836910 | cg03251287 | (cancer vs cancer) (cancer+normal vs cancer+normal) |
| 1-42384365 | cg25607920 | (cancer vs cancer) (cancer+normal vs cancer+normal) | 19-50860847 | cg26952796 | (cancer vs cancer) (cancer+normal vs cancer+normal) |
| 1-42384390 | cg26038582 | (cancer vs cancer) (cancer+normal vs cancer+normal) | 19-50861047 | cg12153593 | (cancer vs cancer) (cancer+normal vs cancer+normal) |
| 1-43814306 | cg07865091 | (cancer vs cancer) (cancer+normal vs cancer+normal) | 19-9473565 | cg05250458 | (cancer vs cancer) |
| 1-43814358 | cg18856478 | (cancer vs cancer) (cancer+normal vs cancer+normal) | 2-110969641 | cg02169857 | (cancer vs cancer) (cancer+normal vs cancer+normal) |
| 1-43814983 | cg08706567 | (cancer vs cancer) (cancer+normal vs cancer+normal) | 2-110969853 | cg08570243 | (cancer vs cancer) (cancer+normal vs cancer+normal) |
| 1-43815035 | cg13393721 | (cancer vs cancer) (cancer+normal vs cancer+normal) | 2-110970097 | cg03250220 | (cancer vs cancer) (cancer+normal vs cancer+normal) |
| 10-114713108 | cg11748187 | (cancer vs cancer) (cancer+normal vs cancer+normal) | 2-110970204 | cg09068086 | (cancer vs cancer) (cancer+normal vs cancer+normal) |
| 10-114713187 | cg00159523 | (cancer vs cancer) (cancer+normal vs cancer+normal) | 2-110970249 | cg10366439 | (cancer vs cancer) (cancer+normal vs cancer+normal) |
| 10-17391003 | cg05576619 | (cancer vs cancer) (cancer+normal vs cancer+normal) | 2-157177665 | cg25371385 | (cancer vs cancer) |
| 10-63422630 | cg11416811 | (cancer vs cancer) (cancer+normal vs cancer+normal) | 2-157178165 | cg11856078 | (cancer vs cancer) |
| 10- | cg07643225 | (cancer vs cancer) | 2- | cg0006317 | (cancer vs cancer) |

| | | | | | |
|---|---|---|---|---|---|
| 63422633 | | (cancer+normal vs cancer+normal) | 228736253 | 4 | |
| 10-63809073 | cg14789659 | (cancer vs cancer) (cancer+normal vs cancer+normal) | 2-228736258 | cg20647610 | (cancer vs cancer) |
| 10-63809170 | cg16401465 | (cancer vs cancer) (cancer+normal vs cancer+normal) | 2-228736357 | cg23807890 | (cancer vs cancer) |
| 10-64565750 | cg20684110 | (cancer vs cancer) (cancer+normal vs cancer+normal) | 2-228736459 | cg01216370 | (cancer vs cancer) |
| 10-64565772 | cg12474798 | (cancer vs cancer) (cancer+normal vs cancer+normal) | 21-38935977 | cg00359604 | (cancer vs cancer) (cancer+normal vs cancer+normal) |
| 10-74035570 | cg04858631 | (cancer vs cancer) (cancer+normal vs cancer+normal) | 21-38935991 | cg23484755 | (cancer vs cancer) (cancer+normal vs cancer+normal) |
| 10-88718317 | cg03677069 | (cancer vs cancer) (cancer+normal vs cancer+normal) | 3-10206396 | cg16188589 | (cancer vs cancer) (cancer+normal vs cancer+normal) |
| 11-120208749 | cg26681847 | (cancer vs cancer) (cancer+normal vs cancer+normal) | 3-10206537 | cg18007850 | (cancer vs cancer) (cancer+normal vs cancer+normal) |
| 11-57090226 | cg11180921 | (cancer vs cancer) (cancer+normal vs cancer+normal) | 3-10206628 | cg04437751 | (cancer vs cancer) (cancer+normal vs cancer+normal) |
| 11-57532299 | cg16127573 | (cancer vs cancer) (cancer+normal vs cancer+normal) | 3-10206633 | cg05002590 | (cancer vs cancer) (cancer+normal vs cancer+normal) |
| 11-72533295 | cg13771313 | (cancer vs cancer) (cancer+normal vs cancer+normal) | 3-10206731 | cg11029301 | (cancer vs cancer) (cancer+normal vs cancer+normal) |
| 12-117131523 | cg13073224 | (cancer vs cancer) (cancer+normal vs cancer+normal) | 3-125709198 | cg02772935 | (cancer vs cancer) (cancer+normal vs cancer+normal) |
| 12-125002332 | cg01054110 | (cancer vs cancer) (cancer+normal vs cancer+normal) | 3-169482358 | cg27020690 | (cancer vs cancer) (cancer+normal vs cancer+normal) |
| 12-58129855 | cg16402452 | (cancer vs cancer) (cancer+normal vs cancer+normal) | 3-169482714 | cg20839041 | (cancer vs cancer) (cancer+normal vs cancer+normal) |
| 14-51431710 | cg05892030 | (cancer vs cancer) (cancer+normal vs cancer+normal) | 3-169482895 | cg15494117 | (cancer vs cancer) (cancer+normal vs cancer+normal) |
| 14-69256677 | cg01424562 | (cancer vs cancer) (cancer+normal vs cancer+normal) | 3-169482899 | cg15599946 | (cancer vs cancer) (cancer+normal vs cancer+normal) |
| 14-69256690 | cg06617636 | (cancer vs cancer) (cancer+normal vs cancer+normal) | 3-169482968 | cg01389761 | (cancer vs cancer) (cancer+normal vs cancer+normal) |
| 14-69256888 | cg08169020 | (cancer vs cancer) (cancer+normal vs cancer+normal) | 3-169483025 | cg23036508 | (cancer vs cancer) (cancer+normal vs cancer+normal) |
| 14-69256977 | cg10099732 | (cancer vs cancer) (cancer+normal vs cancer+normal) | 3-44727069 | cg13348059 | (cancer vs cancer) |
| 15-39872032 | cg10078511 | (cancer vs cancer) (cancer+normal vs cancer+normal) | 3-44727106 | cg08999915 | (cancer vs cancer) |
| 15-39872071 | cg08433504 | (cancer vs cancer) (cancer+normal vs | 3-44727195 | cg24158187 | (cancer vs cancer) |

| | | | | | |
|---|---|---|---|---|---|
| | | cancer+normal) | | | |
| 15-75045126 | cg01359532 | (cancer vs cancer) (cancer+normal vs cancer+normal) | 3-44770937 | cg10108389 | (cancer vs cancer) (cancer+normal vs cancer+normal) |
| 15-83879539 | cg21238234 | (cancer vs cancer) (cancer+normal vs cancer+normal) | 3-44770973 | cg02073558 | (cancer vs cancer) (cancer+normal vs cancer+normal) |
| 16-31159704 | cg14301190 | (cancer vs cancer) (cancer+normal vs cancer+normal) | 3-44771102 | cg24837680 | (cancer vs cancer) (cancer+normal vs cancer+normal) |
| 16-67687754 | cg09316954 | (cancer vs cancer) (cancer+normal vs cancer+normal) | 3-44771105 | cg02933119 | (cancer vs cancer) (cancer+normal vs cancer+normal) |
| 16-87416230 | cg09642825 | (cancer vs cancer) (cancer+normal vs cancer+normal) | 4-4871525 | cg02680341 | (cancer vs cancer) (normal vs normal) (cancer+normal vs cancer+normal) |
| 16-87525461 | cg06330621 | (cancer vs cancer) (cancer+normal vs cancer+normal) | 5-138728685 | cg01054755 | (cancer vs cancer) (cancer+normal vs cancer+normal) |
| 16-87525539 | cg08196561 | (cancer vs cancer) (cancer+normal vs cancer+normal) | 5-138728704 | cg24761867 | (cancer vs cancer) (cancer+normal vs cancer+normal) |
| 17-1464972 | cg24205387 | (cancer vs cancer) (cancer+normal vs cancer+normal) | 5-138729068 | cg05137680 | (cancer vs cancer) (cancer+normal vs cancer+normal) |
| 17-15366916 | cg22980156 | (cancer vs cancer) (cancer+normal vs cancer+normal) | 5-138729245 | cg05449136 | (cancer vs cancer) |
| 17-38473119 | cg19458020 | (cancer vs cancer) (cancer+normal vs cancer+normal) | 5-87981894 | cg15981305 | (cancer vs cancer) (cancer+normal vs cancer+normal) |
| 17-40075879 | cg19443920 | (cancer vs cancer) (cancer+normal vs cancer+normal) | 6-100902470 | cg08041481 | (cancer vs cancer) |
| 17-46681362 | cg17179862 | (cancer vs cancer) (cancer+normal vs cancer+normal) | 6-168533631 | cg17172308 | (cancer vs cancer) |
| 17-46681401 | cg03803541 | (cancer vs cancer) (cancer+normal vs cancer+normal) | 6-26043990 | cg22478210 | (cancer vs cancer) (cancer+normal vs cancer+normal) |
| 17-75283832 | cg17697835 | (cancer vs cancer) (cancer+normal vs cancer+normal) | 6-26044220 | cg07701237 | (cancer vs cancer) |
| 17-76713379 | cg12871285 | (cancer vs cancer) (cancer+normal vs cancer+normal) | 6-26044274 | cg26426142 | (cancer vs cancer) |
| 19-10445516 | cg17126555 | (cancer vs cancer) (cancer+normal vs cancer+normal) | 6-26044338 | cg02221866 | (cancer vs cancer) |
| 19-10736448 | cg21631439 | (cancer vs cancer) (cancer+normal vs cancer+normal) | 6-26044460 | cg05646373 | (cancer vs cancer) |
| 19-15568360 | cg08846870 | (cancer vs cancer) (cancer+normal vs cancer+normal) | 6-26045532 | cg17432453 | (cancer vs cancer) (cancer+normal vs cancer+normal) |
| 19-2462961 | cg23450038 | (cancer vs cancer) (cancer+normal vs cancer+normal) | 6-26045534 | cg07636178 | (cancer vs cancer) (cancer+normal vs cancer+normal) |
| 19-39086923 | cg05258935 | (cancer vs cancer) (cancer+normal vs | 6-26045663 | cg25438963 | (cancer vs cancer) (cancer+normal vs |

| | | | | | |
|---|---|---|---|---|---|
| | | cancer+normal) | | | cancer+normal) |
| 19-41225005 | cg21869046 | (cancer vs cancer) (cancer+normal vs cancer+normal) | 6-28367544 | cg27577527 | (cancer vs cancer) |
| 19-45281140 | cg01057656 | (cancer vs cancer) (cancer+normal vs cancer+normal) | 6-85132263 | cg09222422 | (cancer vs cancer) |
| 19-6459300 | cg17100943 | (cancer vs cancer) (cancer+normal vs cancer+normal) | 8-11059029 | cg24631970 | (cancer vs cancer) (cancer+normal vs cancer+normal) |
| 19-7460671 | cg11734329 | (cancer vs cancer) (cancer+normal vs cancer+normal) | 8-11059176 | cg26675876 | (cancer vs cancer) |
| 19-8590567 | cg22568423 | (cancer vs cancer) (cancer+normal vs cancer+normal) | 8-65488069 | cg24255371 | (cancer vs cancer) (cancer+normal vs cancer+normal) |
| 19-8591364 | cg22987448 | (cancer vs cancer) (cancer+normal vs cancer+normal) | 8-70947044 | cg04445512 | (cancer vs cancer) |
| 20-19866810 | cg23501964 | (cancer vs cancer) (cancer+normal vs cancer+normal) | 9-104248926 | cg06912252 | (cancer vs cancer) |
| 20-19867136 | cg20918393 | (cancer vs cancer) (cancer+normal vs cancer+normal) | **Status - Hypo** | | |
| 22-46423604 | cg00147172 | (cancer vs cancer) (cancer+normal vs cancer+normal) | 1-17350304 | cg04394641 | (cancer vs cancer) |
| 22-51136325 | cg18982286 | (cancer vs cancer) (cancer+normal vs cancer+normal) | 1-174417967 | cg05768419 | (cancer vs cancer) (cancer+normal vs cancer+normal) |
| 3-101882326 | cg24627325 | (cancer vs cancer) (cancer+normal vs cancer+normal) | 1-178992788 | cg03393754 | (cancer vs cancer) |
| 3-111697575 | cg08979515 | (cancer vs cancer) (cancer+normal vs cancer+normal) | 1-220415655 | cg26570574 | (cancer vs cancer) (cancer+normal vs cancer+normal) |
| 3-141087190 | cg13029400 | (cancer vs cancer) (cancer+normal vs cancer+normal) | 1-233815158 | cg07184643 | (cancer vs cancer) (cancer+normal vs cancer+normal) |
| 3-188012918 | cg02524983 | (cancer vs cancer) (cancer+normal vs cancer+normal) | 1-31423071 | cg04248332 | (cancer vs cancer) |
| 3-57743163 | cg03534375 | (cancer vs cancer) (cancer+normal vs cancer+normal) | 1-31423283 | cg00581541 | (cancer vs cancer) |
| 3-57743377 | cg15172734 | (cancer vs cancer) (cancer+normal vs cancer+normal) | 1-32252266 | cg07947531 | (cancer vs cancer) |
| 4-114900431 | cg05601456 | (cancer vs cancer) (cancer+normal vs cancer+normal) | 1-41326317 | cg04011995 | (cancer vs cancer) |
| 4-159969703 | cg10393744 | (cancer vs cancer) (cancer+normal vs cancer+normal) | 1-46921746 | cg00015930 | (cancer vs cancer) (cancer+normal vs cancer+normal) |
| 4-186318356 | cg26741686 | (cancer vs cancer) (cancer+normal vs cancer+normal) | 1-46921844 | cg20977426 | (cancer vs cancer) (cancer+normal vs cancer+normal) |
| 4-40665353 | cg06704884 | (cancer vs cancer) (cancer+normal vs cancer+normal) | 1-56352596 | cg23844910 | (cancer vs cancer) |

| | | | | | |
|---|---|---|---|---|---|
| 5-10551547 | cg24100671 | (cancer vs cancer) (cancer+normal vs cancer+normal) | 10-124316636 | cg19856749 | (cancer vs cancer) (cancer+normal vs cancer+normal) |
| 5-133904674 | cg27024654 | (cancer vs cancer) (cancer+normal vs cancer+normal) | 10-131034796 | cg18892424 | (cancer vs cancer) |
| 5-178986906 | cg26516362 | (cancer vs cancer) (cancer+normal vs cancer+normal) | 10-131592435 | cg18629132 | (cancer vs cancer) (cancer+normal vs cancer+normal) |
| 5-5271554 | cg25485055 | (cancer vs cancer) (cancer+normal vs cancer+normal) | 10-131592483 | cg02656060 | (cancer vs cancer) |
| 6-143167279 | cg13829419 | (cancer vs cancer) (cancer+normal vs cancer+normal) | 10-16822712 | cg02193861 | (cancer vs cancer) |
| 6-30227001 | cg14296767 | (cancer vs cancer) (cancer+normal vs cancer+normal) | 10-21798257 | cg02360445 | (cancer vs cancer) |
| 6-30853608 | cg11975790 | (cancer vs cancer) (cancer+normal vs cancer+normal) | 10-373957 | cg25301103 | (cancer vs cancer) (cancer+normal vs cancer+normal) |
| 6-30881484 | cg10661769 | (cancer vs cancer) (cancer+normal vs cancer+normal) | 10-374010 | cg11423998 | (cancer vs cancer) (cancer+normal vs cancer+normal) |
| 7-15055459 | cg19272540 | (cancer vs cancer) (cancer+normal vs cancer+normal) | 10-374092 | cg09782637 | (cancer vs cancer) (cancer+normal vs cancer+normal) |
| 7-151157166 | cg07748583 | (cancer vs cancer) (cancer+normal vs cancer+normal) | 10-99566075 | cg22658238 | (cancer vs cancer) |
| 7-75367549 | cg13702357 | (cancer vs cancer) (cancer+normal vs cancer+normal) | 11-122543428 | cg26684673 | (cancer vs cancer) |
| 8-124498970 | cg13092738 | (cancer vs cancer) (cancer+normal vs cancer+normal) | 11-1380305 | cg03370490 | (cancer vs cancer) |
| 8-23780241 | cg10855978 | (cancer vs cancer) (cancer+normal vs cancer+normal) | 11-1380355 | cg23628760 | (cancer vs cancer) |
| 8-48437852 | cg08169341 | (cancer vs cancer) (cancer+normal vs cancer+normal) | 11-2826988 | cg13253153 | (cancer vs cancer) |
| 8-61563866 | cg21205855 | (cancer vs cancer) (cancer+normal vs cancer+normal) | 11-8361190 | cg01677628 | (normal vs normal) (cancer vs cancer) (cancer+normal vs cancer+normal) |
| 8-67454546 | cg08620329 | (cancer vs cancer) (cancer+normal vs cancer+normal) | 12-109046825 | cg06251181 | (cancer vs cancer) (cancer+normal vs cancer+normal) |
| 8-67454595 | cg12109823 | (cancer vs cancer) (cancer+normal vs cancer+normal) | 12-12605470 | cg24090908 | (cancer vs cancer) (cancer+normal vs cancer+normal) |
| 9-130700954 | cg21181453 | (cancer vs cancer) (cancer+normal vs cancer+normal) | 12-132639764 | cg09451320 | (cancer vs cancer) (cancer+normal vs cancer+normal) |
| 9-14321079 | cg14519997 | (cancer vs cancer) (cancer+normal vs cancer+normal) | 12-6590163 | cg13847998 | (cancer vs cancer) |
| **Status - Hypo** | | | 12-69054360 | cg07344315 | (cancer vs cancer) |
| 1- | cg19704755 | (cancer vs cancer) | 13- | cg0321589 | (cancer vs cancer) |

| | | | | | |
|---|---|---|---|---|---|
| 10965094 | | (cancer+normal vs cancer+normal) | 100497610 | 5 | |
| 1-151129298 | cg23612220 | (cancer vs cancer) (cancer+normal vs cancer+normal) | 13-100497665 | cg26862247 | (cancer vs cancer) |
| 1-151129844 | cg01903374 | (cancer vs cancer) (cancer+normal vs cancer+normal) | 13-100497789 | cg03562911 | (cancer vs cancer) (cancer+normal vs cancer+normal) |
| 1-156551787 | cg09319828 | (cancer vs cancer) (cancer+normal vs cancer+normal) | 13-101091946 | cg19937572 | (cancer vs cancer) (cancer+normal vs cancer+normal) |
| 1-156551873 | cg01010328 | (cancer vs cancer) (cancer+normal vs cancer+normal) | 13-101161616 | cg06398335 | (cancer vs cancer) (cancer+normal vs cancer+normal) |
| 1-159770136 | cg10016364 | (cancer vs cancer) (cancer+normal vs cancer+normal) | 13-111980272 | cg08019253 | (cancer vs cancer) |
| 1-161184921 | cg26394055 | (cancer vs cancer) (cancer+normal vs cancer+normal) | 13-114872102 | cg16986571 | (cancer vs cancer) (cancer+normal vs cancer+normal) |
| 1-169187004 | cg07948875 | (cancer vs cancer) (cancer+normal vs cancer+normal) | 13-44596411 | cg07210123 | (cancer vs cancer) |
| 1-17508341 | cg23013029 | (cancer vs cancer) (cancer+normal vs cancer+normal) | 13-46297726 | cg21119127 | (cancer vs cancer) (cancer+normal vs cancer+normal) |
| 1-192515127 | cg24602411 | (cancer vs cancer) (cancer+normal vs cancer+normal) | 13-96202378 | cg13662530 | (cancer vs cancer) (cancer+normal vs cancer+normal) |
| 1-202129566 | cg18384097 | (cancer vs cancer) (cancer+normal vs cancer+normal) | 14-104618028 | cg08277679 | (cancer vs cancer) |
| 1-206946166 | cg17744604 | (cancer vs cancer) (cancer+normal vs cancer+normal) | 14-39648415 | cg06918918 | (cancer vs cancer) (cancer+normal vs cancer+normal) |
| 1-74665362 | cg18854765 | (cancer vs cancer) (cancer+normal vs cancer+normal) | 16-1946951 | cg06654431 | (cancer vs cancer) |
| 10-105978651 | cg23130731 | (cancer vs cancer) (cancer+normal vs cancer+normal) | 16-1947055 | cg00546932 | (cancer vs cancer) |
| 10-34925718 | cg00287536 | (cancer vs cancer) (cancer+normal vs cancer+normal) | 16-70563092 | cg08186341 | (cancer vs cancer) (cancer+normal vs cancer+normal) |
| 10-50716478 | cg00025044 | (cancer vs cancer) (cancer+normal vs cancer+normal) | 16-88550450 | cg02071579 | (cancer vs cancer) |
| 11-1680823 | cg04552555 | (cancer vs cancer) (cancer+normal vs cancer+normal) | 17-78878278 | cg09173565 | (cancer vs cancer) (cancer+normal vs cancer+normal) |
| 11-1911547 | cg18723409 | (cancer vs cancer) (cancer+normal vs cancer+normal) | 17-78878530 | cg22673070 | (cancer vs cancer) (cancer+normal vs cancer+normal) |
| 11-33913716 | cg11822932 | (cancer vs cancer) (cancer+normal vs cancer+normal) | 18-20735005 | cg18793086 | (cancer vs cancer) |
| 11-47399980 | cg03106245 | (cancer vs cancer) (cancer+normal vs cancer+normal) | 18-74325812 | cg23553867 | (cancer vs cancer) (cancer+normal vs cancer+normal) |
| 11-47416487 | cg10511890 | (cancer vs cancer) (cancer+normal vs | 18-74325904 | cg07867133 | (cancer vs cancer) (cancer+normal vs |

374

| | | | | | |
|---|---|---|---|---|---|
| | | cancer+normal) | | | cancer+normal) |
| 11-47416886 | cg09739398 | (cancer vs cancer) (cancer+normal vs cancer+normal) | 19-1307484 | cg07962358 | (cancer vs cancer) |
| 11-65408496 | cg24301930 | (cancer vs cancer) (cancer+normal vs cancer+normal) | 19-35649055 | cg12055183 | (cancer vs cancer) |
| 11-65661516 | cg07420137 | (cancer vs cancer) (cancer+normal vs cancer+normal) | 19-3595702 | cg14938419 | (cancer vs cancer) (cancer+normal vs cancer+normal) |
| 11-66883117 | cg13980609 | (cancer vs cancer) (cancer+normal vs cancer+normal) | 19-3595832 | cg04948286 | (cancer vs cancer) |
| 11-67205096 | cg10542975 | (cancer vs cancer) (cancer+normal vs cancer+normal) | 2-130512167 | cg11929750 | (cancer vs cancer) |
| 11-70211408 | cg14088196 | (cancer vs cancer) (cancer+normal vs cancer+normal) | 2-138940416 | cg02204521 | (cancer vs cancer) (cancer+normal vs cancer+normal) |
| 11-70211531 | cg25574765 | (cancer vs cancer) (cancer+normal vs cancer+normal) | 2-180846309 | cg14175777 | (cancer vs cancer) |
| 11-70257149 | cg16061668 | (cancer vs cancer) (cancer+normal vs cancer+normal) | 2-242766284 | cg16514818 | (cancer vs cancer) (cancer+normal vs cancer+normal) |
| 11-70268728 | cg11194613 | (cancer vs cancer) (cancer+normal vs cancer+normal) | 2-242766412 | cg19384073 | (cancer vs cancer) (cancer+normal vs cancer+normal) |
| 12-2030178 | cg19369955 | (cancer vs cancer) (cancer+normal vs cancer+normal) | 2-43598959 | cg16472853 | (cancer vs cancer) (cancer+normal vs cancer+normal) |
| 12-2030223 | cg08621624 | (cancer vs cancer) (cancer+normal vs cancer+normal) | 20-42825248 | cg06466728 | (cancer vs cancer) |
| 12-44226393 | cg02522196 | (cancer vs cancer) (cancer+normal vs cancer+normal) | 20-62421310 | cg08246859 | (cancer vs cancer) |
| 12-51717674 | cg10590292 | (cancer vs cancer) (cancer+normal vs cancer+normal) | 3-178984973 | cg00862290 | (cancer vs cancer) (cancer+normal vs cancer+normal) |
| 12-51717865 | cg12353452 | (cancer vs cancer) (cancer+normal vs cancer+normal) | 3-28889136 | cg08028901 | (cancer vs cancer) (cancer+normal vs cancer+normal) |
| 12-51718088 | cg14556909 | (cancer vs cancer) (cancer+normal vs cancer+normal) | 3-41239648 | cg09489743 | (cancer vs cancer) |
| 12-51718112 | cg08075204 | (cancer vs cancer) (cancer+normal vs cancer+normal) | 3-57432946 | cg15448394 | (cancer vs cancer) |
| 13-113238006 | cg18571045 | (cancer vs cancer) (cancer+normal vs cancer+normal) | 4-1022462 | cg18123043 | (cancer vs cancer) |
| 14-103440432 | cg14191466 | (cancer vs cancer) (cancer+normal vs cancer+normal) | 4-1578077 | cg00175895 | (cancer vs cancer) (cancer+normal vs cancer+normal) |
| 15-101715747 | cg12042714 | (cancer vs cancer) (cancer+normal vs cancer+normal) | 4-185913488 | cg07025505 | (cancer vs cancer) |
| 16-2737340 | cg09866569 | (cancer vs cancer) (cancer+normal vs cancer+normal) | 4-190767168 | cg04392133 | (cancer vs cancer) (cancer+normal vs cancer+normal) |

| | | | | | |
|---|---|---|---|---|---|
| 16-29676071 | cg01681367 | (cancer vs cancer) (cancer+normal vs cancer+normal) | 5-14915807 | cg17749710 | (cancer vs cancer) |
| 16-88866627 | cg10491452 | (cancer vs cancer) (cancer+normal vs cancer+normal) | 5-179643456 | cg25777409 | (cancer vs cancer) |
| 17-263096 | cg25045219 | (cancer vs cancer) (cancer+normal vs cancer+normal) | 5-676784 | cg02371401 | (cancer vs cancer) |
| 17-46190702 | cg01722297 | (cancer vs cancer) (cancer+normal vs cancer+normal) | 5-677840 | cg09884756 | (cancer vs cancer) |
| 17-56408197 | cg03649649 | (cancer vs cancer) (cancer+normal vs cancer+normal) | 5-678009 | cg05458764 | (cancer vs cancer) |
| 17-56409011 | cg26112797 | (cancer vs cancer) (cancer+normal vs cancer+normal) | 5-70746259 | cg07752120 | (cancer vs cancer) |
| 17-56409028 | cg10530767 | (cancer vs cancer) (cancer+normal vs cancer+normal) | 5-70746330 | cg26624732 | (cancer vs cancer) |
| 17-56565286 | cg27366766 | (cancer vs cancer) (cancer+normal vs cancer+normal) | 5-77146197 | cg02455471 | (cancer vs cancer) |
| 17-74565009 | cg01883155 | (normal vs normal) (cancer vs cancer) (cancer+normal vs cancer+normal) | 5-77146318 | cg17034331 | (cancer vs cancer) |
| 17-77916768 | cg20667480 | (cancer vs cancer) (cancer+normal vs cancer+normal) | 6-107114594 | cg18067520 | (cancer vs cancer) |
| 17-78748471 | cg00523683 | (cancer vs cancer) (cancer+normal vs cancer+normal) | 6-170411557 | cg17267654 | (cancer vs cancer) (cancer+normal vs cancer+normal) |
| 17-78748494 | cg02185248 | (cancer vs cancer) (cancer+normal vs cancer+normal) | 6-170531180 | cg05024916 | (cancer vs cancer) (cancer+normal vs cancer+normal) |
| 17-78755379 | cg06153925 | (cancer vs cancer) (cancer+normal vs cancer+normal) | 6-170531660 | cg22099441 | (cancer vs cancer) (cancer+normal vs cancer+normal) |
| 17-800745 | cg14235846 | (cancer vs cancer) (cancer+normal vs cancer+normal) | 6-170531710 | cg27068650 | (cancer vs cancer) |
| 17-80358829 | cg11252953 | (cancer vs cancer) (cancer+normal vs cancer+normal) | 6-34464251 | cg26715883 | (cancer vs cancer) (cancer+normal vs cancer+normal) |
| 17-80358850 | cg25490145 | (cancer vs cancer) (cancer+normal vs cancer+normal) | 7-139690853 | cg03764092 | (cancer vs cancer) (cancer+normal vs cancer+normal) |
| 17-80358876 | cg21465176 | (cancer vs cancer) (cancer+normal vs cancer+normal) | 7-139690881 | cg23625084 | (cancer vs cancer) (cancer+normal vs cancer+normal) |
| 19-13215387 | cg15123730 | (cancer vs cancer) (cancer+normal vs cancer+normal) | 7-1660603 | cg26146855 | (cancer vs cancer) |
| 19-46294482 | cg03758011 | (cancer vs cancer) (cancer+normal vs cancer+normal) | 7-28834178 | cg16624304 | (cancer vs cancer) |
| 2-175499113 | cg13821008 | (cancer vs cancer) (cancer+normal vs cancer+normal) | 7-34941831 | cg06795768 | (cancer vs cancer) |

| | | | | | |
|---|---|---|---|---|---|
| 2-68592737 | cg13468685 | (cancer vs cancer) (cancer+normal vs cancer+normal) | 7-39298343 | cg27443265 | (cancer vs cancer) |
| 2-74264707 | cg01987702 | (cancer vs cancer) (cancer+normal vs cancer+normal) | 7-69246130 | cg09449543 | (cancer vs cancer) |
| 2-88327530 | cg04195125 | (cancer vs cancer) (cancer+normal vs cancer+normal) | 7-77492522 | cg22614518 | (cancer vs cancer) (cancer+normal vs cancer+normal) |
| 20-35944932 | cg00916899 | (cancer vs cancer) (cancer+normal vs cancer+normal) | 7-81240445 | cg27310219 | (cancer vs cancer) |
| 20-3693158 | cg14921416 | (cancer vs cancer) (cancer+normal vs cancer+normal) | 8-2017571 | cg04146801 | (cancer vs cancer) |
| 21-44819445 | cg17122157 | (cancer vs cancer) (cancer+normal vs cancer+normal) | 8-72872845 | cg01706334 | (cancer vs cancer) |
| 3-196351986 | cg00828556 | (cancer vs cancer) (cancer+normal vs cancer+normal) | 8-8898513 | cg05418508 | (cancer vs cancer) |
| 3-56789178 | cg08052292 | (cancer vs cancer) (cancer+normal vs cancer+normal) | **(cancer+normal vs cancer+normal)** | | |
| 4-1221838 | cg26923863 | (cancer vs cancer) (cancer+normal vs cancer+normal) | **Status - Hyper** | | |
| 5-1503763 | cg19503640 | (cancer vs cancer) (cancer+normal vs cancer+normal) | 1-162630153 | cg19264028 | (normal vs normal) (cancer+normal vs cancer+normal) |
| 5-55215955 | cg05650238 | (cancer vs cancer) (cancer+normal vs cancer+normal) | 1-172714273 | cg06191357 | (cancer+normal vs cancer+normal) |
| 5-77783895 | cg18281939 | (cancer vs cancer) (cancer+normal vs cancer+normal) | 1-232940990 | cg02191044 | (cancer vs cancer) (cancer+normal vs cancer+normal) |
| 6-135644405 | cg14564351 | (cancer vs cancer) (cancer+normal vs cancer+normal) | 1-38260410 | cg12900583 | (cancer+normal vs cancer+normal) |
| 6-137105249 | cg02673417 | (cancer vs cancer) (cancer+normal vs cancer+normal) | 1-8014224 | cg12045833 | (cancer+normal vs cancer+normal) |
| 6-151380818 | cg07805500 | (cancer vs cancer) (cancer+normal vs cancer+normal) | 10-113759413 | cg00974685 | (cancer+normal vs cancer+normal) |
| 6-157932130 | cg09783253 | (cancer vs cancer) (cancer+normal vs cancer+normal) | 10-122356491 | cg27245056 | (cancer+normal vs cancer+normal) |
| 6-158406044 | cg10799846 | (cancer vs cancer) (cancer+normal vs cancer+normal) | 10-124639132 | cg12474023 | (cancer vs cancer) (cancer+normal vs cancer+normal) |
| 6-166877002 | cg07253384 | (cancer vs cancer) (cancer+normal vs cancer+normal) | 10-124639167 | cg09019052 | (cancer+normal vs cancer+normal) |
| 6-166877038 | cg02287007 | (cancer vs cancer) (cancer+normal vs cancer+normal) | 10-16566625 | cg19197922 | (normal vs normal) (normal vs normal) (cancer+normal vs cancer+normal) |
| 6-27842098 | cg01247537 | (cancer vs cancer) (cancer+normal vs cancer+normal) | 10-50603359 | cg16966201 | (cancer+normal vs cancer+normal) |

| | | | | | |
|---|---|---|---|---|---|
| 6-31583942 | cg18113826 | (cancer vs cancer) (cancer+normal vs cancer+normal) | 10-8103673 | cg00463367 | (normal vs normal) (cancer+normal vs cancer+normal) |
| 6-31695415 | cg25953692 | (cancer vs cancer) (cancer+normal vs cancer+normal) | 11-45102239 | cg23299618 | (cancer+normal vs cancer+normal) |
| 6-31695552 | cg03900314 | (cancer vs cancer) (cancer+normal vs cancer+normal) | 12-93966317 | cg11225410 | (cancer vs cancer) (cancer+normal vs cancer+normal) |
| 6-31695599 | cg13597051 | (cancer vs cancer) (cancer+normal vs cancer+normal) | 12-93966485 | cg22125370 | (cancer vs cancer) (cancer+normal vs cancer+normal) |
| 6-32155082 | cg09001226 | (cancer vs cancer) (cancer+normal vs cancer+normal) | 16-23724485 | cg16364709 | (cancer vs cancer) (cancer+normal vs cancer+normal) |
| 7-2773782 | cg20336172 | (cancer vs cancer) (cancer+normal vs cancer+normal) | 16-67276940 | cg27054887 | (cancer+normal vs cancer+normal) (cancer+normal vs cancer+normal) |
| 7-2773812 | cg26247093 | (cancer vs cancer) (cancer+normal vs cancer+normal) | 17-42030287 | cg01703581 | (cancer vs cancer) (cancer+normal vs cancer+normal) |
| 7-3134557 | cg09450197 | (cancer vs cancer) (cancer+normal vs cancer+normal) | 17-42091713 | cg12373208 | (cancer vs cancer) (cancer+normal vs cancer+normal) |
| 7-32802765 | cg07573366 | (normal vs normal) (cancer vs cancer) (cancer+normal vs cancer+normal) | 17-42091909 | cg04879755 | (cancer vs cancer) (cancer+normal vs cancer+normal) |
| 7-5258357 | cg20071624 | (cancer vs cancer) (cancer+normal vs cancer+normal) | 17-42092187 | cg18801599 | (cancer vs cancer) (cancer+normal vs cancer+normal) |
| 7-6476003 | cg06488150 | (cancer vs cancer) (cancer+normal vs cancer+normal) | 17-42092247 | cg24638647 | (cancer vs cancer) (cancer+normal vs cancer+normal) |
| 7-6476110 | cg08313420 | (cancer vs cancer) (cancer+normal vs cancer+normal) | 17-42092315 | cg27116819 | (cancer vs cancer) (cancer+normal vs cancer+normal) |
| 8-134559320 | cg02609337 | (cancer vs cancer) (cancer+normal vs cancer+normal) | 17-42092370 | cg08372947 | (cancer vs cancer) (cancer+normal vs cancer+normal) |
| 8-140945810 | cg26769700 | (cancer vs cancer) (cancer+normal vs cancer+normal) | 17-42092403 | cg06511389 | (cancer vs cancer) (cancer+normal vs cancer+normal) |
| 8-141609380 | cg18440839 | (cancer vs cancer) (cancer+normal vs cancer+normal) | 17-42092431 | cg12259256 | (cancer vs cancer) (cancer+normal vs cancer+normal) |
| 8-145052950 | cg00015530 | (cancer vs cancer) (cancer+normal vs cancer+normal) | 17-42092450 | cg13847066 | (cancer vs cancer) (cancer+normal vs cancer+normal) |
| 8-22398190 | cg19021985 | (cancer vs cancer) (cancer+normal vs cancer+normal) | 17-42092472 | cg01635193 | (cancer vs cancer) (cancer+normal vs cancer+normal) |
| 9-134454249 | cg13555415 | (cancer vs cancer) (cancer+normal vs cancer+normal) | 17-42092488 | cg25868286 | (cancer vs cancer) (cancer+normal vs cancer+normal) |
| 9-96080326 | cg20324199 | (cancer vs cancer) (cancer+normal vs cancer+normal) | 17-42092588 | cg01986131 | (cancer vs cancer) (cancer+normal vs cancer+normal) |
| **(cancer+normal vs cancer+normal)** | | | 17-58498871 | cg10179315 | (cancer vs cancer) (cancer+normal vs |

| Status - Hyper | | |
|---|---|---|
| 1-149908313 | cg13476072 | (cancer vs cancer) (cancer+normal vs cancer+normal) |
| 1-149908524 | cg00777271 | (cancer vs cancer) (cancer+normal vs cancer+normal) |
| 1-149908781 | cg24620905 | (cancer vs cancer) (cancer+normal vs cancer+normal) |
| 1-164581169 | cg20812370 | (cancer vs cancer) (cancer+normal vs cancer+normal) |
| 1-205270559 | cg03466326 | (cancer vs cancer) (cancer+normal vs cancer+normal) |
| 1-211643515 | cg05352688 | (cancer vs cancer) (cancer+normal vs cancer+normal) |
| 1-221613658 | cg24686918 | (cancer vs cancer) (cancer+normal vs cancer+normal) |
| 1-247611448 | cg05396897 | (cancer vs cancer) (cancer+normal vs cancer+normal) |
| 1-247611502 | cg09226051 | (cancer vs cancer) (cancer+normal vs cancer+normal) |
| 1-27709850 | cg16400794 | (cancer vs cancer) (cancer+normal vs cancer+normal) |
| 1-42384365 | cg25607920 | (cancer vs cancer) (cancer+normal vs cancer+normal) |
| 1-42384390 | cg26038582 | (cancer vs cancer) (cancer+normal vs cancer+normal) |
| 1-43814306 | cg07865091 | (cancer vs cancer) (cancer+normal vs cancer+normal) |
| 1-43814358 | cg18856478 | (cancer vs cancer) (cancer+normal vs cancer+normal) |
| 1-43814983 | cg08706567 | (cancer vs cancer) (cancer+normal vs cancer+normal) |
| 1-43815035 | cg13393721 | (cancer vs cancer) (cancer+normal vs cancer+normal) |
| 10-114713108 | cg11748187 | (cancer vs cancer) (cancer+normal vs cancer+normal) |
| 10-114713187 | cg00159523 | (cancer vs cancer) (cancer+normal vs cancer+normal) |
| 10-17391003 | cg05576619 | (cancer vs cancer) (cancer+normal vs cancer+normal) |

| | | cancer+normal) |
|---|---|---|
| 17-9479910 | cg25964007 | (cancer vs cancer) (cancer+normal vs cancer+normal) |
| 17-9479912 | cg12610887 | (cancer vs cancer) (cancer+normal vs cancer+normal) |
| 18-5238183 | cg05390530 | (cancer vs cancer) (cancer+normal vs cancer+normal) |
| 18-5238200 | cg14291066 | (cancer vs cancer) (cancer+normal vs cancer+normal) |
| 18-5238219 | cg15258847 | (cancer vs cancer) (cancer+normal vs cancer+normal) |
| 18-5238310 | cg20757519 | (cancer vs cancer) (cancer+normal vs cancer+normal) |
| 18-5238589 | cg10885961 | (cancer vs cancer) (cancer+normal vs cancer+normal) |
| 18-5238608 | cg21311023 | (cancer vs cancer) (cancer+normal vs cancer+normal) |
| 18-5238708 | cg20134241 | (cancer vs cancer) (cancer+normal vs cancer+normal) |
| 19-50836391 | cg13861524 | (cancer vs cancer) (cancer+normal vs cancer+normal) |
| 19-50836561 | cg01017355 | (cancer vs cancer) (cancer+normal vs cancer+normal) |
| 19-50836910 | cg03251287 | (cancer vs cancer) (cancer+normal vs cancer+normal) |
| 19-50860847 | cg26952796 | (cancer vs cancer) (cancer+normal vs cancer+normal) |
| 19-50861047 | cg12153593 | (cancer vs cancer) (cancer+normal vs cancer+normal) |
| 19-53541287 | cg05294813 | (cancer+normal vs cancer+normal) |
| 2-106567892 | cg00160872 | (cancer+normal vs cancer+normal) |
| 2-110969641 | cg02169857 | (cancer vs cancer) (cancer+normal vs cancer+normal) |
| 2-110969853 | cg08570243 | (cancer vs cancer) (cancer+normal vs cancer+normal) |
| 2-110970097 | cg03250220 | (cancer vs cancer) (cancer+normal vs cancer+normal) |
| 2-110970204 | cg09068086 | (cancer vs cancer) (cancer+normal vs cancer+normal) |

| | | | | | |
|---|---|---|---|---|---|
| 10-63422630 | cg11416811 | (cancer vs cancer) (cancer+normal vs cancer+normal) | 2-110970249 | cg10366439 | (cancer vs cancer) (cancer+normal vs cancer+normal) |
| 10-63422633 | cg07643225 | (cancer vs cancer) (cancer+normal vs cancer+normal) | 2-118845443 | cg22650315 | (cancer+normal vs cancer+normal) |
| 10-63809073 | cg14789659 | (cancer vs cancer) (cancer+normal vs cancer+normal) | 2-168275428 | cg22940655 | (cancer+normal vs cancer+normal) |
| 10-63809170 | cg16401465 | (cancer vs cancer) (cancer+normal vs cancer+normal) | 2-197787991 | cg02539829 | (cancer+normal vs cancer+normal) |
| 10-64565750 | cg20684110 | (cancer vs cancer) (cancer+normal vs cancer+normal) | 2-81420861 | cg01887388 | (cancer+normal vs cancer+normal) |
| 10-64565772 | cg12474798 | (cancer vs cancer) (cancer+normal vs cancer+normal) | 21-38935977 | cg00359604 | (cancer vs cancer) (cancer+normal vs cancer+normal) |
| 10-74035570 | cg04858631 | (cancer vs cancer) (cancer+normal vs cancer+normal) | 21-38935991 | cg23484755 | (cancer vs cancer) (cancer+normal vs cancer+normal) |
| 10-88718317 | cg03677069 | (cancer vs cancer) (cancer+normal vs cancer+normal) | 3-10206396 | cg16188589 | (cancer vs cancer) (cancer+normal vs cancer+normal) |
| 11-120208749 | cg26681847 | (cancer vs cancer) (cancer+normal vs cancer+normal) | 3-10206537 | cg18007850 | (cancer vs cancer) (cancer+normal vs cancer+normal) |
| 11-57090226 | cg11180921 | (cancer vs cancer) (cancer+normal vs cancer+normal) | 3-10206628 | cg04437751 | (cancer vs cancer) (cancer+normal vs cancer+normal) |
| 11-57532299 | cg16127573 | (cancer vs cancer) (cancer+normal vs cancer+normal) | 3-10206633 | cg05002590 | (cancer vs cancer) (cancer+normal vs cancer+normal) |
| 11-72533295 | cg13771313 | (cancer vs cancer) (cancer+normal vs cancer+normal) | 3-10206731 | cg11029301 | (cancer vs cancer) (cancer+normal vs cancer+normal) |
| 12-117131523 | cg13073224 | (cancer vs cancer) (cancer+normal vs cancer+normal) | 3-125709198 | cg02772935 | (cancer vs cancer) (cancer+normal vs cancer+normal) |
| 12-125002332 | cg01054110 | (cancer vs cancer) (cancer+normal vs cancer+normal) | 3-169482358 | cg27020690 | (cancer vs cancer) (cancer+normal vs cancer+normal) |
| 12-58129855 | cg16402452 | (cancer vs cancer) (cancer+normal vs cancer+normal) | 3-169482714 | cg20839041 | (cancer vs cancer) (cancer+normal vs cancer+normal) |
| 14-51431710 | cg05892030 | (cancer vs cancer) (cancer+normal vs cancer+normal) | 3-169482895 | cg15494117 | (cancer vs cancer) (cancer+normal vs cancer+normal) |
| 14-69256677 | cg01424562 | (cancer vs cancer) (cancer+normal vs cancer+normal) | 3-169482899 | cg15599946 | (cancer vs cancer) (cancer+normal vs cancer+normal) |
| 14-69256690 | cg06617636 | (cancer vs cancer) (cancer+normal vs cancer+normal) | 3-169482968 | cg01389761 | (cancer vs cancer) (cancer+normal vs cancer+normal) |
| 14-69256888 | cg08169020 | (cancer vs cancer) (cancer+normal vs cancer+normal) | 3-169483021 | cg17527574 | (cancer+normal vs cancer+normal) |
| 14-69256977 | cg10099732 | (cancer vs cancer) (cancer+normal vs cancer+normal) | 3-169483025 | cg23036508 | (cancer vs cancer) (cancer+normal vs cancer+normal) |
| 15- | cg10078511 | (cancer vs cancer) | 3-44754398 | cg0497432 | (cancer+normal vs |

| | | | | | |
|---|---|---|---|---|---|
| 39872032 | | (cancer+normal vs cancer+normal) | | 3 | cancer+normal) |
| 15-39872071 | cg08433504 | (cancer vs cancer) (cancer+normal vs cancer+normal) | 3-44770937 | cg10108389 | (cancer vs cancer) (cancer+normal vs cancer+normal) |
| 15-75045126 | cg01359532 | (cancer vs cancer) (cancer+normal vs cancer+normal) | 3-44770954 | cg25574812 | (cancer+normal vs cancer+normal) |
| 15-83879539 | cg21238234 | (cancer vs cancer) (cancer+normal vs cancer+normal) | 3-44770973 | cg02073558 | (cancer vs cancer) (cancer+normal vs cancer+normal) |
| 16-31159704 | cg14301190 | (cancer vs cancer) (cancer+normal vs cancer+normal) | 3-44771102 | cg24837680 | (cancer vs cancer) (cancer+normal vs cancer+normal) |
| 16-67687754 | cg09316954 | (cancer vs cancer) (cancer+normal vs cancer+normal) | 3-44771105 | cg02933119 | (cancer vs cancer) (cancer+normal vs cancer+normal) |
| 16-87416230 | cg09642825 | (cancer vs cancer) (cancer+normal vs cancer+normal) | 3-44771113 | cg17913259 | (cancer+normal vs cancer+normal) |
| 16-87525461 | cg06330621 | (cancer vs cancer) (cancer+normal vs cancer+normal) | 4-189931880 | cg03729462 | (cancer+normal vs cancer+normal) |
| 16-87525539 | cg08196561 | (cancer vs cancer) (cancer+normal vs cancer+normal) | 4-189932474 | cg05255537 | (cancer+normal vs cancer+normal) |
| 17-1464972 | cg24205387 | (cancer vs cancer) (cancer+normal vs cancer+normal) | 4-21699373 | cg08125031 | (cancer+normal vs cancer+normal) |
| 17-15366916 | cg22980156 | (cancer vs cancer) (cancer+normal vs cancer+normal) | 4-2757764 | cg20809690 | (cancer+normal vs cancer+normal) |
| 17-38473119 | cg19458020 | (cancer vs cancer) (cancer+normal vs cancer+normal) | 4-4871525 | cg02680341 | (cancer vs cancer) (normal vs normal) (cancer+normal vs cancer+normal) |
| 17-40075879 | cg19443920 | (cancer vs cancer) (cancer+normal vs cancer+normal) | 5-138728685 | cg01054755 | (cancer vs cancer) (cancer+normal vs cancer+normal) |
| 17-46681362 | cg17179862 | (cancer vs cancer) (cancer+normal vs cancer+normal) | 5-138728704 | cg24761867 | (cancer vs cancer) (cancer+normal vs cancer+normal) |
| 17-46681401 | cg03803541 | (cancer vs cancer) (cancer+normal vs cancer+normal) | 5-138729068 | cg05137680 | (cancer vs cancer) (cancer+normal vs cancer+normal) |
| 17-75283832 | cg17697835 | (cancer vs cancer) (cancer+normal vs cancer+normal) | 5-139494937 | cg14640670 | (cancer+normal vs cancer+normal) |
| 17-76713379 | cg12871285 | (cancer vs cancer) (cancer+normal vs cancer+normal) | 5-87981894 | cg15981305 | (cancer vs cancer) (cancer+normal vs cancer+normal) |
| 19-10445516 | cg17126555 | (cancer vs cancer) (cancer+normal vs cancer+normal) | 6-26043990 | cg22478210 | (cancer vs cancer) (cancer+normal vs cancer+normal) |
| 19-10736448 | cg21631439 | (cancer vs cancer) (cancer+normal vs cancer+normal) | 6-26045532 | cg17432453 | (cancer vs cancer) (cancer+normal vs cancer+normal) |
| 19-15568360 | cg08846870 | (cancer vs cancer) (cancer+normal vs cancer+normal) | 6-26045534 | cg07636178 | (cancer vs cancer) (cancer+normal vs cancer+normal) |
| 19- | cg23450038 | (cancer vs cancer) | 6-26045663 | cg2543896 | (cancer vs cancer) |

| | | | | | |
|---|---|---|---|---|---|
| 2462961 | | (cancer+normal vs cancer+normal) | | 3 | (cancer+normal vs cancer+normal) |
| 19-39086923 | cg05258935 | (cancer vs cancer) (cancer+normal vs cancer+normal) | 6-26045987 | cg10199913 | (cancer+normal vs cancer+normal) |
| 19-41225005 | cg21869046 | (cancer vs cancer) (cancer+normal vs cancer+normal) | 7-35291644 | cg00598758 | (cancer+normal vs cancer+normal) |
| 19-45281140 | cg01057656 | (cancer vs cancer) (cancer+normal vs cancer+normal) | 8-11059029 | cg24631970 | (cancer vs cancer) (cancer+normal vs cancer+normal) |
| 19-6459300 | cg17100943 | (cancer vs cancer) (cancer+normal vs cancer+normal) | 8-49442567 | cg14219543 | (cancer+normal vs cancer+normal) |
| 19-7460671 | cg11734329 | (cancer vs cancer) (cancer+normal vs cancer+normal) | 8-65488069 | cg24255371 | (cancer vs cancer) (cancer+normal vs cancer+normal) |
| 19-8590567 | cg22568423 | (cancer vs cancer) (cancer+normal vs cancer+normal) | 8-95906439 | cg20527070 | (cancer+normal vs cancer+normal) |
| 19-8591364 | cg22987448 | (cancer vs cancer) (cancer+normal vs cancer+normal) | 9-19127804 | cg06797533 | (cancer+normal vs cancer+normal) |
| 20-19866810 | cg23501964 | (cancer vs cancer) (cancer+normal vs cancer+normal) | 9-92023797 | cg21234471 | (cancer+normal vs cancer+normal) |
| 20-19867136 | cg20918393 | (cancer vs cancer) (cancer+normal vs cancer+normal) | **Status - Hypo** | | |
| 22-46423604 | cg00147172 | (cancer vs cancer) (cancer+normal vs cancer+normal) | 1-112960391 | cg14172846 | (normal vs normal) (cancer+normal vs cancer+normal) |
| 22-51136325 | cg18982286 | (cancer vs cancer) (cancer+normal vs cancer+normal) | 1-150448943 | cg04165759 | (cancer+normal vs cancer+normal) |
| 3-101882326 | cg24627325 | (cancer vs cancer) (cancer+normal vs cancer+normal) | 1-155181615 | cg12759597 | (cancer+normal vs cancer+normal) |
| 3-111697575 | cg08979515 | (cancer vs cancer) (cancer+normal vs cancer+normal) | 1-174417967 | cg05768419 | (cancer vs cancer) (cancer+normal vs cancer+normal) |
| 3-141087190 | cg13029400 | (cancer vs cancer) (cancer+normal vs cancer+normal) | 1-183841259 | cg11678039 | (cancer+normal vs cancer+normal) |
| 3-188012918 | cg02524983 | (cancer vs cancer) (cancer+normal vs cancer+normal) | 1-220415655 | cg26570574 | (cancer vs cancer) (cancer+normal vs cancer+normal) |
| 3-57743163 | cg03534375 | (cancer vs cancer) (cancer+normal vs cancer+normal) | 1-233815158 | cg07184643 | (cancer vs cancer) (cancer+normal vs cancer+normal) |
| 3-57743377 | cg15172734 | (cancer vs cancer) (cancer+normal vs cancer+normal) | 1-46921746 | cg00015930 | (cancer vs cancer) (cancer+normal vs cancer+normal) |
| 4-114900431 | cg05601456 | (cancer vs cancer) (cancer+normal vs cancer+normal) | 1-46921844 | cg20977426 | (cancer vs cancer) (cancer+normal vs cancer+normal) |
| 4-159969703 | cg10393744 | (cancer vs cancer) (cancer+normal vs cancer+normal) | 1-86081701 | cg19800670 | (cancer+normal vs cancer+normal) |
| 4-186318356 | cg26741686 | (cancer vs cancer) (cancer+normal vs | 10-116640938 | cg01450600 | (cancer+normal vs cancer+normal) |

| | | | | | |
|---|---|---|---|---|---|
| | | cancer+normal) | | | |
| 4-40665353 | cg06704884 | (cancer vs cancer) (cancer+normal vs cancer+normal) | 10-124316636 | cg19856749 | (cancer vs cancer) (cancer+normal vs cancer+normal) |
| 5-10551547 | cg24100671 | (cancer vs cancer) (cancer+normal vs cancer+normal) | 10-131592435 | cg18629132 | (cancer vs cancer) (cancer+normal vs cancer+normal) |
| 5-133904674 | cg27024654 | (cancer vs cancer) (cancer+normal vs cancer+normal) | 10-17277756 | cg19170009 | (cancer+normal vs cancer+normal) |
| 5-178986906 | cg26516362 | (cancer vs cancer) (cancer+normal vs cancer+normal) | 10-373957 | cg25301103 | (cancer vs cancer) (cancer+normal vs cancer+normal) |
| 5-5271554 | cg25485055 | (cancer vs cancer) (cancer+normal vs cancer+normal) | 10-374010 | cg11423998 | (cancer vs cancer) (cancer+normal vs cancer+normal) |
| 6-143167279 | cg13829419 | (cancer vs cancer) (cancer+normal vs cancer+normal) | 10-374092 | cg09782637 | (cancer vs cancer) (cancer+normal vs cancer+normal) |
| 6-30227001 | cg14296767 | (cancer vs cancer) (cancer+normal vs cancer+normal) | 10-616959 | cg06677151 | (cancer+normal vs cancer+normal) |
| 6-30853608 | cg11975790 | (cancer vs cancer) (cancer+normal vs cancer+normal) | 11-12783675 | cg10427430 | (cancer+normal vs cancer+normal) |
| 6-30881484 | cg10661769 | (cancer vs cancer) (cancer+normal vs cancer+normal) | 11-14367071 | cg01646268 | (cancer+normal vs cancer+normal) |
| 7-15055459 | cg19272540 | (cancer vs cancer) (cancer+normal vs cancer+normal) | 11-2826951 | cg12851635 | (cancer+normal vs cancer+normal) |
| 7-151157166 | cg07748583 | (cancer vs cancer) (cancer+normal vs cancer+normal) | 11-8361190 | cg01677628 | (normal vs normal) (cancer vs cancer) (cancer+normal vs cancer+normal) |
| 7-75367549 | cg13702357 | (cancer vs cancer) (cancer+normal vs cancer+normal) | 11-968936 | cg04372535 | (cancer+normal vs cancer+normal) |
| 8-124498970 | cg13092738 | (cancer vs cancer) (cancer+normal vs cancer+normal) | 12-109046825 | cg06251181 | (cancer vs cancer) (cancer+normal vs cancer+normal) |
| 8-23780241 | cg10855978 | (cancer vs cancer) (cancer+normal vs cancer+normal) | 12-113633320 | cg16465882 | (cancer+normal vs cancer+normal) |
| 8-48437852 | cg08169341 | (cancer vs cancer) (cancer+normal vs cancer+normal) | 12-124556975 | cg09480470 | (cancer+normal vs cancer+normal) |
| 8-61563866 | cg21205855 | (cancer vs cancer) (cancer+normal vs cancer+normal) | 12-124911358 | cg01485790 | (cancer+normal vs cancer+normal) |
| 8-67454546 | cg08620329 | (cancer vs cancer) (cancer+normal vs cancer+normal) | 12-124911444 | cg12509268 | (cancer+normal vs cancer+normal) |
| 8-67454595 | cg12109823 | (cancer vs cancer) (cancer+normal vs cancer+normal) | 12-12605470 | cg24090908 | (cancer vs cancer) (cancer+normal vs cancer+normal) |
| 9-130700954 | cg21181453 | (cancer vs cancer) (cancer+normal vs cancer+normal) | 12-132639764 | cg09451320 | (cancer vs cancer) (cancer+normal vs cancer+normal) |
| 9-14321079 | cg14519997 | (cancer vs cancer) (cancer+normal vs | 12-71862327 | cg13484813 | (cancer+normal vs cancer+normal) |

| | | | | | |
|---|---|---|---|---|---|
| | | cancer+normal) | 13-100497789 | cg0356291 1 | (cancer vs cancer) (cancer+normal vs cancer+normal) |
| **Status - Hypo** | | | | | |
| 1-10965094 | cg19704755 | (cancer vs cancer) (cancer+normal vs cancer+normal) | 13-101091946 | cg1993757 2 | (cancer vs cancer) (cancer+normal vs cancer+normal) |
| 1-151129298 | cg23612220 | (cancer vs cancer) (cancer+normal vs cancer+normal) | 13-101161616 | cg0639833 5 | (cancer vs cancer) (cancer+normal vs cancer+normal) |
| 1-151129844 | cg01903374 | (cancer vs cancer) (cancer+normal vs cancer+normal) | 13-114799776 | cg2292668 6 | (cancer+normal vs cancer+normal) |
| 1-156551787 | cg09319828 | (cancer vs cancer) (cancer+normal vs cancer+normal) | 13-114872102 | cg1698657 1 | (cancer vs cancer) (cancer+normal vs cancer+normal) |
| 1-156551873 | cg01010328 | (cancer vs cancer) (cancer+normal vs cancer+normal) | 13-46297726 | cg2111912 7 | (cancer vs cancer) (cancer+normal vs cancer+normal) |
| 1-159770136 | cg10016364 | (cancer vs cancer) (cancer+normal vs cancer+normal) | 13-96202378 | cg1366253 0 | (cancer vs cancer) (cancer+normal vs cancer+normal) |
| 1-161184921 | cg26394055 | (cancer vs cancer) (cancer+normal vs cancer+normal) | 13-98126882 | cg0823662 3 | (cancer+normal vs cancer+normal) |
| 1-169187004 | cg07948875 | (cancer vs cancer) (cancer+normal vs cancer+normal) | 14-24909125 | cg2338314 9 | (cancer+normal vs cancer+normal) |
| 1-17508341 | cg23013029 | (cancer vs cancer) (cancer+normal vs cancer+normal) | 14-39648415 | cg0691891 8 | (cancer vs cancer) (cancer+normal vs cancer+normal) |
| 1-192515127 | cg24602411 | (cancer vs cancer) (cancer+normal vs cancer+normal) | 16-1511419 | cg0632833 8 | (cancer+normal vs cancer+normal) |
| 1-202129566 | cg18384097 | (cancer vs cancer) (cancer+normal vs cancer+normal) | 16-22296770 | cg0130414 5 | (cancer+normal vs cancer+normal) |
| 1-206946166 | cg17744604 | (cancer vs cancer) (cancer+normal vs cancer+normal) | 16-30795743 | cg2753538 0 | (cancer+normal vs cancer+normal) |
| 1-74665362 | cg18854765 | (cancer vs cancer) (cancer+normal vs cancer+normal) | 16-65789547 | cg1038926 2 | (cancer+normal vs cancer+normal) |
| 10-105978651 | cg23130731 | (cancer vs cancer) (cancer+normal vs cancer+normal) | 16-69358580 | cg0399859 8 | (cancer vs cancer) (cancer+normal vs cancer+normal) |
| 10-34925718 | cg00287536 | (cancer vs cancer) (cancer+normal vs cancer+normal) | 16-70563092 | cg0818634 1 | (cancer vs cancer) (cancer+normal vs cancer+normal) |
| 10-50716478 | cg00025044 | (cancer vs cancer) (cancer+normal vs cancer+normal) | 16-84701635 | cg0100593 7 | (cancer+normal vs cancer+normal) |
| 11-1680823 | cg04552555 | (cancer vs cancer) (cancer+normal vs cancer+normal) | 17-35118924 | cg0143114 0 | (cancer+normal vs cancer+normal) |
| 11-1911547 | cg18723409 | (cancer vs cancer) (cancer+normal vs cancer+normal) | 17-35119063 | cg1522372 6 | (cancer+normal vs cancer+normal) |
| 11-33913716 | cg11822932 | (cancer vs cancer) (cancer+normal vs cancer+normal) | 17-38245542 | cg2352682 4 | (cancer+normal vs cancer+normal) |

| | | | | | |
|---|---|---|---|---|---|
| 11-47399980 | cg03106245 | (cancer vs cancer) (cancer+normal vs cancer+normal) | 17-62208434 | cg12779575 | (cancer+normal vs cancer+normal) |
| 11-47416487 | cg10511890 | (cancer vs cancer) (cancer+normal vs cancer+normal) | 17-73316454 | cg01884715 | (cancer+normal vs cancer+normal) |
| 11-47416886 | cg09739398 | (cancer vs cancer) (cancer+normal vs cancer+normal) | 17-78878278 | cg09173565 | (cancer vs cancer) (cancer+normal vs cancer+normal) |
| 11-65408496 | cg24301930 | (cancer vs cancer) (cancer+normal vs cancer+normal) | 17-78878530 | cg22673070 | (cancer vs cancer) (cancer+normal vs cancer+normal) |
| 11-65661516 | cg07420137 | (cancer vs cancer) (cancer+normal vs cancer+normal) | 17-78894393 | cg06096901 | (cancer+normal vs cancer+normal) |
| 11-66883117 | cg13980609 | (cancer vs cancer) (cancer+normal vs cancer+normal) | 18-19410150 | cg17106157 | (cancer+normal vs cancer+normal) |
| 11-67205096 | cg10542975 | (cancer vs cancer) (cancer+normal vs cancer+normal) | 18-74325812 | cg23553867 | (cancer vs cancer) (cancer+normal vs cancer+normal) |
| 11-70211408 | cg14088196 | (cancer vs cancer) (cancer+normal vs cancer+normal) | 18-74325904 | cg07867133 | (cancer vs cancer) (cancer+normal vs cancer+normal) |
| 11-70211531 | cg25574765 | (cancer vs cancer) (cancer+normal vs cancer+normal) | 19-11304272 | cg08437802 | (cancer+normal vs cancer+normal) |
| 11-70257149 | cg16061668 | (cancer vs cancer) (cancer+normal vs cancer+normal) | 19-3595702 | cg14938419 | (cancer vs cancer) (cancer+normal vs cancer+normal) |
| 11-70268728 | cg11194613 | (cancer vs cancer) (cancer+normal vs cancer+normal) | 19-45150725 | cg23696432 | (cancer+normal vs cancer+normal) |
| 12-2030178 | cg19369955 | (cancer vs cancer) (cancer+normal vs cancer+normal) | 19-6414019 | cg01504784 | (cancer+normal vs cancer+normal) |
| 12-2030223 | cg08621624 | (cancer vs cancer) (cancer+normal vs cancer+normal) | 19-643457 | cg02775417 | (cancer+normal vs cancer+normal) |
| 12-44226393 | cg02522196 | (cancer vs cancer) (cancer+normal vs cancer+normal) | 2-138940416 | cg02204521 | (cancer vs cancer) (cancer+normal vs cancer+normal) |
| 12-51717674 | cg10590292 | (cancer vs cancer) (cancer+normal vs cancer+normal) | 2-174082101 | cg22854985 | (normal vs normal) (cancer+normal vs cancer+normal) |
| 12-51717865 | cg12353452 | (cancer vs cancer) (cancer+normal vs cancer+normal) | 2-240015886 | cg16326123 | (cancer+normal vs cancer+normal) |
| 12-51718088 | cg14556909 | (cancer vs cancer) (cancer+normal vs cancer+normal) | 2-241290895 | cg04003871 | (cancer vs cancer) (cancer vs cancer) (cancer+normal vs cancer+normal) |
| 12-51718112 | cg08075204 | (cancer vs cancer) (cancer+normal vs cancer+normal) | 2-242766284 | cg16514818 | (cancer vs cancer) (cancer+normal vs cancer+normal) |
| 13-113238006 | cg18571045 | (cancer vs cancer) (cancer+normal vs cancer+normal) | 2-242766412 | cg19384073 | (cancer vs cancer) (cancer+normal vs cancer+normal) |
| 14-103440432 | cg14191466 | (cancer vs cancer) (cancer+normal vs cancer+normal) | 2-29116256 | cg00320608 | (cancer+normal vs cancer+normal) |

| | | | | | |
|---|---|---|---|---|---|
| 15-101715747 | cg12042714 | (cancer vs cancer) (cancer+normal vs cancer+normal) | 2-3173279 | cg07948095 | (cancer+normal vs cancer+normal) |
| 16-2737340 | cg09866569 | (cancer vs cancer) (cancer+normal vs cancer+normal) | 2-43598959 | cg16472853 | (cancer vs cancer) (cancer+normal vs cancer+normal) |
| 16-29676071 | cg01681367 | (cancer vs cancer) (cancer+normal vs cancer+normal) | 21-46378243 | cg02930963 | (cancer+normal vs cancer+normal) |
| 16-88866627 | cg10491452 | (cancer vs cancer) (cancer+normal vs cancer+normal) | 22-39547181 | cg12193277 | (cancer+normal vs cancer+normal) |
| 17-263096 | cg25045219 | (cancer vs cancer) (cancer+normal vs cancer+normal) | 3-123414733 | cg18731398 | (cancer+normal vs cancer+normal) |
| 17-46190702 | cg01722297 | (cancer vs cancer) (cancer+normal vs cancer+normal) | 3-128825270 | cg08524190 | (cancer+normal vs cancer+normal) |
| 17-56408197 | cg03649649 | (cancer vs cancer) (cancer+normal vs cancer+normal) | 3-151778972 | cg08929399 | (cancer+normal vs cancer+normal) |
| 17-56409011 | cg26112797 | (cancer vs cancer) (cancer+normal vs cancer+normal) | 3-178984973 | cg00862290 | (cancer vs cancer) (cancer+normal vs cancer+normal) |
| 17-56409028 | cg10530767 | (cancer vs cancer) (cancer+normal vs cancer+normal) | 3-194833983 | cg07250128 | (cancer+normal vs cancer+normal) |
| 17-56565286 | cg27366766 | (cancer vs cancer) (cancer+normal vs cancer+normal) | 3-28889136 | cg08028901 | (cancer vs cancer) (cancer+normal vs cancer+normal) |
| 17-74565009 | cg01883155 | (normal vs normal) (cancer vs cancer) (cancer+normal vs cancer+normal) | 3-69732819 | cg04058837 | (normal vs normal) (cancer+normal vs cancer+normal) |
| 17-77916768 | cg20667480 | (cancer vs cancer) (cancer+normal vs cancer+normal) | 4-1578077 | cg00175895 | (cancer vs cancer) (cancer+normal vs cancer+normal) |
| 17-78748471 | cg00523683 | (cancer vs cancer) (cancer+normal vs cancer+normal) | 4-169952114 | cg00033165 | (cancer+normal vs cancer+normal) |
| 17-78748494 | cg02185248 | (cancer vs cancer) (cancer+normal vs cancer+normal) | 4-190767168 | cg04392133 | (cancer vs cancer) (cancer+normal vs cancer+normal) |
| 17-78755379 | cg06153925 | (cancer vs cancer) (cancer+normal vs cancer+normal) | 5-179302907 | cg07321730 | (cancer+normal vs cancer+normal) |
| 17-800745 | cg14235846 | (cancer vs cancer) (cancer+normal vs cancer+normal) | 5-64308317 | cg24020222 | (cancer+normal vs cancer+normal) |
| 17-80358829 | cg11252953 | (cancer vs cancer) (cancer+normal vs cancer+normal) | 5-64334489 | cg11190987 | (normal vs normal) (cancer+normal vs cancer+normal) |
| 17-80358850 | cg25490145 | (cancer vs cancer) (cancer+normal vs cancer+normal) | 6-138915521 | cg11507780 | (cancer+normal vs cancer+normal) |
| 17-80358876 | cg21465176 | (cancer vs cancer) (cancer+normal vs cancer+normal) | 6-170411557 | cg17267654 | (cancer vs cancer) (cancer+normal vs cancer+normal) |
| 19-13215387 | cg15123730 | (cancer vs cancer) (cancer+normal vs cancer+normal) | 6-170531180 | cg05024916 | (cancer vs cancer) (cancer+normal vs cancer+normal) |

386

| | | | | | |
|---|---|---|---|---|---|
| 19-46294482 | cg03758011 | (cancer vs cancer) (cancer+normal vs cancer+normal) | 6-170531660 | cg22099441 | (cancer vs cancer) (cancer+normal vs cancer+normal) |
| 2-175499113 | cg13821008 | (cancer vs cancer) (cancer+normal vs cancer+normal) | 6-34464251 | cg26715883 | (cancer vs cancer) (cancer+normal vs cancer+normal) |
| 2-68592737 | cg13468685 | (cancer vs cancer) (cancer+normal vs cancer+normal) | 7-101629430 | cg24183566 | (cancer+normal vs cancer+normal) |
| 2-74264707 | cg01987702 | (cancer vs cancer) (cancer+normal vs cancer+normal) | 7-120702049 | cg08554603 | (cancer+normal vs cancer+normal) |
| 2-88327530 | cg04195125 | (cancer vs cancer) (cancer+normal vs cancer+normal) | 7-139690853 | cg03764092 | (cancer vs cancer) (cancer+normal vs cancer+normal) |
| 20-35944932 | cg00916899 | (cancer vs cancer) (cancer+normal vs cancer+normal) | 7-139690881 | cg23625084 | (cancer vs cancer) (cancer+normal vs cancer+normal) |
| 20-3693158 | cg14921416 | (cancer vs cancer) (cancer+normal vs cancer+normal) | 7-4807556 | cg04725442 | (cancer+normal vs cancer+normal) |
| 21-44819445 | cg17122157 | (cancer vs cancer) (cancer+normal vs cancer+normal) | 7-77492522 | cg22614518 | (cancer vs cancer) (cancer+normal vs cancer+normal) |
| 3-196351986 | cg00828556 | (cancer vs cancer) (cancer+normal vs cancer+normal) | 8-41113257 | cg18144647 | (cancer+normal vs cancer+normal) |
| 3-56789178 | cg08052292 | (cancer vs cancer) (cancer+normal vs cancer+normal) | **(normal vs normal)** | | |
| 4-1221838 | cg26923863 | (cancer vs cancer) (cancer+normal vs cancer+normal) | **Status - Hyper** | | |
| 5-1503763 | cg19503640 | (cancer vs cancer) (cancer+normal vs cancer+normal) | 1-102308808 | cg09180564 | (normal vs normal) |
| 5-55215955 | cg05650238 | (cancer vs cancer) (cancer+normal vs cancer+normal) | 1-150552817 | cg17724175 | (normal vs normal) |
| 5-77783895 | cg18281939 | (cancer vs cancer) (cancer+normal vs cancer+normal) | 1-19278734 | cg16552822 | (normal vs normal) |
| 6-135644405 | cg14564351 | (cancer vs cancer) (cancer+normal vs cancer+normal) | 1-219347279 | cg15558129 | (normal vs normal) |
| 6-137105249 | cg02673417 | (cancer vs cancer) (cancer+normal vs cancer+normal) | 1-219347458 | cg03221247 | (normal vs normal) |
| 6-151380818 | cg07805500 | (cancer vs cancer) (cancer+normal vs cancer+normal) | 1-234658467 | cg00449021 | (normal vs normal) |
| 6-157932130 | cg09783253 | (cancer vs cancer) (cancer+normal vs cancer+normal) | 1-94571619 | cg04100956 | (normal vs normal) |
| 6-158406044 | cg10799846 | (cancer vs cancer) (cancer+normal vs cancer+normal) | 10-12762778 | cg26433640 | (normal vs normal) |
| 6-166877002 | cg07253384 | (cancer vs cancer) (cancer+normal vs cancer+normal) | 10-16566625 | cg19197922 | (normal vs normal) (normal vs normal) (cancer+normal vs cancer+normal) |

| | | | | | |
|---|---|---|---|---|---|
| 6-166877038 | cg02287007 | (cancer vs cancer) (cancer+normal vs cancer+normal) | 10-8103673 | cg00463367 | (normal vs normal) (cancer+normal vs cancer+normal) |
| 6-27842098 | cg01247537 | (cancer vs cancer) (cancer+normal vs cancer+normal) | 11-104817914 | cg12407979 | (normal vs normal) |
| 6-31583942 | cg18113826 | (cancer vs cancer) (cancer+normal vs cancer+normal) | 11-20690223 | cg10964385 | (normal vs normal) |
| 6-31695415 | cg25953692 | (cancer vs cancer) (cancer+normal vs cancer+normal) | 11-20934569 | cg11924499 | (normal vs normal) |
| 6-31695552 | cg03900314 | (cancer vs cancer) (cancer+normal vs cancer+normal) | 11-32421514 | cg01168289 | (normal vs normal) |
| 6-31695599 | cg13597051 | (cancer vs cancer) (cancer+normal vs cancer+normal) | 11-32421752 | cg20449659 | (normal vs normal) |
| 6-32155082 | cg09001226 | (cancer vs cancer) (cancer+normal vs cancer+normal) | 11-32421808 | cg10244666 | (normal vs normal) |
| 7-2773782 | cg20336172 | (cancer vs cancer) (cancer+normal vs cancer+normal) | 11-67186575 | cg04869854 | (normal vs normal) |
| 7-2773812 | cg26247093 | (cancer vs cancer) (cancer+normal vs cancer+normal) | 12-108960008 | cg22277972 | (normal vs normal) |
| 7-3134557 | cg09450197 | (cancer vs cancer) (cancer+normal vs cancer+normal) | 12-113671028 | cg21110337 | (normal vs normal) |
| 7-32802765 | cg07573366 | (normal vs normal) (cancer vs cancer) (cancer+normal vs cancer+normal) | 12-115133159 | cg11302945 | (normal vs normal) |
| 7-5258357 | cg20071624 | (cancer vs cancer) (cancer+normal vs cancer+normal) | 12-130641648 | cg05684715 | (normal vs normal) |
| 7-6476003 | cg06488150 | (cancer vs cancer) (cancer+normal vs cancer+normal) | 12-58150842 | cg17828445 | (normal vs normal) |
| 7-6476110 | cg08313420 | (cancer vs cancer) (cancer+normal vs cancer+normal) | 12-74932579 | cg04257969 | (normal vs normal) |
| 8-134559320 | cg02609337 | (cancer vs cancer) (cancer+normal vs cancer+normal) | 12-92531553 | cg04661457 | (normal vs normal) |
| 8-140945810 | cg26769700 | (cancer vs cancer) (cancer+normal vs cancer+normal) | 13-113379828 | cg11360522 | (normal vs normal) |
| 8-141609380 | cg18440839 | (cancer vs cancer) (cancer+normal vs cancer+normal) | 14-24801191 | cg09822192 | (normal vs normal) |
| 8-145052950 | cg00015530 | (cancer vs cancer) (cancer+normal vs cancer+normal) | 14-24801301 | cg26229990 | (normal vs normal) |
| 8-22398190 | cg19021985 | (cancer vs cancer) (cancer+normal vs cancer+normal) | 14-75894136 | cg10101773 | (normal vs normal) |
| 9-134454249 | cg13555415 | (cancer vs cancer) (cancer+normal vs cancer+normal) | 15-74592665 | cg21565421 | (normal vs normal) |

388

| | | | | | |
|---|---|---|---|---|---|
| 9-96080326 | cg20324199 | (cancer vs cancer) (cancer+normal vs cancer+normal) | 15-92398112 | cg22260869 | (normal vs normal) |
| **UpRCaG (cancer vs cancer)** | | | 15-97244292 | cg27234929 | (normal vs normal) |
| **Status - Hyper** | | | 16-56642024 | cg17886959 | (normal vs normal) |
| 1-152085136 | cg20795569 | (cancer vs cancer) (cancer vs cancer) (cancer+normal vs cancer+normal) | 17-55533254 | cg02770857 | (normal vs normal) |
| 1-33438854 | cg18018890 | (cancer vs cancer) | 17-79265759 | cg25988214 | (normal vs normal) |
| 1-33438978 | cg23012731 | (cancer vs cancer) | 18-77440800 | cg01589580 | (normal vs normal) |
| 1-44401857 | cg26114324 | (cancer vs cancer) (cancer vs cancer) (cancer+normal vs cancer+normal) | 19-17514117 | cg22282590 | (normal vs normal) |
| 1-44401979 | cg18115627 | (cancer vs cancer) (cancer vs cancer) (cancer+normal vs cancer+normal) | 19-21860753 | cg08562672 | (normal vs normal) |
| 1-44402353 | cg23954153 | (cancer vs cancer) (cancer vs cancer) (cancer+normal vs cancer+normal) | 19-51190047 | cg11284196 | (normal vs normal) |
| 1-6546574 | cg24535622 | (cancer vs cancer) (cancer vs cancer) (cancer+normal vs cancer+normal) | 2-128458240 | cg20154206 | (normal vs normal) |
| 10-126136435 | cg05291069 | (cancer vs cancer) (cancer vs cancer) | 2-131278268 | cg13668005 | (normal vs normal) |
| 10-126136607 | cg08932440 | (cancer vs cancer) | 2-131357649 | cg18838133 | (normal vs normal) |
| 10-126136709 | cg06299833 | (cancer vs cancer) (cancer vs cancer) | 2-132430151 | cg09894655 | (normal vs normal) |
| 10-60935910 | cg07062043 | (cancer vs cancer) | 2-133106819 | cg12792250 | (normal vs normal) |
| 10-60936451 | cg26014391 | (cancer vs cancer) | 2-160041968 | cg08384239 | (normal vs normal) |
| 10-60936626 | cg13686115 | (cancer vs cancer) | 2-177012117 | cg14298457 | (normal vs normal) |
| 10-95326801 | cg13262165 | (cancer vs cancer) (cancer vs cancer) | 2-177012372 | cg15990972 | (normal vs normal) |
| 10-98480124 | cg04115659 | (cancer vs cancer) (cancer+normal vs cancer+normal) | 2-177016490 | cg19509393 | (normal vs normal) |
| 11-22850866 | cg01948390 | (cancer vs cancer) (cancer vs cancer) (cancer+normal vs cancer+normal) | 2-177024502 | cg05266564 | (normal vs normal) |
| 11-22850891 | cg03988778 | (cancer vs cancer) (cancer vs cancer) (cancer+normal vs cancer+normal) | 2-177025859 | cg23676302 | (normal vs normal) |
| 11-44340839 | cg21064916 | (cancer vs cancer) | 2-177025975 | cg07930539 | (normal vs normal) |
| 12-113661270 | cg24281668 | (cancer vs cancer) (cancer vs cancer) (cancer+normal vs | 2-177029459 | cg24541426 | (normal vs normal) |

| | | cancer+normal) | | | |
|---|---|---|---|---|---|
| 12-24717001 | cg17405840 | (cancer vs cancer) (cancer vs cancer) | 2-177037364 | cg10159630 | (normal vs normal) |
| 13-96294131 | cg11337289 | (cancer vs cancer) | 2-177037631 | cg13053653 | (normal vs normal) |
| 14-59931110 | cg16322262 | (cancer vs cancer) (cancer vs cancer) (cancer+normal vs cancer+normal) | 2-218898788 | cg23940612 | (normal vs normal) |
| 14-59931541 | cg22821834 | (cancer vs cancer) (cancer vs cancer) (cancer+normal vs cancer+normal) | 2-240291657 | cg06117093 | (normal vs normal) |
| 14-59931922 | cg15436096 | (cancer vs cancer) (cancer vs cancer) (cancer+normal vs cancer+normal) | 2-60778395 | cg08627352 | (normal vs normal) |
| 14-59932033 | cg09859398 | (cancer vs cancer) (cancer vs cancer) (cancer+normal vs cancer+normal) | 2-80549973 | cg01146063 | (normal vs normal) |
| 14-59932101 | cg04186360 | (cancer vs cancer) (cancer vs cancer) (cancer+normal vs cancer+normal) | 20-43378953 | cg25301532 | (normal vs normal) |
| 14-59932113 | cg14847688 | (cancer vs cancer) (cancer vs cancer) (cancer+normal vs cancer+normal) | 20-45986173 | cg02767841 | (cancer vs cancer) (normal vs normal) |
| 14-59932128 | cg05217962 | (cancer vs cancer) (cancer vs cancer) (cancer+normal vs cancer+normal) | 20-55839008 | cg07781736 | (normal vs normal) |
| 14-59932146 | cg01678172 | (cancer vs cancer) (cancer vs cancer) (cancer+normal vs cancer+normal) | 20-57042304 | cg03960424 | (normal vs normal) |
| 14-59932153 | cg21893185 | (cancer vs cancer) (cancer vs cancer) (cancer+normal vs cancer+normal) | 22-51066142 | cg13909895 | (normal vs normal) |
| 14-59932250 | cg16411251 | (cancer vs cancer) (cancer vs cancer) (cancer+normal vs cancer+normal) | 3-123476518 | cg12235788 | (normal vs normal) |
| 14-59932273 | cg10014563 | (cancer vs cancer) (cancer vs cancer) (cancer+normal vs cancer+normal) | 3-18464935 | cg18808261 | (normal vs normal) |
| 15-40545050 | cg24498031 | (cancer vs cancer) | 4-152967131 | cg01653942 | (normal vs normal) |
| 15-40545143 | cg13892980 | (cancer vs cancer) | 4-155410860 | cg22744079 | (normal vs normal) |
| 15-40545145 | cg18097281 | (cancer vs cancer) | 4-174422908 | cg08125755 | (normal vs normal) |
| 15-40545175 | cg12635019 | (cancer vs cancer) | 4-778924 | cg06673130 | (normal vs normal) |
| 15-40545215 | cg04305879 | (cancer vs cancer) | 4-779173 | cg05448404 | (normal vs normal) |
| 15-40545296 | cg05068428 | (cancer vs cancer) | 5-1108651 | cg08543327 | (normal vs normal) |

| | | | | | |
|---|---|---|---|---|---|
| 16-55514378 | cg09350341 | (cancer vs cancer) (cancer vs cancer) | 5-1110226 | cg26244838 | (normal vs normal) |
| 16-66878654 | cg10026203 | (cancer vs cancer) (cancer vs cancer) | 5-113029364 | cg09815251 | (normal vs normal) |
| 16-66878676 | cg06524479 | (cancer vs cancer) (cancer vs cancer) | 5-138923267 | cg12303215 | (normal vs normal) |
| 16-66878685 | cg10373196 | (cancer vs cancer) (cancer vs cancer) | 5-171605778 | cg04192740 | (normal vs normal) |
| 16-66878687 | cg05440435 | (cancer vs cancer) (cancer vs cancer) | 5-175561172 | cg22210497 | (normal vs normal) |
| 16-66878871 | cg05552717 | (cancer vs cancer) | 5-179124978 | cg04652522 | (normal vs normal) |
| 17-38498914 | cg16276850 | (cancer vs cancer) (cancer vs cancer) (cancer+normal vs cancer+normal) | 5-4942469 | cg18101138 | (normal vs normal) |
| 17-42061328 | cg24704730 | (cancer vs cancer) (normal vs normal) | 5-72594735 | cg17373554 | (normal vs normal) |
| 17-5404330 | cg22298430 | (cancer vs cancer) (cancer vs cancer) | 5-92909434 | cg15143788 | (normal vs normal) |
| 17-5404337 | cg27230784 | (cancer vs cancer) (cancer vs cancer) | 5-92929319 | cg17774851 | (normal vs normal) |
| 17-5404581 | cg18671949 | (cancer vs cancer) (cancer vs cancer) (cancer+normal vs cancer+normal) | 5-92929372 | cg25449484 | (normal vs normal) |
| 17-72920359 | cg04886642 | (cancer vs cancer) | 5-92929573 | cg08241477 | (normal vs normal) |
| 17-72920362 | cg25306087 | (cancer vs cancer) (cancer vs cancer) | 5-92930634 | cg09560082 | (normal vs normal) |
| 17-72920378 | cg06353318 | (cancer vs cancer) (cancer+normal vs cancer+normal) | 5-92931173 | cg18290233 | (normal vs normal) |
| 17-72920385 | cg12483561 | (cancer vs cancer) | 5-92931623 | cg18307783 | (normal vs normal) |
| 17-72920598 | cg09461395 | (cancer vs cancer) (cancer vs cancer) | 5-92932045 | cg08802053 | (normal vs normal) |
| 18-47087419 | cg17012181 | (cancer vs cancer) (cancer vs cancer) | 5-92935283 | cg07533422 | (normal vs normal) |
| 18-47088248 | cg27113059 | (cancer vs cancer) | 5-92938149 | cg22736807 | (normal vs normal) |
| 19-12163582 | cg26626525 | (cancer vs cancer) | 5-92938232 | cg06820621 | (normal vs normal) |
| 19-12163679 | cg06899953 | (cancer vs cancer) | 7-69447465 | cg09703727 | (normal vs normal) |
| 19-18335182 | cg18816098 | (cancer vs cancer) (cancer vs cancer) (cancer+normal vs cancer+normal) | 8-114444356 | cg24759795 | (normal vs normal) |
| 19-37341870 | cg03100040 | (cancer vs cancer) | 8-32985289 | cg06106484 | (normal vs normal) |
| 19-40314862 | cg19396867 | (cancer vs cancer) | 8-49231688 | cg15186638 | (normal vs normal) |
| 19-40314899 | cg06528584 | (cancer vs cancer) (cancer vs cancer) | 8-49352435 | cg22303083 | (normal vs normal) |
| 19-40314927 | cg06936564 | (cancer vs cancer) (cancer vs cancer) | 8-77592946 | cg14595683 | (normal vs normal) |
| 19-40315143 | cg00498691 | (cancer vs cancer) | 8-91640288 | cg07171609 | (normal vs normal) |
| 19-46974886 | cg10788735 | (cancer vs cancer) (cancer vs cancer) | 8-97657097 | cg14332120 | (normal vs normal) |

| | | | | | | |
|---|---|---|---|---|---|---|
| 19-53400545 | cg19660239 | (cancer vs cancer) (cancer+normal vs cancer+normal) | 9-110249749 | cg03066050 | (normal vs normal) |
| 19-53696649 | cg01620580 | (cancer vs cancer) | 9-140173347 | cg14018215 | (normal vs normal) |
| 2-171387064 | cg19702675 | (cancer vs cancer) (cancer vs cancer) (cancer+normal vs cancer+normal) | 9-140173549 | cg09381737 | (normal vs normal) |
| 2-238395852 | cg24603464 | (cancer vs cancer) (cancer vs cancer) | **Status - Hypo** | | |
| 21-38119946 | cg09556952 | (cancer vs cancer) | 1-112960391 | cg14172846 | (normal vs normal) (cancer+normal vs cancer+normal) |
| 21-46131923 | cg11631644 | (cancer vs cancer) | 1-156050126 | cg16549043 | (normal vs normal) |
| 22-28010528 | cg19343611 | (cancer vs cancer) (cancer vs cancer) | 1-172352681 | cg26020878 | (normal vs normal) |
| 22-37213466 | cg21789136 | (cancer vs cancer) | 1-203156625 | cg19081101 | (normal vs normal) |
| 22-42679804 | cg13836638 | (cancer vs cancer) | 1-205326071 | cg08159989 | (normal vs normal) |
| 3-42814854 | cg09008417 | (cancer vs cancer) (cancer vs cancer) (cancer+normal vs cancer+normal) | 1-205326437 | cg13459217 | (normal vs normal) |
| 3-75834335 | cg27409478 | (cancer vs cancer) (cancer vs cancer) (cancer+normal vs cancer+normal) | 1-205819179 | cg14159672 | (normal vs normal) |
| 4-143766916 | cg21943416 | (cancer vs cancer) (cancer vs cancer) | 1-205819251 | cg14893161 | (normal vs normal) |
| 4-143767118 | cg00183107 | (cancer vs cancer) (cancer vs cancer) | 1-205819406 | cg11965913 | (normal vs normal) |
| 5-1155853 | cg26330510 | (cancer vs cancer) | 1-221057558 | cg09515921 | (normal vs normal) |
| 5-1594676 | cg21167402 | (cancer vs cancer) (cancer vs cancer) | 1-245499719 | cg16591381 | (normal vs normal) |
| 5-1594678 | cg24960960 | (cancer vs cancer) (cancer vs cancer) (normal vs normal) | 1-3028577 | cg06773295 | (normal vs normal) |
| 5-1594715 | cg21931717 | (cancer vs cancer) (cancer vs cancer) (normal vs normal) | 1-3269252 | cg27187555 | (normal vs normal) |
| 5-170289021 | cg20780811 | (cancer vs cancer) (cancer vs cancer) | 1-94560697 | cg09234454 | (normal vs normal) (cancer vs cancer) (cancer+normal vs cancer+normal) |
| 5-94956017 | cg02320454 | (cancer vs cancer) | 10-1402917 | cg01033356 | (normal vs normal) |
| 6-166722053 | cg27369401 | (cancer vs cancer) (cancer vs cancer) (cancer+normal vs cancer+normal) | 10-3158994 | cg24872610 | (normal vs normal) |
| 6-166722060 | cg02151625 | (cancer vs cancer) (cancer vs cancer) (cancer+normal vs cancer+normal) | 10-33524393 | cg03442322 | (normal vs normal) |
| 6-28092048 | cg13045351 | (cancer vs cancer) (cancer vs cancer) (cancer+normal vs | 10-87682317 | cg24057204 | (normal vs normal) |

392

| | | | | | |
|---|---|---|---|---|---|
| | | cancer+normal) | | | |
| 6-46097521 | cg00567696 | (cancer vs cancer) (cancer+normal vs cancer+normal) | 11-102651753 | cg00347729 | (normal vs normal) |
| 6-46097671 | cg23634318 | (cancer vs cancer) (cancer vs cancer) (cancer+normal vs cancer+normal) | 11-115581830 | cg22821289 | (normal vs normal) |
| 6-46097695 | cg06768203 | (cancer vs cancer) (cancer vs cancer) (cancer+normal vs cancer+normal) | 11-1482176 | cg10002860 | (normal vs normal) |
| 6-46097784 | cg08211306 | (cancer vs cancer) (cancer vs cancer) (cancer+normal vs cancer+normal) | 11-1901748 | cg04948649 | (normal vs normal) |
| 8-145027948 | cg24507266 | (cancer vs cancer) (cancer vs cancer) (cancer+normal vs cancer+normal) | 11-44899805 | cg01562537 | (normal vs normal) |
| 8-145028093 | cg06452769 | (cancer vs cancer) (cancer vs cancer) (cancer+normal vs cancer+normal) | 11-57364841 | cg09061733 | (normal vs normal) |
| 8-145028103 | cg14234406 | (cancer vs cancer) (cancer vs cancer) (cancer+normal vs cancer+normal) | 11-68472905 | cg25218797 | (normal vs normal) |
| 8-37605978 | cg00450319 | (cancer vs cancer) | 11-8361190 | cg01677628 | (normal vs normal) (cancer vs cancer) (cancer+normal vs cancer+normal) |
| 9-35757158 | cg13439181 | (cancer vs cancer) (cancer vs cancer) (cancer+normal vs cancer+normal) | 12-124929704 | cg08143046 | (normal vs normal) |
| 9-35757314 | cg20227976 | (cancer vs cancer) (cancer vs cancer) (cancer+normal vs cancer+normal) | 13-112627458 | cg25849642 | (normal vs normal) |
| 9-4299830 | cg13513288 | (cancer vs cancer) (cancer vs cancer) (cancer+normal vs cancer+normal) | 13-113797929 | cg15605744 | (normal vs normal) |
| 9-96713643 | cg07852402 | (cancer vs cancer) (cancer vs cancer) (normal vs normal) (cancer+normal vs cancer+normal) | 13-29290660 | cg26650383 | (normal vs normal) |
| 9-96715256 | cg13398482 | (cancer vs cancer) (normal vs normal) | 13-46426238 | cg08605991 | (normal vs normal) |
| **Status - Hypo** | | | 13-46426263 | cg19432362 | (normal vs normal) |
| 1-10704953 | cg16401490 | (cancer vs cancer) (cancer vs cancer) | 13-99174312 | cg10533161 | (normal vs normal) |
| 1-147101904 | cg07790752 | (cancer vs cancer) (cancer vs cancer) | 14-100435663 | cg10115368 | (normal vs normal) |
| 1-1564422 | cg00078456 | (cancer vs cancer) (cancer vs cancer) | 15-58246538 | cg19847244 | (normal vs normal) |
| 1-1564920 | cg17122213 | (cancer vs cancer) (cancer+normal vs | 15-62917317 | cg26456304 | (normal vs normal) |

| | | | | | | |
|---|---|---|---|---|---|---|
| 1-15681735 | cg10313278 | (cancer vs cancer) | 16-1175678 | cg10385692 | (normal vs normal) |
| 1-161928549 | cg10230257 | (cancer vs cancer)<br>(cancer vs cancer)<br>(cancer+normal vs cancer+normal) | 16-1231723 | cg00300794 | (normal vs normal) |
| 1-162337248 | cg11687863 | (cancer vs cancer) | 16-1493637 | cg08401703 | (normal vs normal) |
| 1-162337320 | cg12486795 | (cancer vs cancer)<br>(cancer vs cancer)<br>(cancer+normal vs cancer+normal) | 16-4442918 | cg02351655 | (normal vs normal) |
| 1-223567955 | cg06475894 | (cancer vs cancer) | 16-54407990 | cg02583618 | (normal vs normal) |
| 1-228362309 | cg24846680 | (cancer vs cancer) | 16-55067613 | cg04340928 | (normal vs normal) |
| 1-228362509 | cg18277682 | (cancer vs cancer)<br>(cancer vs cancer) | 16-85508110 | cg16682899 | (normal vs normal) |
| 1-2303963 | cg18106803 | (cancer vs cancer)<br>(cancer vs cancer) | 16-875966 | cg05813818 | (normal vs normal) |
| 1-43971496 | cg25211006 | (cancer vs cancer)<br>(cancer vs cancer)<br>(cancer+normal vs cancer+normal) | 16-88738418 | cg03682568 | (normal vs normal) |
| 1-76262648 | cg22035229 | (cancer vs cancer) | 16-88990161 | cg00762830 | (normal vs normal) |
| 1-76262711 | cg17028301 | (cancer vs cancer) | 17-10002773 | cg03594506 | (normal vs normal) |
| 10-1156482 | cg03444838 | (cancer vs cancer)<br>(cancer vs cancer) | 17-13899286 | cg11492886 | (normal vs normal) |
| 10-116097581 | cg04784635 | (cancer vs cancer)<br>(cancer vs cancer)<br>(cancer+normal vs cancer+normal) | 17-17714347 | cg09015921 | (normal vs normal) |
| 10-29578013 | cg24075136 | (cancer vs cancer)<br>(cancer vs cancer)<br>(cancer+normal vs cancer+normal) | 17-17714414 | cg22946219 | (normal vs normal) |
| 10-88445516 | cg16862260 | (cancer vs cancer)<br>(cancer+normal vs cancer+normal) | 17-3791952 | cg19713980 | (normal vs normal) |
| 10-95847671 | cg11322432 | (cancer vs cancer)<br>(cancer vs cancer) | 17-53713264 | cg27142354 | (normal vs normal) |
| 11-1543206 | cg02376282 | (cancer vs cancer)<br>(cancer vs cancer)<br>(cancer+normal vs cancer+normal) | 17-74160634 | cg18272307 | (normal vs normal) |
| 11-1543275 | cg08373904 | (cancer vs cancer)<br>(cancer vs cancer)<br>(cancer+normal vs cancer+normal) | 17-80573287 | cg14060471 | (normal vs normal) |
| 11-17803389 | cg08129093 | (cancer vs cancer)<br>(cancer+normal vs cancer+normal) | 18-10488229 | cg18208602 | (normal vs normal) |
| 11-47213196 | cg05584439 | (cancer vs cancer)<br>(cancer vs cancer)<br>(cancer+normal vs cancer+normal) | 18-33485300 | cg03599197 | (normal vs normal) |
| 11-67892272 | cg04113632 | (cancer vs cancer) | 18-46501202 | cg07704235 | (normal vs normal) |

| | | | | | |
|---|---|---|---|---|---|
| 11-69089119 | cg24121967 | (cancer vs cancer) (cancer vs cancer) | 19-3942217 | cg24428653 | (normal vs normal) |
| 11-78053252 | cg13226797 | (cancer vs cancer) | 19-4446031 | cg24419436 | (normal vs normal) |
| 12-109006223 | cg03600432 | (cancer vs cancer) | 2-105363536 | cg18703066 | (normal vs normal) |
| 12-131355465 | cg25201101 | (cancer vs cancer) | 2-121487296 | cg13024761 | (normal vs normal) |
| 12-1726076 | cg07896667 | (cancer vs cancer) (cancer vs cancer) (cancer+normal vs cancer+normal) | 2-16430654 | cg19911430 | (normal vs normal) |
| 12-28186450 | cg19224639 | (cancer vs cancer) | 2-174082101 | cg22854985 | (normal vs normal) (cancer+normal vs cancer+normal) |
| 13-33743647 | cg13370010 | (cancer vs cancer) (cancer vs cancer) | 2-20001490 | cg16233797 | (normal vs normal) |
| 15-63062631 | cg14101284 | (cancer vs cancer) (cancer vs cancer) | 2-236100751 | cg25730564 | (normal vs normal) |
| 16-57759992 | cg05881753 | (cancer vs cancer) | 20-48183673 | cg06357305 | (normal vs normal) |
| 16-66887815 | cg27078786 | (cancer vs cancer) | 21-48018608 | cg19434199 | (normal vs normal) |
| 16-74752765 | cg18642576 | (cancer vs cancer) | 22-20229013 | cg22677723 | (normal vs normal) |
| 16-84446919 | cg00838040 | (cancer vs cancer) (cancer vs cancer) (cancer+normal vs cancer+normal) | 3-10327453 | cg22156128 | (normal vs normal) |
| 16-84871952 | cg00308659 | (cancer vs cancer) (cancer vs cancer) | 3-24887420 | cg20581674 | (normal vs normal) |
| 16-928338 | cg09080920 | (cancer vs cancer) (cancer vs cancer) | 3-69732819 | cg04058837 | (normal vs normal) (cancer+normal vs cancer+normal) |
| 17-78820391 | cg23019125 | (cancer vs cancer) (cancer vs cancer) | 4-3948578 | cg20694303 | (normal vs normal) |
| 17-80671131 | cg01902059 | (cancer vs cancer) | 4-8073804 | cg10754215 | (normal vs normal) |
| 17-8128726 | cg07847788 | (cancer vs cancer) | 5-150029215 | cg27039501 | (normal vs normal) |
| 18-580228 | cg20731110 | (cancer vs cancer) | 5-171094937 | cg06635150 | (normal vs normal) |
| 19-14552350 | cg13964781 | (cancer vs cancer) (cancer vs cancer) | 5-171533681 | cg25645199 | (normal vs normal) |
| 2-240008233 | cg09468399 | (cancer vs cancer) (cancer vs cancer) (cancer+normal vs cancer+normal) | 5-2169769 | cg27369096 | (normal vs normal) |
| 2-25600752 | cg17774001 | (cancer vs cancer) (cancer vs cancer) (cancer+normal vs cancer+normal) | 5-77935323 | cg19781637 | (normal vs normal) |
| 2-29149902 | cg21005412 | (cancer vs cancer) | 6-124857134 | cg10156493 | (normal vs normal) |
| 2-97652489 | cg12055459 | (cancer vs cancer) (cancer vs cancer) | 6-152126080 | cg07619683 | (normal vs normal) |
| 2-97652491 | cg13571852 | (cancer vs cancer) (cancer vs cancer) | 6-152126092 | cg07189962 | (normal vs normal) |
| 20-56888536 | cg15032960 | (cancer vs cancer) (cancer vs cancer) | 6-167655053 | cg13046724 | (normal vs normal) |
| 22- | cg08425796 | (cancer vs cancer) | 6- | cg2733344 | (normal vs normal) |

| | | | | | |
|---|---|---|---|---|---|
| 50981121 | | (cancer vs cancer) | 167680455 | 1 | |
| 3-114035950 | cg08723913 | (cancer vs cancer) | 6-17116181 | cg25637972 | (normal vs normal) |
| 3-195947062 | cg17572056 | (cancer vs cancer) (cancer vs cancer) | 6-19180751 | cg18747742 | (normal vs normal) |
| 4-129643491 | cg01266375 | (cancer vs cancer) (cancer vs cancer) | 6-31980836 | cg20161227 | (normal vs normal) |
| 4-151179898 | cg06676006 | (cancer vs cancer) (cancer vs cancer) | 6-37109522 | cg21950107 | (normal vs normal) |
| 5-140868130 | cg03898832 | (cancer vs cancer) (cancer vs cancer) | 6-43714275 | cg07148341 | (normal vs normal) |
| 5-149675916 | cg14105467 | (cancer vs cancer) (cancer vs cancer) | 6-54156824 | cg16523850 | (normal vs normal) |
| 5-175955925 | cg20748242 | (cancer vs cancer) (cancer+normal vs cancer+normal) | 7-100744067 | cg27092935 | (normal vs normal) |
| 5-421733 | cg00976097 | (cancer vs cancer) | 7-101079617 | cg15127702 | (normal vs normal) |
| 5-465330 | cg15026243 | (cancer vs cancer) (cancer vs cancer) (cancer+normal vs cancer+normal) | 7-1121657 | cg04206484 | (normal vs normal) |
| 5-465665 | cg11869150 | (cancer vs cancer) | 7-1369374 | cg01422767 | (normal vs normal) |
| 6-134911061 | cg05368910 | (cancer vs cancer) (cancer vs cancer) | 7-140227195 | cg18541042 | (normal vs normal) |
| 6-14305310 | cg00273754 | (cancer vs cancer) (cancer vs cancer) | 7-140227273 | cg13675721 | (normal vs normal) |
| 6-157939700 | cg09484852 | (cancer vs cancer) (cancer vs cancer) | 7-142637936 | cg21443699 | (normal vs normal) |
| 6-163730283 | cg10584587 | (cancer vs cancer) (cancer vs cancer) (cancer+normal vs cancer+normal) | 7-151142178 | cg22326328 | (normal vs normal) |
| 6-168393930 | cg00664730 | (cancer vs cancer) (cancer vs cancer) | 7-155580742 | cg03510186 | (normal vs normal) |
| 6-168394191 | cg19527959 | (cancer vs cancer) (cancer vs cancer) (cancer+normal vs cancer+normal) | 8-120599284 | cg07236691 | (normal vs normal) |
| 6-168394198 | cg14041603 | (cancer vs cancer) (cancer vs cancer) (cancer+normal vs cancer+normal) | 9-132262821 | cg14582917 | (normal vs normal) |
| 6-168394477 | cg10367069 | (cancer vs cancer) (cancer vs cancer) | | | |
| 6-168684623 | cg06251420 | (cancer vs cancer) (cancer vs cancer) (cancer+normal vs cancer+normal) | | | |
| 6-169689773 | cg03126567 | (cancer vs cancer) | | | |
| 6-6971315 | cg18679635 | (cancer vs cancer) (cancer vs cancer) (cancer+normal vs cancer+normal) | | | |
| 6-6971335 | cg09146892 | (cancer vs cancer) (cancer+normal vs cancer+normal) | | | |
| 6-6971371 | cg19751937 | (normal vs normal) (cancer vs cancer) | | | |

| | | |
|---|---|---|
| | | (cancer vs cancer) (cancer+normal vs cancer+normal) |
| 7-1102344 | cg12692727 | (cancer vs cancer) (cancer vs cancer) (cancer+normal vs cancer+normal) |
| 7-112431576 | cg04876978 | (cancer vs cancer) |
| 7-149257723 | cg27053157 | (cancer vs cancer) |
| 7-157202405 | cg18753341 | (cancer vs cancer) |
| 7-157204364 | cg18362281 | (cancer vs cancer) (cancer vs cancer) (cancer+normal vs cancer+normal) |
| 7-157204960 | cg07993586 | (cancer vs cancer) (cancer vs cancer) |
| 7-1936854 | cg00236141 | (cancer vs cancer) |
| 7-1937120 | cg04326741 | (cancer vs cancer) (cancer vs cancer) |
| 7-5682666 | cg08180025 | (cancer vs cancer) |
| 7-8002109 | cg06640047 | (cancer vs cancer) |
| 7-88425758 | cg16781484 | (cancer vs cancer) |
| 7-94924376 | cg08533136 | (cancer vs cancer) |
| 7-94927713 | cg22798737 | (cancer vs cancer) (cancer vs cancer) |
| 8-100708621 | cg06258648 | (cancer vs cancer) (cancer vs cancer) |
| 8-119344904 | cg22533992 | (cancer vs cancer) (cancer vs cancer) |
| 8-144462851 | cg25555098 | (cancer vs cancer) |
| 8-144788492 | cg16530595 | (cancer vs cancer) (cancer vs cancer) |
| 8-145599544 | cg24513449 | (cancer vs cancer) (cancer vs cancer) (cancer+normal vs cancer+normal) |
| 8-145758588 | cg18811093 | (cancer vs cancer) (cancer vs cancer) |
| 8-40319233 | cg03521348 | (cancer vs cancer) |
| 9-139907691 | cg14173587 | (cancer vs cancer) (cancer vs cancer) (cancer+normal vs cancer+normal) |
| 9-139913237 | cg14341551 | (cancer vs cancer) |
| 9-140401576 | cg13461130 | (cancer vs cancer) |
| 9-86149293 | cg13858974 | (cancer vs cancer) (cancer vs cancer) |
| 9-96069142 | cg19989242 | (cancer vs cancer) (cancer vs cancer) |
| 9-96069195 | cg14505733 | (cancer vs cancer) (cancer vs cancer) |

## Claims

1. A method of detecting a methylation pattern of a set of biomarkers in a subject suspected of having a colorectal cancer, the method comprising:

   (a) processing an extracted genomic DNA with a deaminating agent to generate a genomic DNA sample comprising deaminated nucleotides, wherein the extracted genomic DNA is obtained from a biological sample from the subject; and
   (b) detecting the methylation pattern of the set of biomarkers from the processed extracted genomic DNA, wherein the set of biomarkers comprises cg10673833, cg10493436, cg10428836, cg27284288, cg16959747, cg17494199, cg23678254, cg24067911, and cg25459300, by contacting the extracted genomic DNA with a set of probes, wherein the set of probes hybridizes to the one or more biomarkers, and perform a DNA sequencing analysis to determine the methylation patter of the one or more biomarkers.

2. The method of claim 1, wherein the detecting the methylation pattern comprises contacting the processed extracted genomic DNA with a probe.

3. The method of claim 1 or 2, wherein said detecting comprises a real-time quantitative probe-based PCR or a digital probe-based PCR.

4. The method of claim 3, wherein the digital probe-based PCR is a digital droplet PCR.

5. The method of any of claims 1-4, wherein the detecting the methylation pattern comprises performing a DNA sequencing analysis to determine the methylation pattern of the one or more biomarkers.

6. The method of any of claims 1-5, wherein the methylation pattern comprises the methylation rate at each biomarker.

7. The method of claim 6, wherein the methylation rate is a Beta value.

8. The method of any of claims 1-7, wherein the methylation pattern is a combined diagnosis score (cd-score) or a combined prognosis score (cp-scope).

9. The method of any of claims 1-8, further comprising diagnosing the subject as having colorectal cancer.

10. The method of any of claims 1-9, wherein the biological sample is a blood sample, a urine sample, a saliva sample, a sweat sample, or a tear sample.

11. The method of any of claims 1-9, wherein the biological sample is a cell-free DNA sample.

12. The method of any of claims 1-9, wherein the biological sample comprises circulating tumor cells.

## Patentansprüche

1. Verfahren zum Nachweisen eines Methylierungsmusters eines Satzes von Biomarkern bei einem Patienten, bei dem der Verdacht auf Darmkrebs besteht, wobei das Verfahren Folgendes umfasst:

   (a) Verarbeiten einer extrahierten genomischen DNA mit einem Deaminierungsmittel, um eine genomische DNA-Probe zu erzeugen, die deaminierte Nukleotide umfasst, wobei die extrahierte genomische DNA aus einer biologischen Probe des Patienten erhalten wird; und
   (b) Nachweisen des Methylierungsmusters des Satzes von Biomarkern aus der verarbeiteten extrahierten genomischen DNA, wobei der Satz von Biomarkern cg10673833, cg10493436, cg10428836, cg27284288, cg16959747, cg17494199, cg23678254, cg24067911 und cg25459300 umfasst, indem die extrahierte genomische DNA mit einem Satz von Sonden in Kontakt gebracht wird, wobei der Satz von Sonden mit dem einen oder den mehreren Biomarkern hybridisiert, und eine DNA-Sequenzierungsanalyse durchgeführt wird, um das Methylierungsmuster des einen oder der mehreren Biomarker zu bestimmen.

2. Verfahren nach Anspruch 1, wobei das Nachweisen des Methylierungsmusters das Inkontaktbringen der verarbeiteten extrahierten genomischen DNA mit einer Sonde umfasst.

3. Verfahren nach Anspruch 1 oder 2, wobei das Nachweisen eine quantitative sondenbasierte Echtzeit-PCR oder eine digitale sondenbasierte PCR umfasst.

4. Verfahren nach Anspruch 3, wobei die digitale sondenbasierte PCR eine digitale Tröpfchen-PCR ist.

5. Verfahren nach einem der Ansprüche 1-4, wobei das Nachweisen des Methylierungsmusters das Durchführen einer DNA-Sequenzierungsanalyse umfasst, um das Methylierungsmuster des einen oder der mehreren Biomarker zu bestimmen.

6. Verfahren nach einem der Ansprüche 1-5, wobei das Methylierungsmuster die Methylierungsrate bei jedem Biomarker umfasst.

7. Verfahren nach Anspruch 6, wobei die Methylierungsrate ein Beta-Wert ist.

8. Verfahren nach einem der Ansprüche 1-7, wobei das Methylierungsmuster ein kombinierter Diagnose-Score (cd-score) oder ein kombinierter Prognose-Score (cp-score) ist.

9. Verfahren nach einem der Ansprüche 1-8, ferner umfassend das Diagnostizieren, dass der Patient an Darmkrebs leidet.

10. Verfahren nach einem der Ansprüche 1-9, wobei die biologische Probe eine Blutprobe, eine Urinprobe, eine Speichelprobe, eine Schweißprobe oder eine Tränenprobe ist.

11. Verfahren nach einem der Ansprüche 1-9, wobei die biologische Probe eine zellfreie DNA-Probe ist.

12. Verfahren nach einem der Ansprüche 1-9, wobei die biologische Probe zirkulierende Tumorzellen umfasst.

## Revendications

1. Procédé de détection d'un profil de méthylation d'un ensemble de biomarqueurs chez un sujet suspecté d'être atteint d'un cancer colorectal, le procédé consistant à :

(a) traiter un ADN génomique extrait avec un agent de désamination pour générer un échantillon d'ADN génomique comprenant des nucléotides désaminés, dans lequel l'ADN génomique extrait est obtenu à partir d'un échantillon biologique du sujet ; et
(b) détecter le profil de méthylation de l'ensemble de biomarqueurs à partir de l'ADN génomique extrait traité, dans lequel l'ensemble de biomarqueurs comprend cg10673833, cg10493436, cg10428836, cg27284288, cg16959747, cg17494199, cg23678254, cg24067911 et cg25459300, en mettant en contact l'ADN génomique extrait avec un ensemble de sondes, dans lequel l'ensemble de sondes s'hybrident auxdits un ou plusieurs biomarqueurs, et effectuer une analyse de séquençage d'ADN pour déterminer le profil de méthylation desdits un ou plusieurs biomarqueurs.

2. Procédé selon la revendication 1, dans lequel la détection du profil de méthylation comprend la mise en contact de l'ADN génomique extrait traité avec une sonde.

3. Procédé selon la revendication 1 ou 2, dans lequel ladite détection comprend une PCR quantitative en temps réel basée sur une sonde ou une PCR numérique basée sur une sonde.

4. Procédé selon la revendication 3, dans lequel la PCR numérique basée sur une sonde est une PCR numérique à gouttelettes.

5. Procédé selon l'une quelconque des revendications 1 à 4, dans lequel la détection du profil de méthylation comprend la réalisation d'une analyse de séquençage d'ADN pour déterminer le profil de méthylation du ou des biomarqueurs.

6. Procédé selon l'une quelconque des revendications 1 à 5, dans lequel le profil de méthylation comprend le taux de méthylation à chaque biomarqueur.

7. Procédé selon la revendication 6, dans lequel le taux de méthylation est une valeur bêta.

8. Procédé selon l'une quelconque des revendications 1 à 7, dans lequel le profil de méthylation est un score de diagnostic combiné (score cd) ou un score de pronostic combiné (score cp).

9. Procédé selon l'une quelconque des revendications 1 à 8, comprenant en outre le diagnostic du sujet comme étant atteint d'un cancer colorectal.

10. Procédé selon l'une quelconque des revendications 1 à 9, dans lequel l'échantillon biologique est un échantillon de sang, un échantillon d'urine, un échantillon de salive, un échantillon de sueur ou un échantillon de larmes.

11. Procédé selon l'une quelconque des revendications 1 à 9, dans lequel l'échantillon biologique est un échantillon d'ADN acellulaire.

12. Procédé selon l'une quelconque des revendications 1 à 9, dans lequel l'échantillon biologique comprend des cellules tumorales circulantes.

**Fig. 1**

**Fig. 2**

**Fig. 3**

Fig. 4

404

Fig. 5

Fig. 6

**Fig. 7**

# Fig. 8

EP 3 692 164 B1

**Fig. 9A**

**Fig. 9B**

**Fig. 9C**

| Training Dataset | Real HCC | Real Normal | |
|---|---|---|---|
| Predict HCC | 613 | 32 | |
| Predict Normal | 102 | 528 | Totals |
| Totals | 715 | 560 | 1275 |
| Correct | 613 | 528 | 1162 |
| False Negative | 102 | | |
| False Positive | | 32 | |
| Sensitivity(%) | 85.7 | | |
| Specificity (%) | | 94.3 | |

**Fig. 9D**

| Validation Dataset | Real HCC | Real Normal | |
|---|---|---|---|
| Predict HCC | 319 | 26 | |
| Predict Normal | 64 | 249 | Totals |
| Totals | 383 | 275 | 658 |
| Correct | 319 | 249 | 568 |
| False Negative | 64 | | |
| False Positive | | 26 | |
| Sensitivity (%) | 83.3 | | |
| Specificity(%) | | 90.5 | |

409

**Fig. 9E**

Performance of cd-score in traning datase

AUC=0.966
95%CI 0.958-0.975
Data Point
715T/560N

**Fig. 9F**

Performance of cd-score in Validation dataset

AUC=0.944
95%CI 0.928-0.961
Data Point
383T/275N

**Fig. 9G**

Training dataset

**Fig. 9H** Validation dataset

**Fig. 10A**

**Fig. 10B**

**Fig. 10C**

**Fig. 10D**

**Fig. 10E**

**Fig. 10F**

**Fig. 10G**

AUC of cd-score: 0.969 (0.926-0.977)
AUC of AFP: 0.816 (0.792-0.839)

**Fig. 10H**

**Fig. 10I**

**Fig. 10J**

**Fig. 10K**

**Fig. 11A**

Training dataset

Log-rank test *p*<0.0001
Hazard Ratio=0.15(0.09-0.25)

| Number at risk | | | | | | |
|---|---|---|---|---|---|---|
| Low-risk | | | | | | |
| 339 | 285 | 71 | 35 | 12 | 1 | 0 |
| High-risk | | | | | | |
| 341 | 260 | 93 | 47 | 11 | 0 | 0 |

**Fig. 11B**

Validation dataset

Log-rank test *p*=0.0014
Hazard Ratio=0.32(0.16-0.61)

| Number at risk | | | | | | |
|---|---|---|---|---|---|---|
| Low-risk | | | | | | |
| 172 | 139 | 47 | 21 | 12 | 1 | 0 |
| High-risk | | | | | | |
| 197 | 158 | 46 | 19 | 5 | 0 | 0 |

**Fig. 11C**

Training dataset

Log-rank test *p*<0.0001
Hazard Ratio=0.18(0.11-0.30)

| Number at risk | | | | | | |
|---|---|---|---|---|---|---|
| stage I/II | | | | | | |
| 285 | 239 | 59 | 33 | 12 | 0 | 0 |
| stage III/IV | | | | | | |
| 395 | 306 | 105 | 49 | 11 | 1 | 0 |

**Fig. 11D**

Validation dataset

Log-rank test *p*=0.0014
Hazard Ratio=0.22(0.11-0.43)

| Number at risk | | | | | | |
|---|---|---|---|---|---|---|
| stage I/II | | | | | | |
| 119 | 94 | 33 | 16 | 9 | 0 | 0 |
| stage III/IV | | | | | | |
| 251 | 203 | 60 | 24 | 8 | 1 | 0 |

EP 3 692 164 B1

**Fig. 11E**

ROC for 6 months survival predicting in training dataset

True negative rate

False negative rate

AUC of cp-score+stage: 0.7935
AUC of cp-score:0.7533
AUC of stage: 0.651

**Fig. 11F**

ROC for 6 months survival predicting in validation dataset

True negative rate

False negative rate

AUC of cp-score+stage: 0.7588
AUC of cp-score:0.675
AUC of stage:0.693

**Fig. 11G**

Log-rank test *p*<0.0001
Hazard Ratio=0.16(0.09-0.29)
Hazard Ratio=1.06(0.58-1.96)
Hazard Ratio=0.22(0.12-0.40)

Legend:
- Low-risk+stage I/II  (a)
- Low-risk+stage III/IV  (b)
- High-risk+stage I/II  (c)
- High-risk+stage III/IV  (d)

Number at risk

| Low-risk+stage I/II | | | | | | |
|---|---|---|---|---|---|---|
| 281 | 240 | 52 | 22 | 11 | 0 | 0 |

| Low-risk+stage III/IV | | | | | | |
|---|---|---|---|---|---|---|
| 227 | 181 | 64 | 34 | 13 | 2 | 0 |

| High-risk+stage I/II | | | | | | |
|---|---|---|---|---|---|---|
| 121 | 92 | 40 | 27 | 10 | 0 | 0 |

| High-risk+stage III/IV | | | | | | |
|---|---|---|---|---|---|---|
| 412 | 321 | 95 | 37 | 6 | 0 | 0 |

Fig. 12

**Fig. 13A**

**Fig. 13B**

# Fig. 14

Fig. 15A

Fig. 15B

# Fig. 16

**Fig. 17A**

**Fig. 17B**

| DNA type | Fraction of normal cfDNA | DNA type | Fraction of HCC gDNA | Methylated copy | Non-Methylated copy | M/(M+NM) | total copies |
|---|---|---|---|---|---|---|---|
| nor cfDNA | 100% | HCC gDNA | 0% | 58 | 924 | 5.90% | 982 |
| nor cfDNA | 90% | HCC gDNA | 10% | 134 | 784 | 14.60% | 918 |
| nor cfDNA | 70% | HCC gDNA | 30% | 266 | 654 | 28.90% | 920 |
| nor cfDNA | 40% | HCC gDNA | 60% | 420 | 532 | 44.10% | 952 |
| nor cfDNA | 0% | HCC gDNA | 100% | 694 | 334 | 67.50% | 1028 |

## Fig. 17C

cg10873833

## Fig. 18

**Fig. 19A**

**Fig. 19B**

**Fig. 19C**

**Fig. 19D**

Fig. 20A

Fig. 20C

Fig. 20B

Fig. 20D

EP 3 692 164 B1

**Fig. 21A**

**Fig. 21B**

LUNC

**Fig. 21C**

**Fig. 21D**

**Fig. 21E**

**Fig. 21F**

HCC

**Fig. 21G**

**Fig. 21H**

**Fig. 21I**                                        **Fig. 21J**

LUNC

**Fig. 21K**                                        **Fig. 21L**

**Fig. 21M**

**Fig. 21N**

HCC

**Fig. 21O**

**Fig. 21P**

**Fig. 21Q**

LUNC

cd-score (AUC:0.977)
n: normal=2247, LUNC=599
CEA (AUC:0.856)
n: normal=956, LUNC=205

**Fig. 21R**

**Fig. 22A**

**Fig. 22B**

**Fig. 22C**

**Fig. 22D**

**Fig. 22E**

**Fig. 22F**

**Fig. 23A**  Stage I LUNC: n=94
Benign nodules: n=126

**Fig. 23B**

| Validation Cohort | Stage I LUNC | Benign nodules | |
|---|---|---|---|
| Stage I LUNC | 77 | 22 | |
| Benign nodules | 17 | 104 | Totals |
| Totals | 94 | 126 | 220 |
| Correct | 77 | 104 | 181 |
| Sensitivity (%) | 81.9 | | |
| Specificity (%) | | 82.5 | |
| PPV (%) | 77.8 | | |
| NPV (%) | | 86.0 | |

**Fig. 24A**

| | Stage I HCC | Cirrhosis |
|---|---|---|
| Training | 148 | 162 |
| Validation | 75 | 79 |

**Fig. 24B**

**Fig. 24C**

| Validation Cohort | Stage I HCC | Cirrhosis | |
|---|---|---|---|
| Stage I HCC | 62 | 15 | |
| Cirrhosis | 13 | 64 | Totals |
| Totals | 75 | 79 | 154 |
| Correct | 62 | 64 | 126 |
| Sensitivity (%) | 82.7 | | |
| Specificity (%) | | 81.0 | |
| PPV (%) | 80.5 | | |
| NPV (%) | | 83.1 | |

**Fig. 24D**

| Cirrhosis Cohort | Progressed to Stage I HCC | Cirrhosis | |
|---|---|---|---|
| Stage I HCC | 17 | 4 | |
| Cirrhosis | 3 | 238 | Totals |
| Totals | 20 | 241 | 261 |
| Correct | 17 | 238 | 255 |
| Sensitivity (%) | 85.0 | | |
| Specificity (%) | | 98.8 | |
| PPV (%) | 85.0 | | |
| NPV (%) | | 98.8 | |

**Fig. 25A**

**Fig. 25B**

# Fig. 25C

Fig. 26

Fig. 27

**Fig. 28A**

**Fig. 28B**

# Fig. 29

Fig. 30

EP 3 692 164 B1

441

**Fig. 31A**

**Fig. 31B**

Fig. 32A

**Fig. 32B**

**Fig. 32C**

**Fig. 32D**

Training data set

**Fig. 32E**

Validation data set

**Fig. 32F**

Fig. 32G

| Training Dataset | Real CRC | Real normal | |
|---|---|---|---|
| Predict CRC | 46.2 | 6.8 | |
| Predict normal | 66 | 60.6 | Totals |
| Totals | 52.8 | 67.4 | 12.02 |
| Correct | 46.2 | 60.6 | 10.68 |
| Sensitivity (%) | 87.5 | | |
| Specificity (%) | | 89.9 | 88.9 |

Fig. 32H

| Validation Dataset | Real CRC | Real normal | |
|---|---|---|---|
| Predict CRC | 240 | 36 | |
| Predict normal | 33 | 311 | Totals |
| Totals | 273 | 347 | 620 |
| Correct | 240 | 311 | 551 |
| Sensitivity (%) | 87.9 | | |
| Specificity (%) | | 89.6 | 88.9 |

**Fig. 33A**

**Fig. 33B**

**Fig. 33C**

**Fig. 33D**

**Fig. 33E**

Fig. 34A

Fig. 34B

448

**Fig. 35A**

**Fig. 35B**

**Fig. 35C**

**Fig. 35E**

**Fig. 35D**

Fig. 36A

Fig. 36B

EP 3 692 164 B1

**Fig. 37A**

**Fig. 37B**

**Fig. 37C**

**Fig. 37D**

**Fig. 37E**

**Fig. 37F**

Fig. 38A

**Fig. 38B**

**Fig. 38C**

**Fig. 39**

**Fig. 40A**

**Fig. 40B**

## Fig. 41

**Fig. 42**

**Fig. 43**

# Fig. 44

## Fig. 45

## Fig. 46

**Fig. 47**

## Fig. 48

# Fig. 49

**Fig. 50**

# Fig. 51

# Fig. 52

## Fig. 53

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62569459
- US 62673593
- US 5786146 A
- US 6017704 A
- US 6200756 B
- US 6265171 B, Herman and Baylin
- US 20100144836 A, Van Engeland
- US 6331393 B, Laird
- WO 2006056480 A2, Relhi
- US 7186512 B
- US 7910296 B
- US 7901880 B
- US NO7459274 A
- US 7700324 B, Issa
- US 7553627 B
- US 476981
- US 20050069879 A
- US 20090155791 A, Wojdacz
- WO 2005012578 A1, Beaulieu
- WO 2009021141 A, Feinberg and Irizarry
- US 7611869 B, Fan
- WO 2009049916 A, Bayeyt
- US 6180349 B
- US 6033854 A
- US 5972602 A
- US 5525462 A, Takarada
- US 6114117 A, Hepp
- US 6127120 A, Graham
- US 6344317 B, Urnovitz
- US 6448001 B, Oku
- US 6528632 B, Catanzariti
- WO 2005111209 A, Nakajima
- US 4683202 A, Mullis
- US 7011944 B, Prudent
- US 6306597 B
- US 7598035 B, Macevicz
- US 7232656 B, Balasubramanian
- US 6797470 B
- US 7083917 B
- US 7166434 B
- US 7320865 B
- US 7332285 B
- US 7364858 B
- US 7429453 B, Barany
- US 7037687 B
- US 7645596 B, Williams
- US 7169560 B, Lapidus
- US 7769400 B, Harris

### Non-patent literature cited in the description

- **BETTEGOWDA et al.** Detection of Circulating Tumor DNA in Early- and Late-Stage Human Malignancies.. *Sci. Transl. Med*, 2014, vol. 6 (224), ra24
- **SADRI** ; **HORNSBY**. *Nucl. Acids Res.*, 1996, vol. 24, 5058-5059
- **XIONG** ; **LAIRD**. *Nucleic Acids Res.*, 1997, vol. 25, 2532-2534
- **FROMMER et al.** *Proc. Nat. Acad. Sci. USA*, 1992, vol. 89, 1827-1831
- **HERMAN et al.** *Proc. Nat. Acad. Sci. USA*, 1996, vol. 93, 9821-9826
- **FACKLER et al.** *Cancer Res.*, 2004, vol. 64 (13), 4442-4452
- **FACKLER et al.** *Clin. Cancer Res.*, 2006, vol. 12 (11), 3306-3310
- **EADS, C.A et al.** *Nucleic Acid Res.*, 2000, vol. 28, 32
- **COTTRELL et al.** *J. Urology*, 2007, vol. 177, 1753
- **COTTRELL et al.** *Nuc. Acids Res.*, 2004, vol. 32, e10
- **GONZALGO** ; **JONES**. *Nucleic Acids Res.*, 1997, vol. 25, 2529-2531
- **GEBHARD et al.** *Cancer Res.*, 2006, vol. 66, 6118-6128
- **GEBHARD et al.** *Nucleic Acids Res.*, 2006, vol. 34, e82
- **PELIZZOLA et al.** *Genome Res.*, vol. 18, 1652-1659
- **O'GEEN et al.** *BioTechniques*, 2006, vol. 41 (5), 577-580
- **WEBER et al.** *Nat. Genet.*, 2005, vol. 37, 853-862
- **HORAK** ; **SNYDER**. *Methods Enzymol*, 2002, vol. 350, 469-83
- **LIEB**. *Methods Mol Biol*, 2003, vol. 224, 99-109
- **SHIRAISHI et al.** *Proc. Natl. Acad. Sci. USA*, 1999, vol. 96 (6), 2913-2918
- **SAMBROOK et al.** Molecular Biology: A Laboratory Approach. Cold Spring Harbor, 1989

- **MCCLELLAND et al.** *NUCLEIC ACIDS RES.*, 1994, vol. 22 (17), 3640-59
- **TOYOTA et al.** *Cancer Res.*, 1999, vol. 59, 2307-2312
- **REIN et al.** *NUCLEIC ACIDS RES.*, 1998, vol. 26 (10), 2255-64
- **OLEK et al.** *NAT. GENET.*, 1997, vol. 17 (3), 275-6
- **DOBROVIC**. *Nuc. Acids Res.*, 2007, vol. 35 (6), e41
- **WOJDACZ et al.** *Nat. Prot.*, 2008, vol. 3 (12), 1903-1908
- **BALIC et al.** *J. Mol. Diagn.*, 2009, vol. 11, 102-108
- **CANDILORO et al.** *Epigenetics*, 2011, vol. 6 (4), 500-507
- **COSTELLO et al.** *Meth. Mol Biol*, 2002, vol. 200, 53-70
- **USHIJIMA** ; **YAMASHITA**. *Methods Mol Biol*, 2009, vol. 507 (1), 17-130
- **KOGA et al.** *Genome Res.*, 2009, vol. 19, 1462-1470
- **ACEVEDO et al.** *Cancer Res.*, 2008, vol. 68, 2641-2651
- **RAUCH et al.** *Proc. Nat. Acad. Sci. USA*, 2008, vol. 105, 252-257
- **DENG et al.** *Nat. Biotechnol*, 2009, vol. 27, 353-360
- **BALL et al.** *Nat. Biotechnol*, 2009, vol. 27, 361-368
- **DEGRAVES et al.** *BIOTECHNIQUES*, 2003, vol. 34 (1), 106-15
- **DEIMAN B et al.** *MOL. BIOTECHNOL.*, 2002, vol. 20 (2), 163-79
- **GIBSON et al.** *GENOME RESEARCH*, 1996, vol. 6, 995-1001
- **NOLTE**. *Adv. Clin. Chem.*, 1998, vol. 33, 201-235
- PCR Protocols, A Guide to Methods and Application. Academic Press, Inc., 1990
- **ZOU et al.** Sensitive Quantification of Methylated Markers with a Novel Methylation Specific Technology. *Association of Clinical Chemistry (AACC)*, 28 July 2010
- **MARGULIES et al.** *Nature*, vol. 437, 376-380
- **BIBKOVA et al.** *Genome Res.*, 2006, vol. 16, 383-393
- **HARRIS et al.** *Science*, 2008, vol. 320, 106-109
- **SONI** ; **MELLER**. *Clin. Chem.*, 2007, vol. 53, 1996-2001
- **TOST** ; **GUT**. *Nat. Prot.*, 2007, vol. 2, 2265-2275
- **NAKANO et al.** *J. Biotech.*, 2003, vol. 102, 117-124
- **RUCZINSKI et al.** *J. OF COMPUTATIONAL AND GRAPHICAL STATISTICS*, 2003, vol. 12, 475-511
- **FRIEDMAN, J. H.** *J. OF THE AMERICAN STATISTICAL ASSOCIATION*, 1989, vol. 84, 165-75
- **HASTIE** ; **TREVOR** ; **TIBSHIRANI** ; **ROBERT** ; **FRIEDMAN** ; **JEROME**. The Elements of Statistical Learning. Springer Series in Statistics, 2001
- **BREIMAN, L.** ; **FRIEDMAN, J. H** ; **OLSHEN, R. A** ; **STONE, C. J.** *Classification and regression trees*, 1984
- **BREIMAN, L.** *MACHINE LEARNING*, 2001, vol. 45, 5-32
- **PEPE, M. S.** The Statistical Evaluation of Medical Tests for Classification and Prediction. *Oxford Statistical Science Series*, 2003, vol. 28
- **DUDA, R. O** ; **HART, P. E** ; **STORK, D. O**. Pattern Classification. Wiley Interscience, 2001
- **RADMACHER et al.** *Journal of Computational Biology*, 2002, vol. 9, 505-511
- **DUDOIT et al.** *Journal of the American Statistical Association*, 2002, vol. 97, 77-87
- **RAMASWAMY et al.** *PNAS USA*, 2001, vol. 98, 15149-54
- **WRIGHT G. W.** ; **SIMON R.** *Bioinformatics*, 2003, vol. 19, 2448-2455
- **SIMON et al.** *Journal of the National Cancer Institute*, 2003, vol. 95, 14-18
- **BO** ; **JONASSEN**. *Genome Biology*, 2002, vol. 3 (4)
- **SIMON R** ; **LAM A.** BRB-ArrayTools User Guide, version 3.2.. *Biometric Research Branch*
- **DIEP, D**. *Nat Methods.*, 05 February 2012, vol. 9 (3), 270-272
- **LANGMEAD B** ; **SALZBERG S.** Fast gapped-read alignment with Bowtie 2.. *Nature Methods.*, 2012, vol. 9, 357-359
- **HANNUM et al.** *Mol Cell*, 2013, vol. 49, 359-367
- **DENG et al.** *Nature Biotechnology*, 2009, vol. 27, 353-360
- **DIEP et al.** *Nature Methods*, 2012, vol. 9, 270-272
- **PORRECA et al.** *Nature Methods*, 2007, vol. 4, 931-936
- **FRIEDMAN et al.** *J Stat Softw*, 2010, vol. 33, 1-22